# EUROPEAN PATENT SPECIFICATION

(11) **EP 1 261 718 B1**
(45) Date of publication and mention of the grant of the patent: **03.11.2010**
(21) Application number: 00987602.0
(22) Date of filing: 22.12.2000
(51) Int. Cl.: C12N 15/54, C12N 9/90, C07K 14/34, C12P 13/12, C12P 13/08

(54) **CORYNEBACTERIUM GLUTAMICUM GENES ENCODING METABOLIC PATHWAY PROTEINS**
CORYNEBACTERIUM GLUTAMICUM GENE, DIE FÜR STOFFWECHSELPROTEINE KODIEREN
GENES DE CORYNEBACTERIUM GLUTAMICUM CODANT DES PROTEINES DE VOIE METABOLIQUE

(30) Priority: 09.03.2000 US 187970 P; 23.06.2000 US 606740
(43) Date of publication of application: 04.12.2002
(73) Proprietor: Evonik Degussa GmbH, 45128 Essen (DE)
(72) Inventor: POMPEJUS, Markus, 67251 Freinsheim (DE); KRÖGER, Burkhard, 67117 Limburgerhof (DE); SCHRÖDER, Hartwig, 69226 Nussloch (DE); ZELDER, Oskar, 67346 Speyer (DE); HABERHAUER, Gregor, 67117 Limburgerhof (DE); KIM, Jun-Won, Seoul 136-701 (KR); LEE, Heung-Shick, Seoul 163-701 (KR); HWANG, Byung-Joon, Seoul 163-701 (KR)
(86) International application number: PCT/IB2000/002035
(87) International publication number: WO 2001/066573

(56) References cited:
- EP-A- 0 857 784
- EP-A2- 1 108 790
- WO-A-01/00843
- DATABASE EMBASE [Online] ELSEVIER SCIENCE PUBLISHERS, AMSTERDAM, NL; "Corynebacterium glutamicum beta C-S lyase (aecD) and branched-chain amino acid uptake carier (brnQ) gene." retrieved from NCBI/EBI Database accession no. M89931 XP002289620 & ROSSOL I ET AL: "THE CORYNEBACTERIUM GLUTAMICUM AECD GENE ENCODES A C-S LYASE WITH ALPHA,BETA-ELIMINATION ACTIVITY THAT DEGRADES MAINOETHYLCYSTEINE" JOURNAL OF BACTERIOLOGY, WASHINGTON, DC, US, vol. 174, no. 9, 1992, pages 2968-2977, XP001078779 ISSN: 0021-9193
- DATABASE EMBASE [Online] ELSEVIER SCIENCE PUBLISHERS, AMSTERDAM, NL; "Hypothetical protein TB31.7 from Mycobacterium tuberculosis" retrieved from EBI (EMBL) Database accession no. O06189 XP002289621
- INAMINE J.M. ET AL.: "Molecular and genetic characterization of lactose-metabolic genes" JOURNAL OF BACTERIOLOGY, vol. 167, no. 3, 1986, pages 855-862, XP002289619
- PARK S.D. ET AL.: "Isolation and analysis of metA, a methionine biosynthetic gene encoding homoserine acetyltransferase in Corynebacterium glutamicum." MOLECULES AND CELLS, vol. 8, no. 3, 30 June 1998 (1998-06-30), pages 286-294, XP001002218
- DATABASE EMBL SEQUENCES [Online] Accession No. AL021841, 13 February 1998 (1998-02-13) COLE S.T.: "Mycobacterium tubercolosis H37Rv complete genome." XP002168725 & COLE S.T. ET AL.: "Deciphering the biology of Mycobacterium tuberculosis from the complete genome sequence." NATURE, vol. 393, 1998, pages 537-344,
- PETERS-WENDISCH ET AL: "Pyruvate carboxylase as an anaplerotic enzyme in Corynebacterium glutamicum" MICROBIOLOGY,SOCIETY FOR GENERAL MICROBIOLOGY, READING,GB, vol. 143, no. PART 04, April 1997 (1997-04), pages 1095-1103, XP002110209 ISSN: 1350-0872
- CREMER J ET AL: "CONTROL OF THE LYSINE BIOSYNTHESIS SEQUENCE IN CORYNEBACTERIUM GLUTAMICUM AS ANALYZED BY OVEREXPRESSION OF THE INDIVIDUAL CORRESPONDING GENES" APPLIED AND ENVIRONMENTAL MICROBIOLOGY,WASHINGTON,DC,US, vol. 57, no. 6, 1 June 1991 (1991-06-01), pages 1746-1752, XP000616281 ISSN: 0099-2240

## Description

### Background of the Invention

Certain products and by-products of naturally-occucring metabolic processes in cells have utility in a wide array of industries, including the food, feed, cosmetics, and pharmaceutical industries. These molecules, collectively termed 'fine chemicals', include organic acids, both proteinogenic and non-proteinogenic amino acids, nucleotides and nucleosides, lipids and fatty acids, diols, carbohydrates, aromatic compounds, vitamins and cofactors, and enzymes. Their production is most conveniently performed through large-scale culture of bacteria developed to produce and secrete large quantities of a particular desired molecule. One particularly useful organism for this purpose is *Corynebacterium glutamicum,* a gram positive, nonpathogenic bacterium. Through strain selection, a number of mutant strains have been developed which produce an array of desirable compounds. However, selection of strains improved for the production of a particular molecule is a time-consuming and difficult process.

### Summery of the Invention

The invention provides a novel bacterial nucleic acid molecule which has a variety of uses. These uses include the identification of microorganisms which can be used to produce amino acids, such as, lysine and methionine), the modulation of said production in *C*. *glutamicum* or related bacteria, the typing or identification of *C. glutamicum* or related bacteria, as reference points for mapping the *C. glutamicum* genome, and as markers for transformation. This novel nucleic acid molecule encodes a protein, referred to herein as metabolic pathway (MP) protein.

*C. glutamicum* is a gram positive, aerobic bacterium which is commonly used in industry for the large-scale production of a variety of fine chemicals, and also for the degradation of hydrocarbons (such as in petroleum spills) and for the oxidation of terpenoids. The MP nucleic acid molecule of the invention, therefore, can be used to identify microorganisms which can be used to produce fine chemicals, *e.g*., by fermentation processes. Modulation of the expression of the MP nucleic acid of the invention, or modification of the sequence of the MP nucleic acid molecule of the invention, can be used to modulate the production of one or more fine chemicals from a microorganism (*e.g*., to improve the yield or production of one or more fine chemicals from a Corynebacterium or Brevibacterium species). In a preferred embodiment, the MP gene of the invention is combined with one or more genes involved in the same or different metabolic pathway to modulate the production of the above mentioned amino acids from a microorganism.

The MP nucleic acid of the invention may also be used to identify an organism as being *Corynebacterium glutamicum* or a close relative thereof, or to identify the presence of *C. glutamicum* or a relative thereof in a mixed population of microorganisms. The invention provides the nucleic acid sequence of a *C*. *glutamicum* gene; by probing the extracted genomic DNA of a culture of a unique or mixed population of microorganisms under stringent conditions with a probe spanning a region of a *C*. *glutamicum* gene which is unique to this organism, one can ascertain whether this organism is present. Although *Corynebacterium glutamicum* itself is nonpathogenic, it is related to species pathogenic in humans, such as "*Corynebacterium diphtheriae* (the causative agent of diphtheria); the detection of such organisms is of significant clinical relevance.

The MP nucleic acid molecule of the invention may also serve as reference points for mapping of the *C. glutamicum* genome, or of genomes of related organisms. Similarly, this molecule, or variants or portions thereof, may serve as markers for genetically engineered Corynebacterium or Brevibacterium species.

The MP protein encoded by the novel nucleic acid molecule of the invention is capable of, for example, performing an enzymatic step involved in the metabolism of amino acids, especially lysine and methionine. Given the availability of cloning vectors for use in *Corynebacterium glutamicum,* such as those disclosed in Sinskey *et al.,* U.S. Patent No. 4,649,119, and techniques for genetic manipulation of *C*. *glutamicum* and the related *Brevibacterium* species (*e.g., lactofermentum*) (Yoshihama et al, J. Bacteriol. 162: 591-597 (1985); Katsumata et al., J. Bacteriol. 159: 306-311 (1984); and Santamaria et al., J. Gen. Microbiol. 130: 2237-2246 (1984)), the nucleic acid molecule of the invention may be utilized in the genetic engineering of this organism to make it a better or more efficient producer of said amino acids.

This improved production or efficiency of production of said chemical may be due to a direct effect of manipulation of the gene of the invention, or it may be due to an indirect effect of such manipulation. Specifically, alterations in *C. glutamicum* metabolic pathways for amino acids, especially lysine and methionine, may have a direct impact on the overall production of one or more of these desired compounds from this organism. For example, optimizing the activity of a lysine or a methionine biosynthetic pathway protein or decreasing the activity of a lysine or methionine degradative pathway protein may result in an increase in the yield or efficiency of production of lysine or methionine from such an engineered ' organism. Alterations in the proteins involved in these metabolic pathways may also have an indirect impact on the production or efficiency of production of a desired fine chemical. For example, a reaction which is in competition for an intermediate necessary for the production of a desired molecule may be eliminated, or a pathway necessary for the production of a particular intermediate for a desired compound may be optimized. Further, modulations in the biosynthesis or degradation of, lysine or methionine may increase the overall ability of the microorganism to rapidly grow and divide, thus increasing the number and/or production capacities of the microorganism in culture and thereby increasing the possible yield of the desired fine chemical.

The nucleic acid and protein molecule of the invention, alone or in combination with one or more nucleic acid and protein molecules of the same or different metabolic pathway, may be utilized to directly improve the production or efficiency of production of said fine chemicals from *Corynebacterium glutamicum* (*e.g*., methionine or lysine). Using recombinant genetic techniques well known in the art, one or more of the biosynthetic or degradative enzymes of the invention for amino acids, especially lysine and methionine may be manipulated such that its function is modulated. For example, a biosynthetic enzyme may be improved in efficiency, or its allosteric control region destroyed such that feedback inhibition of production of the compound is prevented. Similarly, a degradative enzyme may be deleted or modified by substitution, deletion, or addition such that its degradative activity is lessened for the desired compound without impairing the viability of the cell. In each case, the overall yield or rate of production of the desired fine chemical may be increased.

It is also possible that such alterations in the protein and nucleotide molecule of the invention may improve the production of other fine chemicals besides the amino acids, *e.g.*, lysine and methionine, vitamins, cofactors, nutraceuticals, nucleotides, nucleosides, and trehalose through indirect mechanisms. Metabolism of any one compound is necessarily intertwined with other biosynthetic and degradative pathways within the cell, and necessary cofactors, intermediates, or substrates in one pathway are likely supplied or limited by another such pathway. Therefore, by modulating the activity of the protein of the invention, the production or efficiency of activity of another fine chemical biosynthetic or degradative pathway may be impacted. For example, amino acids serve as the structural units of all proteins, yet may be present intracellularly in levels which are limiting for protein synthesis; therefore, by increasing the efficiency of production or the yields of one or more amino acids within the cell, proteins, such as biosynthetic or degradative proteins, may be more readily synthesized. Likewise, an alteration in a metabolic pathway enzyme such that a particular side reaction becomes more or less favored may result in the over- or under-production of one or more compounds which are utilized as intermediates or substrates for the production of a desired fine chemical.

Genes encoding metabolic pathway ("MP") proteins of Corynebacterium glutamicum are known in prior art (Peters-Wendisch; Microbiology 143(4):1095-1103 (1997)). Moreover, methods of modulating the yield of fine chemicals (namely lysine) from a cell, comprising the introduction of one or more metabolic genes into said cell are known in prior art (Cremer; Appl. Environm. Microbiol 57(6):1746-1752 (1991)).

The present description discloses other examples of MP proteins and their corresponding nucleic acids in Table 1 and SEQ ID NO:s 3-122. Among these, the proteins of SEQ ID NO:4 and SEQ ID NO:6 and their corresponding nucleic acids (SEQ ID NO:3 and SEQ ID NO:5, respectively) are presented as potentially useful.

In addition, the present invention discloses and claims protection for a further MP protein, namely that of SEQ ID NO:2, and for the corresponding nucleic acid (SEQ ID NO:1). This new gene has been called metZ.

The protein of the invention, like other MP protein, is capable of, for example, performing an enzymatic step involved in the metabolism of molecules important for the normal functioning of cells, such as amino acids, *e.g*., lysine and Methionine. Nucleic acid molecules encoding an MP protein are referred to herein as MP nucleic acid molecules. In a preferred embodiment, the MP protein of the invention alone or in combination with one or more other proteins of the same or different metabolic pathway, performs an enzymatic step related to the metabolism of one or more of the following: amino acids, *e.g*., lysine and methionine, Examples of such other proteins include those encoded by the genes set forth in Table 1.

Accordingly, one aspect of the description pertains to a isolated nucleic acid molecule (*e.g*., cDNAs, DNAs, or RNAs) comprising a nucleotide sequence encoding an MP protein or biologically active portions thereof, as well as nucleic acid fragments suitable as primers or hybridization probes for the detection or amplification of MP-encoding nucleic acid (*e.g*., DNA or mRNA). The isolated nucleic acid molecule comprises the nucleotide sequence set forth as SEQ ID NO:1 ), or the coding region or a complement thereof of one of these nucleotide sequences. In other embodiments, the isolated nucleic acid molecule of the description comprises a nucleotide sequence which hybridizes to or is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to the nucleotide sequence set fourth as SEQ ID NO:1, or a portion thereof. In other part of invention, the isolated nucleic acid molecule encodes the amino acid sequences set forth in SEQ ID NO:2 The MP protein of the present invention also preferably possess at least one of the MP activities described herein.

In Another embodiment of the description the isolated nucleic acid molecule encodes a protein or portion thereof wherein the protein or portion thereof includes an amino acid sequence which is sufficiently homologous to the amino acid sequence of the invention ( SEQ ID NO:2), *e.g*., sufficiently homologous to an amino acid sequence of the invention such that the protein or portion thereof maintains an MP activity. Preferably, the protein or portion thereof encoded by the nucleic acid molecule maintains the ability to perform an enzymatic reaction in a amino acid, *e.g.*, lysine or methionine, vitamin, cofactor, nutraceutical, nucleotide, nucleoside, or trehalose metabolic pathway. In one embodiment, the protein encoded by the nucleic acid molecule is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to the amino acid sequence of the invention (*e.g*., an entire amino acid sequence SEQ ID NO:2). In another preferred embodiment, the protein is a full length *C*. *glutamicum* protein which is substantially homologous to the entire amino acid sequence of the invention (encoded by an open reading frame shown in SEQ ID NO: 1,)

In another preferred embodiment of description, the isolated nucleic acid molecule is derived from *C. glutamicum* and encodes a protein (*e.g*., an MP fusion protein) which includes a biologically active domain which is at least about 50% or more homologous to one of the amino acid sequences of the invention ( SEQ ID NO:2 ) and is able to catalyze a reaction in a metabolic pathway for an amino acid, *e.g*., lysine or methionine, vitamin, cofactor, nutraceutical, nucleotide, nucleoside, or trehalose, or one or more of the activities set forth in Table 1, and which also includes heterologous nucleic acid sequences encoding a heterologous polypeptide or regulatory regions.

In another embodiment of description, the isolated nucleic acid molecule is at least 15 nucleotides in length and hybridizes under stringent conditions to a nucleic acid molecule comprising a nucleotide sequence of the invention ( SEQ ID NO:1. ). Preferably, the isolated nucleic acid molecule corresponds to a naturally-occurring nucleic acid molecule. More preferably, the isolated nucleic acid encodes a naturally-occurring *C*. *glutamicum* MP protein, or a biologically active portion thereof.

Another aspect of the invention pertains to vectors, *e.g*., recombinant expression vectors, containing the nucleic acid molocule of the invention, alone or in combination with one or more nucleic acid molecules involved in the same or different pathway, and host cells into which such vectors have been introduced. In one embodiment, such a host cell is used to produce an MP protein by culturing the host cell in a suitable medium. The MP protein can be then isolated from the medium or the host cell.

Yet another aspect of the invention pertains to a genetically altered microorganism in which the MP genes of the invention, alone or in combination with one or more genes involved in the same or different metabolic pathway, have been introduced or altered. In one embodiment, the genome of the microorganism has been altered by introduction of the nucleic acid molecule of the invention encoding one or more wild-type or mutated MP sequences as transgenes alone or in combination with one or more nucleic acid molecules involved in the same or different metabolic pathway. In another embodiment, the endogenous MP genes within the genome of the microorganism have been altered, *e.g*., functionally disrupted, by homologous recombination with one or more altered MP genes. In another embodiment, the endogenous or introduced MP gene, alone or in combination with one or more genes of the same or different metabolic pathway in a microorganism have been altered by one or more point mutations, deletions, or inversions, but still encode functional MP proteins. In still another embodiment, one or more of the regulatory regions (*e*.g., a promoter, repressor, or inducer) of the MP gene in a microorganism, alone or in combination with one or more MP genes or in combination with one or more genes of the same or different metabolic pathway, has been altered (*e.g.,* by deletion, truncation, inversion, or point mutation) such that the expression of one or more MP genes is modulated. In a preferred embodiment, the microorganism belongs to the genus *Corynebacterium* or *Brevibacterium,* with *Corynebacterium glutamicum* being particularly preferred. In a preferred embodiment, the microorganism is also utilized for the production of a desired compound, such as an amino acid, with lysine and methionine being particularly preferred. The invention discloses the MP gene *metZ* gene (SEQ ID NO:1), and the description contains *metC* gene (SEQ ID NO:3), or the RXA00657 gene (SEQ ID NO:5), alone or in combination with the MP gene of the invention or in combination with one or more genes involved in methionine and/or lysine metabolism.

In another aspect, the description provides a method of identifying the presence or activity of *Cornryebacterium diphtheriae* in a subject. This method includes detection of the nucleic acid or amino acid sequences of the invention alone or in combination with other MP disclosed herein, i.e the sequences set forth in Table 1 and in the Sequence Listing as SEQ ID NOs 3 through 122) in a subject, thereby detecting the presence or activity of *Corynebacterium diphtheriae* in the subject.

Still another aspect of the invention pertains to an isolated MP protein or portion, *e.g*., biologically active portion, thereof. In a preferred embodiment, the isolated MP protein, alone or in combination with one or more MP proteins of the description or in combination with one or more proteins of the same or different metabolic description or in combination with one or more proteins of the same or different metabolic pathway, can catalyze an enzymatic reaction involved in one or more pathways for the metabolism of an amino acid, *e.g*., lysine or methionine, a vitamin, a cofactor, a nutraceutical, a nucleotide, a nucleoside, or trehalose. In another embodiment of the description the isolated MP protein or portion thereof, is sufficiently homologous to an amino acid sequence of the invention ( SEQ ID NO:2) such that the protein or portion thereof maintains the ability to catalyze an enzymatic reaction involved once or more pathways for the metabolism of an amino acid, a vitamin, a cofactor, a nutraceutical, a nucleotide, a nucleoside, or trehalose.

The invention also provides an isolated preparation of an MP protein. In preferred embodiments, the MP protein comprises the amino acid sequence of the invention SEQ ID NO:2. In another preferred embodiment, the description pertains to an isolated full length protein which is substantially homologous to an entire amino acid sequence of the invention ( SEQ ID NO:2) (encoded by an open reading frame set forth in SEQ ID NO:1 The protein may be at least about 50%, 51%, 52%, 53%, 54%, 5.5%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to an entire amino acid sequence of the invention SEQ ID NO:2, In other embodiments, of the description the isolated MP protein comprises an amino acid sequence which is at least about 50% or more homologous to one of the amino acid sequences of the invention ( SEQ ID NO:2) and is able to catalyze an enzymatic reaction in an amino acid, vitamin, cofactor, nutraceutical, nucleotide, nucleoside, or trehalose metabolic pathways either alone or in combination one or more MP proteins of the invention or any protein of the same or different metabolic pathway, or has one or more of the activities set forth in Table 1.

Alternatively, the isolated MP protein of the description can comprise an amino acid sequence which is encoded by a nucleotide sequence which hybridizes, *e.g*., hybridizes under stringent conditions, or is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to a nucleotide sequence of one of the invention. It is also preferred that the preferred forms of MP proteins also have one or more of the MP bioactivities described herein.

The MP polypeptide, or a biologically active portion thereof, can be operatively linked to a non-MP polypeptide to form a fusion protein. Preferably fusion protein has an activity which differs from that of the MP protein alone. In other preferred embodiments, this fusion protein, when introduced into a *C. glutamicum* pathway for the metabolism of an amino acid, vitamin, cofactor, nutraceutical, results in increased yields and/or efficiency of production of a desired fine chemical from *C*. *glutamicum.* Preferably, integration of this fusion protein into an amino acid, vitamin, cofactor, nutraceutical, nucleotide, nucleoside, or trehalose metabolic pathway of a host cell modulates production of a desired compound from the cell.

In another aspect, the description provides methods for screening molecules which modulate the activity of an MP protein, either by interacting with the protein itself or a substrate or binding partner of the MP protein, or by modulating the transcription or translation of an MP nucleic acid molecule of the invention.

Another aspect of the invention pertains to a method for producing said chemical. This method involves the culturing of a cell containing one or more vectors directing the expression of the claimed MP nucleic acid molecule of the invention either alone or in combination with one or more other MP nucleic acid molecules , such that said chemical is produced. In a preferred embodiment, this method further includes the step of obtaining a cell containing such a vector, in which a cell is transfected with a vector directing the expression of an MP nucleic acid. In another preferred embodiment, this method further includes the step of recovering the chemical from the culture. In a particularly preferred embodiment, the cell is from the genus *Corynebacterium* or *Brevibacterium,* or is selected from those strains set forth in Table 3. In another embodiment, the MP gene is used in combination with one or more MP nucleic acid molecules involved in methionine and/or lysine metabolism. The fine chemical is an amino acid, *e.g*., L-lysine and L-methionine.

Another aspect of the description pertains to methods for modulating production of a molecule from a microorganism. Such methods include contacting the cell with an agent which modulates MP protein activity or MP nucleic acid expression such that a cell associated activity is altered relative to this same activity in the absence of the agent In a preferred embodiment, the cell is modulated for one or more *C*. *glutamicum* amino acid, vitamin, cofactor, nutraceutical, nucleotide, nucleoside, or trehalose metabolic pathways, such that the yields or rate of production of a desired fine chemical by this microorganism is improved. The agent which modulates MP protein activity can be an agent which stimulates MP protein activity or MP nucleic acid expression. Examples of agents which stimulate MP protein activity or MP nucleic acid expression include small molecules, active MP proteins; and nucleic acids encoding MP proteins that have been introduced into the cell. Examples of agents which inhibit MP activity or expression include small molecules and antisense MP nucleic acid molecules.

Another aspect of the invention pertains to methods for modulating yields of a desired compound from a cell, involving the introduction of a wild-type or mutant MP gene into a cell, either alone or in combination one or more MP nucleic acid molecules of the invention or any nucleic acid molecule of the same or different metabolic pathway, either maintained on a separate plasmid or integrated into the genome of the host cell. If integrated into the genome, such integration can be random, or it can take place by homologous recombination such that the native gene is replaced by the introduced copy, causing the production of the desired compound from the cell to be modulated. In a preferred embodiment, said yields are increased. In another preferred embodiment, said chemical is a fine chemical. The fine chemical is an amino acid. In especially preferred embodiments, said amino acid are L-lysine and L-methionine. In another preferred embodiment, said gene is the *metZ* gene (SEQ ID NO:1), alone or combination with *metC gene* (SEQ ID NO:3), or the RXA00657 gene (SEQ ID NO:5), or in combination with one or more MP nucleic acid molecules of the description or with one or more genes involved in methionine and/or lysine metabolism.

### Detailed Description of the Invention

The present invention provides a MP nucleic acid and protein involved in the metabolism of amino acids in *Corynebacterium glutamicum,* especially lysine and methionine. The molecules of the invention may be utilized in the modulation of production of said chemicals from microorganisms, such as *C*. *glutamicum,* either directly (*e.g*., where modulation of the activity of a lysine or methionine biosynthesis protein has a direct impact on the production or efficiency of production of lysine or methionine from that organism), or may have an indirect impact which nonetheless results in an increase of yield or efficiency of production of the desired compound (*e.g*., where modulation of the activity of a nucleotide biosynthesis protein has an impact on the production of an organic acid or a fatty acid from the bacterium, perhaps due to improved growth or an increased supply of necessary co-factors, energy compounds, or precursor molecules). The MP molecules may be utilized alone or in combination with other MP molecules of the description, or in combination with other molecules involved in the same or a different metabolic pathway (*e.g*., lysine or methione metabolism). In a preferred embodiment, the MP molecules are the *metZ* (SEQ ID NO:1)*,* alone or in combination with *metC* (SEQ ID NO:3), or RXA00657 (SEQ ID NO:5) nucleic acid molecules or the proteins encoded by these nucleic acid molecules ( SEQ ID NO.:4 and SEQ ID NO.:6, respectively). Aspects of the invention are further explicated below.

### I. Fine Chemicals

The term 'fine chemical' is art-recognized and includes molecules produced by an organisim which have applications in various industries, such as, but not limited to, the pharmaceutical, agriculture, and cosmetics industries. Such compounds include organic acids, such as tartaric acid, itaconic acid, and diaminopimelic acid, both proteinogenic and non-proteinogenic amino acids, purine and pyrimidine bases, nucleosides, and nucleotides (as described *e.g*. in Kuninaka, A. (1996) Nucleotides and related compounds, p. 561-612, in Biotechnology vol. 6, Rehm *et al.,* eds. VCH: Weinheim, and references contained therein), lipids, both saturated and unsaturated fatty acids (*e.g*., arachidonic acid), diols (*e.g*., propane diol, and butane diol), carbohydrates (*e.g*., hyaluronic acid and trehalose), aromatic compounds (*e.g*., aromatic amines, vanillin, and indigo), vitamins and cofactors (as described in Ullmann's Encyclopedia of Industrial Chemistry, vol. A27, "Vitamins", p. 443-613 (1996) VCH: Weinheim and references therein; and Ong, A.S., Niki, E. & Packer, L. (1995) "Nutrition, Lipids, Health, and Disease" Proceedings of the UNESCO/Confederation of Scientific and Technological Associations in Malaysia, and the Society for Free Radical Research - Asia, held Sept. 1-3, 1994 at Penang, Malaysia, AOCS Press, (1995)), enzymes, polyketides (Cane et al. (1998) Science 282: 63-68), and all other chemicals described in Gutcho (1983) Chemicals by Fermentation, Noyes Data Corporation, ISBN: 0818805086 and references therein. The metabolism and uses of certain of these fine chemicals are further explicated below.

### A. Amino Acid Metabolism and Uses

Amino acids comprise the basic structural units of all proteins, and as such are essential for normal cellular functioning in all organisms. The term "amino acid" is art-recognized. The proteinogenic amino acids, of which there are 20 species, serve as structural units for proteins, in which they are linked by peptide bonds, while the nonproteinogenic amino acids (hundreds of which are known) are not normally found in proteins (see Ulmann's Encyclopedia of Industrial Chemistry, vol. A2, p. 57-97 VCH: Weinheim (1985)). Amino acids may be in the D- or L- optical configuration, though L-amino acids are generally the only type found in naturally-occurring proteins. Biosynthetic and degradative pathways of each of the 20 proteinogenic amino acids have been well characterized in both prokaryotic and eukaryotic cells (see, for example, Stryer, L. Biochemistry, 3^{rd} edition, pages 578-590 (1988)). The essential' amino acids (histidine, isoleucine, leucine, lysine, methionine, phenylalanine, threonine, tryptophan, and valine), so named because they are generally a nutritional requirement due to the complexity of their biosyntheses, are readily converted by simple biosynthetic pathways to the remaining 11 'nonessential' amino acids (alanine, arginine, asparagine, aspartate, cysteine, glutamate, glutamine, glycine, proline, serine, and tyrosine). Higher animals do retain the ability to synthesize some of these amino acids, but the essential amino acids must be supplied from the diet in order for normal protein synthesis to occur.

Aside from their function in protein biosynthesis, these amino acids are interesting chemicals in their own right, and many have been found to have various applications in the food, feed, chemical, cosmetics, agriculture, and pharmaceutical industries. Lysine is an important amino acid in the nutrition not only of humans, but also ofmonogastric animals such as poultry and swine. Glutamate is most commonly used as a flavor additive (mono-sodium glutamate, MSG) and is widely used throughout the food industry, as are aspartate, phenylalanine, glycine, and cysteine. Glycine, L-methionine and tryptophan are all utilized in the pharmaceutical industry. Glutamine, valine, leucine, isoleucine, histidine, arginine, proline, serine and alanine are of use in both the pharmaceutical and cosmetics industries. Threonine, tryptophan, and D/ L-methionine are common feed additives. (Leuchtenberger, W. (1996) Amino aids - technical production and use, p. 466-502 in Rehm *et al.* (eds.) Biotechnology vol. 6, chapter 14a, VCH: Weinheim). Additionally, these amino acids have been found to be useful as precursors for the synthesis of synthetic amino acids and proteins, such as N-acetylcysteine, S-carboxymethyl-L-cysteine, (S)-5-hydroxytryptophan, and others described in Ulmann's Encyclopedia of Industrial Chemistry, vol. A2, p. 57-97, VCH: Weinheim, 1985.

The biosynthesis of these natural amino acids in organisms capable of producing them, such as bacteria, has been well characterized (for review of bacterial amino acid biosynthesis and regulation thereof, see Umbarger, H.E. (1978) Ann. Rev. Biochem. 47: 533-606). Glutamate is synthesized by the reductive amination of α-ketoglutarate, an intermediate in the citric acid cycle. Glutamine, proline, and arginine are each subsequently produced from glutamate. The biosynthesis of serine is a three-step process beginning with 3-phosphoglycerate (an intermediate in glycolysis), and resulting in this amino acid after oxidation, transamination, and hydrolysis steps. Both cysteine and glycine are produced from serine; the former by the condensation of homocysteine with serine, and the latter by the transferal of the side-chain β-carbon atom to tetrahydrofolate, in a reaction catalyzed by serine transhydroxymethylase. Phenylalanine and tyrosine are synthesized from the glycolytic and pentose phosphate pathway precursors erythrose 4-phosphate and phosphoenolpyruvate in a 9-step biosynthetic pathway that differ only at the final two steps after synthesis of prephenate. Tryptophan is also produced from these two initial molecules, but its synthesis is an 11-step pathway. Tyrosine may also be synthesized from phenylalanine, in a reaction catalyzed by phenylalanine hydroxylase. Alanine, valine, and leucine are all biosynthetic products of pyruvate, the final product of glycolysis. Aspartate is formed from oxaloacetate, an intermediate of the citric acid cycle. Asparagine, methionine, threonine, and lysine are each produced by the conversion of aspartate. Isoleucine is formed from threonine.

The biosynthetic pathways leading to methionine have been studied in diverse organisms. The first step, acylation of homoserine, is common to all of the organisms, even though the source of the transferred acyl groups is different. *Escherichia* coli and the related species use succinyl-CoA (Michaeli, S. and Ron, E. Z. (1981) Mol. Gen. Genet. 182, 349-354), while *Saccharomyces cerevisiae* (Langin, T., et al. (1986) Gene 49, 283-293), *Brevibacteriumflavum* (Miyajima, R. and Shiio, I. (1973) J. Biochem. 73, 1061-1068; Ozaki, H. and Shiio, I. (1982) J. Biochem. 91, 1.163-1171), C. *glutamicum* (Park, S.-D., et al. (1998) Mol. Cells 8, 286-294), and *Leptospira meyeri* (Belfaiza, J. *et al.* (1998) 180, 250-255; Bourhy, P., et al. (1997) J. Bacteriol. 179, 4396-4398) use acetyl-CoA as the acyl donor. Formation ofhomocysteine from acylhomoserine can occur in two different ways. *E. coli* uses the transsulfuration pathway which is catalyzed by cystathionine γ-synthase (the product of *metB*) and cystathionine β-lyase (the product of *metC*). *S*. *cerevisiae* (Cherest, H. and Surdin-Kerjan, Y. (1992) Genetics 130,51-58), *B. flavum* (Ozaki, H. and Shiio, I. (1982) J. Biochem. 91, 1163-1171), *Pseudomonas aeruginosa* (Foglino, M., et al. (1995) Microbiology 141, 431-439), and *L. meyeri* (Belfaiza, J., et al. (1998) J. Bacteriol. 180, 250-255) utilize the direct sulfhydrylation pathway which is catalyzed by acylhomoserine sulfhydrylase. Unlike closely related *B. flavum* which uses only the direct sulfhydrylation pathway, enzyme activities of the transsulfuration pathway have been detected in the extracts of the *C*. *glutamicum* cells and the pathway has been proposed to be the route for methionine biosynthesis in the organism (Hwang, B-J., et al. (1999) Mol. Cells 9, 300-308; Kase, H. and Nakayama, K. (1974) Agr. Biol. Chem. 38, 2021-2030; Park, S.-D., et al. 1998) Mol. Cells 8, 286-294).

Although some genes involved in methionine biosynthesis in *C. glutamicum* have been isolated, information on the biosynthesis of methionine in *C*. *glutamicum* is still very limited. No genes other than *metA* and *metB* have been isolated from the organism. To understand the biosynthetic pathways leading to methionine in *C*. *glutamicum,* we have isolated and characterized the *metC* gene (SEQ ID NO:3) and the *metZ* (also called *metY*) gene (SEQ ID NO:1) of *C*. *glutamicum* (see Table 1).

Amino acids in excess of the protein synthesis needs of the cell cannot be stored, and are instead degraded to provide intermediates for the major metabolic pathways of the cell (for review see Stryer, L. Biochemistry 3rd ed. Ch. 21 "Amino Acid Degradation and the Urea Cycle" p. 495-516 (1988)). Although the cell is able to convert unwanted amino acids into useful metabolic intermediates, amino acid production is costly in terms of energy, precursor molecules, and the enzymes necessary to synthesize them. Thus it is not surprising that amino acid biosynthesis is regulated by feedback inhibition, in which the presence of a particular amino acid serves to slow or entirely stop its own production (for overview of feedback mechanisms in amino acid biosynthetic pathways, see Stryer, L. Biochemistry, 3rd ed. Ch. 24: "Biosynthesis of Amino Acids and Heme" p. 575-600 (1988)). Thus, the output of any particular amino acid is limited by the amount of that amino acid present in the cell.

### B. Vitamin, Cofactor, and Nutraceutical Metabolism and Uses

Vitamins, cofactors, and nutraceuticals comprise another group of molecules which the higher animals have lost the ability to synthesize and so must ingest, although they are readily synthesized by other organisms, such as bacteria. These molecules are either bioactive substances themselves, or are precursors of biologically active substances which may serve as electron carriers or intermediates in a variety of metabolic pathways. Aside from their nutritive value, these compounds also have significant industrial value as coloring agents, antioxidants, and catalysts or other processing aids. (For an overview of the structure, activity, and industrial applications of these compounds, see, for example, Ullman's Encyclopedia of Industrial Chemistry, "Vitamins" vol. A27, p. 443-613, VCH: Weinheim, 1996.) The term "vitamin" is art-recognized, and includes nutrients which are required by an organism for normal functioning, but which that organism cannot synthesize by itself. The group of vitamins may encompass cofactors and nutraceutical compounds. The language "cofactor" includes nonproteinaceous compounds required for a normal enzymatic activity to occur. Such compounds may be organic or inorganic; the cofactor molecules of the invention are preferably organic. The term "nutraceutical" includes dietary supplements having health benefits in plants and animals, particularly humans. Examples of such molecules are vitamins, antioxidants, and also certain lipids (*e.g*., polyunsaturated fatty acids).

The biosynthesis of these molecules in organisms capable of producing them, such as bacteria, has been largely characterized (Ullman's Encyclopedia of Industrial Chemistry, "Vitamins" vol. A27, p. 443-613, VCH: Weinheim, 1996; Michal, G. (1999) Biochemical Pathways: An Atlas of Biochemistry and Molecular Biology, John Wiley & Sons; Ong, A.S., Niki, E. & Packer, L. (1995) "Nutrition, Lipids, Health, and Disease" Proceedings of the UNESCO/Confederation of Scientific and Technological Associations in Malaysia, and the Society for Free Radical Research - Asia, held Sept. 1-3, 1994 at Penang, Malaysia, AOCS Press: Champaign, IL X, 374 S).

Thiamin (vitamin B₁) is produced by the chemical coupling of pyrimidine and thiazole moieties. Riboflavin (vitamin B₂) is synthesized from guanosine-5'-triphosphate (GTP) and ribose-5'-phosphate. Riboflavin, in turn, is utilized for the synthesis of flavin mononucleotide (FMN) and flavin adenine dinucleotide (FAD). The family of compounds collectively termed 'vitamin B₆' (*e.g*., pyridoxine, pyridoxamine, pyridoxa-5'-phosphate, and the commercially used pyridoxin hydrochloride) are all derivatives of the common structural unit, 5-hydroxy-6-methylpyridine. Pantothenate (pantothenic acid, (R)-(+)-N-(2,4-dihydroxy-3,3-dimethyl-1-oxobutyl)-β-alanine) can be produced either by chemical synthesis or by fermentation. The final steps in pantothenate biosynthesis consist of the ATP-driven condensation of β-alanine and pantoic acid. The enzymes responsible for the biosynthesis steps for the conversion to pantoic acid, to β-alanine and for the condensation to panthotenic acid are known. The metabolically active form of pantothenate is Coenzyme A, for which the biosynthesis proceeds in 5 enzymatic steps. Pantothenate, pyridoxal-5'-phosphate, cysteine and ATP are the precursors of Coenzyme A. These enzymes not only catalyze the formation of panthothante, but also the production of (R)-pantoic acid, (R)-pantolacton, (R)-panthenol (provitamin B₅), pantetheine (and its derivatives) and coenzyme A.

Biotin biosynthesis from the precursor molecule pimeloyl-CoA in microorganisms has been studied in detail and several of the genes involved have been identified. Many of the corresponding proteins have been found to also be involved in Fe-cluster synthesis and are members of the nifS class of proteins. Lipoic acid is derived from octanoic acid, and serves as a coenzyme in energy metabolism, where it becomes part of the pyruvate dehydrogenase complex and the α-ketoglutarate dehydrogenase complex. The folates are a group of substances which are all derivative of folic acid, which is turn is derived from L-glutamic acid, p-amino-benzoic acid and 6-methylpterin. The biosynthesis of folic acid and its derivatives, starting from the metabolism intermediates guanosine-5'-triphosphate (GTP), L-glutamic acid and p-amino-benzoic acid has been studied in detail in certain microorganisms.

Corrinoids (such as the cobalamines and particularly vitamin B₁₂) and porphyrines belong to a group of chemicals characterized by a tetrapyrole ring system. The biosynthesis of vitamin B₁₂ is sufficiently complex that it has not yet been completely characterized, but many of the enzymes and substrates involved are now known. Nicotinic acid (nicotinate), and nicotinamide are pyridine derivatives which are also termed 'niacin'. Niacin is the precursor of the important coenzymes NAD (nicotinamide adenine dinucleotide) and NADP (nicotinamide adenine dinucleotide phosphate) and their reduced forms.

The large-scale production of these compounds has largely relied on cell-free chemical syntheses, though some of these chemicals have also been produced by large-scale culture of microorganisms, such as riboflavin, Vitamin B₆, pantothenate, and biotin. Only Vitamin B₁₂ is produced solely by fermentation, due to the complexity of its synthesis. *In vitro* methodologies require significant inputs of materials and time, often at great cost

### C. Purine, Pyrimidine, Nucleoside and Nucleotide Metabolism and Uses

Purine and pyrimidine metabolism genes and their corresponding proteins are important targets for the therapy of tumor diseases and viral infections. The language "purine" or "pyrimidine" includes the nitrogenous bases which are constituents of nucleic acids, co-enzymes, and nucleotides. The term "nucleotide" includes the basic structural units of nucleic acid molecules, which are comprised of a nitrogenous base, a pentose sugar (in the case of RNA, the sugar is ribose; in the case of DNA, the sugar is D-deoxyribose), and phosphoric acid. The language "nucleoside" includes molecules which serve as precursors to nucleotides, but which are lacking the phosphoric acid moiety that nucleotides possess. By inhibiting the biosynthesis of these molecules, or their mobilization to form nucleic acid molecules, it is possible to inhibit RNA and DNA synthesis; by inhibiting this activity in a fashion targeted to cancerous cells, the ability of tumor cells to divide and replicate may be inhibited. Additionally, there are nucleotides which do not form nucleic acid molecules, but rather serve as energy stores (*i.e.,* AMP) or as coenzymes (*i.e.,* FAD and NAD).

Several publications have described the use of these chemicals for these medical indications, by influencing purine and/or pyrimidine metabolism (*e.g*. Christopherson, R.I. and Lyons, S.D. (1990) "Potent inhibitors of de novo pyrimidine and purine biosynthesis as chemotherapeutic agents." Med. Res. Reviews 10: 505-548). Studies of enzymes involved in purine and pyrimidine metabolism have been focused on the development of new drugs which can be used, for example, as immunosuppressants or anti-proliferants (Smith, J.L., (1995) "Enzymes in nucleotide synthesis." Curr. Opin. Struct. Biol. 5: 752-757; (1995) Biochem Soc. Transact. 23: 877-902). However, purine and pyrimidine bases, nucleosides and nucleotides have other utilities: as intermediates in the biosynthesis of several fine chemicals (*e.g*., thiamine, S-adenosyl-methionine, folates, or riboflavin), as energy carriers for the cell (*e.g*., ATP or GTP), and for chemicals themselves, commonly used as flavor enhancers (*e.g*., IMP or GMP) or for several medicinal applications (see, for example, Kuninaka, A. (1996) Nucleotides and Related Compounds in Biotechnology vol. 6, Rehm et al., eds. VCH: Weinheim, p. 561-612). Also, enzymes involved in purine, pyrimidine, nucleoside, or nucleotide metabolism are increasingly serving as targets against which chemicals for crop protection, including fungicides, herbicides and insecticides, are developed.

The metabolism of these compounds in bacteria has been characterized (for reviews see, for example, Zalkin, H. and Dixon, J.E. (1992) "de novo purine nucleotide biosynthesis", in: Progress in Nucleic Acid Research and Molecular Biology, vol. 42, Academic Press:, p. 259-287; and Michal, G. (1999) "Nucleotides and Nucleotides", Chapter 8 in: Biochemical Pathways: An Atlas of Biochemistry and Molecular Biology, Wiley: New York). Purine metabolism has been the subject of intensive research, and is essential to the normal functioning of the cell. Impaired purine metabolism in higher animals can cause severe disease, such as gout. Purine nucleotides are synthesized from ribose-5-phosphate, in a series of steps through the intermediate compound inosine-5'-phosphate (IMP), resulting in the production of guanosine-5'-monophosphate (GMP) or adenosine-5'-monophosphate (AMP), from which the triphosphate forms utilized as nucleotides are readily formed. These compounds are also utilized as energy stores, so their degradation provides energy for many different biochemical processes in the cell. Pyrimidine biosynthesis proceeds by the formation of uridine-5'-monophosphate (UMP) from ribose-5-phosphate. UMP, in turn, is converted to cytidine-5'-triphosphate (CTP). The deoxy- forms of all of these nucleotides are produced in a one step reduction reaction from the diphosphate ribose form of the nucleotide to the diphosphate deoxyribose form of the nucleotide. Upon phosphorylation, these molecules are able to participate in DNA synthesis.

### D. Trehalose Metabolism and Uses

Trehalose consists of two glucose molecules, bound in α, α-1,1 linkage. It is commonly used in the food industry as a sweetener, an additive for dried or frozen foods, and in beverages. However, it also has applications in the pharmaceutical, cosmetics and biotechnology industries (see, for example, Nishimoto *et al.,* (1998) U.S. Patent No. 5,759,610; Singer, M.A. and Lindquist, S. (1998) Trends Biotech. 16: 460-467; Paiva, C.L.A. and Panek, A.D. (1996) Biotech Ann. Rev. 2: 293-314; and Shiosaka, M. (1997) J. Japan 172: 97-102). Trehalose is produced by enzymes from many microorganisms and is naturally released into the surrounding medium, from which it can be collected using methods known in the art.

### II. Elements and Methods of the Invention

Tables 1 and 2 provide lists of MP nucleic acids and proteins that a play a role in or function in one or more cellular metabolic pathways of C. glutamicum (the list of Table 2 consists of molecules known in prior art).

A MP molecule has an impact on the production of a desired chemical, either alone or in combination with molecules involved in the same or different metabolic pathways, or in one or more other metabolic pathways for amino acids, vitamins, cofactors, nutraceuticals, nucleotides, nucleosides or trehalose. A MP molecule may be modulated in activity, such that the C. glutamicum metabolic pathways in which the MP protein is involved are modulated in efficiency of output, which either directly or indirectly modulates the production or efficiency or production of a desired fine chemical by C. glutamicum.

MP molecules that catalyze an enzymatic reaction involving one or more amino acids, are useful for the production of a fine chemical. Among these, metZ, metC and RXA00657 are particularly useful. The present invention concerns metZ, which plays a role in the production of lysine or methionine.

The language, "MP protein" or "MP polypeptide" includes proteins which play a role in, *e.g*., catalyze an enzymatic reaction, in one or more amino acid, vitamin, cofactor, nutraceutical, nucleotide, nucleoside or trehalose metabolic pathways. Examples of MP proteins include those encoded by the MP genes set forth in Table 1 and by the odd-numbered SEQ ID NOs. The terms "MP gene" or "MP nucleic acid sequence" include nucleic acid sequences encoding an MP protein, which consist of a coding region and also corresponding untranslated 5' and 3' sequence regions. Examples of MP genes include those set forth in Table 1. The terms "production" or "productivity" are art-recognized and include the concentration of the fermentation product (for example, the desired fine chemical) formed within a given time and a given fermentation volume (*e.g*., kg product per hour per liter). The term "efficiency of production" includes the time required for a particular level of production to be achieved (for example, how long it takes for the cell to attain a particular rate of output of a fine chemical). The term "yield" or "product/carbon yield" is art-recognized and includes the efficiency of the conversion of the carbon source into the product (*i.e*., fine chemical). This is generally written as, for example, kg product per kg carbon source. By increasing the yield or production of the compound, the quantity of recovered molecules, or of useful recovered molecules of that compound in a given amount of culture over a given amount of time is increased. The terms "biosynthesis" or a "biosynthetic pathway" are art-recognized and include the synthesis of a compound, preferably an organic compound, by a cell from intermediate compounds in what may be a multistep and highly regulated process. The terms "degradation" or a "degradation pathway" are art-recognized and include the breakdown of a compound, preferably an organic compound, by a cell to degradation products (generally speaking, smaller or less complex molecules) in what may be a multistep and highly regulated process. The language "metabolism" is art-recognized and includes the totality of the biochemical reactions that take place in an organism. The metabolism of a particular compound, then, (e.g., the metabolism of an amino acid such as glycine) comprises the overall biosynthetic, modification, and degradation pathways in the cell related to this compound.

The MP molecule of the present invention may be combined with one or more further MP molecules of the description or one or more molecules of the same or different metabolic pathway to increase the yield of a desired amino acid, especially, lysine or methionine. Alternatively, or in addition, a byproduct which is not desired may be reduced by combination or disruption of MP molecules or other metabolic molecules (*e.g*., molecules involved in lysine or methionine metabolism). MP molecules combined with other molecules of the same or a different metabolic pathway may be altered in their nucleotide sequence and in the corresponding amino acid, sequence to alter their activity under physiological conditions, which leads to an increase in productivity and/or yield of a desired fine chemical. In a further embodiment, an MP molecule in its original or in its above-described altered form may be combined with other molecules of the same or a different metabolic pathway which are altered in their nucleotide sequence in such a way that their activity is altered under physiological conditions which leads to an increase in productivity and/or yield of methionine or lysine.

In another embodiment, the MP molecule of the invention, alone or in combination with one or more molecules of the same or different metabolic pathway, are capable of modulating the production of a desired molecule, such as a fine chemical, in a microorganism such as *C*. *glutamicum.* Using recombinant genetic techniques, one or more of the biosynthetic or degradative enzymes of the invention for amino acids, *e.g*., lysine or methionine, vitamins, cofactors, nutraceuticals, nucleotides, nucleosides, or trehalose may be manipulated such that its function is modulated. For example, a biosynthetic enzyme may be improved in efficiency, or its allosteric control region destroyed such that feedback inhibition of production of the compound is prevented. Similarly, a degradative enzyme may be deleted or modified by substitution, deletion, or addition such that its degradative activity is lessened for the desired compound without impairing the viability of the cell. In each case, the overall yield or rate of production of one of these desired fine chemicals may be increased.

It is also possible that such alterations in the protein and nucleotide molecule of the invention may improve the production of other fine chemicals besides the amino acids. Metabolism of any one compound is necessarily intertwined with other biosynthetic and degradative pathways within the cell, and necessary cofactors, intermediates, or substrates in one pathway are likely supplied or limited by another such pathway. Therefore, by modulating the activity of the protein of the invention, the production or efficiency of activity of another fine chemical biosynthetic or degradative pathway may be impacted. For example, amino acids serve as the structural units of all proteins, yet may be present intracellularly in levels which are limiting for protein synthesis; therefore, by increasing the efficiency of production or the yields of one or more amino acids within the cell, proteins, such as biosynthetic or degradative proteins, may be more readily synthesized. Likewise, an alteration in a metabolic pathway enzyme such that a particular side reaction becomes more or less favored may result in the over- or under-production of one or more compounds which are utilized as intermediates or substrates for the production of a desired fine chemical.

The isolated nucleic acid sequence of the invention is contained within the genome of a *Corynebacterium glutamicum* strain available through the American Type Culture Collection, given designation ATCC 13032. The nucleotide sequence of the isolated *C*. *glutamicum* MP DNAs and the predicted amino acid sequences of the *C*. *glutamicum* MP proteins are shown in the Sequence Listing as odd-numbered SEQ ID NOs and even-numbered SEQ ID NOs, respectively. Computational analyses were performed which classified and/or identified these nucleotide sequences as sequences which encode metabolic pathway proteins, *e.g*., proteins involved in the methionine or lysine metabolic pathways.

The present description also pertains to proteins which have an amino acid sequence which is substantially homologous to an amino acid sequence of the invention (*e.g*., the sequence of an even-numbered SEQ ID NO of the Sequence Listing). As used herein, a protein which has an amino acid sequence which is substantially homologous to a selected amino acid sequence is least about 50% homologous to the selected amino acid sequence, *e.g*., the entire selected amino acid sequence. A protein which has an amino acid sequence which is substantially homologous to a selected amino acid sequence can also be least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81 %, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to the selected amino acid sequence.

The MP protein of the invention, alone or in combination with one or more proteins of the same or different metabolic pathway, can catalyze an enzymatic reaction in one or more amino acid, vitamin, cofactor, nutraceutical, nucleotide, nucleoside, or trehalose metabolic pathways, or have one or more of the activities set forth in Table 1 (especially metabolism of methionine or lysine biosynthesis).

Various aspects of the invention are described in further detail in the following subsections:

### A. Isolated Nucleic Acid Molecules

One aspect of the description pertains to isolated nucleic acid molecules that encode MP polypeptides or biologically active portions thereof, as well as nucleic acid fragments sufficient for use as hybridization probes or primers for the identification or amplification of MP-encoding nucleic acid (*e.g*., MP DNA). As used herein, the term "nucleic acid molecule" is intended to include DNA molecules (*e.g.,* cDNA or genomic DNA) and RNA molecules (*e.g*., mRNA) and analogs of the DNA or RNA generated using nucleotide analogs. This term also encompasses untranslated sequence located at both the 3' and 5' ends of the coding region of the gene: at least about 100 nucleotides of sequence upstream from the 5' end of the coding region and at least about 20 nucleotides of sequence downstream from the 3' end of the coding region of the gene. The nucleic acid molecule can be single-stranded or double-stranded, but preferably is double-stranded DNA. An "isolate" nucleic acid molecule is one which is separated from other nucleic acid molecules which are present in the natural source of the nucleic acid. Preferably, an "isolated" nucleic acid is free of sequences which naturally flank the nucleic acid (*i.e*., sequences located at the 5' and 3' ends of the nucleic acid) in the genomic DNA of the organism from which the nucleic acid is derived. For example, in various embodiments, the isolated MP nucleic acid molecule can contain less than about 5 kb, 4kb, 3kb, 2kg, 1 kb, 0.5 kb or 0.1 kb of nucleotide sequences which naturally flank the nucleic acid molecule in genomic DNA of the cell from which the nucleic acid is derived (e.g, a C. *glutamicum* cell). Moreover, an "isolated" nucleic acid molecule, such as a DNA molecule, can be substantially free of other cellular material, or culture medium when produced by recombinant techniques, or chemical precursors or other chemicals when chemically synthesized.

A MP nucleic acid molecule disclosed herein, *e.g*., a nucleic acid molecule having a nucleotide sequence of an odd-numbered SEQ ID NO of the Sequence Listing, or a portion thereof, can be isolated using standard molecular biology techniques and the sequence information provided herein. For example, a *C*. *glutamicum* MP DNA can be isolated from a C. *glutamicum* library using all or portion of one of the odd-numbered SEQ ID NO sequences of the Sequence Listing as a hybridization probe and standard hybridization techniques (*e.g*., as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989). Moreover, a nucleic acid molecule encompassing all or a portion the nucleic acid sequence of the invention (*e.g*., an odd-numbered SEQ ID NO: 1) can be isolated by the polymerase chain reaction using oligonucleotide primers designed based upon this sequence (*e.g*., a nucleic acid molecule encompassing all or a portion of the nucleic acid sequence of the invention (*e.g*., an odd-numbered SEQ ID NO 1 ) can be isolated by the polymerase chain reaction using oligonucleotide primers designed based upon this same sequence). For example, mRNA can be isolated from normal endothelial cells (*e.g*., by the guanidinium-thiocyanate extraction procedure of Chirgwin et al. (1979) Biochemistry 18: 5294-5299) and DNA can be prepared using reverse transcriptase (*e.g*., Moloney MLV reverse transcriptase, available from Gibco/BRL, Bethesda, MD; or AMV reverse transcriptase, available from Seikagaku America, Inc., St Petersburg, FL). Synthetic oligonucleotide primers for polymerase chain reaction amplification can be designed based upon one of the nucleotide sequences shown in the Sequence Listing. The nucleic acid of the invention can be amplified using cDNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to an MP nucleotide sequence can be prepared by standard synthetic techniques, *e.g*., using an automated DNA synthesizer.

An isolated nucleic acid molecule of the description comprises preferably one of the nucleotide sequences shown in the Sequence Listing. The DNAs comprise sequences encoding MP proteins (*i.e*., the "coding region", indicated in each odd-numbered SEQ ID NO: sequence in the Sequence Listing), as well as 5' untranslated' sequences and 3' untranslated sequences, also indicated in each odd-numbered SEQ ID NO: in the Sequence Listing. Alternatively, the nucleic acid molecule can comprise only the coding region of any of the nucleic acid sequences of the Sequence Listing.

For the purposes of this application, it will be understood that some of the MP nucleic acid and amino acid sequences set forth in the Sequence Listing have an identifying RXA, RXN, RXS, or RXC number having the designation "RXA", "RXN", "RXS", or "RXC" followed by 5 digits (*i.e*., RXA, RXN, RXS, or RXC). Each of the nucleic acid sequences comprises up to three parts: a 5' upstream region, a coding region, and a downstream region. Each of these three regions is identified by the same RXA, RXN, RXS, or RXC designation to eliminate confusion. The recitation "one of the odd-numbered sequences of the Sequence Listing", then, refers to any of the nucleic acid sequences in the Sequence Listing, which may also be distinguished by their differing RXA, RXN, RXS, or RXC designations. The coding region of each of these sequences is translated into a corresponding amino acid sequence, which is also set forth in the Sequence Listing, as an even-numbered SEQ ID NO: immediately following the corresponding nucleic acid sequence . For example, the coding region for RXA00115 is set forth in SEQ ID NO:69, while the amino acid sequence which it encodes is set forth as SEQ ID NO:70. The sequences of the nucleic acid molecules of the invention are identified by the same RXA, RXN, RXS, or RXC designations as the amino acid molecules which they encode, such that they can be readily correlated. For example, the amino acid sequences designated RXA00115, RXN00403, and RXS03158 are translations of the coding regions of the nucleotide sequences of nucleic acid molecules RXA00115, RXN00403, and RXS03158, respectively. The correspondence between the RXA, RXN, RXS, and RXC nucleotide and amino acid sequences of the invention and their assigned SEQ ID NOs is set forth in Table 1.

Several of the genes of the description are "F-designated genes". An F-designated gene includes those genes set forth in Table 1 which have an 'F' in front of the RXA, RXN, RXS, or RXC designation. For example, SEQ ID NO:77, designated, as indicated on Table 1, as "F RXA00254", is an F-designated gene.

Also listed on Table 1 are the *metZ* (or *metY*) and *metC* genes (designated as SEQ ID NO:1 and SEQ ID NO:3, respectively. The corresponding amino acid sequence encoded by the *metZ* and *metC* genes are designated as SEQ ID NO:2 and SEQ ID NO: 4, respectively.

Disclosed is also an isolated nucleic acid molecule of the invention comprises a nucleic acid molecule which is a complement of one of the nucleotide sequences of the invention (*e.g*., a sequence of an odd-numbered SEQ ID NO: of the Sequence Listing), or a portion thereof. A nucleic acid molecule which is complementary to one of the nucleotide sequences of the invention is one which is sufficiently complementary to one of the nucleotide sequences shown in the Sequence Listing (*e.g*., the sequence of an odd-numbered SEQ ID NO:) such that it can hybridize to one of the nucleotide sequences of the invention thereby forming a stable duplex.

In still another preferred embodiment of the description, an isolated nucleic acid molecule of the invention comprises a nucleotide sequence which is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91 %, 92%, 93%, 94%, and even more preferably at least about 95%, 96%, 97%, 98%, 99%, 99.7% or more homologous to an odd-numbered SEQ ID NO: of the Sequence Listing, or a portion thereof. Ranges and identity values intermediate to the above-recited ranges, (*e.g*., 70-90% identical or 80-95% identical) are also disclosed. . For example, ranges of identity values using a combination of any of the above values recited as upper and/or lower limits are intended to be included. In an additional preferred embodiment, an isolated nucleic acid molecule of the invention comprises a nucleotide sequence which hybridizes, *e.g*., hybridizes under stringent conditions, to one of the nucleotide sequence of the invention, or a portion thereof.

Moreover, the nucleic acid molecule of the description can comprise only a portion of the coding region of the sequence of one of the odd-numbered SEQ ID NOs of the Sequence Listing, for example a fragment which can be used as a probe or primer or a fragment encoding a biologically active portion of an MP protein. The nucleotide sequences determined from the cloning of the MP genes from *C*. *glutamicum* allows for the generation of probes and primers designed for use in identifying and/or cloning MP homologues in other cell types and organisms, as well as MP homologues from other *Corynebacteria* or related species. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense strand of one of the nucleotide sequences of the invention (*e.g*., a sequence of one of the odd-numbered SEQ ID NOs of the Sequence Listing), an anti-sense sequence of one of these sequences, or naturally occurring mutants thereof. Primers based on a nucleotide sequence of the invention can be used in PCR reactions to clone MP homologues. Probes based on the MP nucleotide sequences can be used to detect transcripts or genomic sequences encoding the same or homologous proteins. Preferably the probe further comprises a label group attached thereto, *e.g.* the label group can be a radioisotope, a fluorescent compound, an enzyme, or an enzyme cofactor. Such probes can be used as a part of a diagnostic test kit for identifying cells which misexpress an MP protein, such as by measuring a level of an MP-encoding nucleic acid in a sample of cells from a subject *e.g*., detecting MP mRNA levels or determining whether a genomic MP gene has been mutated or deleted.

In one embodiment, the nucleic acid molecule of the description encodes a protein or portion thereof which includes an amino acid sequence which is sufficiently homologous to an amino acid sequence of the invention (*e.g*., a sequence of an even-numbered SEQ ID NO of the Sequence Listing) such that the protein or portion thereof maintains the ability to catalyze an enzymatic reaction in an amino acid, vitamin, cofactor, nutraceutical, nucleotide, nucleoside, or trehalose metabolic pathway. As used herein, the language "sufficiently homologous" refers to proteins or portions thereof which have amino acid sequences which include a minimum number of identical or equivalent (*e.g*., an amino acid residue which has a similar side chain as an amino acid residue in a sequence of one of the even-numbered SEQ ID NOs of the Sequence Listing) amino acid residues to an amino acid sequence of the invention such that the protein or portion thereof is able to catalyze an enzymatic reaction in a *C*. *glutamicum* amino acid, vitamin, cofactor, nutraceutical, nucleotide, nucleoside or trehalose metabolic pathway. Protein members of such metabolic pathways, as described herein, function to catalyze the biosynthesis or degradation of one or more of: amino acids, vitamins, cofactors, nutraceuticals, nucleotides, nucleosides, or trehalose. Examples of such activities are also described herein. Thus, "the function of an MP protein" contributes to the overall functioning of one or more such metabolic pathway and contributes, either directly or indirectly, to the yield, production, and/or efficiency of production of one or more fine chemicals. Examples of MP protein activities are set forth in Table 1.

In another embodiment of the description, the protein is at least about 50%, 51%, 52%, 53%, 54%, 55%, 56%, 57%, 58%, 59%, or 60%, preferably at least about 61 %, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to an entire amino acid sequence of an even-numbered SEQ ID NO: of the Sequence Listing.

Portions of proteins encoded by the MP nucleic acid molecules of the description are preferably biologically active portions of one of the MP proteins. As used herein, the term "biologically active portion of an MP protein" is intended to include a portion, *e.g*., a domain/motif, of an MP protein that catalyzes an enzymatic reaction in one or more *C. glutamicum* amino acid, vitamin, cofactor, nutraceutical, nucleotide, nucleoside, or trehalose metabolic pathways, or has an activity as set forth in Table 1. To determine whether an MP protein or a biologically active portion thereof can catalyze an enzymatic reaction in an amino acid, vitamin, cofactor, nutraceutical, nucleotide, nucleoside, or trehalose metabolic pathway, an assay of enzymatic activity may be performed Such assay methods are well known to those of ordinary skill in the art, as detailed in Example 8 of the Exemplification.

Additional nucleic acid fragments encoding biologically active portions of an MP protein can be prepared by isolating a portion of one of the amino acid sequences of the description (*e.g*., a sequence of an even-numbered SEQ ID NO: of the Sequence Listing), expressing the encoded portion of the MP protein or peptide (*e.g*., by recombinant expression in vitro) and assessing the activity of the encoded portion of the MP protein or peptide.

The description further encompasses nucleic acid molecules that differ from one of the nucleotide sequences disclosed herein (*e.g*., a sequence of an odd-numbered SEQ ID NO: of the Sequence Listing) (and portions thereof) due to degeneracy of the genetic code and thus encode the same MP protein as that encoded by the nucleotide sequences of the invention. In another embodiment, the isolated nucleic acid molecule has a nucleotide sequence encoding a protein having an amino acid sequence shown in the Sequence Listing (*e.g*., an even-numbered SEQ ID NO:). In a still further embodiment, the nucleic acid molecule encodes a full length *C*. *glutamicum* protein which is substantially homologous to an amino acid sequence encoded by an open reading frame shown in an odd-numbered SEQ ID NO: of the Sequence Listing.

In one embodiment, the description includes nucleotide and amino acid sequences having a percent identity to a nucleotide or amino acid sequence of the invention which is greater than that of a sequence of the prior art (*e.g*., a Genbank sequence (or the protein encoded by such a sequence) set forth in Table 2). For example, the description includes a nucleotide sequence which is greater than and/or at least 45% identical to the nucleotide sequence designated RXA00657 SEQ ID NO:5 One of ordinary skill in the art would be able to calculate the lower threshold of percent identity for any given sequence of the invention by examining the GAP-calculated percent identity scores set forth in Table 4 for each of the three top hits for the given sequence, and by subtracting the highest GAP-calculated percent identity from 100 percent. One of ordinary skill in the art will also appreciate that nucleic acid and amino acid sequences having percent identities greater than the lower threshold so calculated (*e.g.,* at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more identical) are also encompassed.

In addition to the *C*. *glutamicum* MP nucleotide sequences set forth in the Sequence Listing as odd-numbered SEQ ID NOs, it will be appreciated by one of ordinary skill in the art that DNA sequence polymorphisms that lead to changes in the amino acid sequences of MP proteins may exist within a population (*e.g*., the *C*. *glutamicum* population). Such genetic polymorphism in the MP gene may exist among individuals within a population due to natural variation. As used herein, the terms "gene" and "recombinant gene" refer to nucleic acid molecules comprising an open reading frame encoding an MP protein, preferably a *C*. *glutamicum* MP protein. Such natural variations can typically result in 1-5% variance in the nucleotide sequence of the MP gene. Any and all such nucleotide variations and resulting amino acid polymorphisms in MP that are the result of natural variation and that do not alter the functional activity of MP proteins can be used.

Nucleic acid molecules corresponding to natural variants and non-*C*. *glutamicum* homologues of the *C*. *glutamicum* MP DNA of the description can be isolated based on their homology to the *C*. *glutamicum* MP nucleic acid disclosed herein using the *C*. *glutamicum* DNA, or a portion thereof, as a hybridization probe according to standard hybridization techniques under stringent hybridization conditions. Accordingly, in another embodiment, an isolated nucleic acid molecule of the description is at least 15 nucleotides in length and hybridizes under stringent conditions to the nucleic acid molecule comprising a nucleotide sequence of an odd-numbered SEQ ID NO: of the Sequence Listing. In other embodiments, the nucleic acid is at least 30, 50, 100, 250 or more nucleotides in length. As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 65%, more preferably at least about 70%, and even more preferably at least about 75% or more homologous to each other typically remain hybridized to each other. Such stringent conditions are known to one of ordinary skill in the art and can be found in *Current Protocols in Molecular Biology,* John Wiley & Sons, N.Y. (1989), 6.3.1-6.3.6.. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule discloses herein that hybridizes under stringent conditions to a nucleotide sequence of the invention corresponds to a naturally-occurring nucleic acid molecule. As used herein, a "naturally-occurring" nucleic acid molecule refers to an RNA or DNA molecule having a nucleotide sequence that occurs in nature (*e.g*., encodes a natural protein). In one embodiment, the nucleic acid encodes a natural *C. glutamicum* MP protein.

In addition to naturally-occurring variants of the MP sequence that may exist in the population, one of ordinary skill in the art will further appreciate that changes can be introduced by mutation into a nucleotide sequence of the invention, thereby leading to changes in the amino acid sequence of the encoded MP protein, without altering the functional ability of the MP protein. For example, nucleotide substitutions leading to amino acid substitutions at "non-essential" amino acid residues can be made in a nucleotide sequence of the invention. A "non-essential" amino acid residue is a residue that can be altered from the wild-type sequence of one of the MP proteins (*e.g*., an even-numbered SEQ ID NO: of the Sequence Listing) without altering the activity of said MP protein, whereas an "essential" amino acid residue is required for MP protein activity. Other amino acid residues, however, (*e.g*., those that are not conserved or only semi-conserved in the domain having MP activity) may not be essential for activity and thus are likely to be amenable to alteration without altering MP activity.

Accordingly, another aspect of the description pertains to nucleic acid molecules encoding MP proteins that contain changes in amino acid residues that are not essential for MP activity. Such MP proteins differ in amino acid sequence from a sequence of an even-numbered SEQ ID NO: of the Sequence Listing yet retain at least one of the MP activities described herein. In one embodiment, the isolated nucleic acid molecule comprises a nucleotide sequence encoding a protein, wherein the protein comprises an amino acid sequence at least about 50% homologous to an amino acid sequence of the invention and is capable of catalyzing an enzymatic reaction in an amino acid, vitamin, cofactor, nutraceutical, nucleotide, nucleoside, or trehalose metabolic pathway, or has one or more activities set forth in Table 1. Preferably, the protein encoded by the nucleic acid molecule is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% homologous to one of the amino acid sequences disclosed herein

To determine the percent homology of two amino acid sequences (*e.g*., one of the amino acid sequences of the invention and a mutant form thereof) or of two nucleic acids, the sequences are aligned for optimal comparison purposes (*e.g*., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence (*e.g*., one of the amino acid sequences of the invention) is occupied by the same amino acid residue or nucleotide as the corresponding position in the other sequence (*e.g*., a mutant form of the amino acid sequence), then the molecules are homologous at that position (*i.e.,* as used herein amino acid or nucleic acid "homology" is equivalent to amino acid or nucleic acid "identity"). The percent homology between the two sequences is a function of the number of identical positions shared by the sequences (*i.e*.,% homology = # of identical positions/total # of positions x 100).

An isolated nucleic acid molecule encoding an MP protein homologous to a protein sequence of the description (*e.g.,* a sequence of an even-numbered SEQ ID NO: of the Sequence Listing) can be created by introducing one or more nucleotide substitutions, additions or deletions into a nucleotide sequence of the invention such that one or more amino acid substitutions, additions or deletions are introduced into the encoded protein. Mutations can be introduced into one of the nucleotide sequences of the invention by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (*e.g*., tyrosine, phenylalanine, tryptophan, histidine). Thus, a predicted nonessential amino acid residue in an MP protein is preferably replaced with another amino acid residue from the same side chain family. Alternatively, mutations can be introduced randomly along all or part of an MP coding sequence, such as by saturation mutagenesis, and the resultant mutants can be screened for an MP activity described herein to identify mutants that retain MP activity. Following mutagenesis of the nucleotide sequence of one of the odd-numbered SEQ ID NOs of the Sequence Listing, the encoded protein can be expresses recombinantly and the activity of the protein can be determined using, for example, assays described herein (see Example 8 of the Exemplification).

In addition to the nucleic acid molecules encoding MP proteins described above, another aspect of the description pertains to isolated nucleic acid molecules which are antisense thereto. An "antisense" nucleic acid comprises a nucleotide sequence which is complementary to a "sense" nucleic acid encoding a protein, *e.g*., complementary to the coding strand of a double-stranded DNA molecule or complementary to an mRNA sequence. Accordingly, an antisense nucleic acid can hydrogen bond to a sense nucleic acid. The antisense nucleic acid can be complementary to an entire MP coding strand, or to only a portion thereof In one embodiment, an antisense nucleic acid molecule is antisense to a "coding region" of the coding strand of a nucleotide sequence encoding an MP protein. The term "coding region" refers to the region of the nucleotide sequence comprising codons which are translated into amino acid residues (*e.g*., the entire coding region of SEQ ID NO.:1 (*metZ)* comprises nucleotides 363 to 1673). In another embodiment, the antisense nucleic acid molecule is antisense to a "noncoding region" of the coding strand of a nucleotide sequence encoding MP. The term "noncoding region" refers to 5' and 3' sequences which flank the coding region that are not translated into amino acids (*i.e.,* also referred to as 5' and 3' untranslated regions).

Given the coding strand sequences encoding MP disclosed herein (*e.g*., the sequences set forth as odd-numbered SEQ ID NOs in the Sequence Listing), antisense nucleic acids can be designed according to the rules of Watson and Crick base pairing. The antisense nucleic acid molecule can be complementary to the entire coding region of MP mRNA, but more preferably is an oligonucleotide which is antisense to only a portion of the coding or noncoding region of MP mRNA. For example, the antisense oligonucleotide can be complementary to the region surrounding the translation start site of MP mRNA. An antisense oligonucleotide can be, for example, about 5,10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g*., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e*., RNA transcribed from the inserted nucleic acid will be of an antisense orientation to a target nucleic acid of interest, described further in the following subsection).

The antisense nucleic acid molecules of the description are typically administered to a cell or generated *in situ* such that they hybridize with or bind to cellular mRNA and/or genomic DNA encoding an MP protein to thereby inhibit expression of the protein, *e.g*., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, *e.g*., by linking the antisense nucleic acid molecule to a peptide or an antibody which binds to a cell surface receptor or antigen. The antisense nucleic acid molecules can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic promoter are preferred.

In yet another embodiment, the antisense nucleic acid molecule of the description is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

In still another embodiment, an antisense nucleic acid of the description is a ribozyme. Ribozymes are catalytic RNA molecules with ribonuclease activity which are capable of cleaving a single-stranded nucleic acid, such as an mRNA, to which they have a complementary region. Thus, ribozymes (*e.g*., hammerhead ribozymes (described in Haselhoff and Gerlach (1988) Nature 334:585-591)) can be used to catalytically cleave MP mRNA transcripts to thereby inhibit translation of MP mRNA. A ribozyme having specificity for an MP-encoding nucleic acid can be designed based upon the nucleotide sequence of an MP DNA disclosed herein (*i.e.,* SEQ ID NO:1 (*metZ*)*.* For example, a derivative of a *Tetrahymena* L-19 IVS RNA can be constructed in which the nucleotide sequence of the active site is complementary to the nucleotide sequence to be cleaved in an MP-encoding mRNA.. See, *e.g.,* Cech *et al.* U.S. Patent No. 4,987,071 and Cech *et al.* U.S. Patent No. 5,116,742. Alternatively, MP mRNA can be used to select a catalytic RNA having a specific ribonuclease activity from a pool of DNA molecules. See, *e.g.,* Bartel, D. and Szostak, J.W. (1993) Science 261:1411-1418.

Alternatively, MP gene expression can be inhibited by targeting nucleotide sequences complementary to the regulatory region of an MP nucleotide sequence (*e.g*., an MP promoter and/or enhancers) to form triple helical structures that prevent transcription of an MP gene in target cells. See generally, Helene, C. (1991) Anticancer Drug Des. 6(6):569-84; Helene, C. èt al. (1992) Ann. N.Y. Acad Sci. 660:27-36; and Maher, L.J. (1992) Bioassays 14(12):807-15.

Another aspect of the description pertain to combinations of genes involved in methionine and/or lysine metabolism and the use of to combinations of genes involved in methionine and/or lysine metabolism in the methods of the invention. Preferred combinations are the combination of *metZ* with *metC, metB* (encoding Cystathionine-Synthase), *metA* (encoding homoserine-O-acetyltransferase), *metE* (encoding Methionine Synthase), *metH* (encoding Methionine Synthase), *hom* (encoding homoserine dehydrogenase), asd (encoding aspartatesemialdehyd dehydrogenase), *lysC* /*ask* (encoding aspartokinase) and rxa00657 (herein designated as SEQ ID NO.:5), *dapA,* (gene encoding DIHYDRODIPICOLINATE SYNTHASE), *dapB* (gene encoding DIHYDRODIPICOLINATE REDUCTASE), *dapC* (gene encoding 2,3,4,5-tetrahydropyridine-2-carboxylate N-succinyltransferase), *dapD*/*argD* (gene encoding, acetylornithine transaminase), *dapE* (gene encoding succinyldiaminopimelate desuccinylase), *dapF* (gene encoding diaminopimelate epimerase), *lysA* (gene encoding diaminopimelate decarboxylase), *ddh* (gene encoding diaminopimelate dehydrogenase), *lysE* (gene encoding for the lysine exporter ), *lysG* (gene encoding for the exporter regulator), *hsk* (gene encoding homoserine kinase) as well as genes involved in anaplerotic reaction such as *ppc* (gene encoding phosphoenolpyruvate carboxylase), *ppcK* (gene encoding phosphoenolpyruvate carboxykinase), *pycA* (gene encoding pyruvate carboxylase), *accD, accA, accB, accC* (genes encoding for subunits of acetyl-CoA-carboxylase), as well as genes of the pentose-phosphate pathway, gpdh genes encoding glucose-6 phophate-dehydrogenase, *opcA, pgdh* (gene encoding 6-phosphogluconate-dehydrogenase), *ta* (gene encoding transaldolase), *tk* (gene encoding gene encoding transketolase), *pgl* (gene encoding 6-PHOSPHOGLUCONO-LACTONASE), *rlpe* (gene encoding RIBULOSE-PHOSPHATE 3-EPIMERASE) *rpe* (gene encoding RIBOSE 5-PHOSPHATE EPIMERASE) or combinations the above-mentioned genes of the pentose-phosphate-pathways, or other MP genes disclosed herein.

The genes may be altered in their nucleotide sequence and in the corresponding amino acid sequence resulting in derivatives in such a way that their activity is altered under physiological conditions which leads to an increase in productivity and/or yield of a desired amino acid such as methionine or lysine. One class of such alterations or derivatives is well known for the nucleotide sequence of the *ask* gene encoding aspartokinase. These alterations lead to removal of feed back inhibition by the amino acids lysine and threonine and subsequently to lysine overproduction. In a preferred embodiment the *metZ* gene is used in a *Corynebacterium* strain in combination with *ask hom, metA* and *metH* . In another preferred embodiment *metZ* is used in a *Corynebacterium* strain in combination with *ask, hom, metA* and *metE* . In a more preferred embodiment, the gene combinations *metZ* are combined with *ask hom, metA* and *metH* or *metZ* is combined with *ask hom, metA* and *metE* in a *Corynebacterium* strain and sulfur sources such as sulfates, thiosulfates, sulfites and also more reduced sulfur sources such as H₂S and sulfides and derivatives are used in the growth medium. Also, sulfur sources such as methyl mercaptan, methanesulfonic acid, thioglycolates, thiocyanates, thiourea, sulfur containing amino acids such as cysteine and other sulfur containing compounds can be used. Another aspect of the description pertains to the use of the above mentioned gene combinations in a *Corynebacterium* strain which is, before or after introduction of the genes, mutagenized by radiation or by mutagenic chemicals well-known to one of ordinary skill in the art and selected for resistance against high concentrations of the fine chemical of interest, *e.g,* lysine or methionine or analogues of the desired fine chemical such as the methionine analogues ethionine, methyl methionine, or others. The gene combinations mentioned above can be expressed in a *Corynebacterium* strain having particular gene disruptions. Preferred are gene disruptions that encode proteins that favor carbon flux to undesired metabolites. Where methionine is the desired fine chemical the formation of lysine may be unfavorable. In such a case the combination of the above mentioned genes should proceed in a *Corynebacterium* strain bearing a gene disruption of the *lysA* gene (encoding diaminopimelate decarboxylase) or the *ddh* gene (encoding the meso-diaminopimelate dehydrogenase catalysing the conversion of tetrahydropicolinate to meso-diaominopimelate). In a preferred embodiment a favorable combination of the above-mentioned genes are all altered in such a way that their gene products are not feed back inhibited by end products or metabolites of the biosynthetic pathway leading to the desired fine chemical. In the case that the desired fine chemical is methionine, the gene combinations may be expressed in a strain previously treated with mutagenic agents or radiation and selected for the above-mentioned resistance. Additionally, the strain should be grown in a growth medium containing one or more of the above mentioned sulfur sources.

### B. Becombinant Expression Vectors and Host Cells

Also disclosed are vectors, preferably expression vectors, containing a nucleic acid encoding an MP protein (or a portion thereof) or combinations of genes wherein at least one gene encodes for an MP protein. As used herein, the term "vector" refers to a nucleic acid molecule capable of transporting another nucleic acid to which it has been linked. One type of vector is a "plasmid", which refers to a circular double stranded DNA loop into which additional DNA. segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

The recombinant expression vectors comprise a nucleic acid of the description in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (*e.g.,* in an *in vitro* transcription/translation system or in a host cell when the vector is introduced into the host cell). The term "regulatory sequence" is intended to include promoters, repressor binding sites, activator binding sites, enhancers and other expression control elements (*e.g*., terminators, polyadenylation signals, or other elements of mRNA secondary structure). Such regulatory sequences are described, for example, in Goeddel; Gene Expression Technology: Methods in Enzymology 185, Academic Press, San Diego, CA (1990). regulator sequences include those which direct constitutive expression of a nucleotide sequence in many types of host cell and those which direct expression of the nucleotide sequence only in certain host cells. Preferred regulatory sequences are, for example, promoters such as cos-, tac-, trp-, tet-, trp-tet-, lpp-, lac-, lpp-lac-, lacI^{q}-, T7-, T5-, T3-, gal-, trc-, ara-, SP6-, arny, SPO2, λ-P_{R}- or λ P_{L}, which are used preferably in bacteria. Additional regulatory sequences are, for example, promoters from yeasts and fungi, such as ADC1, MFα, AC, P-60, CYC1, GAPDH, TEF, rp28, ADH, promoters from plants such as CaMV/35S, SSU, OCS, lib4, usp, STLS1, B33, nos or ubiquitin- or phaseolin-promoters. It is also possible to use artificial promoters. It will be appreciated by one of ordinary skill in the art that the design of the expression vector can depend on such factors as the choice of the host cell to be transformed, the level of expression of protein desired, etc. The expression vectors can be introduced into host cells to thereby produce proteins or peptides, including fusion proteins or peptides, encoded by nucleic acids as described herein (*e.g*., MP proteins, mutant forms of MP proteins, fusion proteins, etc.).

The recombinant expression vectors can be designed for expression of MP proteins in prokaryotic or eukaryotic cells, for example, in bacterial cells such as C. glutamicum, "Foreign gene expression in yeast: a review", Yeast 8: 423-488; van den Hondel, C.A.M.J.J. et al. (1991) "Heterologous gene expression in filamentous fungi" in: More Gene Manipulations in Fungi, J.W. Bennet & L.L. Lasure, eds., p. 396-428: Academic Press: San Diego; and van den Hondel, C.A.M.J.J. & Punt, P.J. (1991) "Gene transfer systems and vector development for filamentous fungi, in: Applied Molecular Genetics of Fungi, Peberdy, J.F. et al., eds., p. 1-28, Cambridge University Press: Cambridge), algae and multicellular plant cells (see Schmidt, R. and Willmitzer, L. (1988) High efficiency Agrobacterium tumefaciens- mediated transformation of Arabidopsis thaliana leaf and cotyledon explants" Plant Cell Rep.: 583-586), or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out with vectors containing constitutive or inducible promoters directing the expression of either fusion or non-fusion proteins. Fusion vectors add a number of amino acids to a protein encoded therein, usually to the amino terminus of the recombinant protein but also to the C-terminus or fused within suitable regions in the proteins. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors; a proteolytic cleavage site is introduced at the junction of the fusion moiety and the recombinant protein to enable separation of the recombinant protein from the fusion moiety subsequent to purification of the fusion protein. Such enzymes; and their cognate recognition sequences, include Factor Xa, thrombin and enterokinase.

Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. The coding sequence of the MP protein may be cloned into a pGEX expression vector to create a vector encoding a fusion protein comprising, from the N-terminus to the C-terminus, GST-thrombin cleavage site-X protein. The fusion protein can be purified by affinity chromatography using glutathione-agarose resin. Recombinant MP protein unfused to GST can be recovered by cleavage of the fusion protein with thrombin.

Examples of suitable inducible non-fusion *E. coli* expression vectors include pTrc (Amann et al., (1988) Gene 69:301-315) pLG338, pACYC184, pBR322, pUC18, pUC19, pKC30, pRep4, pHS1, pHS2, pPLc236, pMBL24, pLG200, pUR290, pIN-III113-B 1, λgt11, pBdCl, and pET 11d (Studier et al., Gene Expression Technology: Methods in Enzymology 185, Academic Press, San Diego, California (1990) 60-89; and Pouwels et al., eds. (1985) Cloning Vectors. Elsevier: New York IBSN 0 444 904018). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21 (DE3) or HMS174(DE3) from a resident λ prophage harboring a T7 gn1 gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming Streptomyces, while plasmids pUB110, pC 194, or pBD214 are suited for transformation of Bacillus species. Several plasmids of use in the transfer of genetic information into Corynebacterium include pHM1519, pBL1, pSA77, or pAJ667 (Pouwels *et al.,* eds. (1985) Cloning Vectors. Elsevier: New York IBSN 0 444 904018).

One strategy to maximize recombinant protein expression is to express the protein in a host bacteria with an impaired capacity to proteolytically cleave the recombinant protein (Gottesman, S., Gene Expression Technology: Methods in Enzymology 185, Academic Press, San Diego, California (1990) 119-128). Another strategy is to alter the nucleic acid sequence of the nucleic acid to be inserted into an expression vector so that the individual codons for each amino acid are those preferentially utilized in the bacterium chosen for expression, such as *C. glutamicum* (Wada et al. (1992) Nucleic Acids Res 20:2111-2118). Such alteration of nucleic acid sequence of the invention can be carried out by standard DNA synthesis techniques.

In another embodiment of the description the MP protein expression vector is a yeast expression vector. Examples of vectors for expression in yeast *S. cerevisiae* include pYepSec1 (Baldari, et al., (1987) Embd J. 6:229-234), , 2 µ, pAG-1, Yep6, Yep13, pEMBLYe23, pMFa (Kurjan and Herskowitz, (1982) Cell 30:933-943)*,* pJRY88 (Schultz et al., (1987) Gene 54:113-123), and pYES2 (Invitrogen Corporation, San Diego, CA). Vectors and methods for the construction of vectors appropriate for use in other fungi, such as the filamentous fungi, include those detailed in: van den Hondel, C.A.M.J.J. & Punt, P.J. (1991) "Genes transfer systems and vector development for filamentous fungi, in: Applied Molecular Genetics of Fungi, J.F. Peberdy, et al., eds., p. 1-28, Cambridge University Press: Cambridge, and Pouwels et al., eds. (1985) Cloning Vectors. Elsevier: new York (IBSN 0 444 904018).

Alternatively, the MP proteins can be expressed in insect cells using baculovirus expression vectors. Baculovirus vectors available for expression of proteins in cultured insect cells (*e.g*., Sf 9 cells) include the pAc series (Smith et al. (1983) Mol. Cell Biol. 3:2156-2165) and the pVL series (Lucklow and Summers (1989) Virology 170:31-39).

In another embodiment of the descriptions the MP proteins may be expressed in unicellular plant cells (such as algae) or in plant cells from higher plants (*e.g*., the spermatophytes, such as crop plants). Examples of plant expression vectors include those detailed in: Becker, D., Kemper, E., Schell, J. and Masterson, R. (1992) "New plant binary vectors with selectable markers located proximal to the left border", Plant Mol. Biol. 20: 1195-1197; and Bevan, M.W. (1984) "Binary Agrobacterium vectors for plant transformation", Nucl. Acid. Res. 12: 8711-8721, and include pLGV23, pGHlac+, pBIN19, pAK2004, and pDH51 (Pouwels et al., eds. (1985) Cloning Vectors. Elsevier: New York IBSN 0 444 904018).

In yet another embodiment of the description a nucleic acid is expressed in mammalian cells using a mammalian expression vector. Examples of mammalian expression vectors include pCDM8 (Seed, B. (1987) Nature 329:840) and pMT2PC (Kaufman et al. (1987) EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989.

The recombinant mammalian expression vector may be capable of directing expression of the nucleic acid preferentially in a particular cell type (*e.g*., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) PNAS 86:5473-5477), pancreas-specific promoters (Edlund et al. (1985) Science 230:912-916), and mammary gland-specific promoters (*e.g*., milk whey promoter; U.S. Patent No. 4,873,316 and European Application Publication No. 264,166). Developmentally-regulated promoters are also encompassed, for example the murine hox promoters (Kessel and Gruss (1990) Science 249:374-379) and the α-fetoprotein promoter (Campes and Tilghman (1989) Genes Dev. 3:537-546).

The description further provides a recombinant expression vector comprising a DNA molecule disclosed herein cloned into the expression vector in an antisense orientation. That is, the DNA molecule is operatively linked to a regulatory sequence in a manner which allows for expression (by transcription of the DNA molecule) of an RNA molecule which is antisense to MP mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. et al., Antisense RNA as a molecular tool for genetic analysis, Reviews - Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent.cell, but are still included within the scope of the term as used herein.

A host cell can be any prokaryotic or eukaryotic cell. For example, an MP protein can be expressed in bacterial cells such as *C. glutamicum*. Other suitable host cells are known to those of ordinary skill in the art. Microorganisms related to *Corynebacterium glutamicum* which may be conveniently used as host cells for the nucleic acid and protein molecules of the description are set forth in Table 3.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid (*e.g*., linear DNA or RNA (*e.g*., a linearized vector or a gene construct alone without a vector) or nucleic acid in the form of a vector (*e.g*., a plasmid, phage, phasmid, phagemid, transposon or other DNA) into a host cell, including calcium phosphate or calcium chloride co-precipitation, DEAE-dextran-mediated transfection, lipofection, natural competence, chemical-mediated transfer, or electroporation. Suitable methods for transforming or transfecting host cells can be found in Sambrook, et al. (Molecular Cloning: A Laboratory Manual. 2nd, ed, Cold Spring Harbor Laboratory, Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest Preferred selectable markers include those which confer resistance to drugs, such as G418, hygromycin and methotrexate. Nucleic acid encoding a selectable marker can be introduced into a host cell on the same vector as that encoding an MP protein or can be introduced on a separate vector. Cells stably transfected with the introduced nucleic acid can be identified by drug selection (*e.g*., cells that have incorporated the selectable marker gene will survive, while the other cells die).

To create a homologous recombinant microorganism, a vector is prepared which contains at least a portion of an MP gene into which a deletion, addition or substitution has been introduced to thereby alter, *e.g*., functionally disrupt, the MP gene. Preferably, this MP gene is a *Corynebacterium glutamicum* MP gene, but it can be a homologue from a related bacterium or even from a mammalian, yeast, or insect source. In a preferred embodiment, the vector is designed such that, upon homologous recombination, the endogenous MP gene is functionally disrupted (*i.e.,* no longer encodes a functional protein; also referred to as a "knock out" vector). Alternatively, the vector can be designed such that, upon homologous recombination, the endogenous MP gene is mutated or otherwise altered but still encodes functional protein (*e*.g., the upstream regulatory region can be altered to thereby alter the expression of the endogenous MP protein). In the homologous recombination vector, the altered portion of the MP gene is flanked at its 5' and 3' ends by additional nucleic acid of the MP gene to allow for homologous recombination to occur between the exogenous MP gene carried by the vector and an endogenous MP gene in a microorganism. The additional flanking MP nucleic acid is of sufficient length for successful homologous recombination with the endogenous gene. Typically, several kilobases of flanking DNA (both at the 5' and 3' ends) are included in the vector (see *e.g*., Thomas, K.R., and Capecchi, M.R. (1987) Cell 51: 503 for a description of homologous recombination vectors). The vector is introduced into a microorganism (*e.g*., by electroporation) and cells in which the introduced MP gene has homologously recombined with the endogenous MP gene are selected, using art-known techniques.

Recombinant microorganisms can be produced which contain selected systems which allow for regulated expression of the introduced gene. For example, inclusion of an MP gene on a vector placing it under control of the lac operon permits expression of the MP gene only in the presence of IPTG. Such regulatory systems are well known in the art.

In another of embodiment of the description, an endogenous MP gene in a host ce is disrupted (*e.g*., by homologous recombination or other genetic means known in the art) such that expression of its protein product does not occur. In another embodiment, an endogenous or introduced MP gene in a host cell has been altered by one or more point mutations, deletions, or inversions, but still encodes a functional MP protein. One or more of the regulatory regions (*e.*g., a promoter, repressor, or inducer) of an MP gene in a microorganism can be altered (*e.g*., by deletion, truncation, inversion, or point mutation) such that the expression of the MP gene is modulated. One of ordinary skill in the art will appreciate that host cells containing more than one of the described MP gene and protein modifications may be readily produced using the methods disclosed herein.

A host cell disclosed herein, such such as a prokaryotic or eukaryotic host cell in culture, can be used to produce (*i.e.,* express) an MP protein. Accordingly, methods for producing MP proteins using the host cell are also provided.

In one embodiment, the method comprises culturing the host cell of invention (into which a recombinant expression vector encoding an MP protein has been introduced, or into which genome has been introduced a gene encoding a wild-type or altered MP protein) in a suitable medium until MP protein is produced. In another embodiment, the method further comprises isolating MP proteins from the medium or the host cell.

### C. Isolated MP Proteins

Another aspect, the description provides isolated MP proteins, and biologically active portions thereof. An "isolated" or "purified" protein or biologically active portion thereof is substantially free of cellular material when produced by recombinant DNA techniques, or chemical precursors or other chemicals when chemically synthesized. The language "substantially free of cellular material" includes preparations of MP protein in which the protein is separated from cellular components of the cells in which it is naturally or recombinantly produced. In one embodiment, the language "substantially free of cellular material" includes preparations of MP protein having less than about 30% (by dry weight) of non-MP protein (also referred to herein as a "contaminating protein"), more preferably less than about 20% of non-MP protein, still more preferably less than about 10% of non-MP protein, and most preferably less than about 5% non-MP protein. When the MP protein or biologically active portion thereof is recombinantly produced, it is also preferably substantially free of culture medium, *i.e.,* culture medium represents less than about 20%, more preferably less than about 10%, and most preferably less than about 5% of the volume of the protein preparation. The language "substantially free of chemical precursors or other chemicals" includes preparations of MP protein in which the protein is separated from chemical precursors or other chemicals which are involved in the synthesis of the protein. In one embodiment, the language "substantially free of chemical precursors or other chemicals" includes preparations of MP protein having less than about 30% (by dry weight) of chemical precursors or non-MP chemicals, more preferably less than about 20% chemical precursors or non-MP chemicals, still more preferably less than about 10% chemical precursors or non-MP chemicals, and most preferably less than about 5% chemical precursors or non-MP chemicals. In preferred embodiments, isolated proteins or biologically active portions thereof lack contaminating proteins from the same organism from which the MP protein is derived. Typically, such proteins are produced by recombinant expression of, for example, a *C*. *glutamicum* MP protein in a microorganism such as *C*. *glutamicum.*

An isolated MP protein or a portion thereof can catalyze an enzymatic reaction in an amino acid, vitamin, cofactor, nutraceutical, nucleotide, nucleoside, or trehalose metabolic pathway, or has one or more of the activities set forth in Table 1. In preferred embodiments of the description, the protein or portion thereof comprises an amino acid sequence which is sufficiently homologous to an amino acid sequence an even-numbered SEQ ID NO: of the Sequence Listing such that the protein or portion thereof maintains the ability to catalyze an enzymatic reaction in an amino acid, vitamin, cofactor, nutraceutical, nucleotide, nucleoside, or trehalose metabolic pathway. The portion of the protein is preferably a biologically active portion as described herein. An other MP proteins can have an amino acid sequence set forth as an even-numbered SEQ ID NO: of the Sequence Listing, or an amino acid sequence which is encoded by a nucleotide sequence which hybridizes, e.g., hybridizes under stringent conditions, to a nucleotide sequence of an odd-numbered SEQ ID NO: of the Sequence Listing. Such MP protein can have an amino acid sequence which is encoded by a nucleotide sequence that is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71%, 72%, 73%, 74%, 75%, 76%, 77%, 78%, 79%, or 80%, 81%, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to one of the nucleic acid sequences disclosed herein, or a portion thereof. Ranges and identity values intermediate to the above-recited values, (*e.g*., 70-90% identical or 80-95% identical) are also useful . For example, ranges of identity values using a combination of any of the above values recited as upper and/or lower limits are intended to be included. The preferred MP proteins also preferably possess at least one of the MP activities described herein. For example, a preferred MP protein includes an amino acid sequence encoded by a nucleotide sequence which hybridizes, *e.g*., hybridizes under stringent conditions, to a nucleotide sequence of the invention, and which can catalyze an enzymatic reaction in an amino acid, vitamin, cofactor, nutraceutical, nucleotide, nucleoside, or trehalose metabolic pathway, or which has one or more of the activities set fort in Table 1.

A useful MP protein is substantially homologous to an amino acid sequence of an even-numbered SEQ ID NO: of the Sequence Listing and retains the functional activity of the protein of one of the amino acid sequences yet differs in amino acid sequence due to natural variation or mutagenesis, as described in detail in subsection I above. Such MP protein is a protein which comprises an amino acid sequence which is at least about 50%, 51%, 52%, 53%, 54%, 55%, 56%, 57%, 58%, 59%, or 60%, preferably at least about 61%, 62%, 63%, 64%, 65%, 66%, 67%, 68%, 69%, or 70%, more preferably at least about 71%, 72%, 73%, 74%, 75%, 76%, 77%, 78%, 79%, or 80%, 81 %, 82%, 83%, 84%, 85%, 86%, 87%, 88%, 89%, or 90%, or 91%, 92%, 93%, 94%, and even more preferably at least about 95%, 96%, 97%, 98%, 99%, 99.7% or more homologous to an entire amino acid sequence and which has at least one of the MP activities described herein. Ranges and identity values intermediate to the above-recited values, (*e.g*., 70-90% identical or 80-95% identical) are also useful. For example, ranges of identity values using a combination of any of the above values recited as upper and/or lower limits are intended to be included.

Biologically active portions of an MP protein include peptides comprising amino acid sequences derived from the amino acid sequence of an MP protein, *e.g*., an amino acid sequence of an even-numbered SEQ ID NO: of the Sequence Listing or the amino acid sequence of a protein homologous to an MP protein, which include fewer amino acids than a full length MP protein or the full length protein which is homologous to an MP protein, and exhibit at least one activity of an MP protein. Typically, biologically active portions (peptides, *e.g*., peptides which are, for example, 5, 10,15,20,30, 35, 36, 37, 38, 39, 40, 50, 100 or more amino acids in length) comprise a domain or motif with at least one activity of an MP protein. Moreover, other biologically active portions, in which other regions of the protein are deleted, can be prepared by recombinant techniques and evaluated for one or more of the activities described herein. Preferably, the biologically active portions of an MP protein include one or more selected domains/motifs or portions thereof having biological activity.

MP proteins are preferably produced by recombinant DNA techniques. For example, a nucleic acid molecule encoding the protein is cloned into an expression vector (as described above), the expression vector is introduced into a host cell (as described above) and the MP protein is expressed in the host cell. The MP protein can then be isolated from the cells by an appropriate purification scheme using standard protein purification techniques. Alternative to recombinant expression, an MP protein, polypeptide, or peptide can be synthesized chemically using standard peptide synthesis techniques. Moreover, native MP protein can be isolated from cells (*e.g*., endothelial cells), for example using an anti-MP antibody, which can be produced by standard techniques utilizing an MP protein or fragment thereof of this description.

The description also provides MP chimeric or fusion proteins. As used herein, an MP "chimeric protein" or "fusion protein" comprises an MP polypeptide operatively linked to a non-MP polypeptide. An "MP polypeptide" refers to a polypeptide having an amino acid sequence corresponding to MP, whereas a "non-MP polypeptide" refers to a polypeptide having an amino acid sequence corresponding to a protein which is not substantially homologous to the MP protein, *e.g*., a protein which is different from the MP protein and which is derived from the same or a different organism. Within the fusion protein, the term "operatively linked" is intended to indicate that the MP polypeptide and the non-MP polypeptide are fused in-frame to each other. The non-MP polypeptide can be fused to the N-terminus or C-terminus of the MP polypeptide. For example, in one embodiment the fusion protein is a GST-MP fusion protein in which the MP sequences are fused to the C-terminus of the GST sequences. Such fusion proteins can facilitate the purification of recombinant MP proteins. In another embodiment, the fusion protein is an MP protein containing a heterologous signal sequence at its N-terminus. In certain host cells (*e.g*., mammalian host cells), expression and/or secretion of an MP protein can be increased through use of a heterologous signal sequence.

Preferably, an MP chimeric or fusion protein of the description is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel *et al.* John Wiley & Sons: 1992). Moreover, many expression vectors are commercially available that already encode a fusion moiety (*e.g*., a GST polypeptide). An MP-encoding nucleic acid can be cloned into such an expression vector such that the fusion moiety is linked in-frame to the MP protein.

Homologues of the MP protein can be generated by mutagenesis, *e.g*., discrete point mutation or truncation of the MP protein. As used herein, the term "homologue" refers to a variant form of the MP protein which acts as an agonist or antagonist of the activity of the MP protein. An agonist of the MP protein can retain substantially the same, or a subset, of the biological activities of the protein. An antagonist of the MP protein can inhibit one or more of the activities of the naturally occurring form of the MP protein, by, for example, competitively binding to a downstream or upstream member of the MP cascade which includes the MP proteins Thus, the *C*. *glutamicum* MP protein and homologues thereof of the present description may modulate the activity of one or more metabolic pathways in which MP proteins play a role in this microorganism.

In an alternative embodiment of the description, homologues of the MP protein can be identified by screening combinatorial libraries of mutants, *e.g*., truncation mutants, of the MP protein for MP protein agonist or antagonist activity. A variegated library of MP variants can be generated by combinatorial mutagenesis at the nucleic acid level and is encoded by a variegated gene library. A variegated library of MP variants can be produced by, for example, enzymatically ligating a mixture of synthetic oligonucleotides into gene sequences such that a degenerate set of potential MP sequences is expressible as individual polypeptides, or alternatively, as a set of larger fusion proteins (*e.g*., for phage display) containing the set of MP sequences therein. There are a variety of methods which can be used to produce libraries of potential MP homologues from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential MP sequences. Methods for synthesizing degenerate oligonucleotides are known in the art (see, *e.g*., Narang, S.A. (1983) Tetrahedron 39:3; Itakura et al. (1984) Annu. Rev. Biochem. 53:323; Itakura et al. (1984) Science 198:1056; Ike et al. (1983) Nucleic Acid Res. 11:477.

In addition, libraries of fragments of the MP protein coding can be used to generate a variegated population of MP fragments for screening and subsequent selection of homologues of an MP protein. A library of coding sequence fragments can be generated by treating a double stranded PCR fragment of an MP coding sequences with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the MP protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of MP homologues. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique which enhances the frequency of functional mutants in the libraries, can be used in combination with the screening assays to identify MP homologues (Arkin and Yourvan (1992) PNAS 89:7811-7815; Delgrave et al. (1993) Protein Engineering 6(3):327-331).

Cell based assays can be exploited to analyze a variegated MP library, using methods well known in the art.

### D. Uses and Methods of the Invention

The nucleic acid molecules, proteins, protein homologues, fusion proteins, primers, vectors, and host cells described herein can be used in one or more of the following methods: identification of *C*. *glutamicum* and related organisms; mapping of genomes of organisms related to *C*. *glutamicum;* identification and localization of *C*. *glutamicum* sequences of interest; evolutionary studies; determination of MP protein regions required for function; modulation of an MP protein activity; modulation of the activity of an MP pathway; and modulation of cellular production of a desired compound, such as a fine chemical.

The MP nucleic acid molecule of the invention has a variety of uses. First, it may be used to identify an organism as being *Corynebacterium glutamicum* or a close relative thereof. Also, they may be used to identify the presence of C. *glutamicum* or a relative thereof in a mixed population of microorganisms. The invention provides the nucleic acid sequence of a *C*. *glutamicum* gene; by probing the extracted genomic DNA of a culture of a unique or mixed population of microorganisms under stringent conditions with a probe spanning a region of a *C*. *glutamicum* gene which is unique to this organism, one can ascertain whether this organism is present. Although *Corynebacterium glutamicum* itself is not pathogenic to humans, it is related to species which are human pathogens, such as *Corynebacterium diphtheriae. Corynebacterium diphtheriae* is the causative agent of diphtheria, a rapidly developing, acute, febrile infection which involves both local and systemic pathology. In this disease, a local lesion develops in the upper respiratory tract and involves necrotic injury to epithelial cells; the bacilli secrete toxin which is disseminated through this lesion to distal susceptible tissues of the body. Degenerative changes brought about by the inhibition of protein synthesis in these tissues, which include heart, muscle, peripheral nerves, adrenals, kidney, liver and spleen, result in the systemic pathology of the disease. Diphtheria continues to have high incidence in many parts of the world, including Africa, Asia, Eastern Europe and the independent states of the former Soviet Union. An ongoing epidemic of diphtheria in the latter two regions has resulted in at least 5,000 deaths since 1990.

In one embodiment, the description provides a method of identifying the presence or activity of *Cornyebacterium diphtheriae* in a subject. This method includes detection of one or more of the nucleic acid or amino acid sequences set forth as odd-numbered or even-numbered SEQ ID NOs, respectively, in the Sequence Listing in a subject, thereby detecting the presence or activity of *Corynebacterium diphtheriae* in the subject. *C. glutamicum* and *C*. *diphtheriae* are related bacteria, and many of the nucleic acid and protein molecules in C. *glutamicum* are homologous to *C*. *diphtheriae* nucleic acid and protein molecules, and can therefore be used to detect *C*. *diphtheriae* in a subject.

The nucleic acid and protein molecule of the invention may also serve as markers for specific regions of the genome. This has utility not only in the mapping of the genome, but also for functional studies of *C*. *glutamicum* proteins. For example, to identify the region of the genome to which a particular *C*. *glutamicum* DNA-binding protein binds, the *C*. *glutamicum* genome could be digested, and the fragments incubated with the DNA-binding protein. Those which bind the protein may be additionally probed with the nucleic acid molecule of the invention, preferably with readily detectable labels; binding of such a nucleic acid molecule to the genome fragment enables the localization of the fragment to the genome map of *C*. *glutamicum,* and, when performed multiple times with different enzymes, facilitates a rapid determination of the nucleic acid sequence to which the protein binds. Further, the nucleic acid molecule of the invention may be sufficiently homologous to the sequences of related species such that these nucleic acid molecules may serve as markers for the construction of a genomic map in related bacteria, such as *Brevibacterium lactofermentum.*

The MP nucleic acid molecule of the invention is also useful for evolutionary and protein structural studies. The metabolic processes in which the molecule of the invention participates are utilized by a wide variety of prokaryotic and eukaryotic cells; by comparing the sequences of the nucleic acid molecules of the present invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Manipulation of the MP nucleic acid molecule of the invention may result in the production of a MP protein having functional differences from the wild-type MP proteins. This protein may be improved in efficiency or activity, may be present in greater numbers in the cell than is usual, or may be decreased in efficiency or activity.

The description also provides methods for screening molecules which modulate the activity of an MP protein, either by interacting with the protein itself or a substrate or binding partner of the MP protein, or by modulating the transcription or translation of an MP nucleic acid molecule. In such methods, a microorganism expressing an MP protein of the invention is contacted with one or more test compounds, and the effect of each test compound on the activity or level of expression of the MP protein is assessed.

When the desired fine chemical to be isolated from large-scale fermentative culture of *C*. *glutamicum* is an amino acid, modulation of the activity or efficiency of activity of a protein disclosed herein by recombinant genetic mechanisms may directly impact the production of one of these amino acids. For example, in the case of an enzyme in a biosynthetic pathway for a desired amino acid, improvement in efficiency or activity of the enzyme (including the presence of multiple copies of the gene) should lead to an increased production or efficiency of production of that desired amino acid. In the case of an enzyme in a biosynthetic pathway for an amino acid whose synthesis is in competition with the synthesis of a desired amino acid, any decrease in the efficiency or activity of this enzyme (including deletion of the gene) should result in an increase in production or efficiency of production of the desired amino acid, due to decreased competition for intermediate compounds and/or energy. In the case of an enzyme in a degradation pathway for a desired amino acid, any decrease in efficiency or activity of the enzyme should result in a greater yield or efficiency of production of the desired product due to a decrease in its degradation. Lastly, mutagenesis of an enzyme involved in the biosynthesis of a desired amino acid such that this enzyme is no longer is capable of feedback inhibition should result in increased yields or efficiency of production of the desired amino acid. The same should apply to the biosynthetic and degradative enzymes involved in the metabolism of vitamins, cofactors, nutraceuticals, nucleotides, nucleosides and trehalose.

Similarly, when said desired chemical is not one of the aforementioned compounds, the modulation of activity of one of the proteins disclosed herein may still impact the yield and/or efficiency of production of the compound from large-scale culture of *C*. *glutamicum.* The metabolic pathways of any organism are closely interconnnected; the intermediate used by one pathway is often supplied by a different pathway. Enzyme expression and function may be regulated based on the cellular levels of a compound from a different metabolic process, and the cellular levels of molecules necessary for basic growth, such as amino acids and nucleotides, may critically affect the viability of the microorganism in large-scale culture. Thus, modulation of an amino acid biosynthesis enzyme, for example, such that it is no longer responsive to feedback inhibition or such that it is improved in efficiency or turnover may result in increased cellular levels of one or more amino acids. In turn, this increased pool of amino acids provides not only an increased supply of molecules necessary for protein synthesis, but also of molecules which are utilized as intermediates and precursors in a number of other biosynthetic pathways. If a particular amino acid had been limiting in the cell, its increased production might increase the ability of the cell to perform numerous other metabolic reactions, as well as enabling the cell to more efficiently produce proteins of all kinds, possibly increasing the overall growth rate or survival ability of the cell in large scale culture. Increased viability improves the number of cells capable of producing the desired fine chemical in fermentative culture, thereby increasing the yield of this compound. Similar processes are possible by the modulation of activity of a degradative enzyme of the invention such that the enzyme no longer catalyzes, or catalyzes less efficiently, the degradation of a cellular compound which is important for the biosynthesis of a desired compound, or which will enable the cell to grow and reproduce more efficiently in large-scale culture. It should be emphasized that optimizing the degradative activity or decreasing the biosynthetic activity of certain molecules of the invention may also have a beneficial effect on the production of certain fine chemicals from *C*. *glutamicum.* For example, by decreasing the efficiency of activity of a biosynthetic enzyme in a pathway which competes with the biosynthetic pathway of a desired compound for one or more intermediates, more of those intermediates should be available for conversion to the desired product. A similar situation may call for the improvement of degradative ability or efficiency of one or more proteins of the invention.

This aforementioned list of mutagenesis strategies for MP proteins to result in increased yields of a desired compound is not meant to be limiting; variations on these mutagenesis strategies will be readily apparent to one of ordinary skill in the art. By these mechanisms, the nucleic acid and protein molecules of the description may be utilized to generate *C. glutamicum* or related strains of bacteria expressing mutated MP nucleic acid and protein molecules such that the yield, production, and/or efficiency of production of a desired compound is improved. This desired compound may be any natural product of *C*. *glutamicum,* which includes the final products of biosynthesis pathways and intermediates of naturally-occurring metabolic pathways, as well as molecules which do not naturally occur in the metabolism of *C. glutamicum,* but which are produced by a *C*. *glulamicum* strain. Preferred compounds to be produced by *Corynebacterium glutamicum* strains are the amino acids L-lysine and L-methionine.

### E) Particularly useful embodiments

Among the MP molecules disclosed herein, three stand out as being particularly useful.

The first is a gene that was identified from the genome of *Corynebacterium glutamicum* as a gene coding for a hypothetical transcriptional regulatory protein. This gene is described as RXA00657. The nucleotide sequence of RNA00657 corresponds to SEQ ID NO:5. The amino acid sequence of RXA00657 corresponds to SEQ ID NO:6. It was found that when the RXA00657 gene, as well as upstream and downstream regulatory regions described in the examples, was cloned into a vector capable of replicating in *Corynebacterium glutamicum* and transformed and expressed in a lysine producing strain such as ATCC 13286, that this strain produced more lysine compared to the strain transformed with the same plasmid lacking the aforementioned nucleotide fragment RNA00657. In addition to the observation that the lysine titer was increased in the mentioned strain, the selectivity determined by the molar amount af lysine produced compared to the molar amount of sucrose consumed was increased (see Example 14). Overexpression of RNA00657 in combination with the overexpression of other genes either directly involved in the lysine specific pathway such as *lysC, dapA., dapB, dapC, dapD, dapF, ddh, lysE, lysG,* and *lysR* results in an increase in the production of lysine compared to RXA00657 alone.

The second is the metC gene (SEQ ID NO:3), encoding the cystathionine b-lyase of the methionine biosynthetic pathway of C. glutamicum. The translational product of the gene (SEQ ID NO:4) showed no significant homology with that of *metC* gene from other organisms. Introduction of the plasmid containing the *metC* gene into *C*. *glutamicum* resulted in a 5-fold increase in the activity of cystathionine β-lyase. The protein product, now designated MetC (corresponding to SEQ ID NO:4), which encodes a protein product of 35,574 Daltons and consists of 325 amino acids, is identical to the previously reported *aecD* gene (Rossol, I. and Puhler, A. (1992) J. Bacteriology 174, 2968-2977) except the existence of two different amino acids. Like *aecD* gene, when present in multiple copies, *metC* gene conferred resistance to *S*-(β-aminoethyl)-cysteine which is a toxic lysine analog. However, genetic and biochemical evidences suggest that the natural activity of *metC* gene product is to mediate methionine biosynthesis in *C*. *glutamicum.* Mutant strains of *metC* were constructed and the strains showed methionine prototrophy. The mutant strains completely lost their ability to show resistance to *S*-(γ-aminoethyl)-cysteine. These results show that, in addition to the transsulfuration, which is another biosynthetic pathway, the direct sulfhydrylation pathway is functional in *C*. *glutamicum* as a parallel biosynthetic route for methionine.

The third one is the new gene to which the invention relates, metZ (or metY). The isolation of this gene (SEQ ID NO:1), and of its product (SEQ ID NO:2), demonstrates the presence of a sulfhydrylation pathway catalyzed by O-acetylhomoserine sulfidrylase. Among the eukaryotes, fungi and yeast species have been reported to have both the transsulfuration and direct sulfhydrylation pathway. Thus far, no prokaryotic organism which possesses both pathways has been found. Unlike *E. coli* which only possesses single biosynthetic route for lysine, *C*. *glutamicum* possesses two parallel biosynthetic pathways for the amino acid. The biosynthetic pathway for methionine in *C*. *glutamicum* is analogous to that of lysine in that aspect.

The gene *metZ* is located in the upstream region of *metA,* which is the gene encoding the enzyme catalysing the first step of methionine biosynthesis (Park, S.-D., et al. (1998) Mol. Cells 8, 286-294). Regions upstream and downstream of *metA* were sequenced to identify other *met* genes. It appears that *metZ* and *metA* form an operon. Expression of the genes encoding MetA and MetZ leads to overproduction of the corresponding polypeptides.

Surprisingly, *metZ* clones can complement methionine auxotrophic *Escherichia coli metB* mutant strains. This shows that the protein product of *metZ* catalyzes a step that can bypass the step catalyzed by the protein product of *metB. MetZ* was also disrupted and the mutant strain showed methionine prototrophy. *Corynebacterium glutamicum metB* and *metZ* double mutants were also constructed. The double mutant is auxotrophic for methionine. Thus, *metZ* encodes a protein catalysing the reaction from O-Acetyl-Homoserine to Homocysteine, which is one step in the sulfhydrylation pathway of Methionine biosynthesis. *Corynebacterium glutamicum* contains both the transsulfuration and the sulfhydrylation pathway of methionine biosynthesis.

Introduction of *metZ* into *C. glutamicum* resulted in the expression of a 47,000 Dalton protein. Combined introduction of *metZ* and *metA* in *C. glutamicum* resulted in the appearance of *metA* and *metZ* proteins as shown by gel electrophoresis. If the Corynebacterium strain is a lysine overproducer, introduction of a plasmid containing *metZ* and *metA* resulted in a lower lysine titer but accumulation of homocysteine and methionine is detected.

In another embodiment *metZ* and *metA* were introduced into *Corynebacterium glutamicum* strains together with the *hom* gene, encoding the homoserine dehydrogenase, catalyzing the conversion from aspartate semialdehyde to homoserine. Different *hom* genes from different organisms were chosen for this experiment. The *Corynebacterium glutamicum hom* gene can be used as well as *hom* genes from other procaryotes like *Escherichia coli* or *Bacillus subtilis* or the *hom* gene of eukaryotes such as *Saccharomyces cerevisiae, Shizosaccharomyces pombe, Ashbya gossypii* or algae, higher plants or animals. It may be that the *hom* gene is insensitive against feed back inhibition mediated by any metabolites that occur in the biosynthetic routes of the amino acids of the aspartate family, like aspatrate, lysine, threonine or methionine. Such metabolites are for example aspartate, lysine, methionine, threonine, aspartyl-phosphate, aspartate senialdehyd, homoserine, cystathionine, homocysteine or any other metabolite that occurs in this biosynthetic routes. In addition to the metabolites, the homoserine dehydrogenase may be insensitive against inhibition by analogues of all those metabolites or even against other compounds involved in this metabolism as there are other amino acids like cysteine or cofactors like vitamin B 12 and all of its derivatives and S-adenosylmethionine and its metabolites and derivatives and analogues. The insensitivity of the Homoserine dehydrogenase against all these, a part of these or only one of these compounds may either be its natural attitude or it may be the result from one or more mutations that resulted from classical mutation and selection using chemicals or irradiation or other mutagens. The mutations could also be introduced into the *hom* gene using gene technology, for example the introduction of site specific point mutations or by any method aforementioned for the MP or MP encoding DNA-sequences.

When a *hom* gene was combined with the *metZ* and *metA* genes and introduced into a *Corynebacterium glutamicum* strain that is a lysine overproducer, lysine accumulation was reduced and homocysteine and methionine accumulation was enhanced. A further enhancement of homocysteine and methionine concentrations can be achieved, if a lysine overproducing *Corynebacterium glutamicum* strain is used and a disruption of the ddh gene or the *lysA* gene was introduced prior to the transformation with DNA containing a *hom* gene and *metZ* and *metA* in combination. The overproduction of homocysteine and methionine was possible using different sulfur sources. Sulfates, thiosulfates, sulfites and also more reduced sulfur sources like H₂S and sulfides and derivatives could be used. Also, organic sulfur sources like methyl mercaptan, thioglycolates, thiocyanates, thiourea, sulfur containing amino acids like cysteine and other sulfur containing compounds can be used to achieve homocysteine and methionine overproduction.

The *metC* gene was introduced into *a Corynebacterium glutamicum* strain using aforementioned methods. The *metC* gene can be transformed into the strain in combination with other genes like *metB, metA* and *metA.* The *hom* gene can also be added. When the *hom* gene, the *met C, metA* and *metB* genes were combined on a vector and introduced into a *Corynebacterium glutamicum* strain, homocysteine and methionine overproduction was achieved. The overproduction of homocysteine and methionine was possible using different sulfur sources. Sulfates, thiosulfates, sulfites and also more reduced sulfur sources like H₂S and sulfides and derivatives could be used. Also, organic sulfur sources like methyl mercaptan, thioglycolates, thiocyanates, thiourea, sulfur containing amino acids like cysteine and other sulfur containing compounds can be used to achieve homocysteine and methionine overproduction.

### F) Subject matter of the invention

The subject matter of the invention encompasses the following embodiments:
1. An isolated polypeptide from Corynebacterium glutamicum, said protein being involved in the metabolism of Methionine and Lysine and comprising the amino acid sequence set forth in SEQ ID NO: 2.
2. An isolated polypeptide according to claim 1, further comprising heterologous amino acid sequences.
3. An isolated nucleic acid molecule from Corynebacterium glutamicum encoding the protein of claim 1.
4. The isolated nucleic acid of a claim 3, wherein said nucleic acid comprises the nucleotide sequence set forth in SEQ ID NO: 1 or a complement thereof.
5. A vector comprising the nucleic acid of claim 4.
6. The vector of claim 5, further comprising one or more nucleic acids encoding proteins involved in metabolic pathways.
7. The vector of claim 6, wherein the second and further nucleic acids are selected from the odd-numbered sequences listed in Table 1, excluding any F-designated nucleic acid molecule.
8. The vector of any one of claims 5 or 6, which is an expression vector.
9. A host cell transfected with the expression vector of claim 8, wherein said cell is a microorganism.
10. The host cell of claim 9, wherein the cell belongs to the genus Corynebacterium or Brevibacterium.
11. The cell of claim 10, selected from the group consisting of
   *Corynebacterium glutamicum, Corynebacterium herculis, Corynebacterium lilium, Corynebacterium acetoacidophilum, Corynebacterium acetoglutamicum, Corynebacterium acetophilum, Corynebacterium ammoniagenes, Corynebacterium fujiokense, Corynebacterium nitrilophilus, Brevibacterium ammoniagenes, Brevibacterium butanicum, Brevibacterium divaricatum, Breibacterium flavum, Brevibacterium heali, Brevibacterium ketoglutamicum, Brevibacterium ketosoreductum, Brevibacterium lactogermentum, Brevibacterium linens, Brevirbacterium paraffinolyticum, and those strains set forth in Table 3.*
12. The host cell of any one of claims 9, 10 or 11, wherein the expression of said nucleic acid molecule results in the modulation of the production of methionine and/or lysine.
13. A method of producing a polypeptide of claim 1 comprising culturing the host cell of claim 9 in an appropriate culture medium.
14. A method of producing a fine chemical, comprising culturing a cell of claim 12.
15. A method of claim 14, wherein said cell is cultured in the presence of a sulphur source.
16. The method of claim 14, wherein said method further comprises the step of recovering the fine chemical from said culture.
17. The method of any one of claims 14, 15 or 16, wherein the fine chemical is an amino acid.
18. The method of claim 17, wherein the amino acid is methionine or lysine.
19. A method for producing a fine chemical comprising culturing a cell whose genomic DNA has been altered by the inclusion of a nucleic acid molecule of claim 4.
20. The method of claim 19, wherein the cell's genomic DNA has been further altered by the inclusion of a nucleic acid encoding a protein involved in metabolic pathways.
21. The method of claim 20, wherein said further nucleic acid is selected from the odd-numbered sequences listed in Table 1, excluding any F-designated nucleic acid molecule.
22. The method of claims 19 or 20, wherein the nucleic acid molecule is selected from the group consisting of metC, metB, metA, metE, metH, hom, asd, lysC, lysC/ask, rxa00657, dapA, dapB, dapC, dapD/argD, dapE, dapF, LysA, ddh, lysE, lysG, lysR, hsk, ppc, pycA, accD, accA, accB, addD, gpdh genes or any combination of the above-mentioned genes.

The invention is further illustrated by the following examples, which should not be construed as limiting.

### Exemplification

### Example 1: Preparation of total genomic DNA of Corynebacterium glutamicum ATCC13032

A culture of *Corynebacterium glutamicum* (ATCC 13032) was grown overnight at 30°C with vigorous shaking in BHI medium (Difco). The cells were harvested by centrifugation, the supernatant was discarded and the cells were resuspended in 5 ml buffer-I (5% of the original volume of the culture - all indicated volumes have been calculated for 100 ml of culture volume). Composition of buffer-I: 140.34 g/l sucrose, 2.46 g/l MgSO₄ x 7H₂O, 10 ml/l KH₂PO₄ solution (100 g/l, adjusted to pH 6.7 with KOH), 50 ml/l M12 concentrate (10 g/l (NH₄)₂SO₄, 1 g/l NaCl, 2 g/l MgSO₄ x 7H₂O, 0.2 g/l CaCl₂, 0.5 g/l yeast extract (Difco), 10 ml/l trace-elements-mix (200 mg/l FeSO₄ x H₂O, 10 mg/l ZnSO₄ x 7 H₂O, 3 mg/l MnCl₂ x 4 H₂O, 30 mg/l H₃BO₃ 20 mg/l CoCl₂, x 6 H₂O, 1 mg/l NiCl₂ x 6 H₂O, 3 mg/l Na₂MoO₄ x 2 H₂O, 500 mg/l complexing agent (EDTA or critic acid), 100 ml/l vitamins-mix (0.2 mg/l biotin, 0.2 mg/l folic acid, 20 mg/l p-amino benzoic acid, 20 mg/l riboflavin, 40 mg/l ca-panthothenate, 140 mg/l nicotinic acid, 40 mg/l pyridoxole hydrochloride, 200 mg/l myo-inositol). Lysozyme was added to the suspension to a final concentration of 2.5 mg/ml. After an approximately 4 h incubation at 37°C, the cell wall was degraded and the resulting protoplasts are harvested by centrifugation. The pellet was washed once with 5 ml buffer-I and once with 5 ml TE-buffer (10 mM Tris-HCl, 1 mM EDTA, pH 8). The pellet was resuspended in 4 ml TE-bufFer and 0.5 ml SDS solution (10%) and 0.5 ml NaCl solution (5 M) are added. After adding of proteinase K to a final concentration of 200 µg/ml, the suspension is incubated for ca.18 h at 37°C. The DNA was purified by extraction with phenol, phenol-chloroform-isoamylalcohol and chloroform-isoamylalcohol using standard procedures. Then, the DNA was precipitated by adding 1/50 volume of 3 M sodium acetate and 2 volumes of ethanol, followed by a 30 min incubation at -20°C and a 30 min centrifugation at 12,000 rpm in a high speed centrifuge using a SS34 rotor (Sorvall). The DNA was dissolved in 1 ml TE-buffer containing 20 µg/ml RNaseA and dialysed at 4°C against 1000 ml TE-buffer for at least 3 hours.

During this time, the buffer was exchanged 3 times. To aliquots of 0.4 ml of the dialysed DNA solution, 0.4 ml of 2 M LiCl and 0.8 ml of ethanol are added. After a 30 min incubation at -20°C, the DNA was collected by centrifugation (13,000 rpm, Biofuge Fresco, Heraeus, Hanau, Germany). The DNA pellet was dissolved in TE-buffer. DNA prepared by this procedure could be used for all purposes, including southern blotting or construction of genomic libraries.

### Example 2: Construction of genomic libraries in Escherichia coli of Corynebacterium glutamicum ATCC13032.

Using DNA prepared as described in Example 1, cosmid and plasmid libraries were constructed according to known and well established methods (*see e.g.,* Sambrook, J. *et al.* (1989) "Molecular Cloning : A Laboratory Manual", Cold Spring Harbor Laboratory Press, or Ausubel, F.M. *et al.* (1994) "Current Protocols in Molecular Biology", John Wiley & Sons.)

Any plasmid or cosmid could be used. Of particular use were the plasmids pBR322 (Sutcliffe, J.G. (1979) Proc. Natl. Acad Sci. USA, 75:3737-3741); pACYC177 (Change & Cohen (1978) J. Bacteriol 134:1141-1156), plasmids of the pBS series (pBSSK+, pBSSK- a others; Stratagene, LaJolla, USA), or cosmids as SuperCos1 (Stratagene, LaJolla, USA) or Lorist6 (Gibson, T.J., Rosenthal A. and Waterson, R.H. (1987) Gene 53:283-286. Gene libra specifically for use in *C*. *glutamicum* may be constructed using plasmid pSL109 (Lee, H.-S. A. J. Sinskey (1994) J. Microbiol. Biotechnol. 4: 256-263).

For the isolation of *metC* clones, *E*. *coli* JE6839 cells were transformed with the library DNA and plated onto the M9 minimal medium containing ampicillin and appropriate supplements. The plates were incubated at 37°C for 5 days. Colonies were isolated and screened for the plasmid content. The complete nucleotide sequence of the isolated *metC* gene was determined by methods well-known to one of ordinary skill in the art.

### Example 3: DNA Sequencing and Computational Functional Analysis

Genomic libraries as described in Example 2 were used for DNA sequencing according to standard methods, in particular by the chain termination method using ABI377 sequencing machines (see *e.g*., Fleischman, R.D. et al. (1995) "Whole-genome Random Sequencing and Assembly of Haemophilus Influenzae Rd., Science, 269:496-512). Sequencing primers with the following nucleotide sequences were used: 5'-GGAAACAGTATGACCATG-3' (SEQ ID NO:123) or 5'-GTAAAACGACGGCCAGT-3'(SEQ ID NO.:124).

### Example 4: In vivo Mutagenesis

*In vivo* mutagenesis of *Corynebacterium glutamicum* can be performed by passage or plasmid (or other vector) DNA through *E. coli* or other microorganisms (*e.g. Bacillus* spp. or yeasts such as *Saccharomyces cerevisiae*) which are impaired in their capabilities to maintain the integrity of their genetic information. Typical mutator strains have mutations in the genes for the DNA repair system (*e.g*., mutHLS, mutD, mutT, etc.; for reference, see Rupp, W.D. (1996) DNA repair mechanisms, in: *Escherichia coli* and *Salmonella,* p. 2277-2294, ASM: Washington.) Such strains are well known to those of ordinary skill in the art. The use of such strains is illustrated, for example, in Greener, A. and Callahan, M. (1994) Strategies 7: 32-34.

### Example 5: DNA Transfer Between Escherichia coli and Corynebacterium glutamicum

Several *Corynebacterium* and *Brevibacterium* species contain endogenous plasmids (as *e.g.,* pHM1519 or pBL1) which replicate autonomously (for review see, *e.g.,* Martin, J.F. et al. (1987) Biotechnology, 5:137-146). Shuttle vectors for *Escherichia coli* and *Corynebacterium glutamicum* can be readily constructed by using standard vectors for *E. coli* (Sambrook, J. *et al.* (1989), "Molecular Cloning: A Laboratory Manual", Cold Spring Harbor Laboratory Press or Ausubel, F.M. *et al.* (1994) "Current Protocols in Molecular Biology", John Wiley & Sons) to which a origin or replication for and a suitable marker from *Corynebacterium glutamicum* is added. Such origins of replication are preferably taken from endogenous plasmids isolated from *Corynebacterium* and *Brevibacterium* species. Of particular use as transformation markers for these species are genes for kanamycin resistance (such as those derived from the Tn5 or Tn903 transposons) or chloramphenicol (Winnacker, E.L. (1987) "From Genes to Clones - Introduction to Gene Technology, VCH, Weinheim). There are numerous examples in the literature of the construction of a wide variety of shuttle vectors which replicate in both *E*. *coli and C. glutamicum,* and which can be used for several purposes, including gene overexpression (for reference, see *e.g*., Yoshihama, M. et al. (1985) J. Bacteriol. 162:591-597, Martin J.F. et al. (1987) Biotechnology, 5:137-146 and Eikmanns, B.J. et al. (1991) Gene, 102:93-98).

Using standard methods, it is possible to clone a gene of interest into one of the shuttle vectors described above and to introduce such a hybrid vectors into strains of *Corynebacterium glutamicum.* Transformation of *C*. *glutamicum* can be achieved by protoplast transformation (Kastsumata, R. et al. (1984) J. Bacteriol. 159306-311), electroporation (Liebl, E. et al. (1989) FEMS Microbiol. Letters, 53:399-303) and in cases where special vectors are used, also by conjugation (as described *e.g*. in Schäfer, A et al. (1990) J. Bacteriol. 172:1663-1666). It is also possible to transfer the shuttle vectors for *C. glutamicum* to *E*. *coli* by preparing plasmid DNA from C. *glutamicum* (using standard methods well-known in the art) and transforming it into E. *coli.* This transformation step can be performed using standard methods, but it is advantageous to use an Mcr-deficient *E. coli* strain, such as NM522 (Gough & Murray (1983) J. Mol. Biol. 166:1-19).

Genes may be overexpressed in C. *glutamicum* strains using plasmids which comprise pCG1 (U.S. Patent No. 4,617,267) or fragments thereof, and optionally the gene for kanamycin resistance from TN903 (Grindley, N.D. and Joyce, C.M. (1980) Proc. Natl. Acad. Sci. USA 77(12): 7176-7180). In addition, genes may be overexpressed in *C*. *glutamicum* strains using plasmid pSL109 (Lee, H.-S. and A. J. Sinskey (1994) J. Microbiol. Biotechnol. 4: 256-263).

Aside from the use of replicative plasmids, gene overexpression can also be achieved by integration into the genome. Genomic integration in *C*. *glutamicum* or other Corynebacterium or Brevibacterium species may be accomplished by well-known methods, such as homologous recombination with genomic region(s), restriction endonuclease mediated integration (REMI) (see, *e.g*., DE Patent 19823834), or through the use of transposons. It is also possible to modulate the activity of a gene of interest by modifying the regulatory regions (*e.g*., a promoter, a repressor, and/or an enhancer) by sequence modification, insertion, or deletion using site-directed methods (such as homologous recombination) or methods based on random events (such as transposon mutagenesis or REMI). Nucleic acid sequences which function as transcriptional terminators may also be inserted 3' to the coding region of one or more genes of the invention; such terminators are well-known in the art and are described, for example, in Winnacker, E.L. (1987) From Genes to Clones - Introduction to Gene Technology. VCH: Weinheim.

### Example 6: Assessment of the Expression of the Mutant Protein

Observations of the activity of a mutated protein in a transformed host cell rely on the fact that the mutant protein is expressed in a similar fashion and in a similar quantity to that of the wild-type protein. A useful method to ascertain the level of transcription of the mutant gene (an indicator of the amount of mRNA available for translation to the gene product) is to perform a Northern blot (for reference see, for example, Ausubel *et al.* (1988) Current Protocols in Molecular Biology, Wiley: New York), in which a primer designed to bind to the gene of interest is labeled with a detectable tag (usually radioactive or chemiluminescent), such that when the total RNA of a culture of the organism is extracted, run on gel, transferred to a stable matrix and incubated with this probe, the binding and quantity of binding of the probe indicates the presence and also the quantity of mRNA for this gene. This information is evidence of the degree of transcription of the mutant gene. Total cellular RNA can be prepared from *Corynebacterium glutamicum* by several methods, all well-known in the art, such as that described in Bormann, E.R. et al. (1992) Mol. Microbiol. 6: 317-326.

To assess the presence or relative quantity of protein translated from this mRNA, standard techniques, such as SDS-acrylamide gel electrophoresis, were employed. The overproduction of *metC* and *metZ* in combination with *metA* in *Corynebacterium glutamicum* was demonstrated by this method. Western blot may also be employed (see, for example, Ausubel *et al.* (1988) Current Protocols in Molecular Biology, Wiley: New York). In this process, total cellular proteins are extracted, separated by gel electrophoresis, transferred to a matrix such as nitrocellulose, and incubated with a probe, such as an antibody, which specifically binds to the desired protein. This probe is generally tagged with a chemiluminescent or colorimetric label which may be readily detected. The presence and quantity of label observed indicates the presence and quantity of the desired mutant protein present in the cell.

### Example 7: Growth of Escherichia coli and Genetically Modified Corynebacterium glutamicum - Media and Culture Conditions

*E. coli* strains are routinely grown in MB and LB broth, respectively (Follettie, M. T., et al. (1993) J. Bacterial. 175, 4096-4103). Minimal media for *E*. *coli* is M9 and modified MCGC (Yoshihama, M., et al. (1985) J. Bacteriol. 162, 591-507). Glucose was added to a final concentration of 1%. Antibiotics were added in the following amounts (micrograms per milliliter): ampicillin, 50; kanamycin, 25; nalidixic acid, 25. Amino acids, vitamins, and other supplements were added in the following amounts: methionine, 9.3 mM; arginine, 9.3 mM; histidine, 9.3 mM; thiamine, 0.05 mM. *E*. *coli* cells were routinely grown at 37°C, respectively.

Genetically modified *Corynebacteria* are cultured in synthetic or natural growth media. A number of different growth media for Corynebacteria are both well-known and readily available (Lieb et al. (1989) Appl. Microbiol. Biotechnol., 32:205-210; von der Osten et al. (1998) Biotechnology Letters, 11:11-16; Patent DE 4,120,867; Liebl (1992) "The Genus *Corynebacterium,* in: The Procaryotes, Volume II, Balows, A. *et al.,* eds. Springer-Verlag). These media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, ribulose, lactose, maltose, sucrose, raffinose, starch or cellulose serve as very good carbon sources. It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NH₄)₂SO₄, NH₄OH, nitrates, urea, amino acids or complex nitrogen sources like corn steep liquor, soy bean flour, soy bean protein, yeast extract, meat extract and others.

The overproduction of sulfur containing amino acids like homocysteine and methionine was made possible using different sulfur sources. Sulfates, thiosulfates, sulfites and also more reduced sulfur sources like H₂S and sulfides and derivatives can be used. Also, organic sulfur sources like methyl mercaptan, thioglycolates, thiocyanates, thiourea, sulfur containing amino acids like cysteine and other sulfur containing compounds can be used to achieve homocysteine and methionine overproduction

Inorganic salt compounds which may be included in the media include the chloride-, phosphorous- or sulfate- salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include biotin, riboflavin, thiamin, folic acid, nicotinic acid, pantothenate and pyridoxin. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach (eds. P.M. Rhodes, P.F. Stanbury, IRL Press (1997) pp. 53-73, ISBN 0 19 963577 3). It is also possible to select growth media from commercial suppliers, like standard 1 (Merck) or BHI (grain heart infusion, DIFCO) or others.

All medium components are sterilized, either by heat (20 minutes at 1.5 bar and 121°C) or by sterile filtration. The components can either be sterilized together or, if necessary, separately. All media components can be present at the beginning of growth, or they can optionally be added continuously or batchwise.

Culture conditions are defined separately for each experiment. The temperature should be in a range between 15°C and 45°C. The temperature can be kept constant or can be altered during the experiment. The pH of the medium should be in the range of 5 to 8.5, preferably around 7.0, and can be maintained by the addition of buffers to the media. An exemplary buffer for this purpose is a potassium phosphate buffer. Synthetic buffers such as MOPS, HEPES, ACES and others can alternatively or simultaneously be used. It is also possible to maintain a constant culture pH through the addition of NaOH or NK₄OH during growth. If complex medium components such as yeast extract are utilized, the necessity for additional buffers may be reduced, due to the fact that many complex compounds have high buffer capacities. If a fermentor is utilized for culturing the micro-organisms, the pH can also be controlled using gaseous ammonia.

The incubation time is usually in a range from several hours to several days. This time is selected in order to permit the maximal amount of product to accumulate in the broth. The disclosed growth experiments can be carried out in a variety of vessels, such as microtiter plates, glass tubes, glass flasks or glass or metal fermentors of different sizes. For screening a large number of clones, the microorganisms should be cultured in microtiter plates, glass tubes or shake flasks, either with or without baffles. Preferably 100 ml shake flasks are used, filled with 10% (by volume) of the required growth medium. The flasks should be shaken on a rotary shaker (amplitude 25 mm) using a speed-range of 100 - 300 rpm. Evaporation losses can be diminished by the maintenance of a humid atmosphere; alternatively, a mathematical correction for evaporation losses should be performed.

If genetically modified clones are tested, an unmodified control clone or a control clone containing the basic plasmid without any insert should also be tested. The medium is inoculated to an OD₆₀₀ of 0.5 - 1.5 using cells grown on agar plates, such as CM plates (10 g/l glucose, 2,5 g/l NaCl, 2 g/l urea, 10 g/l polypeptone, 5 g/l yeast extract, 5 g/l meat extract, 22 g/l NaCl, 2 g/l urea, 10 g/l polypeptone, 5 g/l yeast extract, 5 g/l meat extract, 22 g/l agar, pH 6.8 with 2M NaOH) that had been incubated at 30°C. Inoculation of the media is accomplished by either introduction of a saline suspension of *C*. *glutamicum* cells from CM plates or addition of a liquid preculture of this bacterium.

### Example 8 - In vitro Analysis of the Function of Mutant Proteins

The determination of activities and kinetic parameters of enzymes is well established in the art. Experiments to determine the activity of any given altered enzyme must be tailored to the specific activity of the wild-type enzyme, which is well within the ability of one of ordinary skill in the art. Overviews about enzymes in general, as well as specific details concerning structure, kinetics, principles, methods, applications and examples for the determination of many enzyme activities may be found, for example, in the following references: Dixon, M., and Webb, E.C., (1979) Enzymes. Longmans: London; Fersht, (1985) Enzyme Structure and Mechanism. Freeman: New York; Walsh, (1979) Enzymatic Reaction Mechanisms. Freeman: San Francisco; Price, N.C., Stevens, L. (1982) Fundamentals of Enzymology. Oxford Univ. Press: Oxford; Boyer, P.D., ed. (1983) The Enzymes, 3^{rd} ed. Academic Press: New York; Bisswanger, H., (1994) Enzymkinetik, 2^{nd} ed. VCH: Weinheim (ISBN 3527300325); Bergmeyer, H.U., Bergmeyer, J., Graß1, M., eds. (1983-1986) Methods of Enzymatic Analysis, 3^{rd} ed., vol. I-XII, Verlag Chemie: Weinheim; and Ullmann's Encyclopedia of Industrial Chemistry (1987) vol. A9, "Enzymes". VCH: Weinheim, p. 352-363.

Cell extracts from *Corynebacterium glutamicum* were prepared as described previously (Park, S.-D., et al. (1998) Mol. Cells 8, 286-294). Cystathionine β-lyase was assayed as follows. The assay mixture contained 100 mM Tris-HCl (pH8.5), 0.1 mM NADH, 1 mM *L*-cystathionine, 5 units of *L*-lactate dehydrogenase, and appropriate amounts of crude extract. Optical changes were monitored at 340 nm. Assay for *S*-(□-aminoethyl)-cysteine (AEC) resistance was carried out as described in Rossol, I. and Pühler, A. (1992) J. Bacteriol. 174, 2968-77. The results of cystathionin β-lyase assays from extracts of different *Corynebacterium glutamicum* strains as well as results of AEC resistance assays of the same strain are summarized in Table 5, below.

**Table 5. Expression of cystathionine β-lyase^{a}**

| Strains | Properties | Activity (nmol min⁻¹ mg⁻¹) | Growth on MM^{b} | Resistance to AEC^{c} |
|---|---|---|---|---|
| *C. glutamicum* ASO19E12 | - | 146 | + | + |
| *C. glutamicum* ASO19E12/pMT1 | Empty vector | 145 | + | + |
| *C. glutamicum* ASO19E12/pSL173 | *metC* clone | 797 | + | ++ |
| *C. glutamicum* HL457 | *metC* mutant^{d} | 19 | + | - |
| *C. glutamicum* HL459 | *metC* mutant^{d} | 23 | + | - |
| *E. coli* JE6839 | *metC* mutant | 21 | - | *ND^{e}* |

| | | | | |
|---|---|---|---|---|
| ^{a} The enzyme was induced by growth to the stationary phase on the minimal medium containing 1% glucose. Cells were harvested, disrupted, and assayed for the activity as described in the Materials and Methods. ^{b} MCGC minimal media was used. Growth was monitored on plates. ^{c} Cells were grown on plates containing 40 mM *S*-(β-aminoethyl)-cysteine (AEC) for 5 days. ^{d} The mutants were generated in this study. ^{e} Not determined. | | | | |

The ability of the *metC* clones to express cystathionine β-lyase was tested by enzymatic assay. Crude extracts prepared from the *C*. *gtutamicum* ASO19E12 cells harboring plasmid pSL 173 were assayed. Cells harboring the plasmid showed approximately a 5-fold increase in the activity of cystathionine β-lyase compared to those harboring the empty vector pMT1 (Table 5), apparently due to the gene-dose effect. SDS-PAGE analysis of crude extracts revealed a putative cystathionine β-lyase band with approximate *M*ᵣ of 41,000. Intensity of each putative cystathionine β-lyase band agreed with the complementation and enzymatic assay data (Table 5). As described above, a region of *metC* appeared to be nearly identical to the previously reported *aecD.* Since the *aecD* gene was isolated on the basis of its ability to confer resistance to *S*-(β-aminoethyl)-cysteine (AEC), a toxic lysine analogue, we tested the protein product of *metC* for the presence of the activity. As shown in Table 5, cells overexpressing cystathionine β-lyase showed increased resistance to AEC. The strain carrying a mutation in *metC* gene (see below) completely lost its ability to show a resistant phenotype to AEC.

Assay for O-acetylhmoserine sulphydrylase was performed as follows (Belfaiza, J., et al. (1998) J. Bacteriol. 180, 250-255; Ravanel, S., M. Droux, and R. Douce (1995) Arch. Biochem. Biophys. 316, 572-584; Foglino, M. (1995) Microbiology 141, 431-439). Assay mixture of 0.1 ml contained 20 mM MOPS-NaOH (pH7.5), 10 mM O-acetylhomoserine, 2 mM Na₂S in 50 mM NaOH, and an appropriate amount of enzyme. Immediately after the addition of Na₂S which was added last, the reaction mixture was overlayed with 50 ul of mineral oil. After 30 minute incubation at 30°C, the reaction was stopped by boiling the mixture for 3 minutes. Homocysteine produced in the reaction was quantified as previously described (Yamagata, S. (1987) Method Enzymol. 143, 478-483.). Reaction mixture of 0.1 ml was taken and mixed with 0.1 ml of H₂O, 0.6 ml of saturated NaCl, 0.1 ml of 1.5 M Na₂CO₃ containing 67 mM KCN, and 0.1 ml of 2% nitroprusside. After 1 minute incubation at room temperature, optical density was measured at 520 nm. *Corynebacterium* cells harboring additional copies of the *metZ* gene, *e.g.,* a plasmid containing the *metZ* gene, exhibited significantly higher *metZ* enzyme activities than the same type of *Corynebacterium* cells without additional copies of the *metZ* gene.

The activity of proteins which bind to DNA can be measured by several well-established methods, such as DNA band-shift assays (also called gel retardation assays). The effect of such proteins on the expression of other molecules can be measured using reporter gene assays (such as that described in Kolmar, H. et al. (1995) EMBO J. 14: 3895-3904 and references cited therein). Reporter gene test systems are well known and established for applications in both pro- and eukaryotic cells, using enzymes such as beta-galactosidase, green fluorescent protein, and several others.

The determination of activity of membrane-transport proteins can be performed according to techniques such as those described in Gennis, R.B. (1989) "Pores, Channels and Transporters", in Biomembranes, Molecular Structure and Function, Springer: Heidelberg, p. 85-137; 199-234; and 270-322.

### Example 9: Analysis of Impact of Mutant Protein on the Production of the Desired Product

The effect of the genetic modification in *C. glutamicum* on production of a desired compound (such as an amino acid) can be assessed by growing the modified microorganism under suitable conditions (such as those described above) and analyzing the medium and/or the cellular component for increased production of the desired product (*i*.*e.,* an amino acid). Such analysis techniques are well known to one of ordinary skill in the art, and include spectroscopy, thin layer chromatography, staining methods of various kinds, enzymatic and microbiological methods, and analytical chromatography such as high performance liquid chromatography (see, for example, Ullman, Encyclopedia of Industrial Chemistry, vol. A2, p. 89-90 and p. 443-613, VCH: Weinheim (1985); Fallon, A*. et al*., (1987) "Applications of HPLC in Biochemistry" in: Laboratory Techniques in Biochemistry and Molecular Biology, vol. 17; Rehm *et al*. (1993) Biotechnology, vol. 3, Chapter III: "Product recovery and purification", page 469-714, VCH: Weinheim; Belter, P.A. *et al*. (1988) Bioseparations: downstream processing for biotechnology, John Wiley and Sons; Kennedy, J.F. and Cabral, J.M.S. (1992) Recovery processes for biological materials, John Wiley and Sons; Shaeiwitz, J.A. and Henry, J.D. (1988) Biochemical separations, in: Ulmann's Encyclopedia of Industrial Chemistry, vol. B3, Chapter 11, page 1-27, VCH: Weinheim; and Dechow, F.J. (1989) Separation and purification techniques in biotechnology, Noyes Publications.)

In addition to the measurement of the final product of fermentation, it is also possible to analyze other components of the metabolic pathways utilized for the production of the desired compound, such as intermediates and side-products, to determine the overall efficiency of production of the compound. Analysis methods include measurements of nutrient levels in the medium (*e.g*., sugars, hydrocarbons, nitrogen sources, phosphate, and other ions), measurements of biomass composition and growth, analysis of the production of common metabolites of biosynthetic pathways, and measurement of gasses produced during fermentation. Standard methods for these measurements are outlined in Applied Microbial Physiology, A Practical Approach, P.M. Rhodes and P.F. Stanbury, eds., IRL Press, p. 103-129; 131-163; and 165-192 (ISBN: 0199635773) and references cited therein.

### Example 10: Purification of the Desired Product from C. glutamicum Culture

Recovery of the desired product from the *C. glutamicum* cells or supernatant of the above-described culture can be performed by various methods well known in the art. If the desired product is not secreted from the cells, the cells can be harvested from the culture by low-speed centrifugation, the cells can be lysed by standard techniques, such as mechanical force or sonication. The cellular debris is removed by centrifugation, and the supernatant fraction containing the soluble proteins is retained for further purification of the desired compound. If the product is secreted from the *C. glutamicum* cells, then the cells are removed from the culture by low-speed centrifugation, and the supernate fraction is retained for further purification.

The supernatant fraction from either purification method is subjected to chromatography with a suitable resin, in which the desired molecule is either retained on a chromatography resin while many of the impurities in the sample are not, or where the impurities are retained by the resin while the sample is not. Such chromatography steps may be repeated as necessary, using the same or different chromatography resins. One of ordinary skill in the art would be well-versed in the selection of appropriate chromatography resins and in their most efficacious application for a particular molecule to be purified. The purified product may be concentrated by filtration or ultrafiltration, and stored at a temperature at which the stability of the product is maximized.

There are a wide array of purification methods known to the art and the preceding method of purification is not meant to be limiting. Such purification techniques are described, for example, in Bailey, J.E. & Ollis, D.F. Biochemical Engineering Fundamentals, McGraw-Hill: New York (1986).

The identity and purity of the isolated compounds may be assessed by techniques standard in the art. These include high-performance liquid chromatography (HPLC), spectroscopic methods, staining methods, thin layer chromatography, NIRS, enzymatic assay, or microbiologically. Such analysis methods are reviewed in: Patek et al. (1994) Appl. Environ. Microbiol. 60: 133-140; Malakhova et al. (1996) Biotekhnologiya 11: 27-32; and Schmidt et al. (1998) Bioprocess Engineer. 19: 67-70. Ulmann's Encyclopedia of Industrial Chemistry, (1996) vol. A27, VCH: Weinheim, p. 89-90, p. 521-540, p. 540-547, p. 559-566, 575-581 and p. 581-587; Michal, G. (1999) Biochemical Pathways: An Atlas of Biochemistry and Molecular Biology, John Wiley and Sons; Fallon, A. *et al.* (1987) Applications of HPLC in Biochemistry in: Laboratory Techniques in Biochemistry and Molecular Biology, vol. 17.

### Example 11: Analysis of the Gene Sequences of the Invention

The comparison of sequences and determination of percent homology between two sequences are art-known techniques, and can be accomplished using a mathematical algorithm, such as the algorithm of Karlin and Altschul (1990) *Proc. Natal. Acad. Sci.* USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to MP nucleic acid molecules of the invention. BLAST protein searches can be performed with the XBLAST program, score = 50, wordlength = 3 to obtain amino acid sequences homologous to MP protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, one of ordinary skill in the art will know how to optimize the parameters of the program (*e.g*., XBLAST and NBLAST) for the specific sequence being analyzed.

Another example of a mathematical algorithm utilized for the comparison of sequences is the algorithm of Meyers and Miller ((1988) Comput. Appl. Biosci. 4: 11-17). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art, and include ADVANCE and ADAM. described in Torelli and Robotti (1994) Comput. Appl. Biosci. 10:3-5; and FASTA, described in Pearson and Lipman (1988) P.N.A.S. 85:2444-8.

The percent homology between two amino acid sequences can also be accomplished using the GAP program in the GCG software package (available at http://www.gcg.com), using either a Blosum 62 matrix or a PAM250 matrix, and a gap weight of 12, 10, 8, 6, or 4 and a length weight of 2, 3, or 4. The percent homology between two nucleic acid sequences can be accomplished using the GAP program in the GCG software package, using standard parameters, such as a gap weight of 50 and a length weight of 3.

A comparative analysis of the gene sequences of the invention with those present in Genbank, has been performed using techniques known in the art (see, *e.g*., Bexevanis and Ouellette, eds. (1998) Bioinformatics: A Practical Guide to the Analysis of Genes and Proteins. John Wiley and Sons: New York). The gene sequences of the invention were compared to genes present in Genbank in a three-step process. In a first step, a BLASTN analysis (*e.g*., a local alignment analysis) was performed for each of the sequences of the invention against the nucleotide sequences present in Genbank, and the top 500 hits were retained for further analysis. A subsequent FASTA search (*e.g*., a combined local and global alignment analysis, in which limited regions of the sequences are aligned) was performed on these 500 hits. Each gene sequence of the invention was subsequently globally aligned to each of the top three FASTA hits, using the GAP program in the GCG software package (using standard parameters). In order to obtain correct results, the length of the sequences extracted from Genbank were adjusted to the length of the query sequences by methods well-known in the art. The results of this analysis are set forth in Table 4. The resulting data is identical to that which would have been obtained had a GAP (global) analysis alone been performed on each of the genes of the invention in comparison with each of the references in Genbank, but required significantly reduced computational time as compared to such a database-wide GAP (global) analysis. Sequences of the invention for which no alignments above the cutoff values were obtained are indicated on Table 4 by the absence of alignment information. It will further be understood by one of ordinary skill in the art that the GAP alignment homology percentages set forth in Table 4 under the heading "% homology (GAP)" are listed in the European numerical format, wherein a ',' represents a decimal point. For example, a value of "40,345" in this column represents "40.345%".

### Example 12: Construction and Operation of DNA Microarrays

The sequences of the invention may additionally be used in the construction and application of DNA microarrays (the design, methodology, and uses of DNA arrays are well known in the art, and are described, for example, in Schena, M. et al. (1995) Science 270: 467-470; Wodicka, L. et al. (1997) Nature Biotechnology 15: 1359-1367; DeSaizieu, A. et al. (1998) Nature Biotechnology 16: 45-48; and DeRisi, J.L. et al. (1997) Science 278: 680-686).

DNA microarrays are solid or flexible supports consisting of nitrocellulose, nylon, glass, silicone, or other materials. Nucleic acid molecules may be attached to the surface in an ordered manner. After appropriate labeling, other nucleic acids or nucleic acid mixtures can be hybridized to the immobilized nucleic acid molecules, and the label may be used to monitor and measure the individual signal intensities of the hybridized molecules at defined regions. This methodology allows the simultaneous quantification of the relative or absolute amount of all or selected nucleic acids in the applied nucleic acid sample or mixture. DNA microarrays, therefore, permit an analysis of the expression of multiple (as many as 6800 or more) nucleic acids in parallel (see, *e.g*., Schena, M. (1996) BioEssays 18(5): 427-431).

The sequences of the invention may be used to design oligonucleotide primers which are able to amplify defined regions of one or more *C. glutamicum* genes by a nucleic acid amplification reaction such as the polymerase chain reaction. The choice and design of the 5' or 3' oligonucleotide primers or of appropriate linkers allows the covalent attachment of the resulting PCR products to the surface of a support medium described above (and also described, for example, Schena, M. et al. (1995) Science 270: 467-470).

Nucleic acid microarrays may also be constructed by *in situ* oligonucleotide synthesis as described by Wodicka, L. et al. (1997) Nature Biotechnology 15: 1359-1367. By photolithographic methods, precisely defined regions of the matrix are exposed to light. Protective groups which are photolabile are thereby activated and undergo nucleotide addition, whereas regions that are masked from light do not undergo any modification. Subsequent cycles of protection and light activation permit the synthesis of different oligonucleotides at defined positions. Small, defined regions of the genes of the invention may be synthesized on microarrays by solid phase oligonucleotide synthesis.

The nucleic acid molecules of the invention present in a sample or mixture of nucleotides may be hybridized to the microarrays. These nucleic acid molecules can be labeled according to standard methods. In brief, nucleic acid molecules (*e.g*., mRNA molecules or DNA molecules) are labeled by the incorporation of isotopically or fluorescently labeled nucleotides, *e.g*., during reverse transcription or DNA synthesis. Hybridization of labeled nucleic acids to microarrays is described (*e.g*., in Schena, M. *et al.* (1995) *supra;* Wodicka, L. *et al.* (1997), *supra;* and DeSaizieu A. *et al.* (1998), *supra).* The detection and quantification of the hybridized molecule are tailored to the specific incorporated label. Radioactive labels can be detected, for example, as described in Schena, M. *et al.* (1995) *supra*) and fluorescent labels may be detected, for example, by the method of Shalon et al. (1996) Genome Research 6: 639-645).

The application of the sequences of the invention to DNA microarray technology, as described above, permits comparative analyses of different strains of *C. glutamicum* or other Corynebacteria. For example, studies of inter-strain variations based on individual transcript profiles and the identification of genes that are important for specific and/or desired strain properties such as pathogenicity, productivity and stress tolerance are facilitated by nucleic acid array methodologies. Also, comparisons of the profile of expression of genes of the invention during the course of a fermentation reaction are possible using nucleic acid array technology.

### Example 13: Analysis of the Dynamics of Cellular Protein Populations (Proteomics)

The genes, compositions, and methods of the invention may be applied to study the interactions and dynamics of populations of proteins, termed 'proteomics'. Protein populations of interest include, but are not limited to, the total protein population of *C. glutamicum* (*e.g.,* in comparison with the protein populations of other organisms), those proteins which are active under specific environmental or metabolic conditions (*e.g*., during fermentation, at high or low temperature, or at high or low pH), or those proteins which are active during specific phases of growth and development.

Protein populations can be analyzed by various well-known techniques, such as gel electrophoresis. Cellular proteins may be obtained, for example, by lysis or extraction, and may be separated from one another using a variety of electrophoretic techniques. Sodium dodecyl sulfate polyacrylamide gel electrophoresis (SDS-PAGE) separates proteins largely on the basis of their molecular weight. Isoelectric focusing polyacrylamide gel electrophoresis (IEF-PAGE) separates proteins by their isoelectric point (which reflects not only the amino acid sequence but also posttranslational modifications of the protein). Another, more preferred method of protein analysis is the consecutive combination of both IEF-PAGE and SDS-PAGE, known as 2-D-gel electrophoresis (described, for example, in Hermann et al. (1998) Electrophoresis 19: 3217-3221; Fountoulakis et al. (1998) Electrophoresis 19: 1193-1202; Langen et al. (1997) Electrophoresis 18: 1184-1192; Antelmann et al. (1997) Electrophoresis 18: 1451-1463). Other separation techniques may also be utilized for protein separation, such as capillary gel electrophoresis; such techniques are well known in the art.

Proteins separated by these methodologies can be visualized by standard techniques, such as by staining or labeling. Suitable stains are known in the art, and include Coomassie Brilliant Blue, silver stain, or fluorescent dyes such as Sypro Ruby (Molecular Probes). The inclusion of radioactively labeled amino acids or other protein precursors (*e.g*., ³⁵S-methionine, ³⁵S-cysteine, ¹⁴C-labelled amino acids, ¹⁵N-amino acids, ¹⁵NO₃ or ¹⁵NH₄⁺ or ¹³C-labelled amino acids) in the medium of *C. glutamicum* permits the labeling of proteins from these cells prior to their separation. Similarly, fluorescent labels may be employed. These labeled proteins can be extracted, isolated and separated according to the previously described techniques.

Proteins visualized by these techniques can be further analyzed by measuring the amount of dye or label used. The amount of a given protein can be determined quantitatively using, for example, optical methods and can be compared to the amount of other proteins in the same gel or in other gels. Comparisons of proteins on gels can be made, for example, by optical comparison, by spectroscopy, by image scanning and analysis of gels, or through the use of photographic films and screens. Such techniques are well-known in the art.

To determine the identity of any given protein, direct sequencing or other standard techniques may be employed. For example, N- and/or C-terminal amino acid sequencing (such as Edman degradation) may be used, as may mass spectrometry (in particular MALDI or ESI techniques (see, *e.g.,* Langen et al. (1997) Electrophoresis 18: 1184-1192)). The protein sequences provided herein can be used for the identification of *C. glutamicum* proteins by these techniques.

The information obtained by these methods can be used to compare patterns of protein presence, activity, or modification between different samples from various biological conditions (*e.g*., different organisms, time points of fermentation, media conditions, or different biotopes, among others). Data obtained from such experiments alone, or in combination with other techniques, can be used for various applications, such as to compare the behavior of various organisms in a given (*e.g*., metabolic) situation, to increase the productivity of strains which produce fine chemicals or to increase the efficiency of the production of fine chemicals.

### Example 14: Cloning of Genes by Application of the Polymerase Chain Reaction (PCR)

Genes can be amplified using specific oligonucleotides comprising either nucleotide sequences homologous to sequences of *Corynebacterium glutamicum* or other strains as well as recognition sites of restriction enzymes well known in the art (*e.g.,* as described in Sambrook, J., Fritsh, E. F., and Maniatis, T. *Molecular Cloning: A Laboratory Manual. 2nd, ed*., *Cold Spring Harbor Laboratory,* Cold Spring Harbor Laboratory Press, Cold Spring Harbor, NY, 1989). Theses oligonucleotides can be used to amplify specific DNA-fragments containing parts of the chromosome of mentioned strains using DNA-polymerases such as *T. aquaticus* DNA-polymerase, *P. furiosus* DNA-polymerase, or *P. woesei* DNA-polymerase and dNTPs nucleotides in an appropriate buffer solution as described by the manufacturer.

Gene fragments such as coding sequences from RXA00657 including appropriate upstream and downstream regions not contained in the coding region of the mentioned gene can be amplified using the aforementioned technologies. Furthermore, these fragments can be purified from unincorporated oligonucleotides and nucleotides. DNA restriction enzymes can be used to produce protruding ends that can be used to ligate DNA fragments to vectors digested with complementary enzymes or compatible enzymes producing ends that can be used to ligate the DNA into the vectors mentioned in Sinskey *et al*., U.S. Patent No. 4,649,119, and techniques for genetic manipulation of *C. glutamicum* and the related *Brevibacterium* species *(e.g., lactofermentum)* (Yoshihama et al, J. Bacteriol. 162: 591-597 (1985); Katsumata et al., J. Bacteriol. 159: 306-311 (1984); and Santamaria et al., J. Gen. Microbiol. 130: 2237-2246 (1984). Oligonucleotides used as primers for the amplification of upstream DNA sequence, the coding region sequence and the downstream region of RXA00657 were as follows:
TCGGGTATCCGCGCTACACTTAGA (SEQ ID NO:121);
GGAAACCGGGGCATCGAAACTTA (SEQ ID NO:122).

*Corynebacterium glutamicum* chromosomal DNA with an amount of 200ng was used as a template in a 100µl reaction volume containing 2,5U Pfu Turbo-Polymerase™ (Stratagene™), and 200µM dNTP-nucleotides The PCR was performed on a PCR-Cycler™ (Perkin Elmer 2400™) using the following temperature/time protocol:
1 cycle: 94 °C: 2 min.;
20 cycle: 94°C : 1 min.;
52°C: 1 min, 72°C: 1.5 min.,
1 cycle: 72 °C: 5 min.

Primers were removed from the resulting amplified DNA fragment and the resulting fragment was cloned into the blunt EcoRV site of pBS KS (Stratagene™). The fragment was excised by digestion with the restriction enzymes BamHI/XhoI and ligated into a BamHI Sall digested vector pB (SEQ ID NO.:125). The resulting vector is called pB RXA00657.

Resulting recombinant vectors can be analyzed using standard techniques described in *e.g*., Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989), and can be transferred into *C. glutamicum* using aforementioned techniques.

*A Corynebacterium* strain (ATCC 13286) was treated for a transformation as described. Transformation of *C*. *glutamicum* can be achieved by protoplast transformation (Kastsumata, R. *et al.* (1984) *J*. *Bacteriol*. 159306-311), electroporation (Liebl, E. et al. (1989) FEMS Microbiol. Letters, 53:399-303) and in cases where special vectors are used, also by conjugation (as described, *e.g.,* in Schäfer, A. et al. (1990) J. Bacteriol. 172:1663-1666). It is also possible to transfer the shuttle vectors for *C. glutamicum* to *E. coli* by preparing plasmid DNA from *C. glutamicum* (using standard methods well-known in the art) and transforming it into *E. coli.* This transformation step can be performed using standard methods, but it is advantageous to use an Mcr-deficient *E*. *coli* strain, such as NM522 (Gough & Murray (1983) J. Mol. Biol. 166:1-19)*.*

Transformation of a bacterial strain such as *Corynebacterium glutamicum* strain (ATCC 13286) was performed with a plasmid pB containing the aforementioned DNA regions of RXA00657 (SEQ ID NO.:6) and in another case with the vector pB (SEQ ID NO.:) carrying no additional insertion of nucleic acids.

The resulting strains were plated on and isolated from CM-Medium (10 g/l Glucose 2,5 g/l NaCl, 2,0 g/l Urea, 10 g/l Bacto Peptone (Difco/Becton Dicinson/Sparks USA™), 5 g/l yeast extract (Difco/Becton Dicinson/Sparks USA™), 5g/l meat extract (Difco/Becton Dicinson/Sparks USA™), 22g/l Agar (Difco/Becton Dickinson/Sparks USA™) and 15µg/ml kanamycin sulfate (Serva, Germany) with a adjusted with NaOH to pH of 6.8.

Strains isolated from the aforementioned agar medium were inoculated in 10 ml in a 100ml shake flask containing no baffles in liquid medium containing 100 g/l sucrose 50µg/l (NH4)₂SO₄, 2,5 g/l NaCl, 2,0 g/l Urea, 10 g/l Bacto Peptone (Difco/Becton Dickinson/Sparks USA), 5 g/l yeast extract (Difco/Becton Dickinson/Sparks USA), 5g/l meat extract (Difco/Becton Dickinson/Sparks USA), and 25g/l CaCO3 (Riedel de Haen, Germany). Medium was a adjusted with NaOH to pH of 6.8.

Strains were incubated at 30°C for 48h. Supernatants of incubations were prepared by centrifugation 20'at 12,000 rpm in an Eppendorf™ microcentrifuge. Liquid supernatants were diluted and subjected to amino acid analysis (Standard methods for these measurements are outlined in Applied Microbial Physiology, A Practical Approach, P.M. Rhodes and P.F. Stanbury, eds., IRL Press, p. 103-129; 131-163; and 165-192 (ISBN: 0199635773) and references cited therein).

The results are shown in Table 6, below.

**Results: Table 6:**

| **Strain ATCC 13286** | **Plasmid contained** | **pB** | **pB RXA00657** |
|---|---|---|---|
| | | | |
| **lysin produced** **(g/l)** | | 13.5 | 14.93 |
| **Selectivity** **(mol lysine/ mol consumed Saccharose)** | | 0.235 | 0.25 |

### Equivalents

Those of ordinary skill in the art will recognize, or will be able to ascertain using no more than routine experimentation, many equivalents to the specific embodiments of the invention described herein. Such equivalents are intended to be encompassed by the following claims.

**TABLE 1: Included Genes**

| **Lysine biosynthesis** | | | | | | |
|---|---|---|---|---|---|---|
| | | | | | | |
| Nucleic Acid SEQ ID NO | Amino Acid SEQ ID NO | Identification Code | Contig. | NT Start | NT Stop | Function |
| 5 | 6 | RXA00657 | | | | AMINOACID BIOSYNTHESIS REGULATOR |
| 7 | 8 | RXA02229 | GR00653 | 2793 | 3617 | DIAMINOPIMELATE EPIMERASE (EC 5.1.1.7) |
| 9 | 10 | RXS02970 | | | | ACETYLORNITHINE AMINOTRANSFERASE (EC 2.6.1.11) |
| 11 | 12 | F RXA01009 | GR00287 | 4714 | 5943 | ACETYLORNITHINE AMINOTRANSFERASE (EC 2.6.1.11) |
| 13 | 14 | RXC02390 | | | | MEMBRANE SPANNING PROTEIN INVOLVED IN LYSINE METABOLISM |
| 15 | 16 | RXC01796 | | | | MEMBRANE ASSOCIATED PROTEIN INVOLVED IN LYSINE METABOLISM |
| 17 | 18 | RXC01207 | | | | CYTOSOLIC PROTEIN INVOLVED IN METABOLISM OF LYSINE AND |
| 19 | 20 | RXC00657 | | | | THREONINE TRANSCRIPTIONAL REGULATOR INVOLVED IN LYSINE METABOLISM |
| 21 | 22 | RXC00552 | | | | CYTOSOLIC PROTEIN INVOLVED IN LYSINE METABOLISM |
| | | | | | | |

| **Lysine biosynthesis** | | | | | | |
|---|---|---|---|---|---|---|
| | | | | | | |
| Nucleic Acid SEQ ID NO | Amino Acid SEQ ID NO | Identification Code | Contig. | NT Start | NT Stop | Function |
| 23 | 24 | RXA00534 | GR00137 | 4758 | 3496 | ASPARTOKINASE ALPHA AND BETA SUBUNITS (EC 2.7.2.4) |
| 25 | 26 | RXA00533 | GR00137 | 3469 | 2438 | ASPARTATE-SEMIALDEHYDE DEHYDROGENASE (EC 1.2.1.11) |
| 27 | 28 | RXA02843 | GR00842 | 543 | 4 | 2,3,4,5-TETRAHYDROPYRIDINE-2-CARBOXYLATE N-SUCCINYLTRANSFERASE (EC 2.3.1.117) |
| 29 | 30 | RXA02022 | GR00613 | 2063 | 3169 | SUCCINYL-DIAMINOPIMELATE DESUCCINYLASE (EC 3.5.1.18) |
| 31 | 32 | RXA00044 | GR00007 | 3458 | 4393 | DIHYDRODIPICOLINATE SYNTHASE (EC 4.2.1.52) |
| 33 | 34 | RXA00863 | GR00236 | 896 | 1639 | DIHYDRODIPICOLINATE REDUCTASE (EC 1.3.1.26) |
| 35 | 36 | RXA00864 | GR00236 | 1694 | 2443 | probable 2,3-dihydrodipicolinate N-C6-lyase (cyclizing) (EC 4.3.3.-) - Corynebacterium glutamicum |
| 37 | 38 | RXA02843 | GR00842 | 543 | 4 | 2,3,4,5-TETRAHYDROPYRIDINE-2-CARBOXYLATE N-SUCCINYLTRANSFERASE (EC 2.3.1.117) |
| 39 | 40 | RXN00355 | VV0135 | 31980 | 30961 | MESO-DIAMINOPIMELATE D-DEHYDROGENASE |
| 41 | 42 | F RXA00352 | GR00068 | 861 | 4 | MESO-DIAMINOPIMELATE D-DEHYDROGENASE (EC 1.4.1.16) |
| | | | | | | |
| 43 | 44 | RXA00972 | GR00274 | 3 | 1379 | DIAMINOPIMELATE DECARBOXYLASE (EC 4.1.1.20) |
| 45 | 46 | RXA02653 | GR00752 | 5237 | 7234 | DIAMINOPIMELATE DECARBOXYLASE (EC 4.1.1.20) |
| 47 | 48 | RXA01393 | GR00408 | 4249 | 3380 | LYSINE EXPORT REGULATOR PROTEIN |
| 49 | 50 | RXA00241 | GR00036 | 5443 | 6945 | L-LYSINE TRANSPORT PROTEIN |
| 51 | 52 | RXA01394 | GR00408 | 4320 | 5018 | LYSINE EXPORTER PROTEIN |
| 53 | 54 | RXA00865 | GR00236 | 2647 | 3549 | DIHYDRODIPICOLINATE SYNTHASE (EC 4.2.1.52) |
| 55 | 56 | RXS02021 | | | | 2,3,4,5-TETRAHYDROPYRIDINE-2-CARBOXYLATE N-SUCCINYLTRANSFERASE (EC 2.3.1.117) |
| 57 | 58 | RXS02157 | | | | ACETYLORNITHINE AMINOTRANSFERASE (EC 2.6.1.11) |
| 59 | 60 | RXC00733 | | | | ABC TRANSPORTER ATP-BINDING PROTEIN INVOLVED IN LYSINE |
| | | | | | | METABOLISM |
| 61 | 62 | RXC00861 | | | | PROTEIN INVOLVED IN LYSINE METABOLISM |
| 63 | 64 | RXC00866 | | | | ZN-DEPENDENT HYDROLASE INVOLVED IN LYSINE METABOLISM |
| 65 | 66 | RXC02095 | | | | ABC TRANSPORTER ATP-BINDING PROTEIN INVOLVED IN LYSINE METABOLISM |
| 67 | 68 | RXC03185 | | | | PROTEIN INVOLVED IN LYSINE METABOLISM |
| | | | | | | |

| **Metabolism of methionine and S-adenosyl methionine** | | | | | | |
|---|---|---|---|---|---|---|
| | | | | | | |
| Nucleic Acid SEQ ID NO | Amino Acid SEQ ID NO | Identification Code | Contig. | NT Start | NT Stop | Function |
| 1 | 2 | **metZ or met** | | | | O-ACETYLHOMOSERINE SULFHYDRYLASE (EC 4.2.99.10) |
| 3 | 4 | **metC** | | | | Cystathionine-γ-lyase |
| 69 | 70 | RXA00115 | GR00017 | 5359 | 4313 | HOMOSERINE O-ACETYLTRANSFERASE (EC 2.3.1.31) |
| 71 | 72 | RXN00403 | W0086 | 70041 | 68911 | HOMOSERINE O-ACETYLTRANSFERASE |
| 73 | 74 | F RXA00403 | GR00088 | 723 | 1832 | HOMOSERINE O-ACETYLTRANSFERASE (EC 2.3.1.11) |
| 75 | 76 | RXS03158 | | | | CYSTATHIONINE GAMMA-SYNTHASE (EC 4.2.99.9) |
| 77 | 78 | F RXA00254 | GR00038 | 2404 | 1811 | CYSTATHIONINE GAMMA-SYNTHASE (EC 4.2.99.9) |
| 79 | 80 | RXA02532 | GR00726 | 3085 | 2039 | CYSTATHIONINE GAMMA-SYNTHASE (EC 4.2.99.9) |
| 81 | 82 | RXS03159 | | | | CYSTATHIONINE GAMMA-SYNTHASE (EC 4.2.99.9) |
| 83 | 84 | F RXA02768 | GR00770 | 1919 | 2521 | CYSTATHIONINE GAMMA-SYNTHASE (EC 4.2.99.9) |
| 85 | 86 | RXA00216 | GR00032 | 16286 | 15297 | 5-methyltetrahydrofolate-homocysteine methyltransferase (methionine synthetase) |
| 87 | 94 | RXA02197 | GR00645 | 4552 | 4025 | 5-METHYLTETRAHYDROFOLATE--HOMOCYSTEINE METHYLTRANSFERASE (EC 2.1.1.13) |
| 89 | 90 | RXN02198 | W0302 | 9228 | 11726 | 5-METHYLTETRAHYDROFOI.ATE-HOMOCYSTEINE METHYLTRANSFERASE (EC 2.1.1.13) |
| 91 | 91 | F RXA02198 | GR00646 | 2483 | 6 | 5-METHYLTETRAHYDROFOLATE-HOMOCYSTEINE METHYLTRANSFERASE (EC 2.1.1.13) |
| 93 | 94 | RXN03074 | W0042 | 2238 | 1741 | S-ADENOSYLMETHIONINE:2-DEMETHYLMENAQUINONE METHYLTRANSFERASE (EC 2.1.-.-) |
| 95 | 96 | F RXA02906 | GR10044 | 1142 | 645 | S-ADENOSYLMETHIONINE:2-DEMETHYLMENAQUINONE METHYLTRANSFERASE (EC 2.1.-.-) |
| 97 | 98 | RXN00132 | VV0124 | 3612 | 5045 | ADENOSYLHOMOCYSTEINASE (EC 3.3.1.1) |
| 99 | 100 | F RXA00132 | GR00020 | 7728 | 7624 | ADENOSYLHOMOCYSTEINASE (EC 3.3.1.1) |
| | | | | | | |
| 101 | 102 | F RXA01371 | GR00398 | 2339 | 3634 | ADENOSYLHOMOCYSTEINASE (EC 3.3.1.1) |
| 103 | 104 | RXN02085 | | | | 5-METHYLTETRAHYDROPTEROYLTRIGLUTAMATE-HOMOCYSTEINE METHYLTRANSFERASE (EC 2.1.1.14) |
| 105 | 106 | F RXA02085 | GR00629 | 3496 | 5295 | 5-METHYLTETRAHYDROPTEROYLTRIGLUTAMATE--HOMOCYSTEINE METHYLTRANSFERASE (EC 2.1.1.14) |
| 107 | 108 | F RXA02086 | GR00629 | 5252 | 5731 | 5-METHYLTETRAHYDROPTEROYLTRIGLUTAMATE--HOMOCYSTEINE METHYLTRANSFERASE (EC 2.1.1.14) |
| 109 | 110 | RXN02648 | | | | 5-METHYLTETRAHYDROPTEROYLTRIGLUTAMATE-HOMOCYSTEINE METHYLTRANSFERASE (EC 2.1.1.14) |
| 111 | 112 | F RXA02648 | GR00751 | 5254 | 4730 | 5-METHYLTETRAHYDROPTEROYLTRIGLUTAMATE--HOMOCYSTEINE METHYLTRANSFERASE (EC 2.1.1.14) |
| 113 | 114 | F RXA02658 | GR00752 | 14764 | 15447 | 5-METHYLTETRAHYDROPTEROYLTRIGLUTAMATE-HOMOCYSTEINE METHYLTRANSFERASE (EC 2.1.1.14) |
| 115 | 116 | RXC02238 | | | | PROTEIN INVOLVED IN METABOLISM OF S-ADENOSYLMETHIONINE, PURINES AND PANTOTHENATE |
| 117 | 118 | RXC00128 | | | | EXPORTED PROTEIN INVOLVED IN METABOLISM OF PYRIDIMES AND ADENOSYLHOMOCYSTEINE |
| | | | | | | |

| **S-2adenosyl methionine (SAM) Biosynthesis** | | | | | | |
|---|---|---|---|---|---|---|
| | | | | | | |
| Nucleic Acid SEQ ID NO | Amino Acid SEQ ID NO | Identification Code | Contig. | NT Start | NT Stop | Function |
| 119 | 120 | RXA02240 | GR00654 | 7160 | 8380 | S-ADENOSYLMETHIONINE SYNTHETASE (EC 2.5.1.6) |

**TABLE 2: GENES IDENTIFIES FROM GENBANK**

| **GenBank™ Accession No.** | **Gene Name** | **Gene Function** | **Reference** |
|---|---|---|---|
| A09073 | ppg | Phosphoenol pyruvate carboxylase | Bachmann, B. et al. "DNA fragment coding for phosphoenolpyruvat corboxylase, recombinant DNA carrying said fragment, strains carrying the recombinant DNA and method for producing L-aminino acids using said strains," Patent: EP 0358940-A 3 03/21/90 |
| A45579, A45581, A45583, A45585 A45587 | | Threonine dehydratase | Moeckel, B. et al. "Production of L-isoleucine by means of recombinant micro-organisms with deregulated threonine dehydratase," Patent: WO 9519442-A 5 07/20/95 |
| AB003132 | murC; ftsQ; ftsZ | | Kobayashi, M. et al. "Cloning, sequencing, and characterization of the ftsZ gene from coryneform bacteria," Biochem. Biophys. Res. Commun., 236(2):383-388 (1997) |
| AB015023 | murC; ftsQ | | Wachi, M. et al. "A murC gene from Coryneform bacteria," Appl. Microbiol. Biotechnol., 51(2):223-228 (1999) |
| AB018530 | dtsR | | Kimura, E. et al. "Molecular cloning of a novel gene, dtsR, which rescues the detergent sensitivity of a mutant derived from Brevibacterium lactofermentum," Biosci. Biotechnol. Biochem., 60(10):1565-1570 (1996) |
| AB018531 | dtsR1; dtsR2 | | |
| AB020624 | murI | D-glutamate racemase | |
| AB023377 | tkt | transketolase | |
| AB024708 | gltB; gltD | Glutamine 2-oxoglutarate aminotransferase large and small subunits | |
| AB025424 | acn | aconitase | |
| AB027714 | rep | Replication protein | |
| AB027715 | rep; aad | Replication protein; aminoglycoside adenyltransferase | |
| AF005242 | argC | N-acetylglutaniate-5-semialdehyde dehydrogenase | |
| AF005635 | glnA | Glutamine synthetase | |
| AF030405 | hisF | cyclase | |
| AF030520 | argG | Argininosuccinate synthetase | |
| AF031518 | argF | Ornithine carbamolytransferase | |
| AF036932 | aroD | 3-dehydroquinate dehydratase | |
| AF038548 | pyc | Pyruvate carboxylase | |
| AF038651 | dciAE; apt; rel | Dipeptide-binding protein; adenine phosphoribosyltransferase; GTP pyrophosphokinase | Wehmeier, L. et al. "The role of the Corynebacterium glutamicum rel gene in (p)ppGpp metabolism," Microbiology, 144:1853-1862 (1998) |
| AF041436 | argR | Arginine repressor | |
| AF045998 | impA | Inositol monophosphate phosphatase | |
| AF048764 | argH | Argininosuccinate lyase | |
| AF049897 | argC; argJ; argB; argD; argF; argR; argG; argH | N-acetylglutamylphosphate reductase; ornithine acetyltransferase; N-acetylglutamate kinase; acetylornithine transminase; ornithine carbamoyltransferase; arginine repressor; argininosuccinate synthase; argininosuccinate lyase | |
| AF050109 | inhA | Enoyl-acyl carrier protein reductase | |
| AF050166 | hisG | ATP phosphoribosyltransferase | |
| AF051846 | hisA | Phosphoribosylformimino-5-amino-1-phosphoribosyl-4-iminidazolecarboxamide isomerase | |
| AF052652 | metA | Homoserine O-acetyltransferase | Park, S. et al. "Isolation and analysis of metA, a methionine biosynthetic gene encoding homoserine acetyltransferase in Corynebacterium glutamicum," Mol. Cells., 8(3):286-294 (1998) |
| AF053071 | aroB | Dehydroquinate synthetase | |
| AF060558 | hisH | Glutamine amidotransferase | |
| AF086704 | hisE | Phosphoribosyl-ATP-pyrophosphohydrolase | |
| AF114233 | aroA | 5-enolpyruvylshikimate 3-phosphate synthase | |
| AF116184 | panD | L-aspartate-alpha-decarboxylase precursor | Dusch, N. et al. "Expression of the Corynebacterium glutamicum panD gene encoding L-aspartate-alpha-decarboxylase leads to pantothenate overproduction in Escherichia coli," Appl. Environ. Microbiol., 65(4)1530-1539 (1999) |
| AF124518 | aroD; aroE | 3-dehydroquinase; shikimate dehydrogenase | |
| AF124600 | aroC; aroK; aroB; pepQ | Chorismate synthase; shikimate kinase; 3-dehydroquinate synthase; putative cytoplasmic peptidase | |
| AF145897 | inhA | | |
| AF145898 | inhA | | |
| AJ001436 | ectP | Transport of ectoine, glycine betaine, proline | Peter, H. et al. "Corynebacterium glutamicum is equipped with four secondary carriers for compatible solutes: Identification, sequencing, and characterization of the proline/ectoine uptake system, ProP, and the ectoine/proline/glycine betaine carrier, EctP," J. Bacteriol., 180(22):6005-6012 (1998) |
| AJ004934 | dapD | Tetrahydrodipicolinate succinylase (incompleteⁱ) | Wehrmann, A. et al. "Different modes of diaminopimelate synthesis and their role in cell wall integrity: A study with Corynebacterium glutamicum," J. Bacteriol., 180(12):3159-3165 (1998) |
| AJ007732 | ppc; secG; amt; ocd; soxA | Phosphoenolpyruvate-carboxylase; ?; high affinity ammonium uptake protein; putative ornithine-cyclodecarboxylase; sarcosine oxidase | |
| AJ010319 | ftsY, glnB, glnD; srp; amtP | Involved in cell division; PH protein; uridylyltransferase (uridylyl-removing enzmye); signal recognition particle; low affinity ammonium uptake protein | Jakoby, M. et al. "Nitrogen regulation in Corynebacterium glutamicum; Isolation of genes involved in biochemical characterization of corresponding proteins," FEMS Microbiol., 173(2):303-310 (1999) |
| AJ132968 | cat | Chloramphenicol aceteyl transferase | |
| AJ224946 | mqo | L-malate: quinone oxidoreductase | Molenaar, D. et al. "Biochemical and genetic characterization of the membrane-associated malate dehydrogenase (acceptor) from Corynebacterium glutamicum," Eur. J. Biochem., 254(2):395-403 (1998) |
| AJ238250 | ndh | NADH dehydrogenase | |
| AJ238703 | porA | Porin | Lichtinger, T. et al. "Biochemical and biophysical characterization of the cell wall porin of Corynebacterium glutamicum: The channel is formed by a low molecular mass polypeptide," Biochemistry, 37(43):15024-15032 (1998) |
| D17429 | | Transposable element IS31831 | Vertes et al."Isolation and characterization of IS31831, a transposable element from Corynebacterium glutamicum," Mol. Microbiol., 11(4):739-746 (1994) |
| D84102 | odhA | 2-oxoglutarate dehydrogenase | Usuda, Y. et al. "Molecular cloning of the Corynebacterium glutamicum (Brevibacterium lactofermentum AJ12036) odhA gene encoding a novel type of 2-oxoglutarate dehydrogenase," Microbiology, 142:3347-3354 (1996) |
| E01358 | hdh; hk | Homoserine dehydrogenase; homoserine kinase | Katsumata, R. et al. "Production of L-thereonine and L-isoleucine," Patent: JP 1987232392-A 1 10/12/87 |
| E01359 | | Upstream of the start codon of homoserine kinase gene | Katsumata, R. et al. "Production of L-thereonine and L-isoleucine," Patent: JP 1987232392-A 210/12/87 |
| E01375 | | Tryptophan operon | |
| E01376 | trpL; trpE | Leader peptide; anthranilate synthase | Matsui, K. et al. "Tryptophan operon, peptide and protein coded thereby, utilization of tryptophan operon gene expression and production of tryptophan," Patent: JP 1987244382-A 1 10/24/87 |
| E01377 | | Promoter and operator regions of tryptophan operon | Matsui, K. et al. "Tryptophan operon, peptide and protein coded thereby, utilization of tryptophan operon gene expression and production of tryptophan," Patent: JP 1987244382-A 1 10/24/87 |
| E03937 | | Biotin-synthase | Hatakeyama, K. et al. "DNA fragment containing gene capable of coding biotin synthetase and its utilization," Patent: JP 1992278088-A 1 10/02/92 |
| E04040 | | Diamino pelargonic acid aminotransferase | Kohama, K. et al. "Gene coding diaminopelargonic acid aminotransferase and desthiobiotin synthetase and its utilization," Patent: JP 1992330284-A 1 11/18/92 |
| E04041 | | Desthiobiotinsynthetase | Kohama, K. et al. "Gene coding diaminopelargonic acid aminotransferase and desthiobiotin synthetase and its utilization," Patent: JP 1992330284-A 1 11/18/92 |
| E04307 | | Flavum aspartase | Kurusu, Y. et al. "Gene DNA coding aspartase and utilization thereof," Patent: JP 1993030977-A 1 02/09/93 |
| E04376 | | Isocitric acid lyase | Katsumata, R. et al. "Gene manifestation controlling DNA," Patent: JP 1993056782-A 3 03109193 |
| E04377 | | Isocitric acid lyase N-terminal fragment | Katsumata, R. et al. "Gene manifestation controlling DNA," Patent: JP 1993056782-A 3 03/09/93 |
| E04484 | | Prephenate dehydratase | Sotouchi, N. et al. "Production of L-phenylalanine by fermentation," Patent: JP 1993076352-A 2 03/30/93 |
| E05108 | | Aspartokinase | Fugono, N. et al: "Gene DNA coding Aspartokinase and its use," Patent: JP 1993184366-A 1.07/27/93 |
| E05112 | | Dihydro-dipichorinate synthetase | Hatakeyama, K. et al. "Gene DNA coding dihydrodipicolinic acid synthetase and its use," Patent: JP a 107/27/93 |
| E05776 | | Diaminopimelic acid dehydrogenase | Kobayashi, M. et al. "Gene DNA coding Diaminopimelic acid dehydrogenase and its use," Patent: JP 1993284970-A 1 11/02/93 |
| E05779 | | Threonine synthase | Kohama, K. et al. "Gene DNA coding threonine synthase and its use," Patent: JP 1993284972-A 1 11/02/93 |
| E06110 | | Prephenate dehydratase | Kikuchi, T. et al. "Production of L-phenylalanine by fermentation method," Patent: JP 1993344881-A 1 12/27/93 |
| E06111 | | Mutated Prephenate dehydratase | Kikuchi, T. et al. "Production of L-phenylalanine by fermentation method," Patent: JP 1993344881-A 1 12/27/93 |
| E06146 | | Acetohydroxy acid synthetase | Inui, M. et al. "Gene capable of coding Acetohydroxy acid synthetase and its use," Patent: JP 1993344893-A 1 12/27/93 |
| E06825 | | Aspartokinase | Sugimoto, M. et al. "Mutant aspartokinase gene," patent: JP 1994062866-A 1 03/08/94 |
| E06826 | | Mutated aspartokinase alpha subunit | Sugimoto, M. et al. "Mutant aspartokinase gene," patent: JP 1994062866-A 1 03/08/94 |
| E06827 | | Mutated aspartokinase alpha subunit | Sugimoto, M. et al. "Mutant aspartokinase gene," patent: JP 1994062866-A 1 03/08/94 |
| E07701 | secY | | Honno, N. et al. "Gene DNA participating in integration of membraneous protein to membrane," Patent: JP 1994169780-A 106/21/94 |
| E08177 | | Aspartokinase | Sato, Y. et al. "Genetic DNA capable of coding Aspartokinase released from feedback inhibition and its utilization," Patent: JP 1994261766-A 109/20/94 |
| E08178, E08179, E08180, E08181, E08182 | | Feedback inhibition-released Aspartokinase | Sato, Y. et al. "Genetic DNA capable of coding Aspartokinase released from feedback inhibition and its utilization," Patent: JP 1994261766-A 1 09/20/94 |
| E08232 | | Acetohydroxy-acid isomeroreductase | Inui, M. et al. "Gene DNA coding acetohydroxy acid isomeroreductase," Patent: JP 1994277067-A 1 10/04/94 |
| E08234 | secE | | Asai, Y. et al. "Gene DNA coding for translocation machinery of protein," Patent: JP 1994277073-A 1 10/04/94 |
| E08643 | | FT aminotransferase and desthiobiotin synthetase promoter region | Hatakeyama, K. et al. "DNA fragment having promoter function in coryneform bacterium," Patent: JP 1995031476-A 1 02/03/95 |
| E08646 | | Biotin synthetase, | Hatakeyama, K. et al. "DNA fragment having promoter function in coryneform bacterium," Patent: JP 1995031476-A 1 02/03/95 |
| E08649 | | Aspartase | Kohama, K. et al "DNA fragment having promoter function in coryneform bacterium," Patent: JP 1995031478-A 1 02/03/95 |
| E08900 | | Dihydrodipicolinate reductase | Madori, M. et al. "DNA fragment containing gene coding Dihydrodipicolinate acid reductase and utilization thereof," Patent: JP 1995075578-A 1 03120/95 |
| E08901 | | Diaminopimelic acid decarboxylase | Madori, M. et al. "DNA fragment containing gene coding Diaminopimelic acid decarboxylase and utilization thereof," Patent: JP 1995075579-A 1 03/20/95 |
| E12594 | | Serine hydroxymethyltransferase | Hatakeyama, K. et al. "Production of L-trypophan," Patent: JP 1997028391-A 1 02/04/97 |
| E12760, E12759, E12758 | | transposase | Moriya, M. et al. "Amplification of gene using artificial transposon," Patent: JP 1997070291-A 03/18/97 |
| E12764 | | Arginyl-tRNA synthetase; diaminopimelic acid decarboxylase | Moriya, M. et al. "Amplification of gene using artificial transposon," Patent: JP 1997070291-A 03/18/97 |
| E12767 | | Dihydrodipicolinic acid synthetase | Moriya, M. et al. "Amplification of gene using artificial transposon," Patent: JP 1997070291-A 03/18/97 |
| E12770 | | aspartokinase | Moriya, M. et al. "Amplification of gene using artificial transposon," Patent: JP 1997070291-A 03/18/97 |
| E12773 | | Dihydrodipicolinic acid reductase | Moriya, M. et al. "Amplification of gene using artificial transposon," Patent: JP 1997070291-A 03/18/97 |
| E13655 | | Glucose-6-phosphate dehydrogenase | Hatakeyama, K. et al. "Glucose-6-phosphate dehydrogenase and DNA capable of coding the same," Patent: JP 1997224661-A 1 09/02/97 |
| L01508 | IlvA | Threonine dehydratase | Moeckel, B. et al. "Functional and structural analysis of the threonine dehydratase of Corynebacterium glutamicum," J. Bacteriol., 174:8065-8072 (1992) |
| L07603 | EC 4.2.1.15 | 3-deoxy-D-arabinoheptulosonate-7-phosphate synthase | Chen, C. et al. "The cloning and nucleotide sequence of Corynebacterium glutamicum 3-deoxy-D-arabinoheptulosonate-7-phosphate synthase gene," FEMS Microbiol. Lett., 107:223-230 (1993) |
| L09232 | IlvB; ilvN; ilvC | Acetohydroxy acid synthase large subunit; Acetohydroxy acid synthase small subunit; Acetohydroxy acid isomeroreductase | Keilhauer, C. et al. "Isoleucine synthesis in Corynebacterium glutamicum: molecular analysis of the ilvB-ilvN-ilvC operon," J. Bacteriol., 175(17):5595-5603 (1993) |
| L18874 | PtsM | Phosphoenolpyruvate sugar phosphotransferase | Fouet, A et al. "Bacillus subtilis sucrose-specific enzyme II of the phosphotransferase system: expression in Escherichia coli and homology to enzymes II from enteric bacteria," PNAS USA, 84(24):8773-8777 (1987); Lee, J.K. et al. "Nucleotide sequence of the gene encoding the Corynebacterium glutamicum mannose enzyme II and analyses of the deduced protein sequence, "FEMS Microbiol. Lett., 119(1-2):137-145 (1994) |
| L27123 | aceB | Malate synthase | Lee, H-S. et al. "Molecular characterization of aceB, a gene encoding malate synthase in Corynebacterium glutamicum," J. Microbiol. Biotechnol., 4(4):256-263 (1994) |
| L27126 | | Pyruvate kinase | Jetten, M. S. et al. "Structural and functional analysis of pyruvate kinase from Corynebacterium glutamicum," Appl. Environ. Microbiol., 60(7):2501-2507 (1994) |
| L28760 | aceA | Isocitrate lyase | |
| L35906 | dtxr | Diphtheria toxin repressor | Oguiza, J.A. et al. "Molecular cloning, DNA sequence analysis, and characterization of the Corynebacterium diphtheriae dtxR from Brevibacterium lactofermentum," J. Bacteriol., 177(2):465-467 (1995) |
| M13774 | | Prephenate dehydratase | Follettie, M.T. et al. "Molecular cloning and nucleotide sequence of the Corynebacterium glutamicum pheA gene," J. Bacteriol., 167:695-702 (1986) |
| M16175 | 5SrRNA | | Park, Y-H. et al. "Phylogenetic analysis of the coryneform bacteria by 56 rRNA sequences," J. Bacteriol., 169:1801-1806 (1987) |
| M16663 | trpE | Anthranilate synthase, 5' end | Sano, K. et al. "Structure and function of the trp operon control regions of Brevibacterium lactofermentum, a glutamic-acid-producing bacterium," Gene, 52:191-200(1987) |
| M16664 | trpA | Tryptophan synthase, 3'end | Sano, K. et al. "Structure and function of the trp operon control regions of Brevibacterium lactofermentum, a glutamic-acid-producing bacterium," Gene, 52:191-200(1987) |
| M25819 | | Phosphoenolpyruvate carboxylase | O'Regan, M. et al. "Cloning and nucleotide sequence of the Phosphoenolpyruvate carboxylase-coding gene of Corynebacterium glutamicum ATCC13032," Gene, 77(2):237-251 (1989) |
| M85106 | | 23S rRNA gene insertion sequence | Roller, C. et al. "Gram-positive bacteria with a high DNA G+C content are characterized by a common insertion within their 23S rRNA genes," J. Gen. Microbiol., 138:1167-1175 (1992) |
| M85107, M85108 | | 23S rRNA gene insertion sequence | Roller, C. et al. "Gram-positive bacteria with a high DNA G+C content are characterized by a common insertion within their 23S rRNA genes," J. Gen. Microbiol., 138:1167-1175 (1992) |
| M89931 | aecD; brnQ; yhbw | Beta C-S lyase; branched-chain amino acid uptake carrier; hypothetical protein yhbw | Rossol, I. et al. "The Corynebacterium glutamicum aecD gene encodes a C-S lyase with alpha, beta-elimination activity that degrades aminoethylcysteine," J. Bacteriol., 174(9):2968-2977 (1992); Tauch, A. et al. "Isoleucine uptake in Corynebacterium glutamicum ATCC 13032 is directed by the bio gene product," Arch. Microbiol., 169(4):303-312 (1998) |
| S59299 | trp | Leader gene (promoter) | Herry, D.M. et al. "Cloning of the trp gene cluster from a tryptophan-hyperproducing strain of Corynebacterium glutamicum: identification of a mutation in the trp leader sequence," Appl. Environ. Microbiol., 59(3):791-799 (1993) |
| U11545 | trpD | Anthranilate phosphoribosyltransferase | O'Gara, J.P. and Dunican, L.K. (1994) Complete nucleotide sequence of the Corynebacterium glutamicum ATCC 21850 tpD gene." Thesis, Microbiology Department, University College Galway, Ireland. |
| U13922 | cglIM; cglIR; clgIIR | Putative type II 5-cytosoine methyltransferase; putative type II restriction endonuclease; putative type I or type III restriction endonuclease | Schafer, A. et al. "Cloning and characterization of a DNA region encoding a stress-sensitive restriction system from Corynebacterium glutamicum ATCC 13032 and analysis of its role in intergeneric conjugation with Escherichia coli," J. Bacteriol., 176(23):7309-7319 (1994); Schafer, A. et al. "The Corynebacterium;glutamicum cglIM gene encoding a 5-cytosine in an McrBC-deficient Escherichia coli strain," Gene, 203(2):95-101 (1997) |
| U14965 | recA | | |
| U31224 | ppx | | Ankri, S. et al. "Mutations in the Corynebacterium glutamicumproline biosynthetic pathway: A natural bypass of the proA step," J. Bacteriol., 178(15):4412-4419 (1996) |
| U31225 | proC | L-proline: NADP+ 5-oxidoreductase | Ankri, S. et al. "Mutations in the Corynebacterium glutamicumproline biosynthetic pathway: A natural bypass of the proA step," J. Bacteriol., 178(15):4412-4419 (1996) |
| U31230 | obg; proB; unkdh | ?;gamma glutamyl kinase;similar to D-isomer specific 2-hydroxyacid dehydrogenases | Ankri, S. et al. "Mutations in the Corynebacterium glutamicumproline biosynthetic pathway: A natural bypass of the proA step," J. Bacteriol., 178(15):4412-4419 (1996) |
| U31281 | bioB | Biotin synthase | Serebriiskii, I.G., "Two new members of the bio B superfamily: Cloning, sequencing and expression of bio B genes of Methylobacillus flagellatum and Corynebacterium glutamicum," Gene, 175:15-22 (1996) |
| U35023 | thtR; accBC | Thiosulfate sulfurtransferase; acyl CoA carboxylase | Jager, W. et al. "A Corynebacterium glutamicum gene encoding a two-domain protein similar to biotin carboxylases and biotin-carboxyl-carrier proteins," *Arch. Microbial.,* 166(2);76-82 (1996) |
| U43535 | cmr | Multidrug resistance protein | Jager, W. et al. "A Corynebacterium glutamicum gene conferring multidrug resistance in the heterologous host Escherichia coli," J. Bacteriol., 179(7):2449-2451 (1997) |
| U43536 | clpB | Heat shock ATP-binding protein | |
| U53587 | aphA-3 | 3'5"-aminoglycoside phosphotransferase | |
| U89648 | | Corynebacterium glutamicum unidentified sequence involved in histidine biosynthesis, partial sequence | |
| X04960 | trpA; trpB; trpC; trpD; trpE; trpG; trpL | Tryptophan operon | Matsui, K. et al. "Complete nucleotide and deduced amino acid sequences of the Brevibacterium lactofermentum tryptophan operon," Nucleic Acids Res., 14(24):10113-10114 (1986) |
| X07563 | lys A | DAP decarboxylase (meso-diaminopimelate decarboxylase, EC 4.1.1.20) | Yeh, P. et al. "Nucleic sequence of the lysA gene of Corynebacterium glutamicum and possible mechanisms for modulation of its expression," Mol. Gen. Genet., 212(1):112-119 (1988) |
| X14234 | EC 4.1.1.31 | Phosphoenolpyruvate carboxylase | Eikmanns, B.J. et al. "The Phosphoenolpyruvate carboxylase gene of Corynebacterium glutamicum: Molecular cloning, nucleotide sequence, and expression," Mol. Gen. Genet., 218(2):330-339 (1989); Lepiniec, L. et al. "Sorghum Phosphoenolpyruvate carboxylase gene family: structure, function and molecular evolution," Plant. Mol. Biol., 21 (3):487-502 (1993) |
| X17313 | fda | Fructose-bisphosphate aldolase | Von der Osten, C.H. et al. "Molecular cloning, nucleotide sequence and fine-structural analysis of the Corynebacterium glutamicum fda gene: structural comparison of C. glutamicum fructose-1, 6-biphosphate aldolase to class I and class II aldolases," *Mol. Microbiol.,* |
| X53993 | dapA | L-2, 3-dihydrodipicolinate synthetase (EC -4.2.1.52) | Bonnassie, S. et al. "Nucleic sequence of the dapA gene from Corynebacterium glutamicum," Nucleic Acids Res., 18(21):6421 (1990) |
| X54223 | | AttB-related site | Cianciotto, N. et al. "DNA sequence homology between att B-related sites of Corynebacterium diphtheriae, Corynebacterium ulcerans, Corynebacterium glutamicum , and the attP site of lambdacorynephage," FEMS. Microbiol, Lett., 66:299-302 (1990) |
| X54740 | argS; lysA | Arginyl-tRNA synthetase; Diaminopimelate decarboxylase | Marcel, T. et al. "Nucleotide sequence and organization of the upstream region of the Corynebacterium glutamicum lysA gene," Mol. Microbiol., 4(11):1819-1830 (1990) |
| X55994 | trpL; trpE | Putative leader peptide; anthranilate synthase component 1 | Heery, D.M. et al. "Nucleotide sequence of the Corynebacterium glutamicum trpE gene," Nucleic Acids Res., 18(23):7138 (1990) |
| X56037 | thrC | Threonine synthase | Han, K.S. et al. "The molecular structure of the Corynebacterium glutamicum threonine synthase gene," Mol. Microbiol., 4(10):1693-1702 (1990) |
| X56075 | attB-related site | Attachment site | Cianciotto, N. et al. "DNA sequence homology between att B-related sites of Corynebacterium diphtheriae, Corynebacterium ulcerans, Corynebacterium glutamicum , and the attP site of lambdacorynephage," FEMS. Microbiol, Lett., 66:299-302 (1990) |
| X57226 | lysC-alpha; lysC-beta; asd | Aspartokinase-alpha subunit; Aspartokinase-beta subunit; aspartate beta semialdehyde dehydrogenase | Kalinowski, J. et al. "Genetic and biochemical analysis of the Aspartokinase from Corynebacterium glutamicum," Mol. Microbiol., 5(5):1197-1204 (1991); Kalinowski, J. et al. "Aspartokinase genes lysC alpha and lysC beta overlap and are adjacent to the aspertate beta-semialdehyde dehydrogenase gene asd in Corynebacterium glutamicum," Mol. Gen. Genet., 224(3):317-324 (1990) |
| X59403 | gap;pgk; tpi | Glyceraldehyde-3-phosphate; phosphoglycerate kinase; triosephosphate isomerase | Eikmanns, B.J. "Identification, sequence analysis, and expression of a Corynebacterium glutamicum gene cluster encoding the three glycolytic enzymes glyceraldehyde-3-phosphate dehydrogenase, 3-phosphoglycerate kinase, and triosephosphate isomeras," J. Bacteriol., 174(19):6076-6086 (1992) |
| X59404 | gdh | Glutamate dehydrogenase | Bormann, E.R. et al. "Molecular analysis of the Corynebacterium glutamicum gdh gene encoding glutamate dehydrogenase," Mol. Microbiol., 6(3):317-326 (1992) |
| X60312 | lysI | L-lysine pennease | Seep-Feldhaus, A.H. et al. "Molecular analysis of the Corynebacterium glutamicum lysl gene involved in lysine uptake," Mol. Microbiol., 5(12):2995-3005 (1991) |
| X66078 | cop1 | Psl protein | Joliff, G. et al. "Cloning and nucleotide sequence of the csp1 gene encoding PS1, one of the two major secreted proteins of Corynebacterium glutamicum: The deduced N-terminal region of PS1 is similar to the Mycobacterium antigen 85 complex," Mol. Microbiol., 6(16):2349-2362 (1992) |
| X66112 | glt | Citrate synthase | Eikmanns, B.J. et al. "Cloning sequence, expression and transcriptional analysis of the Corynebacterium glutamicum gltA gene encoding citrate synthase," Microbiol., 140:1817-1828 (1994) |
| X67737 | dapB | Dihydrodipicolinate reductase | |
| X69103 | csp2 | Surface layer protein PS2 | Peyret, J.L. et al. "Characterization of the cspB gene encoding PS2, an ordered surface-layer protein in Corynebacterium glutamicum," Mol. Microbiol., 9(1):97-109 (1993) |
| X69104 | | IS3 related insertion element | Bonamy, C. et al. "Identification of IS1206, a Corynebacterium glutamicum IS3-related insertion sequence and phylogenetic analysis," Mol. Microbiol., 14(3):571-581 (1994) |
| X70959 | leuA | Isopropylmalate synthase | Patek, M. et al. "Leucine synthesis in Corynebacterium glutamicum: enzyme activities, structure of leuA, and effect of leuA inactivation on lysine synthesis," Appl. Environ. Microbiol., 60(1):133-140 (1994) |
| X71489 | icd | Isocitrate dehydrogenase (NADP+) | Eikmanns, B.J. et al. "Cloning sequence analysis, expression, and inactivation of the Corynebacterium glutamicum icd gene encoding isocitrate dehydrogenase and biochemical characterization of the enzyme," J. Bacteriol., 177(3):774-782 (1995) |
| X72855 | GDHA | Glutamate dehydrogenase (NADP+) | |
| X75083, X70584 | mtrA | 5-methyltryptophan resistance | Heery, D.M. et al. "A sequence from a tryptophan-hyperproducing strain of Corynebacterium glutamicum encoding resistance to 5-methyltryptophan," Biochem. Biophys. Res. Commun., 201(3):1255-1262 (1994) |
| X75085 | recA | | Fitzpatrick, R. et al. "Construction and characterization of recA mutant strains of Corynebacterium glutamicum and Brevibacterium lactofermentum," Appl. Microbiol. Biotechnol., 42(4):575-580 (1994) |
| X75504 | aceA; thiX | Partial Isocitrate lyase; ? | Reinscheid, D.J. et al. "Characterization of the isocitrate lyase gene from Corynebacterium glutamicum and biochemical analysis of the enzyme," J. Bacteriol., 176(12):3474-3483 (1994) |
| X76875 | | ATPase beta-subunit | Ludwig, W. et al. "Phylogenetic relationships of bacteria based on comparative sequence analysis of elongation factor Tu and ATP-synthase beta-subunit genes," Antonie Van Leeuwenhoek, 64:285-305 (1993) |
| X77034 | tuf | Elongation factor Tu | Ludwig, W. et al. "Phylogenetic relationships of bacteria based on comparative sequence analysis of elongation factor Tu and ATP-synthase beta-subunit genes," Antonie Van Leeuwenhoek, 64:285-305 (1993) |
| X77384 | recA | | Billman-Jacobe, H. "Nucleotide sequence of a recA gene from Corynebacterium glutamicum," DNA Seq., 4(6):403-404 (1994) |
| X78491 | aceB | Malate synthase | Reinscheid, D.J. et al. "Malate synthase from Corynebacterium glutamicum pta-ack operon encoding phosphotransacetylase: sequence analysis," Microbiology, 140:3099-3108 (1994) |
| X80629 | 16S rDNA | 16S ribosomal RNA | Rainey, F.A. et al. "Phylogenetic analysis of the genera Rhodococcus and Norcardia and evidence for the evolutionary origin of the genus Norcardia from within the radiation of Rhodococcus species," Microbiol., 141:523-528 (1995) |
| X81191 | gluA; gluB; gluC; gluD | Glutamate uptake system | Kronemeyer, W. et al. "Structure of the gluABCD cluster encoding the glutamate uptake system of Corynebacterium glutamicum," J. Bacteriol., 177(5):1152-1158 (1995) |
| X81379 | dapE | Succinyldiaminopimelate desuccinylase | Wehrmann, A. et al. "Analysis of different DNA fragments of Corynebacterium glutamicum complementing dapE of Escherichia coli," Microbiology, 40:3349-56 (1994) |
| X82061 | 16S rDNA | 16S ribosomal RNA | Ruimy, R. et al. "Phylogeny of the genus Corynebacterium deduced from analyses of small-subunit ribosomal DNA sequences," Int. J. Syst, Bacteriol., 45(4):740-746 (1995) |
| X82928 | asd; lysC | Aspartate-semialdehyde dehydrogenase; ? | Serebrijski, I. et al. "Multicopy suppression by asd gene and osmotic stress-dependent complementation by heterologous proA in proA mutants," J. Bacteriol., 177(24):7255-7260 (1995). |
| X82929 | proA | Gamma-glutamyl phosphate reductase | Serebrijski, I. et al. "Multicopy suppression by asd gene and osmotic stress-dependent complementation by heterologous proA in proA mutants," J. Bacteriol., 177(24):7255-7260 (1995) |
| X84257 | 16S rDNA | 16S ribosomal RNA | Pascual, C. et al. "Phylogenetic analysis of the genus Corynebacterium based on 16S rRNA gene sequences," Int. J. Syst. Bacteria/., 45(4):724-728 (1995) |
| X85965 | aroP; dapE | Aromatic amino acid permease; ? | Wehrmann et al. "Functional analysis of sequences adjacent to dapE of C. glutamicum proline reveals the presence of aroP, which encodes the aromatic amino acid transporter," J. Bacteriol., 177(20):5991-5993 (1995) |
| X86157 | argB; argC; argD; argF; argJ | Acetylglutamate kinase; N-acetyl-gamma-glutamyl-phosphate reductase; acetylornithine aminotransferase; ornithine carbamoyltransferase; glutamate N-acetyltransferase | Sakanyan, V. et al. "Genes and enzymes of the acetyl cycle of arginine biosynthesis in Corynebacterium glutamicum: enzyme evolution in the early steps of the arginine pathway," Microbiology, 142:99-108 (1996) |
| X89084 | pta; ackA | Phosphate acetyltransferase; acetate kinase | Reinscheid, D.J. et al. "Cloning, sequence analysis, expression and inactivation of the Corynebacterium glutamicum pta-ack operon encoding phosphotransacetylase and acetate kinase," Microbiology, 145:503-513 (1999) |
| X89850 | attB | Attachment site | Le Marrec, C. et al. "Genetic characterization of site-specific integration functions of phi AAU2 infecting "Arthrobacter aureus C70," J. Bacteriol., 178(7):1996-2004 (1996) |
| X90356 | | Promoter fragment F1 | Patek, M. et al. "Promoters from Corynebacterium glutamicum: cloning, molecular analysis and search for a consensus motif," Microbiology, 142:1297-1309 (1996) |
| X90357 | | Promoter fragment F2 | Patek, M. et al. "Promoters from Corynebacterium glutamicum: cloning, molecular analysis and search for a consensus motif," Microbiology, 142:1297-1309 (1996) |
| X90358 | | Promoter fragment F10 | Patek, M. et al. "Promoters from Corynebacterium glutamicum: cloning, molecular analysis and search for a consensus motif," Microbiology, 142:1297-1309 (1996) |
| X90359 | | Promoter fragment F13 | Patek, M. et al. "Promoters from Corynebacterium glutamicum: cloning, molecular analysis and search for a consensus motif," Microbiology, 142:1297-1309 (1996) |
| X90360 | | Promoter fragment F22 | Patek, M. et al. "Promoters from Corynebacterium glutamicum: cloning, molecular analysis and search for a consensus motif," Microbiology, 142:1297-1309 (1996) |
| X90361 | | Promoter fragment F34 | Patek, M. et al. "Promoters from Corynebacterium glutamicum: cloning, molecular analysis and search for a consensus motif," Microbiology, 142:1297-1309 (1996) |
| X90362 | | Promoter fragment F37 | Patek, M. et al. "Promoters from C. glutamicum: cloning, molecular analysis and search for a consensus motif," Microbiology, 142:1297-1309 (1996) |
| X90363 | | Promoter fragment F45 | Patek, M. et al. "Promoters from Corynebacterium glutamicum: cloning, molecular analysis and search for a consensus motif," Microbiology, 142:1297-1309 (1996) |
| X90364 | | Promoter fragment F64 | Patek, M. et al. "Promoters from Corynebacterium glutamicum: cloning, molecular analysis and search for a consensus motif," Microbiology, 142:1297-1309 (1996) |
| X90365 | | Promoter fragment F75 | Patek, M. et al. "Promoters from Corynebacterium glutamicum: cloning, molecular analysis and search for a consensus motif," Microbiology, 142:1297-1309 (1996) |
| X90366 | | Promoter fragment PF101 | Patek, M. et al. "Promoters from Corynebacterium glutamicum: cloning, molecular analysis and search for a consensus motif," Microbiology, 142:1297-1309 (1996) |
| X90367 | | Promoter fragment PF104 | Patek, M. et al. "Promoters from Corynebacterium glutamicum: cloning, molecular analysis and search for a consensus motif," Microbiology, 142:1297-1309 (1996) |
| X90368 | | Promoter fragment PF109 | Patek, M. et al. "Promoters from Corynebacterium glutamicum: cloning, molecular analysis and search for a consensus motif," Microbiology, 142:1297-1309 (1996) |
| X93513 | amt | Ammonium transport system | Siewe, R.M. et al. "Functional and genetic characterization of the (methyl) ammonium uptake carrier of Corynebacterium glutamicum," J. Biol. Chem., 271(10):5398-5403 (1996) |
| X93514 | betP | Glycine betaine transport system | Peter, H. et al. "Isolation, characterization, and expression of the Corynebacterium glutamicum betP gene, encoding the transport system for the compatible solute glycine betaine," J. Bacteriol., 178(17):5229-5234 (1996) |
| X95649 | orf4 | | Patek, M. et al. "Identification and transcriptional analysis of the dapB-ORF2-dapA-ORF4 operon of Corynebacterium glutamicum, encoding two enzymes involved in L-lysine synthesis," Biotechnol, Lett., 19:1113-1117 (1997) |
| X96471 | lysE; lysG | Lysine exporter protein; Lysine export regulator protein | Vrljic, M. et al. "A new type of transporter with a new type of cellular function: L-lysine export from Corynebacterium glutamicum," Mol. Microbiol., 22(5):815-826 (1996) |
| X96580 | panB; panC; xylB | 3-methyl-2-oxobutanoate hydroxymethyltransferase; pantoate-beta-alanine ligase; xylulokinase | Sahm, H. et al. "D-pantothenate synthesis in Corynebacterium glutamicum and use of panBC and genes encoding L-valine synthesis for D-pantothenate overproduction,"Appl. Environ. Microbiol., 65(5):1973-1979 (1999) |
| X96962 | | Insertion sequence IS1207 and transposase | |
| X99289 | | Elongation factor P | Ramos, A. et al. "Cloning, sequencing and expression of the gene encoding elongation factor P in the amino-acid producer Brevibacterium lactofermentum (Corynebacterium glutamicum ATCC 13869)," Gene, 198:217-222 (1997) |
| Y00140 | thrB | Homoserine kinase | Mateos, L.M. et al. "Nucleotide sequence of the homoserine kinase (thrB) gene of the Brevibacterium lactofermentum," Nucleic Acids Res., 15(9):3922 (1987) |
| Y00151 | ddh | Meso-diaminopimelate D-dehydrogenase (EC 1.4.1.16) | Ishino, S. et al. "Nucleotide sequence of the meso-diaminopimelate D-dehydrogenase gene from Corynebacterium glutamicum," Nucleic Acids Res., 15(9):3917 (1987) |
| Y00476 | thrA | Homoserine dehydrogenase | Mateos, L.M. et al. "Nucleotide sequence of the homoserine dehydrogenase (thrA) gene of the Brevibacterium lactofermentum," Nucleic Acids Res., 15(24):10598 (1987) |
| Y00546 | hom; thrB | Homoserine dehydrogenase; homoserine kinase | Peoples, O.P. et al. "Nucleotide sequence and fine structural analysis of the Corynebacterium glutamicum hom-thrB operon," Mol. Microbiol., 2(1):63-72 (1988) |
| Y08964 | murC; ftsQ/divD; ftsZ | UPD-N-acetylmuramate-alanine ligase; division initiation protein or cell division protein; cell division protein | Honrubia, M.P. et al. "Identification, characterization, and chromosomal organization of the ftsZ gene from Brevibacterium lactofermentum," Mol. Gen. Genet., 259(1):97-104 (1998) |
| Y09163 | putP | High affinity proline transport system | Peter, H. et al. "Isolation of the putP gene of Corynebacterium glutamicumproline and characterization of a low-affinity uptake system for compatible solutes," Arch. Microbiol., 168(2):143-151 (1997) |
| Y09548 | pyc | Pyruvate carboxylase | Peters-Wendisch, P.G. et al. "Pyruvate carboxylase from Corynebacterium glutamicum: characterization, expression and inactivation of the pyc gene," Microbiology, 144:915-927 (1998) |
| Y09578 | leuB | 3-isopropylmalate dehydrogenase | Patek, M. et al. "Analysis of the leuB gene from Corynebacterium glutamicum," Appl. Microbiol. Biotechnol., 50(1):42-47 (1998) |
| Y12472 | | Attachment site bacteriophage Phi-16 | Moreau, S. et al.: "Site-specific integration of corynephage Phi-16: The construction of an integration vector," Microbiol., 145:539-548 (1999) |
| Y12537 | proP | Proline/ectoine uptake system protein | Peter, H. et al. "Corynebacterium glutamicum is equipped with four secondary carriers for compatible solutes: Identification, sequencing, and characterization of the proline/ectoine uptake system, ProP, and the ectoine/proline/glycine betaine carrier, EctP," J. Bacteriol., 180(22):6005-6012 (1998) |
| Y13221 | glnA | Glutamine synthetase I | Jakoby, M. et al. "Isolation of Corynebacterium glutamicum glnA gene encoding glutamine synthetase I," FEMS Microbiol. Lett., 154(1):81-88 (1997) |
| Y16642 | lpd | Dihydrolipoamide dehydrogenase | |
| Y18059 | | Attachment site Corynephage 304L | Moreau, S. et al. "Analysis of the integration functions of φ304L: An integrase module among corynephages," Virology, 255(1):150-159 (1999) |
| Z21501 | argS; lysA | Arginyl-tRNA synthetase; diaminopimelate decarboxylase (partial) | Oguiza, J.A. et al. "A gene encoding arginyl-tRNA synthetase is located in the upstream region of the lysA gene in Brevibacterium lactofermentum: Regulation of argS-lysA cluster expression by arginine," J. Bacteriol.,175(22):7356-7362 (1993) |
| Z21502 | dapA; dapB | Dihydrodipicolinate synthase; dihydrodipicolinate reductase | Pisabarro, A. et al. "A cluster of three genes (dapA, orf2, and dapB) of Brevibacterium lactofermentum encodes dihydrodipicolinate reductase, and a third polypeptide of unknown function," J. Bacteriol., 175(9):2743-2749 (1993) |
| Z29563 | thrC | Threonine synthase | Malumbres, M. et al. "Analysis and expression of the thrC gene of the encoded threonine synthase," Appl. Envirom Microbiol.. 60(7)2209-2219 (1994) |
| Z46753 | 16S rDNA | Gene for 16S ribosomal RNA | |
| Z49822 | sigA | SigA sigma factor | Oguiza, J.A. et al "Multiple sigma factor genes in Brevibacterium lactofermentum: Characterization of sigA and sigB," J. Bacteriol., 178(2):550-553 (1996) |
| Z49823 | galE; dtxR | Catalytic activity UDP-galactose 4-epimerase; diphtheria toxin regulatory protein | Oguiza, J.A. et al "The galE gene encoding the UDP-galactose 4-epimerase of Brevibacterium lactofermentum is coupled transcriptionally to the dmdR gene," Gene, 177:103-107 (1996) |
| Z49824 | oral; sigB | ?; SigB sigma factor | Oguiza, J.A. et al "Multiple sigma factor genes in Brevibacterium lactofermentum: Characterization of sigA and sigB," J. Bacteriol., 178(2):550-553 (1996) |
| Z66534 | | Transposase | Correia, A. et al. "Cloning and characterization of an IS-like element present in the genome of Brevibacterium lactofermentum ATCC 13869," Gene, 170(1):91-94 (1996) |

¹ A sequence for this gene was published in the indicated reference. However, the sequence obtained by the inventors of the present application is significantly longer than the published version. It is believed that the published version relied on an incorrect start codon, and thus represents only a fragment of the actual coding region.

**TABLE 3: Corynebacterium and Brevibacterium Strains Which May be Used in the Practice of the Invention**

| **Genus** | **species** | **ATCC** | **FERM** | **NRRL** | **CECT** | **NCIMB** | **CBS** | **NCTC** | **DSMZ** | |
|---|---|---|---|---|---|---|---|---|---|---|
| Brevibacterium | ammoniagenes | 21054 | | | | | | | | |
| Brevibacterium | ammoniagenes | 19350 | | | | | | | | |
| Brevibacterium | ammoniagenes | 19351 | | | | | | | | |
| Brevibacterium | ammoniagenes | 19352 | | | | | | | | |
| Brevibacterium | ammoniagenes | 19353 | | | | | | | | |
| Brevibacterium | ammoniagenes | 19354 | | | | | | | | |
| Brevibacterium | ammoniagenes | 19355 | | | | | | | | |
| Brevibacterium | ammoniagenes | 19356 | | | | | | | | |
| Brevibacterium | ammoniagenes | 21055 | | | | | | | | |
| Brevibacterium | ammoniagenes | 21077 | | | | | | | | |
| Brevibacterium | ammoniagenes | 21553 | | | | | | | | |
| Brevibacterium | ammoniagenes | 21580 | | | | | | | | |
| Brevibacterium | ammoniagenes | 39101 | | | | | | | | |
| Brevibacterium | butanicum | 21196 | | | | | | | | |
| Brevibacterium | divaricatum | 21792 | P928 | | | | | | | |
| Brevibacterium | flavum | 21474 | | | | | | | | |
| Brevibacterium | flavum | 21129 | | | | | | | | |
| Brevibacterium | flavum | 21518 | | | | | | | | |
| Brevibacterium | flavum | | | B11474 | | | | | | |
| Brevibacterium | flavum | | | B11472 | | | | | | |
| Brevibacterium | flavum | 21127 | | | | | | | | |
| Brevibacterium | flavum | 21128 | | | | | | | | |
| Brevibacterium | flavum | 21427 | | | | | | | | |
| Brevibacterium | flavum | 21475 | | | | | | | | |
| Brevibacterium | flavum | 21517 | | | | | | | | |
| Brevibacterium | flavum | 21528 | | | | | | | | |
| Brevibacterium | flavum | 21529 | | | | | | | | |
| Brevibacterium | flavum | | | B11477 | | | | | | |
| Brevibacterium | flavum | | | B11478 | | | | | | |
| Brevibacterium | flavum | 21127 | | | | | | | | |
| Brevibacterium | flavum | | | B11474 | | | | | | |
| Brevibacterium | healii | 15527 | | | | | | | | |
| Brevibacterium | ketoglutamicum | 21004 | | | | | | | | |
| Brevibacterium | ketoglutamicum | 21089 | | | | | | | | |
| Brevibacterium | ketosoreductum | 21914 | | | | | | | | |
| Brevibacterium | lactofermentum | | | | 70 | | | | | |
| Brevibacterium | lactofermentum | | | | 74 | | | | | |
| Brevibacterium | lactofermentum | | | | 77 | | | | | |
| Brevibacterium | lactofermentum | 21798 | | | | | | | | |
| Brevibacterium | lactofermentum | 21799 | | | | | | | | |
| Brevibacterium | lactofermentum | 21800 | | | | | | | | |
| Brevibacterium | lactofermentum | 21801 | | | | | | | | |
| Brevibacterium | lactofermentum | | | B11470 | | | | | | |
| Brevibacterium | lactofermentum | | | B11471 | | | | | | |
| Brevibacterium | lactofermentum | 21086 | | | | | | | | |
| Brevibacterium | lactofermentum | 21420 | | | | | | | | |
| Brevibacterium | lactofermentum | 21086 | | | | | | | | |
| Brevibacterium | lactofermentum | 31269 | | | | | | | | |
| Brevibacterium | linens | 9174 | | | | | | | | |
| Brevibacterium | linens | 19391 | | | | | | | | |
| Brevibacterium | linens | 8377 | | | | | | | | |
| Brevibacterium | paraffinolyticum | | | | | 11160 | | | | |
| Brevibacterium | spec. | | | | | | 717.73 | | | |
| Brevibacterium | spec. | | | | | | 717.73 | | | |
| Brevibacterium | spec. | 14604 | | | | | | | | |
| Brevibacterium | spec. | 21860 | | | | | | | | |
| Brevibacterium | spec. | 21864 | | | | | | | | |
| Brevibacterium | spec. | 21865 | | | | | | | | |
| Brevibacterium | spec. | 21866 | | | | | | | | |
| Brevibacterium | spec. | 19240 | | | | | | | | |
| Corynebacterium | acetoacidophilum | 21476 | | | | | | | | |
| Corynebacterium | acetoacidophilum | 13870 | | | | | | | | |
| Corynebacterium | acetoglutamicum | | | B11473 | | | | | | |
| Corynebacterium | acetoglutamicum | | | B11475 | | | | | | |
| Corynebacterium | acetoglutamicum | 15806 | | | | | | | | |
| Corynebacterium | acetoglutamicum | 21491 | | | | | | | | |
| Corynebacterium | acetoglutamicum | 31270 | | | | | | | | |
| Corynebacterium | acetophilum | | | B3671 | | | | | | |
| Corynebacterium | ammoniagenes | 6872 | | | | | | 2399 | | |
| Corynebacterium | ammoniagenes | 15511 | | | | | | | | |
| Corynebacterium | fujiokense | 21496 | | | | | | | | |
| Corynebacterium | glutamicum | 14067 | | | | | | | | |
| Corynebacterium | glutamicum | 39137 | | | | | | | | |
| Corynebacterium | glutamicum | 21254 | | | | | | | | |
| Corynebacterium | glutamicum | 21255 | | | | | | | | |
| Corynebacterium | glutamicum | 31830 | | | | | | | | |
| Corynebacterium | glutamicum | 13032 | | | | | | | | |
| Corynebacterium | glutamicum | 14305 | | | | | | | | |
| Corynebacterium | glutamicum | 15455 | | | | | | | | |
| Corynebacterium | glutamicum | 13058 | | | | | | | | |
| Corynebacterium | glutamicum | 13059 | | | | | | | | |
| Corynebacterium | glutamicum | 13060 | | | | | | | | |
| Corynebacterium | glutamicum | 21492 | | | | | | | | |
| Corynebacterium | glutamicum | 21513 | | | | | | | | |
| Corynebacterium | glutamicum | 21526 | | | | | | | | |
| Corynebacterium | glutamicum | 21543 | | | | | | | | |
| Corynebacterium | glutamicum | 13287 | | | | | | | | |
| Corynebacterium | glutamicum | 21851 | | | | | | | | |
| Corynebacterium | glutamicum | 21253 | | | | | | | | |
| Corynebacterium | glutamicum | 21514 | | | | | | | | |
| Corynebacterium | glutamicum | 21516 | | | | | | | | |
| Corynebacterium | glutamicum | 21299 | | | | | | | | |
| Corynebacterium | glutamicum | 21300 | | | | | | | | |
| Corynebacterium | glutamicum | 39684 | | | | | | | | |
| Corynebacterium | glutamicum | 21488 | | | | | | | | |
| Corynebacterium | glutamicum | 21649 | | | | | | | | |
| Corynebacterium | glutamicum | 21650 | | | | | | | | |
| Corynebacterium | glutamicum | 19223 | | | | | | | | |
| Corynebacterium | glutamicum | 13869 | | | | | | | | |
| Corynebacterium | glutamicum | 21157 | | | | | | | | |
| Corynebacterium | glutamicum | 21158 | | | | | | | | |
| Corynebacterium | glutamicum | 21159 | | | | | | | | |
| Corynebacterium | glutamicum | 21355 | | | | | | | | |
| Corynebacterium | glutamicum | 31808 | | | | | | | | |
| Corynebacterium | glutamicum | 21674 | | | | | | | | |
| Corynebacterium | glutamicum | 21562 | | | | | | | | |
| Corynebacterium | glutamicum | 21563 | | | | | | | | |
| Corynebacterium | glutamicum | 21564 | | | | | | | | |
| Corynebacterium | glutamicum | 21565 | | | | | | | | |
| Corynebacterium | glutamicum | 21566 | | | | | | | | |
| Corynebacterium | glutamicum | 21567 | | | | | | | | |
| Corynebacterium | glutamicum | 21568 | | | | | | | | |
| Corynebacterium | glutamicum | 21569 | | | | | | | | |
| Corynebacterium | glutamicum | 21570 | | | | | | | | |
| Corynebacterium | glutamicum | 21571 | | | | | | | | |
| Corynebacterium | glutamicum | 21572 | | | | | | | | |
| Corynebacterium | glutamicum | 21573 | | | | | | | | |
| Corynebacterium | glutamicum | 21579 | | | | | | | | |
| Corynebacterium | glutamicum | 19049 | | | | | | | | |
| Corynebacterium | glutamicum | 19050 | | | | | | | | |
| Corynebacterium | glutamicum | 19051 | | | | | | | | |
| Corynebacterium | glutamicum | 19052 | | | | | | | | |
| Corynebacterium | glutamicum | 19053 | | | | | | | | |
| Corynebacterium | glutamicum | 19054 | | | | | | | | |
| Corynebacterium | glutamicum | 19055 | | | | | | | | |
| Corynebacterium | glutamicum | 19056 | | | | | | | | |
| Corynebacterium | glutamicum | 19057 | | | | | | | | |
| Corynebacterium | glutamicum | 19058 | | | | | | | | |
| Corynebacterium | glutamicum | 19059 | | | | | | | | |
| Corynebacterium | glutamicum | 19060 | | | | | | | | |
| Corynebacterium | glutamicum | 19185 | | | | | | | | |
| Corynebacterium | glutamicum | 13286 | | | | | | | | |
| Corynebacterium | glutamicum | 21515 | | | | | | | | |
| Corynebacterium | glutamicum | 21527 | | | | | | | | |
| Corynebacterium | glutamicum | 21544 | | | | | | | | |
| Corynebacterium | glutamicum | 21492 | | | | | | | | |
| Corynebacterium | glutamicum | | | B8183 | | | | | | |
| Corynebacterium | glutamicum | | | B8182 | | | | | | |
| Corynebacterium | glutamicum | | | B12416 | | | | | | |
| Corynebacterium | glutamicum | | | B12417 | | | | | | |
| Corynebacterium | glutamicum | | | B12418 | | | | | | |
| Corynebacterium | glutamicum | | | B11476 | | | | | | |
| Corynebacterium | glutamicum | 21608 | | | | | | | | |
| Corynebacterium | lilium | | P973 | | | | | | | |
| Corynebacterium | nitrilophilus | 21419 | | | | 11594 | | | | |
| Corynebacterium | spec. | | P4445 | | | | | | | |
| Corynebacterium | spec. | | P4446 | | | | | | | |
| Corynebacterium | spec. | 31088 | | | | | | | | |
| Corynebacterium | spec. | 31089 | | | | | | | | |
| Corynebacterium | spec. | 31090 | | | | | | | | |
| Corynebacterium | spec. | 31090 | | | | | | | | |
| Corynebacterium | spec. | 31090 | | | | | | | | |
| Corynebacterium | spec. | 15954 | | | | | | | 20145 | |
| Corynebacterium | spec. | 21857 | | | | | | | | |
| Corynebacterium | spec. | 21862 | | | | | | | | |
| Corynebacterium | spec. | 21863 | | | | | | | | |
| Corynebacterium | Glutamicum* | | | | | | | | | ASO19 |
| Corynebacterium | Glutamicum** | | | | | | | | | ASO19 E12 |
| Corynebacterium | Glutamicum*** | | | | | | | | | HL457 |
| Corynebacterium | Glutamicum**** | | | | | | | | | HL459 |

| | | | | | | | | | | |
|---|---|---|---|---|---|---|---|---|---|---|
| ATCC: American Type Culture Collection, Rockville, MD, USA FERM: Fermentation Research Institute, Chiba, Japan NRRL: ARS Culture Collection, Northern Regional Research Laboratory, Peoria, IL, USA CECT: Coleccion Espanola de Cultivos Tipo, Valencia, Spain NCIMB; National Collection of Industrial and Marine Bacteria Ltd., Aberdeen, UK CBS: Centraalbureau voor Schimmelcultures, Baam, NL NCTC: National Collection of Type Cultures, London, UK DSMZ: Deutsche Sammlung von Mikroorganismen und Zellkulturen, Braunschweig, Germany For reference see Sugawara, H. et al. (1993) World directory of collections of cultures of microorganisms: Bacteria, fungi and yeasts (4^{th} edn), World federation for culture collections world data center on microorganisms, Saimata, Japen. * Spontaneous rifampin-resistant mutant of *C. glutamicum* ATCC13059^{d} Yoshihama *et al.,* 1985 ** Restriction-deficient variant of ASO19 Follettie *et al.,* 1993 *** *metC*-disrupted mutant of ASO19E12 This study **** *metC*-disrupted mutant of ASO19E12 This study | | | | | | | | | | |

**TABLE 4: ALIGNMENT RESULTS**

| **ID #** | **length (NT)** | **Genbank Hit** | **Length** | **Accession** | **Name of Genbank Hit** | **Source of Genbank Hit** | **% homology (GAP)** | **Date of Deposit** |
|---|---|---|---|---|---|---|---|---|
| rxa00657 | 906 | GB_BA1:AF064700 | 3481 | AF064700 | Rhodococcus sp. NO1-1 CprS and CprR genes, complete cds. | Rhodococcus sp | 40.265 | 15-Jul-98 |
| metz | 1314 | GB_BA2:MTV016 | 53662 | AL021841 | Mycobacterium tuberculosis H37Rv complete genome; segment 143/162. | Mycobacterium tuberculosis | 61.278 | 23-Jun-99 |
| metc | 978 | GB_BA2:CORCSLYS | 2821 | M89931 | Corynebacterium glutamicum beta C-S lyase (aecD) and branched-chain amino acid upta | Corynebacterium glutamicum | 99.591 | 04-JUN-1998 |
| rxa00023 | 3579 | GB_EST33A1776129 | 483 | AI776129 | EST257217 tomato resistant, Cornell Lycopersicon esculentum cDNA clone cLER17D3, mRNA sequence. | Lycopersicon esculentum | 40,956 | 29-Jun-99 |
| | | GB_EST33:AI776129 | 483 | AI776129 | EST257217 tomato resistant, Cornell Lycopersicon esculentum cDNA clone cLER17D3, mRNA sequence. | Lycopersicon esculentum | 40,956 | 29-Jun-99 |
| | | | | | | | | |
| rxa00044 | 1059 | EM_PAT:E11760 | 6911 | E11760 | Base sequence of sucrase gene. | Corynebacterium glutamicum | 42,979 | 08-OCT-1997 |
| | | | | | | | | (Rel. 52, Created) |
| | | GB_PAT:I26124 | 6911 | 126124 | Sequence 4 from patent US 5556776. | Unknown. | 42,979 | 07-OCT-1996 |
| | | GB_BA2:ECOUW89 | 176195 | U00006 | E. coli chromosomal region from 89.2 to 92.8 minutes. | Escherichia coli | 39,097 | 17-DEC-1993 |
| rxa00064 | 1401 | GB_PAT:E16763 | 2517 | E16763 | gDNA encoding aspartate transferase (AAT). | Corynebacterium glulamicum | 95,429 | 28-Jul-99 |
| | | GB_HTG2:AC007892 | 134257 | AC007892 | Drosophila melanogaster chromosome 3 clone BACR02O03 (D797) RPCI-98 02.O.3 map 99B-99B strain y; cn bw sp, *** SEQUENCING IN PROGRESS ***, 113 unordered pieces. | Drosophila melanogaster | 31,111 | 2-Aug-99 |
| | | GB_HTG2:AC007892 | 134257 | AC007892 | Drosophila melanogaster chromosome 3 clone BACR02O03 (D797) RPCI-98 02.O.3 map 99B-99B strain y; cn bw sp, *** SEQUENCING IN PROGRESS***, 113 unordered pieces. | Drosophila melanogaster | 31,111 | 2-Aug-99 |
| rxa00072 | | | | | | | | |
| | | | | | | | | |
| rxa00105 | 798 | GB_BA1:MTV002 | 56414 | AL008967 | Mycobacterium tuberculosis H37Rv complete genome; segment 122/162. | Mycobacterium tuberculosis | 37,753 | 17-Jun-98 |
| | | GB_BA1:ECU29581 | 71128 | U29581 | Escherichia coli K-12 genome; approximately 63 to 64 minutes. | Escherichia coli | 35,669 | 14-Jan-97 |
| | | GB_BA2:AE000366 | 10405 | AE000366 | Escherichia coli K-12 MG1655 section 256 of 400 of the complete genome. | Escherichia coli | 35,669 | 12-Nov-98 |
| rxa00106 | 579 | GB_EST15:AA494237 | 367 | AA494237 | ng83f04.s1 NCI_CGAP_Pr6 Homo sapiens cDNA clone IMAGE:941407 similar to SW:DYR_LACCA P00381 DIHYDROFOLATE REDUCTASE ;, mRNA sequence. | Homo sapiens | 42,896 | 20-Aug-97 |
| | | GB_BA2:AF161327 | 2021 | AF161327 | Corynebacterium diphtheriae histidine kinase ChrS (chrS) and response regulator ChrA (chrA) genes, complete cds. | Corynebacterium diphtheriae | 40,210 | 9-Sep-99 |
| | | GB_PAT:AR041189 | 654 | AR041189 | Sequence 4 from patent US 5811286. | Unknown. | 41,176 | 29-Sep-99 |
| rxa00115 | 1170 | GB_PR4:AC007110 | 148336 | AC007110 | Homo sapiens chromosome 17, clone hRPK.472_J_18, complete sequence. | Homo sapiens | 36,783 | 30-MAR-1999 |
| | | | | | | | | |
| | | GB_HTG3:AC008537 | 170030 | AC008537 | Homo sapiens chromosome 19 clone CIT-HSPC_490E21, *** SEQUENCING IN PROGRESS ***, 93 unordered pieces. | Homo sapiens | 40,296 | 2-Sep-99 |
| | | GB_HTG3:AC008537 | 170030 | AC008537 | Homo sapiens chromosome 19 clone CIT-HSPC_490E21, *** SEQUENCING IN PROGRESS ***, 93 unordered pieces. | Homo sapiens | 40,296 | 2-Sep-99 |
| rxa00116 | 1284 | GB_BA2:AF062345 | 16458 | AF062345 | Caulobacter crescentus Sst1 (sst1), S-layer protein subunit (rsaA), ABC transporter (rsaD), membrane forming unit (rsaE), putative GDP-mannose-4,6-dehydratase (IpsA), putative acetyltransferase (IpsB), putative perosamine synthetase (IpsC), putative mannosyltransferase (IpsD), putative mannosyltransferass (IpsE), outer membrane protein (rsaF), and putative perosamine transferase (IpsE) genes, complete cds. | Caulobacter crescentus | 36,235 | 19-OCT-1999 |
| | | GB_PAT:I18647 | 3300 | 118647 | Sequence 6 from patent US 5500353. | Unknown. | 36,821 | 07-OCT-1996 |
| | | GB_GSS13:AQ446197 | 751 | AQ446197 | nbxb0062D16r CUGI Rice BAC Library Oryza sativa genomic clone nbxb0062D16r, genomic survey sequence. | Oryza sativa | 38,124 | 8-Apr-99 |
| rxa00131 | 732 | GB_BA1:MTY20B11 | 36330 | Z95121 | Mycobacterium tuberculosis H37Rv complete genome; segment 139/162. | Mycobacterium tuberculosis | 43,571 | 17-Jun-98 |
| | | GB_BA1:SAR7932 | 15176 | AJ007932 | Streptomyces argillaceus mithramycin biosynthetic genes. | Streptomyces argillaceus | 41,116 | 15-Jun-99 |
| | | GB_BA1:MTY20B11 | 36330 | Z95121 | Mycobacterium tuberculosis H37Rv complete genome; segment 139/162. | Mycobacterium tuberculosis | 39,726 | 17-Jun-98 |
| rxa00132 | 1557 | GB_BA1:MTY20B11 | 36330 | Z95121 | Mycobacterium tuberculosis H37Rv complete genome; segment 139/162. | Mycobacterium tuberculosis | 36,788 | 17-Jun-98 |
| | | GB_IN2:TVU40872 | 1882 | U40872 | Trichomonas vaginalis S-adenosyl-L-homocysteine hydrolase gene, complete cds. | Trichomonas vaginalis | 61,914 | 31-OCT-1996 |
| | | GB_HTG6:AC010706 | 169265 | AC010706 | Drosophila melanogaster chromosome X clone BACR36D15 (D887) RPCI-98 36.D.15 map 13C-13E strain y; cn bw sp, *** SEQUENCING IN PROGRESS ***, 74 unordered pieces. | Drosophila melanogaster | 51,325 | 22-Nov-99 |
| rxa00145 | 1059 | GB_BA1:MTCY2B12 | 20431 | Z81011 | Mycobacterium tuberculosis H37Rv complete genome; segment 61/162. | Mycobacterium tuberculosis | 63,365 | 18-Jun-98 |
| | | GB_BA1:PSEPYRBX | 2273 | L19649 | Pseudomonas aeruginosa aspartate transcarbamoylase (pyrB) and dihydroorolase-like (pyrX) genes, complete cds's. | Pseudomonas aeruginosa | 56,080 | 26-Jul-93 |
| | | GB_BA1:LLPYRBDNA | 1468 | X84262 | L.leichmannii pyrB gene. | Lactobacillus leichmannil | 47,514 | 29-Apr-97 |
| rxa00146 | 1464 | GB_BA1:MTCY2B12 | 20431 | Z81011 | Mycobacterium tuberculosis H37Rv complete genome; segment 61/162. | Mycobacterium tuberculosis | 60,714 | 18-Jun-98 |
| | | GB_BA1:MTCY154 | 13935 | Z98209 | Mycobacterium tuberculosis H37Rv complete genome; segment 121/162. | Mycobacterium tuberculosis | 39,229 | 17-Jun-98 |
| | | GB_BA1:MSGY154 | 40221 | AD000002 | Mycobacterium tuberculosis sequence from clone y154. | Mycobacterium tuberculosis | 36,618 | 03-DEC-1996 |
| rxa00147 | 1302 | GB_BA1:MTCY2B12 | 20431 | Z81011 | Mycobacterium tuberculosis H37Rv complete genome; segment 61/162. | Mycobacterium tuberculosis | 61,527 | 18-Jun-98 |
| | | GB_BA1:MSGB937CS | 38914 | L78820 | Mycobacterium leprae cosmid 8937 DNA sequence. | Mycobacterium leprae | 59,538 | 15-Jun-96 |
| | | GB_BA1:PAU81259 | 7285 | U81259 | Pseudomonas aeruginosa dihydrodipicolinate reductase (dapB) gene, partial cds, carbamoylphosphate synthetase small subunit (carA) and carbamoylphosphate synthetase large subunit (carB) genes, complete cds, and FtsJ homolog (ftsJ) gene, partial cds. | Pseudomonas aeruginosa | 55,396 | 23-DEC-1996 |
| rxa00156 | 1233 | GB_BA1:SC9B10 | 33320 | AL009204 | Streptomyces coelicolor cosmid 9B10. | Streptomyces coeticalor | 52,666 | 10-Feb-99 |
| | | GB_BA2:AF002133 | 15437 | AF002133 | Mycobacterium avium strain GIR10 transcriptional regulator (mav81) gene, partial cds, aconitase (acn), invasin 1 (inv1), invasin 2 (inv2), transcriptional regulator (moxR), ketoacyl-reductase (fabG), enoyl-reductase (inhA) and ferrochelatase (mav272) genes, complete cds. | Mycobacterium avium | 54,191 | 26-MAR-1998 |
| | | GB_BA1:D85417 | 7984 | D85417 | Propionibacterium freudenreichii hemY, hemH, hemB, hemX, hemR and hemL genes, complete cds. | Propionibacterium freudenreichii | 46,667 | 6-Feb-99 |
| rxa00166 | 783 | GB_HTG3:AC008167 | 174223 | AC008167 | Homo sapiens clone NH0172O13, *** SEQUENCING IN PROGRESS ***, 7 unordered pieces. | Homo sapiens | 37,451 | 21-Aug-99 |
| | | GB_HTG3:AC008167 | 174223 | AC008167 | Homo sapiens clone NH0172O13, *** SEQUENCING IN PROGRESS ***, 7 unordered pieces. | Homo sapiens | 37,451 | 21-Aug-99 |
| | | GB_HTG4:AC010118 | 80605 | AC010118 | Drosophila melanogaster chromosome 3L/62B1 clone RPCI98-10D15, *** SEQUENCING IN PROGRESS ***, 51 unordered pieces. | Drosophila melanogaster | 38,627 | 16-OCT-1999 |
| rxa00198 | 672 | GB_BA1:AB024708 | 8734 | AB024708 | Corynebacterium glutamicum gltB and gltD genes for glutamine 2-oxoglutarate aminotransferase large and small subunits, complete cds. | Corynebacterium glutamicum | 92,113 | 13-MAR-1999 |
| | | GB_BA1:AB024708 | 8734 | AB024708 | Corynebacterium glutamicum gltB and gltD genes for glutamine 2-oxoglutarate aminotransferase large and small subunits, complete cds. | Corynebacterium glutamicum | 93,702 | 13-MAR-1999 |
| | | GB_EST24:AI232702 | 528 | AI232702 | EST229390 Normalized rat kidney, Bento Soares Rattus sp. cDNA clone RKICF35 3' end, mRNA sequence. | Rattus sp. | 34,221 | 31-Jan-99 |
| rxa00216 | 1113 | GB_HTG2:HSDJ850E9 | 117353 | AL121758 | Homo sapiens chromosome 20 clone RP5-850E9, *** SEQUENCING IN PROGRESS ***, in unordered pieces. | Homo sapiens | 37,965 | 03-DEC-1999 |
| | | GB_HTG2:HSDJ850E9 | 117353 | AL121758 | Homo sapiens chromosome 20 clone RP5-850E9, *** SEQUENCING IN PROGRESS ***, in unordered pieces. | Homo sapiens | 37,965 | 03-DEC-1999 |
| | | GB_PR2:CNS01DSA | 159400 | AL121766 | Human chromosome 14 DNA sequence *** IN PROGRESS *** BAC R-412H8 of RPCI-11 library from chromosome 14 of Homo sapiens (Human), complete sequence. | Homo sapiens | 38,796 | 11-Nov-99 |
| rxa00219 | 1065 | GB_HTG2:AC005079_0 | 110000 | AC005079 | Homo sapiens clone RG252P22, *** SEQUENCING IN PROGRESS ***, 3 unordered pieces. | Homo sapiens | 38,227 | 22-Nov-98 |
| | | GB_HTG2AC005079_1 | 110000 | AC005079 | Homo sapiens clone RG252P22, *** SEQUENCING IN PROGRESS ***, 3 unordered pieces. | Homo sapiens | 38,227 | 22-Nov-98 |
| | | GB_HTG2:AC005079_1 | 110000 | AC005079 | Homo sapiens clone RG252P22, *** SEQUENCING IN PROGRESS ***, 3 unordered pieces. | Homo sapiens | 38,227 | 22-Nov-98 |
| rxa00223 | 1212 | GD_BA1:PPEA3NIF | 19771 | X99694 | Plasmid pEA3 nitrogen fixation genes. | Enterobacter agglomerans | 48,826 | 2-Aug-96 |
| | | GB_BA2:AF128444 | 2477 | AF128444 | Rhodobacter capsulatus molybdenum cofactor biosynthetic gene cluster, partial sequence. | Rhodobacter capsulatus | 40,135 | 22-MAR-1999 |
| | | GB_HTG4:AC010111 | 138938 | AC010111 | Drosophila melanogaster chromosome 3L/70C1 clone RPCI98-9B18, *** SEQUENCING IN PROGRESS ***, 64 unordered pieces. | Drosophila melanogaster | 39,527 | 16-OCT-1999 |
| rxa00229 | 803 | GB_BA2:AF124518 | 1758 | AF124518 | Corynebaclerium glutamicum 3-dehydroquinase (aroD) and shikimate dehydrogenase (aroE) genes, complete cds. | Corynebacterium glutamicum | 98,237 | 18-MAY-1999 |
| | | GB_PR3:AC004593 | 150221 | AC004593 | Homo sapiens PAC clone DJ0964C11 from 7p14-p15, complete sequence. | Homo sapiens | 36,616 | 18-Apr-98 |
| | | GB_HTG2:AC006907 | 188972 | AC006907 | Caenorhabditis elegans clone Y76B12, *** SEQUENCING IN PROGRESS ***, 25 unordered pieces. | Caenorhabditis elegans | 37,095 | 26-Feb-99 |
| rxa00241 | 1626 | GB_BA1:CGLYSI | 4232 | X60312 | C.glutamicum lysl gene for L-lysine permease. | Corynebacterium glulamicum | 100,000 | 30-Jan-92 |
| | | GB_HTG1:PFMAL13P1 | 192581 | AL049180 | Plasmodium falciparum chromosome 13 strain 3D7, *** SEQUENCING IN PROGRESS ***, in unordered pieces. | Plasmodium falciparum | 34,947 | 11-Aug-99 |
| | | GB_HTG1:PFMAL13P1 | 192581 | AL049180 | Plasmodium falciparum chromosome 13 strain 3D7, *** SEQUENCING IN PROGRESS ***, In unordered pieces. | Plasmodium falciparum | 34,947 | 11-Aug-99 |
| rxa00262 | 1197 | GB_IN2:EHU89655 | 3219 | U89655 | Entamoeba histolytica unconventional myosin IB mRNA, complete cds. | Entamoeba histolytica | 36,496 | 23-MAY-1997 |
| | | GB_IN2:EHU89655 | 3219 | U89655 | Entamoeba histolytica unconventional myosin IB mRNA, complete cds. | Entamoeba histolytica | 37,544 | 23-MAY-1997 |
| | | | | | | | | |
| rxa00266 | 531 | GB_RO:AF016190 | 2939 | AF016190 | Mus musculus connexin-36 (Cx36) gene, complete cds. | Mus musculus | 41,856 | 9-Feb-99 |
| | | EM_PAT:E09719 | 3505 | E09719 | DNA encoding precursor protein of alkaline cellulase. | Bacillus sp. | 34,741 | 08-OCT-1997 |
| | | | | | | | | (Rel. 52, Created) |
| | | GB_PAT:E02133 | 3494 | E02133 | gDNA encoding alkaline cellulase. | Bacillus sp. | 34,741 | 29-Sep-97 |
| rxa00278 | 1155 | GB_IN1:CELK05F6 | 36912 | AF040653 | Caenorhabditis elegans cosmid K05F6. | Caenorhabditis elegans | 36,943 | 6-Jan-98 |
| | | GB_BA1:CGU43535 | 2531 | U43535 | Corynebacterium glutamicum multidrug resistance protein (cmr) gene, complete cds. | Corynebacterium glutamicum | 36,658 | 9-Apr-97 |
| | | GB_RO:RNU30789 | 3510 | U30789 | Rattus norvegicus clone N27 mRNA. | Rattus norvegicus | 38,190 | 20-Aug-96 |
| rxa00295 | 1125 | GB_BA2:CGU31281 | 1614 | U31281 | Corynebacterium glutamicum biotin synthase (bioB) gene, complete cds. | Corynebacterium glutamicum | 99,111 | 21-Nov-96 |
| | | GB_BA1:BRLBIOBA | 1647 | D14084 | Brevibacterium flavum gene for biotin synthetase, complete cds. | Corynebacterium glutamicum | 98,489 | 3-Feb-99 |
| | | GB_PAT:E03937 | 1005 | E03937 | DNA sequence encoding Brevibacterium flavum biotin-synthase. | Corynebacterium glutamicum | 98,207 | 29-Sep-97 |
| rxa00323 | 1461 | GB_BA1:MTCY427 | 38110 | Z70692 | Mycobacterium tuberculosis H37Rv complete genome; segment 99/162. | Mycobacterium tuberculosis | 35,615 | 24-Jun-99 |
| | | GB_BA1:MSGB32CS | 36404 | L78818 | Mycobacterium leprae cosmid B32 DNA sequence. | Mycobacterium leprae | 60,917 | 15-Jun-96 |
| | | GB_BA1:MTCY427 | 38110 | Z70692 | Mycobacterium tuberculosis H37Rv complete genome; segment 99/162. | Mycobacterium tuberculosis | 44,606 | 24-Jun-99 |
| rxa00324 | 3258 | GB_BA1:MSGB32CS | 36404 | L78818 | Mycobacterium leprae cosmid B32 DNA sequence. | Mycobacterium leprae | 52,516 | 15-Jun-96 |
| | | GB_BA1:MTCY427 | 38110 | Z70692 | Mycobacterium tuberculosis H37Rv complete genome; segment 99/162. | Mycobacterium tuberculosis | 38,079 | 24-Jun-99 |
| | | GB_OM:BOVELA | 3242 | J02717 | Bovine elastin a mRNA, complete cds. | Bos taurus | 39,351 | 27-Apr-93 |
| rxa00330 | 1566 | GB_BA1:CGTHRC | 3120 | X56037 | Corynebacterium glutamicum thrC gene for threonine synthase (EC 4.2.99.2). | Corynebacterium glutamicum | 99,808 | 17-Jun-97 |
| | | | | | | | | |
| | | GB_PAT:I09078 | 3146 | 109078 | Sequence 4 from Patent WO 8809819. | Unknown. | 99,617 | 02-DEC-1994 |
| | | GB_BA1:BLTHRESYN | 1892 | Z29563 | Brevibacterium lactofermentum; ATCC 13869;; DNA (genomic);. | Corynebacterium glutamicum | 99,170 | 20-Sep-95 |
| rxa00335 | 1554 | GB_BA1:CGGLNA | 3686 | Y13221 | Corynebacterium glutamicum glnA gene. | Corynebacterium glutamicum | 100,000 | 28-Aug-97 |
| | | GB_BA2:AF005635 | 1690 | AF005635 | Corynebacterium glutamicum glutamine synthetase (glnA) gene, complete cds. | Corynebacterium glutamicum | 98,906 | 14-Jun-99 |
| | | | | | | | | |
| | | GB_BA1:MSGB27CS | 38793 | L78817 | Mycobacterium leprae cosmid B27 DNA sequence. | Mycobacterium leprae | 66,345 | 15-Jun-96 |
| r00347 | 891 | GB_EST27:AI455217 | 624 | AI455217 | LD21828.3prime LD Drosophila melanogaster embryo pOT2 Drosophila melanogaster cDNA clone LD21828 3prime, mRNA sequence. | Drosophila melanogaster | 34,510 | 09-MAR-1999 |
| | | GB_BA2:SSU30252 | 2891 | U30252 | Synechococcus PCC7942 nucleoside diphosphate kinase and ORF2 protein genes, complete cds, ORF1 protein gene, partial cds, and neutral site I for vector use. | Synechococcus PCC7942 | 37,084 | 29-OCT-1999 |
| | | GB_EST21:AA911262 | 581 | AA911262 | oe75a02.s1 NCI_CGAP _Lu5 Homo sapiens cDNA clone IMAGE:1417418 3' similar to gb:A18757 UROKINASE PLASMINOGEN ACTIVATOR SURFACE RECEPTOR, GPI-ANCHORED (HUMAN);, mRNA sequence. | Homo sapiens | 37,500 | 21-Apr-98 |
| r00351 | 1578 | GB_BA1:MLU15187 | 36138 | U15187 | Mycobacterium leprae cosmid L296. | Mycobacterium leprae | 52,972 | 09-MAR-1995 |
| | | GB_IN2:AC004373 | 72722 | AC004373 | Drosophila melanogaster DNA sequence (P1 DS05273 (D80)), complete sequence. | Drosophila melanogaster | 46,341 | 17-Jul-98 |
| | | GB_IN2:AF145653 | 3197 | AF145653 | Drosophila melanogaster clone GH08860 BcDNA.GH08860 (BcDNA.GH08860) mRNA, complete cds. | Drosophila melanogaster | 49,471 | 14-Jun-99 |
| rxa00365 | 727 | GB_BA1:AB024708 | 8734 | AB024708 | Corynebacterium glutamicum gltB and gltD genes for glutamine 2-oxoglutarate aminotransferase large and small subunits, complete cds. | Corynebacterium glutamicum | 96,556 | 13-MAR-1999 |
| | | GB_BA1:MTCY1A6 | 37751 | Z83864 | Mycobacterium tuberculosis H37Rv complete genome; segment 159/162. | Mycobacterium tuberculosis | 39,496 | 17-Jun-98 |
| | | GB_BA1:SC3A3 | 15901 | AL109849 | Streptomyces coelicolor cosmid 3A3. | Streptomyces coelicolor A3(2) | 37,946 | 16-Aug-99 |
| | | | | | | | | |
| rxa00366 | 480 | GB_BA1:AB024708 | 8734 | AB024708 | Corynebacterium glutamicum gltB and gltD genes for glutamine 2-oxoglutarate aminotransferase large and small subunits, complete cds. | Corynebacterium glutamicum | 99,374 | 13-MAR-1999 |
| | | GB_BA1:MTCY1A6 | 37751 | Z83864 | Mycobacterium tuberculosis H37Rv complete genome; segment 159/162. | Mycobacterium tuberculosis | 41,333 | 17-Jun-98 |
| | | GB_BA1:SC3A3 | 15901 | AL109849 | Streptomyces coelicolor cosmid 3A3. | Streptomyces coelicolor A3(2) | 37,554 | 16-Aug-99 |
| rxa00367 | 4653 | GB_BA1:AB024708 | 8734 | AB024708 | Corynebacterium glutamicum gltB and gltD genes for glutamine 2-oxoglutarate aminotransferase large and small subunits, complete cds. | Corynebacterium glutamicum | 99,312 | 13-MAR-1999 |
| | | GB_BA1:MTCY1A6 | 37751 | Z83864 | Mycobacterium tuberculosis H37Rv complete genome; segment 159/162. | Mycobacterium tuberculosis | 36,971 | 17-Jun-98 |
| | | GB_BA1:SC3A3 | 15901 | AL109849 | Streptomyces coelicolor cosmid 3A3. | Streptomyces coelicolor A3(2) | 37,905 | 16-Aug-99 |
| | | | | | | | | |
| rxa00371 | 1917 | GB_VI:SBVORFS | 7568 | M89923 | Sugarcane bacilliform virus ORF 1,2,and 3 DNA, complete cds. | Sugarcane bacilliform virus | 35,843 | 12-Jun-93 |
| | | GB_EST37:AI967505 | 380 | AI967505 | Ljirnpest03-215-c10 Ljirnp Lambda HybriZap two-hybrid library Lotus japonicu; cDNA clone LP215-03-c10 5' similar to 60S ribosomal protein L39, mRNA sequence. | s Lotus japonicus | 42,593 | 24-Aug-99 |
| | | GB_IN1:CELK09H9 | 37881 | AF043700 | Caenorhabditis elegans cosmid K09H9. | Caenorhabditis elegans | 34,295 | 22-Jan-98 |
| rxa00377 | 1245 | GB_BA1:CCU 13664 | 1678 | U13664 | Caulobacter crescentus uroporphyrinogen decarboxylase homolog (hemE) gene, partial cds. | Caulobacter crescentus | 36,832 | 24-MAR-1995 |
| | | GB_PL1:ANSDGENE | 1299 | Y08866 | A.nidulans sD gene. | Emericella nidulans | 39,603 | 17-OCT-1996 |
| | | GB_GSS4:AQ730303 | 483 | AQ730303 | HS_5505_B1_C04_T7A RPCI-11 Human Male BAC Library Homo sapiens genomic clone Plate=1081 Col=7 Row=F, genomic survey sequence. | Homo sapiens | 36,728 | 15-Jul-99 |
| rxa00382 | 1425 | GB_BA1:PAHEML | 4444 | X82072 | P.aeruginosa hemL gene. | Pseudomonas aeruginosa | 54,175 | 18-DEC-1995 |
| | | GB_BA1:MTY25D10 | 40838 | Z95558 | Mycobacterium tuberculosis H37Rv complete genome; segment 28/162. | Mycobacterium tuberculosis | 61,143 | 17-Jun-98- |
| | | GB_BA1:MSGY224 | 40051 | AD000004 | Mycobacterium tuberculosis sequence from clone y224. | Mycobacterium tuberculosis | 61,143 | 03-DEC-1996 |
| rxa00383 | 1467 | GB_BA1:MLCB1222 | 34714 | AL049491 | Mycobacterium leprae cosmid B1222. | Mycobacterium leprae | 43,981 | 27-Aug-99 |
| | | GB_HTG2:AC006269 | 167171 | AC006269 | Homo sapiens chromosome 17 clone hRPK.515_E_23 map 17, *** SEQUENCING IN PROGRESS ***, 2 ordered pieces. | Homo sapiens | 35,444 | 10-Jun-99 |
| | | GB_HTG2:AC007638 | 178053 3 | AC007638 | Homo sapiens chromosome 17 clone hRPK.515_O_17 map 17, *** SEQUENCING IN PROGRESS ***, 8 unordered pieces. | Homo sapiens | 34,821 | 22-MAY-1999 |
| rxa00391 | 843 | GB_EST38:AW017053 | 613 | AW017053 | EST272398 Schistosoma mansoni male, Phil LoVerde/Joe Merrick Schistosoma mansoni cDNA clone SMMAS14 5' end, mRNA sequence. | Schistosoma mansoni | 40,472 | 10-Sep-99 |
| | | GB_PATAR065852 | 32207 | AR065852 | Sequence 20 from patent US 5849564. | Unknown. | 38,586 | 29-Sep-99 |
| | | GB_VI:AF148805 | 28559 | AF148805 | Kaposi's sarcoma-associated herpesvirus ORF 6B gene, partial cds; and ORF 69, kaposin, v-FLIP, v-cyclin, latent nuclear antigen, ORF K14, v-GPCR, putative phosphoribosylformylglycinamidine synthase, and LAMP (LAMP) genes, complete cds. | Kaposi's sarcoma-associated herpesvirus | 38,509 | 2-Aug-99 |
| rxa00393 | 1017 | GB_BA1:MTY25D10 | 40838 | Z95558 | Mycobacterium tuberculosis H37Rv complete genome; segment 28/162, | Mycobacterium tuberculosis | 36,308 | 17-Jun-98 |
| | | GB_BA1:MSGY224 | 40051 | AD000004 | Mycobacterium tuberculosis sequence from clone y224. | Mycobacterium tuberculosis | 39,282 | 03-DEC-1996 |
| | | GB_BA1:MLB1306 | 7762 | Y13803 | Mycobacterium leprae cosmid B1306 DNA. | Mycobacterium lepras | 39,228 | 24-Jun-97 |
| rxa00402 | 623 | GB_BA2:AF052652 | 2096 | AF052652 | Corynebacterium glutamicum homoserine O-acetyltransferase (metA) gene, complete cds. | Corynebacterium glutamicum | 99,672 | 19-MAR-1998 |
| | | GB_BA2:AF109162 | 4514 | AF109162 | Corynebacterium diphtheriae heme uptake locus, complete sequence. | Corynebacterium diphtheriae | 40,830 | 8-Jun-99 |
| | | GB_BA2:AF092918 | 20758 | AF092918 | Pseudomonas alcaligenes outer membrane Xcp-secretion system gene cluster. | Pseudomonas alcaligenes | 50,161 | 06-DEC-1998 |
| rxa00403 | 1254 | GB_BA2:AF052652 | 2096 | AF052652 | Corynebacterium glutamicum homoserine O-acetyltransferase (metA) gene, complete cds. | Corynebacterium glutamicum | 99,920 | 19-MAR-1998 |
| | | GB_BA1:MTV016 | 53662 | AL021841 | Mycobacterium tuberculosis H37Rv complete genome; segment 143/162. | Mycobacterium tuberculosis | 52,898 | 23-Jun-99 |
| | | GB_EST23:AI111288 | 750 | AI111288 | SWOvAMCAQ02A05SK Onchocerca volvulus adult male cDNA (SAW98MLW OvAM) Onchocerca volvulus cDNA clone SWOvAMCAQ02A05 5', mRNA sequence. | Onchocerca volvulus | 37,565 | 31-Aug-98 |
| rxa00405 | 613 | GB_BA1:MTV016 | 53662 | AL021841 | Mycobacterium tuberculosis 1137Rv complete genome; segment 143/162. | Mycobacterium tuberculosis | 57,259 | 23-Jun-99 |
| | | GB_PR4.AC005145 | 143678 | AC005145 | Homo sapiens Xp22-168-169 GSHB-523A23 (Genome Systems Human BAC library) complete sequence. | Homo sapiens | 34,179 | 08-DEC-1998 |
| | | GB_BA1:MTV016 | 53662 | AL021841 | Mycobacterium tuberculosis H37Rv complete genome; segment 143/162. | Mycobacterium tuberculosis | 40,169 | 23-Jun-99 |
| rxa00420 | 1587 | GB_BA1:MTY13D12 | 37085 | Z80343 | Mycobacterium tuberculosis H37Rv complete genome; segment 156/162. | Mycobacterium tuberculosis | 62,031 | 17-Jun-98 |
| | | GB_BA1:MSGY126 | 37164 | AD000012 | Mycobacterium tuberculosis sequence from clone y126. | Mycobacterium tuberculosis | 61,902 | 10-DEC-1996 |
| | | GB_BA1:MSGB971CS | 37566 | L78821 | Mycobacterium leprae cosmid B971 DNA sequence. | Mycobacterium leprae | 39,651 | 15-Jun-96 |
| rxa00435 | 1296 | GB_BA1:AFACBBTZ | 2760 | M68904 | Alcaligenes eutrophus chromsomal transketolase (cbbTc) and phosphoglycolale phosphatase (cbbZc) genes, complete cds. | Ralstonia eutropha | 38,677 | 27-Jul-94 |
| | | GB_HTG4:AC009541 | 169583 | AC009541 | Homo sapiens chromosome 7, *** SEQUENCING IN PROGRESS ***, 25 unordered pieces. | Homo sapiens | 36,335 | 12-OCT-1999 |
| | | GB_HTG4:AC009541 | 169583 | AC009541 | Homo sapiens chromosome 7, *** SEQUENCING IN PROGRESS ***, 25 unordered pieces. | Homo sapiens | 36,335 | 12-OCT-1999 |
| rxa00437 | 579 | GB_PR4:AC005951 | 155450 | AC005951 | Homo sapiens chromosome 17, clone hRPK.372_K_20, complete sequence. | Homo sapiens | 31,738 | 18-Nov-98 |
| | | | | | | | | |
| | | GB_BA1:SC2A11 | 22789 | AL031184 | Streptomyces coelicolor cosmid 2A11. | Streptomyces coelicolor | 43,262 | 5-Aug-98 |
| | | GB_PR4:AC005951 | 155450 | AC005951 | Homo sapiens chromosome 17, clone hRPK.372_K_20, complete sequence. | Homo sapiens | 37,647 | 18-Nov-98 |
| | | | | | | | | |
| rxa00439 | 591 | GB_BA1:MTV016 | 53662 | AL021841 | Mycobacterium tuberculosis H37Rv complete genome; segment 143/162. | Mycobacterium tuberculosis | 37,088 | 23-Jun-99 |
| | | GB_PL2:AF167358 | 1022 | AF167358 | Rumex acetosa expansin (EXP3) gene, partial cds. | Rumex acetosa | 46,538 | 17-Aug-99 |
| | | GB_HTG3:AC009120 | 269445 | AC009120 | Homo sapiens chromosome 16 clone RPCI-11_484E3, *** SEQUENCING IN PROGRESS ***, 34 unordered pieces. | Homo sapiens | 43,276 | 3-Aug-99 |
| rxa00440 | 582 | GB_BA2:SKZ86111 | 7860 | Z86111 | Streptomyces lividans rpsP, trmD, rplS, sipW, sipX, sipY, sipZ, mutT genes and 4 open reading frames. | Streptomyces lividans | 43,080 | 27-OCT-1999 |
| | | GB_BA1:SC2E1 | 38962 | AL023797 | Streptomyces coelicolor cosmid 2E1. | Streptomyces coelicolor | 42,931 | 4-Jun-98 |
| | | GB_BA1:SC2E1 | 38962 | AL023797 | Streptomyces coelicolor cosmid 2E1. | Streptomyces coelicolor | 36,702 | 4-Jun-98 |
| rxa00441 | 1287 | GB_PR2:HS173D1 | 117338 | AL031984 | Human DNA sequence from clone 173D1 on chromosome 1p36.21-36.33.Contains ESTs, STSs and GSSs, complete sequence. | Homo sapiens | 38,027 | 23-Nov-99 |
| | | GB_HTG2:HSDJ719K3 | 267114 | AL109931 | Homo sapiens chromosome X clone RP4-719K3 map q21.1-21.31, *** SEQUENCING IN PROGRESS ***, in unordered pieces. | Homo sapiens | 34,521 | 03-DEC-1999 |
| | | GB_HTG2:HSDJ719K3 | 267114 | AL109931 | Homo sapiens chromosome X clone RP4-719K3 map q21.1-21.31, *** SEQUENCING IN PROGRESS ***, in unordered pieces. | Homo sapiens | 34,521 | 03-DEC-1999 |
| rxa00446 | 987 | GB_BA1:SCD78 | 36224 | AL034355 | Streptomyces coelicolor cosmid D78. | Streptomyces coelicolor | 56,410 | 26-Nov-9B |
| | | GB_HTG4:AC009367 | 226055 | AC009367 | Drosophila melanogaster chromosome 3L/76A2 clone RPCI98-48B15, *** SEQUENCING IN PROGRESS ***, 44 unordered pieces. | Drosophila melanogaster | 34,959 | 16-OCT-1999 |
| | | GB_HTG4:AC009367 | 226055 | AC009367 | Drosophila melanogaster chromosome 3L/76A2 clone RPCI98-48B15, *** SEQUENCING IN PROGRESS ***, 44 unordered pieces. | Drosophila melanogaster | 34,959 | 16-OCT-1999 |
| rxa00448 | 1143 | GB_PR3:AC003670 | 88945 | AC003670 | Homo sapiens 12q13.1 PAC RPCI1-130F5 (Roswell Park Cancer Institute Human PAC library) complete sequence. | Homo sapiens | 35,682 | 9-Jun-98 |
| | | GB_HTG2:AF029367 | 148676 | AF029367 | Homo sapiens chromosome 12 clone RPCI-1 130F5 map 12q13.1, *** SEQUENCING IN PROGRESS ***, 156 unordered pieces. | Homo sapiens | 31,373 | 18-OCT-1997 |
| | | GB_HTG2:AF029367 | 148676 | AF029367 | Homo sapiens chromosome 12 clone RPCI-1 130F5 map 12q13.1, *** SEQUENCING IN PROGRESS ***, 156 unordered pieces. | Homo sapiens | 31,373 | 18-OCT-1997 |
| rxa00450 | 424 | GB_HTG2:AC007824 | 133361 | AC007824 | Drosophila melanogaster chromosome 3 clone BACR02L16 (D715) RPCI-98 02.L.16 map 89E-90A strain y; cn bw sp, *** SEQUENCING IN PROGRESS ***, 91 unordered pieces. | Drosophila melanogaster | 40,000 | 2-Aug-99 |
| | | GB_HTG2:AC007824 | 133361 | AC007824 | Drosophila melanogaster chromosome 3 clone BACR02L16 (D715) RPCI-98 02.L.16 map 89E-90A strain y; cn bw sp, *** SEQUENCING IN PROGRESS ***, 91 unordered pieces. | Drosophila melanogaster | 40,000 | 2-Aug-99 |
| | | GB_EST35:AI818057 | 412 | AI818057 | wk14a08.x1 NCI_CGAP_Lym12 Homo sapiens cDNA clone IMAGE:2412278 3' similar to gb:Y00764 UBIQUINOL-CYTOCHROME C REDUCTASE 11 KD PROTEIN (HUMAN);, mRNA sequence. | Homo sapiens | 35,714 | 24-Aug-99 |
| rxa00461 | 975 | GB_BA1:MLCB1779 | 43254 | Z98271 | Mycobacterium leprae cosmid B1779. | Mycobacterium leprae | 39,308 | 8-Aug-97 |
| | | GB_IN1:DMC86E4 | 29352 | AL021086 | Drosophila melanogaster cosmid clone 86E4. | Drosophila melanogaster | 37,487 | 27-Apr-99 |
| | | GB_GSS15:AQ640325 | 467 | AQ640325 | 927P1-2H3.TP 927P1 Trypanosoma brucei genomic clone 927P1-2H3, genomic survey sequence. | Trypanosoma brucei | 38,116 | 8-Jul-99 |
| rxa00465 | | | | | | | | |
| | 1692 | | | | | | | |
| rxa00487 | | GB_BA1:BAGUAA | 3866 | Y10499 | B.ammoniagenes guaA gene. | Corynebacterium ammoniagenes | 74,259 | 8-Jan-98 |
| | | GB_BA2:U00015 | 42325 | U00015 | Mycobacterium leprae cosmid 81620. | Mycobacterium leprae | 37,248 | 01-MAR-1994 |
| | | GB_BA1:MTCY78 | 33818 | Z77165 | Mycobacterium tuberculosis H37Rv complete genome; segment 145/162. | Mycobacterium tuberculosis | 39,725 | 17-Jun-98 |
| rxa00488 | 1641 | GB_BA1:MTCY78 | 33818 | Z77165 | Mycobacterium tuberculosis H37Rv complete genome; segment 145/162. | Mycobacterium tuberculosis | 39,451 | 17-Jun-98 |
| | | GB_BA2:U00015 | 42325 | U00015 | Mycobacterium leprae cosmid B1620. | Mycobacterium leprae | 39,178 | 01-MAR-1994 |
| | | GB_BA1:SCAJ10601 | 4692 | AJ010601 | Streptomyces coelicolor A3(2) DNA for whiD and whiK loci. | Streptomyces coelicolor | 60,835 | 17-Sep-98 |
| rxa00489 | 1245 | GB_BA2:U00015 | 42325 | U00015 | Mycobacterium leprae cosmid B1620. | Mycobacterium leprae | 38,041 | 01-MAR-1994 |
| | | GB_HTG2:HS225E12 | 126464 | AL031772 | Homo sapiens chromosome 6 clone RP1-225E12 map q24, *** SEQUENCING IN PROGRESS ***, in unordered pieces. | Homo sapiens | 36,756 | 03-DEC-1999 |
| | | GB_HTG2:HS225E12 | 126464 | AL031772 | Homo sapiens chromosome 6 clone RP1-225E12 map q24, *** SEQUENCING IN PROGRESS ***, in unordered pieces. | Homo sapiens | 36,756 | 03-DEC-1999 |
| rxa00533 | 1155 | GB_BA1:CGLYS | 2803 | X57226 | C. glutamicum lysC-alpha, lysC-beta and asd genes for aspartokinase-alpha and -beta subunits, and aspartate beta semialdehyde dehydrogenase, respectively (EC 2.7.2.4; EC 1.2.1.11). | Corynebacterium glutamicum | 99,913 | 17-Feb-97 |
| | | GB_BA1:CGCYSCASD | 1591 | X82928 | C.glutamicum aspartate-semialdehyde dehydrogenase gene. | Corynebacterium glutamicum | 99,221 | 17-Feb-97 |
| | | | | | | | | |
| | | GB_PAT:A07546 | 2112 | A07546 | Recombinant DNA fragment (Pstl-Xhol). | synthetic construct | 99,391 | 30-Jul-93 |
| rxa00534 | 1386 | GB_BA1:CGLYS | 2803 | X57226 | C. glutamicum lysC-alpha, lysC-beta and asd genes for aspartokinase-alpha and -beta subunits, and aspartate beta semialdehyde dehydrogenase, respectively (EC 2.7.2.4; EC 1.2.1.11). | Corynebacterium glutamicum | 99,856 | 17-Feb-97 |
| | | GB_BA1:CORASKD | 2957 | L16848 | Corynebacterium flavum aspartokinase (ask), and aspartate-semialdehyde dehydrogenase (asd) genes, complete cds. | Corynebacterium flavescens | 98,701 | 11-Jun-93 |
| | | GB_PAT:E14514 | 1643 | E14514 | DNA encoding Brevibacterium aspartokinase. | Corynebacterium glutamicum | 98,773 | 28-Jul-99 |
| rxa00536 | 1494 | GB_BA1:CGLEUA | 3492 | X70959 | C.glutamicum gene leuA for isopropylmalate synthase. | Corynebacterium glutamicum | 100,000 | 10-Feb-99 |
| | | GB_BA1:MTV025 | 121125 | AL022121 | Mycobacterium tuberculosis H37Rv complete genome; segment 155/162. | Mycobacterium tuberculosis | 68,003 | 24-Jun-99 |
| | | GB_BA1:MTU88526 | 2412 | U88526 | Mycobacterium tuberculosis putative alpha-isopropyl malate synthase (leuA) gene, complete cds. | Mycobacterium tuberculosis | 68,185 | 26-Feb-97 |
| rxa00537 | 2409 | GB_BA2:SCD25 | 41622 | AL118514 | Streptomyces coelicolor cosmid D25. | Streptomyces coelicolor A3(2) | 63,187 | 21-Sep-99 |
| | | | | | | | | |
| | | GB_BA1:MTCY7H7A | 10451 | Z95618 | Mycobacterium tuberculosis H37Rv complete genome; segment 39/162. | Mycobacterium tuberculosis | 62,401 | 17-Jun-98 |
| | | GB_BA1:MTU34956 | 2462 | U34956 | Mycobacterium tuberculosis phosphoribosylformylglycinamidine synthase (purL) gene, complete cds. | Mycobacterium tuberculosis | 62,205 | 28-Jan-97 |
| rxa00541 | 792 | GB_PAT:I92052 | 2115 | I92052 | Sequence 19 from patent US 5726299. | Jnknown. | 98,359 | 01-DEC-1998 |
| | | GB_BA1:MLCB5 | 38109 | Z95151 | Mycobacterium leprae cosmid B5. | Mycobacterium leprae | 62,468 | 24-Jun-97 |
| | | GB_BA1:MTCY369 | 36850 | Z80226 | Mycobacterium tuberculosis H37Rv complete genome; segment 36/162. | Mycobacterium tuberculosis | 60,814 | 17-Jun-98 |
| rxa00558 | 1470 | GB_BA1:BAPURF | 1885 | X91252 | B.ammoniagenes purF gene. | Corynebacterium ammoniagenes | 66,095 | 5-Jun-97 |
| | | GB_BA1:MLU15182 | 40123 | U15182 | Mycobacterium leprae cosmid B2266. | Mycobacterium leprae | 64,315 | 09-MAR-1995 |
| | | GB_BA1:MTCY7H7A | 10451 | Z95618 | Mycobacterium tuberculosis H37Rv complete genome; segment 39/162. | Mycobacterium tuberculosis | 64,863 | 17-Jun-98 |
| rxa00579 | 1983 | GB_PAT:AR016483 | 2104 | AR016483 | Sequence 1 from patent US 5776740. | Jnknown. | 98,810 | 05-DEC-1998 |
| | | EM_PAT:E11273 | 2104 | E11273 | DNA encoding serine hydroxymethyl transferase. | Corynebacterium glulamicum | 98,810 | 08-OCT-1997 |
| | | | | | | | | (Rel. 52, Created) |
| | | GB_PAT:E12594 | 2104 | E12594 | DNA encoding serine hydroxymethyltransferase from Brevibacterium flavum. | Corynebacterium glutamicum | 98,810 | 24-Jun-98 |
| rxa00580 | 1425 | GB_PAT:E12594 | 2104 | E12594 | DNA encoding serine hydroxymethyltransferase from Brevibacterium flavum. | Corynebacterium glutamicum | 99,368 | 24-Jun-98 |
| | | GB_PAT:AR016483 | 2104 | AR016483 | Sequence 1 from patent US 5776740. | Unknown. | 99,368 | 05-DEC-1998 |
| | | EM_PAT:E11273 | 2104 | E11273 | DNA encoding serine hydroxymethyl transferase. | Corynebacterium glutamicum | 99,368 | 08-OCT-1997 |
| | | | | | | | | (Rel. 52, Created) |
| rxa00581 | 1092 | GB_PAT:E12594 | 2104 | E12594 | DNA encoding serine hydroxymethyltransferase from Brevibacterium flavum. | Corynebacterium glutamicum | 37,071 | 24-Jun-98 |
| | | EM_PAT:E11273 | 2104 | E11273 | DNA encoding serine hydroxymethyl transferase. | Corynebacterium glutamicum | 37,071 | 08-OCT-1997 |
| | | | | | | | | (Rel. 52, Created) |
| | | GB_PAT:AR016483 | 2104 | AR016483 | Sequence 1 from patent US 5776740. | Unknown. | 37,071 | 05-DEC-1998 |
| rxa00584 | 1248 | GB_BA1:CORAHPS | 2570 | L07603 | Corynebacterium glutamicum 3-deoxy-D-arabinoheptulosonate-7-phosphate synthase gene, complete cds. | Corynebacterium glutamicum | 98,236 | 26-Apr-93 |
| | | GB_BA1:AOPCZA361 | 37941 | AJ223998 | Amycolatopsis orientalis cosmid PCZA361. | Amycolatopsis orientalis | 54,553 | 29-MAR-1999 |
| | | GB_BA1:D90714 | 14358 | D90714 | Escherichia coli genomic DNA. (16.8 - 17.1 min). | Escherichia coli | 53,312 | 7-Feb-99 |
| rxa00618 | 1230 | GB_EST19:AA802737 | 280 | AA802737 | GM06236.5prime GM Drosophila melanogaster ovary BlueScript Drosophila melanogaster cDNA clone GM06236 5prime, mRNA sequence. | Drosophila melanogaster | 39,928 | 25-Nov-98 |
| | | GB_EST28:AI534381 | 581 | AI534381 | SD07186.5prime SD Drosophila melanogaster Schneider L2 cell culture pOT2 Drosophila melanogaster cDNA clone SD07186 5prime similar to X89858: Ani FBgn0011558 PID:g927407 SPTREMBL:Q24240, mRNA sequence. | Drosophila melanogaster | 41,136 | 18-MAR-1999 |
| | | | | | | | | |
| | | GB_IN1:DMANILLIN | 4029 | X89858 | D.melanogaster mRNA for anillin protein. | Drosophila melanogaster | 34,398 | 8-Nov-95 |
| rxa00619 | 1551 | GB_BA1:MTCY369 | 36850 | Z80226 | Mycobacterium tuberculosis H37Rv complete genome; segment 36/162. | Mycobacterium tuberculosis | 62,776 | 17-Jun-98 |
| | | GB_BA1:MLCB5 | 38109 | Z95151 | Mycobacterium leprae cosmid B5. | Mycobacterium leprae | 61,831 | 24-Jun-97 |
| | | GB_PAT:A60305 | 1845 | A60305 | Sequence 5 from Patent WO9708323. | unidentified | 61,785 | 06-MAR-1998 |
| rxa00620 | 1014 | GB_PL2:AF063247 | 1450 | AF063247 | Pneumocystis carinii f. sp. ratti enolase mRNA, complete cds. | Pneumocystis carinii f. sp. ratti ti | 41,060 | 5-Jan-99 |
| | | | | | | | | |
| | | GB_BA1:STMAPP | 2069 | M91546 | Streptomyces lividans aminopeptidase P (PepP) gene, complete cds. | Streptomyces lividans | 37,126 | 12-Jun-93 |
| | | GB_HTG3:AC008763 | 214575 | AC008763 | Homo sapiens chromosome 19 clone CITB-E1_3214H19, *** SEQUENCING IN PROGRESS ***, 21 unordered pieces. | Homo sapiens | 40,020 | 3-Aug-99 |
| rxa00624 | 810 | GB_IN1:CEY41E3 | 150641 | Z95559 | Caenorhabditis elegans cosmid Y41E3, complete sequence. | Caenorhabditis elegans | 36,986 | 2-Sep-99 |
| | | GB_EST13:AA362167 | 372 | AA362167 | EST71561 Macrophage I Homo sapiens cDNA 5' end, mRNA sequence. | Homo sapiens | 38,378 | 21-Apr-97 |
| | | GB_IN1:CEY41E3 | 150641 | Z95559 | Caenorhabditis elegans cosmid Y41 E3, complete sequence. | Caenorhabditis elegans | 37,694 | 2-Sep-99 |
| rxa00626 | 1386 | GB_BA1:MTCY369 | 36850 | Z80226 | Mycobacterium tuberculosis H37Rv complete genome; segment 36/162. | Mycobacterium tuberculosis | 57,971 | 17-Jun-98 |
| | | GB_BA1:MLCB5 | 38109 | Z95151 | Mycobacterium leprae cosmid B5. | Mycobacterium leprae | 58,806 | 24-Jun-97 |
| | | GB_BA1:MLU15187 | 36138 | U15187 | Mycobacterium leprae cosmid L296. | Mycobacterium leprae | 38,007 | 09-MAR-1995 |
| rxa00632 | 795 | GB_BA1:BRLBIOAD | 2272 | D14083 | Brevibacterium flavum genes for 7,8-diaminopelargonic acid aminotransferase and dethiobiotin synthetase, complete cds. | Corynebacterium glutamicum | 97,358 | 3-Feb-99 |
| | | GB_PAT:E04041 | 675 | E04041 | DNA sequence coding for desthiobiotinsynthetase. | Corynebacterium glutamicum | 98,074 | 29-Sep-97 |
| | | GB_PAT:E04040 | 1272 | E04040 | DNA sequence coding for diamino pelargonic acid aminotransferase. | Corynebacterium glutamicum | 93,814 | 29-Sep-97 |
| rxa00633 | 1392 | GB_BA1:BRLBIOAD | 2272 | D14083 | Brevibacterium flavum genes for 7,8-diaminopelargonic acid aminotransferase and dethiobiotin synthetase, complete cds. | Corynebacterium glutamicum | 95,690 | 3-Feb-99 |
| | | GB_PAT:E04040 | 1272 | E04040 | DNA sequence coding for diamino pelargonic acid aminotransferase. | Corynebacterium glutamicum | 95,755 | 29-Sep-97 |
| | | GB_BA2:EHU38519 | 1290 | U38519 | Erwinia herbicola adenosylmethionine-8-amino-7-oxononanoate transaminase (bioA) gene, complete cds. | Erwinia herbicola | 55,564 | 4-Nov-96 |
| rxa00688 | 666 | GB_BA1:MTV041 | 28826 | AL021958 | Mycobacterium tuberculosis H37Rv complete genome; segment 35/162. | Mycobacterium tuberculosis | 60,030 | 17-Jun-98 |
| | | GB_BA1:BRLSECY | 1516 | D14162 | Brevibacterium flavum gene for SecY protein (complete cds) and gene or adenylate kinase (partial cds). | Corynebacterium glutamicum | 99,563 | 3-Feb-99 |
| | | GB_BA2:MBU77912 | 7163 | U77912 | Mycobacterium bovis MBE50a gene, partial cds; and MBE50b, MBE50c, preprotein translocase SecY subunit (secY), adenylate kinase (adk), methionine aminopeptidase (map), RNA polymerase ECF sigma factor (sigE50), MBE50d, and MBE50e genes, complete cds. | Mycobacterium bovis | 60,030 | 27-Jan-99 |
| rxa00708 | 930 | GB_BA2:AF157493 | 25454 | AF157493 | Zymomonas mobilis ZM4 fosmid clone 42D7, complete sequence. | Zymomonas mobilis | 39,116 | 5-Jul-99 |
| | | GB_PAT:I00836 | 1853 | 100836 | Sequence 1 from Patent US 4758514. | Unknown. | 47,419 | 21-MAY-1993 |
| | | GB_PAT:E00311 | 1853 | E00311 | DNA coding of 2,5-diketogluconic acid reductase. | unidentified | 47,419 | 29-Sep-97 |
| rxa00717 | 1083 | GB_PAT:I78753 | 1187 | 178753 | Sequence 9 from patent US 5693781. | Unknown. | 37,814 | 3-Apr-98 |
| | | GB_PAT:I92042 | 1187 | 192042 | Sequence 9 from patent US 5726299. | Unknown. | 37,814 | 01-DEC-1998 |
| | | GB_BA1:MTC1125 | 37432 | Z98268 | Mycobacterium tuberculosis H37Rv complete genome; segment 76/162. | Mycobacterium tuberculosis | 50,647 | 17-Jun-98 |
| rxa00718 | 831 | GB_BA1:MTCI125 | 37432 | Z98268 | Mycobacterium tuberculosis H37Rv complete genome; segment 76/162. | Mycobacterium tuberculosis | 55,228 | 17-Jun-98 |
| | | GB_BA1:MTCI125 | 37432 | Z98268 | Mycobacterium tuberculosis H37Rv complete segment 76/162. | Mycobacterium tuberculosis | 40,300 | 17-Jun-98 |
| | | GB_GSS12:AQ420755 | 671 | AQ420755 | genome; RPCI-11-168G18.TJ RPCI-11 Homo sapiens genomic clone RPCI-11-168G18, genomic survey sequence. | Homo sapiens | 35,750 | 23-MAR-1999 |
| rxa00727 | 1035 | GB_HTG3:AC008332 | 118545 | AC008332 | Drosophila melanogaster chromosome 2 clone BACR48D10 (D867) RPCI-98 48.D.10 map 34A-34A strain y; cn bw sp, *** SEQUENCING IN PROGRESS ***, 78 unordered pieces. | Drosophila melanogaster | 40,634 | 6-Aug-99 |
| | | GB_HTG3:AC008332 | 118545 | AC008332 | Drosophila melanogaster chromosome 2 clone BACR48D10 (D867) RPCI-98 48.D.10 map 34A-34A strain y; cn bw sp, *** SEQUENCING IN PROGRESS***, 78 unordered pieces. | Drosophila melanogaster | 40,634 | 6-Aug-99 |
| | | GB_HTG3:AC008332 | 118545 | AC008332 | Drosophila melanogaster chromosome 2 clone BACR48D10 (D867) RPCI-98 48.D.10 map 34A-34A strain y; cn bw sp, *** SEQUENCING IN PROGRESS***, 78 unordered pieces. | Drosophila melanogaster | 33,888 | 6-Aug-99 |
| rxa00766 | 966 | GB_HTG2:AC006789 | 83823 | AC006789 | Caenorhabditis elegans clone Y49F6, *** SEQUENCING IN PROGRESS ***, 2 unordered pieces. | Caenorhabditis elegans | 36,737 | 25-Feb-99 |
| | | GB_HTG2:AC006789 | 83823 | AC006789 | Caenorhabditis elegans clone Y49F6, *** SEQUENCING IN PROGRESS ***, 2 unordered pieces. | Caenorhabditis elegans | 36,737 | 25-Feb-99 |
| | | GB_BA1:D90810 | 20476 | D90810 | E.coli genomic DNA, Kohara clone #319(37.4-37.8 min.). | Escherichia coli | 36,526 | 29-MAY-1997 |
| rxa00770 | 1293 | GB_BA1:MTV043 | 68848 | AL022004 | Mycobacterium tuberculosis H37Rv complete genome; segment 40/162. | Mycobacterium tuberculosis | 66,193 | 24-Jun-99 |
| | | GB_BA1:MLU15182 | 40123 | U15182 | Mycobacterium leprae cosmid B2266. | Mycobacterium leprae | 61,443 | 09-MAR-1995 |
| | | GB_BA2:SCD25 | 41622 | AL118514 | Streptomyces coelicolor cosmid D25. | Streptomyces coelicolor A3(2) | 59,938 | 21-Sep-99 |
| | | | | | | | | |
| rxa00779 | 1056 | GB_HTG1:CER08A5 | 51920 | Z82281 | Caenorhabditis elegans chromosome V clone R08A5, *** SEQUENCING IN PROGRESS ***, in unordered pieces. | Caenorhabditis elegans | 64,896 | 14-OCT-1998 |
| | | GB_HTG1:CER08A5 | 51920 | Z82281 | Caenorhabditis elegans chromosome V clone R08A5, *** SEQUENCING IN PROGRESS ***, in unordered pieces. | Caenorhabditis elegans | 64,896 | 14-OCT-1998 |
| | | GB_PL2:AF078693 | 1492 | AF078693 | Chlamydomonas reinhardtii putative O-acetylserine(thiol)lyase precursor (Crcys-1A) mRNA, nuclear gene encoding organellar protein, complete cds. | Chlamydomonas reinhardtii | 57,970 | 3-Nov-99 |
| rxa00780 | 669 | GB_BA1:MTCY98 | 31225 | Z83860 | Mycobacterium tuberculosis H37Rv complete genome; segment 103/162. | Mycobacterium tuberculosis | 54,410 | 17-Jun-98 |
| | | GB_BA1:AVINIFREG | 7099 | M60090 | Azotobacter chroococcum nifU, nifS, nifV, nifP, nitW, nifZ and nifM genes, complete cds. | Azotobacter chroococcum | 51,729 | 26-Apr-93 |
| | | GB_BA2:AF001780 | 6701 | AF001780 | Cyanothece PCC 8801 NifP (nifP), nitrogenase (nifB), FdxN (fdxN), NifS (nifS) and NifU (nifU) genes, complete cds, and NifH (nifH) gene, partial cds. | Cyanothece PCC8801 | 36,309 | 08-MAR-1999 |
| rxa00838 | 1023 | GB_EST1:Z30506 | 329 | Z30506 | ATTS2430 AC16H Arabidopsis thaliana cDNA clone TAI306 3', mRNA sequence. | Arabidopsis thaliana | 44,308 | 11-MAR-1994 |
| | | GB_PL2:AC006258 | 110469 | AC006258 | Arabidopsis thaliana BAC F18G18 from chromosome V near 60.5 cM, complete sequence. | Arabidopsis thaliana | 35,571 | 28-DEC-1998 |
| | | GB_EST37:AI998439 | 455 | AI998439 | 701545695 A. thaliana, Columbia Col-0, rosette-2 Arabidopsis thaliana cDNA clone 701545695, mRNA sequence. | Arabidopsis thaliana | 36,044 | 8-Sep-99 |
| rxa00863 | 867 | GB_BA1:BLDAPAB | 3572 | Z21502 | B.lactofermentum dapA and dapB genes for dihydrodipicolinate synthase and dihydrodipicolinate reductase. | Corynebacterium glutamicum | 99,539 | 16-Aug-93 |
| | | GB_PAT:E16749 | 2001 | E16749 | gDNA encoding dihydrodipicolinate synthase (DDPS). | Corynebacterium glutamicum | 99,539 | 28-Jul-99 |
| | | GB_PAT:E14520 | 2001 | E14520 | DNA encoding Brevibacterium dihydrodipicolinic acid synthase. | Corynebacterium glutamicum | 99,539 | 28-Jul-99 |
| rxa00864 | 873 | GB_BA1:BLDAPAB | 3572 | Z21502 | B.lactofermentum dapA and dapB genes for dihydrodipipolinate synthase and dihydrodipicolinate reductase. | Corynebacterium glutamicum | 99,885 | 16-Aug-93 |
| | | GB_BA1:CGDAPB | 1902 | X67737 | C.glutamicum dapB gene for dihydrodipicolinate reductase. | Corynebacterium glutamicum | 100,000 | 1-Apr-93 |
| | | GB_PAT:E14520 | 2001 | E14520 | DNA encoding Brevibacterium dihydrodipicolinic acid synthase. | Corynebacterium glutamicum | 100,000 | 28-Jul-99 |
| rxa00865 | 1026 | GB_BA1:BLDAPAB | 3572 | Z21502 | B.lactofermentum dapA and dapB genes for dihydrodipicolinate synthase and dihydrodipicolinate reductase. | Corynebacterium glutamicum | 100,000 | 16-Aug-93 |
| | | GB_PAT:E16752 | 1411 | E16752 | gDNA encoding dihydrodipicolinale reductase (DDPR). | Corynebacterium glutamicum | 99,805 | 28-Jul-99 |
| | | GB_PAT:AR038113 | 1411 | AR038113 | Sequence 18 from patent US 5804414. | Unknown. | 99,805 | 29-Sep-99 |
| rxa00867 | 650 | GB_BA1:MTV002 | 56414 | AL008967 | Mycobacterium tuberculosis H37Rv complete genome; segment 122/162. | Mycobacterium tuberculosis | 39,179 | 17-Jun-98 |
| | | GB_BA1:MLCB22 | 40281 | Z98741 | Mycobacterium leprae cosmid B22. | Mycobacterium leprae | 39,482 | 22-Aug-97 |
| | | GB_BA1:SAU19858 | 2838 | U19858 | Streptomyces antibioticus guanosine pentaphosphate synthetase (gpsl) gene, complete cds. | Streptomyces antibioticus | 69,706 | 25-OCT-1996 |
| rxa00873 | 779 | GB_BA1:SCO001206 | 9184 | AJ001206 | Streptomyces coelicolor A3(2), glycogen metabolism cluster II. | Streptomyces coelicolor | 63,415 | 29-MAR-1999 |
| | | GB_BA1:SCO001205 | 9589 | AJ001205 | Streptomyces coelicolor A3(2) glycogen metabolism clusterI. | Streptomyces coelicolor | 61,617 | 29-MAR-1999 |
| | | GB_BA1:D78198 | 2304 | D78198 | Pimelobacter sp. DNA for trehalose synthase, complete cds. | Pimelobacter sp. | 60,594 | 5-Feb-99 |
| rxa00884 | 1263 | GB_BA1:MTCY253 | 41230 | Z81368 | Mycobacterium tuberculosis H37Rv complete genome; segment 106/162. | Mycobacterium tuberculosis | 37,785 | 17-Jun-98 |
| | | GB_BA1:MSGY222 | 41156 | AD000010 | Mycobacterium tuberculosis sequence from clone y222. | Mycobacterium tuberculosis | 38,006 | 03-DEC-1996 |
| | | GB_GSS15:AQ654600 | 468 | AQ654600 | Sheared DNA-1014.TF Sheared DNA Trypanosoma brucei genomic clone Sheared DNA-1014, genomic survey sequence. | Trypanosoma brucei | 33,974 | 22-Jun-99 |
| rxa00891 | 1102 | GB_BA1:MTCI418B | 11700 | Z96071 | Mycobacterium tuberculosis H37Rv complete genome; segment 7/162. | Mycobacterium tuberculosis | 63,297 | 18-Jun-98 |
| | | GB_BA1:SCO001206 | 9184 | AJ001206 | Streptomyces coelicolor A3(2), glycogen metabolism cluster II. | Streptomyces coelicolor | 61,965 | 29-MAR-1999 |
| | | GB_BA1:SCO001205 | 9589 | AJ001205 | Streptomyces coelicolor A3(2) glycogen metabolism cluster I. | Streptomyces coelicolor | 61,727 | 29-MAR-1999 |
| rxa00952 | 963 | EM_PAT:E10963 | 3118 | E10963 | gDNA encoding tryptophan synthase. | Corynebacterium glutamicum | 99,688 | 08-OCT-1997 |
| | | | | | | | | (Rel. 52, Created) |
| | | GB_BA1:BLTRP | 7725 | X04960 | Brevibacterium lactofermentum tryptophan operon. | Corynebacterium glutamicum | 98,847 | 10-Feb-99 |
| | | GB_PAT:E01688 | 7725 | E01688 | Genomic DNA of trp operon of prepibacterium latopheimentamn. | unidentified | 98,428 | 29-Sep-97 |
| rxa00954 | 644 | GB_PAT:E01375 | 7726 | E01375 | DNA sequence of tryptophan operon. | Corynebacterium glutamicum | 98,758 | 29-Sep-97 |
| | | GB_PAT:E01688 | 7725 | E01688 | Genomic DNA of trp operon of prepibacterium latophelmentamn. | unidentified | 98,758 | 29-Sep-97 |
| | | GB_BA1:BLTRP | 7725 | X04960 | Brevibacterium lactofermentum tryptophan operon. | Corynebacterium glutamicum | 98,758 | 10-Feb-99 |
| rxa00955 | 1545 | GB_PAT:E01375 | 7726 | E01375 | DNA sequence of tryptophan operon. | Corynebacterium glutamicum | 98,372 | 29-Sep-97 |
| | | GB_BA1:BLTRP | 7725 | X04960 | Brevibacterium lactofermentum tryptophan operon. | Corynebacterium glutamicum | 98,372 | 10-Feb-99 |
| | | GB_PAT:E01688 | 7725 | E01688 | Genomic DNA of trp operon of prepibacterium latophelmentamn. | unidentified | 98,242 | 29-Sep-97 |
| rxa00956 | 1237 | EM_PAT:E10963 | 3118 | E10963 | gDNA encoding tryptophan synthase. | Corynebacierium glutamicum | 98,949 | 08-OCT-1997 |
| | | | | | | | | (Rel. 52, Created) |
| | | GB_BA1:BLTRP | 7725 | X04960 | Brevibacterium lactofermentum tryptophan operon. | Corynebacterium glutamicum | 99,107 | 10-Feb-99 |
| | | GB_PAT:E01375 | 7726 | E01375 | DNA sequence of tryptophan operon. | Corynebacterium glutamicum | 98,945 | 29-Sep-97 |
| rxa00957 | 1677 | GB_BA1:BLTRP | 7725 | X04960 | Brevibacterium lactofermentum tryptophan operon. | Corynebacterium glutamicum | 99,165 | 10-Feb-99 |
| | | GB_PAT:E01375 | 7726 | E01375 | DNA sequence of tryptophan operon. | Corynebacterium glutamicum | 98,927 | 29-Sep-97 |
| | | GB_PAT:E01688 | 7725 | E01688 | Genomic DNA of trp operon of prepibacterium latophelmentamn. | unidentified | 98,867 | 29-Sep-97 |
| rxa00958 | 747 | GB_BA1:BLTRP | 7725 | X04960 | Brevibacterium lactofermentum tryptophan operon. | Corynebacterium glutamicum | 98,792 | 10-Feb-99 |
| | | GB_PAT:E01375 | 7726 | E01375 | DNA sequence of tryptophan operon. | Corynebacterium glutamicum | 98,792 | 29-Sep-97 |
| rxa00970 | 1050 | GB_PAT:E01688 | 7725 | E01688 | Genomic DNA of trp operon of prepibacterium latophelmentamn. | unidentified | 98,658 | 29-Sep-97 |
| | | GB_BA1:CGHOMTHR | 3685 | Y00546 | Corynebacterium glutamicum hom-thrB genes for homoserine dehydrogenase and homoserine kinase. | Corynebacterium glutamicum | 99,905 | 12-Sep-93 |
| | | | | | | | | |
| | | GB_PAT:I09077 | 3685 | 109077 | Sequence 1 from Patent WO 8809819. | Unknown. | 99,810 | 02-DEC-1994 |
| | | GB_PAT:E01358 | 2615 | E01358 | DNA encoding for homoserine dehydrogenase(HDH)and homoserine kinase(HK). | Corynebacterium glutamicum | 97,524 | 29-Sep-97 |
| rxa00972 | 1458 | GB_PAT:E16755 | 3579 | E16755 | gDNA encoding diaminopimelate decarboxylase (DDC) and arginyl-tRNA synthase. | Corynebacterium glutamicum | 99,931 | 28-Jul-99 |
| | | GB_PAT:AR038110 | 3579 | AR038110 | Sequence 15 from patent US 5804414. | Unknown. | 99,931 | 29-Sep-99 |
| | | GB_PAT:E14508 | 3579 | E14508 | DNA encoding Brevibacterium diaminopimelic acid decarboxylase and arginyl-tRNA synthase. | Corynebacterium glutamicum | 99,931 | 28-Jul-99 |
| rxa00981 | 753 | GB_OV:GGA245664 | 512 | AJ245664 | Gallus gallus partial mRNA for ATP-citrate lyase (ACL gene). | Gallus gallus | 37,538 | 28-Sep-99 |
| | | GB_PL2:AC007887 | 159434 | AC007887 | Genomic sequence for Arabidopsis thaliana BAC F15O4 from chromosome I, complete sequence. | Arabidopsis thaliana | 37,600 | 04-OCT-1999 |
| | | GB_GSS1:CNS00RNW | 542 | AL087338 | Arabidopsis thaliana genome survey sequence T7 end of BAC F14D7 of IGF library from strain Columbia of Arabidopsis thaliana, genomic survey sequence. | Arabidopsis thaliana | 41,264 | 28-Jun-99 |
| rxa00989 | 1644 | GB_BA1:MTV008 | 63033 | AL021246 | Mycobacterium tuberculosis H37Rv complete genome; segment 108/162. | Mycobacterium tuberculosis | 40,773 | 17-Jun-98 |
| | | GB_BA1:SCVALSFP | 3619 | Y13070 | S.coelicolor valS, fpgs, ndk genes. | Streptomyces coelicolor | 58,119 | 03-MAR-1998 |
| | | GB_BA1:MTV008 | 63033 | AL021246 | Mycobacterium tuberculosis H37Rv complete genome; segment 108/162. | Mycobacterium tuberculosis | 38,167 | 17-Jun-98 |
| rxa00997 | 705 | GB_BA2:CGU31225 | 1817 | U31225 | Corynebacterium glutamicum L-proline:NADP+ 5-oxidoreductase (proC) gene, complete cds. | Corynebacterium glutamicum | 40,841 | 2-Aug-96 |
| | | GB_HTG1:CEY39C12 | 282838 | AL009026 | Caenorhabditis elegans chromosome IV clone Y39C12, *** SEQUENCING IN PROGRESS *** in unordered pieces. | Caenorhabditis elegans | 36,416 | 26-OCT-1999 |
| | | GB_IN1:CEB0001 | 39416 | Z69634 | Caenorhabditis elegans cosmid B0001, complete sequence. | Caenorhabditis elegans | 36,416 | 2-Sep-99 |
| rxa01019 | 1110 | GB_HTG2:AC005052 | 144734 | AC005052 | Homo sapiens clone RG038K21, *** SEQUENCING IN PROGRESS ***, 3 unordered pieces. | Homo sapiens | 39,172 | 12-Jun-98 |
| | | GB_HTG2:AC005052 | 144734 | AC005052 | Homo sapiens clone RG038K21, *** SEQUENCING IN PROGRESS***, 3 unordered pieces. | Homo sapiens | 39,172 | 12-Jun-98 |
| | | GB_GSS9:AQ171808 | 512 | AQ171808 | HS_3179_A1_G03_T7 CIT Approved Human Genomic Sperm Library D Homo sapiens genomic clone Plate=3179 Col=5 Row=M, genomic survey sequence. | Homo sapiens | 34,661 | 17-OCT-1998 |
| | | | | | | | | |
| rxa01026 | 1782 | GB_BA1:SC1C2 | 42210 | AL031124 | Streptomyces coelicolor cosmid 1C2. | Streptomyces coelicolor | 68,275 | 15-Jan-99 |
| | | GB_BA1:ATLEUCD | 2982 | X84647 | A.teichomyceticus leuC and leuD genes. | Actinoplanes teichomyceticus | 65,935 | 04-OCT-1995 |
| | | | | | | | | |
| | | GB_BA1:MTV012 | 70287 | AL021287 | Mycobacterium tuberculosis H37Rv complete genome; segment 132/162. | Mycobacterium tuberculosis | 40,454 | 23-Jun-99 |
| rxa01027 | 1131 | GB_BA1:MLCB637 | 44882 | Z99263 | Mycobacterium leprae cosmid B637. | Mycobacterium leprae | 38,636 | 17-Sep-97 |
| | | GB_BA1:MTCY349 | 43523 | Z83018 | Mycobacterium tuberculosis H37Rv complete genome; segment 131/162. | Mycobacterium tuberculosis | 51,989 | 17-Jun-98 |
| | | GB_BA1:SPUNGMUTX | 1172 | Z21702 | S.pneumoniae ung gene and mutX genes encoding uracil-DNA glycosylase and 8-oxodGTP nucleoside triphosphatase. | Streptococcus pneumoniae | 38,088 | 15-Jun-94 |
| rxa01073 | 954 | GB_BA1:BACOUTB | 1004 | M15811 | Bacillus subtilis outB gene encoding a sporulation protein, complete cds. | Bacillus subtilis | 53,723 | 26-Apr-93 |
| | | GB_PR4:AC007938 | 167237 | AC007938 | Homo sapiens clone UWGC:djs201 from 7q31, complete sequence. | Homo sapiens | 34,322 | 1-Jul-99 |
| | | GB_PL2:ATAC006282 | 92577 | AC006282 | Arabidopsis thaliana chromosome II BAC F13K3 genomic sequence, complete sequence. | Arabidopsis thaliana | 36,181 | 13-MAR-1999 |
| rxa01079 | 2226 | GB_BA2:AF112535 | 4363 | AF112535 | Corynebacterium glutamicum putative glularedoxin NrdH (nrdH), Nrdl (nrdl), and ribonucleotide reductase alpha-chain (nrdE) genes, complete cds. | Corynebacterium glutamicum | 99,820 | 5-Aug-99 |
| | | GB_BA1:CANRDFGEN | 6054 | Y09572 | Corynebacterium ammoniagenes nrdH, nrdI, nrdE, nrdF genes. | Corynebacterium ammoniagenes | 75,966 | 18-Apr-98 |
| | | GB_BA1:MTV012 | 70287 | AL021287 | Mycobacterium tuberculosis H37Rv complete genome; segment 132/162. | Mycobacterium tuberculosis | 38,296 | 23-Jun-99 |
| rxa01080 | 567 | GB_BA2:AF112535 | 4363 | AF112535 | Corynebacterium glutamicum putative glutaredoxin NrdH (nrdH), Nrdl (nrdl), and ribonucleotide reductase alpha-chain (nrdE) genes, complete cds. | Corynebacterium glutamicum | 100,000 | 5-Aug-99 |
| | | GB_BA1:CANRDFGEN | 6054 | Y09572 | Corynebacterium ammoniagenes nrdH, nrdI, nrdE, nrdF genes. | Corynebacterium | 65,511 | 18-Apr-98 |
| | | | | | | ammoniagenes | | |
| | | GB_BA1:STNRD | 4894 | X73226 | S.typhimurium nrdEF operon. | Salmonella typhimurium | 52,477 | 03-MAR-1997 |
| rxa01087 | 999 | GB_IN2:AF063412 | 1093 | AF063412 | Limnadia lenticularis elongation factor 1-alpha mRNA, partial cds. | Limnadia lenticularis | 43,750 | 29-MAR-1999 |
| | | GB_PR3:HS24M15 | 134539 | Z94055 | Human DNA sequence from PAC 24M15 on chromosome 1. Contains | Homo sapiens | 37,475 | 23-Nov-99 |
| | | | | | tenascin-R (restrictin), EST. | | | |
| | | GB_IN2:ARU85702 | 1240 | U85702 | Anathix ralla elongation factor-1 alpha (EF-1a) gene, partial cds. | Anathix ralla | 37,319 | 16-Jul-97 |
| rxa01095 | 857 | GB_BA1:MTCY0182 | 35938 | Z95554 | Mycobacterium tuberculosis H37Rv complete genome; segment 72/162. | Mycobacterium tuberculosis | 43,243 | 17-Jun-98 |
| | | GB_HTG5:AC011632 | 175917 | AC011632 | Homo sapiens clone RP11-3N13, WORKING DRAFT SEQUENCE, 9 unordered pieces. | Homo sapiens | 36,471 | 19-Nov-99 |
| | | GB_HTG5:AC011632 | 175917 | AC011632 | Homo sapiens clone RP11-3N13, WORKING DRAFT SEQUENCE, 9 unordered pieces. | Homo sapiens | 36,836 | 19-Nov-99 |
| rxa01097 | 477 | GB_BA2:AF030405 | 774 | AF030405 | Corynebacterium glutamicum cyclase (hisF) gene, complete cds. | Corynebacterium glutamicum | 100,000 | 13-Nov-97 |
| | | GB_BA2:AF030405 | 774 | AF030405 | Corynebacterium glutamicum cyclase (hisF) gene, complete cds. | Corynebacterium glutamicum | 41,206 | 13-Nov-97 |
| | | | | | | | | |
| rxa01098 | 897 | GB_BA2:AF030405 | 774 | AF030405 | Corynebacterium glutamicum cyclase (hisF) gene, complete cds. | Corynebacterium glutamicum | 97,933 | 13-Nov-97 |
| | | GB_BA1:MSGY223 | 42061 | AD000019 | Mycobacterium tuberculosis sequence from clone y223. | Mycobacterium tuberculosis | 40,972 | 10-DEC-1996 |
| | | GB_BA1:MLCB1610 | 40055 | AL049913 | Mycobacterium leprae cosmid B1610. | Mycobacterium leprae | 61,366 | 27-Aug-99 |
| rxa01100 | 861 | GB_BA2:AF051846 | 738 | AF051846 | Corynebacterium glutamicum phosphoribosylformimino-5-amino-1-phosphoribosyl-4- imidazolecarboxamide isomerase (hisA) gene, complete cds. | Corynebacterium glulamicum | 97,154 | 12-MAR-1998 |
| | | GB_BA2:AF060558 | 636 | AF060558 | Corynebacterium glutamicum glutamine amidotransferase (hisH) gene, complete cds. | Corynebacterium glutamicum | 95,455 | 29-Apr-98 |
| | | GB_HTG1:HSDJ140A9 | 221755 | AL109917 | Homo sapiens chromosome 1 clone RP1-140A9, *** SEQUENCING IN PROGRESS ***, in unordered pieces. | Homo sapiens | 30,523 | 23-Nov-99 |
| rxa01101 | 756 | GB_BA2:AF060558 | 636 | AF060558 | Corynebacterium glutamicum glutamine amidotransferase (hisH) gene, complete cds. | Corynebacterium glutamicum | 94,462 | 29-Apr-98 |
| | | GB_BA1:SC4G6 | 36917 | AL096884 | Streptomyces coelicolor cosmid 4G6. | Streptomyces coelicolor A3(2) | 38,378 | 23-Jul-99 |
| | | | | | | | | |
| | | GB_BA1:STMHISOPA | 3981 | M31628 | S.coelicolor histidine biosynthesis operon encoding hisD, partial cds., and hisC, hisB, hisH, and hisA genes, complete cds. | Streptomyces coelicolor | 60,053 | 26-Apr-93 |
| rxa01104 | 729 | GB_BA1:STMHISOPA | 3981 | M31628 | S.coelicolor histidine biosynthesis operon encoding hisD, partial cds., and hisC, hisB, hisH, and hisA genes, complete cds. | Streptomyces coelicolor | 58,333 | 26-Apr-93 |
| | | GB_BA1:SC4G6 | 36917 | AL096884 | Streptomyces coelicolor cosmid 4G6. | Streptomyces coelicolor A3(2) | 39,045 | 23-Jul-99 |
| | | | | | | | | |
| | | GB_BA1:MTCY336 | 32437 | Z95586 | Mycobacterium tuberculosis H37Rv complete genome; segment 70/162. | Mycobacterium tuberculosis | 60,364 | 24-Jun-99 |
| rxa01105 | 1221 | GB_BA1:MTCY336 | 32437 | Z95586 | Mycobacterium tuberculosis H37Rv complete genome; segment 70/162. | Mycobacterium tuberculosis | 60,931 | 24-Jun-99 |
| | | GB_BA1:MSGY223 | 42061 | AD000019 | Mycobacterium tuberculosis sequence from clone y223. | Mycobacterium tuberculosis | 36,851 | 10-DEC-1996 |
| | | GB_BA1:MLCB1610 | 40055 | AL049913 | Mycobacterium leprae cosmid B1610. | Mycobacterium leprae | 60,902 | 27-Aug-99 |
| rxa01106 | 1449 | GB_BA1:MSGY223 | 42061 | AD000019 | Mycobacterium tuberculosis sequence from clone y223. | Mycobacterium tuberculosis | 37,233 | 10-DEC-1996 |
| | | GB_BA1:MSHISCD | 2298 | X65542 | M.smegmatis genes hisD and hisC for histidinol dehydrogenase and histidinol-phosphate aminotransferase, respectively. - | Mycobacterium smegmatis | 60,111 | 30-Jun-93 |
| | | GB_BA1:MTCY336 | 32437 | Z95586 | Mycobacterium tuberculosis H37Rv complete genome; segment 70/162. | Mycobacterium tuberculosis | 58,420 | 24-Jun-99 |
| rxa01145 | 1137 | GB_BA1:CORAIA | 4705 | L09232 | Corynebacterium glutamicum acetohydroxy acid synthase (ilvB) and (ilvN) genes, and acetohydroxy acid isomeroreductase (ilvC) gene, complete cds. | Corynebacterium glutamicum | 100,000 | 23-Feb-95 |
| | | GB_BA1:BRLILVCA | 1364 | D14551 | Brevibacterium flavum ilvC gene for acetohydroxy acid isomeroreductase, complete cds. | Corynebacterium glutamicum | 99,560 | 3-Feb-99 |
| | | GB_PAT:E08232 | 1017 | E08232 | DNA encoding acetohydroxy-acid isomeroreductase. | Corynebacterium glutamicum | 99,803 | 29-Sep-97 |
| rxa01162 | 1449 | GB_PAT:A60299 | 2869 | A60299 | Sequence 18 from Patent WO9706261. | Aspergillus niger | 38,675 | 06-MAR-1998 |
| | | GB_PR3:HS24E5 | 35506 | Z82185 | Human DNA sequence from Fosmid 24E5 on chromosome 22q11.2-qter contains parvalbumin, ESTs, STS. | Homo sapiens | 36,204 | 23-Nov-99 |
| | | GB_PR3:AC005265 | 43900 | AC005265 | Homo sapiens chromosome 19, cosmid F19750, complete sequence. | Homo sapiens | 38,363 | 6-Jul-98 |
| rxa01208 | 846 | GB_HTG2:AC004965 | 323792 | AC004965 | Homo sapiens clone DJ1106H14, *** SEQUENCING IN PROGRESS ***, 42 unordered pieces. | Homo sapiens | 36,058 | 12-Jun-98 |
| | | GB_HTG2:AC004965 | 323792 | AC004965 | Homo sapiens clone DJ1106H14, *** SEQUENCING IN PROGRESS ***, 42 unordered pieces. | Homo sapiens | 36,058 | 12-Jun-98 |
| | | GB_PL2:TAU55859 | 2397 | U55859 | Triticum aestivum heat shock protein 80 mRNA, complete cds. | Triticum aestivum | 37,269 | 1-Feb-99 |
| rxa01209 | 1528 | GB_HTG3:AC011469 | 113436 | AC011469 | Homo sapiens chromosome 19 clone CIT-HSPC_475D23, *** SEQUENCING IN PROGRESS ***, 31 unordered pieces. | Homo sapiens | 40,000 | 07-OCT-1999 |
| | | GB_HTG3:AC011469 | 113436 | AC011469 | Homo sapiens chromosome 19 clone CIT-HSPC_475D23, *** SEQUENCING IN PROGRESS ***, 31 unordered pieces. | Homo sapiens | 40,000 | 07-OCT-1999 |
| | | GB_PL1:AB010077 | 77380 | AB010077 | Arabidopsis thaliana genomic DNA, chromosome 5, P1 clone: MYH19, complete sequence. | Arabidopsis thaliana | 36,803 | 20-Nov-99 |
| rxa01215 | 1098 | GB_BA1:MTCY10G2 | 38970 | Z92539 | Mycobacterium tuberculosis H37Rv complete genome; segment 47/162. | Mycobacterium tuberculosis | 37,047 | 17-Jun-98 |
| | | GB_IN1:LEIPRPP | 1887 | M76553 | Leishmania donovani phosphoribosylpyrophosphate synthetase gene, complete cds. | Leishmania donovani | 50,738 | 7-Jun-93 |
| | | GB_HTG2:HSJ799D16 | 130149 | AL050344 | Homo sapiens chromosome 1 clone RP4-799D16 map p34.3-36.1, *** SEQUENCING IN PROGRESS ***, in unordered pieces. | Homo sapiens | 38,135 | 29-Nov-99 |
| rxa01239 | 2556 | GB_BA1:MTCY48 | 35377 | Z74020 | Mycobacterium tuberculosis H37Rv complete genome; segment 69/162. | Mycobacterium tuberculosis | 38,139 | 17-Jun-98 |
| | | GB_PR2:AB029032 | 6377 | AB029032 | Homo sapiens mRNA for KIAA1109 protein, partial cds. | Homo sapiens | 39,394 | 4-Aug-99 |
| | | GB_GSS9:AQ107201 | 355 | AQ107201 | HS_309B_A1_C03_T7 CIT Approved Human Genomic Sperm Library D Homo sapiens genomic clone Plate=3098 Col=5 Row=E, genomic survey sequence. | Homo sapiens | 41,408 | 28-Aug-98 |
| | | | | | | | | |
| rxa01253 | 873 | GB_PL2:F508 | 99923 | AC005990 | Arabidopsis thaliana chromosome 1 BAC F5O8 sequence, complete sequence. | Arabidopsis thaliana | 36,118 | 23-DEC-1998 |
| | | GB_PL2:F5O8 | 99923 | AC005990 | Arabidopsis thaliana chromosome 1 BAC F5O8 sequence, complete sequence. | Arabidopsis thaliana | 35,574 | 23-DEC-1998 |
| | | GB_IN1:CELC06G1 | 31205 | U41014 | Caenorhabditis elegans cosmid C06G1. | Caenorhabditis elegans | 38,560 | 30-Nov-95 |
| rxa01321 | 1044 | GB_GSS14:AQ518843 | 441 | AQ518843 | HS_5106_A1_D10_SP6E RPCI-11 Human Male BAC Library Homo sapiens genomic clone Plate=682 Col=19 Row=G, genomic survey sequence. | Homo sapiens | 41,121 | 05-MAY-1999 |
| | | GB_HTG2:AC007473 | 194859 | AC007473 | Drosophila melanogaster chromosome 2 clone BACR38Q12 (D590) RPCI-98 38.D.12 map 48A-488 strain y; cn bw sp, *** SEQUENCING IN PROGRESS ***, 60 unordered pieces. | Drosophila melanogaster | 40,634 | 2-Aug-99 |
| | | GB_HTG4:AC011696 | 115847 | AC011696 | Drosophila melanogaster chromosome 2 clone BACR35F01 (D1156) RPCI-98 35.F.1 map 48A-48C strain y; cn bw sp, *** SEQUENCING IN PROGRESS ***, 108 unordered pieces. | Drosophila melanogaster | 38,290 | 26-OCT-1999 |
| rxa01352 | 706 | GB_PL2:ATAC005167 | 83260 | AC005167 | Arabidopsis thaliana chromosome II BAC F12A24 genomic sequence, complete sequence. | Arabidopsis thaliana | 34,311 | 15-OCT-1998 |
| | | GB_PL2:ATAC005825 | 97380 | AC005825 | Arabidopsis thaliana chromosome II BAC T24I21 genomic sequence, complete sequence. | Arabidopsis thaliana | 34,311 | 12-Apr-99 |
| | | GB_HTG3:AC011150 | 127222 | AC011150 | Homo sapiens clone 4_K_17, LOW-PASS SEQUENCE SAMPLING. | Homo sapiens | 37,722 | 01-OCT-1999 |
| rxa01360 | 259 | GB_EST32:AI725583 | 728 | AI725583 | BNLGHi12371 Six-day Cotton fiber Gossypium hirsutum cDNA 5' similar to (U86081) root hair defective 3 [Arabidopsis thaliana], mRNA sequence. | Gossypium hirsutum | 38,492 | 11-Jun-99 |
| | | GB_PR2:HS22TP17 | 82951 | Z81007 | Human DNA sequence from PAC 227P17, between markers DXS6791 and DXS8038 on chromosome X contains CpG island, EST. | Homo sapiens | 39,738 | 23-Nov-99 |
| | | GB_EST34:AV171099 | 173 | AV171099 | AV171099 Mus musculus head C57BL/6J 14,17 day embryo Mus musculus cDNA clone 3200002M11, mRNA sequence. | Mus musculus | 46,237 | 6-Jul-99 |
| rxa01361 | 629 | GB_RO:AB008915S1 | 530 | AB008915 | Mus musculus mGpi1 gene, exon 1. | Mus musculus | 45,574 | 28-Sep-99 |
| | | GB_EST22:AI050532 | 293 | AI050532 | uc83d10.y1 Sugano mouse kidney mkia Mus musculus cDNA clone IMAGE:1432243 5' similar to TR:O35120 035120 MGPI1P.;, mRNA sequence. | Mus musculus | 44,097 | 9-Jul-98 |
| | | GB_RO:AB008895 | 3062 | AB008895 | Mus musculus mRNA for mGpi1p, complete cds. | Mus musculus | 41,316 | 23-Nov-97 |
| rxa01381 | 944 | GB_PL1:AB005237 | 87835 | AB005237 | Arabidopsis thaliana genomic DNA, chromosome 5, P1 clone: MJJ3, complete sequence. | Arabidopsis thaliana | 36,606 | 20-Nov-99 |
| | | GB_GSS5:AQ766840 | 491 | AQ766840 | HS_2026_A2_C09_T7C CIT Approved Human Genomic Sperm Library D Homo sapiens genomic clone Plate=2026 Col=18 Row=E, genomic survey sequence. | Homo sapiens | 37,916 | 28-Jul-99 |
| | | GB_BA1:MTV043 | 68848 | AL022004 | Mycobacterium tuberculosis H37Rv complete genome; segment 40/162. | Mycobacterium tuberculosis | 37,419 | 24-Jun-99 |
| rxa01393 | 993 | GB_BA1:CGLYSEG | 2374 | X96471 | C.glutamicum lysE and lysG genes. | Corynebacterium glutamicum | 34,831 | 24-Feb-97 |
| | | GB_BA1:SC5A7 | 40337 | AL031107 | Streptomyces coelicolor cosmid 5A7. | Streptomyces coelicolor | 35,138 | 27-Jul-98 |
| | | GB_PR3:AC004054 | 112184 | AC004054 | Homo sapiens chromosome 4 clone B220G8 map 4q21, complete sequence. | Homo sapiens | 37,277 | 9-Jul-98 |
| | | | | | | | | |
| rxa01394 | 822 | GB_BA1:CGLYSEG | 2374 | X96471 | C.glutamicum lysE and lysG genes. | Corynebacterium glutamicum | 100,000 | 24-Feb-97 |
| | | GB_GSS5:AQ769223 | 500 | AQ769223 | HS_3155_B2_G10_T7C CIT Approved Human Genomic Sperm Library D Homo sapiens genomic clone Plate=3155 Col=20 Row=N, genomic survey sequence. | Homo sapiens | 38,400 | 28-Jul-99 |
| | | GB_BA1:CGLYSEG | 2374 | X96471 | C.glutamicum lysE and lysG genes. | Corynebacterium glutamicum | 33,665 | 24-Feb-97 |
| rxa01416 | 630 | GB_BA1:SC3C3 | 31382 | AL031231 | Streptomyces coelicolor cosmid 3C3. | Streptomyces coelicolor | 62,726 | 10-Aug-98 |
| | | GB_BA1:MLCB22 | 40281 | Z98741 | Mycobacterium leprae cosmid B22. | Mycobacterium leprae | 39,159 | 22-Aug-97 |
| | | GB_BA1:MTV002 | 56414 | AL008967 | Mycobacterium tuberculosis H37Rv complete genome; segment 122/162. | Mycobacterium tuberculosis | 37,340 | 17-Jun-98 |
| rxa01442 | 1347 | GB_BA1:D90827 | 18886 | D90827 | E.coli genomic DNA, Kohara clone #336(41.2-41.6 min.). | Escherichia coli | 58,517 | 21-MAR-1997 |
| | | GB_BA1:D90828 | 14590 | D90828 | E.coli genomic DNA, Kohara clone #336gap(41.6-41.9 min.). | Escherichia coli | 56,161 | 21-MAR-1997 |
| | | GB_BA2:AE000279 | 10855 | AE000279 | Escherichia coli K-12 MG1655 section 169 of 400 of the complete genome. | Escherichia coli | 56,021 | 12-Nov-98 |
| rxa01446 | 1413 | GB_BA1:SCH10 | 39524 | AL049754 | Streptomyces coelicolor cosmid H10. | Streptomyces coelicolor | 39,037 | 04-MAY-1999 |
| | | GB_BA1:MTY13E10 | 35019 | Z95324 | Mycobacterium tuberculosis H37Rv complete genome; segment 18/162. | Mycobacterium tuberculosis | 40,130 | 17-Jun-98 |
| | | GB_BA1:MLCB4 | 36310 | AL023514 | Mycobacterium leprae cosmid B4. | Mycobacterium leprae | 37,752 | 27-Aug-99 |
| rxa01483 | 1395 | GB_BA1:MTCY98 | 31225 | Z83860 | Mycobacterium tuberculosis H37Rv complete genome; segment 103/162. | Mycobacterium tuberculosis | 39,057 | 17-Jun-98 |
| | | GB_BA1:MSGB1229CS | 30670 | L78812 | Mycobacterium leprae cosmid B1229 DNA sequence. | Mycobacterium leprae | 54,382 | 15-Jun-96 |
| | | | | | | | | |
| | | GB_BA2:AF027507 | 5168 | AF027507 | Mycobacterium smegmatis dGTPase (dgt), and primase (dnaG) genes, complete cds; tRNA-Asn gene, complete sequence. | Mycobacterium smegmatis | 52,941 | 16-Jan-98 |
| rxa01486 | 757 | GB_BA1:MTV002 | 56414 | AL008967 | Mycobacterium tuberculosis H37Rv complete genome; segment 122/162. | Mycobacterium tuberculosis | 40,941 | 17-Jun-98 |
| | | GB_BA1:MLCB22 | 40281 | Z98741 | Mycobacterium leprae cosmid B22. | Mycobacterium leprae | 38,451 | 22-Aug-97 |
| | | GB_BA1:SC3C3 | 31382 | AL031231 | Streptomyces coelicolor cosmid 3C3. | Streptomyces coelicolor | 61,194 | 10-Aug-98 |
| rxa01489 | 1146 | GB_BA1:CORFADS | 1547 | D37967 | Corynebacterium ammoniagenes gene for FAD synthetase, complete cds. | Corynebacterium ammoniagenes | 58,021 | 8-Feb-99 |
| | | GB_BA1:MLCB22 | 40281 | Z98741 | Mycobacterium leprae cosmid B22. | Mycobacterium leprae | 38,414 | 22-Aug-97 |
| | | GB_BA1:SC10A7 | 39739 | AL078618 | Streptomyces coelicolor cosmid 10A7. | Streptomyces coelicolor | 36,930 | 9-Jun-99 |
| rxa01491 | 774 | GB_BA1:MTV002 | 56414 | AL008967 | Mycobacterium tuberculosis H37Rv complete genome; segment 122/162. | Mycobacterium tuberculosis | 37,062 | 17-Jun-98 |
| | | GB_EST13:AA356956 | 255 | AA356956 | EST65614 Jurkat T-cells III Homo sapiens cDNA 5' end, mRNA sequence. | Homo sapiens | 37,647 | 21-Apr-97 |
| | | GB_OV:OMDNAPROI | 7327 | X92380 | O.mossambicus prolactin I gene. | Tilapia mossambica | 38,289 | 19-OCT-1995 |
| rxa01508 | 1662 | GB_IN1:CEF26C12 | 14653 | Z93380 | Caenorhabditis elegans cosmid F28C12, complete sequence. | Caenorhabditis elegans | 37,984 | 23-Nov-98 |
| | | GB_IN1:CEF28C12 | 14653 | Z93380 | Caenorhabditis elegans cosmid F28C12, complete sequence. | Caenorhabditis elegans | 38,469 | 23-Nov-98 |
| | | | | | | | | |
| rxa01512 | 723 | GB_BA1:SCE9 | 37730 | AL049841 | Streptomyces coelicolor cosmid E9. | Streptomyces coelicolor | 39,021 | 19-MAY-1999 |
| | | GB_BA1:MAU88875 | 840 | U88875 | Mycobacterium avium hypoxanthine-guanine phosphoribosyl transferase gene, complete cds. | Mycobacterium avium | 57,521 | 05-MAR-1997 |
| | | GB_BA1:MTY15C10 | 33050 | Z95436 | Mycobacterium tuberculosis H37Rv complete genome; segment 154/162. | Mycobacterium tuberculosis | 40,086 | 17-Jun-98 |
| rxa01514 | 711 | GB_BA1:MTCY7H7B | 24244 | Z95557 | Mycobacterium tuberculosis H37Rv complete genome; segment 153/162. | Mycobacterium tuberculosis | 43,343 | 18-Jun-98 |
| | | GB_BA1:MLCB2548 | 38916 | AL023093 | Mycobacterium leprae cosmid B2548. | Mycobacterium leprae | 38,177 | 27-Aug-99 |
| | | GB_PL1:EGGTPCHI | 242 | Z49757 | E.gracilis mRNA for GTP cyclohydrolase I (core region). | Euglena gracilis | 64,876 | 20-OCT-1995 |
| rxa01515 | 975 | GB_BA1:ECOUW93 | 338534 | U14003 | Escherichia coli K-12 chromosomal region from 92.8 to 00.1 minutes. | Escherichia coli | 38,943 | 17-Apr-96 |
| | | G8_BA1:ECOUW93 | 338534 | U14003 | Escherichia coli K-12 chromosomal region from 92.8 to 00.1 minutes. | Escherichia coli | 37,500 | 17-Apr-96 |
| | | GB_BA1:MTCY49 | 39430 | Z73966 | Mycobacterium tuberculosis H37Rv complete genome; segment 93/162. | Mycobacterium tuberculosis | 38,010 | 24-Jun-99 |
| rxa01516 | 513 | GB_IN1:DME238847 | 5419 | AJ238847 | Drosophila melanogaster mRNA for drosophila dodeca-satellite protein 1 (DDP 1). | Drosophila melanogaster | 36,346 | 13-Aug-99 |
| | | GB_HTG3:AC009210 | 103814 | AC009210 | Drosophila melanogaster chromosome 2 clone BACR01I06 (D1054) RPCI-98 01.1.6 map 55D-55D strain y; cn bw sp, *** SEQUENCING IN PROGRESS***, 86 unordered pieces. | Drosophila melanogaster | 37,897 | 20-Aug-99 |
| | | GB_IN2:AF132179 | 4842 | AF132179 | Drosophila melanogaster clone LD21677 unknown mRNA. | Drosophila melanogaster | 36,149 | 3-Jun-99 |
| rxa01517 | 600 | GB_PL2:F6H8 | 82596 | AF178045 | Arabidopsis thaliana BAC F6H8. | Arabidopsis thaliana | 35,846 | 19-Aug-99, |
| | | GB_PL2:AF038831 | 647 | AF038831 | Sorosporium saponariae internal transcribed spacer 1,5.8S ribosomal RNA gene; and internal transcribed spacer 2, complete sequence. | Sorosporium saponariae | 40,566 | 13-Apr-999 |
| | | GB_PL2:ATAC005957 | 108355 | AC005957 | Arabidopsis thaliana chromosome II BAC T15J14 genomic sequence, complete sequence. | Arabidopsis thaliana | 38,095 | 7-Jan-99 |
| rxa01521 | 921 | GB_BA1:ANANIFBH | 5936 | J05111 | Anabaena sp. (clone AnH20.1) nitrogen fixation operon nifB, fdxN, nifS, nifU, and nifH genes, complete cds. | Anabaena sp. | 38,206 | 26-Apr-93 |
| | | GB_PR2:AC002461 | 197273 | AC002461 | Human BAC clone RG204I16 from 7q31, complete sequence. | Homo sapiens | 36,623 | 20-Aug-97 |
| | | GB_PR2:AC002461 | 197273 | AC002461 | Human BAC clone RG204I16 from 7q31, complete sequence. | Homo sapiens | 34,719 | 20-Aug-97 |
| rxa01528 | 651 | GB_RO:MM437P9 | 165901 | AL049866 | Mus musculus chromosome X, clone 437P9. | Mus musculus | 37,500 | 29-Jun-99 |
| | | GB_PR3:AC005740 | 186780 | AC005740 | Homo sapiens chromosome 5p, BAC clone 50g21 (LBNL H154), complete sequence. | Homo sapiens | 37,031 | 01-OCT-1998 |
| | | GB_PR3:AC005740 | 186780 | AC005740 | Homo sapiens chromosome 5p, BAC clone 50g21 (LBNL H154), complete sequence. | Homo sapiens | 38,035 | 01-OCT-1998 |
| rxa01551 | 1998 | GB_BA1:MTCY22G10 | 35420 | Z84724 | Mycobacterium tuberculosis H37Rv complete genome; segment 21/162. | Mycobacterium tuberculosis | 38,371 | 17-Jun-98 |
| | | GB_BA2:ECOUW89 | 176195 | U00006 | E. coli chromosomal region from 89.2 to 92.8 minutes. | Escherichia coli | 38,064 | 17-DEC-1993 |
| | | GB_BA1:SCQ11 | 15441 | AL096823 | Streptomyces coelicolor cosmid Q11. | Streptomyces coelicolor | 60,775 | 8-Jul-99 |
| rxa01561 | 1053 | GB_IN1:CEY62H9A | 47396 | AL032630 | Caenorhabditis elegans cosmid Y62H9A, complete sequence. | Caenorhabditis elegans | 38,514 | 2-Sep-99 |
| | | GB_PR4:HSU51003 | 3202 | U51003 | Homo sapiens DLX-2 (DLX-2) gene, complete cds. | Homo sapiens | 37,730 | 07-DEC-1999 |
| | | GB_OM:PIGDAO1 | 395 | M18444 | Pig D-amino acid oxidase (DAO) gene, exon 1. | Sus scrofa | 39,340 | 27-Apr-93 |
| rxa01599 | 1785 | GB_BA1:MTCI125 | 37432 | Z98268 | Mycobacterium tuberculosis H37Rv complete genome; segment 76/162. | Mycobacterium tuberculosis | 63,300 | 17-Jun-98 |
| | | GB_BA1:U00021 | 39193 | U00021 | Mycobacterium leprae cosmid L247. | Mycobacterium leprae | 36,756 | 29-Sep-94 |
| | | GB_BA1:MLCB1351 | 38936 | Z95117 | Mycobacterium leprae cosmid B1351. | Mycobacterium leprae | 36,756 | 24-Jun-97 |
| rxa01617 | 795 | GB_PR2:HSMTM0 | 217657 | AL034384 | Human chromosome Xq28, cosmid clones 7H3, 14D7, C1230, 11E7, F1096, A12197, 12G8, A09100; complete sequence bases 1..217657. | Homo sapiens | 40,811 | 5-Jul-99 |
| | | GB_PR2:HS13D10 | 153147 | AL021407 | Homo sapiens DNA sequence from PAC 13D10 on chromosome 6p22.3-23. Contains CpG island. | Homo sapiens | 38,768 | 23-Nov-99 |
| | | GB_PR2:HSMTM0 | 217657 | AL034384 | Human chromosome Xq28, cosmid clones 7H3, 14D7, C1230, 11E7, F1096, A12197, 12G8, A09100; complete sequence bases 1..217657. | Homo sapiens | 39,018 | 5-Jul-99 |
| rxa01657 | 723 | GB_BA1:MTCY1A10 | 25949 | Z95387 | Mycobacterium tuberculosis H37Rv complete genome; segment 117/162. | Mycobacterium tuberculosis | 40,656 | 17-Jun-98 |
| | | GB_EST6:D79278 | 392 | D79278 | HUM213D06B Human aorta polyA+ (TFujiwara) Homo sapiens cDNA clone GEN-213D06 5', mRNA sequence. | Homo sapiens | 44,262 | 9-Feb-96 |
| | | GB_BA2:AF129925 | 10243 | AF129925 | Thiobacillus ferrooxidans carboxysome operon, complete cds. | Thiobacillus ferrooxidans | 40,709 | 17-MAY-1999 |
| rxa01660 | 675 | GB_BA1:MTV013 | 11364 | AL021309 | Mycobacterium tuberculosis H37Rv complete genome; segment 134/162. | Mycobacterium tuberculosis | 40,986 | 17-Jun-98 |
| | | GB_RO:MMFV1 | 6480 | X97719 | M.musculus retrovirus restriction gene Fv1. | Mus musculus | 35,364 | 29-Aug-96 |
| | | GB_PAT:A67508 | 6480 | A67508 | Sequence 1 from Patent WO9743410. | Mus musculus | 35,364 | 05-MAY-1999 |
| rxa01678 | 651 | GB_VI:TVU95309 | 600 | U95309 | Tula virus 064 nucleocapsid protein gene, partial cds. | Tula virus | 41,894 | 28-OCT-1997 |
| | | GB_VI:TVU95303 | 600 | U95303 | Tula virus 052 nucleocapsid protein gene, partial cds. | Tula virus | 41,712 | 28-OCT-1997 |
| | | GB_VI:TVU95302 | 600 | U95302 | Tula virus 024 nucleocapsid protein gene, partial cds. | Tula virus | 39,576 | 28-OCT-1997 |
| rxa01679 | 1359 | GB_EST5:H91843 | 362 | H91843 | ys81e01.s1 Soares retina N2b4HR Homo sapiens cDNA clone IMAGE:221208 3' similar to gb:X63749_rna1 GUANINE NUCLEOTIDE-BINDING PROTEIN G(T), ALPHA-1 (HUMAN);, mRNA sequence. | Homo sapiens | 39,157 | 29-Nov-95 |
| | | GB_STS:G26925 | 362 | G26925 | human STS SHGC-30023, sequence tagged site. | Homo sapiens | 39,157 | 14-Jun-96' |
| | | GB_PL2:AF139451 | 1202 | AF139451 | Gossypium robinsonii CelA2 pseudogene, partial sequence. | Gossypium robinsonii | 38,910 | 1-Jun-99 |
| rxa01690 | 1224 | GB_BA1:SC1C2 | 42210 | AL031124 | Streptomyces coelicolor cosmid 1C2. | Streptomyces coelicolor | 60,644 | 15-Jan-99 |
| | | GB_EST22:AI064232 | 493 | AI064232 | GH04563.5prime GH Drosophila melanogaster head pOT2 Drosophila melanogaster cDNA clone GH04563 5prime, mRNA sequence. | Drosophila melanogaster | 38,037 | 24-Nov-98 |
| | | GB_IN2:AF117896 | 1020 | AF117896 | Drosophila melanogaster neuropeptide F (npf) gene, complete cds. | Drosophila melanogaster | 36,122 | 2-Jul-99 |
| rxa01692 | 873 | GB_BA2:AF067123 | 1034 | AF067123 | Lactobacillus reuteri cobalamin biosynthesis protein J (cbiJ) gene, partial cds; and uroporphyrin-III C-methyltransferase (sumT) gene, complete cds. | Lactobacillus reuteri | 48,079 | 3-Jun-98 |
| | | GB_RO:RATNFHPEP | 3085 | M37227 | Rat heavy neurofilament (NF-H) polypeptide, partial cds. | Rattus norvegicus | 37,093 | 27-Apr-93 |
| | | GB_RO:RSNFH | 3085 | X13804 | Rat mRNA for heavy neurofilament polypeptide NF-H C-terminus. | Rattus sp. | 37,093 | 14-Jul-95 |
| rxa01698 | 1353 | GB_BA2:AF124600 | 4115 | AF124600 | Corynebacterium glutamicum chorismate synthase (aroC), shikimate kinase (aroK), and 3-dehydroquinate synthase (aroB) genes, complete cds; and putative cytoplasmic peptidase (pepQ) gene, partial cds. | Corynebacterium glutamicum | 100,000 | 04-MAY-1999 |
| | | GB_BA1:MTCY159 | 33818 | Z83863 | Mycobacterium tuberculosis H37Rv complete genome; segment 111/162. | Mycobacterium tuberculosis | 36,323 | 17-Jun-98 |
| | | GB_BA1:MSGB937CS | 38914 | L78820 | Mycobacterium leprae cosmid B937 DNA sequence. | Mycobacterium leprae | 62,780 | 15-Jun-96 |
| rxa01699 | 693 | GB_BA2:AF124600 | 4115 | AF124600 | Corynebacterium glutamicum chorismate synthase (aroC), shikimate kinase (aroK), and 3-dehydroquinate synthase (aroB) genes, complete cds; and putative cytoplasmic peptidase (pepQ) gene, partial cds. | Corynebacterium glutamicum | 100,000 | 04-MAY-1999 |
| | | GB_BA2:AF016585 | 41097 | AF016585 | Streptomyces caelestis cytochrome P-450 hydroxylase homolog (nidi) gene, partial cds; polyketide synthase modules 1 through 7 (nidA) genes, complete cds; and N-methyltransferase homolog gene, partial cds: | Streptomyces caelestis | 40,260 | 07-DEC-1997 |
| | | GB_EST9:C19712 | 399 | C19712 | C19712 Rice panicle at ripening stage Oryza sativa cDNA clone E10821_1A, mRNA sequence. | Oryza sativa | 45,425 | 24-OCT-1996 |
| rxa01712 | 805 | GB_EST21:AA952466 | 278 | AA952466 | TENS1404 T. cruzi epimastigote normalized cDNA Library Trypanosoma cruzi cDNA clone 1404 5', mRNA sequence. | Trypanosoma cruzi | 40,876 | 29-OCT-1998 |
| | | GB_EST21:AA952466 | 278 | AA952466 | TENS1404 T. cruzi epimastigote normalized cDNA Library Trypanosoma cruzi cDNA clone 1404 5', mRNA sequence. | Trypanosoma cruzi | 41,367 | 29-OCT-1998 |
| | | | | | | | | |
| rxa01719 | 684 | GB_HTG1:HSDJ534K7 | 154416 | AL109925 | I Homo sapiens chromosome 1 clone RP4-534K7, *** SEQUENCING IN PROGRESS ***, in unordered pieces. | Homo sapiens | 35,651 | 23-Nov-99 |
| | | GB_HTG1:HSDJ534K7 | 154416 | AL109925 | I Homo sapiens chromosome 1 clone RP4-534K7,*** SEQUENCING IN PROGRESS ***, in unordered pieces. | Homo sapiens | 35,651 | 23-Nov-99 |
| | | GB_EST27:AI447108 | 431 | AI447108 | mq91e08.x1 Stratagene mouse heart (#937316) Mus musculus cDNA clone IMAGE:586118 3', mRNA sequence. | Mus musculus | 39,671 | 09-MAR-1999 |
| rxa01720 | 1332 | GB_PR4:AC006322 | 179640 | AC006322 | Homo sapiens PAC clone DJ1060B11 from 7q11.23-q21.1, complete sequence. | Homo sapiens | 35,817 | 18-MAR-1999 |
| | | GB_PL2:TM018A10 | 106184 | AF013294 | Arabidopsis thaliana BAC TM018A10. | Arabidopsis thaliana | 35,698 | 12-Jul-97 |
| | | GB_PR4:AC006322 | 179640 | AC006322 | Homo sapiens PAC clone DJ1060B11 from 7q11.23-q21.1, complete sequence. | Homo sapiens | 37,243 | 18-MAR-1999 |
| rxa01746 | 876 | GB_EST3:R46227 | 443 | R46227 | yg52a03.s1 Soares infant brain 1NIB Homo sapiens cDNA clone IMAGE:36000 3', mRNA sequence. | Homo sapiens | 42,812 | 22-MAY-1995 |
| | | GB_EST3:R46227 | 443 | R46227 | yg52a03.s1 Soares infant brain 1NIB Homo sapiens cDNA clone IMAGE:36000 3', mRNA sequence. | Homo sapiens | 42,655 | 22-MAY-1995 |
| | | | | | | | | |
| rxa01747 | 1167 | GB_BA1:MTCY190 | 34150 | Z70283 | Mycobacterium tuberculosis H37Rv complete genome; segment 98/162. | Mycobacterium tuberculosis | 59,294 | 17-Jun-98 |
| | | GB_BA1:MLCB22 | 40281 | Z98741 | Mycobacterium leprae cosmid B22. | Mycobacterium leprae | 57,584 | 22-Aug-97 |
| | | GB_BA1:SC5F7 | 40024 | AL096872 | Streptomyces coelicolor cosmid 5F7. | Streptomyces coelicolor A3(2) | 61,810 | 22-Jul-99 |
| | | | | | | | | |
| rxa01757 | 924 | GB_EST21:AA918454 | 416 | AA918454 | om38c02.s1 Soares_NFL_T_GBC_S1 Homo sapiens cDNA clone IMAGE:1543298 3' similar to WP:F28F8.3 CE09757 SMALL NUCLEAR RIBONUCLEOPROTEIN E ;, mRNA sequence. | Homo sapiens | 39,655 | 23-Jun-98 |
| | | GB_EST4:H34042 | 345 | H34042 | EST110563 Rat PC-12 cells, NGF-treated (9 days) Rattus sp. cDNA clone RPNBI81 5' end, mRNA sequence. | Rattus sp. | 35,942 | 2-Apr-98 |
| | | GB_EST20:AA899038 | 450 | AA899038 | NCP6G8T7 Perithecial Neurospora crassa cDNA clone NP6G8 3' end, mRNA sequence. | Neurospora crassa | 40,000 | 12-Apr-98 |
| rxa01807 | 915 | GB_BA1:AP000063 | 185300 | AP000063 | Aeropyrum pernix genomic DNA, section 6/7. | Aeropyrum pernix | 40,067 | 22-Jun-99 |
| | | GB_HTG4:AC010694 | 115857 | AC010694 | Drosophila melanogaster clone RPCI98-6H2, *** SEQUENCING IN PROGRESS ***, 75 unordered pieces. | Drosophila melanogaster | 35,450 | 16-OCT-1999 |
| | | GB_HTG4:AC010694 | 115857 | AC010694 | Drosophila melanogaster clone RPCI98-6H2, *** SEQUENCING IN PROGRESS ***, 75 unordered pieces. | Drosophila melanogaster | 35,450 | 16-OCT-1999 |
| rxa01821 | 401 | GB_BA1:CGL007732 | 4460 | AJ007732 | Corynebacterium glutamicum 3' ppc gene, secG gene, amt gene, ocd gene and 5' soxA gene. | Corynebacterium glutamicum | 100,000 | 7-Jan-99 |
| | | GB_RO:RATALGL | 7601 | M24108 | Rattus norvegicus (clone A2U42) alpha2u globulin gene, exons 1-7. | Rattus norvegicus | 38,692 | 15-DEC-1994 |
| | | GB_OV:APIGY2 | 1381 | X78272 | Anas platyrhynchos (Super M) IgY upsilon heavy chain gene, exon 2. | Anas platyrhynchos | 36,962 | 15-Feb-99 |
| rxa01835 | 654 | GB_EST30:AI629479 | 353 | AI629479 | 486101D10.x1 486 - leaf primordia cDNA library from Hake lab Zea mays cDNA, mRNA sequence. | Zea mays | 38,109 | 26-Apr-99 |
| | | GB_STS:G48245 | 515 | G48245 | SHGC-62915 Human Homo sapiens STS genomic, sequence tagged site. | Homo sapiens | 37,021 | 26-MAR-1999 |
| | | GB_GSS3:B49052 | 515 | B49052 | RPCI11-4I12.TV RPCI-11 Homo sapiens genomic clone RPCI-11-4I12, genomic survey sequence. | Homo sapiens | 37,021 | 8-Apr-99 |
| rxa01850 | 1470 | GB_BA2:ECOUW67_0 | 110000 | U18997 | Escherichia coli K-12 chromosomal region from 67.4 to 76.0 minutes. | Escherichia coli | 37,196 | U18997 |
| | | GB_BA2:AE000392 | 10345 | AE000392 | Escherichia coli K-12 MG1655 section 282 of 400 of the complete genome. | Escherichia coli | 38,021 | 12-Nov-98 |
| | | GB_BA2:U32715 | 13136 | U32715 | Haemophilus influenzae Rd section 30 of 163 of the complete genome. | Haemophilus influenzae Rd | 39,860 | 29-MAY-1998 |
| rxa01878 | 1002 | GB_HTG1:CEY64F11 | 177748 | Z99776 | Caenorhabditis elegans chromosome IV clone Y64F11, *** SEQUENCING IN PROGRESS in unordered | Caenorhabditis elegans | 37,564 | 14-OCT-1998 |
| | | GB_HTG1:CEY64F11 | 177748 | Z99776 | ***, pieces. Caenorhabditis elegans chromosome IV clone Y64F11, *** SEQUENCING IN PROGRESS ***, in unordered pieces. | Caenorhabditis elegans | 37,564 | 14-OCT-1998 |
| | | GB_HTG 1:CEY64F11 | 177748 | Z99776 | Caenorhabditis elegans chromosome IV clone Y64F11, *** SEQUENCING IN PROGRESS ***, in unordered pieces. | Caenorhabditis elegans | 37,576 | 14-OCT-1998 |
| rxa01892 | 852 | GB_BA1:MTCY274 | 39991 | Z74024 | Mycobacterium tuberculosis H37Rv complete genome; segment 126/162. | Mycobacterium tuberculosis | 35,910 | 19-Jun-98 |
| | | GB_BA1:MLCB250 | 40603 | Z97369 | Mycobacterium leprae cosmid B250. | Mycobacterium leprae | 64,260 | 27-Aug-99 |
| | | GB_BA1:MSGB1529CS | 36985 | L78824 | Mycobacterium leprae cosmid B1529 DNA sequence. | Mycobacterium leprae | 64,260 | 15-Jun-96 |
| | | | | | | | | |
| rxa01894 | 978 | GB_BA1:MTCY274 | 39991 | Z74024 | Mycobacterium tuberculosis H37Rv complete genome; segment 126/162. | Mycobacterium tuberculosis | 37,229 | 19-Jun-98 |
| | | GB_IN1:CELF46H5 | 38886 | U41543 | Caenorhabditis elegans cosmid F46H5. | Caenorhabditis elegans | 38,525 | 29-Nov-96 |
| | | GB_HTG3:AC009204 | 115633 | AC009204 | Drosophila melanogaster chromosome 2 clone BACR03E19 (D1033) RPCI-98 03.E.19 map 36E-37C strain y; cn bw sp,*** SEQUENCING IN PROGRESS ***, 94 unordered pieces. | Drosophila melanogaster | 31,579 | 18-Aug-99 |
| rxa01920 | 1125 | GB_BA2:AF112536 | 1798 | AF112536 | Corynebacterium glutamicum ribonucleotide reductase beta-chain (nrdF) gene, complete cds. | Corynebacterium glutamicum | 99,733 | 5-Aug-99 |
| | | GB_BA1:CANRDFGEN | 6054 | Y09572 | Corynebacterium ammoniagenes nrdH, nrdI, nrdE, nrdF genes. | Corynebacterium ammoniagenes | 70,321 | 18-Apr-98 |
| | | GB_BA2:AF050168 | 1228 | AF050168 | Corynebacterium ammoniagenes ribonucleoside diphosphate reductase small subunit (nrdF) gene, complete cds. | Corynebacterium ammoniagenes | 72,082 | 23-Apr-98 |
| rxa01928 | 960 | GB_BA1:CGPAN | 2164 | X96580 | C.glutamicum panB, panC & xylB genes. | Corynebacterium glutamicum | 100,000 | 11-MAY-1999 |
| | | GB_PL1:AP000423 | 154478 | AP000423 | Arabidopsis thaliana chloroplast genomic DNA, complete sequence, strain:Columbia. | Chloroplast Arabidopsis thaliana | 35,917 | 15-Sep-99 |
| | | GB_PL1:AP000423 | 154478 | AP000423 | Arabidopsis thaliana chloroplast genomic DNA, complete sequence, strain:Columbia. | Chloroplast Arabidopsis thaliana | 33,925 | 15-Sep-99 |
| rxa01929 | 936 | GB_BA1:CGPAN | 2164 | X96580 | C.glutamicum panB, panC & xylB genes. | Corynebacterium glutamicum | 100,000 | 11-MAY-1999 |
| | | GB_BA1:XCU33548 | 8429 | U33548 | Xanthomonas campestris hrpB pathogenicity locus proteins HrpB1, HrpB2, HrpB3, HrpB4, HrpB5, HrpB6, HrpB7, HrpB8, HrpA1, and ORF62 genes, complete cds. | Xanthomonas campestris pv. vesicatoria | 38,749 | 19-Sep-96 |
| | | GB_BA1:XANHRPB6A | 1329 | M99174 | Xanthomonas campestris hrpB6 gene, complete cds. | Xanthomonas campestris | 39,305 | 14-Sep-93 |
| rxa01940 | 1059 | GB_IN2:CFU43371 | 1060 | U43371 | Crithidia fasciculata inosine-uridine preferring nucleoside hydrolase (IUNH) gene, complete cds. | Crithidia fasciculata | 61,417 | 18-Jun-96 |
| | | GB_BA2:AE001467 | 11601 | AE001467 | Helicobacter pylori, strain J99 section 28 of 132 of the complete | Helicobacter pylori J99 | 38,560 | 20-Jan-99 |
| | | GB_RO:AF175967 | 3492 | AF175967 | genome. Homo sapiens Leman coiled-coil protein (LCCP) mRNA, complete cds. | Mus musculus | 40,275 | 26-Sep-99 |
| rxa02022 | 1230 | GB_BA1:CGDAPE | 1966 | X81379 | C.glutamicum dapE gene and orf2. | Corynebacterium glutamicum | 100,000 | 8-Aug-95 |
| | | GB_BA1:CGDNAAROP | 2612 | X85965 | C.glutamicum ORF3 and aroP gene. | Corynebacterium glutamicum | 38,889 | 30-Nov-97 |
| | | | | | | | | |
| | | GB_BA1:APU47055 | 6469 | U47055 | Anabaena PCC7120 nitrogen fixation proteins (nifE, nifN, nifX, nifW) genes, complete cds, and nitrogenase (nifK) and hesA genes, partial cds. | Anabaena PCC7120 | 36,647 | 17-Feb-96 |
| rxa02024 | 859 | GB_BA1:MTCI364 | 29540 | Z93777 | Mycobacterium tuberculosis H37Rv complete genome; segment 52/162. | Mycobacterium tuberculosis | 59,415 | 17-Jun-98 |
| | | GB_BA1:MSGB1912CS | 38503 | L01536 | M. leprae genomic dna sequence, cosmid b1912. | Mycobacterium leprae | 57,093 | 14-Jun-96 |
| | | | | | | | | |
| | | GB_BA1:MLU15180 | 38675 | U15180 | Mycobacterium leprae cosmid B1756. | Mycobacterium leprae | 57,210 | 09-MAR-1995 |
| rxa02027 | | | | | | | | |
| | | | | | | | | |
| rxa02031 | | | | | | | | |
| | | | | | | | | |
| rxa02072 | 1464 | GB_BA1:CGGDHA | 2037 | X72855 | C.glutamicum GDHA gene. | Corynebacterium glutamicum | 99,317 | 24-MAY-1993 |
| | | GB_BA1:CGGDH | 2037 | X59404 | Corynebacterium glutamicum, gdh gen for glutamate dehydrogenase. | Corynebacterium glutamicum | 94,387 | 30-Jul-99 |
| | | GB_BA1:PAE18494 | 1628 | Y18494 | Pseudomonas aeruginosa gdhA gene, strain PAC1. | Pseudomonas aeruginosa | 62,247 | 6-Feb-99 |
| rxa02085 | 2358 | GB_BA1:MTCY22G8 | 22550 | Z95585 | Mycobacterium tuberculosis H37Rv complete genome; segment 49/162. | Mycobacterium tuberculosis | 38,442 | 17-Jun-98 |
| | | GB_BA1:MLCB33 | 42224 | Z94723 | Mycobacterium leprae cosmid B33. | Mycobacterium leprae | 56,466 | 24-Jun-97 |
| | | GB_BA1:ECOUW85 | 91414 | M87049 | E. coli genomic sequence of the region from 84.5 to 86.5 minutes. | Escherichia coli | 52,127 | 29-MAY-1995 |
| rxa02093 | 927 | GB_EST14:AA448146 | 452 | AA448146 | zw82h01.r1 Soares_testis_NHT Homo sapiens cDNA clone IMAGE:782737 5', mRNA sequence. | Homo sapiens | 34,163 | 4-Jun-97 |
| | | GB_EST17:AA641937 | 444 | AA641937 | ns18b10.r1 NCI_CGAP_GCB1 Homo sapiens cDNA clone IMAGE:1183963 5', mRNA sequence. | Homo sapiens | 35,586 | 27-OCT-1997 |
| | | GB_PR3:AC003074 | 143029 | AC003074 | Human PAC clone DJ0596O09 from 7p15, complete sequence. | Homo sapiens | 31,917 | 6-Nov-97 |
| rxa02106 | 1179 | GB_BA1:SC1A6 | 37620 | AL023496 | Streptomyces coelicolor cosmid 1A6. | Streptomyces coelicolor | 35,818 | 13-Jan-99 |
| | | GB_PR4:AC005553 | 179651 | AC005553 | Homo sapiens chromosome 17, clone hRPK.112_J_9, complete sequence. | Homo sapiens | 34,274 | 31-DEC-1998 |
| | | GB_EST3:R49746 | 397 | R49746 | yg71g10.r1 Soares infant brain 1NIB Homo sapiens cDNA clone IMAGE:38768 5' similar to gb:V00567 BETA-2-MICROGLOBULIN PRECURSOR (HUMAN);, mRNA sequence. | Homo sapiens | 41,162 | 18-MAY-1995 |
| rxa02111 | 1407 | GB_BA1:SC6G10 | 36734 | AL049497 | Streptomyces coelicolor cosmid 6G10. | Streptomyces coelicolor | 50,791 | 24-MAR-1999 |
| | | GB_BA1:U00010 | 41171 | U00010 | Mycobacterium leprae cosmid B1170. | Mycobacterium leprae | 37,563 | 01-MAR-1994 |
| | | GB_BA1:MTCY336 | 32437 | Z95586 | Mycobacterium tuberculosis H37Rv complete genome; segment 70/162. | Mycobacterium tuberculosis | 39.504 | 24-Jun-99 |
| rxa02112 | 960 | GB_HTG3:AC010579 | 157658 | AC010579 | Drosophila melanogaster chromosome 3 clone BACR09D08 (D1101) RPCI-98 09.D.8 map 96F-96F strain y; cn bw sp, *** SEQUENCING IN PROGRESS *** 121 unordered pieces. | Drosophila melanogaster | 37,909 | 24-Sep-99 |
| | | GB_GSS3:B09839 | 1191 | 809839 | T12A12-Sp6 TAMU Arabidopsis thaliana genomic clone T12A12, genomic survey sequence. | Arabidopsis thaliana | 37,843 | 14-MAY-1997 |
| | | GB_HTG3:AC010579 | 157658 | AC010579 | Drosophila melanogaster chromosome 3 clone BACR09D08 (D1101) RPCI-98 09.D.8 map 96F-96F strain y; cn bw sp, *** SEQUENCING IN PROGRESS *** 121 unordered pieces. | Drosophila melanogaster | 37,909 | 24-Sep-99 |
| rxa02134 | 1044 | GB_BA1:SCSECYDNA | 6154 | X83011 | S.coelicolor secY locus DNA. | Streptomyces coelicolor | 36,533 | 02-MAR-1998 |
| | | | | | | | | |
| | | GB_EST32:AI731596 | 568 | AI731596 | BNLGHi10185 Six-day Cotton fiber Gossypium hirsutum cDNA 5' similar to (AC004005) putative ribosomal protein L7 [Arabidopsis thaliana], mRNA sequence. | Gossypium hirsutum | 33,451 | 11-Jun-99 |
| | | GB_BA1:SCSECYDNA | 6154 | X83011 | S.coelicolor secY locus DNA. | Streptomyces coelicolor | 36,756 | 02-MAR-1998 |
| rxa02135 | 1197 | GB_PR3:HS525L6 | 168111 | AL023807 | Human DNA sequence from clone RP3-525L6 on chromosome 6p22.3-23 Contains CA repeat, STSs, GSSs and a CpG Island, complete sequence. | Homo sapiens | 34,365 | 23-Nov-99 |
| | | GB_PL2:ATF21P8 | 85785 | AL022347 | Arabidopsis thaliana DNA chromosome 4, BAC clone F21P8 (ESSA project). | Arabidopsis thaliana | 34,325 | 9-Jun-99 |
| | | | | | | | | |
| | | GB_PL2:U89959 | 106973 | U89959 | Arabidopsis thaliana BAC T7I23, complete sequence. | Arabidopsis thaliana | 33,874 | 26-Jun-98 |
| rxa02136 | 645 | GB_PL2:ATAC005819 | 57752 | AC005819 | Arabidopsis thaliana chromosome II BAC T3A4 genomic sequence, complete sequence. | Arabidopsis thaliana | 34,123 | 3-Nov-98 |
| | | GB_PL2:F15K9 | 71097 | AC005278 | Arabidopsis thaliana chromosome 1 BAC F15K9 sequence, complete sequence. | Arabidopsis thaliana | 31,260 | 7-Nov-98 |
| | | GB_PL2:U89959 | 106973 | U89959 | Arabidopsis thaliana BAC T7I23, complete sequence. | Arabidopsis thaliana | 34,281 | 26-Jun-98 |
| rxa02139 | 1962 | GB_BA1:MTCY190 | 34150 | Z70283 | Mycobacterium tuberculosis H37Rv complete genome; segment 98/162. | Mycobacterium tuberculosis | 62,904 | 17-Jun-98 |
| | | GB_BA1:MSGB1554CS | 36548 | L78814 | Mycobacterium leprae cosmid B1554 DNA sequence. | Mycobacterium leprae | 36,648 | 15-Jun-96 |
| | | | | | | | | |
| | | GB_BA1:MSGB1551CS | 36548 | L78813 | Mycobacterium leprae cosmid B1551 DNA sequence. | Mycobacterium leprae | 36,648 | 15-Jun-96 |
| | | | | | | | | |
| rxa02153 | 903 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 99,104 | 1-Jul-98 |
| | | GB_BA1:AF005242 | 1044 | AF005242 | Corynebacterium glutamicum N-acetylglutamate-5-semialdehyde dehydrogenase (argC) gene, complete cds. | Corynebacterium glutamicum | 99,224 | 2-Jul-97 |
| | | GB_BA1:CGARGCJBD | 4355 | X86157 | C.glutamicum argC, argJ, argB, argD, and argF genes. | Corynebacterium glutamicum | 100,000 | 25-Jul-96 |
| | | | | | | | | |
| rxa02154 | 414 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 98,551 | 1-Jul-98 |
| | | GB_BA1:AF005242 | 1044 | AF005242 | Corynebacterium glutamicum N-acetylglutamate-5-semialdehyde dehydrogenase (argC) gene, complete cds. | Corynebacterium glutamicum | 98,477 | 2-Jul-97 |
| | | GB_BA1:CGARGCJBD | 4355 | X86157 | C.glutamicum argC, argJ, argB, argD, and argF genes. | Corynebacterium glutamicum | 100,000 | 25-Jul-96 |
| | | | | | | | | |
| rxa02155 | 1287 | GB_BA1:CGARGCJBD | 4355 | X86157 | C.glutamicum argC, argJ, argB, argD, and argF genes. | Corynebacterium glutamicum | 99,767 | 25-Jul-96 |
| | | | | | | | | |
| | | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 99,378 | 1-Jul-98 |
| | | GB_BA1:MSGB1133CS | 42106 | L78811 | Mycobacterium leprae cosmid B1133 DNA sequence. | Mycobacterium leprae | 55,504 | 15-Jun-96 |
| rxa02156 | 1074 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 100,000 | 1-Jul-98 |
| | | GB_BA1:CGARGCJBD | 4355 | X86157 | C.glutamicum argC, argJ, argB, argD, and argF genes. | Corynebacterium glutamicum | 100,000 | 25-Jul-96 |
| | | | | | | | | |
| | | GB_BA2:AE001816 | 10007 | AE001816 | Thermotoga maritima section 128 of 136 of the complete genome. | Thermotoga maritima | 50,238 | 2-Jun-99 |
| rxa02157 | 1296 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 99,612 | 1-Jul-98 |
| | | GB_BA1:CGARGCJBD | 4355 | X86157 | C.glutamicum argC, argJ, argB, argD, and argF genes. | Corynebacterium glutamicum | 99,612 | 25-Jul-96 |
| | | | | | | | | |
| | | GB_BA1:MTCY06H11 | 38000 | Z85982 | Mycobacterium tuberculosis H37Rv complete genome; segment 73/162. | Mycobacterium tuberculosis | 57,278 | 17-Jun-98 |
| rxa02158 | 1080 | GB_BA2:AF049897 | 9196 | AF049897 | Corynebacterium glutamicum N-acetytgiutamylphosphate reductase (argC), ornithine acetyltransferase (argJ), N-acetylglutomate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 100,000 | 1-Jul-98 |
| | | GB_BA2:AF031518 | 2045 | AF031518 | Corynebacterium glutamicum ornithine carbamolytransferase (argF) gene, complete cds. | Corynebacterium glulamicum | 99,898 | 5-Jan-99 |
| | | GB_BA1:CGARGCJBD | 4355 | X86157 | C.glutamicum argC, argJ, argB, argD, and argF genes. | Corynebacterium glutamicum | 100,000 | 25-Jul-96 |
| | | | | | | | | |
| rxa02159 | 636 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 99,843 | 1-Jul-98 |
| | | GB_BA2:AF031518 | 2045 | AF031518 | Corynebacterium glutamicum ornithine carbamolytransferase (argF) gene, complete cds. | Corynebacterium glutamicum | 88,679 | 5-Jan-99 |
| | | GB_BA2:AF041436 | 516 | AF041436 | Corynebacterium glutamicum arginine repressor (argR) gene, complete cds. | Corynebacterium glutamicum | 100,000 | 5-Jan-99 |
| rxa02160 | 1326 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 99,774 | 1-Jul-98 |
| | | GB_BA2:AF030520 | 1206 | AF030520 | Corynebacterium glutamicum argininosuccinate synthetase (argG) gene, complete cds. | Corynebacterium glutamicum | 99,834 | 19-Nov-97 |
| | | GB_BA1:SCARGGH | 1909 | Z49111 | S.clavuligerus argG gene and argH gene (partial). | Streptomyces clavuligerus | 65,913 | 22-Apr-96 |
| rxa02162 | 1554 | GB_BA2:AF049897 | 9196 | AF049897 | Corynebacterium glutamicum N-acetylglutamylphosphate reductase (argC), ornithine acetyltransferase (argJ), N-acetylglutamate kinase (argB), acetylornithine transaminase (argD), ornithine carbamoyltransferase (argF), arginine repressor (argR), argininosuccinate synthase (argG), and argininosuccinate lyase (argH) genes, complete cds. | Corynebacterium glutamicum | 88,524 | 1-Jul-98 |
| | | GB_BA2:AF048764 | 1437 | AF048764 | Corynebacterium glutamicum argininosuccinate lyase (argH) gene,complete cds. | Corynebacterium glutamicum | 87,561 | 1-Jul-98 |
| | | GB_BA1:MTCY06H11 | 38000 | Z85982 | Mycobacterium tuberculosis H37Rv complete genome: segment 73/162. | Mycobacterium tuberculosis | 64,732 | 17-Jun-98 |
| rxa02176 | 1251 | GB_BA1:MTCY31 | 37630 | Z73101 | Mycobacterium tuberculosis H37Rv complete genome; segment 41/162. | Mycobacterium tuberculosis | 36,998 | 17-Jun-98 |
| | | GB_BA1:CGGLTG | 3013 | X66112 | C.glutamicum glt gene for citrate synthase and ORF. | Corynebacterium glutamicum | 39,910 | 17-Feb-95 |
| | | GB_PL2:PGU65399 | 2700 | U65399 | Basidiomycete CECT 20197 phenoloxidase (pox1) gene, complete cds. | basidiomycete CECT 20197 | 38,474 | 19-Jul-97 |
| rxa02189 861 | 1251 | GB_PR3:AC002468 | 115888 | AC002468 | Human Chromosome 15q26.1 PAC clone pDJ417d7, complete sequence. | Homo sapiens | 35,941 | 16-Sep-98 |
| | | GB_BA1:MSGB1970CS | 39399 | L78815 | Mycobacterium leprae cosmid B1970 DNA sequence. | Mycobacterium leprae | 40,286 | 15-Jun-96 |
| | | | | | | | | |
| | | GB_PR3:AC002468 | 115888 | AC002468 | Human Chromosome 15q26.1 PAC clone pDJ417d7, complete sequence. | Homo sapiens | 33,689 | 16-Sep-98 |
| rxa02193 | 1701 | GB_BA1:BRLASPA | 1987 | D25316 | Brevibacterium flavum aspA gene for aspartase, complete cds. | Corynebacterium glutamicum | 99,353 | 6-Feb-99 |
| | | GB_PAT:E04307 | 1581 | E04307 | DNA encoding Brevibacterium flavum aspartase. | Corynebacterium glutamicum | 99,367 | 29-Sep-97 |
| | | GB_BA1:ECOUW93 | 338534 | U14003 | Escherichia coli K-12 chromosomal region from 92.8 to 00.1 minutes. | Escherichia coli | 37,651 | 17-Apr-96 |
| rxa02194 | 966 | GB_BA2:AF050166 | 840 | AF050166 | Corynebacterium glutamicum ATP phosphoribosyltransferase (hisG) gene, complete cds. | Corynebacterium glutamicum | 98,214 | 5-Jan-99 |
| | | GB_BA1:BRIASPA | 1987 | D25316 | Brevibacterium flavum aspA gene for aspartase, complete cds. | Corynebacterium glutamicum | 93,805 | 6-Feb-99 |
| | | GB_PAT:E08649 | 188 | E08649 | DNA encoding part of aspartase from coryneform bacteria. | Corynebacterium glutamicum | 100,000 | 29-Sep-97 |
| rxa02195 | 393 | GB_BA2:AF086704 | 264 | AF086704 | Corynebacterium glutamicum phosphoribosyl-ATP-pyrophosphohydrolase (hisE) gene, complete cds. | Corynebacterium glutamicum | 100,000 | 8-Feb-99 |
| | | GB_BA1:EAY17145 | 6019 | Y17145 | Eubacterium acidaminophilum grdR, grdl, grdH genes and partial ldc, grdT genes. | Eubacterium acidaminophilum | 39,075 | 5-Aug-98 |
| | | GB_STS:G01195 | 332 | G01195 | fruit fly STS Dm1930 clone DS06959 T7. | Drosophila melanogaster | 35,542 | 28-Feb-95 |
| rxa02197 | 551 | GB_BA1:MTCY261 | 27322 | Z97559 | Mycobacterium tuberculosis H37Rv complete genome; segment 95/162. | Mycobacterium tuberculosis | 33,938 | 17-Jun-98 |
| | | GB_BA1:MLCB2533 | 40245 | AL035310 | Mycobacterium leprae cosmid B2533. | Mycobacterium leprae | 65,517 | 27-Aug-99 |
| | | GB_BA1:U00017 | 42157 | U00017 | Mycobacterium leprae cosmid B2126. | Mycobacterium leprae | 36,770 | 01-MAR-1994 |
| rxa02198 | 2599 | GB_BA1: U00017 | 42157 | U00017 | Mycobacterium leprae cosmid B2126. | Mycobacterium leprae | 38,674 | 01-MAR-1994 |
| | | GB_BA1:MLCB2533 | 40245 | AL035310 | Mycobacterium leprae cosmid B2533. | Mycobacterium leprae | 65,465 | 27-Aug-99 |
| | | GB_BA1:MTCY261 | 27322 | Z97559 | Mycobacterium tuberculosis H37Rv complete genome; segment 95/162. | Mycobacterium tuberculosis | 37,577 | 17-Jun-98 |
| rxa02208 | 1025 | GB_BA1:U00017 | 42157 | U00017 | Mycobacterium leprae cosmid B2126. | Mycobacterium leprae | 59,823 | 01-MAR-1994 |
| | | GB_BA1:AP000063 | 185300 | AP000063 | Aeropyrum pernix genomic DNA, section 6/7. | Aeropyrum pernix | 39,442 | 22-Jun-99 |
| | | GB_PR4:AC006236 | 127593 | AC006236 | Homo sapiens chromosome 17, clone hCIT.162_E_12, complete sequence. | Homo sapiens | 37,191 | 29-DEC-1998 |
| rxa02229 | 948 | GB_BA1:MSGY154 | 40221 | AD000002 | Mycobacterium tuberculosis sequence from clone y154. | Mycobacterium tuberculosis | 53,541 | 03-DEC-1996 |
| | | GB_BA1:MTCY154 | 13935 | Z98209 | Mycobacterium tuberculosis H37Rv complete genome; segment 121/162. | Mycobacterium tuberculosis | 40,407 | 17-Jun-98 |
| | | GB_BA1:U00019 | 36033 | U00019 | Mycobacterium leprae cosmid B2235. | Mycobacterium leprae | 40,541 | 01-MAR-1994 |
| rxa02234 3462 | 948 | GB_BA1:MSGB937CS | 38914 | L78820 | Mycobacterium leprae cosmid B937 DNA sequence. | Mycobacterium leprae | 66,027 | 15-Jun-96 |
| | | GB_BA1:MTCY2B12 | 20431 | Z81011 | Mycobacterium tuberculosis H37Rv complete genome; segment 61/162. | Mycobacterium tuberculosis | 71,723 | 18-Jun-98 |
| | | GB_BA2:U01072 | 4393 | U01072 | Mycobacterium bovis BCG orotidine-5'-monophosphate decarboxylase (uraA) gene. | Mycobacterium bovis | 67,101 | 22-DEC-1993 |
| rxa02235 | 727 | GB_BA1:MSU91572 | 960 | U91572 | Mycobacterium smegmatis carbamoyl phosphate synthetase (pyrAB) gene, partial cds and orotidine 5'-monophosphate decarboxylase (pyrF) gene, complete cds. | Mycobacterium smegmatis | 60,870 | 22-MAR-1997 |
| | | GB_HTG3:AC009364 | 192791 | AC009364 | Homo sapiens chromosome 7, ***SEQUENCING IN PROGRESS***, 57 unordered pieces. | Homo sapiens | 37,994 | 1-Sep-99 |
| | | GB_HTG3:AC009364 | 192791 | AC009364 | Homo sapiens chromosome 7, *** SEQUENCING IN PROGRESS***, 57 unordered pieces. | Homo sapiens | 37,994 | 1-Sep-99 |
| rxa02237 | 693 | GB_BA1:MTCY2184 | 39150 | Z80108 | Mycobacterium tuberculosis H37Rv complete genome; segment 62/162. | Mycobacterium tuberculosis | 55,844 | 23-Jun-98 |
| | | GB_BA2:AF077324 | 5228 | AF077324 | Rhodococcus equi strain 103 plasmid RE-VP1 fragment f. | Rhodococcus equi | 41,185 | 5-Nov-98 |
| | | GB_EST22:AU017763 | 586 | AU017763 | AU017763 Mouse two-cell stage embryo cDNA Mus musculus cDNA clone J0744A04 3', mRNA sequence. | Mus musculus | 38,616 | 19-OCT-1998 |
| rxa02239 | 1389 | GB_BA1:MTCY21 B4 | 39150 | Z80108 | Mycobacterium tuberculosis H37Rv complete genome; segment 62/162. | Mycobacterium tuberculosis | 56,282 | 23-Jun-98 |
| | | GB_HTG3:AC010745 | 193862 | AC010745 | Homo sapiens clone NH0549D18, *** SEQUENCING IN PROGRESS ***, 30 unordered pieces. | Homo sapiens | 36,772 | 21-Sep-99 |
| | | GB_HTG3:AC010745 | 193862 | AC010745 | Homo sapiens clone NH0549D18, *** SEQUENCING IN PROGRESS ***, 30 unordered pieces. | Homo sapiens | 36,772 | 21-Sep-99 |
| rxa02240 | 1344 | EM_PAT:E09855 | 1239 | E09855 | gDNA encoding S-adenosylmethionine synthetase. | Corynebacterium glutamicum | 99,515 | 07-OCT-1997 |
| | | | | | | | | (Rel. 52, Created) |
| | | GB_PAT:A37831 | 5392 | A37831 | Sequence 1 from Patent WO9408014. | Streptomyces pristinaespiralis | 63,568 | 05-MAR-1997 |
| | | | | | | | | |
| | | GB_BA2:AF117274 | 2303 | AF117274 | Streptomyces spectabilis flavoprotein homolog Dfp (dfp) gene, partial cds; and | Streptomyces spectabilis | 65,000 | 31-MAR-1999 |
| | | | | | S-adenosylmethionine synthetase (metK) gene, complete cds. | | | |
| rxa02246 | 1107 | EM_BA1:AB003693 | 5589 | AB003693 | Corynebacterium ammoniagenes DNA for rib operon, complete cds. | Corynebacterium | 52,909 | 03-OCT-1997 |
| | | | | | | ammoniagenes | | (Rel. 52, Created) |
| | | GB_PAT:E07957 | 5589 | E07957 | gDNA encoding at least guanosine triphosphate cyclohydrolase and riboflavin synthase. | Corynebacterium ammoniagenes | 52,909 | 29-Sep-97 |
| | | GB_PAT:I32742 | 5589 | 132742 | Sequence 1 from patent US 5589355. | Unknown. | 52,909 | 6-Feb-97 |
| rxa02247 | 756 | GB_PAT:I32743 | 2689 | 132743 | Sequence 2 from patent US 5589355. | Unknown. | 57,937 | 6-Feb-97 |
| | | EM_BA1:AB003693 | 5589 | AB003693 | Corynebacterium ammoniagenes DNA for rib operon, complete cds. | Corynebacterium | 57,937 | 03-OCT-1997 |
| | | | | | | ammoniagenes | | (Rel. 52, Created) |
| | | GB_PAT:I32742 | 5589 | I32742 | Sequence 1 from patent US 5589355. | Unknown. | 57,937 | 6-Feb-97 |
| rxa02248 | 1389 | GB_PAT:I32742 | 5589 | 132742 | Sequence 1 from patent US 5589355. | Unknown. | 61,843 | 6-Feb-97 |
| | | EM_BA1:AB003693 | 5589 | AB003693 | Corynebacterium ammoniagenes DNA for rib operon, complete cds. | Corynebacterium | 61,843 | 03-OCT-1997 |
| | | | | | | ammoniagenes | | (Rel. 52, Created) |
| | | GB_PAT:E07957 | 5589 | E07957 | gDNA encoding at least guanosine triphosphate cyclohydrolase and riboflavin synthase. | Corynebacterium ammoniagenes | 61,843 | 29-Sep-97 |
| rxa02249 | 600 | GB_PAT:E07957 | 5589 | E07957 | gDNA encoding at least guanosine triphosphate cyclohydrolase and riboflavin synthase. | Corynebacterium ammoniagenes | 64,346 | 29-Sep-97 |
| | | GB_PAT:I32742 | 5589 | 132742 | Sequence 1 from patent US 5589355. | Unknown. | 64,346 | 6-Feb-97 |
| | | GB_PAT:I32743 | 2689 | I32743 | Sequence 2 from patent US 5589355. | Unknown. | 64,346 | 6-Feb-97 |
| rxa02250 | 643 | GB_PAT:E07957 | 5589 | E07957 | gDNA encoding at least guanosine triphosphate cyclohydrolase and riboflavin synthase. | Corynebacterium ammoniagenes | 56,318 | 29-Sep-97 |
| | | GB_PAT:I32742 | 5589 | 132742 | Sequence 1 from patent US 5589355. | Unknown. | 56,318 | 6-Feb-97 |
| | | EM_BA1:AB003693 | 5589 | AB003693 | Corynebacterium ammoniagenes DNA for rib operon, complete cds. | Corynebacterium | 56,318 | 03-OCT-1997 |
| | | | | | | ammoniagenes | | (Rel. 52, Created) |
| rxa02262 | 1269 | GB_BA1:CGL007732 | 4460 | AJ007732 | Corynebacterium glutamicum 3' ppc gene, secG gene, amt gene, ocd gene and 5' soxA gene. | Corynebacterium glutamicum | 100,000 | 7-Jan-99 |
| | | GB_BA1:CGAMTGENE | 2028 | X93513 | C.glutamicum amt gene. -- | Corynebacterium glutamicum | 100,000 | 29-MAY-1996 |
| | | | | | | | | |
| | | GB_VI:HEHCMVCG | 229354 | X17403 | Human cytomegalovirus strain AD169 complete genome. | human herpesvirus 5 | 38,651 | 10-Feb-99 |
| rxa02263 | 488 | GB_BA1:CGL007732 | 4460 | AJ007732 | Corynebacterium glutamicum 3' ppc gene, secG gene, amt gene, ocd gene and 5' soxA gene. | Corynebacterium glutamicum | 100,000 | 7-Jan-99 |
| | | GB_BA1:CGL007732 | 4460 | AJ007732 | Corynebacterium glutamicum 3' ppc gene, secG gene, amt gene, ocd gene and 5' soxA gene. | Corynebacterium glutamicum | 37,526 | 7-Jan-99 |
| | | | | | | | | |
| rxa02272 | 1368 | EM_PAT:E09373 | 1591 | E09373 | Creatinine deiminase gene. | Bacillus sp. | 96,928 | 08-OCT-1997 |
| | | | | | | | | (Rel. 52, Created) |
| | | GB_BA1:D38505 | 1591 | D38505 | Bacillus sp. gene for creatinine deaminase, complete cds. | Bacillus sp. | 96,781 | 7-Aug-98 |
| | | GB_HTG2:AC006595 | 146070 | AC006595 | Homo sapiens, *** SEQUENCING IN PROGRESS ***, 4 unordered pieces. | Homo sapiens | 36,264 | 20-Feb-99 |
| rxa02281 | 1545 | GB_GSS12:AQ411010 | 551 | AQ411010 | HS_2257_B1_H02_MR CIT Approved Human Genomic Sperm Library D Homo sapiens genomic clone Plate=2257 Col=3 Row=P, genomic survey sequence. | Homo sapiens | 36,197 | 17-MAR-1999 |
| | | GB_EST23:AI128623 | 363 | AI128623 | qa62c01.s1 Soares_fetal_heart_NbHH19W Homo sapiens cDNA clone IMAGE:1691328 3', mRNA sequence. | Homo sapiens | 37,017 | 05-OCT-1998 |
| | | GB_PL2:ATAC007019 | 102335 | AC007019 | Arabidopsis thaliana chromosome II BAC F7D8 genomic sequence, complete sequence. | Arabidopsis thaliana | 33,988 | 16-MAR-1999 |
| rxa02299 | 531 | GB_BA2:AF116184 | 540 | AF116184 | Corynebacterium glutamicum L-aspartate-alpha-decarboxylase precursor (panD) gene, complete cds. | Corynebacterium glutamicum | 100,000 | 02-MAY-1999 |
| | | GB_GSS9:AQ164310 | 507 | AQ164310 | HS_2171_A2_E01_MR CIT Approved Human Genomic Sperm Library D Homo sapiens genomic clone Plate=2171 Col=2 Row=I, genomic survey sequence. | Homo sapiens | 37,278 | 16-OCT-1998 |
| | | GB_VI:MH68TKH | 4557 | X93468 | Murine herpesvirus type 68 thymidine kinase and glycoprotein H genes. | murine herpesvirus 68 | 40,288 | 3-Sep-96 |
| rxa02311 | 813 | GB_HTG4:AC006091 | 176878 | AC006091 | Drosophila melanogaster chromosome 3 clone BACR48G05 (D475) RPCI-98 48.G.5 map 91F1-91F13 strain y; cn bw sp, *** SEQUENCING IN PROGRESS ***, 4 unordered pieces. | Drosophila melanogaster | 36,454 | 27-OCT-1999 |
| | | GB_HTG4:AC006091 | 176878 | AC006091 | Drosophila melanogaster chromosome 3 clone BACR48G05 (D475) RPCI-98 48.G.5 map 91F1-91F13 strain y; cn bw sp, *** SEQUENCING IN PROGRESS ***, 4 unordered pieces. | Drosophila melanogaster | 36,454 | 27-OCT-1999 |
| | | GB_BA2:RRU65510 | 16259 | U65510 | Rhodospirillum rubrum CO-induced hydrogenase operon (cooM, cooK, cooL, cooX, cooU, cooH) genes, iron sulfur protein (cooF) gene, carbon monoxide dehydrogenase (cooS) gene, carbon monoxide dehydrogenase accessory proteins (cooC, cooT, cooJ) genes, putative transcriptional activator (cooA) gene, nicotinate-nucleotide pyrophosphorylase (nadC) gene, complete cds, L-aspartate oxidase (nadB) gene, and alkyl hydroperoxide reductase (ahpC) gene, partial cds. | Rhodospirillum rubrum | 37,828 | 9-Apr-97 |
| | | | | | | | | |
| rxa02315 1752 | 813 | GB_BA1:MSGY224 | 40051 | AD000004 | Mycobacterium tuberculosis sequence from clone y224 | Mycobacterium tuberculosis | 49,418 | 03-DEC-1996 |
| | | GB_BA1:MTY25D10 | 40838 | Z95558 | Mycobacterium tuberculosis H37Rv complete genome; segment 28/162. | Mycobacterium tuberculosis | 49,360 | 17-Jun-98 |
| | | GB_BA1:MSGY224 | 40051 | AD000004 | Mycobacterium tuberculosis sequence from clone y224. | Mycobacterium tuberculosis | 38,150 | 03-DEC-1996 |
| rxa02318 | 402 | GB_HTG3:AC011348 | 111083 | AC011348 | Homo sapiens chromosome 5 clone CIT-HSPC_303E13, *** SEQUENCING IN PROGRESS ***, 3 ordered pieces. | Homo sapiens | 35,821 | 06-OCT-1999 |
| | | GB_HTG3:AC011348 | 111083 | AC011346 | Homo sapiens chromosome 5 clone CIT-HSPC_303E13, *** SEQUENCING IN PROGRESS ***, 3 ordered pieces. | Homo sapiens | 35,821 | 06-OCT-1999 |
| | | GB_HTG3:AC011412 | 89234 | AC011412 | Homo sapiens chromosome 5 clone CIT978SKB_81K21, *** SEQUENCING IN PROGRESS ***, 3 ordered pieces. | Homo sapiens | 36,181 | 06-OCT-1999 |
| rxa02319 | 1080 | GB_BA1:MSGY224 | 40051 | AD000004 | Mycobacterium tuberculosis sequence from clone y224. | Mycobacterium tuberculosis | 37,792 | 03-DEC-1996 |
| | | GB_BA1:MTY25D10 | 40838 | Z95558 | Mycobacterium tuberculosis H37Rv complete genome; segment 28/162. | Mycobacterium tuberculosis | 37,792 | 17-Jun-98 |
| | | GB_EST23:AI117213 | 476 | AI117213 | ub83h02.r1 Soares 2NbMT Mus musculus cDNA clone IMAGE:1395123 5',mRNA sequence. | Mus musculus | 35,084 | 2-Sep-98 |
| rxa02345 | 1320 | GB_BA1:BAPURKE | 2582 | X91189 | B.ammoniagenes purK and purE genes. | Corynebacterium ammoniagenes | 61,731 | 14-Jan-97 |
| | | GB_BA1:MTCY71 | 42729 | Z92771 | Mycobacterium tuberculosis H37Rv complete genome; segment 141/162. | Mycobacterium tuberculosis | 39,624 | 10-Feb-99 |
| | | GB_BA1:MTCY71 | 42729 | Z92771 | Mycobacterium tuberculosis H37Rv complete genome; segment 141/162. | Mycobacterium tuberculosis | 39,847 | 10-Feb-99 |
| rxa02350 | 618 | GB_BA1:BAPURKE | 2582 | X91189 | B.ammoniagenes purK and purE genes. | Corynebacterium ammoniagenes | 64,286 | 14-Jan-97 |
| | | GB_PL1:SC130KBXV | 129528 | X94335 | S.cerevisiae 130kb DNA fragment from chromosome XV. | Saccharomyces cerevisiae | 36,617 | 15-Jul-97 |
| | | GB_PL1:SCXVORFS | 50984 | X90518 | S.cerevisiae DNA of 51 Kb from chromosome XV right arm. | Saccharomyces cerevisiae | 36,617 | 1-Nov-95 |
| rxa02373 | 1038 | GB_PAT:E00311 | 1853 | E00311 | DNA coding of 2,5-diketogluconic acid reductase. | unidentified | 56,123 | 29-Sep-97 |
| | | GB_PAT:I06030 | 1853 | I06030 | Sequence 4 from Patent EP 0305608. | Unknown. | 56,220 | 02-DEC-1994 |
| | | GB_PAT:I00836 | 1853 | I00836 | Sequence 1 from Patent US 4758514. | Unknown. | 56,220 | 21-MAY-1993 |
| rxa02375 | 1350 | GB_BA2:CGU31230 | 3005 | U31230 | Corynebacterium glutamicum Obg protein homolog gene, partial cds, gamma glutamyl kinase (proB) gene, complete cds, and (unkdh) gene, complete cds. | Corynebacterium glutamicum | 99,332 | 2-Aug-96 |
| | | | | | | | | |
| | | GB_HTG3:AC009946 | 169072 | AC009946 | Homo sapiens clone NH0012C17, *** SEQUENCING IN PROGRESS ***, 1 unordered pieces. | Homo sapiens | 36,115 | 8-Sep-99 |
| | | GB_HTG3:AC009946 | 169072 | AC009946 | Homo sapiens clone NH0012C17, *** SEQUENCING IN PROGRESS ***, 1 unordered pieces. | Homo sapiens | 36,115 | 8-Sep-99 |
| rxa02380 | 777 | GB_BA1:MTCY253 | 41230 | Z81368 | Mycobacterium tuberculosis H37Rv complete genome; segment 106/162. | Mycobacterium tuberculosis | 38,088 | 17-Jun-98 |
| | | GB_HTG4:AC010658 | 120754 | AC010658 | Drosophila melanogaster chromosome 3L/75C1 clone RPCI98-3B20, *** SEQUENCING IN PROGRESS ***, 78 unordered pieces. | Drosophila melanogaster | 35,817 | 16-OCT-1999 |
| | | GB_HTG4:AC010658 | 120754 | AC010658 | Drosophila melanogaster chromosome 3L/75C1 clone RPCI98-3B20, *** SEQUENCING IN PROGRESS***, 78 unordered pieces. | Drosophila melanogaster | 35,817 | 16-OCT-1999 |
| rxa02382 | 1419 | GB_BA1:CGPROAGEN | 1783 | X82929 | C.glutamicum proA gene. | Corynebacterium glutamicum | 98,802 | 23-Jan-97 |
| | | | | | | | | |
| | | GB_BA1:MTCY428 | 26914 | Z81451 | Mycobacterium tuberculosis H37Rv complete genome; segment 107/162. | Mycobacterium tuberculosis | 38,054 | 17-Jun-98 |
| | | GB_BA2:CGU31230 | 3005 | U31230 | Corynebacterium glutamicum Obg protein homolog gene, partial cds, gamma glutamyl kinase (proB) gene, complete cds, and (unkdh) gene, complete cds. | Corynebacterium glutamicum | 98,529 | 2-Aug-96 |
| | | | | | | | | |
| rxa02400 | 693 | GB_BA1:CGACEA | 2427 | X75504 | C.glutamicum aceA gene and thiX genes (partial). | Corynebacterium glutamicum | 100,000 | 9-Sep-94 |
| | | GB_PAT:186191 | 2135 | 186191 | Sequence 3 from patent US 5700661. | Unknown. | 100,000 | 10-Jun-98 |
| | | GB_PAT:113693 | 2135 | I13693 | Sequence 3 from patent US 5439822. | Unknown. | 100,000 | 26-Sep-95 |
| rxa02432 | 1098 | GB_GSS15:AQ606842 | 574 | AQ606842 | HS_5404_B2_E07_T7A RPCI-11 Human Male BAC Library Homo sapiens genomic clone Plate=980 Col=14 Row=J, genomic survey sequence. | Homo sapiens | 39,716 | 10-Jun-99 |
| | | GB_EST1:T05804 | 406 | T05804 | EST03693 Fetal brain, Stratagene (cat#936206) Homo sapiens cDNA clone HFBDG63 similar to EST containing Alu repeat, mRNA sequence. | Homo sapiens | 37,915 | 30-Jun-93 |
| | | GB_PL1:AB006699 | 77363 | AB006699 | Arabidopsis thaliana genomic DNA, chromosome 5, P1 clone: MDJ22, complete sequence. | Arabidopsis thaliana | 35,526 | 20-Nov-99 |
| rxa02458 | 1413 | GB_BA2:AF114233 | 1852 | AF114233 | Corynebacterium glutamicum 5-enolpyruvylshikimate 3-phosphate synthase (aroA) gene, complete cds. | Corynebacterium glutamicum | 100,000 | 7-Feb-99 |
| | | GB_EST37:AW013061 | 578 | AW013061 | ODT-0033 Winter flounder ovary Pleuronectes americanus cDNA clone ODT-0033 5' similar to FRUCTOSE-BISPHOSPHATE ALDOLASE B (LIVER), mRNA sequence. | Pleuronectes americanus | 39,175 | 10-Sep-99 |
| | | GB_GSS15:AQ650027 | 728 | AQ650027 | Sheared DNA-5L2.TF Sheared DNA Trypanosoma brucei genomic clone Sheared DNA-5L2, genomic survey sequence. | Trypanosoma brucei | 39,281 | 22-Jun-99 |
| rxa02469 | 1554 | GB_BA1:MTCY359 | 36021 | Z83859 | Mycobacterium tuberculosis H37Rv complete genome; segment 84/162. | Mycobacterium tuberculosis | 39,634 | 17-Jun-98 |
| | | GB_BA1:MLCB1788 | 39228 | AL008609 | Mycobacterium leprae cosmid B1788. | Mycobacterium leprae | 59,343 | 27-Aug-99 |
| | | GB_BA1:SCAJ10601 | 4692 | AJ010601 | Streptomyces coelicolor A3(2) DNA for whiD and whiK loci. | Streptomyces coelicolor | 48,899 | 17-Sep-98 |
| rxa02497 | 1050 | GB_BA2:CGU31224 | 422 | U31224 | Corynebacterium glutamicum (ppx) gene, partial cds. | Corynebacterium glutamicum | 96,445 | 2-Aug-96 |
| | | GB_BA1:MTCY20G9 | 37218 | Z77162 | Mycobacterium tuberculosis H37Rv complete genome; segment 25/162. | Mycobacterium tuberculosis | 59,429 | 17-Jun-98 |
| | | GB_BA1:SCE7 | 16911 | AL049819 | Streptomyces coelicolor cosmid E7. | Streptomyces coelicolor | 39,510 | 10-MAY-1999 |
| rxa02499 | 933 | GB_BA2:CGU31225 | 1817 | U31225 | Corynebacterium glutamicum L-proline:NADP+ 5-oxidoreductase (proC) gene, complete cds. | Corynebacterium glutamicum | 97,749 | 2-Aug-96 |
| | | GB_BA1:NG17PILA | 1920 | X13965 | Neisseria gonorrhoeae pilA gene. | Neisseria gonorrhoeae | 43,249 | 30-Sep-93 |
| | | GB_HTG2:AC007984 | 129715 | AC007984 | Drosophila melanogaster chromosome 3 clone BACR05C10 (D781) RPCI-98 05.C.10 map 97D-97E strain y; cn bw sp, *** SEQUENCING IN PROGRESS ***, 87 unordered pieces. | Drosophila melanogaster | 33,406 | 2-Aug-99 |
| rxa02501 | 1188 | GB_BA1:MTCY20G9 | 37218 | Z77162 | Mycobacterium tuberculosis H37Rv complete genome; segment 25/162. | Mycobacterium tuberculosis | 39,357 | 17-Jun-98 |
| | | GB_BA1:U00018 | 42991 | U00018 | Mycobacterium leprae cosmid B2168. | Mycobacterium leprae | 51,768 | 01-MAR-1994 |
| | | GB_VI:HE1CG | 152261 | X14112 | Herpes simplex virus (HSV) type 1 complete genome. | human herpesvirus 1 | 39,378 | 17-Apr-97 |
| rxa02503 | 522 | GB_PR3:AC005328 | 35414 | AC005328 | Homo sapiens chromosome 19, cosmid R26660, complete sequence. | Homo sapiens | 39,922 | 28-Jul-98 |
| | | GB_PR3:AC005545 | 43514 | AC005545 | Homo sapiens chromosome 19, cosmid R26634, complete sequence. | Homo sapiens | 39,922 | 3-Sep-98 |
| | | GB_PR3:AC005328 | 35414 | AC005328 | Homo sapiens chromosome 19, cosmid R26660, complete sequence. | Homo sapiens | 34,911 | 28-Jul-98 |
| rxa02504 | 681 | GB_BA1:MTCY20G9 | 37218 | Z77162 | Mycobacterium tuberculosis H37Rv complete genome; segment 25/162. | Mycobacterium tuberculosis | 54,940 | 17-Jun-98 |
| | | GB_PR3:AC005328 | 35414 | AC005328 | Homo sapiens chromosome 19, cosmid R26660, complete sequence. | Homo sapiens | 41,265 | 28-Jul-98 |
| | | GB_PR3:AC005545 | 43514 | AC005545 | Homo sapiens chromosome 19, cosmid R26634, complete sequence. | Homo sapiens | 41,265 | 3-Sep-98 |
| rxa02516 | 1386 | GB_BA1:MLCL536 | 36224 | Z99125 | Mycobacterium leprae cosmid L536. | Mycobacterium leprae | 37,723 | 04-DEC-1998 |
| | | GB_BA1:U00013 | 35881 | U00013 | Mycobacterium leprae cosmid B1496. | Mycobacterium leprae | 37,723 | 01-MAR-1994 |
| | | GB_BA1:MTV007 | 32806 | AL021184 | Mycobacterium tuberculosis H37Rv complete genome; segment 64/162. | Mycobacterium tuberculosis | 61,335 | 17-Jun-98 |
| rxa02517 | 570 | GB_BA1:MLCL536 | 36224 | Z99125 | Mycobacterium leprae cosmid L536. | Mycobacterium leprae | 37,018 | 04-DEC-1998 |
| | | GB BA1:U00013 | 35881 | U00013 | Mycobacterium leprae cosmid B1496. | Mycobacterium leprae | 37,018 | 01-MAR-1994 |
| | | GB_BA1:SCC22 | 22115 | AL096839 | Streptomyces coelicolor cosmid C22. | Streptomyces coelicolor | 37,071 | 12-Jul-99 |
| rxa02532 | 1170 | GB_OV:AF137219 | 831 | AF137219 | Amia calva mixed lineage leukemia-like protein (Mil) gene, partial cds. | Amia calva | 36,853 | 7-Sep-99 |
| | | GB_EST30:AI64S0S7 | 301 | AI645057 | vs52a10.y1 Stratagene mouse Tcell 937311 Mus musculus cDNA clone IMAGE:1149882 5', mRNA sequence. | Mus musculus | 41,860 | 29-Apr-99 |
| | | GB_EST20:AA822595 | 429 | AA822595 | vs52a10.r1 Stratagene mouse Tcell 937311 Mus musculus cDNA clone IMAGE: 1149882 5', mRNA sequence. | Mus musculus | 42,353 | 17-Feb-98 |
| rxa02536 | 879 | GB_HTG2:AF130866 | 118874 | AF130866 | Homo sapiens chromosome 8 clone PAC 172N13 map 8q24, *** SEQUENCING IN PROGRESS ***, in unordered pieces. | Homo sapiens | 40,754 | 21-MAR-1999 |
| | | GB_HTG2:AF130866 | 118874 | AF130866 | Homo sapiens chromosome 8 clone PAC 172N13 map 8q24, *** SEQUENCING IN PROGRESS ***, in unordered pieces. | Homo sapiens | 40,754 | 21-MAR-1999 |
| | | GB_PL1:ATT12J5 | 84499 | AL035522 | Arabidopsis thaliana DNA chromosome 4, BAC clone T12J5 (ESSAII project). | Arabidopsis thaliana | 35,063 | 24-Feb-99 |
| | | | | | | | | |
| rxa02550 | 1434 | GB_BA1:MTCY279 | 9150 | Z97991 | Mycobacterium tuberculosis H37Rv complete genome; segment 17/162. | Mycobacterium tuberculosis | 37,773 | 17-Jun-98 |
| | | GB_BA1:MSGB1970CS | 39399 | L78815 | Mycobacterium leprae cosmid B1970 DNA sequence. | Mycobacterium leprae | 39,024 | 15-Jun-96 |
| | | | | | | | | |
| | | GB_BA2:SC2H4 | 25970 | AL031514 | Streptomyces coelicolor cosmid 2H4. | Streptomyces coelicolor A3(2) | 37,906 | 19-OCT-1999 |
| | | | | | | | | |
| rxa02559 | 1026 | GB_BA1:MTV004 | 69350 | AL009198 | Mycobacterium tuberculosis H37Rv complete genome; segment 144/162. | Mycobacterium tuberculosis | 47,358 | 18-Jun-98 |
| | | GB_PAT:128684 | 5100 | I28684 | Sequence 1 from patent US 5573915. | Unknown. | 39,138 | 6-Feb-97 |
| | | GB_BA1:MTU27357 | 5100 | U27357 | Mycobacterium tuberculosis cyclopropane mycolic acid synthase (cma1) gene, complete cds. | Mycobacterium tuberculosis | 39,138 | 26-Sep-95 |
| rxa02622 | 1683 | GB_BA2:AE001780 | 11997 | AE001780 | Thermotoga maritima section 92 of 136 of the complete genome. | Thermotoga maritima | 44,914 | 2-Jun-99 |
| | | GB_OV:AF064564 | 49254 | AF064564 | Fugu rubripes neurofibromatosis type 1 (NF1), A-kinase anchor protein (AKAP84), BAW protein (BAW), and WSB1 protein (WSB1) genes, complete cds. | Fugu rubripes | 39,732 | 17-Aug-99 |
| | | GB_OV:AF064564 | 49254 | AF064564 | Fugu rubripes neurofibromatosis type 1 (NF1), A-kinase anchor protein (AKAP84), BAW protein (BAW), and WSB1 protein (W5B1) genes, complete cds. | Fugu rubripes | 36,703 | 17-Aug-99 |
| rxa02623 | 714 | GB_GSS5:AQ818728 | 444 | AQ818728 | HS_5268_A1_G09_SP6E RPCI-11 Human Male BAC Library Homo sapiens genomic clone Plate=844 Col=17 Row=M, genomic survey sequence. | Homo sapiens | 38,801 | 26-Aug-99 |
| | | | | | | | | |
| | | GB_HTG5:AC011083 | 198586 | AC011083 | Homo sapiens chromosome 9 clone RP11-111M7 map 9, WORKING DRAFT SEQUENCE, 51 unordered pieces. | Homo sapiens | 35,714 | 19-Nov-99 |
| | | GB_GSS6:AQ826948 | 544 | AQ826948 | HS_5014_A2_C12_T7A RPCI-11 Human Male BAC Library Homo sapiens genomic clone Pale=590 Col=24 Row=E, genomic survey sequence. | Homo sapiens | 39,146 | 27-Aug-99 |
| rxa02629 | 708 | GB_VI:BRSMGP | 462 | M86652 | Bovine respiratory syncytial virus membrane glycoprotein mRNA, complete cds. | Bovine respiratory syncytial virus | 37,013 | 28-Apr-93 |
| | | GB_VI:BRSMGP | 462 | M86652 | Bovine respiratory syncytial virus membrane glycoprotein mRNA, complete cds. | Bovine respiratory syncytial virus | 37,013 | 28-Apr-93 |
| rxa02645 | 1953 | GB_PAT:A45577 | 1925 | A45577 | Sequence 1 from Patent WO9519442. | Corynebacterium glutamicum | 39,130 | 07-MAR-1997 |
| | | GB_PAT:A45581 | 1925 | A45581 | Sequence 5 from Patent WO9519442. | Corynebacterium glutamicum | 39,130 | 07-MAR-1997 |
| | | GB_BA1:CORILVA | 1925 | L01508 | Corynebacterium glutamicum threonine dehydratase (ilvA) gene, complete cds. | Corynebacterium glutamicum | 39,130 | 26-Apr-93 |
| rxa02646 | 1392 | GB_BA1:CORILVA | 1925 | L01508 | Corynebacterium glutamicum threonine dehydratase (ilvA) gene, complete cds. | Corynebacterium glutamicum | 99,138 | 26-Apr-93 |
| | | GB_PAT:A45585 | 1925 | A45585 | Sequence 9 from Patent WO9519442. | Corynebacterium glutamicum | 99,066 | 07-MAR-1997 |
| | | GB_PAT:A45563 | 1925 | A45583 | Sequence 7 from Patent WO9519442. | Corynebacterium glutamicum | 99,066 | 07-MAR-1997 |
| rxa02648 | 1326 | GB_OV:ICTCNC | 2049 | M83111 | Ictalurus punctatus cyclic nucleotide-gated channel RNA sequence. | Ictalurus punctatus | 38,402 | 24-MAY-1993 |
| | | GB_EST11:AA265464 | 345 | AA265464 | mx91 c06.r1 Soares mouse NML Mus musculus cDNA clone IMAGE:693706 5', mRNA sequence. | Mus musculus | 38,655 | 20-MAR-1997 |
| | | GB_GSS8:AQ006950 | 480 | AQ006950 | CIT-HSP-2294E14.TR CIT-HSP Homo sapiens genomic clone 2294E14, genomic survey sequence. | Homo sapiens | 36,074 | 27-Jun-98 |
| rxa02653 | | | | | | | | |
| | | | | | | | | |
| rxa02687 | 1068 | GD_BA1:CORPHEA | 1088 | M13774 | C.glutamicum pheA gene encoding prephenate dehydratase, complete cds. | Corynebacterium glutamicum | 99,715 | 26-Apr-93 |
| | | GB_PAT:E04483 | 948 | E04483 | DNA encoding prephenate dehydratase. | Corynebacterium glutamicum | 98,523 | 29-Sep-97 |
| | | GB_PAT:E06110 | 948 | E06110 | DNA encoding prephenate dehydratase. | Corynebacterium glutamicum | 98,523 | 29-Sep-97 |
| rxa02717 | 1005 | GB_PL1:HVCH4H | 59748 | Y14573 | Hordeum vulgare DNA for chromosome 4H. | Hordeum vulgare | 36,593 | 25-MAR-1999 |
| | | GB_PR2:HS310H5 | 29718 | Z69705 | Human DNA sequence from cosmid 310H5 from a contig from the tip of the short arm of chromosome 16, spanning 2Mb of 16p13.3. Contains EST and CpG island. | Homo sapiens | 36,089 | 22-Nov-99 |
| | | GB_PR3:AC004754 | 39188 | AC004754 I | Homo sapiens chromosome 16, cosmid clone RT286 (LANL), complete sequence. | Homo sapiens | 36,089 | 28-MAY-1998 |
| rxa02754 | 1461 | GB_HTG2:AC008223 | 130212 | AC008223 | Drosophila melanogaster chromosome 3 clone BACR16I18 (D815) RPCI-98 16.I.18 map 95A-95A strain y; cn bw sp, *** SEQUENCING IN PROGRESS***, 101 unordered pieces. | Drosophila melanogaster | 32,757 | 2-Aug-99 |
| | | GB_HTG2:AC008223 | 130212 | AC008223 3 | Drosophila melanogaster chromosome 3 clone BACR16I18 (D815) RPCI-98 16.I.18 map 95A-95A strain y; cn bw sp, *** SEQUENCING IN PROGRESS ***, 101 unordered pieces. | Drosophila melanogaster | 32,757 | 2-Aug-99 |
| | | GB_BA1:MTCY71 | 42729 | Z92771 | Mycobacterium tuberculosis H37Rv complete genome; segment 141/162. | Mycobacterium tuberculosis | 37,838 | 10-Feb-99 |
| rxa02758 | 1422 | GB_HTG5:AC011678 | 171967 | AC011678 | Homo sapiens clone 14_B_7, *** SEQUENCING IN PROGRESS ***, 20 unordered pieces. | Homo sapiens | 35,331 | 5-Nov-99 |
| | | GB_HTG5:AC011678 | 171967 | AC011678 | Homo sapiens clone 14_B_7, *** SEQUENCING IN PROGRESS ***, 20 unordered pieces. | Homo sapiens | 33,807 | 5-Nov-99 |
| | | GB_BA2:AF064070 | 23183 | AF064070 | Burkholderia pseudomallei putative dihydroorotase (pyrC) gene, partial cds; putative 1-acyl-sn-glycerol-3-phosphate acyltransferase (plsC), putative diadenosine tetraphosphatase (apaH), complete cds; type II O-antigen biosynthesis gene cluster, complete sequence; putative undecaprenyl phosphate N-acetylglucosaminyltransferase, and putative UDP-glucose 4-epimerase genes, complete cds; and putative galactosyl transferase gene, partial cds. | Burkholderia pseudomallei | 36,929 | 20-Jan-99 |
| rxa02771 | 678 | GB_BA2:AF038651 | 4077 | AF038651 | Corynebacterium glulamicum dipeptide-binding protein (dciAE) gene, partial cds; adenine phosphoribosyltransferase (apt) and GTP pyrophosphokinase (rel) genes, complete cds; and unknown gene. | Corynebacterium glutamicum | 99,852 | 14-Sep-98 |
| | | GB_IN1:CELT19B4 | 37121 | U80438 | Caenorhabditis elegans cosmid T19B4. | Caenorhabditis elegans | 43,836 | 04-DEC-1996 |
| | | GB_EST36:AV193572 | 360 | AV193572 | AV193572 Yuji Kohara unpublished cDNA:Strain N2 hermaphrodite embryo Caenorhabditis elegans cDNA clone yk618h8 5', mRNA sequence. | Caenorhabditis elegans | 48,588 | 22-Jul-99 |
| rxa02772 | 1158 | GB_BA2:AF038651 | 4077 | AF038651 | Corynebacterium glutamicum dipeptide-binding protein (dciAE) gene, partial cds; adenine phosphoribosyltransferase (apt) and GTP pyrophosphokinase (rel) genes, complete cds; and unknown gene. | Corynebacterium glutamicum | 99,914 | 14-Sep-98 |
| | | GB_BA1:MTCY227 | 35946 | Z77724 | Mycobacterium tuberculosis H37Rv complete genome; segment 114/162. | Mycobacterium tuberculosis | 38,339 | 17-Jun-98 |
| | | GB_BA1:U00011 | 40429 | U00011 | Mycobacterium leprae cosmid B1177. | Mycobacterium leprae | 38,996 | 01-MAR-1994 |
| rxa02790 | 1266 | GB_BA1:MTCY159 | 33818 | Z83863 | Mycobacterium tuberculosis H37Rv complete genome; segment 111/162. | Mycobacterium tuberculosis | 37,640 | 17-Jun-98 |
| | | GB_PR4:AC006581 | 172931 | AC006581 | Homo sapiens 12p21 BAC RPCI11-259O18 (Roswell Park Cancer Institute Human BAC Library) complete sequence. | Homo sapiens | 37,906 | 3-Jun-99 |
| | | GB_PR4:AC006581 | 172931 | AC006581 | Homo sapiens 12p21 BAC RPC111-259018 (Roswell Park Cancer Institute Human BAC Library) complete sequence. | Homo sapiens | 35,280 | 3-Jun-99 |
| rxa02791 | 951 | GB_BA1:MTCY159 | 33818 | Z83863 | Mycobacterium tuberculosis H37Rv complete genome; segment 111/162. | Mycobacterium tuberculosis | 39,765 | 17-Jun-98 |
| | | GB_OV:CHKCEK2 | 3694 | M35195 | Chicken tyrosine kinase (cek2) mRNA, complete cds. | Gallus gallus | 38,937 | 28-Apr-93 |
| | | GB_BA1:MSASDASK | 5037 | Z17372 | M.smegmatis asd, ask-alpha, and ask-beta genes. | Mycobacterium smegmatis | 38,495 | 9-Aug-94 |
| rxa02802 | 1194 | GB_EST24:AI223401 | 169 | AI223401 | qg48g01.x1 Soares_testis_NHT Homo sapiens cDNA clone IMAGE:1838448 3' similar to WP:C25D7.8 CE08394 ;, mRNA sequence. | Homo sapiens | 40,828 | 27-OCT-1998 |
| | | GB_EST24:AI223401 | 169 | AI223401 | qg48g01.x1 Soares_testis_NHT Homo sapiens cDNA clone IMAGE:1838448 3' similar to WP:C25D7.8 CE08394 ;, mRNA sequence. | Homo sapiens | 40,828 | 27-OCT-1998 |
| | | | | | | | | |
| rxa02814 | 494 | GB_BA1:MTCY7D11 | 22070 | Z95120 | Mycobacterium tuberculosis H37Rv complete genome; segment 138/162. | Mycobacterium tuberculosis | 58,418 | 17-Jun-98 |
| | | GB_BA1:MTCY7D11 | 22070 | Z95120 | Mycobacterium tuberculosis H37Rv complete genome; segment 138/162. | Mycobacterium tuberculosis | 40,496 | 17-Jun-98 |
| | | GB_PR1:HSAJ2962 | 778 | AJ002962 | Homo sapiens mRNA for hB-FABP. | Homo sapiens | 39,826 | 8-Jan-98 |
| rxa02843 | 608 | GB_BA1:CGAJ4934 | 1160 | AJ004934 | Corynebacterium glutamicum dapD gene, complete CDS. | Corynebacterium glutamicum | 100,000 | 17-Jun-98 |
| | | GB_BA1:MTCI364 | 29540 | Z93777 | Mycobacterium tuberculosis H37Rv complete genome; segment 52/162. | Mycobacterium tuberculosis | 37,710 | 17-Jun-98 |
| | | GB_BA1:MLU15180 | 38675 | U15180 | Mycobacterium leprae cosmid B1756. | Mycobacterium leprae | 39,626 | 09-MAR-1995 |
| rxs03205 | 963 | GB_BA1:BLSIGBGN | 2906 | Z49824 | B.lactofermentum orf1 gene and sigB gene. | Corynebacterium glutamicum | 98,854 | 25-Apr-96 |
| | | GB_EST21:A980237 | 377 | AA980237 | ua32a12.r1 Soares_mammary_gland_NbMMG Mus musculus cDNA clone IMAGE:1348414 5' similar to TR:Q61025 Q61025 HYPOTHETICAL 152 KD PROTEIN. ;, mRNA sequence. | Mus musculus | 41,489 | 27-MAY-1998 |
| | | GB_EST23:AI158316 | 371 | AI158316 | ud27c05.r1 Soares_thymus_2NbMT Mus musculus cDNA clone IMAGE: 1447112 5', mRNA sequence. | Mus musculus | 38,005 | 30-Sep-98 |
| rxs03223 | 1237 | GB_IN1:LMFL2743 | 38368 | AL031910 | Leishmania major Friedlin chromosome 4 cosmid L2743. | Leishmania major | 39,869 | 15-DEC-1999 |
| | | GB_PR3:HSDJ61B2 | 119666 | AL096710 | Human DNA sequence from clone RP1-61B2 on chromosome 6p11.2-12.3 Contains isoforms 1 and 3 of BPAG1 (bullous pemphigoid antigen 1 (230/240kD), an exon of a gene similar to murine MACF cytoskeletal protein, STSs and GSSs, complete sequence. | Homo sapiens | 34,930 | 17-DEC-1999 |
| | | GB_PR3:HSDJ61B2 | 119666 | AL096710 | Human DNA sequence from clone RP1-61B2 on chromosome 6p11.2-12.3 Contains isoforms 1 and 3 of BPAG1 (bullous pemphigoid antigen 1 (230/240kD), an exon of a gene similar to murine MACF cytoskeletal protein, STSs and GSSs, complete sequence. | Homo sapiens | 34,634 | 17-DEC-1999 |

### SEQUENCE LISTING

<110> BASF Aktiengesellschaft
<120> CORYNEBACTERIUM GLUTAMICUM GENES ENCODING METABOLIC PATHWAY PROTEINS
<130> BGI-121CP2PC
<140>
   <141>
<150> 09/606740
   <151> 2000-06-23
<150> 60/187970
   <151> 2000-03-09
<160> 125
<170> PatentIn Vers. 2.0
<210> 1
   <211> 1840
   <212> DNA
   <213> Corynebacterium glutamicum
<220>
   <221> CDS
   <222> (363)..(1676)
<400> 1
   cagaaactgt gtgcagaaat gcatgcagaa aaaggaaagt tcgggccaag atgggtgttt 60
   ctgtatgccg atgatcggat ctttgacagc tgggtatgcg acaaatcacc gagagttgtt 120
   aattcttaac aatggaaaag taacattgag agatgattta taccatcctg caccatttag 180
   agtggggcta gtcatacccc cataacccta gctgtacgca atcgatttca aatcagttgg 240
   aaaaagtcaa gaaaattacc cgagaattaa tttataccac acagtctatt gcaatagacc 300
   aagctgttca gtagggtgca tgggagaaga atttcctaat aaaaactctt aaggacctcc 360
   aa atg cca aag tac gac aat tcc aat gct gac cag tgg ggc ttt gaa    407
      Met Pro Lys Tyr Asp Asn Ser Asn Ala Asp Gln Trp Gly Phe Glu
       1               5                  10                  15
   acc cgc tcc att cac gca ggc cag tca gta gac gca cag acc agc gca    455
   Thr Arg Ser Ile His Ala Gly Gln Ser Val Asp Ala Gln Thr Ser Ala
                    20                  25                  30
   cga aac ctt ccg atc tac caa tcc acc gct ttc gtg ttc gac tcc gct    503
   Arg Asn Leu Pro Ile Tyr Gln Ser Thr Ala Phe Val Phe Asp Ser Ala
                35                  40                  45
   gag cac gcc aag cag cgt ttc gca ctt gag gat cta ggc cct gtt tac    551
   Glu His Ala Lys Gln Arg Phe Ala Leu Glu Asp Leu Gly Pro Val Tyr
            50                  55                  60
   tcc cgc ctc acc aac cca acc gtt gag gct ttg gaa aac cgc atc gct    599
   Ser Arg Leu Thr Asn Pro Thr Val Glu Ala Leu Glu Asn Arg Ile Ala
        65                  70                  75
   tcc ctc gaa ggt ggc gtc cac gct gta gcg ttc tcc tcc gga cag gcc     647
   Ser Leu Glu Gly Gly Val His Ala Val Ala Phe Ser Ser Gly Gln Ala
    80                  85                  90                  95
   gca acc acc aac gcc att ttg aac ctg gca gga gcg ggc gac cac atc     695
   Ala Thr Thr Asn Ala Ile Leu Asn Leu Ala Gly Ala Gly Asp His Ile
                   100                 105                 110
   gtc acc tcc cca cgc ctc tac ggt ggc acc gag act cta ttc ctt atc     743
   Val Thr Ser Pro Arg Leu Tyr Gly Gly Thr Glu Thr Leu Phe Leu Ile
               115                 120                 125
   act ctt aac cgc ctg ggt atc gat gtt tcc ttc gtg gaa aac ccc gac     791
   Thr Leu Asn Arg Leu Gly Ile Asp Val Ser Phe Val Glu Asn Pro Asp
           130                 135                 140
   gac cct gag tcc tgg cag gca gcc gtt cag cca aac acc aaa gca ttc     839
   Asp Pro Glu Ser Trp Gln Ala Ala Val Gln Pro Asn Thr Lys Ala Phe
       145                 150                 155
   ttc ggc gag act ttc gcc aac cca cag gca gac gtc ctg gat att cct     887
   Phe Gly Glu Thr Phe Ala Asn Pro Gln Ala Asp Val Leu Asp Ile Pro
   160                 165                 170                 175
   gcg gtg gct gaa gtt gcg cac cgc aac agc gtt cca ctg atc atc gac     935
   Ala Val Ala Glu Val Ala His Arg Asn Ser Val Pro Leu Ile Ile Asp
                   180                 185                 190
   aac acc atc gct acc gca gcg ctc gtg cgc ccg ctc gag ctc ggc gca     983
   Asn Thr Ile Ala Thr Ala Ala Leu Val Arg Pro Leu Glu Leu Gly Ala
               195                 200                 205
   gac gtt gtc gtc gct tcc ctc acc aag ttc tac acc ggc aac ggc tcc    1031
   Asp Val Val Val Ala Ser Leu Thr Lys Phe Tyr Thr Gly Asn Gly Ser
           210                 215                 220
   gga ctg ggc ggc gtg ctt atc gac ggc gga aag ttc gat tgg act gtc    1079
   Gly Leu Gly Gly Val Leu Ile Asp Gly Gly Lys Phe Asp Trp Thr Val
       225                 230                 235
   gaa aag gat gga aag cca gta ttc ccc tac ttc gtc act cca gat gct    1127
   Glu Lys Asp Gly Lys Pro Val Phe Pro Tyr Phe Val Thr Pro Asp Ala
   240                 245                 250                 255
   gct tac cac gga ttg aag tac gca gac ctt ggt gca cca gcc ttc ggc    1175
   Ala Tyr His Gly Leu Lys Tyr Ala Asp Leu Gly Ala Pro Ala Phe Gly
                   260                 265                 270
   ctc aag gtt cgc gtt ggc ctt cta cgc gac acc ggc tcc acc ctc tcc    1223
   Leu Lys Val Arg Val Gly Leu Leu Arg Asp Thr Gly Ser Thr Leu Ser
               275                 280                 285
   gca ttc aac gca tgg gct gca gtc cag ggc atc gac acc ctt tcc ctg    1271
   Ala Phe Asn Ala Trp Ala Ala Val Gln Gly Ile Asp Thr Leu Ser Leu
           290                 295                 300
   cgc ctg gag cgc cac aac gaa aac gcc atc aag gtt gca gaa ttc ctc    1319
   Arg Leu Glu Arg His Asn Glu Asn Ala Ile Lys Val Ala Glu Phe Leu
       305                 310                 315
   aac aac cac gag aag gtg gaa aag gtt aac ttc gca ggc ctg aag gat     1367
   Asn Asn His Glu Lys Val Glu Lys Val Asn Phe Ala Gly Leu Lys Asp
   320                 325                 330                 335
   tcc cct tgg tac gca acc aag gaa aag ctt ggc ctg aag tac acc ggc     1415
   Ser Pro Trp Tyr Ala Thr Lys Glu Lys Leu Gly Leu Lys Tyr Thr Gly
                   340                 345                 350
   tcc gtt ctc acc ttc gag atc aag ggc ggc aag gat gag gct tgg gca     1463
   Ser Val Leu Thr Phe Glu Ile Lys Gly Gly Lys Asp Glu Ala Trp Ala
               355                 360                 365
   ttt atc gac gcc ctg aag cta cac tcc aac ctt gca aac atc ggc gat     1511
   Phe Ile Asp Ala Leu Lys Leu His Ser Asn Leu Ala Asn Ile Gly Asp
           370                 375                 380
   gtt cgc tcc ctc gtt gtt cac cca gca acc acc acc cat tca cag tcc     1559
   Val Arg Ser Leu Val Val His Pro Ala Thr Thr Thr His Ser Gln Ser
       385                 390                 395
   gac gaa gct ggc ctg gca cgc gcg ggc gtt acc cag tcc acc gtc cgc     1607
   Asp Glu Ala Gly Leu Ala Arg Ala Gly Val Thr Gln Ser Thr Val Arg
   400                 405                 410                 415
   ctg tcc gtt ggc atc gag acc att gat gat atc atc gct gac ctc gaa     1655
   Leu Ser Val Gly Ile Glu Thr Ile Asp Asp Ile Ile Ala Asp Leu Glu
                   420                 425                 430
   ggc ggc ttt gct gca atc tag ctttaaatag actcacccca gtgcttaaag        1706
   Gly Gly Phe Ala Ala Ile
               435
   cgctgggttt ttctttttca gactcgtgag aatgcaaact agactagaca gagctgtcca 1766
   tatacactgg acgaagtttt agtcttgtcc acccagaaca ggcggttatt ttcatgccca 1826
   ccctcgcgcc ttca                                                  1840 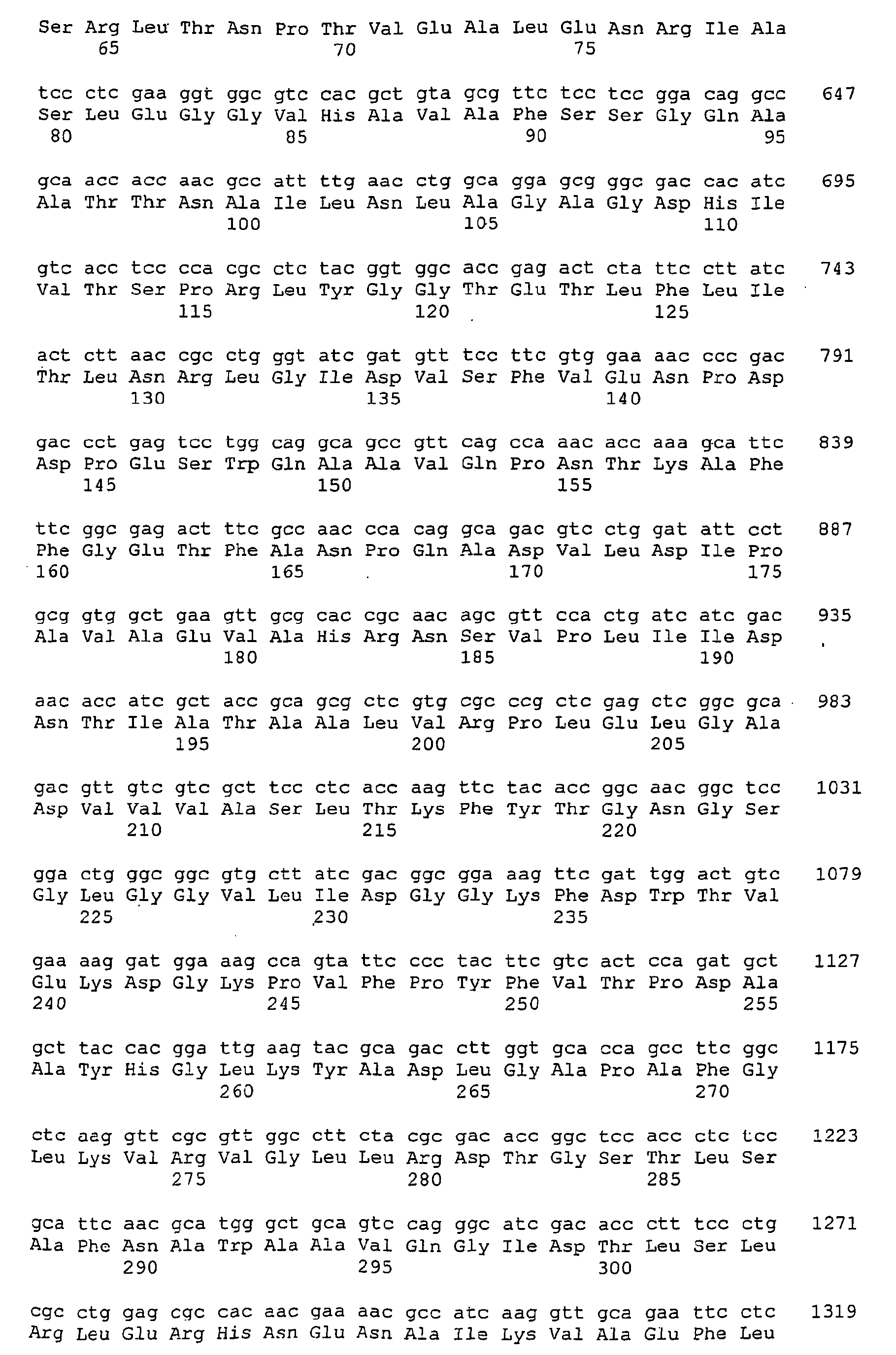
<210> 2
   <211> 437
   <212> PRT
   <213> Corynebacterium glutamicum
<400> 2
   Met Pro Lys Tyr Asp Asn Ser Asn Ala Asp Gln Trp Gly Phe Glu Thr
    1               5                  10                  15
   Arg Ser Ile His Ala Gly Gln Ser Val Asp Ala Gln Thr Ser Ala Arg
               20                  25                  30
   Asn Leu Pro Ile Tyr Gln Ser Thr Ala Phe Val Phe Asp Ser Ala Glu
           35                  40                  45
   His Ala Lys Gln Arg Phe Ala Leu Glu Asp Leu Gly Pro Val Tyr Ser
       50                  55                  60
   Arg Leu Thr Asn Pro Thr Val Glu Ala Leu Glu Asn Arg Ile Ala Ser
   65                  70                  75                  80
   Leu Glu Gly Gly Val His Ala Val Ala Phe Ser Ser Gly Gln Ala Ala
   85 90 95
   Thr Thr Asn Ala Ile Leu Asn Leu Ala Gly Ala Gly Asp His Ile Val
   100 105 110
   Thr Ser Pro Arg Leu Tyr Gly Gly Thr Glu Thr Leu Phe Leu Ile Thr
   115 120 125
   Leu Asn Arg Leu Gly Ile Asp Val Ser Phe Val Glu Asn Pro Asp Asp
   130 135 140
   Pro Glu Ser Trp Gln Ala Ala Val Gln Pro Asn Thr Lys Ala Phe Phe
   145 150 155 160
   Gly Glu Thr Phe Ala Asn Pro Gln Ala Asp Val Leu Asp Ile Pro Ala
   165 170 175
   Val Ala Glu Val Ala His Arg Asn Ser Val Pro Leu Ile Ile Asp Asn
   180 185 190
   Thr Ile Ala Thr Ala Ala Leu Val Arg Pro Leu Glu Leu Gly Ala Asp
   195 200 205
   Val Val Val Ala Ser Leu Thr Lys Phe Tyr Thr Gly Asn Gly Ser Gly
   210 215 220
   Leu Gly Gly Val Leu Ile Asp Gly Gly Lys Phe Asp Trp Thr Val Glu
   225 230 235 240
   Lys Asp Gly Lys Pro Val Phe Pro Tyr Phe Val Thr Pro Asp Ala Ala
   245 250 255
   Tyr His Gly Leu Lys Tyr Ala Asp Leu Gly Ala Pro Ala Phe Gly Leu
   260 265 270
   Lys Val Arg Val Gly Leu Leu Arg Asp Thr Gly Ser Thr Leu Ser Ala
   275 280 285
   Phe Asn Ala Trp Ala Ala Val Gln Gly Ile Asp Thr Leu Ser Leu Arg
   290 295 300
   Leu Glu Arg His Asn Glu Asn Ala Ile Lys Val Ala Glu Phe Leu Asn
   305 310 315 320
   Asn His Glu Lys Val Glu Lys Val Asn Phe Ala Gly Leu Lys Asp Ser
   325 330 335
   Pro Trp Tyr Ala Thr Lys Glu Lys Leu Gly Leu Lys Tyr Thr Gly Ser
   340 345 350
   Val Leu Thr Phe Glu Ile Lys Gly Gly Lys Asp Glu Ala Trp Ala Phe
   355 360 365
   Ile Asp Ala Leu Lys Leu His Ser Asn Leu Ala Asn Ile Gly Asp Val
   370 375 380
   Arg Ser Leu Val Val His Pro Ala Thr Thr Thr His Ser Gln Ser Asp
   385 390 395 400
   Glu Ala Gly Leu Ala Arg Ala Gly Val Thr Gln Ser Thr Val Arg Leu
   405 410 415
   Ser Val Gly Ile Glu Thr Ile Asp Asp Ile Ile Ala Asp Leu Glu Gly
   420 425 430
   Gly Phe Ala Ala Ile
   435
<210> 3
   <211> 1495
   <212> DNA
   <213> Corynebacterium glutamicum
<220>
   <221> CDS
   <222> (287)..(1264)
<400> 3
   ccatggtttc ctcagcggaa acggcttggc tatcagcact ttcacccgaa cagcctgcaa 60
   gaagtgcgac ggctaacagg gctgggattg tcctcaactt cacttcgggc tccttcttag 120
   taataggttc gtagaaaagt ttactagcct agagagtatg cgatttcctg aactcgaaga 180
   attgaagaat cgccggacct tgaaatggac ccggtttcca gaagacgtgc ttcctttgtg 240
   ggttgcggaa agtgattttg gcacctgccc gcagttgaag gaagct atg gca gat    295
                                                     Met Ala Asp
                                                      1
   gcc gtt gag cgc gag gtc ttc gga tac cca cca gat gct act ggg ttg   343
   Ala Val Glu Arg Glu Val Phe Gly Tyr Pro Pro Asp Ala Thr Gly Leu
       5                   10                  15
   aat gat gcg ttg act gga ttc tac gag cgt cgc tat ggg ttt ggc cca   391
   Asn Asp Ala Leu Thr Gly Phe Tyr Glu Arg Arg Tyr Gly Phe Gly Pro
    20                 25                  30                  35
   aat ccg gaa agt gtt ttc gcc att ccg gat gtg gtt cgt ggc ctg aag   439
   Asn Pro Glu Ser Val Phe Ala Ile Pro Asp Val Val Arg Gly Leu Lys
                   40                  45                  50
   ctt gcc att gag cat ttc act aag cct ggt tcg gcg atc att gtg ccg   487
   Leu Ala Ile Glu His Phe Thr Lys Pro Gly Ser Ala Ile Ile Val Pro
               55                  60                  65
   ttg cct gca tac cct cct ttc att gag ttg cct aag gtg act ggt cgt   535
   Leu Pro Ala Tyr Pro Pro Phe Ile Glu Leu Pro Lys Val Thr Gly Arg
           70                  75                  80
   cag gcg atc tac att gat gcg cat gag tac gat ttg aag gaa att gag   583
   Gln Ala Ile Tyr Ile Asp Ala His Glu Tyr Asp Leu Lys Glu Ile Glu
       85                  90                  95
   aag gcc ttc gct gac ggt gcg gga tca ctg ttg ttc tgc aat cca cac   631
   Lys Ala Phe Ala Asp Gly Ala Gly Ser Leu Leu Phe Cys Asn Pro His
   100                 105                 110                 115
   aac cca ctg ggc acg gtc ttt tct gaa gag tac atc cgc gag ctc acc   679
   Asn Pro Leu Gly Thr Val Phe Ser Glu Glu Tyr Ile Arg Glu Leu Thr
                   120                 125                 130
   gat att gcg gcg aag tac gat gcc cgc atc atc gtc gat gag atc cac     727
   Asp Ile Ala Ala Lys Tyr Asp Ala Arg Ile Ile Val Asp Glu Ile His
               135                 140                 145
   gcg cca ctg gtt tat gaa ggc acc cat gtg gtt gct gct ggt gtt tct     775
   Ala Pro Leu Val Tyr Glu Gly Thr His Val Val Ala Ala Gly Val Ser
           150                 155                 160
   gag aac gct gca aac act tgc atc acc atc acc gca act tct aag gcg     823
   Glu Asn Ala Ala Asn Thr Cys Ile Thr Ile Thr Ala Thr Ser Lys Ala
       165                 170                 175
   tgg aac act gct ggt ttg aag tgt gct cag atc ttc ttc agt aat gaa     871
   Trp Asn Thr Ala Gly Leu Lys Cys Ala Gln Ile Phe Phe Ser Asn Glu
   180                 185                 190                 195
   gcc gat gtg aag gcc tgg aag aat ttg tcg gat att acc cgt gac ggt     919
   Ala Asp Val Lys Ala Trp Lys Asn Leu Ser Asp Ile Thr Arg Asp Gly
                   200                 205                 210
   gtg tcc atc ctt gga ttg atc gct gcg gag aca gtg tac aac gag ggc     967
   Val Ser Ile Leu Gly Leu Ile Ala Ala Glu Thr Val Tyr Asn Glu Gly
               215                 220                 225
   gaa gaa ttc ctt gat gag tca att cag att ctc aag gac aac cgt gac    1015
   Glu Glu Phe Leu Asp Glu Ser Ile Gln Ile Leu Lys Asp Asn Arg Asp
           230                 235                 240
   ttt gcg gct gct gaa ctg gaa aag ctt ggc gtg aag gtc tac gca ccg    1063
   Phe Ala Ala Ala Glu Leu Glu Lys Leu Gly Val Lys Val Tyr Ala Pro
       245                 250                 255
   gac tcc act tat ttg atg tgg ttg gac ttc gct ggc acc aag atc gaa    1111
   Asp Ser Thr Tyr Leu Met Trp Leu Asp Phe Ala Gly Thr Lys Ile Glu
   260                 265                 270                 275
   gag gcg cct tct aaa att ctt cgt gag gag ggt aag gtc atg ctg aat    1159
   Glu Ala Pro Ser Lys Ile Leu Arg Glu Glu Gly Lys Val Met Leu Asn
                   280                 285                 290
   gat ggc gca gct ttt ggt ggt ttc acc acc tgc gct cgt ctt aat ttt    1207
   Asp Gly Ala Ala Phe Gly Gly Phe Thr Thr Cys Ala Arg Leu Asn Phe
               295                 300                 305
   gcg tgt tcc aga gag acc ctt gag gag ggg ctg cgc cgt atc gcc agc    1255
   Ala Cys Ser Arg Glu Thr Leu Glu Glu Gly Leu Arg Arg Ile Ala Ser
           310                 315                 320
   gtg ttg taa ataatgagta aaaagtctgt cctgattact tctttgatgc            1304
   Val Leu
       325
   tgttttccat gttcttcgga gctggaaacc tcatcttccc gccgatgctt ggattgtcgg 1364
   caggaaccaa ctatctacca gctatcttag gatttctagc aacgagtgtt ctgctcccgg 1424
   tgctggcgat tatcgcggtg gtgttgtcgg gagaaaatgt caaggacatg gcttctcgtg 1484
   gcggtaagat c 1495
<210> 4
   <211> 325
   <212> PRT
   <213> Corynebacterium glutamicum
<400> 4
   Met Ala Asp Ala Val Glu Arg Glu Val Phe Gly Tyr Pro Pro Asp Ala
    1               5                  10                  15
   Thr Gly Leu Asn Asp Ala Leu Thr Gly Phe Tyr Glu Arg Arg Tyr Gly
               20                  25                  30
   Phe Gly Pro Asn Pro Glu Ser Val Phe Ala Ile Pro Asp Val Val Arg
           35                  40                  45
   Gly Leu Lys Leu Ala Ile Glu His Phe Thr Lys Pro Gly Ser Ala Ile
       50                  55                  60
   Ile Val Pro Leu Pro Ala Tyr Pro Pro Phe Ile Glu Leu Pro Lys Val
   65                  70                  75                  80
   Thr Gly Arg Gln Ala Ile Tyr Ile Asp Ala His Glu Tyr Asp Leu Lys
                   85                  90                  95
   Glu Ile Glu Lys Ala Phe Ala Asp Gly Ala Gly Ser Leu Leu Phe Cys
               100                 105                 110
   Asn Pro His Asn Pro Leu Gly Thr Val Phe Ser Glu Glu Tyr Ile Arg
           115                 120                 125
   Glu Leu Thr Asp Ile Ala Ala Lys Tyr Asp Ala Arg Ile Ile Val Asp
       130                 135                 140
   Glu Ile His Ala Pro Leu Val Tyr Glu Gly Thr His Val Val Ala Ala
   145                 150                 155                 160
   Gly Val Ser Glu Asn Ala Ala Asn Thr Cys Ile Thr Ile Thr Ala Thr
                   165                 170                 175
   Ser Lys Ala Trp Asn Thr Ala Gly Leu Lys Cys Ala Gln Ile Phe Phe
               180                 185                 190
   Ser Asn Glu Ala Asp Val Lys Ala Trp Lys Asn Leu Ser Asp Ile Thr
           195                 200                 205
   Arg Asp Gly Val Ser Ile Leu Gly Leu Ile Ala Ala Glu Thr Val Tyr
       210                 215                 220
   Asn Glu Gly Glu Glu Phe Leu Asp Glu Ser Ile Gln Ile Leu Lys Asp
   225                 230                 235                 240
   Asn Arg Asp Phe Ala Ala Ala Glu Leu Glu Lys Leu Gly Val Lys Val
                   245                 250                 255
   Tyr Ala Pro Asp Ser Thr Tyr Leu Met Trp Leu Asp Phe Ala Gly Thr
               260                 265                 270
   Lys Ile Glu Glu Ala Pro Ser Lys Ile Leu Arg Glu Glu Gly Lys Val
   275 280 285
   Met Leu Asn Asp Gly Ala Ala Phe Gly Gly Phe Thr Thr Cys Ala Arg
   290 295 300
   Leu Asn Phe Ala Cys Ser Arg Glu Thr Leu Glu Glu Gly Leu Arg Arg
   305 310 315 320
   Ile Ala Ser Val Leu
   325
<210> 5
   <211> 1033
   <212> DNA
   <213> Corynebacterium glutamicum
<220>
   <221> CDS
   <222> (101)..(1006)
<400> 5
   gtgcggatcg ggtatccgcg ctacacttag aggtgttaga gatcatgagt ttccacgaac 60
   tgtaacgcag gattcaccaa tcaatgaaag gtcgaccgac atg agc act gaa gac    115
                                               Met Ser Thr Glu Asp
                                                1               5
   att gtc gtc gta gca gta gat ggc tcg gac gcc tca aaa caa gct gtt    163
   Ile Val Val Val Ala Val Asp Gly Ser Asp Ala Ser Lys Gln Ala Val
                   10                  15                  20
   cgg tgg gct gca aat acc gcc aac aaa cgt ggc att cca ctt cgc ttg    211
   Arg Trp Ala Ala Asn Thr Ala Asn Lys Arg Gly Ile Pro Leu Arg Leu
               25                  30                  35
   gct tcc agc tac acc atg cct cag ttc ctc tac gca gag gga atg gtt    259
   Ala Ser Ser Tyr Thr Met Pro Gln Phe Leu Tyr Ala Glu Gly Met Val
           40                  45                  50
   cca cca caa gag ctt ttc gat gac ctc cag gcc gaa gcc ctg gaa aag    307
   Pro Pro Gln Glu Leu Phe Asp Asp Leu Gln Ala Glu Ala Leu Glu Lys
       55                  60                  65
   att aac gaa gcc cgt gac atc gcc cat gag gta gcg cca gaa atc aag    355
   Ile Asn Glu Ala Arg Asp Ile Ala His Glu Val Ala Pro Glu Ile Lys
   70                  75                  80                  85
   atc ggg cac acc atc gct gaa ggc agt ccc atc gac atg ctg ttg gaa    403
   Ile Gly His Thr Ile Ala Glu Gly Ser Pro Ile Asp Met Leu Leu Glu
                   90                  95                  100
   atg tct ccc gat gcc aca atg atc gtc atg ggt tcc cgc gga ctc ggc    451
   Met Ser Pro Asp Ala Thr Met Ile Val Met Gly Ser Arg Gly Leu Gly
               105                 110                 115
   gga ctc tcc gga atg gtc atg ggc tcc gtc tcc ggt gca gtg gtc agc    499
   Gly Leu Ser Gly Met Val Met Gly Ser Val Ser Gly Ala Val Val Ser
           120                 125                 130
   cac gca aag tgt cca gtc gtt gtt gtc cgt gaa gac agc gca gtc aac    547
   His Ala Lys Cys Pro Val Val Val Val Arg Glu Asp Ser Ala Val Asn
       135                 140                 145
   gaa gac agc aag tac ggc cca gtc gtc gtc ggt gtg gat ggc tcc gaa     595
   Glu Asp Ser Lys Tyr Gly Pro Val Val Val Gly Val Asp Gly Ser Glu
   150                 155                 160                 165
   gtc tcc caa cag gca acc gaa tac gca ttt gcg gaa gct gaa gct cgt     643
   Val Ser Gln Gln Ala Thr Glu Tyr Ala Phe Ala Glu Ala Glu Ala Arg
                   170                 175                 180
   ggc gcc gaa ctc gtt gca gtt cac acc tgg atg gac atg cag gta cag     691
   Gly Ala Glu Leu Val Ala Val His Thr Trp Met Asp Met Gln Val Gln
               185                 190                 195
   gca tca ctt gca ggt ctt gca gct gct caa cag cag tgg gat gaa gtg     739
   Ala Ser Leu Ala Gly Leu Ala Ala Ala Gln Gln Gln Trp Asp Glu Val
           200                 205                 210
   gaa cgt cag caa acc gac atg ctg atc gaa cgc ctc gca cca ctg gtg     787
   Glu Arg Gln Gln Thr Asp Met Leu Ile Glu Arg Leu Ala Pro Leu Val
       215                 220                 225
   gaa aag tac cca agt gta acc gtc aag aag atc atc acc cgt gac cgc     835
   Glu Lys Tyr Pro Ser Val Thr Val Lys Lys Ile Ile Thr Arg Asp Arg
   230                 235                 240                 245
   cca gtt cgc gca ctt gca gaa gca tct gaa aac gcg cag ctc cta gtc     883
   Pro Val Arg Ala Leu Ala Glu Ala Ser Glu Asn Ala Gln Leu Leu Val
                   250                 255                 260
   gtt ggt tcc cat ggt cgt ggc gga ttt aag ggc atg ctc ctt ggc tcc     931
   Val Gly Ser His Gly Arg Gly Gly Phe Lys Gly Met Leu Leu Gly Ser
               265                 270                 275
   acc tcc cgc gca ctg ctg caa tcc gca ccg tgc cca atg atg gtg gtt     979
   Thr Ser Arg Ala Leu Leu Gln Ser Ala Pro Cys Pro Met Met Val Val
           280                 285                 290
   cgc cca cct gag aag att aag aag tag tttcttttaa gtttcgatgc cccggtt 1033
   Arg Pro Pro Glu Lys Ile Lys Lys
       295                 300
<210> 6
   <211> 301
   <212> PRT
   <213> Corynebacterium glutamicum
<400> 6
   Met Ser Thr Glu Asp Ile Val Val Val Ala Val Asp Gly Ser Asp Ala
    1               5                  10                  15
   Ser Lys Gln Ala Val Arg Trp Ala Ala Asn Thr Ala Asn Lys Arg Gly
               20                  25                  30
   Ile Pro Leu Arg Leu Ala Ser Ser Tyr Thr Met Pro Gln Phe Leu Tyr
           35                  40                  45
   Ala Glu Gly Met Val Pro Pro Gln Glu Leu Phe Asp Asp Leu Gln Ala
       50                  55                  60
   Glu Ala Leu Glu Lys Ile Asn Glu Ala Arg Asp Ile Ala His Glu Val
   65                  70                  75                  80
   Ala Pro Glu Ile Lys Ile Gly His Thr Ile Ala Glu Gly Ser Pro Ile
                   85                  90                  95
   Asp Met Leu Leu Glu Met Ser Pro Asp Ala Thr Met Ile Val Met Gly
               100                 105                 110
   Ser Arg Gly Leu Gly Gly Leu Ser Gly Met Val Met Gly Ser Val Ser
           115                 120                 125
   Gly Ala Val Val Ser His Ala Lys Cys Pro Val Val Val Val Arg Glu
       130                 135                 140
   Asp Ser Ala Val Asn Glu Asp Ser Lys Tyr Gly Pro Val Val Val Gly
   145                 150                 155                 160
   Val Asp Gly Ser Glu Val Ser Gln Gln Ala Thr Glu Tyr Ala Phe Ala
                   165                 170                 175
   Glu Ala Glu Ala Arg Gly Ala Glu Leu Val Ala Val His Thr Trp Met
               180                 185                 190
   Asp Met Gln Val Gln Ala Ser Leu Ala Gly Leu Ala Ala Ala Gln Gln
           195                 200                 205
   Gln Trp Asp Glu Val Glu Arg Gln Gln Thr Asp Met Leu Ile Glu Arg
       210                 215                 220
   Leu Ala Pro Leu Val Glu Lys Tyr Pro Ser Val Thr Val Lys Lys Ile
   225                 230                 235                 240
   Ile Thr Arg Asp Arg Pro Val Arg Ala Leu Ala Glu Ala Ser Glu Asn
                   245                 250                 255
   Ala Gln Leu Leu Val Val Gly Ser His Gly Arg Gly Gly Phe Lys Gly
               260                 265                 270
   Met Leu Leu Gly Ser Thr Ser Arg Ala Leu Leu Gln Ser Ala Pro Cys
           275                 280                 285
   Pro Met Met Val Val Arg Pro Pro Glu Lys Ile Lys Lys
       290                 295                 300
<210> 7
   <211> 948
   <212> DNA
   <213> Corynebacterium glutamicum
<220>
   <221> CDS
   <222> (101)..(925)
   <223> RXA02229
<400> 7
   gctggttcaa cagagaccac cgcgtgtcct gggtcgacgc ctctggcgat cccaccgcac 60
   aagccttgga gattttgggt ctacaatagc gagggtgaat ttg acc atc ccc ttt   115
                                                       Leu Thr Ile Pro Phe
                                                        1               5
   gcc aaa ggc cac gcc acc gaa aac gac ttc atc atc atc ccc gat gag     163
   Ala Lys Gly His Ala Thr Glu Asn Asp Phe Ile Ile Ile Pro Asp Glu
                    10                  15                  20
   gat gcg cgc cta gat tta act cca gaa atg gtg gtc acg ctg tgt gac     211
   Asp Ala Arg Leu Asp Leu Thr Pro Glu Met Val Val Thr Leu Cys Asp
                25                  30                  35
   cgc cgc gcc ggg atc ggt gct gat ggt atc ctc cgc gtg gtt aaa gct     259
   Arg Arg Ala Gly Ile Gly Ala Asp Gly Ile Leu Arg Val Val Lys Ala
            40                  45                  50
   gca gac gta gaa ggc tcc acg gtc gac cca tcg ctg tgg ttc atg gat     307
   Ala Asp Val Glu Gly Ser Thr Val Asp Pro Ser Leu Trp Phe Met Asp
        55                  60                  65
   tac cgc aac gcc gat gga tct ttg gct gaa atg tgc ggc aat ggt gtg     355
   Tyr Arg Asn Ala Asp Gly Ser Leu Ala Glu Met Cys Gly Asn Gly Val
    70                  75                  80                  85
   cgc ctg ttc gcg cac tgg ctg tac tcc cgc ggt ctt gtt gat aat acg     403
   Arg Leu Phe Ala His Trp Leu Tyr Ser Arg Gly Leu Val Asp Asn Thr
                    90                  95                 100
   agc ttt gat atc ggt acc cgc gcc ggt gtc cgc cac gtt gat att ttg     451
   Ser Phe Asp Ile Gly Thr Arg Ala Gly Val Arg His Val Asp Ile Leu
               105                 110                 115
   cag gca gat caa cat tct gcg cag gtc cgc gtt gat atg ggc atc cct     499
   Gln Ala Asp Gln His Ser Ala Gln Val Arg Val Asp Met Gly Ile Pro
           120                 125                 130
   gac gtc acg gga tta tcc acc tgc gac atc aac ggc caa gta ttc gct     547
   Asp Val Thr Gly Leu Ser Thr Cys Asp Ile Asn Gly Gln Val Phe Ala
       135                 140                 145
   ggc ctt ggc gtt gat atg ggt aac cca cac cta gcg tgc gtt gtg ccg     595
   Gly Leu Gly Val Asp Met Gly Asn Pro His Leu Ala Cys Val Val Pro
   150                 155                 160                 165
   ggc tta agt gcg tcg gct ctt gcc gat atg gaa ctg cgc gca cct acg     643
   Gly Leu Ser Ala Ser Ala Leu Ala Asp Met Glu Leu Arg Ala Pro Thr
                   170                 175                 180
   ttt gat cag gaa ttc ttc ccc cac ggt gtg aac gta gaa atc gtc aca     691
   Phe Asp Gln Glu Phe Phe Pro His Gly Val Asn Val Glu Ile Val Thr
               185                 190                 195
   gaa tta gaa gat gac gca gta tcg atg cgc gtg tgg gaa cgc gga gtg     739
   Glu Leu Glu Asp Asp Ala Val Ser Met Arg Val Trp Glu Arg Gly Val
           200                 205                 210
   ggc gaa acc cgc tcc tgt ggc acg gga acc gtt gct gca gcg tgt gct     787
   Gly Glu Thr Arg Ser Cys Gly Thr Gly Thr Val Ala Ala Ala Cys Ala
       215                 220                 225
   gct tta gct gat gct gga ttg gga gaa ggc aca gct aaa gtg tgc gtt     835
   Ala Leu Ala Asp Ala Gly Leu Gly Glu Gly Thr Ala Lys Val Cys Val
   230                 235                 240                 245
   cca cgt ggg gaa gta gaa gtc cag atc ttt gac gac ggc tcc aca ctc    883
   Pro Arg Gly Glu Val Glu Val Gln Ile Phe Asp Asp Gly Ser Thr Leu
                   250                 255                 260
   acc ggc cca agc gcc atc atc gca ctc ggt gag gtg cag atc            925
   Thr Gly Pro Ser Ala Ile Ile Ala Leu Gly Glu Val Gln Ile
               265                 270                 275
   taagattcgc gattgtagtt cgg                                          948
<210> 8
   <211> 275
   <212> PRT
   <213> Corynebacterium glutamicum
<400> 8
   Leu Thr Ile Pro Phe Ala Lys Gly His Ala Thr Glu Asn Asp Phe Ile
   1 5 10 15
   Ile Ile Pro Asp Glu Asp Ala Arg Leu Asp Leu Thr Pro Glu Met Val
   20 25 30
   Val Thr Leu Cys Asp Arg Arg Ala Gly Ile Gly Ala Asp Gly Ile Leu
   35 40 45
   Arg Val Val Lys Ala Ala Asp Val Glu Gly Ser Thr Val Asp Pro Ser
   50 55 60
   Leu Trp Phe Met Asp Tyr Arg Asn Ala Asp Gly Ser Leu Ala Glu Met
   65 70 75 80
   Cys Gly Asn Gly Val Arg Leu Phe Ala His Trp Leu Tyr Ser Arg Gly
   85 90 95
   Leu Val Asp Asn Thr Ser Phe Asp Ile Gly Thr Arg Ala Gly Val Arg
   100 105 110
   His Val Asp Ile Leu Gln Ala Asp Gln His Ser Ala Gln Val Arg Val
   115 120 125
   Asp Met Gly Ile Pro Asp Val Thr Gly Leu Ser Thr Cys Asp Ile Asn
   130 135 140
   Gly Gln Val Phe Ala Gly Leu Gly Val Asp Met Gly Asn Pro His Leu
   145 150 155 160
   Ala Cys Val Val Pro Gly Leu Ser Ala Ser Ala Leu Ala Asp Met Glu
   165 170 175
   Leu Arg Ala Pro Thr Phe Asp Gln Glu Phe Phe Pro His Gly Val Asn
   180 185 190
   Val Glu Ile Val Thr Glu Leu Glu Asp Asp Ala Val Ser Met Arg Val
   195 200 205
   Trp Glu Arg Gly Val Gly Glu Thr Arg Ser Cys Gly Thr Gly Thr Val
   210 215 220
   Ala Ala Ala Cys Ala Ala Leu Ala Asp Ala Gly Leu Gly Glu Gly Thr
   225 230 235 240
   Ala Lys Val Cys Val Pro Arg Gly Glu Val Glu Val Gln Ile Phe Asp
   245 250 255
   Asp Gly Ser Thr Leu Thr Gly Pro Ser Ala Ile Ile Ala Leu Gly Glu
   260 265 270
   Val Gln Ile
   275
<210> 9
   <211> 1491
   <212> DNA
   <213> Corynebacterium glutamicum
<220>
   <221> CDS
   <222> (101)..(1468)
   <223> RXS02970
<400> 9
   aaccgacaaa acagccgttc acgtgctaaa gcagctcggc ttgatctagg gtgaggtgag 60
   ttatttaaag acttcataat attttgggga gtgaactggt ttg gca ttg aag ggt   115
                                             Leu Ala Leu Lys Gly
                                             1               5
   tac acc aac ttt gac ggt gaa ttc atc gaa ttc gga tct gtg caa gca   163
   Tyr Thr Asn Phe Asp Gly Glu Phe Ile Glu Phe Gly Ser Val Gln Ala
                   10                  15                  20
   aaa gaa gag gaa aaa cgg gca ttc gac aac gat cgc gcg cac gtt ttc   211
   Lys Glu Glu Glu Lys Arg Ala Phe Asp Asn Asp Arg Ala His Val Phe
               25                  30                  35
   cac tcc tgg tcc gcg cag gac aaa atc agc ccc aaa gta tgg gca gct   259
   His Ser Trp Ser Ala Gln Asp Lys Ile Ser Pro Lys Val Trp Ala Ala
           40                  45                  50
   gcc gaa ggt tcc acg ctg tac gac ttc gac ggc aac gcc ttc atc gac   307
   Ala Glu Gly Ser Thr Leu Tyr Asp Phe Asp Gly Asn Ala Phe Ile Asp
       55                  60                  65
   atg ggt tcc caa ctt gtc tcg gca aac tta ggc cac aac aac cct cga   355
   Met Gly Ser Gln Leu Val Ser Ala Asn Leu Gly His Asn Asn Pro Arg
   70                  75                  80                  85
   tta gtt gag gcg atc cag cgc caa gca gcc cgg ttg acc aac atc aac   403
   Leu Val Glu Ala Ile Gln Arg Gln Ala Ala Arg Leu Thr Asn Ile Asn
                   90                  95                  100
   ccg gcc ttc ggc aat gat gtg cgc tct gat gtt gct gca aag atc gtg   451
   Pro Ala Phe Gly Asn Asp Val Arg Ser Asp Val Ala Ala Lys Ile Val
               105                 110                 115
   tcg atg gcc cgt ggc gaa ttc tcc cac gtg ttt ttc acc aac ggc ggc   499
   Ser Met Ala Arg Gly Glu Phe Ser His Val Phe Phe Thr Asn Gly Gly
           120                 125                 130
   gcc gac gcc atc gag cac tcc atc cgc atg gct cgc ctg cac acc gga     547
   Ala Asp Ala Ile Glu His Ser Ile Arg Met Ala Arg Leu His Thr Gly
       135                 140                 145
   cgc aac aaa att ctg tcc gca tac cgc agc tac cac ggc gca acc gga     595
   Arg Asn Lys Ile Leu Ser Ala Tyr Arg Ser Tyr His Gly Ala Thr Gly
   150                 155                 160                 165
   tcc gcg atg atg ctc acc ggc gaa cac cgc cgc ctg ggc aac ccc acc     643
   Ser Ala Met Met Leu Thr Gly Glu His Arg Arg Leu Gly Asn Pro Thr
                   170                 175                 180
   acc gac cca gat atc tac cac ttc tgg gca cca ttc ctg cac cac tcc     691
   Thr Asp Pro Asp Ile Tyr His Phe Trp Ala Pro Phe Leu His His Ser
               185                 190                 195
   tca ttc ttt gcc acc acc caa gaa gaa gaa tgc gaa cgc gca ctc aag     739
   Ser Phe Phe Ala Thr Thr Gln Glu Glu Glu Cys Glu Arg Ala Leu Lys
           200                 205                 210
   cac ttg gaa gat gtc atc gcg ttt gaa ggt gct ggc atg atc gca gcg     787
   His Leu Glu Asp Val Ile Ala Phe Glu Gly Ala Gly Met Ile Ala Ala
       215                 220                 225
   atc gtc ctg gag cca gtg gtg gga tca tca gga atc atc ctg cca cca     835
   Ile Val Leu Glu Pro Val Val Gly Ser Ser Gly Ile Ile Leu Pro Pro
   230                 235                 240                 245
   gca ggt tac tta aat ggc gtg cgc gaa ctt tgc aac aag cac ggc atc     883
   Ala Gly Tyr Leu Asn Gly Val Arg Glu Leu Cys Asn Lys His Gly Ile
                   250                 255                 260
   ctc ttc atc gcc gac gaa gtc atg gtc gga ttc gga cgc acc gga aaa     931
   Leu Phe Ile Ala Asp Glu Val Met Val Gly Phe Gly Arg Thr Gly Lys
               265                 270                 275
   ctg ttt gct tac gag cat gct ggc gac gat ttc cag cca gac atg atc     979
   Leu Phe Ala Tyr Glu His Ala Gly Asp Asp Phe Gln Pro Asp Met Ile
           280                 285                 290
   acc ttc gcc aag ggt gtt aac gca ggt tac gcc cca ctc ggt ggc atc     1027
   Thr Phe Ala Lys Gly Val Asn Ala Gly Tyr Ala Pro Leu Gly Gly Ile
       295                 300                 305
   gtg atg acc caa tca atc cgc gat acc ttc gga tca gag gca tac tcc     1075
   Val Met Thr Gln Ser Ile Arg Asp Thr Phe Gly Ser Glu Ala Tyr Ser
   310                 315                 320                 325
   ggc gga ctc acc tac tcc gga cac cca ctt gca gta gca ccc gcc aag     1123
   Gly Gly Leu Thr Tyr Ser Gly His Pro Leu Ala Val Ala Pro Ala Lys
                   330                 335                 340
   gca gcg ctg gag att tac gcg gaa gga gag atc att cca cgc gta gct     1171
   Ala Ala Leu Glu Ile Tyr Ala Glu Gly Glu Ile Ile Pro Arg Val Ala
               345                 350                 355
   cga ctt ggc gct gaa ctg atc gaa cct cgc ctt cgt gaa cta gcg gaa     1219
   Arg Leu Gly Ala Glu Leu Ile Glu Pro Arg Leu Arg Glu Leu Ala Glu
           360                 365                 370
   gaa aac gta gcg atc gct gac gtg cgg ggc atc gga ttc ttc tgg gca      1267
   Glu Asn Val Ala Ile Ala Asp Val Arg Gly Ile Gly Phe Phe Trp Ala
       375                 380                 385
   gtg gag ttc aat gca gac gcc act gcc atg gct gcc ggt gct gca gaa      1315
   Val Glu Phe Asn Ala Asp Ala Thr Ala Met Ala Ala Gly Ala Ala Glu
   390                 395                 400                 405
   ttc aag gaa cgc ggc gtg tgg ccg atg atc tcc ggc aac cga ttc cac      1363
   Phe Lys Glu Arg Gly Val Trp Pro Met Ile Ser Gly Asn Arg Phe His
                   410                 415                 420
   atc gcg ccg ccg ctg acc acc act gat gac gaa ttg gta gca ctg ctg      1411
   Ile Ala Pro Pro Leu Thr Thr Thr Asp Asp Glu Leu Val Ala Leu Leu
               425                 430                 435
   gac gcg gtg gaa gct gca gcc caa gct gtc gag ctg acc ttc gct ggg      1459
   Asp Ala Val Glu Ala Ala Ala Gln Ala Val Glu Leu Thr Phe Ala Gly
           440                 445                 450
   gcg ttg ttc taagttttct agataacaag gcc                                1491
   Ala Leu Phe
       455
<210> 10
   <211> 456
   <212> PRT
   <213> Corynebacterium glutamicum
<400> 10
   Leu Ala Leu Lys Gly Tyr Thr Asn Phe Asp Gly Glu Phe Ile Glu Phe
    1               5                  10                  15
   Gly Ser Val Gln Ala Lys Glu Glu Glu Lys Arg Ala Phe Asp Asn Asp
               20                  25                  30
   Arg Ala His Val Phe His Ser Trp Ser Ala Gln Asp Lys Ile Ser Pro
           35                  40                  45
   Lys Val Trp Ala Ala Ala Glu Gly Ser Thr Leu Tyr Asp Phe Asp Gly
       50                  55                  60
   Asn Ala Phe Ile Asp Met Gly Ser Gln Leu Val Ser Ala Asn Leu Gly
   65                  70                  75                  80
   His Asn Asn Pro Arg Leu Val Glu Ala Ile Gln Arg Gln Ala Ala Arg
                   85                  90                  95
   Leu Thr Asn Ile Asn Pro Ala Phe Gly Asn Asp Val Arg Ser Asp Val
               100                 105                 110
   Ala Ala Lys Ile Val Ser Met Ala Arg Gly Glu Phe Ser His Val Phe
           115                 120                 125
   Phe Thr Asn Gly Gly Ala Asp Ala Ile Glu His Ser Ile Arg Met Ala
       130                 135                 140
   Arg Leu His Thr Gly Arg Asn Lys Ile Leu Ser Ala Tyr Arg Ser Tyr
   145                 150                 155                 160
   His Gly Ala Thr Gly Ser Ala Met Met Leu Thr Gly Glu His Arg Arg
   165 170 175
   Leu Gly Asn Pro Thr Thr Asp Pro Asp Ile Tyr His Phe Trp Ala Pro
   180 185 190
   Phe Leu His His Ser Ser Phe Phe Ala Thr Thr Gln Glu Glu Glu Cys
   195 200 205
   Glu Arg Ala Leu Lys His Leu Glu Asp Val Ile Ala Phe Glu Gly Ala
   210 215 220
   Gly Met Ile Ala Ala Ile Val Leu Glu Pro Val Val Gly Ser Ser Gly
   225 230 235 240
   Ile Ile Leu Pro Pro Ala Gly Tyr Leu Asn Gly Val Arg Glu Leu Cys
   245 250 255
   Asn Lys His Gly Ile Leu Phe Ile Ala Asp Glu Val Met Val Gly Phe
   260 265 270
   Gly Arg Thr Gly Lys Leu Phe Ala Tyr Glu His Ala Gly Asp Asp Phe
   275 280 285
   Gln Pro Asp Met Ile Thr Phe Ala Lys Gly Val Asn Ala Gly Tyr Ala
   290 295 300
   Pro Leu Gly Gly Ile Val Met Thr Gln Ser Ile Arg Asp Thr Phe Gly
   305 310 315 320
   Ser Glu Ala Tyr Ser Gly Gly Leu Thr Tyr Ser Gly His Pro Leu Ala
   325 330 335
   Val Ala Pro Ala Lys Ala Ala Leu Glu Ile Tyr Ala Glu Gly Glu Ile
   340 345 350
   Ile Pro Arg Val Ala Arg Leu Gly Ala Glu Leu Ile Glu Pro Arg Leu
   355 360 365
   Arg Glu Leu Ala Glu Glu Asn Val Ala Ile Ala Asp Val Arg Gly Ile
   370 375 380
   Gly Phe Phe Trp Ala Val Glu Phe Asn Ala Asp Ala Thr Ala Met Ala
   385 390 395 400
   Ala Gly Ala Ala Glu Phe Lys Glu Arg Gly Val Trp Pro Met Ile Ser
   405 410 415
   Gly Asn Arg Phe His Ile Ala Pro Pro Leu Thr Thr Thr Asp Asp Glu
   420 425 430
   Leu Val Ala Leu Leu Asp Ala Val Glu Ala Ala Ala Gln Ala Val Glu
   435 440 445
   Leu Thr Phe Ala Gly Ala Leu Phe
   450 455
<210> 11
   <211> 1330
   <212> DNA
   <213> Corynebacterium glutamicum
<220>
   <221> CDS
   <222> (101)..(1330)
   <223> FRXA01009
<400> 11
   aaccgacaaa acagccgttc acgtgctaaa gcagctcggc ttgatctagg gtgaggtgag 60
   ttatttaaag acttcataat attttgggga gtgaactggt ttg gca ttg aag ggt   115
                                             Leu Ala Leu Lys Gly
                                              1               5
   tac acc aac ttt gac ggt gaa ttc atc gaa ttc gga tct gtg caa gca   163
   Tyr Thr Asn Phe Asp Gly Glu Phe Ile Glu Phe Gly Ser Val Gln Ala
                   10                  15                  20
   aaa gaa gag gaa aaa cgg gca ttc gac aac gat cgc gcg cac gtt ttc   211
   Lys Glu Glu Glu Lys Arg Ala Phe Asp Asn Asp Arg Ala His Val Phe
               25                  30                  35
   cac tcc tgg tcc gcg cag gac aaa atc agc ccc aaa gta tgg gca gct   259
   His Ser Trp Ser Ala Gln Asp Lys Ile Ser Pro Lys Val Trp Ala Ala
           40                  45                  50
   gcc gaa ggt tcc acg ctg tac gac ttc gac ggc aac gcc ttc atc gac   307
   Ala Glu Gly Ser Thr Leu Tyr Asp Phe Asp Gly Asn Ala Phe Ile Asp
       55                  60                  65
   atg ggt tcc caa ctt gtc tcg gca aac tta ggc cac aac aac cct cga   355
   Met Gly Ser Gln Leu Val Ser Ala Asn Leu Gly His Asn Asn Pro Arg
   70                  75                  80                  85
   tta gtt gag gcg atc cag cgc caa gca gcc cgg ttg acc aac atc aac   403
   Leu Val Glu Ala Ile Gln Arg Gln Ala Ala Arg Leu Thr Asn Ile Asn
                   90                  95                  100
   ccg gcc ttc ggc aat gat gtg cgc tct gat gtt gct gca aag atc gtg   451
   Pro Ala Phe Gly Asn Asp Val Arg Ser Asp Val Ala Ala Lys Ile Val
               105                 110                 115
   tcg atg gcc cgt ggc gaa ttc tcc cac gtg ttt ttc acc aac ggc ggc   499
   Ser Met Ala Arg Gly Glu Phe Ser His Val Phe Phe Thr Asn Gly Gly
           120                 125                 130
   gcc gac gcc atc gag cac tcc atc cgc atg gct cgc ctg cac acc gga   547
   Ala Asp Ala Ile Glu His Ser Ile Arg Met Ala Arg Leu His Thr Gly
       135                 140                 145
   cgc aac aaa att ctg tcc gca tac cgc agc tac cac ggc gca acc gga   595
   Arg Asn Lys Ile Leu Ser Ala Tyr Arg Ser Tyr His Gly Ala Thr Gly
   150                 155                 160                 165
   tcc gcg atg atg ctc acc ggc gaa cac cgc cgc ctg ggc aac ccc acc   643
   Ser Ala Met Met Leu Thr Gly Glu His Arg Arg Leu Gly Asn Pro Thr
                   170                 175                 180
   acc gac cca gat atc tac cac ttc tgg gca cca ttc ctg cac cac tcc   691
   Thr Asp Pro Asp Ile Tyr His Phe Trp Ala Pro Phe Leu His His Ser
               185                 190                 195
   tca ttc ttt gcc acc acc caa gaa gaa gaa tgc gaa cgc gca ctc aag      739
   Ser Phe Phe Ala Thr Thr Gln Glu Glu Glu Cys Glu Arg Ala Leu Lys
           200                 205                 210
   cac ttg gaa gat gtc atc gcg ttt gaa ggt gct ggc atg atc gca gcg      787
   His Leu Glu Asp Val Ile Ala Phe Glu Gly Ala Gly Met Ile Ala Ala
       215                 220                 225
   atc gtc ctg gag cca gtg gtg gga tca tca gga atc atc ctg cca cca      835
   Ile Val Leu Glu Pro Val Val Gly Ser Ser Gly Ile Ile Leu Pro Pro
   230                 235                 240                 245
   gca ggt tac tta aat ggc gtg cgc gaa ctt tgc aac aag cac ggc atc      883
   Ala Gly Tyr Leu Asn Gly Val Arg Glu Leu Cys Asn Lys His Gly Ile
                   250                 255                 260
   ctc ttc atc gcc gac gaa gtc atg gtc gga ttc gga cgc acc gga aaa      931
   Leu Phe Ile Ala Asp Glu Val Met Val Gly Phe Gly Arg Thr Gly Lys
               265                 270                 275
   ctg ttt gct tac gag cat gct ggc gac gat ttc cag cca gac atg atc      979
   Leu Phe Ala Tyr Glu His Ala Gly Asp Asp Phe Gln Pro Asp Met Ile
           280                 285                 290
   acc ttc gcc aag ggt gtt aac gca ggt tac gcc cca ctc ggt ggc atc      1027
   Thr Phe Ala Lys Gly Val Asn Ala Gly Tyr Ala Pro Leu Gly Gly Ile
       295                 300                 305
   gtg atg acc caa tca atc cgc gat acc ttc gga tca gag gca tac tcc      1075
   Val Met Thr Gln Ser Ile Arg Asp Thr Phe Gly Ser Glu Ala Tyr Ser
   310                 315                 320                 325
   ggc gga ctc acc tac tcc gga cac cca ctt gca gta gca ccc gcc aag      1123
   Gly Gly Leu Thr Tyr Ser Gly His Pro Leu Ala Val Ala Pro Ala Lys
                   330                 335                 340
   gca gcg ctg gag att tac gcg gaa gga gag atc att cca cgc gta gct      1171
   Ala Ala Leu Glu Ile Tyr Ala Glu Gly Glu Ile Ile Pro Arg Val Ala
               345                 350                 355
   cga ctt ggc gct gaa ctg atc gaa cct cgc ctt cgt gaa cta gcg gaa      1219
   Arg Leu Gly Ala Glu Leu Ile Glu Pro Arg Leu Arg Glu Leu Ala Glu
           360                 365                 370
   gaa aac gta gcg atc gct gac gtg cgg ggc atc gga ttc ttc tgg gca      1267
   Glu Asn Val Ala Ile Ala Asp Val Arg Gly Ile Gly Phe Phe Trp Ala
       375                 380                 385
   gtg gag ttc aat gca gac gcc act gcc atg gct gcc ggt gct gca gaa      1315
   Val Glu Phe Asn Ala Asp Ala Thr Ala Met Ala Ala Gly Ala Ala Glu
   390                 395                 400                 405
   ttc aag gaa cgc ggc                                                  1330
   Phe Lys Glu Arg Gly
                   410
<210> 12
   <211> 410
   <212> PRT
   <213> Corynebacterium glutamicum
<400> 12
   Leu Ala Leu Lys Gly Tyr Thr Asn Phe Asp Gly Glu Phe Ile Glu Phe
   1               5                   10                  15
   Gly Ser Val Gln Ala Lys Glu Glu Glu Lys Arg Ala Phe Asp Asn Asp
               20                  25                  30
   Arg Ala His Val Phe His Ser Trp Ser Ala Gln Asp Lys Ile Ser Pro
           35                  40                  45
   Lys Val Trp Ala Ala Ala Glu Gly Ser Thr Leu Tyr Asp Phe Asp Gly
       50                  55                  60
   Asn Ala Phe Ile Asp Met Gly Ser Gln Leu Val Ser Ala Asn Leu Gly
   65                  70                  75                  80
   His Asn Asn Pro Arg Leu Val Glu Ala Ile Gln Arg Gln Ala Ala Arg
                   85                  90                  95
   Leu Thr Asn Ile Asn Pro Ala Phe Gly Asn Asp Val Arg Ser Asp Val
               100                 105                 110
   Ala Ala Lys Ile Val Ser Met Ala Arg Gly Glu Phe Ser His Val Phe
           115                 120                 125
   Phe Thr Asn Gly Gly Ala Asp Ala Ile Glu His Ser Ile Arg Met Ala
       130                 135                 140
   Arg Leu His Thr Gly Arg Asn Lys Ile Leu Ser Ala Tyr Arg Ser Tyr
   145                 150                 155                 160
   His Gly Ala Thr Gly Ser Ala Met Met Leu Thr Gly Glu His Arg Arg
                   165                 170                 175
   Leu Gly Asn Pro Thr Thr Asp Pro Asp Ile Tyr His Phe Trp Ala Pro
               180                 185                 190
   Phe Leu His His Ser Ser Phe Phe Ala Thr Thr Gln Glu Glu Glu Cys
           195                 200                 205
   Glu Arg Ala Leu Lys His Leu Glu Asp Val Ile Ala Phe Glu Gly Ala
       210                 215                 220
   Gly Met Ile Ala Ala Ile Val Leu Glu Pro Val Val Gly Ser Ser Gly
   225                 230                 235                 240
   Ile Ile Leu Pro Pro Ala Gly Tyr Leu Asn Gly Val Arg Glu Leu Cys
                   245                 250                 255
   Asn Lys His Gly Ile Leu Phe Ile Ala Asp Glu Val Met Val Gly Phe
               260                 265                 270
   Gly Arg Thr Gly Lys Leu Phe Ala Tyr Glu His Ala Gly Asp Asp Phe
           275                 280                 285
   Gln Pro Asp Met Ile Thr Phe Ala Lys Gly Val Asn Ala Gly Tyr Ala
       290                 295                 300
   Pro Leu Gly Gly Ile Val Met Thr Gln Ser Ile Arg Asp Thr Phe Gly
   305 310 315 320
   Ser Glu Ala Tyr Ser Gly Gly Leu Thr Tyr Ser Gly His Pro Leu Ala
   325 330 335
   Val Ala Pro Ala Lys Ala Ala Leu Glu Ile Tyr Ala Glu Gly Glu Ile
   340 345 350
   Ile Pro Arg Val Ala Arg Leu Gly Ala Glu Leu Ile Glu Pro Arg Leu
   355 360 365
   Arg Glu Leu Ala Glu Glu Asn Val Ala Ile Ala Asp Val Arg Gly Ile
   370 375 380
   Gly Phe Phe Trp Ala Val Glu Phe Asn Ala Asp Ala Thr Ala Met Ala
   385 390 395 400
   Ala Gly Ala Ala Glu Phe Lys Glu Arg Gly
   405 410
<210> 13
   <211> 792
   <212> DNA
   <213> Corynebacterium glutamicum
<220>
   <221> CDS
   <222> (101)..(769)
   <223> RXC02390
<400> 13
   gctggtggtg ctgacccata cgctggaact ccaactgctg ttgataccgc caagatgttt 60
   ggccgcgagg atctcgtagc tcgcttcgag tcataggccg gtg gag tgg acc gct   115
                                            Val Glu Trp Thr Ala
                                             1               5
   ttt ggc acc ctg att ctg ctc aat ttg gtg ggc agt tta tcc ccg ggg   163
   Phe Gly Thr Leu Ile Leu Leu Asn Leu Val Gly Ser Leu Ser Pro Gly
                   10                  15                  20
   cct gat acc ttt ttc ctc ctc cgc tta gcc acc cgc tcc aga gcg cac   211
   Pro Asp Thr Phe Phe Leu Leu Arg Leu Ala Thr Arg Ser Arg Ala His
               25                  30                  35
   gcg atc gct ggc gtc gcc ggc atc gtc acc gga ctc acg gtg tgg gtg   259
   Ala Ile Ala Gly Val Ala Gly Ile Val Thr Gly Leu Thr Val Trp Val
           40                  45                  50
   acg ctg acg gtc gtg gga gca gcg gcg ctg ctc acc act tat ccg tcg   307
   Thr Leu Thr Val Val Gly Ala Ala Ala Leu Leu Thr Thr Tyr Pro Ser
       55                  60                  65
   att ctc gga atc atc cag ctc gtc ggc ggc acg tac cta agc ttc att   355
   Ile Leu Gly Ile Ile Gln Leu Val Gly Gly Thr Tyr Leu Ser Phe Ile
   70                  75                  80                  85
   ggg tac aag ttg ctg cgc tcg gcg tcg aga gag ctt atc gac gcc cgc   403
   Gly Tyr Lys Leu Leu Arg Ser Ala Ser Arg Glu Leu Ile Asp Ala Arg
                   90                  95                  100
   cag ttc cgt ttc aac gcc gat gcc cga cct atc ccg gat gcg gta gaa     451
   Gln Phe Arg Phe Asn Ala Asp Ala Arg Pro Ile Pro Asp Ala Val Glu
               105                 110                 115
   gca ctg gga acc cgc act cag gta tat cga caa ggt ttg gcc acc aac     499
   Ala Leu Gly Thr Arg Thr Gln Val Tyr Arg Gln Gly Leu Ala Thr Asn
           120                 125                 130
   ctg tca aac cct aaa gtt gtc atg tac ttc gcg gca att ctg gct ccg     547
   Leu Ser Asn Pro Lys Val Val Met Tyr Phe Ala Ala Ile Leu Ala Pro
       135                 140                 145
   ttg atg cca gcg cac cca tca ccg gtg ctg gcg ttc tct atc atc gtg     595
   Leu Met Pro Ala His Pro Ser Pro Val Leu Ala Phe Ser Ile Ile Val
   150                 155                 160                 165
   gcg att tta gtg cag acc ttt gtt acc ttc tct gct gtg tgc ctc att     643
   Ala Ile Leu Val Gln Thr Phe Val Thr Phe Ser Ala Val Cys Leu Ile
                   170                 175                 180
   gtc tct acg gag cgt gtg cgc aaa gca atg ctg cgt gca ggt ccc tgg     691
   Val Ser Thr Glu Arg Val Arg Lys Ala Met Leu Arg Ala Gly Pro Trp
               185                 190                 195
   ttt gac ctg ctt gct ggc gtt gtc ttc ctc gtt gtg ggt gtg act ctg     739
   Phe Asp Leu Leu Ala Gly Val Val Phe Leu Val Val Gly Val Thr Leu
           200                 205                 210
   ctg tat gaa ggc ctg acc ggt tta ctc ggg taaaggcata aaaaatggct       789
   Leu Tyr Glu Gly Leu Thr Gly Leu Leu Gly
       215                 220
   tcc                                                                 792
<210> 14
   <211> 223
   <212> PRT
   <213> Corynebacterium glutamicum
<400> 14
   Val Glu Trp Thr Ala Phe Gly Thr Leu Ile Leu Leu Asn Leu Val Gly
    1               5                  10                  15
   Ser Leu Ser Pro Gly Pro Asp Thr Phe Phe Leu Leu Arg Leu Ala Thr
               20                  25                  30
   Arg Ser Arg Ala His Ala Ile Ala Gly Val Ala Gly Ile Val Thr Gly
           35                  40                  45
   Leu Thr Val Trp Val Thr Leu Thr Val Val Gly Ala Ala Ala Leu Leu
       50                  55                  60
   Thr Thr Tyr Pro Ser Ile Leu Gly Ile Ile Gln Leu Val Gly Gly Thr
   65                  70                  75                  80
   Tyr Leu Ser Phe Ile Gly Tyr Lys Leu Leu Arg Ser Ala Ser Arg Glu
                   85                  90                  95
   Leu Ile Asp Ala Arg Gln Phe Arg Phe Asn Ala Asp Ala Arg Pro Ile
               100                 105                 110
   Pro Asp Ala Val Glu Ala Leu Gly Thr Arg Thr Gln Val Tyr Arg Gln
           115                 120                 125
   Gly Leu Ala Thr Asn Leu Ser Asn Pro Lys Val Val Met Tyr Phe Ala
       130                 135                 140
   Ala Ile Leu Ala Pro Leu Met Pro Ala His Pro Ser Pro Val Leu Ala
   145                 150                 155                 160
   Phe Ser Ile Ile Val Ala Ile Leu Val Gln Thr Phe Val Thr Phe Ser
                   165                 170                 175
   Ala Val Cys Leu Ile Val Ser Thr Glu Arg Val Arg Lys Ala Met Leu
               180                 185                 190
   Arg Ala Gly Pro Trp Phe Asp Leu Leu Ala Gly Val Val Phe Leu Val
           195                 200                 205
   Val Gly Val Thr Leu Leu Tyr Glu Gly Leu Thr Gly Leu Leu Gly
       210                 215                 220
<210> 15
   <211> 897
   <212> DNA
   <213> Corynebacterium glutamicum
<220>
   <221> CDS
   <222> (101)..(874)
   <223> RXC01796
<400> 15
   atgtaactcg atcaggtgga aatgcccgca aaagtggcgg cggtggccga gggatggccg 60
   ttggtgcggc atcggtggcc tgctactagt cgggctcttc ttg ctc ctt ggc ggt   115
                                               Leu Leu Leu Gly Gly
                                                1               5
   aac cct gcc gag atc gac cag gtt tta ggt ggc gat caa acc cag atc   163
   Asn Pro Ala Glu Ile Asp Gln Val Leu Gly Gly Asp Gln Thr Gln Ile
                   10                  15                  20
   gag tct gga gag tcc acc gga gcc ggc gac ttt gat cac tgc caa acc   211
   Glu Ser Gly Glu Ser Thr Gly Ala Gly Asp Phe Asp His Cys Gln Thr
               25                  30                  35
   ggc gca gat gcc aac gcc agt gat gat tgt cgc ctt tac tac acc tca   259
   Gly Ala Asp Ala Asn Ala Ser Asp Asp Cys Arg Leu Tyr Tyr Thr Ser
           40                  45                  50
   ttc tcc gtc aat gaa atg tgg cag act ttg ctt cca gct cag gct ggt   307
   Phe Ser Val Asn Glu Met Trp Gln Thr Leu Leu Pro Ala Gln Ala Gly
       55                  60                  65
   atc gaa tac acc gag ccg aca ttg act ctt ttc aaa aac tcc acc caa   355
   Ile Glu Tyr Thr Glu Pro Thr Leu Thr Leu Phe Lys Asn Ser Thr Gln
   70                  75                  80                  85
   acc ggc tgc ggt ttc gct tct gcg tcc act ggg ccg ttt tac tgt ccg     403
   Thr Gly Cys Gly Phe Ala Ser Ala Ser Thr Gly Pro Phe Tyr Cys Pro
                    90                  95                  100
   tca gac caa gat gct tat ttt gac ttg act ttc ttc gat cag atg cgt     451
   Ser Asp Gln Asp Ala Tyr Phe Asp Leu Thr Phe Phe Asp Gln Met Arg
               105                 110                 115
   cag ttc ggt gca gaa aac gcc ccg ctt gcc cag atg tac atc gtg gcg     499
   Gln Phe Gly Ala Glu Asn Ala Pro Leu Ala Gln Met Tyr Ile Val Ala
           120                 125                 130
   cac gag tac ggc cac cac gtc caa aac ctc gag ggc aca ctc gga ctg     547
   His Glu Tyr Gly His His Val Gln Asn Leu Glu Gly Thr Leu Gly Leu
       135                 140                 145
   tcc aat tac aac gat ccg ggc gct gat tcc aac gcc gtc aag atc gag     595
   Ser Asn Tyr Asn Asp Pro Gly Ala Asp Ser Asn Ala Val Lys Ile Glu
   150                 155                 160                 165
   ttg cag gcc gat tgc tac gca ggc att tgg gct aat cac tcc agc gaa     643
   Leu Gln Ala Asp Cys Tyr Ala Gly Ile Trp Ala Asn His Ser Ser Glu
                   170                 175                 180
   ggc ccg gat ccg cta ctc caa ccc atc acc gaa tct gag cta gat tcc     691
   Gly Pro Asp Pro Leu Leu Gln Pro Ile Thr Glu Ser Glu Leu Asp Ser
               185                 190                 195
   gct ctc ctt gct gca agc gcc gtg ggc gac gac aat atc cag caa cga     739
   Ala Leu Leu Ala Ala Ser Ala Val Gly Asp Asp Asn Ile Gln Gln Arg
           200                 205                 210
   tcc ggt ggc gat gtc aat cct gaa agc tgg act cac ggc tca tcg cag     787
   Ser Gly Gly Asp Val Asn Pro Glu Ser Trp Thr His Gly Ser Ser Gln
       215                 220                 225
   cag cgc aaa gac gcg ttc ctc gcc ggc tac aac acc ggc cag atg agc     835
   Gln Arg Lys Asp Ala Phe Leu Ala Gly Tyr Asn Thr Gly Gln Met Ser
   230                 235                 240                 245
   gcc tgc gac ttc ctc ggc cgg ggc gtc tac aac gac gct taaagcattg      884
   Ala Cys Asp Phe Leu Gly Arg Gly Val Tyr Asn Asp Ala
                   250                 255
   cttttcgacg tct                                                      897
<210> 16
   <211> 258
   <212> PRT
   <213> Corynebacterium glutamicum
<400> 16
   Leu Leu Leu Gly Gly Asn Pro Ala Glu Ile Asp Gln Val Leu Gly Gly
   1               5                   10                  15
   Asp Gln Thr Gln Ile Glu Ser Gly Glu Ser Thr Gly Ala Gly Asp Phe
               20                  25                  30
   Asp His Cys Gln Thr Gly Ala Asp Ala Asn Ala Ser Asp Asp Cys Arg
           35                  40                  45
   Leu Tyr Tyr Thr Ser Phe Ser Val Asn Glu Met Trp Gln Thr Leu Leu
       50                  55                  60
   Pro Ala Gln Ala Gly Ile Glu Tyr Thr Glu Pro Thr Leu Thr Leu Phe
   65                  70                  75                  80
   Lys Asn Ser Thr Gln Thr Gly Cys Gly Phe Ala Ser Ala Ser Thr Gly
                   85                  90                  95
   Pro Phe Tyr Cys Pro Ser Asp Gln Asp Ala Tyr Phe Asp Leu Thr Phe
               100                 105                 110
   Phe Asp Gln Met Arg Gln Phe Gly Ala Glu Asn Ala Pro Leu Ala Gln
           115                 120                 125
   Met Tyr Ile Val Ala His Glu Tyr Gly His His Val Gln Asn Leu Glu
       130                 135                 140
   Gly Thr Leu Gly Leu Ser Asn Tyr Asn Asp Pro Gly Ala Asp Ser Asn
   145                 150                 155                 160
   Ala Val Lys Ile Glu Leu Gln Ala Asp Cys Tyr Ala Gly Ile Trp Ala
                   165                 170                 175
   Asn His Ser Ser Glu Gly Pro Asp Pro Leu Leu Gln Pro Ile Thr Glu
               180                 185                 190
   Ser Glu Leu Asp Ser Ala Leu Leu Ala Ala Ser Ala Val Gly Asp Asp
           195                 200                 205
   Asn Ile Gln Gln Arg Ser Gly Gly Asp Val Asn Pro Glu Ser Trp Thr
       210                 215                 220
   His Gly Ser Ser Gln Gln Arg Lys Asp Ala Phe Leu Ala Gly Tyr Asn
   225                 230                 235                 240
   Thr Gly Gln Met Ser Ala Cys Asp Phe Leu Gly Arg Gly Val Tyr Asn
                   245                 250                 255
   Asp Ala
<210> 17
   <211> 771
   <212> DNA
   <213> Corynebacterium glutamicum
<220>
   <221> CDS
   <222> (101)..(748)
   <223> RXC01207
<400> 17
   cttcatgatc tcaccggcag agcgcgtttt gttacagcgc gtaaactgtg actttgaaaa 60
   atttttgaac aatccgtaca ccaacttcag gagaaaaaca gtg agc aga atc tat   115
                                              Val Ser Arg Ile Tyr
                                                1               5
   gac tgt gcc gac caa gac tcc cgt gca gca ggc cta aag gcg gct gtc      163
   Asp Cys Ala Asp Gln Asp Ser Arg Ala Ala Gly Leu Lys Ala Ala Val
                   10                  15                  20
   gat gca gtc aaa gcc ggt cag ctc gtt gtc ctt ccc acg gat acc ctt      211
   Asp Ala Val Lys Ala Gly Gln Leu Val Val Leu Pro Thr Asp Thr Leu
               25                  30                  35
   tat gga ctc ggc tgc gac gct ttc aac aac gag gca gta gcc aac ctt      259
   Tyr Gly Leu Gly Cys Asp Ala Phe Asn Asn Glu Ala Val Ala Asn Leu
           40                  45                  50
   ctg gcc acc aaa cac cgt ggc ccc gat atg ccc gtt cca gtg ctc gtc      307
   Leu Ala Thr Lys His Arg Gly Pro Asp Met Pro Val Pro Val Leu Val
       55                  60                  65
   ggc agc tgg gac acc att caa gga ctt gtg cac tcc tat tct gcg cag      355
   Gly Ser Trp Asp Thr Ile Gln Gly Leu Val His Ser Tyr Ser Ala Gln
   70                  75                  80                  85
   gca aaa gcg ctt gtg gag gcg ttc tgg cct ggt gga ctg tcc atc atc      403
   Ala Lys Ala Leu Val Glu Ala Phe Trp Pro Gly Gly Leu Ser Ile Ile
                   90                  95                  100
   gtt ccg cag gca cca agc ctt ccg tgg aac ctt ggc gat acc cgt ggc      451
   Val Pro Gln Ala Pro Ser Leu Pro Trp Asn Leu Gly Asp Thr Arg Gly
               105                 110                 115
   acc gta atg ctg cgc atg cca ctg cac cca gtt gcc att gaa ttg ctg      499
   Thr Val Met Leu Arg Met Pro Leu His Pro Val Ala Ile Glu Leu Leu
           120                 125                 130
   cgc caa acc gga cca atg gct gtc tcc tcc gcc aac atc tcc gga cat      547
   Arg Gln Thr Gly Pro Met Ala Val Ser Ser Ala Asn Ile Ser Gly His
       135                 140                 145
   act cct cca acc acc gtg ctg gag gct cgt cag cag ctc aac caa aat      595
   Thr Pro Pro Thr Thr Val Leu Glu Ala Arg Gln Gln Leu Asn Gln Asn
   150                 155                 160                 165
   gtc gct gtc tac ctc gat ggt ggc gaa tgc gcg ctg gcc acc cct tca      643
   Val Ala Val Tyr Leu Asp Gly Gly Glu Cys Ala Leu Ala Thr Pro Ser
                   170                 175                 180
   acc atc gtg gat att tca ggc ccc gca cca aag att ttg cgt gag ggt      691
   Thr Ile Val Asp Ile Ser Gly Pro Ala Pro Lys Ile Leu Arg Glu Gly
               185                 190                 195
   gcc atc agc gca gaa cgc gtt ggc gaa gta ctt gga gtg tcg gca gaa      739
   Ala Ile Ser Ala Glu Arg Val Gly Glu Val Leu Gly Val Ser Ala Glu
           200                 205                 210
   agc ctg cgc taaatgggag tcggtttcgc ggg                                771
   Ser Leu Arg
       215
<210> 18
   <211> 216
   <212> PRT
   <213> Corynebacterium glutamicum
<400> 18
   Val Ser Arg Ile Tyr Asp Cys Ala Asp Gln Asp Ser Arg Ala Ala Gly
   1               5                   10                  15
   Leu Lys Ala Ala Val Asp Ala Val Lys Ala Gly Gln Leu Val Val Leu
               20                  25                  30
   Pro Thr Asp Thr Leu Tyr Gly Leu Gly Cys Asp Ala Phe Asn Asn Glu
           35                  40                  45
   Ala Val Ala Asn Leu Leu Ala Thr Lys His Arg Gly Pro Asp Met Pro
       50                  55                  60
   Val Pro Val Leu Val Gly Ser Trp Asp Thr Ile Gln Gly Leu Val His
   65                  70                  75                  80
   Ser Tyr Ser Ala Gln Ala Lys Ala Leu Val Glu Ala Phe Trp Pro Gly
                   85                  90                  95
   Gly Leu Ser Ile Ile Val Pro Gln Ala Pro Ser Leu Pro Trp Asn Leu
               100                 105                 110
   Gly Asp Thr Arg Gly Thr Val Met Leu Arg Met Pro Leu His Pro Val
           115                 120                 125
   Ala Ile Glu Leu Leu Arg Gln Thr Gly Pro Met Ala Val Ser Ser Ala
       130                 135                 140
   Asn Ile Ser Gly His Thr Pro Pro Thr Thr Val Leu Glu Ala Arg Gln
   145                 150                 155                 160
   Gln Leu Asn Gln Asn Val Ala Val Tyr Leu Asp Gly Gly Glu Cys Ala
                   165                 170                 175
   Leu Ala Thr Pro Ser Thr Ile Val Asp Ile Ser Gly Pro Ala Pro Lys
               180                 185                 190
   Ile Leu Arg Glu Gly Ala Ile Ser Ala Glu Arg Val Gly Glu Val Leu
           195                 200                 205
   Gly Val Ser Ala Glu Ser Leu Arg
       210                 215
<210> 19
   <211> 1026
   <212> DNA
   <213> Corynebacterium glutamicum
<220>
   <221> CDS
   <222> (101)..(1003)
   <223> RXC00657
<400> 19
   gtgcggatcg ggtatccgcg ctacacttag aggtgttaga gatcatgagt ttccacgaac 60
   tgtaacgcag gattcaccaa tcaatgaaag gtcgaccgac atg agc act gaa gac 115
   Met Ser Thr Glu Asp
   1               5
   att gtc gtc gta gca gta gat ggc tcg gac gcc tca aaa caa gct gtt      163
   Ile Val Val Val Ala Val Asp Gly Ser Asp Ala Ser Lys Gln Ala Val
                   10                  15                  20
   cgg tgg gct gca aat acc gcc aac aaa cgt ggc att cca ctt cgc ttg      211
   Arg Trp Ala Ala Asn Thr Ala Asn Lys Arg Gly Ile Pro Leu Arg Leu
               25                  30                  35
   gct tcc agc tac acc atg cct cag ttc ctc tac gca gag gga atg gtt      259
   Ala Ser Ser Tyr Thr Met Pro Gln Phe Leu Tyr Ala Glu Gly Met Val
           40                  45                  50
   cca cca caa gag ctt ttc gat gac ctc cag gcc gaa gcc ctg gaa aag      307
   Pro Pro Gln Glu Leu Phe Asp Asp Leu Gln Ala Glu Ala Leu Glu Lys
       55                  60                  65
   att aac gaa gcc cgt gac atc gcc cat gag gta gcg cca gaa atc aag      355
   Ile Asn Glu Ala Arg Asp Ile Ala His Glu Val Ala Pro Glu Ile Lys
   70                  75                  80                  85
   atc ggg cac acc atc gct gaa ggc agt ccc atc gac atg ctg ttg gaa      403
   Ile Gly His Thr Ile Ala Glu Gly Ser Pro Ile Asp Met Leu Leu Glu
                   90                  95                  100
   atg tct ccc gat gcc aca atg atc gtc atg ggt tcc cgc gga ctc ggc      451
   Met Ser Pro Asp Ala Thr Met Ile Val Met Gly Ser Arg Gly Leu Gly
               105                 110                 115
   gga ctc tcc gga atg gtc atg ggc tcc gtc tcc ggt gca gtg gtc agc      499
   Gly Leu Ser Gly Met Val Met Gly Ser Val Ser Gly Ala Val Val Ser
           120                 125                 130
   cac gca aag tgt cca gtc gtt gtt gtc cgt gaa gac agc gca gtc aac      547
   His Ala Lys Cys Pro Val Val Val Val Arg Glu Asp Ser Ala Val Asn
       135                 140                 145
   gaa gac agc aag tac ggc cca gtc gtc gtc ggt gtg gat ggc tcc gaa      595
   Glu Asp Ser Lys Tyr Gly Pro Val Val Val Gly Val Asp Gly Ser Glu
   150                 155                 160                 165
   gtc tcc caa cag gca acc gaa tac gca ttt gcg gaa gct gaa gct cgt      643
   Val Ser Gln Gln Ala Thr Glu Tyr Ala Phe Ala Glu Ala Glu Ala Arg
                   170                 175                 180
   ggc gcc gaa ctc gtt gca gtt cac acc tgg atg gac atg cag gta cag      691
   Gly Ala Glu Leu Val Ala Val His Thr Trp Met Asp Met Gln Val Gln
               185                 190                 195
   gca tca ctt gca ggt ctt gca gct gct caa cag cag tgg gat gaa gtg      739
   Ala Ser Leu Ala Gly Leu Ala Ala Ala Gln Gln Gln Trp Asp Glu Val
           200                 205                 210
   gaa cgt cag caa acc gac atg ctg atc gaa cgc ctc gca cca ctg gtg      787
   Glu Arg Gln Gln Thr Asp Met Leu Ile Glu Arg Leu Ala Pro Leu Val
       215                 220                 225
   gaa aag tac cca agt gta acc gtc aag aag atc atc acc cgt gac cgc      835
   Glu Lys Tyr Pro Ser Val Thr Val Lys Lys Ile Ile Thr Arg Asp Arg
   230                 235                 240                 245
   cca gtt cgc gca ctt gca gaa gca tct gaa aac gcg cag ctc cta gtc    883
   Pro Val Arg Ala Leu Ala Glu Ala Ser Glu Asn Ala Gln Leu Leu Val
                   250                 255                 260
   gtt ggt tcc cat ggt cgt ggc gga ttt aag ggc atg ctc ctt ggc tcc    931
   Val Gly Ser His Gly Arg Gly Gly Phe Lys Gly Met Leu Leu Gly Ser
               265                 270                 275
   acc tcc cgc gca ctg ctg caa tcc gca ccg tgc cca atg atg gtg gtt    979
   Thr Ser Arg Ala Leu Leu Gln Ser Ala Pro Cys Pro Met Met Val Val
           280                 285                 290
   cgc cca cct gag aag att aag aag tagtttcttt taagtttcga tgc         1026
   Arg Pro Pro Glu Lys Ile Lys Lys
       295                 300
<210> 20
   <211> 301
   <212> PRT
   <213> Corynebacterium glutamicum
<400> 20
   Met Ser Thr Glu Asp Ile Val Val Val Ala Val Asp Gly Ser Asp Ala
    1               5                  10                  15
   Ser Lys Gln Ala Val Arg Trp Ala Ala Asn Thr Ala Asn Lys Arg Gly
               20                  25                  30
   Ile Pro Leu Arg Leu Ala Ser Ser Tyr Thr Met Pro Gln Phe Leu Tyr
           35                  40                  45
   Ala Glu Gly Met Val Pro Pro Gln Glu Leu Phe Asp Asp Leu Gln Ala
       50                  55                  60
   Glu Ala Leu Glu Lys Ile Asn Glu Ala Arg Asp Ile Ala His Glu Val
   65                  70                  75                  80
   Ala Pro Glu Ile Lys Ile Gly His Thr Ile Ala Glu Gly Ser Pro Ile
                   85                  90                  95
   Asp Met Leu Leu Glu Met Ser Pro Asp Ala Thr Met Ile Val Met Gly
               100                 105                 110
   Ser Arg Gly Leu Gly Gly Leu Ser Gly Met Val Met Gly Ser Val Ser
           115                 120                 125
   Gly Ala Val Val Ser His Ala Lys Cys Pro Val Val Val Val Arg Glu
       130                 135                 140
   Asp Ser Ala Val Asn Glu Asp Ser Lys Tyr Gly Pro Val Val Val Gly
   145                 150                 155                 160
   Val Asp Gly Ser Glu Val Ser Gln Gln Ala Thr Glu Tyr Ala Phe Ala
                   165                 170                 175
   Glu Ala Glu Ala Arg Gly Ala Glu Leu Val Ala Val His Thr Trp Met
               180                 185                 190
   Asp Met Gln Val Gln Ala Ser Leu Ala Gly Leu Ala Ala Ala Gln Gln
           195                 200                 205
   Gln Trp Asp Glu Val Glu Arg Gln Gln Thr Asp Met Leu Ile Glu Arg
       210                 215                 220
   Leu Ala Pro Leu Val Glu Lys Tyr Pro Ser Val Thr Val Lys Lys Ile
   225                 230                 235                 240
   Ile Thr Arg Asp Arg Pro Val Arg Ala Leu Ala Glu Ala Ser Glu Asn
                   245                 250                 255
   Ala Gln Leu Leu Val Val Gly Ser His Gly Arg Gly Gly Phe Lys Gly
               260                 265                 270
   Met Leu Leu Gly Ser Thr Ser Arg Ala Leu Leu Gln Ser Ala Pro Cys
           275                 280                 285
   Pro Met Met Val Val Arg Pro Pro Glu Lys Ile Lys Lys
       290                 295                 300
<210> 21
   <211> 1059
   <212> DNA
   <213> Corynebacterium glutamicum
<220>
   <221> CDS
   <222> (101)..(1036)
   <223> RXC00552
<400> 21
   ccgccaacaa ggcagcaaag ctcgatccaa ttgacgcctt gcgttatgag taaaagcctc 60
   gtttttaagg tagccacaca tcgcactaga ctgaagaact gtg gct acc tca aaa   115
                                             Val Ala Thr Ser Lys
                                             1               5
   att ctt ctt tat tac gca ttc acc ccg ctc tct gac cct aaa gcg gtt   163
   Ile Leu Leu Tyr Tyr Ala Phe Thr Pro Leu Ser Asp Pro Lys Ala Val
                   10                  15                  20
   cag ctg tgg cag cgt gag ctc tgc gag tca ctg aat ctt cgt ggc cgc   211
   Gln Leu Trp Gln Arg Glu Leu Cys Glu Ser Leu Asn Leu Arg Gly Arg
               25                  30                  35
   atc ctg atc tcc act cac ggc atc aat gga acc gtg ggc gga gat att   259
   Ile Leu Ile Ser Thr His Gly Ile Asn Gly Thr Val Gly Gly Asp Ile
           40                  45                  50
   gat gat tgc aag gcg tac att aaa aag acc cgc gag tac cca ggt ttc   307
   Asp Asp Cys Lys Ala Tyr Ile Lys Lys Thr Arg Glu Tyr Pro Gly Phe
       55                  60                  65
   aac cgc atg cag ttt aag tgg tcc gag ggt ggc gct gag gat ttc cca   355
   Asn Arg Met Gln Phe Lys Trp Ser Glu Gly Gly Ala Glu Asp Phe Pro
   70                  75                  80                  85
   aag ctc agt gtc aaa gtc cgc gat gag atc gtt gcc ttc ggc gct cca   403
   Lys Leu Ser Val Lys Val Arg Asp Glu Ile Val Ala Phe Gly Ala Pro
                   90                  95                  100
   gat gag ctc aaa gtg gat gaa aac ggc gtc gtc ggt ggc ggc gtt cac      451
   Asp Glu Leu Lys Val Asp Glu Asn Gly Val Val Gly Gly Gly Val His
               105                 110                 115
   ctg aaa cca cag cag gtc aat gag ctt gtg gaa gcc cgt ggc gat gaa      499
   Leu Lys Pro Gln Gln Val Asn Glu Leu Val Glu Ala Arg Gly Asp Glu
           120                 125                 130
   gtt gtg ttc ttt gac ggc cgc aac gca atg gaa gcc cag atc ggc aag      547
   Val Val Phe Phe Asp Gly Arg Asn Ala Met Glu Ala Gln Ile Gly Lys
       135                 140                 145
   ttc aag gac gct gtt gtc cct gac gta gaa acc act cat gat ttc atc      595
   Phe Lys Asp Ala Val Val Pro Asp Val Glu Thr Thr His Asp Phe Ile
   150                 155                 160                 165
   gca gaa att gag tct gga aaa tac gac gat ctc aaa gac aag cct gtg      643
   Ala Glu Ile Glu Ser Gly Lys Tyr Asp Asp Leu Lys Asp Lys Pro Val
                   170                 175                 180
   gtc acc tac tgc acc ggc gga att cgt tgt gag atc ctg agt tca ctc      691
   Val Thr Tyr Cys Thr Gly Gly Ile Arg Cys Glu Ile Leu Ser Ser Leu
               185                 190                 195
   atg atc aac cgt ggt ttc aaa gag gtc tac caa atc gat ggc ggc atc      739
   Met Ile Asn Arg Gly Phe Lys Glu Val Tyr Gln Ile Asp Gly Gly Ile
           200                 205                 210
   gtt cgc tac ggc gag cag ttt ggc aac aag ggc ctg tgg gaa ggc tcc      787
   Val Arg Tyr Gly Glu Gln Phe Gly Asn Lys Gly Leu Trp Glu Gly Ser
       215                 220                 225
   ctc tac gtt ttc gat aag cgc atg cat atg gaa ttc ggc gag gat tac      835
   Leu Tyr Val Phe Asp Lys Arg Met His Met Glu Phe Gly Glu Asp Tyr
   230                 235                 240                 245
   aaa gag gtc gga cac tgc atc cat tgc gat act ccc acc aac aaa ttt      883
   Lys Glu Val Gly His Cys Ile His Cys Asp Thr Pro Thr Asn Lys Phe
                   250                 255                 260
   gag cac tgc ctc aac gaa gat gat tgc cgc gag ctc gtg ttg atg tgc      931
   Glu His Cys Leu Asn Glu Asp Asp Cys Arg Glu Leu Val Leu Met Cys
               265                 270                 275
   cct gat tgc ttc gcc aat gtt gag acc cgt cat tgc aag cgc gaa cgc      979
   Pro Asp Cys Phe Ala Asn Val Glu Thr Arg His Cys Lys Arg Glu Arg
           280                 285                 290
   tgt gca gca att gct gcg gat ttc gct gag caa gga att gat ccg ctc     1027
   Cys Ala Ala Ile Ala Ala Asp Phe Ala Glu Gln Gly Ile Asp Pro Leu
       295                 300                 305
   gtt act tct taaaaagggt atggtggctg ggt                               1059
   Val Thr Ser
   310
<210> 22
   <211> 312
   <212> PRT
   <213> Corynebacterium glutamicum
<400> 22
   Val Ala Thr Ser Lys Ile Leu Leu Tyr Tyr Ala Phe Thr Pro Leu Ser
   1               5                   10                  15
   Asp Pro Lys Ala Val Gln Leu Trp Gln Arg Glu Leu Cys Glu Ser Leu
               20                  25                  30
   Asn Leu Arg Gly Arg Ile Leu Ile Ser Thr His Gly Ile Asn Gly Thr
           35                  40                  45
   Val Gly Gly Asp Ile Asp Asp Cys Lys Ala Tyr Ile Lys Lys Thr Arg
       50                  55                  60
   Glu Tyr Pro Gly Phe Asn Arg Met Gln Phe Lys Trp Ser Glu Gly Gly
   65                  70                  75                  80
   Ala Glu Asp Phe Pro Lys Leu Ser Val Lys Val Arg Asp Glu Ile Val
                   85                  90                  95
   Ala Phe Gly Ala Pro Asp Glu Leu Lys Val Asp Glu Asn Gly Val Val
               100                 105                 110
   Gly Gly Gly Val His Leu Lys Pro Gln Gln Val Asn Glu Leu Val Glu
           115                 120                 125
   Ala Arg Gly Asp Glu Val Val Phe Phe Asp Gly Arg Asn Ala Met Glu
       130                 135                 140
   Ala Gln Ile Gly Lys Phe Lys Asp Ala Val Val Pro Asp Val Glu Thr
   145                 150                 155                 160
   Thr His Asp Phe Ile Ala Glu Ile Glu Ser Gly Lys Tyr Asp Asp Leu
                   165                 170                 175
   Lys Asp Lys Pro Val Val Thr Tyr Cys Thr Gly Gly Ile Arg Cys Glu
               180                 185                 190
   Ile Leu Ser Ser Leu Met Ile Asn Arg Gly Phe Lys Glu Val Tyr Gln
           195                 200                 205
   Ile Asp Gly Gly Ile Val Arg Tyr Gly Glu Gln Phe Gly Asn Lys Gly
       210                 215                 220
   Leu Trp Glu Gly Ser Leu Tyr Val Phe Asp Lys Arg Met His Met Glu
   225                 230                 235                 240
   Phe Gly Glu Asp Tyr Lys Glu Val Gly His Cys Ile His Cys Asp Thr
                   245                 250                 255
   Pro Thr Asn Lys Phe Glu His Cys Leu Asn Glu Asp Asp Cys Arg Glu
               260                 265                 270
   Leu Val Leu Met Cys Pro Asp Cys Phe Ala Asn Val Glu Thr Arg His
           275                 280                 285
   Cys Lys Arg Glu Arg Cys Ala Ala Ile Ala Ala Asp Phe Ala Glu Gln
       290                 295                 300
   Gly Ile Asp Pro Leu Val Thr Ser
   305                 310
<210> 23
   <211> 1386
   <212> DNA
   <213> Corynebacterium glutamicum
<220>
   <221> CDS
   <222> (101)..(1363)
   <223> RXA00534
<400> 23
   ctgtgcagaa agaaaacact cctctggcta ggtagacaca gtttataaag gtagagttga 60
   gcgggtaact gtcagcacgt agatcgaaag gtgcacaaag gtg gcc ctg gtc gta   115
                                             Val Ala Leu Val Val
                                             1               5
   cag aaa tat ggc ggt tcc tcg ctt gag agt gcg gaa cgc att aga aac   163
   Gln Lys Tyr Gly Gly Ser Ser Leu Glu Ser Ala Glu Arg Ile Arg Asn
                   10                  15                  20
   gtc gct gaa cgg atc gtt gcc acc aag aag gct gga aat gat gtc gtg   211
   Val Ala Glu Arg Ile Val Ala Thr Lys Lys Ala Gly Asn Asp Val Val
               25                  30                  35
   gtt gtc tgc tcc gca atg gga gac acc acg gat gaa ctt cta gaa ctt   259
   Val Val Cys Ser Ala Met Gly Asp Thr Thr Asp Glu Leu Leu Glu Leu
           40                  45                  50
   gca gcg gca gtg aat ccc gtt ccg cca gct cgt gaa atg gat atg ctc   307
   Ala Ala Ala Val Asn Pro Val Pro Pro Ala Arg Glu Met Asp Met Leu
       55                  60                  65
   ctg act gct ggt gag cgt att tct aac gct ctc gtc gcc atg gct att   355
   Leu Thr Ala Gly Glu Arg Ile Ser Asn Ala Leu Val Ala Met Ala Ile
   70                  75                  80                  85
   gag tcc ctt ggc gca gaa gcc caa tct ttc acg ggc tct cag gct ggt   403
   Glu Ser Leu Gly Ala Glu Ala Gln Ser Phe Thr Gly Ser Gln Ala Gly
                   90                  95                  100
   gtg ctc acc acc gag cgc cac gga aac gca cgc att gtt gat gtc act   451
   Val Leu Thr Thr Glu Arg His Gly Asn Ala Arg Ile Val Asp Val Thr
               105                 110                 115
   cca ggt cgt gtg cgt gaa gca ctc gat gag ggc aag atc tgc att gtt   499
   Pro Gly Arg Val Arg Glu Ala Leu Asp Glu Gly Lys Ile Cys Ile Val
           120                 125                 130
   gct ggt ttc cag ggt gtt aat aaa gaa acc cgc gat gtc acc acg ttg   547
   Ala Gly Phe Gln Gly Val Asn Lys Glu Thr Arg Asp Val Thr Thr Leu
       135                 140                 145
   ggt cgt ggt ggt tct gac acc act gca gtt gcg ttg gca gct gct ttg   595
   Gly Arg Gly Gly Ser Asp Thr Thr Ala Val Ala Leu Ala Ala Ala Leu
   150                 155                 160                 165
   aac gct gat gtg tgt gag att tac tcg gac gtt gac ggt gtg tat acc   643
   Asn Ala Asp Val Cys Glu Ile Tyr Ser Asp Val Asp Gly Val Tyr Thr
                   170                 175                 180
   gct gac ccg cgc atc gtt cct aat gca cag aag ctg gaa aag ctc agc    691
   Ala Asp Pro Arg Ile Val Pro Asn Ala Gln Lys Leu Glu Lys Leu Ser
               185                 190                 195
   ttc gaa gaa atg ctg gaa ctt gct gct gtt ggc tcc aag att ttg gtg    739
   Phe Glu Glu Met Leu Glu Leu Ala Ala Val Gly Ser Lys Ile Leu Val
           200                 205                 210
   ctg cgc agt gtt gaa tac gct cgt gca ttc aat gtg cca ctt cgc gta    787
   Leu Arg Ser Val Glu Tyr Ala Arg Ala Phe Asn Val Pro Leu Arg Val
       215                 220                 225
   cgc tcg tct tat agt aat gat ccc ggc act ttg att gcc ggc tct atg    835
   Arg Ser Ser Tyr Ser Asn Asp Pro Gly Thr Leu Ile Ala Gly Ser Met
   230                 235                 240                 245
   gag gat att cct gtg gaa gaa gca gtc ctt acc ggt gtc gca acc gac    883
   Glu Asp Ile Pro Val Glu Glu Ala Val Leu Thr Gly Val Ala Thr Asp
                   250                 255                 260
   aag tcc gaa gcc aaa gta acc gtt ctg ggt att tcc gat aag cca ggc    931
   Lys Ser Glu Ala Lys Val Thr Val Leu Gly Ile Ser Asp Lys Pro Gly
               265                 270                 275
   gag gct gcg aag gtt ttc cgt gcg ttg gct gat gca gaa atc aac att    979
   Glu Ala Ala Lys Val Phe Arg Ala Leu Ala Asp Ala Glu Ile Asn Ile
           280                 285                 290
   gac atg gtt ctg cag aac gtc tct tct gta gaa gac ggc acc acc gac    1027
   Asp Met Val Leu Gln Asn Val Ser Ser Val Glu Asp Gly Thr Thr Asp
       295                 300                 305
   atc acc ttc acc tgc cct cgt tcc gac ggc cgc cgc gcg atg gag atc    1075
   Ile Thr Phe Thr Cys Pro Arg Ser Asp Gly Arg Arg Ala Met Glu Ile
   310                 315                 320                 325
   ttg aag aag ctt cag gtt cag ggc aac tgg acc aat gtg ctt tac gac    1123
   Leu Lys Lys Leu Gln Val Gln Gly Asn Trp Thr Asn Val Leu Tyr Asp
                   330                 335                 340
   gac cag gtc ggc aaa gtc tcc ctc gtg ggt gct ggc atg aag tct cac    1171
   Asp Gln Val Gly Lys Val Ser Leu Val Gly Ala Gly Met Lys Ser His
               345                 350                 355
   cca ggt gtt acc gca gag ttc atg gaa gct ctg cgc gat gtc aac gtg    1219
   Pro Gly Val Thr Ala Glu Phe Met Glu Ala Leu Arg Asp Val Asn Val
           360                 365                 370
   aac atc gaa ttg att tcc acc tct gag att cgt att tcc gtg ctg atc    1267
   Asn Ile Glu Leu Ile Ser Thr Ser Glu Ile Arg Ile Ser Val Leu Ile
       375                 380                 385
   cgt gaa gat gat ctg gat gct gct gca cgt gca ttg cat gag cag ttc    1315
   Arg Glu Asp Asp Leu Asp Ala Ala Ala Arg Ala Leu His Glu Gln Phe
   390                 395                 400                 405
   cag ctg ggc ggc gaa gac gaa gcc gtc gtt tat gca ggc acc gga cgc    1363
   Gln Leu Gly Gly Glu Asp Glu Ala Val Val Tyr Ala Gly Thr Gly Arg
                   410                 415                 420
   taaagtttta aaggagtagt ttt 1386
<210> 24
   <211> 421
   <212> PRT
   <213> Corynebacterium glutamicum
<400> 24
   Val Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 25
   <211> 1155
   <212> DNA
   <213> Corynebacterium glutamicum
<220>
   <221> CDS
   <222> (101)..(1132)
   <223> RXA00533
<400> 25
   ctgcacgtgc attgcatgag cagttccagc tgggcggcga agacgaagcc gtcgtttatg 60
   caggcaccgg acgctaaagt tttaaaggag tagttttaca atg acc acc atc gca   115
                                               Met Thr Thr Ile Ala
                                                1               5
   gtt gtt ggt gca acc ggc cag gtc ggc cag gtt atg cgc acc ctt ttg   163
   Val Val Gly Ala Thr Gly Gln Val Gly Gln Val Met Arg Thr Leu Leu
                   10                  15                  20
   gaa gag cgc aat ttc cca gct gac act gtt cgt ttc ttt gct tcc cca   211
   Glu Glu Arg Asn Phe Pro Ala Asp Thr Val Arg Phe Phe Ala Ser Pro
               25                  30                  35
   cgt tcc gca ggc cgt aag att gaa ttc cgt ggc acg gaa atc gag gta   259
   Arg Ser Ala Gly Arg Lys Ile Glu Phe Arg Gly Thr Glu Ile Glu Val
           40                  45                  50
   gaa gac att act cag gca acc gag gag tcc ctc aag gac atc gac gtt   307
   Glu Asp Ile Thr Gln Ala Thr Glu Glu Ser Leu Lys Asp Ile Asp Val
        55                  60                  65
   gcg ttg ttc tcc gct gga ggc acc gct tcc aag cag tac gct cca ctg      355
   Ala Leu Phe Ser Ala Gly Gly Thr Ala Ser Lys Gln Tyr Ala Pro Leu
    70                  75                  80                  85
   ttc gct gct gca ggc gcg act gtt gtg gat aac tct tct gct tgg cgc      403
   Phe Ala Ala Ala Gly Ala Thr Val Val Asp Asn Ser Ser Ala Trp Arg
                    90                  95                 100
   aag gac gac gag gtt cca cta atc gtc tct gag gtg aac cct tcc gac      451
   Lys Asp Asp Glu Val Pro Leu Ile Val Ser Glu Val Asn Pro Ser Asp
               105                 110                 115
   aag gat tcc ctg gtc aag ggc att att gcg aac cct aac tgc acc acc      499
   Lys Asp Ser Leu Val Lys Gly Ile Ile Ala Asn Pro Asn Cys Thr Thr
           120                 125                 130
   atg gct gcg atg cca gtg ctg aag cca ctt cac gat gcc gct ggt ctt      547
   Met Ala Ala Met Pro Val Leu Lys Pro Leu His Asp Ala Ala Gly Leu
       135                 140                 145
   gta aag ctt cac gtt tcc tct tac cag gct gtt tcc ggt tct ggt ctt      595
   Val Lys Leu His Val Ser Ser Tyr Gln Ala Val Ser Gly Ser Gly Leu
   150                 155                 160                 165
   gca ggt gtg gaa acc ttg gca aag cag gtt gct gca gtt gga gac cac      643
   Ala Gly Val Glu Thr Leu Ala Lys Gln Val Ala Ala Val Gly Asp His
                   170                 175                 180
   aac gtt gag ttc gtc cat gat gga cag gct gct gac gca ggc gat gtc      691
   Asn Val Glu Phe Val His Asp Gly Gln Ala Ala Asp Ala Gly Asp Val
               185                 190                 195
   gga cct tat gtt tca cca atc gct tac aac gtg ctg cca ttc gcc gga      739
   Gly Pro Tyr Val Ser Pro Ile Ala Tyr Asn Val Leu Pro Phe Ala Gly
           200                 205                 210
   aac ctc gtc gat gac ggc acc ttc gaa acc gat gaa gag cag aag ctg      787
   Asn Leu Val Asp Asp Gly Thr Phe Glu Thr Asp Glu Glu Gln Lys Leu
       215                 220                 225
   cgc aac gaa tcc cgc aag att ctc ggt ctc cca gac ctc aag gtc tca      835
   Arg Asn Glu Ser Arg Lys Ile Leu Gly Leu Pro Asp Leu Lys Val Ser
   230                 235                 240                 245
   ggc acc tgc gtc cgc gtg ccg gtt ttc acc ggc cac acg ctg acc att      883
   Gly Thr Cys Val Arg Val Pro Val Phe Thr Gly His Thr Leu Thr Ile
                   250                 255                 260
   cac gcc gaa ttc gac aag gca atc acc gtg gac cag gcg cag gag atc      931
   His Ala Glu Phe Asp Lys Ala Ile Thr Val Asp Gln Ala Gln Glu Ile
               265                 270                 275
   ttg ggt gcc gct tca ggc gtc aag ctt gtc gac gtc cca acc cca ctt      979
   Leu Gly Ala Ala Ser Gly Val Lys Leu Val Asp Val Pro Thr Pro Leu
           280                 285                 290
   gca gct gcc ggc att gac gaa tcc ctc gtt gga cgc atc cgt cag gac     1027
   Ala Ala Ala Gly Ile Asp Glu Ser Leu Val Gly Arg Ile Arg Gln Asp
       295                 300                 305
   tcc act gtc gac gat aac cgc ggt ctg gtt ctc gtc gta tct ggc gac    1075
   Ser Thr Val Asp Asp Asn Arg Gly Leu Val Leu Val Val Ser Gly Asp
   310                 315                 320                 325
   aac ctc cgc aag ggt gct gcg cta aac acc atc cag atc gct gag ctg    1123
   Asn Leu Arg Lys Gly Ala Ala Leu Asn Thr Ile Gln Ile Ala Glu Leu
                   330                 335                 340
   ctg gtt aag taaaaacccg ccattaaaaa ctc                              1155
   Leu Val Lys
<210> 26
   <211> 344
   <212> PRT
   <213> Corynebacterium glutamicum
<400> 26
   Met Thr Thr Ile Ala Val Val Gly Ala Thr Gly Gln Val Gly Gln Val
   1               5                   10                  15
   Met Arg Thr Leu Leu Glu Glu Arg Asn Phe Pro Ala Asp Thr Val Arg
               20                  25                  30
   Phe Phe Ala Ser Pro Arg Ser Ala Gly Arg Lys Ile Glu Phe Arg Gly
           35                  40                  45
   Thr Glu Ile Glu Val Glu Asp Ile Thr Gln Ala Thr Glu Glu Ser Leu
       50                  55                  60
   Lys Asp Ile Asp Val Ala Leu Phe Ser Ala Gly Gly Thr Ala Ser Lys
   65                  70                  75                  80
   Gln Tyr Ala Pro Leu Phe Ala Ala Ala Gly Ala Thr Val Val Asp Asn
                   85                  90                  95
   Ser Ser Ala Trp Arg Lys Asp Asp Glu Val Pro Leu Ile Val Ser Glu
               100                 105                 110
   Val Asn Pro Ser Asp Lys Asp Ser Leu Val Lys Gly Ile Ile Ala Asn
           115                 120                 125
   Pro Asn Cys Thr Thr Met Ala Ala Met Pro Val Leu Lys Pro Leu His
       130                 135                 140
   Asp Ala Ala Gly Leu Val Lys Leu His Val Ser Ser Tyr Gln Ala Val
   145                 150                 155                 160
   Ser Gly Ser Gly Leu Ala Gly Val Glu Thr Leu Ala Lys Gln Val Ala
                   165                 170                 175
   Ala Val Gly Asp His Asn Val Glu Phe Val His Asp Gly Gln Ala Ala
               180                 185                 190
   Asp Ala Gly Asp Val Gly Pro Tyr Val Ser Pro Ile Ala Tyr Asn Val
           195                 200                 205
   Leu Pro Phe Ala Gly Asn Leu Val Asp Asp Gly Thr Phe Glu Thr Asp
   210                 215                 220
   Glu Glu Gln Lys Leu Arg Asn Glu Ser Arg Lys Ile Leu Gly Leu Pro
   225                 230                 235                 240
   Asp Leu Lys Val Ser Gly Thr Cys Val Arg Val Pro Val Phe Thr Gly
                   245                 250                 255
   His Thr Leu Thr Ile His Ala Glu Phe Asp Lys Ala Ile Thr Val Asp
               260                 265                 270
   Gln Ala Gln Glu Ile Leu Gly Ala Ala Ser Gly Val Lys Leu Val Asp
           275                 280                 285
   Val Pro Thr Pro Leu Ala Ala Ala Gly Ile Asp Glu Ser Leu Val Gly
       290                 295                 300
   Arg Ile Arg Gln Asp Ser Thr Val Asp Asp Asn Arg Gly Leu Val Leu
   305                 310                 315                 320
   Val Val Ser Gly Asp Asn Leu Arg Lys Gly Ala Ala Leu Asn Thr Ile
                   325                 330                 335
   Gln Ile Ala Glu Leu Leu Val Lys
               340
<210> 27
   <211> 608
   <212> DNA
   <213> Corynebacterium glutamicum
<220>
   <221> CDS
   <222> (69)..(608)
   <223> RXA02843
<400> 27
   cccattgcgc ggaggtcgca ccccttccga cttgaactga taggccgata gaaattattc 60
   tggacgtc atg act act gct tcc gca acc gga att gca aca ctg acc tcc  110
            Met Thr Thr Ala Ser Ala Thr Gly Ile Ala Thr Leu Thr Ser
             1               5                  10
   acc ggc gac gtc ctg gac gtg tgg tat cca gaa atc ggg tcc acc gac   158
   Thr Gly Asp Val Leu Asp Val Trp Tyr Pro Glu Ile Gly Ser Thr Asp
    15                  20                  25                  30
   cag tcc gcg ctc aca cct cta gaa ggc gtc gat gaa gat cga aac gtc   206
   Gln Ser Ala Leu Thr Pro Leu Glu Gly Val Asp Glu Asp Arg Asn Val
                    35                  40                  45
   acc cgc aaa atc gtg acg aca act atc gac acc gac gca gcc ccc acc   254
   Thr Arg Lys Ile Val Thr Thr Thr Ile Asp Thr Asp Ala Ala Pro Thr
                50                  55                  60
   gac acc tac gat gca tgg ctg cgc ctt cac ctc ctc tcc cac cgc gtt   302
   Asp Thr Tyr Asp Ala Trp Leu Arg Leu His Leu Leu Ser His Arg Val
            65                  70                  75
   ttc cgc cct cac acc atc aac cta gac ggc att ttc ggc ctc ctc aac   350
   Phe Arg Pro His Thr Ile Asn Leu Asp Gly Ile Phe Gly Leu Leu Asn
        80                  85                  90
   aat gtc gtg tgg acc aac ttc gga ccg tgc gca gtt gac ggt ttc gca   398
   Asn Val Val Trp Thr Asn Phe Gly Pro Cys Ala Val Asp Gly Phe Ala
    95                 100                 105                 110
   ctc acc cgc gcg cgc ctg tca cgc cga ggc caa gtt acg gtt tat agc   446
   Leu Thr Arg Ala Arg Leu Ser Arg Arg Gly Gln Val Thr Val Tyr Ser
                   115                 120                 125
   gtc gac aag ttc cca cgc atg gtc gac tat gtg gtt ccc tcg ggc gtg   494
   Val Asp Lys Phe Pro Arg Met Val Asp Tyr Val Val Pro Ser Gly Val
               130                 135                 140
   cgc atc ggt gac gcc gac cgc gtc cga ctt ggc gcg tac ctg gca gat   542
   Arg Ile Gly Asp Ala Asp Arg Val Arg Leu Gly Ala Tyr Leu Ala Asp
           145                 150                 155
   ggc acc acc gtg atg cat gag ggc ttc gtg aac ttc aac gct ggc acg   590
   Gly Thr Thr Val Met His Glu Gly Phe Val Asn Phe Asn Ala Gly Thr
       160                 165                 170
   ctc ggc gct tcc atg gtt                                           608
   Leu Gly Ala Ser Met Val
   175                 180
<210> 26
   <211> 180
   <212> PRT
   <213> Corynebacterium glutamicum
<400> 28
<210> 29
   <211> 1230
   <212> DNA
   <213> Corynebacterium glutamicum
<220>
   <221> CDS
   <222> (101)..(1207)
   <223> RXA02022
<400> 29
   tatttgcgat tccaactgct tgggctccgc gaatgttttc actcattttt taatcgaccg 60
   cttccatcat gttttaacta aggtttgtag gcttaaacct gtg aac tct gaa ctc   115
                                               Val Asn Ser Glu Leu
                                                1               5
   aaa cca gga tta gat ctc ctc ggc gac cca att gtc ctt act caa cgt   163
   Lys Pro Gly Leu Asp Leu Leu Gly Asp Pro Ile Val Leu Thr Gln Arg
                   10                  15                  20
   ttg gta gat ata ccg agt ccg tcg ggt cag gaa aag cag att gct gat   211
   Leu Val Asp Ile Pro Ser Pro Ser Gly Gln Glu Lys Gln Ile Ala Asp
               25                  30                  35
   gaa att gaa gat gcc ctt cgg aac ctt aat cta cct ggt gta gag gtc   259
   Glu Ile Glu Asp Ala Leu Arg Asn Leu Asn Leu Pro Gly Val Glu Val
           40                  45                  50
   ttc cgc ttc aac aac aac gtt ctt gct cgc acg aac agg gga ttg gcc   307
   Phe Arg Phe Asn Asn Asn Val Leu Ala Arg Thr Asn Arg Gly Leu Ala
       55                  60                  65
   tcg agg gtc atg ctt gct ggt cat atc gat aca gtg ccg atc gcg gac   355
   Ser Arg Val Met Leu Ala Gly His Ile Asp Thr Val Pro Ile Ala Asp
   70                  75                  80                  85
   aat ctg cca agc cgt gtg gaa gac ggc atc atg tat ggc tgt ggc acc   403
   Asn Leu Pro Ser Arg Val Glu Asp Gly Ile Met Tyr Gly Cys Gly Thr
                   90                  95                  100
   gtc gat atg aaa tct ggg ttg gcg gtg tat ttg cat act ttt gcc acc   451
   Val Asp Met Lys Ser Gly Leu Ala Val Tyr Leu His Thr Phe Ala Thr
               105                 110                 115
   ttg gcc acg tcg act gag ctt aaa cat gat ctg acg ctg att gcg tat   499
   Leu Ala Thr Ser Thr Glu Leu Lys His Asp Leu Thr Leu Ile Ala Tyr
           120                 125                 130
   gag tgc gag gaa gtt gct gat cac ctc aat ggt ttg ggc cac att cgc   547
   Glu Cys Glu Glu Val Ala Asp His Leu Asn Gly Leu Gly His Ile Arg
       135                 140                 145
   gat gag cat ccg gag tgg ttg gcg gct gat ttg gcg ttg ttg ggt gag      595
   Asp Glu His Pro Glu Trp Leu Ala Ala Asp Leu Ala Leu Leu Gly Glu
   150                 155                 160                 165
   cct act ggc ggc tgg att gag gcg ggc tgc cag ggc aat ctg cgc atc      643
   Pro Thr Gly Gly Trp Ile Glu Ala Gly Cys Gln Gly Asn Leu Arg Ile
                   170                 175                 180
   aag gtg acg gcg cat ggt gtg cgt gcc cat tcg gcg aga agc tgg ttg      691
   Lys Val Thr Ala His Gly Val Arg Ala His Ser Ala Arg Ser Trp Leu
               185                 190                 195
   ggt gat aat gcg atg cat aag ttg tcg ccg atc att tcg aag gtt gct      739
   Gly Asp Asn Ala Met His Lys Leu Ser Pro Ile Ile Ser Lys Val Ala
           200                 205                 210
   gcg tat aag gcc gca gaa gtc aac att gat ggc ttg acc tac cgt gaa      787
   Ala Tyr Lys Ala Ala Glu Val Asn Ile Asp Gly Leu Thr Tyr Arg Glu
       215                 220                 225
   ggc ctc aac atc gtt ttc tgc gaa tcg ggc gtg gca aac aac gtc att      835
   Gly Leu Asn Ile Val Phe Cys Glu Ser Gly Val Ala Asn Asn Val Ile
   230                 235                 240                 245
   cca gac ctc gcg tgg atg aac ctc aac ttc cgt ttc gcg ccg aat cgc      883
   Pro Asp Leu Ala Trp Met Asn Leu Asn Phe Arg Phe Ala Pro Asn Arg
                   250                 255                 260
   gat ctc aac gag gcg atc gag cat gtc gtc gaa acg ctt gag ctt gac      931
   Asp Leu Asn Glu Ala Ile Glu His Val Val Glu Thr Leu Glu Leu Asp
               265                 270                 275
   ggt caa gac ggc atc gaa tgg gcc gta gaa gac ggg gca ggc ggt gcc      979
   Gly Gln Asp Gly Ile Glu Trp Ala Val Glu Asp Gly Ala Gly Gly Ala
           280                 285                 290
   ctt cca ggc ttg ggg cag cag gtg aca agc ggg ctt atc gac gcc gtc     1027
   Leu Pro Gly Leu Gly Gln Gln Val Thr Ser Gly Leu Ile Asp Ala Val
       295                 300                 305
   ggc cgc gaa aaa atc cgc gca aaa ttc ggc tgg acc gat gtc tca cgt     1075
   Gly Arg Glu Lys Ile Arg Ala Lys Phe Gly Trp Thr Asp Val Ser Arg
   310                 315                 320                 325
   ttt tca gcc atg gga att cca gcc cta aac ttt ggc gct ggt gat cca     1123
   Phe Ser Ala Met Gly Ile Pro Ala Leu Asn Phe Gly Ala Gly Asp Pro
                   330                 335                 340
   agt ttc gcg cat aaa cgc gac gag cag tgc cca gtg gag caa atc acg     1171
   Ser Phe Ala His Lys Arg Asp Glu Gln Cys Pro Val Glu Gln Ile Thr
               345                 350                 355
   gat gtg gca gca att ttg aag cag tac ctg agc gag taaccgcatt          1217
   Asp Val Ala Ala Ile Leu Lys Gln Tyr Leu Ser Glu
           360                 365
   cggggttatc gtg                                                      1230
<210> 30
   <211> 369
   <212> PRT
   <213> Corynebacterium glutamicum
<400> 30
   Val Asn Ser Glu Leu Lys Pro Gly Leu Asp Leu Leu Gly Asp Pro Ile
    1               5                  10                  15
   Val Leu Thr Gln Arg Leu Val Asp Ile Pro Ser Pro Ser Gly Gln Glu
               20                  25                  30
   Lys Gln Ile Ala Asp Glu Ile Glu Asp Ala Leu Arg Asn Leu Asn Leu
           35                  40                  45
   Pro Gly Val Glu Val Phe Arg Phe Asn Asn Asn Val Leu Ala Arg Thr
       50                  55                  60
   Asn Arg Gly Leu Ala Ser Arg Val Met Leu Ala Gly His Ile Asp Thr
   65                  70                  75                  80
   Val Pro Ile Ala Asp Asn Leu Pro Ser Arg Val Glu Asp Gly Ile Met
                   85                  90                  95
   Tyr Gly Cys Gly Thr Val Asp Met Lys Ser Gly Leu Ala Val Tyr Leu
               100                 105                 110
   His Thr Phe Ala Thr Leu Ala Thr Ser Thr Glu Leu Lys His Asp Leu
           115                 120                 125
   Thr Leu Ile Ala Tyr Glu Cys Glu Glu Val Ala Asp His Leu Asn Gly
       130                 135                 140
   Leu Gly His Ile Arg Asp Glu His Pro Glu Trp Leu Ala Ala Asp Leu
   145                 150                 155                 160
   Ala Leu Leu Gly Glu Pro Thr Gly Gly Trp Ile Glu Ala Gly Cys Gln
                   165                 170                 175
   Gly Asn Leu Arg Ile Lys Val Thr Ala His Gly Val Arg Ala His Ser
               180                 185                 190
   Ala Arg Ser Trp Leu Gly Asp Asn Ala Met His Lys Leu Ser Pro Ile
           195                 200                 205
   Ile Ser Lys Val Ala Ala Tyr Lys Ala Ala Glu Val Asn Ile Asp Gly
       210                 215                 220
   Leu Thr Tyr Arg Glu Gly Leu Asn Ile Val Phe Cys Glu Ser Gly Val
   225                 230                 235                 240
   Ala Asn Asn Val Ile Pro Asp Leu Ala Trp Met Asn Leu Asn Phe Arg
                   245                 250                 255
   Phe Ala Pro Asn Arg Asp Leu Asn Glu Ala Ile Glu His Val Val Glu
               260                 265                 270
   Thr Leu Glu Leu Asp Gly Gln Asp Gly Ile Glu Trp Ala Val Glu Asp
           275                 280                 285
   Gly Ala Gly Gly Ala Leu Pro Gly Leu Gly Gln Gln Val Thr Ser Gly
   290 295 300
   Leu Ile Asp Ala Val Gly Arg Glu Lys Ile Arg Ala Lys Phe Gly Trp
   305 310 315 320
   Thr Asp Val Ser Arg Phe Ser Ala Met Gly Ile Pro Ala Leu Asn Phe
   325 330 335
   Gly Ala Gly Asp Pro Ser Phe Ala His Lys Arg Asp Glu Gln Cys Pro
   340 345 350
   Val Glu Gln Ile Thr Asp Val Ala Ala Ile Leu Lys Gln Tyr Leu Ser
   355 360 365
   Glu
<210> 31
   <211> 1059
   <212> DNA
   <213> Corynebacterium glutamicum
<220>
   <221> CDS
   <222> (101)..(1036)
   <223> RXA00044
<400> 31
   attacctcag ccttccaagc tgatgatgca ttacttaaaa actgcagaca cttgaaaaac 60
   ttctcacccg cactcgttcc ctcaacccac aaggagcacc atg gct tcc gca act   115
                                               Met Ala Ser Ala Thr
                                                1               5
   ttc acc ggc gtg atc cca ccc gta atg acc cca ctc cac gcc gac ggc   163
   Phe Thr Gly Val Ile Pro Pro Val Met Thr Pro Leu His Ala Asp Gly
                    10                  15                  20
   agt gtg gat gta gaa agc ctc cgc aag ctc gtt gac cac ctc atc aat   211
   Ser Val Asp Val Glu Ser Leu Arg Lys Leu Val Asp His Leu Ile Asn
                25                  30                  35
   ggt ggc gtc gac gga ctt ttc gca ctg ggc tcc tca ggc gaa gcg gca   259
   Gly Gly Val Asp Gly Leu Phe Ala Leu Gly Ser Ser Gly Glu Ala Ala
            40                  45                  50
   ttc ctc acc cgc gcc cag cgc aaa ctc gca ctg acc acc atc atc gag   307
   Phe Leu Thr Arg Ala Gln Arg Lys Leu Ala Leu Thr Thr Ile Ile Glu
        55                  60                  65
   cac acc gca ggc cgc gtt ccc gta act gct ggt gtc att gaa acc acc   355
   His Thr Ala Gly Arg Val Pro Val Thr Ala Gly Val Ile Glu Thr Thr
    70                  75                  80                  85
   act gct cgc gtg att gag ctc gtg gaa gat gcc ctg gag gct ggt gcc   403
   Thr Ala Arg Val Ile Glu Leu Val Glu Asp Ala Leu Glu Ala Gly Ala
                    90                  95                  100
   gaa ggc ctc gtt gcc act gca cct ttc tac acc cgc acc cac gat gtg   451
   Glu Gly Leu Val Ala Thr Ala Pro Phe Tyr Thr Arg Thr His Asp Val
               105                 110                 115
   gaa att gaa gaa cac ttc cgc aag atc cac gcc gcc gct cca gag ctt      499
   Glu Ile Glu Glu His Phe Arg Lys Ile His Ala Ala Ala Pro Glu Leu
           120                 125                 130
   cca ctg ttt gcc tac aac atc cca gtg tcg gtg cac tcc aac ctc aac      547
   Pro Leu Phe Ala Tyr Asn Ile Pro Val Ser Val His Ser Asn Leu Asn
       135                 140                 145
   cca gtc atg ctt ttg acg ctg gcc aag gat ggc gtt ctt gca ggc acc      595
   Pro Val Met Leu Leu Thr Leu Ala Lys Asp Gly Val Leu Ala Gly Thr
   150                 155                 160                 165
   aag gat tcc agt ggc aat gat ggc gca atc cgc tca ctg atc gaa gct      643
   Lys Asp Ser Ser Gly Asn Asp Gly Ala Ile Arg Ser Leu Ile Glu Ala
                   170                 175                 180
   cgt gat gat gct gga ctc act gag cag ttc aag atc ctc acc ggc agc      691
   Arg Asp Asp Ala Gly Leu Thr Glu Gln Phe Lys Ile Leu Thr Gly Ser
               185                 190                 195
   gaa acc acc gtt gat ttc gcc tac ctt gcg ggt gcc gat gga gtt gtc      739
   Glu Thr Thr Val Asp Phe Ala Tyr Leu Ala Gly Ala Asp Gly Val Val
           200                 205                 210
   cca ggc ctg ggc aat gtt gat cct gca gca tac gca gct tta gca aaa      787
   Pro Gly Leu Gly Asn Val Asp Pro Ala Ala Tyr Ala Ala Leu Ala Lys
       215                 220                 225
   ctc tgc ctc gat gga aag tgg gca gaa gct gct gct ttg cag aag cgc      835
   Leu Cys Leu Asp Gly Lys Trp Ala Glu Ala Ala Ala Leu Gln Lys Arg
   230                 235                 240                 245
   atc aac cac ctc ttc cac atc gtc ttc gtg gga gac acc tcc cat atg      883
   Ile Asn His Leu Phe His Ile Val Phe Val Gly Asp Thr Ser His Met
                   250                 255                 260
   tcc gga tcc agc gct ggt ttg ggc ggt ttc aag aca gca ctc gca cac      931
   Ser Gly Ser Ser Ala Gly Leu Gly Gly Phe Lys Thr Ala Leu Ala His
               265                 270                 275
   ctt ggc att att gaa tcc aat gcg atg gca gtt cct cac cag agc ctc      979
   Leu Gly Ile Ile Glu Ser Asn Ala Met Ala Val Pro His Gln Ser Leu
           280                 285                 290
   agc gac gaa gaa act gct cgc att cac gcc att gtt gat gaa ttc ctg     1027
   Ser Asp Glu Glu Thr Ala Arg Ile His Ala Ile Val Asp Glu Phe Leu
       295                 300                 305
   tac acc gct taaggcccac acctcatgac tga                              1059
   Tyr Thr Ala
   310
<210> 32
   <211> 312
   <212> PRT
   <213> Corynebacterium glutamicum
<400> 32
   Met Ala Ser Ala Thr Phe Thr Gly Val Ile Pro Pro Val Met Thr Pro
   1               5                   10                  15
   Leu His Ala Asp Gly Ser Val Asp Val Glu Ser Leu Arg Lys Leu Val
               20                  25                  30
   Asp His Leu Ile Asn Gly Gly Val Asp Gly Leu Phe Ala Leu Gly Ser
           35                  40                  45
   Ser Gly Glu Ala Ala Phe Leu Thr Arg Ala Gln Arg Lys Leu Ala Leu
       50                  55                  60
   Thr Thr Ile Ile Glu His Thr Ala Gly Arg Val Pro Val Thr Ala Gly
   65                  70                  75                  80
   Val Ile Glu Thr Thr Thr Ala Arg Val Ile Glu Leu Val Glu Asp Ala
                   85                  90                  95
   Leu Glu Ala Gly Ala Glu Gly Leu Val Ala Thr Ala Pro Phe Tyr Thr
               100                 105                 110
   Arg Thr His Asp Val Glu Ile Glu Glu His Phe Arg Lys Ile His Ala
           115                 120                 125
   Ala Ala Pro Glu Leu Pro Leu Phe Ala Tyr Asn Ile Pro Val Ser Val
       130                 135                 140
   His Ser Asn Leu Asn Pro Val Met Leu Leu Thr Leu Ala Lys Asp Gly
   145                 150                 155                 160
   Val Leu Ala Gly Thr Lys Asp Ser Ser Gly Asn Asp Gly Ala Ile Arg
                   165                 170                 175
   Ser Leu Ile Glu Ala Arg Asp Asp Ala Gly Leu Thr Glu Gln Phe Lys
               180                 185                 190
   Ile Leu Thr Gly Ser Glu Thr Thr Val Asp Phe Ala Tyr Leu Ala Gly
           195                 200                 205
   Ala Asp Gly Val Val Pro Gly Leu Gly Asn Val Asp Pro Ala Ala Tyr
       210                 215                 220
   Ala Ala Leu Ala Lys Leu Cys Leu Asp Gly Lys Trp Ala Glu Ala Ala
   225                 230                 235                 240
   Ala Leu Gln Lys Arg Ile Asn His Leu Phe His Ile Val Phe Val Gly
                   245                 250                 255
   Asp Thr Ser His Met Ser Gly Ser Ser Ala Gly Leu Gly Gly Phe Lys
               260                 265                 270
   Thr Ala Leu Ala His Leu Gly Ile Ile Glu Ser Asn Ala Met Ala Val
           275                 280                 285
   Pro His Gln Ser Leu Ser Asp Glu Glu Thr Ala Arg Ile His Ala Ile
       290                 295                 300
   Val Asp Glu Phe Leu Tyr Thr Ala
   305                 310
<210> 33
   <211> 867
   <212> DNA
   <213> Corynebacterium glutamicum
<220>
   <221> CDS
   <222> (101)..(844)
   <223> RXA00863
<400> 33
   aacggtcagt taggtatgga tatcagcacc ttctgaacgg gtacgtctag actggtgggc 60
   gtttgaaaaa ctcttcgccc cacgaaaatg aaggagcata atg gga atc aag gtt   115
                                              Met Gly Ile Lys Val
                                               1               5
   ggc gtt ctc gga gcc aaa ggc cgt gtt ggt caa act att gtg gca gca   163
   Gly Val Leu Gly Ala Lys Gly Arg Val Gly Gln Thr Ile Val Ala Ala
                   10                  15                  20
   gtc aat gag tcc gac gat ctg gag ctt gtt gca gag atc ggc gtc gac   211
   Val Asn Glu Ser Asp Asp Leu Glu Leu Val Ala Glu Ile Gly Val Asp
               25                  30                  35
   gat gat ttg agc ctt ctg gta gac aac ggc gct gaa gtt gtc gtt gac   259
   Asp Asp Leu Ser Leu Leu Val Asp Asn Gly Ala Glu Val Val Val Asp
           40                  45                  50
   ttc acc act cct aac gct gtg atg ggc aac ctg gag ttc tgc atc aac   307
   Phe Thr Thr Pro Asn Ala Val Met Gly Asn Leu Glu Phe Cys Ile Asn
       55                  60                  65
   aac ggc att tct gcg gtt gtt gga acc acg ggc ttc gat gat gct cgt   355
   Asn Gly Ile Ser Ala Val Val Gly Thr Thr Gly Phe Asp Asp Ala Arg
   70                  75                  80                  85
   ttg gag cag gtt cgc gac tgg ctt gaa gga aaa gac aat gtc ggt gtt   403
   Leu Glu Gln Val Arg Asp Trp Leu Glu Gly Lys Asp Asn Val Gly Val
                   90                  95                  100
   ctg atc gca cct aac ttt gct atc tct gcg gtg ttg acc atg gtc ttt   451
   Leu Ile Ala Pro Asn Phe Ala Ile Ser Ala Val Leu Thr Met Val Phe
               105                 110                 115
   tcc aag cag gct gcc cgc ttc ttc gaa tca gct gaa gtt att gag ctg   499
   Ser Lys Gln Ala Ala Arg Phe Phe Glu Ser Ala Glu Val Ile Glu Leu
           120                 125                 130
   cac cac ccc aac aag ctg gat gca cct tca ggc acc gcg atc cac act   547
   His His Pro Asn Lys Leu Asp Ala Pro Ser Gly Thr Ala Ile His Thr
       135                 140                 145
   gct cag ggc att gct gcg gca cgc aaa gaa gca ggc atg gac gca cag   595
   Ala Gln Gly Ile Ala Ala Ala Arg Lys Glu Ala Gly Met Asp Ala Gln
   150                 155                 160                 165
   cca gat gcg acc gag cag gca ctt gag ggt tcc cgt ggc gca agc gta   643
   Pro Asp Ala Thr Glu Gln Ala Leu Glu Gly Ser Arg Gly Ala Ser Val
                   170                 175                 180
   gat gga atc ccg gtt cat gca gtc cgc atg tcc ggc atg gtt gct cac     691
   Asp Gly Ile Pro Val His Ala Val Arg Met Ser Gly Met Val Ala His
               185                 190                     195
   gag caa gtt atc ttt ggc acc cag ggt cag acc ttg acc atc aag cag     739
   Glu Gln Val Ile Phe Gly Thr Gln Gly Gln Thr Leu Thr Ile Lys Gln
           200                 205                     210
   gac tcc tat gat cgc aac tca ttt gca cca ggt gtc ttg gtg ggt gtg     787
   Asp Ser Tyr Asp Arg Asn Ser Phe Ala Pro Gly Val Leu Val Gly Val
       215                     220                 225
   cgc aac att gca cag cac cca ggc cta gtc gta gga ctt gag cat tac     835
   Arg Asn Ile Ala Gln His Pro Gly Leu Val Val Gly Leu Glu His Tyr
   230                     235                 240                     245
   cta ggc ctg taaaggctca tttcagcagc ggg                               867
   Leu Gly Leu
<210> 34
   <211> 248
   <212> PRT
   <213> Corynebacterium glutamicum
<400> 34
   Met Gly Ile Lys Val Gly Val Leu Gly Ala Lys Gly Arg Val Gly Gln
   1 5 10 15
   Thr Ile Val Ala Ala Val Asn Glu Ser Asp Asp Leu Glu Leu Val Ala
   20 25 30
   Glu Ile Gly Val Asp Asp Asp Leu Ser Leu Leu Val Asp Asn Gly Ala
   35 40 45
   Glu Val Val Val Asp Phe Thr Thr Pro Asn Ala Val Met Gly Asn Leu
   50 55 60
   Glu Phe Cys Ile Asn Asn Gly Ile Ser Ala Val Val Gly Thr Thr Gly
   65 70 75 80
   Phe Asp Asp Ala Arg Leu Glu Gln Val Arg Asp Trp Leu Glu Gly Lys
   85 90 95
   Asp Asn Val Gly Val Leu Ile Ala Pro Asn Phe Ala Ile Ser Ala Val
   100 105 110
   Leu Thr Met Val Phe Ser Lys Gln Ala Ala Arg Phe Phe Glu Ser Ala
   115 120 125
   Glu Val Ile Glu Leu His His Pro Asn Lys Leu Asp Ala Pro Ser Gly
   130 135 140
   Thr Ala Ile His Thr Ala Gln Gly Ile Ala Ala Ala Arg Lys Glu Ala
   145 150 155 160
   Gly Met Asp Ala Gln Pro Asp Ala Thr Glu Gln Ala Leu Glu Gly Ser
   165 170 175
   Arg Gly Ala Ser Val Asp Gly Ile Pro Val His Ala Val Arg Met Ser
   180 185 190
   Gly Met Val Ala His Glu Gln Val Ile Phe Gly Thr Gln Gly Gln Thr
   195 200 205
   Leu Thr Ile Lys Gln Asp Ser Tyr Asp Arg Asn Ser Phe Ala Pro Gly
   210 215 220
   Val Leu Val Gly Val Arg Asn Ile Ala Gln His Pro Gly Leu Val Val
   225 230 235 240
   Gly Leu Glu His Tyr Leu Gly Leu
   245
<210> 35
   <211> 873
   <212> DNA
   <213> Corynebacterium glutamicum
<220>
   <221> CDS
   <222> (101)..(850)
   <223> RXA00864
<400> 35
   acagcaccca ggcctagtcg taggacttga gcattaccta ggcctgtaaa ggctcatttc 60
   agcagcgggt ggaatttttt aaaaggagcg tttaaaggct gtg gcc gaa caa gtt   115
                                              Val Ala Glu Gln Val
                                               1               5
   aaa ttg agc gtg gag ttg ata gcg tgc agt tct ttt act cca ccc gct   163
   Lys Leu Ser Val Glu Leu Ile Ala Cys Ser Ser Phe Thr Pro Pro Ala
                   10                  15                  20
   gat gtt gag tgg tca act gat gtt gag ggc gcg gaa gca ctc gtc gag   211
   Asp Val Glu Trp Ser Thr Asp Val Glu Gly Ala Glu Ala Leu Val Glu
               25                  30                  35
   ttt gcg ggt cgt gcc tgc tac gaa act ttt gat aag ccg aac cct cga   259
   Phe Ala Gly Arg Ala Cys Tyr Glu Thr Phe Asp Lys Pro Asn Pro Arg
           40                  45                  50
   act gct tcc aat gct gcg tat ctg cgc cac atc atg gaa gtg ggg cac   307
   Thr Ala Ser Asn Ala Ala Tyr Leu Arg His Ile Met Glu Val Gly His
       55                  60                  65
   act gct ttg ctt gag cat gcc aat gcc acg atg tat atc cga ggc att   355
   Thr Ala Leu Leu Glu His Ala Asn Ala Thr Met Tyr Ile Arg Gly Ile
   70                  75                  80                  85
   tct cgg tcc gcg acc cat gaa ttg gtc cga cac cgc cat ttt tcc ttc   403
   Ser Arg Ser Ala Thr His Glu Leu Val Arg His Arg His Phe Ser Phe
                   90                  95                  100
   tct caa ctg tct cag cgt ttc gtg cac agc gga gaa tcg gaa gta gtg   451
   Ser Gln Leu Ser Gln Arg Phe Val His Ser Gly Glu Ser Glu Val Val
               105                 110                 115
   gtg ccc act ctc atc gat gaa gat ccg cag ttg cgt gaa ctt ttc atg   499
   Val Pro Thr Leu Ile Asp Glu Asp Pro Gln Leu Arg Glu Leu Phe Met
           120                 125                 130
   cac gcc atg gat gag tct cgg ttc gct ttc aat gag ctg ctt aat gcg     547
   His Ala Met Asp Glu Ser Arg Phe Ala Phe Asn Glu Leu Leu Asn Ala
       135                 140                 145
   ctg gaa gaa aaa ctt ggc gat gaa ccg aat gca ctt tta agg aaa aag     595
   Leu Glu Glu Lys Leu Gly Asp Glu Pro Asn Ala Leu Leu Arg Lys Lys
   150                 155                 160                 165
   cag gct cgt caa gca gct cgc gct gtg ctg ccc aac gct aca gag tcc     643
   Gln Ala Arg Gln Ala Ala Arg Ala Val Leu Pro Asn Ala Thr Glu Ser
                   170                 175                 180
   aga atc gtg gtg tct gga aac ttc cgc acc tgg agg cat ttc att ggc     691
   Arg Ile Val Val Ser Gly Asn Phe Arg Thr Trp Arg His Phe Ile Gly
               185                 190                 195
   atg cga gcc agt gaa cat gca gac gtc gaa atc cgc gaa gta gcg gta     739
   Met Arg Ala Ser Glu His Ala Asp Val Glu Ile Arg Glu Val Ala Val
           200                 205                 210
   gaa tgt tta aga aag ctg cag gta gca gcg cca act gtt ttc ggt gat     787
   Glu Cys Leu Arg Lys Leu Gln Val Ala Ala Pro Thr Val Phe Gly Asp
       215                 220                 225
   ttt gag att gaa act ttg gca gac gga tcg caa atg gca aca agc ccg     835
   Phe Glu Ile Glu Thr Leu Ala Asp Gly Ser Gln Met Ala Thr Ser Pro
   230                 235                 240                 245
   tat gtc atg gac ttt taacgcaaag ctcacaccca cga                       873
   Tyr Val Met Asp Phe
                   250
<210> 36
   <211> 250
   <212> PRT
   <213> Corynebacterium glutamicum
<400> 36
   Val Ala Glu Gln Val Lys Leu Ser Val Glu Leu Ile Ala Cys Ser Ser
    1               5                  10                  15
   Phe Thr Pro Pro Ala Asp Val Glu Trp Ser Thr Asp Val Glu Gly Ala
               20                  25                  30
   Glu Ala Leu Val Glu Phe Ala Gly Arg Ala Cys Tyr Glu Thr Phe Asp
           35                  40                  45
   Lys Pro Asn Pro Arg Thr Ala Ser Asn Ala Ala Tyr Leu Arg His Ile
       50                  55                  60
   Met Glu Val Gly His Thr Ala Leu Leu Glu His Ala Asn Ala Thr Met
   65                  70                  75                  80
   Tyr Ile Arg Gly Ile Ser Arg Ser Ala Thr His Glu Leu Val Arg His
                   85                  90                  95
   Arg His Phe Ser Phe Ser Gln Leu Ser Gln Arg Phe Val His Ser Gly
               100                 105                 110
   Glu Ser Glu Val Val Val Pro Thr Leu Ile Asp Glu Asp Pro Gln Leu
           115                 120                 125
   Arg Glu Leu Phe Met His Ala Met Asp Glu Ser Arg Phe Ala Phe Asn
       130                 135                 140
   Glu Leu Leu Asn Ala Leu Glu Glu Lys Leu Gly Asp Glu Pro Asn Ala
   145                 150                 155                 160
   Leu Leu Arg Lys Lys Gln Ala Arg Gln Ala Ala Arg Ala Val Leu Pro
                   165                 170                 175
   Asn Ala Thr Glu Ser Arg Ile Val Val Ser Gly Asn Phe Arg Thr Trp
               180                 185                 190
   Arg His Phe Ile Gly Met Arg Ala Ser Glu His Ala Asp Val Glu Ile
           195                 200                 205
   Arg Glu Val Ala Val Glu Cys Leu Arg Lys Leu Gln Val Ala Ala Pro
       210                 215                 220
   Thr Val Phe Gly Asp Phe Glu Ile Glu Thr Leu Ala Asp Gly Ser Gln
   225                 230                 235                 240
   Met Ala Thr Ser Pro Tyr Val Met Asp Phe
                   245                 250
<210> 37
   <211> 608
   <212> DNA
   <213> Corynebacterium glutamicum
<220>
   <221> CDS
   <222> (69)..(608)
   <223> RXA02843
<400> 37
   cccattgcgc ggaggtcgca ccccttccga cttgaactga taggccgata gaaattattc 60
   tggacgtc atg act act gct tcc gca acc gga att gca aca ctg acc tcc  110
            Met Thr Thr Ala Ser Ala Thr Gly Ile Ala Thr Leu Thr Ser
             1               5                  10
   acc ggc gac gtc ctg gac gtg tgg tat cca gaa atc ggg tcc acc gac   158
   Thr Gly Asp Val Leu Asp Val Trp Tyr Pro Glu Ile Gly Ser Thr Asp
    15                  20                  25                  30
   cag tcc gcg ctc aca cct cta gaa ggc gtc gat gaa gat cga aac gtc   206
   Gln Ser Ala Leu Thr Pro Leu Glu Gly Val Asp Glu Asp Arg Asn Val
                    35                  40                  45
   acc cgc aaa atc gtg acg aca act atc gac acc gac gca gcc ccc acc   254
   Thr Arg Lys Ile Val Thr Thr Thr Ile Asp Thr Asp Ala Ala Pro Thr
                50                  55                  60
   gac acc tac gat gca tgg ctg cgc ctt cac ctc ctc tcc cac cgc gtt   302
   Asp Thr Tyr Asp Ala Trp Leu Arg Leu His Leu Leu Ser His Arg Val
            65                  70                  75
   ttc cgc cct cac acc atc aac cta gac ggc att ttc ggc ctc ctc aac   350
   Phe Arg Pro His Thr Ile Asn Leu Asp Gly Ile Phe Gly Leu Leu Asn
        80                  85                  90
   aat gtc gtg tgg acc aac ttc gga ccg tgc gca gtt gac ggt ttc gca   398
   Asn Val Val Trp Thr Asn Phe Gly Pro Cys Ala Val Asp Gly Phe Ala
    95                 100                 105                 110
   ctc acc cgc gcg cgc ctg tca cgc cga ggc caa gtt acg gtt tat agc   446
   Leu Thr Arg Ala Arg Leu Ser Arg Arg Gly Gln Val Thr Val Tyr Ser
                   115                 120                 125
   gtc gac aag ttc cca cgc atg gtc gac tat gtg gtt ccc tcg ggc gtg   494
   Val Asp Lys Phe Pro Arg Met Val Asp Tyr Val Val Pro Ser Gly Val
               130                 135                 140
   cgc atc ggt gac gcc gac cgc gtc cga ctt ggc gcg tac ctg gca gat   542
   Arg Ile Gly Asp Ala Asp Arg Val Arg Leu Gly Ala Tyr Leu Ala Asp
           145                 150                 155
   ggc acc acc gtg atg cat gag ggc ttc gtg aac ttc aac gct ggc acg   590
   Gly Thr Thr Val Met His Glu Gly Phe Val Asn Phe Asn Ala Gly Thr
       160                 165                 170
   ctc ggc gct tcc atg gtt                                           608
   Leu Gly Ala Ser Met Val
   175                 180
<210> 38
   <211> 180
   <212> PRT
   <213> Corynebacterium glutamicum
<400> 38
   Met Thr Thr Ala Ser Ala Thr Gly Ile Ala Thr Leu Thr Ser Thr Gly
   1               5                   10                  15
   Asp Val Leu Asp Val Trp Tyr Pro Glu Ile Gly Ser Thr Asp Gln Ser
               20                  25                  30
   Ala Leu Thr Pro Leu Glu Gly Val Asp Glu Asp Arg Asn Val Thr Arg
           35                  40                  45
   Lys Ile Val Thr Thr Thr Ile Asp Thr Asp Ala Ala Pro Thr Asp Thr
       50                  55                  60
   Tyr Asp Ala Trp Leu Arg Leu His Leu Leu Ser His Arg Val Phe Arg
   65                  70                  75                  80
   Pro His Thr Ile Asn Leu Asp Gly Ile Phe Gly Leu Leu Asn Asn Val
                   85                  90                  95
   Val Trp Thr Asn Phe Gly Pro Cys Ala Val Asp Gly Phe Ala Leu Thr
               100                 105                 110
   Arg Ala Arg Leu Ser Arg Arg Gly Gln Val Thr Val Tyr Ser Val Asp
           115                 120                 125
   Lys Phe Pro Arg Met Val Asp Tyr Val Val Pro Ser Gly Val Arg Ile
       130                 135                 140
   Gly Asp Ala Asp Arg Val Arg Leu Gly Ala Tyr Leu Ala Asp Gly Thr
   145                 150                 155                 160
   Thr Val Met His Glu Gly Phe Val Asn Phe Asn Ala Gly Thr Leu Gly
                   165                 170                 175
   Ala Ser Met Val
               180
<210> 39
   <211> 1143
   <212> DNA
   <213> Corynebacterium glutamicum
<220>
   <221> CDS
   <222> (101)..(1120)
   <223> RXN00355
<400> 39
   aatagatcag cgcatccgtg gtggaaccaa aaggctcaac aatacgaaac gttcgctttc 60
   ggtcctgatg aaagagatgt ccctgaatca tcatctaagt atg cat ctc ggt aag   115
                                               Met His Leu Gly Lys
                                                1               5
   ctc gac cag gac agt gcc acc aca att ttg gag gat tac aag aac atg   163
   Leu Asp Gln Asp Ser Ala Thr Thr Ile Leu Glu Asp Tyr Lys Asn Met
                   10                  15                  20
   acc aac atc cgc gta gct atc gtg ggc tac gga aac ctg gga cgc agc   211
   Thr Asn Ile Arg Val Ala Ile Val Gly Tyr Gly Asn Leu Gly Arg Ser
               25                  30                  35
   gtc gaa aag ctt att gcc aag cag ccc gac atg gac ctt gta gga atc   259
   Val Glu Lys Leu Ile Ala Lys Gln Pro Asp Met Asp Leu Val Gly Ile
           40                  45                  50
   ttc tcg cgc cgg gcc acc ctc gac aca aag acg cca gtc ttt gat gtc   307
   Phe Ser Arg Arg Ala Thr Leu Asp Thr Lys Thr Pro Val Phe Asp Val
       55                  60                  65
   gcc gac gtg gac aag cac gcc gac gac gtg gac gtg ctg ttc ctg tgc   355
   Ala Asp Val Asp Lys His Ala Asp Asp Val Asp Val Leu Phe Leu Cys
   70                  75                  80                  85
   atg ggc tcc gcc acc gac atc cct gag cag gca cca aag ttc gcg cag   403
   Met Gly Ser Ala Thr Asp Ile Pro Glu Gln Ala Pro Lys Phe Ala Gln
                   90                  95                  100
   ttc gcc tgc acc gta gac acc tac gac aac cac cgc gac atc cca cgc   451
   Phe Ala Cys Thr Val Asp Thr Tyr Asp Asn His Arg Asp Ile Pro Arg
               105                 110                 115
   cac cgc cag gtc atg aac gaa gcc gcc acc gca gcc ggc aac gtt gca   499
   His Arg Gln Val Met Asn Glu Ala Ala Thr Ala Ala Gly Asn Val Ala
           120                 125                 130
   ctg gtc tct acc ggc tgg gat cca gga atg ttc tcc atc aac cgc gtc      547
   Leu Val Ser Thr Gly Trp Asp Pro Gly Met Phe Ser Ile Asn Arg Val
       135                 140                 145
   tac gca gcg gca gtc tta gcc gag cac cag cag cac acc ttc tgg ggc      595
   Tyr Ala Ala Ala Val Leu Ala Glu His Gln Gln His Thr Phe Trp Gly
   150                 155                 160                 165
   cca ggt ttg tca cag ggc cac tcc gat gct ttg cga cgc atc cct ggc      643
   Pro Gly Leu Ser Gln Gly His Ser Asp Ala Leu Arg Arg Ile Pro Gly
                   170                 175                 180
   gtt caa aag gca gtc cag tac acc ctc cca tcc gaa gac gcc ctg gaa      691
   Val Gln Lys Ala Val Gln Tyr Thr Leu Pro Ser Glu Asp Ala Leu Glu
               185                 190                 195
   aag gcc cgc cgc ggc gaa gcc ggc gac ctt acc gga aag caa acc cac      739
   Lys Ala Arg Arg Gly Glu Ala Gly Asp Leu Thr Gly Lys Gln Thr His
           200                 205                 210
   aag cgc caa tgc ttc gtg gtt gcc gac gcg gcc gat cac gag cgc atc      787
   Lys Arg Gln Cys Phe Val Val Ala Asp Ala Ala Asp His Glu Arg Ile
       215                 220                 225
   gaa aac gac atc cgc acc atg cct gat tac ttc gtt ggc tac gaa gtc      835
   Glu Asn Asp Ile Arg Thr Met Pro Asp Tyr Phe Val Gly Tyr Glu Val
   230                 235                 240                 245
   gaa gtc aac ttc atc gac gaa gca acc ttc gac tcc gag cac acc ggc      883
   Glu Val Asn Phe Ile Asp Glu Ala Thr Phe Asp Ser Glu His Thr Gly
                   250                 255                 260
   atg cca cac ggt ggc cac gtg att acc acc ggc gac acc ggt ggc ttc      931
   Met Pro His Gly Gly His Val Ile Thr Thr Gly Asp Thr Gly Gly Phe
               265                 270                 275
   aac cac acc gtg gaa tac atc ctc aag ctg gac cga aac cca gat ttc      979
   Asn His Thr Val Glu Tyr Ile Leu Lys Leu Asp Arg Asn Pro Asp Phe
           280                 285                 290
   acc gct tcc tca cag atc gct ttc ggt cgc gca gct cac cgc atg aag      1027
   Thr Ala Ser Ser Gln Ile Ala Phe Gly Arg Ala Ala His Arg Met Lys
       295                 300                 305
   cag cag ggc caa agc gga gct ttc acc gtc ctc gaa gtt gct cca tac      1075
   Gln Gln Gly Gln Ser Gly Ala Phe Thr Val Leu Glu Val Ala Pro Tyr
   310                 315                 320                 325
   ctg ctc tcc cca gag aac ttg gac gat ctg atc gca cgc gac gtc          1120
   Leu Leu Ser Pro Glu Asn Leu Asp Asp Leu Ile Ala Arg Asp Val
                   330                 335                 340
   taatttagct cgaggggcaa gga                                            1143
<210> 40
   <211> 340
   <212> PRT
   <213> Corynebacterium glutamicum
<400> 40
   Met His Leu Gly Lys Leu Asp Gln Asp Ser Ala Thr Thr Ile Leu Glu
   1               5                   10                  15
   Asp Tyr Lys Asn Met Thr Asn Ile Arg Val Ala Ile Val Gly Tyr Gly
               20                  25                  30
   Asn Leu Gly Arg Ser Val Glu Lys Leu Ile Ala Lys Gln Pro Asp Met
           35                  40                  45
   Asp Leu Val Gly Ile Phe Ser Arg Arg Ala Thr Leu Asp Thr Lys Thr
       50                  55                  60
   Pro Val Phe Asp Val Ala Asp Val Asp Lys His Ala Asp Asp Val Asp
   65                  70                  75                  80
   Val Leu Phe Leu Cys Met Gly Ser Ala Thr Asp Ile Pro Glu Gln Ala
                   85                  90                  95
   Pro Lys Phe Ala Gln Phe Ala Cys Thr Val Asp Thr Tyr Asp Asn His
               100                 105                 110
   Arg Asp Ile Pro Arg His Arg Gln Val Met Asn Glu Ala Ala Thr Ala
           115                 120                 125
   Ala Gly Asn Val Ala Leu Val Ser Thr Gly Trp Asp Pro Gly Met Phe
       130                 135                 140
   Ser Ile Asn Arg Val Tyr Ala Ala Ala Val Leu Ala Glu His Gln Gln
   145                 150                 155                 160
   His Thr Phe Trp Gly Pro Gly Leu Ser Gln Gly His Ser Asp Ala Leu
                   165                 170                 175
   Arg Arg Ile Pro Gly Val Gln Lys Ala Val Gln Tyr Thr Leu Pro Ser
               180                 185                 190
   Glu Asp Ala Leu Glu Lys Ala Arg Arg Gly Glu Ala Gly Asp Leu Thr
           195                 200                 205
   Gly Lys Gln Thr His Lys Arg Gln Cys Phe Val Val Ala Asp Ala Ala
       210                 215                 220
   Asp His Glu Arg Ile Glu Asn Asp Ile Arg Thr Met Pro Asp Tyr Phe
   225                 230                 235                 240
   Val Gly Tyr Glu Val Glu Val Asn Phe Ile Asp Glu Ala Thr Phe Asp
                   245                 250                 255
   Ser Glu His Thr Gly Met Pro His Gly Gly His Val Ile Thr Thr Gly
               260                 265                 270
   Asp Thr Gly Gly Phe Asn His Thr Val Glu Tyr Ile Leu Lys Leu Asp
           275                 280                 285
   Arg Asn Pro Asp Phe Thr Ala Ser Ser Gln Ile Ala Phe Gly Arg Ala
       290                 295                 300
   Ala His Arg Met Lys Gln Gln Gly Gln Ser Gly Ala Phe Thr Val Leu
   305                 310                 315                 320
   Glu Val Ala Pro Tyr Leu Leu Ser Pro Glu Asn Leu Asp Asp Leu Ile
                   325                 330                 335
   Ala Arg Asp Val
               340
<210> 41
   <211> 958
   <212> DNA
   <213> Corynebacterium glutamicum
<220>
   <221> CDS
   <222> (101)..(958)
   <223> FRXA00352
<400> 41
   aatagatcag cgcatccgtg gtggaaccaa aaggctcaac aatacgaaac gttcgctttc 60
   ggtcctgatg aaagagatgt ccctgaatca tcatctaagt atg cat ctc ggt aag   115
                                               Met His Leu Gly Lys
                                               1               5
   ctc gac cag gac agt gcc acc aca att ttg gag gat tac aag aac atg   163
   Leu Asp Gln Asp Ser Ala Thr Thr Ile Leu Glu Asp Tyr Lys Asn Met
                   10                  15                  20
   acc aac atc cgc gta gct atc gtg ggc tac gga aac ctg gga cgc agc   211
   Thr Asn Ile Arg Val Ala Ile Val Gly Tyr Gly Asn Leu Gly Arg Ser
               25                  30                  35
   gtc gaa aag ctt att gcc aag cag ccc gac atg gac ctt gta gga atc   259
   Val Glu Lys Leu Ile Ala Lys Gln Pro Asp Met Asp Leu Val Gly Ile
           40                  45                  50
   ttc tcg cgc cgg gcc acc ctc gac aca aag acg cca gtc ttt gat gtc   307
   Phe Ser Arg Arg Ala Thr Leu Asp Thr Lys Thr Pro Val Phe Asp Val
       55                  60                  65
   gcc gac gtg gac aag cac gcc gac gac gtg gac gtg ctg ttc ctg tgc   355
   Ala Asp Val Asp Lys His Ala Asp Asp Val Asp Val Leu Phe Leu Cys
   70                  75                  80                  85
   atg ggc tcc gcc acc gac atc cct gag cag gca cca aag ttc gcg cag   403
   Met Gly Ser Ala Thr Asp Ile Pro Glu Gln Ala Pro Lys Phe Ala Gln
                   90                  95                  100
   ttc gcc tgc acc gta gac acc tac gac aac cac cgc gac atc cca cgc   451
   Phe Ala Cys Thr Val Asp Thr Tyr Asp Asn His Arg Asp Ile Pro Arg
               105                 110                 115
   cac cgc cag gtc atg aac gaa gcc gcc acc gca gcc ggc aac gtt gca   499
   His Arg Gln Val Met Asn Glu Ala Ala Thr Ala Ala Gly Asn Val Ala
           120                 125                 130
   ctg gtc tct acc ggc tgg gat cca gga atg ttc tcc atc aac cgc gtc   547
   Leu Val Ser Thr Gly Trp Asp Pro Gly Met Phe Ser Ile Asn Arg Val
       135                 140                 145
   tac gca gcg gca gtc tta gcc gag cac cag cag cac acc ttc tgg ggc   595
   Tyr Ala Ala Ala Val Leu Ala Glu His Gln Gln His Thr Phe Trp Gly
   150                 155                 160                 165
   cca ggt ttg tca cag ggc cac tcc gat gct ttg cga cgc atc cct ggc      643
   Pro Gly Leu Ser Gln Gly His Ser Asp Ala Leu Arg Arg Ile Pro Gly
                   170                 175                 180
   gtt caa aag gca gtc cag tac acc ctc cca tcc gaa gac gcc ctg gaa      691
   Val Gln Lys Ala Val Gln Tyr Thr Leu Pro Ser Glu Asp Ala Leu Glu
               185                 190                 195
   aag gcc cgc cgc ggc gaa gcc ggc gac ctt acc gga aag caa acc cac      739
   Lys Ala Arg Arg Gly Glu Ala Gly Asp Leu Thr Gly Lys Gln Thr His
           200                 205                 210
   aag cgc caa tgc ttc gtg gtt gcc gac gcg gcc gat cac gag cgc atc      787
   Lys Arg Gln Cys Phe Val Val Ala Asp Ala Ala Asp His Glu Arg Ile
       215                 220                 225
   gaa aac gac atc cgc acc atg cct gat tac ttc gtt ggc tac gaa gtc      835
   Glu Asn Asp Ile Arg Thr Met Pro Asp Tyr Phe Val Gly Tyr Glu Val
   230                 235                 240                 245
   gaa gtc aac ttc atc gac gaa gca acc ttc gac tcc gag cac acc ggc      883
   Glu Val Asn Phe Ile Asp Glu Ala Thr Phe Asp Ser Glu His Thr Gly
                   250                 255                 260
   atg cca cac ggt ggc cac gtg att acc acc ggc gac acc ggt ggc ttc      931
   Met Pro His Gly Gly His Val Ile Thr Thr Gly Asp Thr Gly Gly Phe
               265                 270                 275
   aac cac acc gtg gaa tac atc ctc aag                                  958
   Asn His Thr Val Glu Tyr Ile Leu Lys
           280                 285
<210> 42
   <211> 286
   <212> PRT
   <213> Corynebacterium glutamicum
<400> 42
   Met His Leu Gly Lys Leu Asp Gln Asp Ser Ala Thr Thr Ile Leu Glu
   1 5 10 15
   Asp Tyr Lys Asn Met Thr Asn Ile Arg Val Ala Ile Val Gly Tyr Gly
   20 25 30
   Asn Leu Gly Arg Ser Val Glu Lys Leu Ile Ala Lys Gln Pro Asp Met
   35 40 45
   Asp Leu Val Gly Ile Phe Ser Arg Arg Ala Thr Leu Asp Thr Lys Thr
   50 55 60
   Pro Val Phe Asp Val Ala Asp Val Asp Lys His Ala Asp Asp Val Asp
   65 70 75 80
   Val Leu Phe Leu Cys Met Gly Ser Ala Thr Asp Ile Pro Glu Gln Ala
   85 90 95
   Pro Lys Phe Ala Gln Phe Ala Cys Thr Val Asp Thr Tyr Asp Asn His
   100 105 110
   Arg Asp Ile Pro Arg His Arg Gln Val Met Asn Glu Ala Ala Thr Ala
   115 120 125
   Ala Gly Asn Val Ala Leu Val Ser Thr Gly Trp Asp Pro Gly Met Phe
   130 135 140
   Ser Ile Asn Arg Val Tyr Ala Ala Ala Val Leu Ala Glu His Gln Gln
   145 150 155 160
   His Thr Phe Trp Gly Pro Gly Leu Ser Gln Gly His Ser Asp Ala Leu
   165 170 175
   Arg Arg Ile Pro Gly Val Gln Lys Ala Val Gln Tyr Thr Leu Pro Ser
   180 185 190
   Glu Asp Ala Leu Glu Lys Ala Arg Arg Gly Glu Ala Gly Asp Leu Thr
   195 200 205
   Gly Lys Gln Thr His Lys Arg Gln Cys Phe Val Val Ala Asp Ala Ala
   210 215 220
   Asp His Glu Arg Ile Glu Asn Asp Ile Arg Thr Met Pro Asp Tyr Phe
   225 230 235 240
   Val Gly Tyr Glu Val Glu Val Asn Phe Ile Asp Glu Ala Thr Phe Asp
   245 250 255
   Ser Glu His Thr Gly Met Pro His Gly Gly His Val Ile Thr Thr Gly
   260 265 270
   Asp Thr Gly Gly Phe Asn His Thr Val Glu Tyr Ile Leu Lys
   275 280 285
<210> 43
   <211> 1400
   <212> DNA
   <213> Corynebacterium glutamicum
<220>
   <221> CDS
   <222> (1)..(1377)
   <223> RXA00972
<400> 43
   cct gca cct ggt tgg cgt ttc cgc acc gga gaa gat gta aca atg gct      48
   Pro Ala Pro Gly Trp Arg Phe Arg Thr Gly Glu Asp Val Thr Met Ala
     1               5                  10                  15
   aca gtt gaa aat ttc aat gaa ctt ccc gca cac gta tgg cca cgc aat      96
   Thr Val Glu Asn Phe Asn Glu Leu Pro Ala His Val Trp Pro Arg Asn
                20                  25                  30
   gcc gtg cgc caa gaa gac ggc gtt gtc acc gtc gct ggt gtg cct ctg     144
   Ala Val Arg Gln Glu Asp Gly Val Val Thr Val Ala Gly Val Pro Leu
            35                  40                  45
   cct gac ctc gct gaa gaa tac gga acc cca ctg ttc gta gtc gac gag     192
   Pro Asp Leu Ala Glu Glu Tyr Gly Thr Pro Leu Phe Val Val Asp Glu
    50                  55                  60
   gac gat ttc cgt tcc cgc tgt cgc gac atg gct acc gca ttc ggt gga     240
   Asp Asp Phe Arg Ser Arg Cys Arg Asp Met Ala Thr Ala Phe Gly Gly
   65                  70                  75                  80
   cca ggc aat gtg cac tac gca tct aaa gcg ttc ctg acc aag acc att     288
   Pro Gly Asn Val His Tyr Ala Ser Lys Ala Phe Leu Thr Lys Thr Ile
                   85                  90                  95
   gca cgt tgg gtt gat gaa gag ggg ctg gca ctg gac att gca tcc atc     336
   Ala Arg Trp Val Asp Glu Glu Gly Leu Ala Leu Asp Ile Ala Ser Ile
               100                 105                 110
   aac gaa ctg ggc att gcc ctg gcc gct ggt ttc ccc gcc agc cgt atc     384
   Asn Glu Leu Gly Ile Ala Leu Ala Ala Gly Phe Pro Ala Ser Arg Ile
           115                 120                 125
   acc gcg cac ggc aac aac aaa ggc gta gag ttc ctg cgc gcg ttg gtt     432
   Thr Ala His Gly Asn Asn Lys Gly Val Glu Phe Leu Arg Ala Leu Val
       130                 135                 140
   caa aac ggt gtg gga cac gtg gtg ctg gac tcc gca cag gaa cta gaa     480
   Gln Asn Gly Val Gly His Val Val Leu Asp Ser Ala Gln Glu Leu Glu
   145                 150                 155                 160
   ctg ttg gat tac gtt gcc gct ggt gaa ggc aag att cag gac gtg ttg     528
   Leu Leu Asp Tyr Val Ala Ala Gly Glu Gly Lys Ile Gln Asp Val Leu
                   165                 170                 175
   atc cgc gta aag cca ggc atc gaa gca cac acc cac gag ttc atc gcc     576
   Ile Arg Val Lys Pro Gly Ile Glu Ala His Thr His Glu Phe Ile Ala
               180                 185                 190
   act agc cac gaa gac cag aag ttc gga ttc tcc ctg gca tcc ggt tcc     624
   Thr Ser His Glu Asp Gln Lys Phe Gly Phe Ser Leu Ala Ser Gly Ser
           195                 200                 205
   gca ttc gaa gca gca aaa gcc gcc aac aac gca gaa aac ctg aac ctg     672
   Ala Phe Glu Ala Ala Lys Ala Ala Asn Asn Ala Glu Asn Leu Asn Leu
       210                 215                 220
   gtt ggc ctg cac tgc cac gtt ggt tcc cag gtg ttc gac gcc gaa ggc     720
   Val Gly Leu His Cys His Val Gly Ser Gln Val Phe Asp Ala Glu Gly
   225                 230                 235                 240
   ttc aag ctg gca gca gaa cgc gtg ttg ggc ctg tac tca cag atc cac     768
   Phe Lys Leu Ala Ala Glu Arg Val Leu Gly Leu Tyr Ser Gln Ile His
                   245                 250                 255
   agc gaa ctg ggc gtt gcc ctt cct gaa ctg gat ctc ggt ggc gga tac     816
   Ser Glu Leu Gly Val Ala Leu Pro Glu Leu Asp Leu Gly Gly Gly Tyr
               260                 265                 270
   ggc att gcc tat acc gca gct gaa gaa cca ctc aac gtc gca gaa gtt     864
   Gly Ile Ala Tyr Thr Ala Ala Glu Glu Pro Leu Asn Val Ala Glu Val
           275                 280                 285
   gcc tcc gac ctg ctc acc gca gtc gga aaa atg gca gcg gaa cta ggc     912
   Ala Ser Asp Leu Leu Thr Ala Val Gly Lys Met Ala Ala Glu Leu Gly
       290                 295                 300
   atc gac gca cca acc gtg ctt gtt gag ccc ggc cgc gct atc gca ggc    960
   Ile Asp Ala Pro Thr Val Leu Val Glu Pro Gly Arg Ala Ile Ala Gly
   305                 310                 315                 320
   ccc tcc acc gtg acc atc tac gaa gtc ggc acc acc aaa gac gtc cac   1008
   Pro Ser Thr Val Thr Ile Tyr Glu Val Gly Thr Thr Lys Asp Val His
                   325                 330                 335
   gta gac gac gac aaa acc cgc cgt tac atc gcc gtg gac gga ggc atg   1056
   Val Asp Asp Asp Lys Thr Arg Arg Tyr Ile Ala Val Asp Gly Gly Met
               340                 345                 350
   tcc gac aac atc cgc cca gca ctc tac ggg tcc gaa tac gac gcc cgc   1104
   Ser Asp Asn Ile Arg Pro Ala Leu Tyr Gly Ser Glu Tyr Asp Ala Arg
           355                 360                 365
   gta gta tcc cgc ttc gcc gaa gga gac cca gta agc acc cgc atc gtg   1152
   Val Val Ser Arg Phe Ala Glu Gly Asp Pro Val Ser Thr Arg Ile Val
       370                 375                 380
   ggc tcc cac tgc gaa tcc ggc gat atc ctg atc aac gat gaa atc tac   1200
   Gly Ser His Cys Glu Ser Gly Asp Ile Leu Ile Asn Asp Glu Ile Tyr
   385                 390                 395                 400
   cca tct gac atc acc agc ggc gac ttc ctt gca ctc gca gcc acc ggc   1248
   Pro Ser Asp Ile Thr Ser Gly Asp Phe Leu Ala Leu Ala Ala Thr Gly
                   405                 410                 415
   gca tac tgc tac gcc atg agc tcc cgc tac aac gcc ttc aca cgg ccc   1296
   Ala Tyr Cys Tyr Ala Met Ser Ser Arg Tyr Asn Ala Phe Thr Arg Pro
               420                 425                 430
   gcc gtc gtg tcc gtc cgc gct ggc agc tcc cgc ctc atg ctg cgc cgc   1344
   Ala Val Val Ser Val Arg Ala Gly Ser Ser Arg Leu Met Leu Arg Arg
           435                 440                 445
   gaa acg ctc gac gac atc ctc tca cta gag gca taacgctttt cgacgcctga 1397
   Glu Thr Leu Asp Asp Ile Leu Ser Leu Glu Ala
       450                 455
   ccc                                                               1400
<210> 44
   <211> 459
   <212> PRT
   <213> Corynebacterium glutamicum
<400> 44
   Pro Ala Pro Gly Trp Arg Phe Arg Thr Gly Glu Asp Val Thr Met Ala
     1               5                  10                  15
   Thr Val Glu Asn Phe Asn Glu Leu Pro Ala His Val Trp Pro Arg Asn
               20                  25                  30
   Ala Val Arg Gln Glu Asp Gly Val Val Thr Val Ala Gly Val Pro Leu
           35                  40                  45
   Pro Asp Leu Ala Glu Glu Tyr Gly Thr Pro Leu Phe Val Val Asp Glu
       50                  55                  60
   Asp Asp Phe Arg Ser Arg Cys Arg Asp Met Ala Thr Ala Phe Gly Gly
    65                  70                  75                  80
   Pro Gly Asn Val His Tyr Ala Ser Lys Ala Phe Leu Thr Lys Thr Ile
                    85                  90                  95
   Ala Arg Trp Val Asp Glu Glu Gly Leu Ala Leu Asp Ile Ala Ser Ile
               100                 105                 110
   Asn Glu Leu Gly Ile Ala Leu Ala Ala Gly Phe Pro Ala Ser Arg Ile
           115                 120                 125
   Thr Ala His Gly Asn Asn Lys Gly Val Glu Phe Leu Arg Ala Leu Val
       130                 135                 140
   Gln Asn Gly Val Gly His Val Val Leu Asp Ser Ala Gln Glu Leu Glu
   145                 150                 155                 160
   Leu Leu Asp Tyr Val Ala Ala Gly Glu Gly Lys Ile Gln Asp Val Leu
                   165                 170                 175
   Ile Arg Val Lys Pro Gly Ile Glu Ala His Thr His Glu Phe Ile Ala
               180                 185                 190
   Thr Ser His Glu Asp Gln Lys Phe Gly Phe Ser Leu Ala Ser Gly Ser
           195                 200                 205
   Ala Phe Glu Ala Ala Lys Ala Ala Asn Asn Ala Glu Asn Leu Asn Leu
       210                 215                 220
   Val Gly Leu His Cys His Val Gly Ser Gln Val Phe Asp Ala Glu Gly
   225                 230                 235                 240
   Phe Lys Leu Ala Ala Glu Arg Val Leu Gly Leu Tyr Ser Gln Ile His
                   245                 250                 255
   Ser Glu Leu Gly Val Ala Leu Pro Glu Leu Asp Leu Gly Gly Gly Tyr
               260                 265                 270
   Gly Ile Ala Tyr Thr Ala Ala Glu Glu Pro Leu Asn Val Ala Glu Val
           275                 280                 285
   Ala Ser Asp Leu Leu Thr Ala Val Gly Lys Met Ala Ala Glu Leu Gly
       290                 295                 300
   Ile Asp Ala Pro Thr Val Leu Val Glu Pro Gly Arg Ala Ile Ala Gly
   305                 310                 315                 320
   Pro Ser Thr Val Thr Ile Tyr Glu Val Gly Thr Thr Lys Asp Val His
                   325                 330                 335
   Val Asp Asp Asp Lys Thr Arg Arg Tyr Ile Ala Val Asp Gly Gly Met
               340                 345                 350
   Ser Asp Asn Ile Arg Pro Ala Leu Tyr Gly Ser Glu Tyr Asp Ala Arg
           355                 360                 365
   Val Val Ser Arg Phe Ala Glu Gly Asp Pro Val Ser Thr Arg Ile Val
       370                 375                 380
   Gly Ser His Cys Glu Ser Gly Asp Ile Leu Ile Asn Asp Glu Ile Tyr
   385                 390                 395                 400
   Pro Ser Asp Ile Thr Ser Gly Asp Phe Leu Ala Leu Ala Ala Thr Gly
                   405                 410                 415
   Ala Tyr Cys Tyr Ala Met Ser Ser Arg Tyr Asn Ala Phe Thr Arg Pro
               420                 425                 430
   Ala Val Val Ser Val Arg Ala Gly Ser Ser Arg Leu Met Leu Arg Arg
           435                 440                 445
   Glu Thr Leu Asp Asp Ile Leu Ser Leu Glu Ala
       450                 455
<210> 45
   <211> 2121
   <212> DNA
   <213> Corynebacterium glutamicum
<220>
   <221> CDS
   <222> (101)..(2098)
   <223> RXA02653
<400> 45
   agacagagtg ttagtgcgtg gggcagctct cactttcatc gacatcactc gagtatgctc 60
   accggccgta ttcattccaa taacccgcac agggaaacta atg ata ccg aag ccc   115
                                             Met Ile Pro Lys Pro
                                             1               5
   gac gtg acc gac tta tat tta gag gac ctc tta aat gag ggt tcg gaa   163
   Asp Val Thr Asp Leu Tyr Leu Glu Asp Leu Leu Asn Glu Gly Ser Glu
                   10                  15                  20
   aag att cgg tcc gcc aag gat ctt tcc gaa ctt agg aca gtt cta aaa   211
   Lys Ile Arg Ser Ala Lys Asp Leu Ser Glu Leu Arg Thr Val Leu Lys
               25                  30                  35
   gag gtt tcc tcc caa att cag gaa cga gct ggg aaa aaa gat gaa gaa   259
   Glu Val Ser Ser Gln Ile Gln Glu Arg Ala Gly Lys Lys Asp Glu Glu
           40                  45                  50
   tgg gga atg ggg gcc act tgg cgg gag ctg tac ccc agc atc gtg gaa   307
   Trp Gly Met Gly Ala Thr Trp Arg Glu Leu Tyr Pro Ser Ile Val Glu
       55                  60                  65
   cgc gct tcc tac gaa ggg cgt gac agc cta atc gga ttt gat cac tta   355
   Arg Ala Ser Tyr Glu Gly Arg Asp Ser Leu Ile Gly Phe Asp His Leu
   70                  75                  80                  85
   gcc cgg gaa atg gaa aga tta gcc ttc ggc cca cca tcc gaa agt ttt   403
   Ala Arg Glu Met Glu Arg Leu Ala Phe Gly Pro Pro Ser Glu Ser Phe
                   90                  95                  100
   gaa tac ctc caa gaa ctc gta aaa tcc gga gtg gta gac atc act cac   451
   Glu Tyr Leu Gln Glu Leu Val Lys Ser Gly Val Val Asp Ile Thr His
               105                 110                 115
   ctg cat cgt ggc cgg gaa cca ctg aca gat tta gtt cgt gaa ctt gaa    499
   Leu His Arg Gly Arg Glu Pro Leu Thr Asp Leu Val Arg Glu Leu Glu
           120                 125                 130
   ata act gtg gtg ata gac gct gtt ctt ccc ccg ccg gga gta gtg cca    547
   Ile Thr Val Val Ile Asp Ala Val Leu Pro Pro Pro Gly Val Val Pro
       135                 140                 145
   ggc aca ttg gtg cac aat ttg gta aaa gag gga tat gcc aga atg cgt    595
   Gly Thr Leu Val His Asn Leu Val Lys Glu Gly Tyr Ala Arg Met Arg
   150                 155                 160                 165
   cct ggg act cgg ggg tta gat gta gcg gct gac ggc acc gtt caa ggg    643
   Pro Gly Thr Arg Gly Leu Asp Val Ala Ala Asp Gly Thr Val Gln Gly
                   170                 175                 180
   caa cga cat ttg gct gca gtc gga cgg atg acg gaa gat gtg gtt ttg    691
   Gln Arg His Leu Ala Ala Val Gly Arg Met Thr Glu Asp Val Val Leu
               185                 190                 195
   ggt aat gac aca ttg tcg cga tca tta cat gac ata atc ccg aag tgg    739
   Gly Asn Asp Thr Leu Ser Arg Ser Leu His Asp Ile Ile Pro Lys Trp
           200                 205                 210
   gct cgt cga gtt atc cgc gac gcg agc acg tat ccc gat agg gta cat    787
   Ala Arg Arg Val Ile Arg Asp Ala Ser Thr Tyr Pro Asp Arg Val His
       215                 220                 225
   ggt act cca ccg ctt ccg gca cgg ttg gaa ccc tgg gcg gaa aag ctc    835
   Gly Thr Pro Pro Leu Pro Ala Arg Leu Glu Pro Trp Ala Glu Lys Leu
   230                 235                 240                 245
   act tca gat ccg gcc aca tgc cgc cac ctg att gaa gaa ttc ggg agt    883
   Thr Ser Asp Pro Ala Thr Cys Arg His Leu Ile Glu Glu Phe Gly Ser
                   250                 255                 260
   cct gtg aat gta ctc cat tca ggt tct atg cct cgt aat ata aat gag    931
   Pro Val Asn Val Leu His Ser Gly Ser Met Pro Arg Asn Ile Asn Glu
               265                 270                 275
   ttg gtt gac gcc ggc att cag atg ggg gtg gat act cga ata ttt ttt    979
   Leu Val Asp Ala Gly Ile Gln Met Gly Val Asp Thr Arg Ile Phe Phe
           280                 285                 290
   gcc cgc aaa gcg aat aag ggt ctt acc ttc gtt gat gcc gtt aaa gac    1027
   Ala Arg Lys Ala Asn Lys Gly Leu Thr Phe Val Asp Ala Val Lys Asp
       295                 300                 305
   acc ggt cat ggt gta gat gta gcc agt gaa cga gag tta tct cag gtg    1075
   Thr Gly His Gly Val Asp Val Ala Ser Glu Arg Glu Leu Ser Gln Val
   310                 315                 320                 325
   ctt aat cgt gga gtc cca gga gag cgg atc att cta tcc gca gct atc    1123
   Leu Asn Arg Gly Val Pro Gly Glu Arg Ile Ile Leu Ser Ala Ala Ile
                   330                 335                 340
   aaa ccg gac aga cta ttg gca tta gcg atc gaa aat ggc gtg atc atc    1171
   Lys Pro Asp Arg Leu Leu Ala Leu Ala Ile Glu Asn Gly Val Ile Ile
               345                 350                 355
   tct gtg gat tcg cgt gat gaa tta gat cgc att tcg gct ttg gtt ggt    1219
   Ser Val Asp Ser Arg Asp Glu Leu Asp Arg Ile Ser Ala Leu Val Gly
           360                 365                 370
   gac cgc gtt gca cga gtt gcg cct aga gta gct cca gat cct gca gtc      1267
   Asp Arg Val Ala Arg Val Ala Pro Arg Val Ala Pro Asp Pro Ala Val
       375                 380                 385
   tta cct cca act aga ttt ggt gag cgt gct gca gac tgg ggt aat cgg      1315
   Leu Pro Pro Thr Arg Phe Gly Glu Arg Ala Ala Asp Trp Gly Asn Arg
   390                 395                 400                 405
   ctt acc gag gtg ata ccc ggc gtg gat att gtg ggt ctt cac gtt cac      1363
   Leu Thr Glu Val Ile Pro Gly Val Asp Ile Val Gly Leu His Val His
                   410                 415                 420
   ctc cat ggc tat gct gca aaa gac cgt gct ctg gct ctg cag gaa tgt      1411
   Leu His Gly Tyr Ala Ala Lys Asp Arg Ala Leu Ala Leu Gln Glu Cys
               425                 430                 435
   tgc caa ctc gtc gat tct ctc aga gaa tgc ggg cat tcc cca cag ttt      1459
   Cys Gln Leu Val Asp Ser Leu Arg Glu Cys Gly His Ser Pro Gln Phe
           440                 445                 450
   att gac ctt gga gga ggg gtg cct atg agc tac att gaa tct gag gaa      1507
   Ile Asp Leu Gly Gly Gly Val Pro Met Ser Tyr Ile Glu Ser Glu Glu
       455                 460                 465
   gat tgg atc cgt tat caa tcc gct aaa tct gcg act tca gcc ggg tat      1555
   Asp Trp Ile Arg Tyr Gln Ser Ala Lys Ser Ala Thr Ser Ala Gly Tyr
   470                 475                 480                 485
   gcc gaa tcc ttt acg tgg aaa gac gat ccg tta tct aat acg tac ccg      1603
   Ala Glu Ser Phe Thr Trp Lys Asp Asp Pro Leu Ser Asn Thr Tyr Pro
                   490                 495                 500
   ttc tat cag acc cca gtg cgc ggt aat tgg ttg aaa gac gtg ctt tct      1651
   Phe Tyr Gln Thr Pro Val Arg Gly Asn Trp Leu Lys Asp Val Leu Ser
               505                 510                 515
   aag ggg gta gct cag atg ctc att gac cgg gga ttg cgg tta cac ata      1699
   Lys Gly Val Ala Gln Met Leu Ile Asp Arg Gly Leu Arg Leu His Ile
           520                 525                 530
   gag cct ggt cga agt tta cta gat ggg tgt ggc gtc act ctt gcc gaa      1747
   Glu Pro Gly Arg Ser Leu Leu Asp Gly Cys Gly Val Thr Leu Ala Glu
       535                 540                 545
   gtt gct ttt gtg aaa acc cga agt gac ggg ttg cct cta gtg gga ctg      1795
   Val Ala Phe Val Lys Thr Arg Ser Asp Gly Leu Pro Leu Val Gly Leu
   550                 555                 560                 565
   gct atg aac cga acg cag tgc cgg act aca tcc gat gat ttt ctc att      1843
   Ala Met Asn Arg Thr Gln Cys Arg Thr Thr Ser Asp Asp Phe Leu Ile
                   570                 575                 580
   gat ccc ctg cat atc act gac ggt gat gta ggc gag gaa atc gaa gca      1891
   Asp Pro Leu His Ile Thr Asp Gly Asp Val Gly Glu Glu Ile Glu Ala
               585                 590                 595
   tat cta gtg ggt gcc tac tgc atc gaa gat gag ctg att tta cgc cgg      1939
   Tyr Leu Val Gly Ala Tyr Cys Ile Glu Asp Glu Leu Ile Leu Arg Arg
            600                 605                 610
   cga atc cgc ttc ccg aga gga gtc aaa cca gga gat atc atc gga att    1987
   Arg Ile Arg Phe Pro Arg Gly Val Lys Pro Gly Asp Ile Ile Gly Ile
       615                 620                 625
   cct aac acc gca gga tac ttc atg cat atc ttg gaa agt gca tcg cac    2035
   Pro Asn Thr Ala Gly Tyr Phe Met His Ile Leu Glu Ser Ala Ser His
   630                 635                 640                 645
   caa atc ccg ttg gcg aaa aat gta gtg tgg ccg gag ggg cag tta gac    2083
   Gln Ile Pro Leu Ala Lys Asn Val Val Trp Pro Glu Gly Gln Leu Asp
                   650                 655                 660
   gat atc gat gcg gat taagacataa ccattcgcta atc                      2121
   Asp Ile Asp Ala Asp
               665
<210> 46
   <211> 666
   <212> PRT
   <213> Corynebacterium glutamicum
<400> 46
   Met Ile Pro Lys Pro Asp Val Thr Asp Leu Tyr Leu Glu Asp Leu Leu
    1               5                  10                  15
   Asn Glu Gly Ser Glu Lys Ile Arg Ser Ala Lys Asp Leu Ser Glu Leu
               20                  25                  30
   Arg Thr Val Leu Lys Glu Val Ser Ser Gln Ile Gln Glu Arg Ala Gly
           35                  40                  45
   Lys Lys Asp Glu Glu Trp Gly Met Gly Ala Thr Trp Arg Glu Leu Tyr
       50                  55                  60
   Pro Ser Ile Val Glu Arg Ala Ser Tyr Glu Gly Arg Asp Ser Leu Ile
   65                  70                  75                  80
   Gly Phe Asp His Leu Ala Arg Glu Met Glu Arg Leu Ala Phe Gly Pro
                   85                  90                  95
   Pro Ser Glu Ser Phe Glu Tyr Leu Gln Glu Leu Val Lys Ser Gly Val
               100                 105                 110
   Val Asp Ile Thr His Leu His Arg Gly Arg Glu Pro Leu Thr Asp Leu
           115                 120                 125
   Val Arg Glu Leu Glu Ile Thr Val Val Ile Asp Ala Val Leu Pro Pro
       130                 135                 140
   Pro Gly Val Val Pro Gly Thr Leu Val His Asn Leu Val Lys Glu Gly
   145                 150                 155                 160
   Tyr Ala Arg Met Arg Pro Gly Thr Arg Gly Leu Asp Val Ala Ala Asp
                   165                 170                 175
   Gly Thr Val Gln Gly Gln Arg His Leu Ala Ala Val Gly Arg Met Thr
               180                 185                 190
   Glu Asp Val Val Leu Gly Asn Asp Thr Leu Ser Arg Ser Leu His Asp
   195 200 205
   Ile Ile Pro Lys Trp Ala Arg Arg Val Ile Arg Asp Ala Ser Thr Tyr
   210 215 220
   Pro Asp Arg Val His Gly Thr Pro Pro Leu Pro Ala Arg Leu Glu Pro
   225 230 235 240
   Trp Ala Glu Lys Leu Thr Ser Asp Pro Ala Thr Cys Arg His Leu Ile
   245 250 255
   Glu Glu Phe Gly Ser Pro Val Asn Val Leu His Ser Gly Ser Met Pro
   260 265 270
   Arg Asn Ile Asn Glu Leu Val Asp Ala Gly Ile Gln Met Gly Val Asp
   275 280 285
   Thr Arg Ile Phe Phe Ala Arg Lys Ala Asn Lys Gly Leu Thr Phe Val
   290 295 300
   Asp Ala Val Lys Asp Thr Gly His Gly Val Asp Val Ala Ser Glu Arg
   305 310 315 320
   Glu Leu Ser Gln Val Leu Asn Arg Gly Val Pro Gly Glu Arg Ile Ile
   325 330 335
   Leu Ser Ala Ala Ile Lys Pro Asp Arg Leu Leu Ala Leu Ala Ile Glu
   340 345 350
   Asn Gly Val Ile Ile Ser Val Asp Ser Arg Asp Glu Leu Asp Arg Ile
   355 360 365
   Ser Ala Leu Val Gly Asp Arg Val Ala Arg Val Ala Pro Arg Val Ala
   370 375 380
   Pro Asp Pro Ala Val Leu Pro Pro Thr Arg Phe Gly Glu Arg Ala Ala
   385 390 395 400
   Asp Trp Gly Asn Arg Leu Thr Glu Val Ile Pro Gly Val Asp Ile Val
   405 410 415
   Gly Leu His Val His Leu His Gly Tyr Ala Ala Lys Asp Arg Ala Leu
   420 425 430
   Ala Leu Gln Glu Cys Cys Gln Leu Val Asp Ser Leu Arg Glu Cys Gly
   435 440 445
   His Ser Pro Gln Phe Ile Asp Leu Gly Gly Gly Val Pro Met Ser Tyr
   450 455 460
   Ile Glu Ser Glu Glu Asp Trp Ile Arg Tyr Gln Ser Ala Lys Ser Ala
   465 470 475 480
   Thr Ser Ala Gly Tyr Ala Glu Ser Phe Thr Trp Lys Asp Asp Pro Leu
   485 490 495
   Ser Asn Thr Tyr Pro Phe Tyr Gln Thr Pro Val Arg Gly Asn Trp Leu
   500 505 510
   Lys Asp Val Leu Ser Lys Gly Val Ala Gln Met Leu Ile Asp Arg Gly
   515 520 525
   Leu Arg Leu His Ile Glu Pro Gly Arg Ser Leu Leu Asp Gly Cys Gly
   530 535 540
   Val Thr Leu Ala Glu Val Ala Phe Val Lys Thr Arg Ser Asp Gly Leu
   545 550 555 560
   Pro Leu Val Gly Leu Ala Met Asn Arg Thr Gln Cys Arg Thr Thr Ser
   565 570 575
   Asp Asp Phe Leu Ile Asp Pro Leu His Ile Thr Asp Gly Asp Val Gly
   580 585 590
   Glu Glu Ile Glu Ala Tyr Leu Val Gly Ala Tyr Cys Ile Glu Asp Glu
   595 600 605
   Leu Ile Leu Arg Arg Arg Ile Arg Phe Pro Arg Gly Val Lys Pro Gly
   610 615 620
   Asp Ile Ile Gly Ile Pro Asn Thr Ala Gly Tyr Phe Met His Ile Leu
   625 630 635 640
   Glu Ser Ala Ser His Gln Ile Pro Leu Ala Lys Asn Val Val Trp Pro
   645 650 655
   Glu Gly Gln Leu Asp Asp Ile Asp Ala Asp
   660 665
<210> 47
   <211> 993
   <212> DNA
   <213> Corynebacterium glutamicum
<220>
   <221> CDS
   <222> (101)..(970)
   <223> RXA01393
<400> 47
   caaaagcaga cctgtaatga agatttccat gatcaccatc gtgacctatg gaagtactta 60
   agtaaaatga ttggttctta acatggttta atatagcttc atg aac ccc att caa   115
                                               Met Asn Pro Ile Gln
                                               1               5
   ctg gac act ttg ctc tca atc att gat gaa ggc agc ttc gaa ggc gcc   163
   Leu Asp Thr Leu Leu Ser Ile Ile Asp Glu Gly Ser Phe Glu Gly Ala
                   10                  15                  20
   tcc tta gcc ctt tcc att tcc ccc tcg gcg gtg agt cag cgc gtt aaa   211
   Ser Leu Ala Leu Ser Ile Ser Pro Ser Ala Val Ser Gln Arg Val Lys
               25                  30                  35
   gct ctc gag cat cac gtg ggt cga gtg ttg gta tcg cgc acc caa ccg   259
   Ala Leu Glu His His Val Gly Arg Val Leu Val Ser Arg Thr Gln Pro
           40                  45                  50
   gcc aaa gca acc gaa gcg ggt gaa gtc ctt gtg caa gca gcg cgg aaa   307
   Ala Lys Ala Thr Glu Ala Gly Glu Val Leu Val Gln Ala Ala Arg Lys
           55                  60                  65
   atg gtg ttg ctg caa gca gaa act aaa gcg caa cta tct gga cgc ctt    355
   Met Val Leu Leu Gln Ala Glu Thr Lys Ala Gln Leu Ser Gly Arg Leu
    70                  75                  80                  85
   gct gaa atc ccg tta acc atc gcc atc aac gca gat tcg cta tcc aca    403
   Ala Glu Ile Pro Leu Thr Ile Ala Ile Asn Ala Asp Ser Leu Ser Thr
                    90                  95                 100
   tgg ttt cct ccc gtg ttc aac gag gta gct tct tgg ggt gga gca acg    451
   Trp Phe Pro Pro Val Phe Asn Glu Val Ala Ser Trp Gly Gly Ala Thr
               105                 110                 115
   ctc acg ctg cgc ttg gaa gat gaa gcg cac aca tta tcc ttg ctg cgg    499
   Leu Thr Leu Arg Leu Glu Asp Glu Ala His Thr Leu Ser Leu Leu Arg
           120                 125                 130
   cgt gga gat gtt tta gga gcg gta acc cgt gaa gct aat ccc gtg gcg    547
   Arg Gly Asp Val Leu Gly Ala Val Thr Arg Glu Ala Asn Pro Val Ala
       135                 140                 145
   gga tgt gaa gta gta gaa ctt gga acc atg cgc cac ttg gcc att gca    595
   Gly Cys Glu Val Val Glu Leu Gly Thr Met Arg His Leu Ala Ile Ala
   150                 155                 160                 165
   acc ccc tca ttg cgg gat gcc tac atg gtt gat ggg aaa cta gat tgg    643
   Thr Pro Ser Leu Arg Asp Ala Tyr Met Val Asp Gly Lys Leu Asp Trp
                   170                 175                 180
   gct gcg atg ccc gtc tta cgc ttc ggt ccc aaa gat gtg ctt caa gac    691
   Ala Ala Met Pro Val Leu Arg Phe Gly Pro Lys Asp Val Leu Gln Asp
               185                 190                 195
   cgt gac ctg gac ggg cgc gtc gat ggt cct gtg ggg cgc agg cgc gta    739
   Arg Asp Leu Asp Gly Arg Val Asp Gly Pro Val Gly Arg Arg Arg Val
           200                 205                 210
   tcc att gtc ccg tcg gcg gaa ggt ttt ggt gag gca att cgc cga ggc    787
   Ser Ile Val Pro Ser Ala Glu Gly Phe Gly Glu Ala Ile Arg Arg Gly
       215                 220                 225
   ctt ggt tgg gga ctt ctt ccc gaa acc caa gct gct ccc atg cta aaa    835
   Leu Gly Trp Gly Leu Leu Pro Glu Thr Gln Ala Ala Pro Met Leu Lys
   230                 235                 240                 245
   gca gga gaa gtg atc ctc ctc gat gag ata ccc att gac aca ccg atg    883
   Ala Gly Glu Val Ile Leu Leu Asp Glu Ile Pro Ile Asp Thr Pro Met
                   250                 255                 260
   tat tgg caa cga tgg cgc ctg gaa tct aga tct cta gct aga ctc aca    931
   Tyr Trp Gln Arg Trp Arg Leu Glu Ser Arg Ser Leu Ala Arg Leu Thr
               265                 270                 275
   gac gcc gtc gtt gat gca gca atc gag gga ttg cgg cct tagttacttc     980
   Asp Ala Val Val Asp Ala Ala Ile Glu Gly Leu Arg Pro
           280                 285                 290
   tgaaaaggtt cag                                                     993
<210> 48
   <211> 290
   <212> PRT
   <213> Corynebacterium glutamicum
<400> 48
   Met Asn Pro Ile Gln Leu Asp Thr Leu Leu Ser Ile Ile Asp Glu Gly
   1               5                   10                  15
   Ser Phe Glu Gly Ala Ser Leu Ala Leu Ser Ile Ser Pro Ser Ala Val
               20                  25                  30
   Ser Gln Arg Val Lys Ala Leu Glu His His Val Gly Arg Val Leu Val
           35                  40                  45
   Ser Arg Thr Gln Pro Ala Lys Ala Thr Glu Ala Gly Glu Val Leu Val
       50                  55                  60
   Gln Ala Ala Arg Lys Met Val Leu Leu Gln Ala Glu Thr Lys Ala Gln
   65                  70                  75                  80
   Leu Ser Gly Arg Leu Ala Glu Ile Pro Leu Thr Ile Ala Ile Asn Ala
                   85                  90                  95
   Asp Ser Leu Ser Thr Trp Phe Pro Pro Val Phe Asn Glu Val Ala Ser
               100                 105                 110
   Trp Gly Gly Ala Thr Leu Thr Leu Arg Leu Glu Asp Glu Ala His Thr
           115                 120                 125
   Leu Ser Leu Leu Arg Arg Gly Asp Val Leu Gly Ala Val Thr Arg Glu
       130                 135                 140
   Ala Asn Pro Val Ala Gly Cys Glu Val Val Glu Leu Gly Thr Met Arg
   145                 150                 155                 160
   His Leu Ala Ile Ala Thr Pro Ser Leu Arg Asp Ala Tyr Met Val Asp
                   165                 170                 175
   Gly Lys Leu Asp Trp Ala Ala Met Pro Val Leu Arg Phe Gly Pro Lys
               180                 185                 190
   Asp Val Leu Gln Asp Arg Asp Leu Asp Gly Arg Val Asp Gly Pro Val
           195                 200                 205
   Gly Arg Arg Arg Val Ser Ile Val Pro Ser Ala Glu Gly Phe Gly Glu
       210                 215                 220
   Ala Ile Arg Arg Gly Leu Gly Trp Gly Leu Leu Pro Glu Thr Gln Ala
   225                 230                 235                 240
   Ala Pro Met Leu Lys Ala Gly Glu Val Ile Leu Leu Asp Glu Ile Pro
                   245                 250                 255
   Ile Asp Thr Pro Met Tyr Trp Gln Arg Trp Arg Leu Glu Ser Arg Ser
               260                 265                 270
   Leu Ala Arg Leu Thr Asp Ala Val Val Asp Ala Ala Ile Glu Gly Leu
           275                 280                 285
   Arg Pro
   290
<210> 49
   <211> 1626
   <212> DNA
   <213> Corynebacterium glutamicum
<220>
   <221> CDS
   <222> (101)..(1603)
   <223> RXA00241
<400> 49
   ggtctccagc ctttctaaac aattcatctg cacttgatta attggcccca agattacgcg 60
   aagtttagcg acttcgccgt acgtcaacta cgttaaatga gtg aat act caa tca   115
                                              Val Asn Thr Gln Ser
                                               1               5
   gat tct gcg ggg tct caa ggt gca gcg gcc aca agt cgt act gta tct   163
   Asp Ser Ala Gly Ser Gln Gly Ala Ala Ala Thr Ser Arg Thr Val Ser
                   10                  15                  20
   att aga acc ctc atc gcg ctg atc atc gga tcg acc gtc ggc gcg gga   211
   Ile Arg Thr Leu Ile Ala Leu Ile Ile Gly Ser Thr Val Gly Ala Gly
               25                  30                  35
   att ttc tcc atc cct caa aac atc ggc tca gtc gca ggt ccc ggc gcg   259
   Ile Phe Ser Ile Pro Gln Asn Ile Gly Ser Val Ala Gly Pro Gly Ala
           40                  45                  50
   atg ctc atc ggc tgg ctg atc gcc ggt gtg ggc atg ttg tcc gta gcg   307
   Met Leu Ile Gly Trp Leu Ile Ala Gly Val Gly Met Leu Ser Val Ala
       55                  60                  65
   ttc gtg ttc cat gtt ctt gcc cgc cgt aaa cct cac ctc gat tct ggc   355
   Phe Val Phe His Val Leu Ala Arg Arg Lys Pro His Leu Asp Ser Gly
   70                  75                  80                  85
   gtc tac gca tat gcg cgt gtt gga ttg ggc gat tat gta ggt ttc tcc   403
   Val Tyr Ala Tyr Ala Arg Val Gly Leu Gly Asp Tyr Val Gly Phe Ser
                   90                  95                  100
   tcc gct tgg ggt tat tgg ctg ggt tca gtc atc gcc caa gtt ggc tac   451
   Ser Ala Trp Gly Tyr Trp Leu Gly Ser Val Ile Ala Gln Val Gly Tyr
               105                 110                 115
   gca acg tta ttt ttc tcc acg ttg ggc cac tac gta ccg ctg ttt tcc   499
   Ala Thr Leu Phe Phe Ser Thr Leu Gly His Tyr Val Pro Leu Phe Ser
           120                 125                 130
   caa gat cat cca ttt gtg tca gcg ttg gca gtt agc gct ttg acc tgg   547
   Gln Asp His Pro Phe Val Ser Ala Leu Ala Val Ser Ala Leu Thr Trp
       135                 140                 145
   ctg gtg ttt gga gtt gtt tcc cga gga att agc caa gct gct ttc ttg   595
   Leu Val Phe Gly Val Val Ser Arg Gly Ile Ser Gln Ala Ala Phe Leu
   150                 155                 160                 165
   aca acg gtc acc acc gtg gcc aaa att ctg cct ctg ttg tgc ttc atc   643
   Thr Thr Val Thr Thr Val Ala Lys Ile Leu Pro Leu Leu Cys Phe Ile
                   170                 175                 180
   atc ctt gtt gca ttc ttg ggc ttt agc tgg gag aag ttc act gtt gat     691
   Ile Leu Val Ala Phe Leu Gly Phe Ser Trp Glu Lys Phe Thr Val Asp
               185                 190                 195
   tta tgg gcg cgt gat ggt ggc gtg ggc agc att ttt gat cag gtg cgc     739
   Leu Trp Ala Arg Asp Gly Gly Val Gly Ser Ile Phe Asp Gln Val Arg
           200                 205                 210
   ggc atc atg gtg tac acc gtg tgg gtg ttc atc ggt atc gaa ggt gca     787
   Gly Ile Met Val Tyr Thr Val Trp Val Phe Ile Gly Ile Glu Gly Ala
       215                 220                 225
   tcg gta tat tcc cgc cag gca cgc tca cgc agt gat gtc agc cga gct     835
   Ser Val Tyr Ser Arg Gln Ala Arg Ser Arg Ser Asp Val Ser Arg Ala
   230                 235                 240                 245
   acc gtg att ggt ttt gtg gct gtt ctc ctt ttg ctg gtg tcg att tct     883
   Thr Val Ile Gly Phe Val Ala Val Leu Leu Leu Leu Val Ser Ile Ser
                   250                 255                 260
   tcg ctg agc ttc ggt gta ctg acc caa caa gag ctc gct gcg tta cca     931
   Ser Leu Ser Phe Gly Val Leu Thr Gln Gln Glu Leu Ala Ala Leu Pro
               265                 270                 275
   gat aat tcc atg gcg tcg gtg ctc gaa gct gtt gtt ggt cca tgg ggt     979
   Asp Asn Ser Met Ala Ser Val Leu Glu Ala Val Val Gly Pro Trp Gly
           280                 285                 290
   gcc gca ttg att tcg ttg ggt ctg tgt ctt tcg gtt ctt ggg gcc tat    1027
   Ala Ala Leu Ile Ser Leu Gly Leu Cys Leu Ser Val Leu Gly Ala Tyr
       295                 300                 305
   gtg tcc tgg cag atg ctc tgc gca gaa cca ctg gcg ttg atg gca atg    1075
   Val Ser Trp Gln Met Leu Cys Ala Glu Pro Leu Ala Leu Met Ala Met
   310                 315                 320                 325
   gat ggc ctc att cca agc aaa atc ggg gcc atc aac agc cgc ggt gct    1123
   Asp Gly Leu Ile Pro Ser Lys Ile Gly Ala Ile Asn Ser Arg Gly Ala
                   330                 335                 340
   gcc tgg atg gct cag ctg atc tcc acc atc gtg att cag att ttc atc    1171
   Ala Trp Met Ala Gln Leu Ile Ser Thr Ile Val Ile Gln Ile Phe Ile
               345                 350                 355
   atc att ttc ttc ctc aac gag acc acc tac gtc tcc atg gtg caa ttg    1219
   Ile Ile Phe Phe Leu Asn Glu Thr Thr Tyr Val Ser Met Val Gln Leu
           360                 365                 370
   gct acc aac cta tac ttg gtg cct tac ctg ttc tct gcc ttt tat ctg    1267
   Ala Thr Asn Leu Tyr Leu Val Pro Tyr Leu Phe Ser Ala Phe Tyr Leu
       375                 380                 385
   gtc atg ctg gca aca cgt gga aaa gga atc acc cac cca cat gcc ggc    1315
   Val Met Leu Ala Thr Arg Gly Lys Gly Ile Thr His Pro His Ala Gly
   390                 395                 400                 405
   aca cgt ttt gat gat tcc ggt cca gag ata tcc cgc cga gaa aac cgc    1363
   Thr Arg Phe Asp Asp Ser Gly Pro Glu Ile Ser Arg Arg Glu Asn Arg
                   410                      415                      420
   aaa cac ctc atc gtc ggt tta gta gca acg gtg tat tca gtg tgg ctg    1411
   Lys His Leu Ile Val Gly Leu Val Ala Thr Val Tyr Ser Val Trp Leu
               425                      430                      435
   ttt tac gct gca gaa ccg cag ttt gtc ctc ttc gga gcc atg gcg atg    1459
   Phe Tyr Ala Ala Glu Pro Gln Phe Val Leu Phe Gly Ala Met Ala Met
           440                      445                      450
   ctt ccc ggc tta atc ccc tat gtg tgg aca agg att tat cgt ggc gaa    1507
   Leu Pro Gly Leu Ile Pro Tyr Val Trp Thr Arg Ile Tyr Arg Gly Glu
       455                      460                      465
   cag gtg ttt aac cgc ttt gaa atc ggc gtg gtt gtt gtc ctg gtc gtt    1555
   Gln Val Phe Asn Arg Phe Glu Ile Gly Val Val Val Val Leu Val Val
   470                      475                      480                      485
   gct gcc agc gcg ggc gtt att ggt ttg gtc aac gga tca cta tcg ctt    1603
   Ala Ala Ser Ala Gly Val Ile Gly Leu Val Asn Gly Ser Leu Ser Leu
                   490                      495                      500
   taaacaccga aaccttcctg cta                                          1626
<210> 50
   <211> 501
   <212> PRT
   <213> Corynebacterium glutamicum
<400> 50
   Val Asn Thr Gln Ser Asp Ser Ala Gly Ser Gln Gly Ala Ala Ala Thr
    1               5                  10                  15
   Ser Arg Thr Val Ser Ile Arg Thr Leu Ile Ala Leu Ile Ile Gly Ser
               20                  25                  30
   Thr Val Gly Ala Gly Ile Phe Ser Ile Pro Gln Asn Ile Gly Ser Val
           35                  40                  45
   Ala Gly Pro Gly Ala Met Leu Ile Gly Trp Leu Ile Ala Gly Val Gly
       50                  55                  60
   Met Leu Ser Val Ala Phe Val Phe His Val Leu Ala Arg Arg Lys Pro
    65                  70                  75                  80
   His Leu Asp Ser Gly Val Tyr Ala Tyr Ala Arg Val Gly Leu Gly Asp
                   85                  90                  95
   Tyr Val Gly Phe Ser Ser Ala Trp Gly Tyr Trp Leu Gly Ser Val Ile
               100                 105                 110
   Ala Gln Val Gly Tyr Ala Thr Leu Phe Phe Ser Thr Leu Gly His Tyr
           115                 120                 125
   Val Pro Leu Phe Ser Gln Asp His Pro Phe Val Ser Ala Leu Ala Val
       130                 135                 140
   Ser Ala Leu Thr Trp Leu Val Phe Gly Val Val Ser Arg Gly Ile Ser
   145                 150                 155                 160
   Gln Ala Ala Phe Leu Thr Thr Val Thr Thr Val Ala Lys Ile Leu Pro
                   165                 170                 175
   Leu Leu Cys Phe Ile Ile Leu Val Ala Phe Leu Gly Phe Ser Trp Glu
               180                 185                 190
   Lys Phe Thr Val Asp Leu Trp Ala Arg Asp Gly Gly Val Gly Ser Ile
           195                 200                 205
   Phe Asp Gln Val Arg Gly Ile Met Val Tyr Thr Val Trp Val Phe Ile
       210                 215                 220
   Gly Ile Glu Gly Ala Ser Val Tyr Ser Arg Gln Ala Arg Ser Arg Ser
   225                 230                 235                 240
   Asp Val Ser Arg Ala Thr Val Ile Gly Phe Val Ala Val Leu Leu Leu
                   245                 250                 255
   Leu Val Ser Ile Ser Ser Leu Ser Phe Gly Val Leu Thr Gln Gln Glu
               260                 265                 270
   Leu Ala Ala Leu Pro Asp Asn Ser Met Ala Ser Val Leu Glu Ala Val
           275                 280                 285
   Val Gly Pro Trp Gly Ala Ala Leu Ile Ser Leu Gly Leu Cys Leu Ser
       290                 295                 300
   Val Leu Gly Ala Tyr Val Ser Trp Gln Met Leu Cys Ala Glu Pro Leu
   305                 310                 315                 320
   Ala Leu Met Ala Met Asp Gly Leu Ile Pro Ser Lys Ile Gly Ala Ile
                   325                 330                 335
   Asn Ser Arg Gly Ala Ala Trp Met Ala Gln Leu Ile Ser Thr Ile Val
               340                 345                 350
   Ile Gln Ile Phe Ile Ile Ile Phe Phe Leu Asn Glu Thr Thr Tyr Val
           355                 360                 365
   Ser Met Val Gln Leu Ala Thr Asn Leu Tyr Leu Val Pro Tyr Leu Phe
       370                 375                 380
   Ser Ala Phe Tyr Leu Val Met Leu Ala Thr Arg Gly Lys Gly Ile Thr
   385                 390                 395                 400
   His Pro His Ala Gly Thr Arg Phe Asp Asp Ser Gly Pro Glu Ile Ser
                   405                 410                 415
   Arg Arg Glu Asn Arg Lys His Leu Ile Val Gly Leu Val Ala Thr Val
               420                 425                 430
   Tyr Ser Val Trp Leu Phe Tyr Ala Ala Glu Pro Gln Phe Val Leu Phe
           435                 440                 445
   Gly Ala Met Ala Met Leu Pro Gly Leu Ile Pro Tyr Val Trp Thr Arg
       450                 455                 460
   Ile Tyr Arg Gly Glu Gln Val Phe Asn Arg Phe Glu Ile Gly Val Val
   465                 470                 475                 480
   Val Val Leu Val Val Ala Ala Ser Ala Gly Val Ile Gly Leu Val Asn
   485 490 495
   Gly Ser Leu Ser Leu
   500
<210> 51
   <211> 822
   <212> DNA
   <213> Corynebacterium glutamicum
<220>
   <221> CDS
   <222> (101)..(799)
   <223> RXA01394
<400> 51
   gagcaaagtg tccagttgaa tggggttcat gaagctatat taaaccatgt taagaaccaa 60
   tcattttact taagtacttc cataggtcac gatggtgatc atg gaa atc ttc att   115
                                               Met Glu Ile Phe Ile
                                                1               5
   aca ggt ctg ctt ttg ggg gcc agt ctt tta ctg tcc atc gga ccg cag   163
   Thr Gly Leu Leu Leu Gly Ala Ser Leu Leu Leu Ser Ile Gly Pro Gln
                   10                  15                  20
   aat gta ctg gtg att aaa caa gga att aag cgc gaa gga ctc att gcg   211
   Asn Val Leu Val Ile Lys Gln Gly Ile Lys Arg Glu Gly Leu Ile Ala
               25                  30                  35
   gtt ctt ctc gtg tgt tta att tct gac gtc ttt ttg ttc atc gcc ggc   259
   Val Leu Leu Val Cys Leu Ile Ser Asp Val Phe Leu Phe Ile Ala Gly
           40                  45                  50
   acc ttg ggc gtt gat ctt ttg tcc aat gcc gcg ccg atc gtg ctc gat   307
   Thr Leu Gly Val Asp Leu Leu Ser Asn Ala Ala Pro Ile Val Leu Asp
       55                  60                  65
   att atg cgc tgg ggt ggc atc gct tac ctg tta tgg ttt gcc gtc atg   355
   Ile Met Arg Trp Gly Gly Ile Ala Tyr Leu Leu Trp Phe Ala Val Met
   70                  75                  80                  85
   gca gcg aaa gac gcc atg aca aac aag gtg gaa gcg cca cag atc att   403
   Ala Ala Lys Asp Ala Met Thr Asn Lys Val Glu Ala Pro Gln Ile Ile
                   90                  95                  100
   gaa gaa aca gaa cca acc gtg ccc gat gac acg cct ttg ggc ggt tcg   451
   Glu Glu Thr Glu Pro Thr Val Pro Asp Asp Thr Pro Leu Gly Gly Ser
               105                 110                 115
   gcg gtg gcc act gac acg cgc aac cgg gtg cgg gtg gag gtg agc gtc   499
   Ala Val Ala Thr Asp Thr Arg Asn Arg Val Arg Val Glu Val Ser Val
           120                 125                 130
   gat aag cag cgg gtt tgg gta aag ccc atg ttg atg gca atc gtg ctg   547
   Asp Lys Gln Arg Val Trp Val Lys Pro Met Leu Met Ala Ile Val Leu
       135                 140                 145
   acc tgg ttg aac ccg aat gcg tat ttg gac gcg ttt gtg ttt atc ggc   595
   Thr Trp Leu Asn Pro Asn Ala Tyr Leu Asp Ala Phe Val Phe Ile Gly
   150                 155                 160                 165
   ggc gtc ggc gcg caa tac ggc gac acc gga cgg tgg att ttc gcc gct     643
   Gly Val Gly Ala Gln Tyr Gly Asp Thr Gly Arg Trp Ile Phe Ala Ala
                   170                 175                 180
   ggc gcg ttc gcg gca agc ctg atc tgg ttc ccg ctg gtg ggt ttc ggc     691
   Gly Ala Phe Ala Ala Ser Leu Ile Trp Phe Pro Leu Val Gly Phe Gly
               185                 190                 195
   gca gca gca ttg tca cgc ccg ctg tcc agc ccc aag gtg tgg cgc tgg     739
   Ala Ala Ala Leu Ser Arg Pro Leu Ser Ser Pro Lys Val Trp Arg Trp
           200                 205                 210
   atc aac gtc gtc gtg gca gtt gtg atg acc gca ttg gcc atc aaa ctg     787
   Ile Asn Val Val Val Ala Val Val Met Thr Ala Leu Ala Ile Lys Leu
       215                 220                 225
   atg ttg atg ggt tagttttcgc gggttttgga atc                           822
   Met Leu Met Gly
   230
<210> 52
   <211> 233
   <212> PRT
   <213> Corynebacterium glutamicum
<400> 52
   Met Glu Ile Phe Ile Thr Gly Leu Leu Leu Gly Ala Ser Leu Leu Leu
   1               5                   10                  15
   Ser Ile Gly Pro Gln Asn Val Leu Val Ile Lys Gln Gly Ile Lys Arg
               20                  25                  30
   Glu Gly Leu Ile Ala Val Leu Leu Val Cys Leu Ile Ser Asp Val Phe
           35                  40                  45
   Leu Phe Ile Ala Gly Thr Leu Gly Val Asp Leu Leu Ser Asn Ala Ala
       50                  55                  60
   Pro Ile Val Leu Asp Ile Met Arg Trp Gly Gly Ile Ala Tyr Leu Leu
   65                  70                  75                  80
   Trp Phe Ala Val Met Ala Ala Lys Asp Ala Met Thr Asn Lys Val Glu
                   85                  90                  95
   Ala Pro Gln Ile Ile Glu Glu Thr Glu Pro Thr Val Pro Asp Asp Thr
               100                 105                 110
   Pro Leu Gly Gly Ser Ala Val Ala Thr Asp Thr Arg Asn Arg Val Arg
           115                 120                 125
   Val Glu Val Ser Val Asp Lys Gln Arg Val Trp Val Lys Pro Met Leu
       130                 135                 140
   Met Ala Ile Val Leu Thr Trp Leu Asn Pro Asn Ala Tyr Leu Asp Ala
   145                 150                 155                 160
   Phe Val Phe Ile Gly Gly Val Gly Ala Gln Tyr Gly Asp Thr Gly Arg
                   165                 170                 175
   Trp Ile Phe Ala Ala Gly Ala Phe Ala Ala Ser Leu Ile Trp Phe Pro
               180                 185                 190
   Leu Val Gly Phe Gly Ala Ala Ala Leu Ser Arg Pro Leu Ser Ser Pro
           195                 200                 205
   Lys Val Trp Arg Trp Ile Asn Val Val Val Ala Val Val Met Thr Ala
       210                 215                 220
   Leu Ala Ile Lys Leu Met Leu Met Gly
   225                 230
<210> 53
   <211> 1026
   <212> DNA
   <213> Corynebacterium glutamicum
<220>
   <221> CDS
   <222> (101)..(1003)
   <223> RXA00865
<400> 53
   ttatcggaat gtggcttggg cgattgttat gcaaaagttg ttaggttttt tgcggggttg 60
   tttaaccccc aaatgaggga agaaggtaac cttgaactct atg agc aca ggt tta   115
                                             Met Ser Thr Gly Leu
                                              1               5
   aca gct aag acc gga gta gag cac ttc ggc acc gtt gga gta gca atg   163
   Thr Ala Lys Thr Gly Val Glu His Phe Gly Thr Val Gly Val Ala Met
                   10                  15                  20
   gtt act cca ttc acg gaa tcc gga gac atc gat atc gct gct ggc cgc   211
   Val Thr Pro Phe Thr Glu Ser Gly Asp Ile Asp Ile Ala Ala Gly Arg
               25                  30                  35
   gaa gtc gcg gct tat ttg gtt gat aag ggc ttg gat tct ttg gtt ctc   259
   Glu Val Ala Ala Tyr Leu Val Asp Lys Gly Leu Asp Ser Leu Val Leu
           40                  45                  50
   gcg ggc acc act ggt gaa tcc cca acg aca acc gcc gct gaa aaa cta   307
   Ala Gly Thr Thr Gly Glu Ser Pro Thr Thr Thr Ala Ala Glu Lys Leu
       55                  60                  65
   gaa ctg ctc aag gcc gtt cgt gag gaa gtt ggg gat cgg gcg aag ctc   355
   Glu Leu Leu Lys Ala Val Arg Glu Glu Val Gly Asp Arg Ala Lys Leu
   70                  75                  80                  85
   atc gcc ggt gtc gga acc aac aac acg cgg aca tct gtg gaa ctt gcg   403
   Ile Ala Gly Val Gly Thr Asn Asn Thr Arg Thr Ser Val Glu Leu Ala
                   90                  95                  100
   gaa gct gct gct tct gct ggc gca gac ggc ctt tta gtt gta act cct   451
   Glu Ala Ala Ala Ser Ala Gly Ala Asp Gly Leu Leu Val Val Thr Pro
               105                 110                 115
   tat tac tcc aag ccg agc caa gag gga ttg ctg gcg cac ttc ggt gca   499
   Tyr Tyr Ser Lys Pro Ser Gln Glu Gly Leu Leu Ala His Phe Gly Ala
           120                 125                 130
   att gct gca gca aca gag gtt cca att tgt ctc tat gac att cct ggt     547
   Ile Ala Ala Ala Thr Glu Val Pro Ile Cys Leu Tyr Asp Ile Pro Gly
       135                 140                 145
   cgg tca ggt att cca att gag tct gat acc atg aga cgc ctg agt gaa     595
   Arg Ser Gly Ile Pro Ile Glu Ser Asp Thr Met Arg Arg Leu Ser Glu
   150                 155                 160                 165
   tta cct acg att ttg gcg gtc aag gac gcc aag ggt gac ctc gtt gca     643
   Leu Pro Thr Ile Leu Ala Val Lys Asp Ala Lys Gly Asp Leu Val Ala
                   170                 175                 180
   gcc acg tca ttg atc aaa gaa acg gga ctt gcc tgg tat tca ggc gat     691
   Ala Thr Ser Leu Ile Lys Glu Thr Gly Leu Ala Trp Tyr Ser Gly Asp
               185                 190                 195
   gac cca cta aac ctt gtt tgg ctt gct ttg ggc gga tca ggt ttc att     739
   Asp Pro Leu Asn Leu Val Trp Leu Ala Leu Gly Gly Ser Gly Phe Ile
           200                 205                 210
   tcc gta att gga cat gca gcc ccc aca gca tta cgt gag ttg tac aca     787
   Ser Val Ile Gly His Ala Ala Pro Thr Ala Leu Arg Glu Leu Tyr Thr
       215                 220                 225
   agc ttc gag gaa ggc gac ctc gtc cgt gcg cgg gaa atc aac gcc aaa     835
   Ser Phe Glu Glu Gly Asp Leu Val Arg Ala Arg Glu Ile Asn Ala Lys
   230                 235                 240                 245
   cta tca ccg ctg gta gct gcc caa ggt cgc ttg ggt gga gtc agc ttg     883
   Leu Ser Pro Leu Val Ala Ala Gln Gly Arg Leu Gly Gly Val Ser Leu
                   250                 255                 260
   gca aaa gct gct ctg cgt ctg cag ggc atc aac gta gga gat cct cga     931
   Ala Lys Ala Ala Leu Arg Leu Gln Gly Ile Asn Val Gly Asp Pro Arg
               265                 270                 275
   ctt cca att atg gct cca aat gag cag gaa ctt gag gct ctc cga gaa     979
   Leu Pro Ile Met Ala Pro Asn Glu Gln Glu Leu Glu Ala Leu Arg Glu
           280                 285                 290
   gac atg aaa aaa gct gga gtt cta taaatatgaa tgattcccga aat          1026
   Asp Met Lys Lys Ala Gly Val Leu
       295                 300
<210> 54
   <211> 301
   <212> PRT
   <213> Corynebacterium glutamicum
<400> 54
   Met Ser Thr Gly Leu Thr Ala Lys Thr Gly Val Glu His Phe Gly Thr
   1               5                   10                  15
   Val Gly Val Ala Met Val Thr Pro Phe Thr Glu Ser Gly Asp Ile Asp
               20                  25                  30
   Ile Ala Ala Gly Arg Glu Val Ala Ala Tyr Leu Val Asp Lys Gly Leu
           35                  40                  45
   Asp Ser Leu Val Leu Ala Gly Thr Thr Gly Glu Ser Pro Thr Thr Thr
       50                  55                  60
   Ala Ala Glu Lys Leu Glu Leu Leu Lys Ala Val Arg Glu Glu Val Gly
   65                  70                  75                  80
   Asp Arg Ala Lys Leu Ile Ala Gly Val Gly Thr Asn Asn Thr Arg Thr
                   85                  90                  95
   Ser Val Glu Leu Ala Glu Ala Ala Ala Ser Ala Gly Ala Asp Gly Leu
               100                 105                 110
   Leu Val Val Thr Pro Tyr Tyr Ser Lys Pro Ser Gln Glu Gly Leu Leu
           115                 120                 125
   Ala His Phe Gly Ala Ile Ala Ala Ala Thr Glu Val Pro Ile Cys Leu
       130                 135                 140
   Tyr Asp Ile Pro Gly Arg Ser Gly Ile Pro Ile Glu Ser Asp Thr Met
   145                 150                 155                 160
   Arg Arg Leu Ser Glu Leu Pro Thr Ile Leu Ala Val Lys Asp Ala Lys
                   165                 170                 175
   Gly Asp Leu Val Ala Ala Thr Ser Leu Ile Lys Glu Thr Gly Leu Ala
               180                 185                 190
   Trp Tyr Ser Gly Asp Asp Pro Leu Asn Leu Val Trp Leu Ala Leu Gly
           195                 200                 205
   Gly Ser Gly Phe Ile Ser Val Ile Gly His Ala Ala Pro Thr Ala Leu
       210                 215                 220
   Arg Glu Leu Tyr Thr Ser Phe Glu Glu Gly Asp Leu Val Arg Ala Arg
   225                 230                 235                 240
   Glu Ile Asn Ala Lys Leu Ser Pro Leu Val Ala Ala Gln Gly Arg Leu
                   245                 250                 255
   Gly Gly Val Ser Leu Ala Lys Ala Ala Leu Arg Leu Gln Gly Ile Asn
               260                 265                 270
   Val Gly Asp Pro Arg Leu Pro Ile Met Ala Pro Asn Glu Gln Glu Leu
           275                 280                 285
   Glu Ala Leu Arg Glu Asp Met Lys Lys Ala Gly Val Leu
       290                 295                 300
<210> 55
   <211> 1071
   <212> DNA
   <213> Corynebacterium glutamicum
<220>
   <221> CDS
   <222> (101)..(1098)
   <223> RXS02021
<400> 55
   ttgggtcgcc gaggagatct aatcctggtt tgagttcaga gttcacaggt ttaagcctac 60
   aaaccttagt taaaacatga tggaagcggt cgattaaaaa atg agt gaa aac att   115
                                               Met Ser Glu Asn Ile
                                               1               5
   cgc gga gcc caa gca gtt gga atc gca aat atc gcc atg gac ggg acc   163
   Arg Gly Ala Gln Ala Val Gly Ile Ala Asn Ile Ala Met Asp Gly Thr
                   10                  15                  20
   atc ctg gac acg tgg tac cca gaa ccc caa att ttc aac ccg gat cag   211
   Ile Leu Asp Thr Trp Tyr Pro Glu Pro Gln Ile Phe Asn Pro Asp Gln
               25                  30                  35
   tgg gct gaa cgc tac cca ttg gaa gtg ggc acc aca cgc ctc gga gca   259
   Trp Ala Glu Arg Tyr Pro Leu Glu Val Gly Thr Thr Arg Leu Gly Ala
           40                  45                  50
   aac gaa ctc acc cca cgg atg ctg cag ttg gta aaa ctg gac caa gat   307
   Asn Glu Leu Thr Pro Arg Met Leu Gln Leu Val Lys Leu Asp Gln Asp
       55                  60                  65
   cgc ctc gtc gaa cag gta gca gtc cgc acc gtt atc ccc gat ctg tct   355
   Arg Leu Val Glu Gln Val Ala Val Arg Thr Val Ile Pro Asp Leu Ser
   70                  75                  80                  85
   caa cct cca gta gac gcg cac gat gtt tac ctg cgc ctc cac ctg ctt   403
   Gln Pro Pro Val Asp Ala His Asp Val Tyr Leu Arg Leu His Leu Leu
                   90                  95                  100
   tcc cac cgg ctg gtc cgc ccc cac gaa atg cac atg caa aac acc ttg   451
   Ser His Arg Leu Val Arg Pro His Glu Met His Met Gln Asn Thr Leu
               105                 110                 115
   gag ctg ctg tcc gac gtg gtg tgg aca aac aag ggc cct tgc ctt cct   499
   Glu Leu Leu Ser Asp Val Val Trp Thr Asn Lys Gly Pro Cys Leu Pro
           120                 125                 130
   gaa aac ttt gag tgg gtg cgt ggt gct ctg cgg tcc cgc gga ctc atc   547
   Glu Asn Phe Glu Trp Val Arg Gly Ala Leu Arg Ser Arg Gly Leu Ile
       135                 140                 145
   cac gtc tac tgt gtg gac cgt ctt ccc cgc atg gtc gac tat gtg gtt   595
   His Val Tyr Cys Val Asp Arg Leu Pro Arg Met Val Asp Tyr Val Val
   150                 155                 160                 165
   ccc cct gga gtc cgc atc tcc gaa gca gaa cgc gtg cgc cta ggt gca   643
   Pro Pro Gly Val Arg Ile Ser Glu Ala Glu Arg Val Arg Leu Gly Ala
                   170                 175                 180
   tac ctt gct ccg ggt acc tct gtg ctg cgt gaa ggt ttc gtg tct ttc   691
   Tyr Leu Ala Pro Gly Thr Ser Val Leu Arg Glu Gly Phe Val Ser Phe
               185                 190                 195
   aac tcc ggc acc ttg ggt gcc gca aag gtg gaa ggc cgc ctg agt tcc   739
   Asn Ser Gly Thr Leu Gly Ala Ala Lys Val Glu Gly Arg Leu Ser Ser
           200                 205                 210
   ggt gtg gtc atc ggt gaa ggt tcc gag att gga ctg tct tct act att   787
   Gly Val Val Ile Gly Glu Gly Ser Glu Ile Gly Leu Ser Ser Thr Ile
       215                 220                 225
   cag tcc ccg aga gat gaa cag cgc cgc cgt ttg ccg ttg agc atc ggc    835
   Gln Ser Pro Arg Asp Glu Gln Arg Arg Arg Leu Pro Leu Ser Ile Gly
   230                 235                 240                 245
   caa aac tgc aac ttt ggt gtc agc tcc gga atc atc gga gtc agt ctg    883
   Gln Asn Cys Asn Phe Gly Val Ser Ser Gly Ile Ile Gly Val Ser Leu
                   250                 255                 260
   gga gac aat tgc gac atc gga aat aac att gtc ttg gat gga gat acc    931
   Gly Asp Asn Cys Asp Ile Gly Asn Asn Ile Val Leu Asp Gly Asp Thr
               265                 270                 275
   ccc att tgg ttc gca gcc gat gag gag tta cgc act atc gac tcc atc    979
   Pro Ile Trp Phe Ala Ala Asp Glu Glu Leu Arg Thr Ile Asp Ser Ile
           280                 285                 290
   gaa ggc caa gca aat tgg tca atc aag cgt gaa tcc ggc ttc cat gag    1027
   Glu Gly Gln Ala Asn Trp Ser Ile Lys Arg Glu Ser Gly Phe His Glu
       295                 300                 305
   cca gtt gcc cgc ctc aaa gct tgacccattt tcataaccag tgc              1071
   Pro Val Ala Arg Leu Lys Ala
   310                 315
<210> 56
   <211> 316
   <212> PRT
   <213> Corynebacterium glutamicum
<400> 56
   Met Ser Glu Asn Ile Arg Gly Ala Gln Ala Val Gly Ile Ala Asn Ile
    1               5                  10                  15
   Ala Met Asp Gly Thr Ile Leu Asp Thr Trp Tyr Pro Glu Pro Gln Ile
               20                  25                  30
   Phe Asn Pro Asp Gln Trp Ala Glu Arg Tyr Pro Leu Glu Val Gly Thr
           35                  40                  45
   Thr Arg Leu Gly Ala Asn Glu Leu Thr Pro Arg Met Leu Gln Leu Val
       50                  55                  60
   Lys Leu Asp Gln Asp Arg Leu Val Glu Gln Val Ala Val Arg Thr Val
   65                  70                  75                  80
   Ile Pro Asp Leu Ser Gln Pro Pro Val Asp Ala His Asp Val Tyr Leu
                   85                  90                  95
   Arg Leu His Leu Leu Ser His Arg Leu Val Arg Pro His Glu Met His
               100                 105                 110
   Met Gln Asn Thr Leu Glu Leu Leu Ser Asp Val Val Trp Thr Asn Lys
           115                 120                 125
   Gly Pro Cys Leu Pro Glu Asn Phe Glu Trp Val Arg Gly Ala Leu Arg
       130                 135                 140
   Ser Arg Gly Leu Ile His Val Tyr Cys Val Asp Arg Leu Pro Arg Met
   145                 150                 155                 160
   Val Asp Tyr Val Val Pro Pro Gly Val Arg Ile Ser Glu Ala Glu Arg
                   165                 170                 175
   Val Arg Leu Gly Ala Tyr Leu Ala Pro Gly Thr Ser Val Leu Arg Glu
               180                 185                 190
   Gly Phe Val Ser Phe Asn Ser Gly Thr Leu Gly Ala Ala Lys Val Glu
           195                 200                 205
   Gly Arg Leu Ser Ser Gly Val Val Ile Gly Glu Gly Ser Glu Ile Gly
       210                 215                 220
   Leu Ser Ser Thr Ile Gln Ser Pro Arg Asp Glu Gln Arg Arg Arg Leu
   225                 230                 235                 240
   Pro Leu Ser Ile Gly Gln Asn Cys Asn Phe Gly Val Ser Ser Gly Ile
                   245                 250                 255
   Ile Gly Val Ser Leu Gly Asp Asn Cys Asp Ile Gly Asn Asn Ile Val
               260                 265                 270
   Leu Asp Gly Asp Thr Pro Ile Trp Phe Ala Ala Asp Glu Glu Leu Arg
           275                 280                 285
   Thr Ile Asp Ser Ile Glu Gly Gln Ala Asn Trp Ser Ile Lys Arg Glu
       290                 295                 300
   Ser Gly Phe His Glu Pro Val Ala Arg Leu Lys Ala
   305                 310                 315
<210> 57
   <211> 1296
   <212> DNA
   <213> Corynebacterium glutamicum
<220>
   <221> CDS
   <222> (101)..(1273)
   <223> RXS02157
<400> 57
   gggtggaatt ggcacgatgg tgctgccgga tgtttttgat cgggagaatt atcctgaagg 60
   caccgttttt agaaaagacg acaaggatgg ggaactgtaa atg agc acg ctg gaa   115
                                               Met Ser Thr Leu Glu
                                               1               5
   act tgg cca cag gtc att att aat acg tac ggc acc cca cca gtt gag   163
   Thr Trp Pro Gln Val Ile Ile Asn Thr Tyr Gly Thr Pro Pro Val Glu
                   10                  15                  20
   ctg gtg tcc ggc aag ggc gca acc gtc act gat gac cag ggc aat gtc   211
   Leu Val Ser Gly Lys Gly Ala Thr Val Thr Asp Asp Gln Gly Asn Val
               25                  30                  35
   tac atc gac ttg ctc gcg ggc atc gca gtc aac gcg ttg ggc cac gcc   259
   Tyr Ile Asp Leu Leu Ala Gly Ile Ala Val Asn Ala Leu Gly His Ala
           40                  45                  50
   cac ccg gcg atc atc gag gcg gtc acc aac cag atc ggc caa ctt ggt    307
   His Pro Ala Ile Ile Glu Ala Val Thr Asn Gln Ile Gly Gln Leu Gly
       55                  60                  65
   cac gtc tca aac ttg ttc gca tcc agg ccc gtc gtc gag gtc gcc gag    355
   His Val Ser Asn Leu Phe Ala Ser Arg Pro Val Val Glu Val Ala Glu
   70                  75                  80                  85
   gag ctc atc aag cgt ttt tcg ctt gac gac gcc acc ctc gcc gcg caa    403
   Glu Leu Ile Lys Arg Phe Ser Leu Asp Asp Ala Thr Leu Ala Ala Gln
                   90                  95                  100
   acc cgg gtt ttc ttc tgc aac tcg ggc gcc gaa gca aac gag gct gct    451
   Thr Arg Val Phe Phe Cys Asn Ser Gly Ala Glu Ala Asn Glu Ala Ala
               105                 110                 115
   ttc aag att gca cgc ttg act ggt cgt tcc cgg att ctg gct gca gtt    499
   Phe Lys Ile Ala Arg Leu Thr Gly Arg Ser Arg Ile Leu Ala Ala Val
           120                 125                 130
   cat ggt ttc cac ggc cgc acc atg ggt tcc ctc gcg ctg act ggc cag    547
   His Gly Phe His Gly Arg Thr Met Gly Ser Leu Ala Leu Thr Gly Gln
       135                 140                 145
   cca gac aag cgt gaa gcg ttc ctg cca atg cca agc ggt gtg gag ttc    595
   Pro Asp Lys Arg Glu Ala Phe Leu Pro Met Pro Ser Gly Val Glu Phe
   150                 155                 160                 165
   tac cct tac ggc gac acc gat tac ttg cgc aaa atg gta gaa acc aac    643
   Tyr Pro Tyr Gly Asp Thr Asp Tyr Leu Arg Lys Met Val Glu Thr Asn
                   170                 175                 180
   cca acg gat gtg gct gct atc ttc ctc gag cca atc cag ggt gaa acg    691
   Pro Thr Asp Val Ala Ala Ile Phe Leu Glu Pro Ile Gln Gly Glu Thr
               185                 190                 195
   ggc gtt gtt cca gca cct gaa gga ttc ctc aag gca gtg cgc gag ctg    739
   Gly Val Val Pro Ala Pro Glu Gly Phe Leu Lys Ala Val Arg Glu Leu
           200                 205                 210
   tgc gat gag tac ggc atc ttg atg atc acc gat gaa gtc cag act ggc    787
   Cys Asp Glu Tyr Gly Ile Leu Met Ile Thr Asp Glu Val Gln Thr Gly
       215                 220                 225
   gtt ggc cgt acc ggc gat ttc ttt gca cat cag cac gat ggc gtt gtt    835
   Val Gly Arg Thr Gly Asp Phe Phe Ala His Gln His Asp Gly Val Val
   230                 235                 240                 245
   ccc gat gtg gtg acc atg gcc aag gga ctt ggc ggc ggt ctt ccc atc    883
   Pro Asp Val Val Thr Met Ala Lys Gly Leu Gly Gly Gly Leu Pro Ile
                   250                 255                 260
   ggt gct tgt ttg gcc act ggc cgt gca gct gaa ttg atg acc cca ggc    931
   Gly Ala Cys Leu Ala Thr Gly Arg Ala Ala Glu Leu Met Thr Pro Gly
               265                 270                 275
   aag cac ggc acc act ttc ggt ggc aac cca gtt gct tgt gca gct gcc    979
   Lys His Gly Thr Thr Phe Gly Gly Asn Pro Val Ala Cys Ala Ala Ala
           280                 285                 290
   aag gca gtg ctg tct gtt gtc gat gac gct ttc tgc gca gaa gtt gcc    1027
   Lys Ala Val Leu Ser Val Val Asp Asp Ala Phe Cys Ala Glu Val Ala
       295                 300                 305
   cgc aag ggc gag ctg ttc aag gaa ctt ctt gcc aag gtt gac ggc gtt      1075
   Arg Lys Gly Glu Leu Phe Lys Glu Leu Leu Ala Lys Val Asp Gly Val
   310                 315                 320                 325
   gta gac gtc cgt ggc agg ggc ttg atg ttg ggc gtg gtg ctg gag cgc      1123
   Val Asp Val Arg Gly Arg Gly Leu Met Leu Gly Val Val Leu Glu Arg
                   330                 335                 340
   gac gtc gca aag caa gct gtt ctt gat ggt ttt aag cac ggc gtt att      1171
   Asp Val Ala Lys Gln Ala Val Leu Asp Gly Phe Lys His Gly Val Ile
               345                 350                 355
   ttg aat gca ccg gcg gac aac att atc cgt ttg acc ccg ccg ctg gtg      1219
   Leu Asn Ala Pro Ala Asp Asn Ile Ile Arg Leu Thr Pro Pro Leu Val
           360                 365                 370
   atc acc gac gaa gaa atc gca gac gca gtc aag gct att gcc gag aca      1267
   Ile Thr Asp Glu Glu Ile Ala Asp Ala Val Lys Ala Ile Ala Glu Thr
       375                 380                 385
   atc gca taaaggactc aaacttatga ctt                                    1296
   Ile Ala
   390
<210> 58
   <211> 391
   <212> PRT
   <213> Corynebacterium glutamicum
<400> 58
   Met Ser Thr Leu Glu Thr Trp Pro Gln Val Ile Ile Asn Thr Tyr Gly
   1 5 10 15
   Thr Pro Pro Val Glu Leu Val Ser Gly Lys Gly Ala Thr Val Thr Asp
   20 25 30
   Asp Gln Gly Asn Val Tyr Ile Asp Leu Leu Ala Gly Ile Ala Val Asn
   35 40 45
   Ala Leu Gly His Ala His Pro Ala Ile Ile Glu Ala Val Thr Asn Gln
   50 55 60
   Ile Gly Gln Leu Gly His Val Ser Asn Leu Phe Ala Ser Arg Pro Val
   65 70 75 80
   Val Glu Val Ala Glu Glu Leu Ile Lys Arg Phe Ser Leu Asp Asp Ala
   85 90 95
   Thr Leu Ala Ala Gln Thr Arg Val Phe Phe Cys Asn Ser Gly Ala Glu
   100 105 110
   Ala Asn Glu Ala Ala Phe Lys Ile Ala Arg Leu Thr Gly Arg Ser Arg
   115 120 125
   Ile Leu Ala Ala Val His Gly Phe His Gly Arg Thr Met Gly Ser Leu
   130 135 140
   Ala Leu Thr Gly Gln Pro Asp Lys Arg Glu Ala Phe Leu Pro Met Pro
   145                 150                 155                 160
   Ser Gly Val Glu Phe Tyr Pro Tyr Gly Asp Thr Asp Tyr Leu Arg Lys
                   165                 170                 175
   Met Val Glu Thr Asn Pro Thr Asp Val Ala Ala Ile Phe Leu Glu Pro
               180                 185                 190
   Ile Gln Gly Glu Thr Gly Val Val Pro Ala Pro Glu Gly Phe Leu Lys
           195                 200                 205
   Ala Val Arg Glu Leu Cys Asp Glu Tyr Gly Ile Leu Met Ile Thr Asp
       210                 215                 220
   Glu Val Gln Thr Gly Val Gly Arg Thr Gly Asp Phe Phe Ala His Gln
   225                 230                 235                 240
   His Asp Gly Val Val Pro Asp Val Val Thr Met Ala Lys Gly Leu Gly
                   245                 250                 255
   Gly Gly Leu Pro Ile Gly Ala Cys Leu Ala Thr Gly Arg Ala Ala Glu
               260                 265                 270
   Leu Met Thr Pro Gly Lys His Gly Thr Thr Phe Gly Gly Asn Pro Val
           275                 280                 285
   Ala Cys Ala Ala Ala Lys Ala Val Leu Ser Val Val Asp Asp Ala Phe
       290                 295                 300
   Cys Ala Glu Val Ala Arg Lys Gly Glu Leu Phe Lys Glu Leu Leu Ala
   305                 310                 315                 320
   Lys Val Asp Gly Val Val Asp Val Arg Gly Arg Gly Leu Met Leu Gly
                   325                 330                 335
   Val Val Leu Glu Arg Asp Val Ala Lys Gln Ala Val Leu Asp Gly Phe
               340                 345                 350
   Lys His Gly Val Ile Leu Asn Ala Pro Ala Asp Asn Ile Ile Arg Leu
           355                 360                 365
   Thr Pro Pro Leu Val Ile Thr Asp Glu Glu Ile Ala Asp Ala Val Lys
       370                 375                 380
   Ala Ile Ala Glu Thr Ile Ala
   385                 390
<210> 59
   <211> 1008
   <212> DNA
   <213> Corynebacterium glutamicum
<220>
   <221> CDS
   <222> (101)..(985)
   <223> RXC00733
<400> 59
   acggcgaggt tgtcggtatt ggaacgcaca cgaatttgct gaacacgtgc ggtacctacc 60
   gtgaaattgt tgaatcccaa gagactgcgc aggcgcaatc atg agt aat act gca     115
                                               Met Ser Asn Thr Ala
                                                 1               5
   ggc ccc cgc ggg cgt tcc cat cag gca gac gcc gcg ccg aat caa aag     163
   Gly Pro Arg Gly Arg Ser His Gln Ala Asp Ala Ala Pro Asn Gln Lys
                    10                  15                  20
   gca cag aat ttc gga cca tct gcc aaa agg ctt ttc gga att cta ggc     211
   Ala Gln Asn Phe Gly Pro Ser Ala Lys Arg Leu Phe Gly Ile Leu Gly
                25                  30                  35
   cat gac cgt aac acc tta att ttt gtt atc ttc cta gcc gtc ctg agc     259
   His Asp Arg Asn Thr Leu Ile Phe Val Ile Phe Leu Ala Val Leu Ser
            40                  45                  50
   gtt gga ctt acc gtc ttg ggc cca tgg ttg ctg ggt aaa gcc acc aac     307
   Val Gly Leu Thr Val Leu Gly Pro Trp Leu Leu Gly Lys Ala Thr Asn
        55                  60                  65
   gtg gtg ttt gaa gga ttc cta tct aag cgc atg ccg gct ggt gcg tca     355
   Val Val Phe Glu Gly Phe Leu Ser Lys Arg Met Pro Ala Gly Ala Ser
    70                  75                  80                  85
   aag gaa gat atc atc gcg cag ttg cag gct gca ggt aaa cat aat cag     403
   Lys Glu Asp Ile Ile Ala Gln Leu Gln Ala Ala Gly Lys His Asn Gln
                    90                  95                 100
   gct tcc atg atg gaa gac atg aac ctt gtt cca ggc tca ggc att gat     451
   Ala Ser Met Met Glu Asp Met Asn Leu Val Pro Gly Ser Gly Ile Asp
               105                 110                 115
   ttt gaa aaa tta gcc atg atc ctc gga ctg gtg atc ggt gct tat ctc     499
   Phe Glu Lys Leu Ala Met Ile Leu Gly Leu Val Ile Gly Ala Tyr Leu
           120                 125                 130
   atc ggt agc ctg ttg tcg ttg ttc cag gcg cgg atg ctc aac cgc atc     547
   Ile Gly Ser Leu Leu Ser Leu Phe Gln Ala Arg Met Leu Asn Arg Ile
       135                 140                 145
   gtg caa agt gcc atg cac cgg ctg cgc atg gag gtg gag gaa aaa atc     595
   Val Gln Ser Ala Met His Arg Leu Arg Met Glu Val Glu Glu Lys Ile
   150                 155                 160                 165
   cac cgc cta ccg ctg agc tat ttc gat tcc atc aaa cgt ggt gat ctg     643
   His Arg Leu Pro Leu Ser Tyr Phe Asp Ser Ile Lys Arg Gly Asp Leu
                   170                 175                 180
   ctt agc cgt gtg acc aac gat gtg gat aat atc ggt caa tcc ctg caa     691
   Leu Ser Arg Val Thr Asn Asp Val Asp Asn Ile Gly Gln Ser Leu Gln
               185                 190                 195
   caa acc ttg tca cag gcg atc act tcc cta ctg acc gtc atc ggt gtg     739
   Gln Thr Leu Ser Gln Ala Ile Thr Ser Leu Leu Thr Val Ile Gly Val
           200                 205                 210
   ttg gtg atg atg ttt atc atc tcc cca ctg ctc gca ctc gtg gcg ctg     787
   Leu Val Met Met Phe Ile Ile Ser Pro Leu Leu Ala Leu Val Ala Leu
       215                 220                 225
   gta tcc att ccg gtc acc atc gtg gtc act gtg gtg gtt gcg agc cgt      835
   Val Ser Ile Pro Val Thr Ile Val Val Thr Val Val Val Ala Ser Arg
   230                 235                 240                 245
   tcc cag aaa ctc ttt gcg gaa cag tgg aag cag acc ggt att ttg aat      883
   Ser Gln Lys Leu Phe Ala Glu Gln Trp Lys Gln Thr Gly Ile Leu Asn
                   250                 255                 260
   gcg cgc ctg gag gaa acc tac tct ggc cac gcc gtg gtt aag gtt ttc      931
   Ala Arg Leu Glu Glu Thr Tyr Ser Gly His Ala Val Val Lys Val Phe
               265                 270                 275
   gga cac caa aag gat gtt caa gaa gca ttc gag gaa gaa aat caa gct      979
   Gly His Gln Lys Asp Val Gln Glu Ala Phe Glu Glu Glu Asn Gln Ala
           280                 285                 290
   tgt gta taaggccagc tttggtgccc agt                                    1008
   Cys Val
       295
<210> 60
   <211> 295
   <212> PRT
   <213> Corynebacterium glutamicum
<400> 60
   Met Ser Asn Thr Ala Gly Pro Arg Gly Arg Ser His Gln Ala Asp Ala
   1               5                   10                  15
   Ala Pro Asn Gln Lys Ala Gln Asn Phe Gly Pro Ser Ala Lys Arg Leu
               20                  25                  30
   Phe Gly Ile Leu Gly His Asp Arg Asn Thr Leu Ile Phe Val Ile Phe
           35                  40                  45
   Leu Ala Val Leu Ser Val Gly Leu Thr Val Leu Gly Pro Trp Leu Leu
       50                  55                  60
   Gly Lys Ala Thr Asn Val Val Phe Glu Gly Phe Leu Ser Lys Arg Met
   65                  70                  75                  80
   Pro Ala Gly Ala Ser Lys Glu Asp Ile Ile Ala Gln Leu Gln Ala Ala
                   85                  90                  95
   Gly Lys His Asn Gln Ala Ser Met Met Glu Asp Met Asn Leu Val Pro
               100                 105                 110
   Gly Ser Gly Ile Asp Phe Glu Lys Leu Ala Met Ile Leu Gly Leu Val
           115                 120                 125
   Ile Gly Ala Tyr Leu Ile Gly Ser Leu Leu Ser Leu Phe Gln Ala Arg
       130                 135                 140
   Met Leu Asn Arg Ile Val Gln Ser Ala Met His Arg Leu Arg Met Glu
   145                 150                 155                 160
   Val Glu Glu Lys Ile His Arg Leu Pro Leu Ser Tyr Phe Asp Ser Ile
                   165                 170                 175
   Lys Arg Gly Asp Leu Leu Ser Arg Val Thr Asn Asp Val Asp Asn Ile
               180                 185                 190
   Gly Gln Ser Leu Gln Gln Thr Leu Ser Gln Ala Ile Thr Ser Leu Leu
           195                 200                 205
   Thr Val Ile Gly Val Leu Val Met Met Phe Ile Ile Ser Pro Leu Leu
       210                 215                 220
   Ala Leu Val Ala Leu Val Ser Ile Pro Val Thr Ile Val Val Thr Val
   225                 230                 235                 240
   Val Val Ala Ser Arg Ser Gln Lys Leu Phe Ala Glu Gln Trp Lys Gln
                   245                 250                 255
   Thr Gly Ile Leu Asn Ala Arg Leu Glu Glu Thr Tyr Ser Gly His Ala
               260                 265                 270
   Val Val Lys Val Phe Gly His Gln Lys Asp Val Gln Glu Ala Phe Glu
           275                 280                 285
   Glu Glu Asn Gln Ala Cys Val
       290                 295
<210> 61
   <211> 426
   <212> DNA
   <213> Corynebacterium glutamicum
<220>
   <221> CDS
   <222> (1)..(426)
   <223> RXC00861
<400> 61
   atg gct cct cac aag gtc atg ctg att acc act ggt act cag ggt gag    48
   Met Ala Pro His Lys Val Met Leu Ile Thr Thr Gly Thr Gln Gly Glu
     1               5                  10                  15
   cct atg gct gcg ctg tct cgc atg gcg cgt cgt gag cac cga cag atc    96
   Pro Met Ala Ala Leu Ser Arg Met Ala Arg Arg Glu His Arg Gln Ile
                20                  25                  30
   act gtc cgt gat gga gac ttg att atc ctt tct tcc tcc ctg gtt cca   144
   Thr Val Arg Asp Gly Asp Leu Ile Ile Leu Ser Ser Ser Leu Val Pro
            35                  40                  45
   ggt aac gaa gaa gca gtg ttc ggt gtc atc aac atg ctg gct cag atc   192
   Gly Asn Glu Glu Ala Val Phe Gly Val Ile Asn Met Leu Ala Gln Ile
        50                  55                  60
   ggt gca act gtt gtt acc ggt cgc gac gcc aag gtg cac acc tcg ggc   240
   Gly Ala Thr Val Val Thr Gly Arg Asp Ala Lys Val His Thr Ser Gly
    65                  70                  75                  80
   cac ggc tac tcc gga gag ctg ttg ttc ttg tac aac gcc gct cgt ccg   288
   His Gly Tyr Ser Gly Glu Leu Leu Phe Leu Tyr Asn Ala Ala Arg Pro
                    85                  90                  95
   aag aac gct atg cct gtc cac ggc gag tgg cgc cac ctg cgc gcc aac   336
   Lys Asn Ala Met Pro Val His Gly Glu Trp Arg His Leu Arg Ala Asn
               100                 105                 110
   aag gaa ctg gct atc tcc act ggt gtt aac cgc gac aac gtt gtg ctt   384
   Lys Glu Leu Ala Ile Ser Thr Gly Val Asn Arg Asp Asn Val Val Leu
           115                 120                 125
   gca caa aac ggt gtt gtg gtt gat atg gtc aac ggt cgc gca           426
   Ala Gln Asn Gly Val Val Val Asp Met Val Asn Gly Arg Ala
       130                 135                 140
<210> 62
   <211> 142
   <212> PRT
   <213> Corynebacterium glutamicum
<400> 62
   Met Ala Pro His Lys Val Met Leu Ile Thr Thr Gly Thr Gln Gly Glu
    1               5                  10                  15
   Pro Met Ala Ala Leu Ser Arg Met Ala Arg Arg Glu His Arg Gln Ile
               20                  25                  30
   Thr Val Arg Asp Gly Asp Leu Ile Ile Leu Ser Ser Ser Leu Val Pro
           35                  40                  45
   Gly Asn Glu Glu Ala Val Phe Gly Val Ile Asn Met Leu Ala Gln Ile
       50                  55                  60
   Gly Ala Thr Val Val Thr Gly Arg Asp Ala Lys Val His Thr Ser Gly
   65                  70                  75                  80
   His Gly Tyr Ser Gly Glu Leu Leu Phe Leu Tyr Asn Ala Ala Arg Pro
                   85                  90                  95
   Lys Asn Ala Met Pro Val His Gly Glu Trp Arg His Leu Arg Ala Asn
               100                 105                 110
   Lys Glu Leu Ala Ile Ser Thr Gly Val Asn Arg Asp Asn Val Val Leu
           115                 120                 125
   Ala Gln Asn Gly Val Val Val Asp Met Val Asn Gly Arg Ala
       130                 135                 140
<210> 63
   <211> 1066
   <212> DNA
   <213> Corynebacterium glutamicum
<220>
   <221> CDS
   <222> (101)..(1066)
   <223> RXC00866
<400> 63
   gcatcaacgt aggagatcct cgacttccaa ttatggctcc aaatgagcag gaacttgagg 60
   ctctccgaga agacatgaaa aaagctggag ttctataaat atg aat gat tcc cga   115
                                              Met Asn Asp Ser Arg
                                               1               5
   aat cgc ggc cgg aag gtt acc cgc aag gcg ggc cca cca gaa gct ggt    163
   Asn Arg Gly Arg Lys Val Thr Arg Lys Ala Gly Pro Pro Glu Ala Gly
                   10                  15                  20
   cag gaa aac cat ctg gat acc cct gtc ttt cag gca cca gat gct tcc    211
   Gln Glu Asn His Leu Asp Thr Pro Val Phe Gln Ala Pro Asp Ala Ser
               25                  30                  35
   tct aac cag agc gct gta aaa gct gag acc gcc gga aac gac aat cgg    259
   Ser Asn Gln Ser Ala Val Lys Ala Glu Thr Ala Gly Asn Asp Asn Arg
           40                  45                  50
   gat gct gcg caa ggt gct caa gga tcc caa gat tct cag ggt tcc cag    307
   Asp Ala Ala Gln Gly Ala Gln Gly Ser Gln Asp Ser Gln Gly Ser Gln
       55                  60                  65
   aac gct caa ggt tcc cag aac cgc gag tcc gga aac aac aac cgc aac    355
   Asn Ala Gln Gly Ser Gln Asn Arg Glu Ser Gly Asn Asn Asn Arg Asn
   70                  75                  80                  85
   cgt tcc aac aac aac cgt cgc ggt ggt cgt gga cgt cgt gga tcc gga    403
   Arg Ser Asn Asn Asn Arg Arg Gly Gly Arg Gly Arg Arg Gly Ser Gly
                   90                  95                  100
   aac gcc aat gag ggc gcg aac aac aac agc ggt aac cag aac cgt cag    451
   Asn Ala Asn Glu Gly Ala Asn Asn Asn Ser Gly Asn Gln Asn Arg Gln
               105                 110                 115
   ggc gga aac cgt ggc aac cgc ggt ggc gga cgc cga aac gtt gtt aag    499
   Gly Gly Asn Arg Gly Asn Arg Gly Gly Gly Arg Arg Asn Val Val Lys
           120                 125                 130
   tcg atg cag ggt gcg gat ctg acc cag cgc ctg cca gag cca cca aag    547
   Ser Met Gln Gly Ala Asp Leu Thr Gln Arg Leu Pro Glu Pro Pro Lys
       135                 140                 145
   gca ccg gca aac ggt ctg cgt att tac gca ctt ggt ggc att tcc gaa    595
   Ala Pro Ala Asn Gly Leu Arg Ile Tyr Ala Leu Gly Gly Ile Ser Glu
   150                 155                 160                 165
   atc ggt cgc aac atg acc gtg ttt gag tac aac aac cgt ctg ctc atc    643
   Ile Gly Arg Asn Met Thr Val Phe Glu Tyr Asn Asn Arg Leu Leu Ile
                   170                 175                 180
   gtg gac tgt ggt gtg ctc ttc cca tct tca ggt gag cca ggc gtt gac    691
   Val Asp Cys Gly Val Leu Phe Pro Ser Ser Gly Glu Pro Gly Val Asp
               185                 190                 195
   ctg att ctt cct gac ttc ggc cca att gag gat cac ctg cac cgc gtc    739
   Leu Ile Leu Pro Asp Phe Gly Pro Ile Glu Asp His Leu His Arg Val
           200                 205                 210
   gat gca ttg gtg gtt act cac gga cac gaa gac cac att ggt gct att    787
   Asp Ala Leu Val Val Thr His Gly His Glu Asp His Ile Gly Ala Ile
       215                 220                 225
   ccc tgg ctg ctg aag ctg cgc aac gat atc cca atc ttg gca tcc cgt    835
   Pro Trp Leu Leu Lys Leu Arg Asn Asp Ile Pro Ile Leu Ala Ser Arg
   230                 235                 240                 245
   ttc acc ttg gct ctg att gca gct aag tgt aag gaa cac cgt cag cgt    883
   Phe Thr Leu Ala Leu Ile Ala Ala Lys Cys Lys Glu His Arg Gln Arg
                   250                 255                 260
   ccg aag ctg atc gag gtc aac gag cag tcc aat gag gac cgc gga ccg    931
   Pro Lys Leu Ile Glu Val Asn Glu Gln Ser Asn Glu Asp Arg Gly Pro
               265                 270                 275
   ttc aac att cgc ttc tgg gct gtt aac cac tcc atc cca gac tgc ctt    979
   Phe Asn Ile Arg Phe Trp Ala Val Asn His Ser Ile Pro Asp Cys Leu
           280                 285                 290
   ggt ctt gct atc aag act cct gct ggt ttg gtc atc cac acc ggt gac    1027
   Gly Leu Ala Ile Lys Thr Pro Ala Gly Leu Val Ile His Thr Gly Asp
       295                 300                 305
   atc aag ctg gat cag act cct cct gat gga cgc cca act                1066
   Ile Lys Leu Asp Gln Thr Pro Pro Asp Gly Arg Pro Thr
   310                 315                 320
<210> 64
   <211> 322
   <212> PRT
   <213> Corynebacterium glutamicum
<400> 64
   Met Asn Asp Ser Arg Asn Arg Gly Arg Lys Val Thr Arg Lys Ala Gly
   1 5 10 15
   Pro Pro Glu Ala Gly Gln Glu Asn His Leu Asp Thr Pro Val Phe Gln
   20 25 30
   Ala Pro Asp Ala Ser Ser Asn Gln Ser Ala Val Lys Ala Glu Thr Ala
   35 40 45
   Gly Asn Asp Asn Arg Asp Ala Ala Gln Gly Ala Gln Gly Ser Gln Asp
   50 55 60
   Ser Gln Gly Ser Gln Asn Ala Gln Gly Ser Gln Asn Arg Glu Ser Gly
   65 70 75 80
   Asn Asn Asn Arg Asn Arg Ser Asn Asn Asn Arg Arg Gly Gly Arg Gly
   85 90 95
   Arg Arg Gly Ser Gly Asn Ala Asn Glu Gly Ala Asn Asn Asn Ser Gly
   100 105 110
   Asn Gln Asn Arg Gln Gly Gly Asn Arg Gly Asn Arg Gly Gly Gly Arg
   115 120 125
   Arg Asn Val Val Lys Ser Met Gln Gly Ala Asp Leu Thr Gln Arg Leu
   130 135 140
   Pro Glu Pro Pro Lys Ala Pro Ala Asn Gly Leu Arg Ile Tyr Ala Leu
   145 150 155 160
   Gly Gly Ile Ser Glu Ile Gly Arg Asn Met Thr Val Phe Glu Tyr Asn
   165 170 175
   Asn Arg Leu Leu Ile Val Asp Cys Gly Val Leu Phe Pro Ser Ser Gly
   180 185 190
   Glu Pro Gly Val Asp Leu Ile Leu Pro Asp Phe Gly Pro Ile Glu Asp
   195 200 205
   His Leu His Arg Val Asp Ala Leu Val Val Thr His Gly His Glu Asp
   210 215 220
   His Ile Gly Ala Ile Pro Trp Leu Leu Lys Leu Arg Asn Asp Ile Pro
   225 230 235 240
   Ile Leu Ala Ser Arg Phe Thr Leu Ala Leu Ile Ala Ala Lys Cys Lys
   245 250 255
   Glu His Arg Gln Arg Pro Lys Leu Ile Glu Val Asn Glu Gln Ser Asn
   260 265 270
   Glu Asp Arg Gly Pro Phe Asn Ile Arg Phe Trp Ala Val Asn His Ser
   275 280 285
   Ile Pro Asp Cys Leu Gly Leu Ala Ile Lys Thr Pro Ala Gly Leu Val
   290 295 300
   Ile His Thr Gly Asp Ile Lys Leu Asp Gln Thr Pro Pro Asp Gly Arg
   305 310 315 320
   Pro Thr
<210> 65
   <211> 1527
   <212> DNA
   <213> Corynebacterium glutamicum
<220>
   <221> CDS
   <222> (101)..(1504)
   <223> RXC02095
<400> 65
   ctctcttggt cctctcccca cccattttta agtactcaag acccttccaa cagaaaggat 60
   tactccccca acaggctcaa aaatactgaa aggctcacgc atg aaa act gag caa   115
                                               Met Lys Thr Glu Gln
                                               1               5
   tcc caa aaa gca caa tta gcc cct aag aaa gca cct gaa aag cca caa   163
   Ser Gln Lys Ala Gln Leu Ala Pro Lys Lys Ala Pro Glu Lys Pro Gln
                   10                  15                  20
   cgc atc cgc caa ctt att tcc gtg gcg tgg cag cga cct tgg ctc acc   211
   Arg Ile Arg Gln Leu Ile Ser Val Ala Trp Gln Arg Pro Trp Leu Thr
               25                  30                  35
   tca ttc acc gta atc agc gct tta gct gca acg ttg ttt gaa ctt aca   259
   Ser Phe Thr Val Ile Ser Ala Leu Ala Ala Thr Leu Phe Glu Leu Thr
           40                  45                  50
   ctt cct ctt ttg acc ggt ggc gcc atc gat atc gcg ctc gga aat acc   307
   Leu Pro Leu Leu Thr Gly Gly Ala Ile Asp Ile Ala Leu Gly Asn Thr
           55                  60                  65
   gga gat act tta acc act gac ctg ctg gac cgg ttc act ccg agt gga    355
   Gly Asp Thr Leu Thr Thr Asp Leu Leu Asp Arg Phe Thr Pro Ser Gly
    70                  75                  80                  85
   tta agc gtg ttg acc agc gtc att gcc ctt atc gtg ctt ctc gcg ttg    403
   Leu Ser Val Leu Thr Ser Val Ile Ala Leu Ile Val Leu Leu Ala Leu
                    90                  95                 100
   ctt cgc tat gcc agt caa ttt gga cgg cga tac acc gca ggc aag ctc    451
   Leu Arg Tyr Ala Ser Gln Phe Gly Arg Arg Tyr Thr Ala Gly Lys Leu
               105                 110                 115
   agc atg ggg gta cag cat gat gtc cgg ctt aaa acg atg cgc tca ttg    499
   Ser Met Gly Val Gln His Asp Val Arg Leu Lys Thr Met Arg Ser Leu
           120                 125                 130
   cag aac ctc gat ggg cca ggt cag gac tct att cgc aca ggc caa gta    547
   Gln Asn Leu Asp Gly Pro Gly Gln Asp Ser Ile Arg Thr Gly Gln Val
       135                 140                 145
   gtc agt cgg tcc att tcg gat atc aac atg gtg caa agc ctt gtg gcg    595
   Val Ser Arg Ser Ile Ser Asp Ile Asn Met Val Gln Ser Leu Val Ala
   150                 155                 160                 165
   atg ttg ccg atg ttg atc gga aat gtg gtc aag ctt gtg ctc act ttg    643
   Met Leu Pro Met Leu Ile Gly Asn Val Val Lys Leu Val Leu Thr Leu
                   170                 175                 180
   gtg atc atg ctg gct att tcc ccg ccg ctg acc atc atc gct gca gtg    691
   Val Ile Met Leu Ala Ile Ser Pro Pro Leu Thr Ile Ile Ala Ala Val
               185                 190                 195
   ttg gtg cct ttg ctg ttg tgg gcc gtg gcc tat tcg cga aaa gcg ctt    739
   Leu Val Pro Leu Leu Leu Trp Ala Val Ala Tyr Ser Arg Lys Ala Leu
           200                 205                 210
   ttt gcg tcc acg tgg tcg gcc cag caa aag gct gcg gat ctg acc act    787
   Phe Ala Ser Thr Trp Ser Ala Gln Gln Lys Ala Ala Asp Leu Thr Thr
       215                 220                 225
   cat gtg gaa gaa act gtc acg ggt atc cgc gtg gtc aag gca ttt gcg    835
   His Val Glu Glu Thr Val Thr Gly Ile Arg Val Val Lys Ala Phe Ala
   230                 235                 240                 245
   cag gaa gac cgc gag acc gac aaa ttg gat ctc acc gca cgt gag tta    883
   Gln Glu Asp Arg Glu Thr Asp Lys Leu Asp Leu Thr Ala Arg Glu Leu
                   250                 255                 260
   ttt gcc cag cgc atg cgc act gca cgt ctg acg gca aag ttc atc ccc    931
   Phe Ala Gln Arg Met Arg Thr Ala Arg Leu Thr Ala Lys Phe Ile Pro
               265                 270                 275
   atg gtt gag cag ctt ccg cag ctt gct ttg gtg gtc aac att gtt ggc    979
   Met Val Glu Gln Leu Pro Gln Leu Ala Leu Val Val Asn Ile Val Gly
           280                 285                 290
   ggt ggc tat ttg gcc atg act ggt cac atc acg gtg ggc acg ttt gtg   1027
   Gly Gly Tyr Leu Ala Met Thr Gly His Ile Thr Val Gly Thr Phe Val
       295                 300                 305
   gcg ttt tct tcc tat ctc act agc ttg tcg gcg gtg gct agg tcc ctg    1075
   Ala Phe Ser Ser Tyr Leu Thr Ser Leu Ser Ala Val Ala Arg Ser Leu
   310                 315                 320                 325
   tcg ggc atg ctc atg cgc gtg cag ttg gcg ctg tct tct gtg gag cgc    1123
   Ser Gly Met Leu Met Arg Val Gln Leu Ala Leu Ser Ser Val Glu Arg
                   330                 335                 340
   atc ttt gaa gtc att gat ctt cag cct gaa cgc acc gat cct gca cac    1171
   Ile Phe Glu Val Ile Asp Leu Gln Pro Glu Arg Thr Asp Pro Ala His
               345                 350                 355
   ccc ctg tca ctt ccc gac act ccc ctg ggt ctg tcg ttc aac aac gta    1219
   Pro Leu Ser Leu Pro Asp Thr Pro Leu Gly Leu Ser Phe Asn Asn Val
           360                 365                 370
   gat ttc cgt ggg att ctc aac ggt ttt gag ctg ggt gtt cag gcc ggt    1267
   Asp Phe Arg Gly Ile Leu Asn Gly Phe Glu Leu Gly Val Gln Ala Gly
       375                 380                 385
   gaa acc gtt gtg ttg gtg ggc cct cca ggt tca ggc aag acc atg gct    1315
   Glu Thr Val Val Leu Val Gly Pro Pro Gly Ser Gly Lys Thr Met Ala
   390                 395                 400                 405
   gtg cag ctt gct gga aac ttt tat caa cca gac agc ggc cac atc gcc    1363
   Val Gln Leu Ala Gly Asn Phe Tyr Gln Pro Asp Ser Gly His Ile Ala
                   410                 415                 420
   ttt gat agc aac ggc cat cgc act cgc ttc gac gac ctc acc cac agc    1411
   Phe Asp Ser Asn Gly His Arg Thr Arg Phe Asp Asp Leu Thr His Ser
               425                 430                 435
   gat atc cgc agg aat ctc atc gcg gtt ttt gat gag ccg ttc ttg tac    1459
   Asp Ile Arg Arg Asn Leu Ile Ala Val Phe Asp Glu Pro Phe Leu Tyr
           440                 445                 450
   tcc tcc tcc ata ccg cga gaa cat ctc gat ggg ttt gga tgt cag        1504
   Ser Ser Ser Ile Pro Arg Glu His Leu Asp Gly Phe Gly Cys Gln
       455                 460                 465
   tgatgagcag atcgaacacg cag                                          1527
<210> 66
   <211> 468
   <212> PRT
   <213> Corynebacterium glutamicum
<400> 66
   Met Lys Thr Glu Gln Ser Gln Lys Ala Gln Leu Ala Pro Lys Lys Ala
     1               5                  10                  15
   Pro Glu Lys Pro Gln Arg Ile Arg Gln Leu Ile Ser Val Ala Trp Gln
               20                  25                  30
   Arg Pro Trp Leu Thr Ser Phe Thr Val Ile Ser Ala Leu Ala Ala Thr
           35                  40                  45
   Leu Phe Glu Leu Thr Leu Pro Leu Leu Thr Gly Gly Ala Ile Asp Ile
       50                  55                  60
   Ala Leu Gly Asn Thr Gly Asp Thr Leu Thr Thr Asp Leu Leu Asp Arg
   65 70 75 80
   Phe Thr Pro Ser Gly Leu Ser Val Leu Thr Ser Val Ile Ala Leu Ile
   85 90 95
   Val Leu Leu Ala Leu Leu Arg Tyr Ala Ser Gln Phe Gly Arg Arg Tyr
   100 105 110
   Thr Ala Gly Lys Leu Ser Met Gly Val Gln His Asp Val Arg Leu Lys
   115 120 125
   Thr Met Arg Ser Leu Gln Asn Leu Asp Gly Pro Gly Gln Asp Ser Ile
   130 135 140
   Arg Thr Gly Gln Val Val Ser Arg Ser Ile Ser Asp Ile Asn Met Val
   145 150 155 160
   Gln Ser Leu Val Ala Met Leu Pro Met Leu Ile Gly Asn Val Val Lys
   165 170 175
   Leu Val Leu Thr Leu Val Ile Met Leu Ala Ile Ser Pro Pro Leu Thr
   180 185 190
   Ile Ile Ala Ala Val Leu Val Pro Leu Leu Leu Trp Ala Val Ala Tyr
   195 200 205
   Ser Arg Lys Ala Leu Phe Ala Ser Thr Trp Ser Ala Gln Gln Lys Ala
   210 215 220
   Ala Asp Leu Thr Thr His Val Glu Glu Thr Val Thr Gly Ile Arg Val
   225 230 235 240
   Val Lys Ala Phe Ala Gln Glu Asp Arg Glu Thr Asp Lys Leu Asp Leu
   245 250 255
   Thr Ala Arg Glu Leu Phe Ala Gln Arg Met Arg Thr Ala Arg Leu Thr
   260 265 270
   Ala Lys Phe Ile Pro Met Val Glu Gln Leu Pro Gln Leu Ala Leu Val
   275 280 285
   Val Asn Ile Val Gly Gly Gly Tyr Leu Ala Met Thr Gly His Ile Thr
   290 295 300
   Val Gly Thr Phe Val Ala Phe Ser Ser Tyr Leu Thr Ser Leu Ser Ala
   305 310 315 320
   Val Ala Arg Ser Leu Ser Gly Met Leu Met Arg Val Gln Leu Ala Leu
   325 330 335
   Ser Ser Val Glu Arg Ile Phe Glu Val Ile Asp Leu Gln Pro Glu Arg
   340 345 350
   Thr Asp Pro Ala His Pro Leu Ser Leu Pro Asp Thr Pro Leu Gly Leu
   355 360 365
   Ser Phe Asn Asn Val Asp Phe Arg Gly Ile Leu Asn Gly Phe Glu Leu
   370 375 380
   Gly Val Gln Ala Gly Glu Thr Val Val Leu Val Gly Pro Pro Gly Ser
   385                 390                 395                 400
   Gly Lys Thr Met Ala Val Gln Leu Ala Gly Asn Phe Tyr Gln Pro Asp
                   405                 410                 415
   Ser Gly His Ile Ala Phe Asp Ser Asn Gly His Arg Thr Arg Phe Asp
               420                 425                 430
   Asp Leu Thr His Ser Asp Ile Arg Arg Asn Leu Ile Ala Val Phe Asp
           435                 440                 445
   Glu Pro Phe Leu Tyr Ser Ser Ser Ile Pro Arg Glu His Leu Asp Gly
       450                 455                 460
   Phe Gly Cys Gln
   465
<210> 67
   <211> 295
   <212> DNA
   <213> Corynebacterium glutamicum
<220>
   <221> CDS
   <222> (34)..(272)
   <223> RXC03185
<400> 67
   agcgcccaac cgttcagacc agcggtttct ctgaggatgc aaagtccatg atgggtnagg 60
   tcactgagct gtccgaaacc acc atg aat gat ctt gca gct gaa ggt gaa aac 113
                             Met Asn Asp Leu Ala Ala Glu Gly Glu Asn
                             1               5                   10
   gat cct tac cgc atg gtt cag cag ctg cgc cgc aag ctc tct cgc ttc   161
   Asp Pro Tyr Arg Met Val Gln Gln Leu Arg Arg Lys Leu Ser Arg Phe
                   15                  20                  25
   gtc gag cag aag tgg aag cgc cag ccg gtc atc atg cca acc gtc att   209
   Val Glu Gln Lys Trp Lys Arg Gln Pro Val Ile Met Pro Thr Val Ile
               30                  35                  40
   ccg atg act gcg gaa acc acg cac atc ggt gac gat gag gtt cgc gct   257
   Pro Met Thr Ala Glu Thr Thr His Ile Gly Asp Asp Glu Val Arg Ala
           45                  50                  55
   tca cgc gag tcc ctg taaaagcatt tcgcttttcg acg                     295
   Ser Arg Glu Ser Leu
       60
<210> 68
   <211> 63
   <212> PRT
   <213> Corynebacterium glutamicum
<400> 68
   Met Asn Asp Leu Ala Ala Glu Gly Glu Asn Asp Pro Tyr Arg Met Val
   1               5                   10                  15
   Gln Gln Leu Arg Arg Lys Leu Ser Arg Phe Val Glu Gln Lys Trp Lys
               20                  25                  30
   Arg Gln Pro Val Ile Met Pro Thr Val Ile Pro Met Thr Ala Glu Thr
           35                  40                  45
   Thr His Ile Gly Asp Asp Glu Val Arg Ala Ser Arg Glu Ser Leu
       50                  55                  60
<210> 69
   <211> 1170
   <212> DNA
   <213> Corynebacterium glutamicum
<220>
   <221> CDS
   <222> (101)..(1147)
   <223> RXA00115
<400> 69
   tggattctcg agtctgtaca cccttgatca aagcccgagt gttccgtaga ttaactttgt 60
   cgtatattgt gacctacacc ccatactgtt aggagttttc atg ctc gac aat agt   115
                                               Met Leu Asp Asn Ser
                                                1               5
   ttt tac acc gca gag gtt cag ggc cca tac gaa acc gct tcc att ggc   163
   Phe Tyr Thr Ala Glu Val Gln Gly Pro Tyr Glu Thr Ala Ser Ile Gly
                   10                  15                  20
   cgg ctc gaa ctc gaa gaa ggg ggt gtg att gag gat tgc tgg ttg gct   211
   Arg Leu Glu Leu Glu Glu Gly Gly Val Ile Glu Asp Cys Trp Leu Ala
               25                  30                  35
   tac gct aca gct gga acg ctc aac gag gac aag tcc aac gcc atc ctc   259
   Tyr Ala Thr Ala Gly Thr Leu Asn Glu Asp Lys Ser Asn Ala Ile Leu
           40                  45                  50
   att ccg acg tgg tac tcc gga acc cat cag acc tgg ttc cag cag tac   307
   Ile Pro Thr Trp Tyr Ser Gly Thr His Gln Thr Trp Phe Gln Gln Tyr
       55                  60                  65
   atc ggc act gat cat gcg ctg gat cca tca aag tat ttc atc atc tcc   355
   Ile Gly Thr Asp His Ala Leu Asp Pro Ser Lys Tyr Phe Ile Ile Ser
   70                  75                  80                  85
   atc aac caa atc ggt aat ggt ttg tcg gtc tcc cct gcc aac acg gct   403
   Ile Asn Gln Ile Gly Asn Gly Leu Ser Val Ser Pro Ala Asn Thr Ala
                   90                  95                  100
   gat gac agc atc tcg atg tcc aag ttc ccg aat gtt cgc att ggt gat   451
   Asp Asp Ser Ile Ser Met Ser Lys Phe Pro Asn Val Arg Ile Gly Asp
               105                 110                 115
   gat gtc gtt gcc cag gac cgg ctc ttg cgc caa gag ttt ggt att acc   499
   Asp Val Val Ala Gln Asp Arg Leu Leu Arg Gln Glu Phe Gly Ile Thr
           120                 125                 130
   gag ctc ttt gcc gtc gtt ggt ggt tcg atg ggt gcg cag caa acc tat   547
   Glu Leu Phe Ala Val Val Gly Gly Ser Met Gly Ala Gln Gln Thr Tyr
       135                 140                 145
   gag tgg att gtt cgc ttc cct gac caa gtt cat cga gca gct ccg atc     595
   Glu Trp Ile Val Arg Phe Pro Asp Gln Val His Arg Ala Ala Pro Ile
   150                 155                 160                 165
   gcg ggc act gcg aag aac act cct cat gat ttc atc ttc acc cag act     643
   Ala Gly Thr Ala Lys Asn Thr Pro His Asp Phe Ile Phe Thr Gln Thr
                   170                 175                 180
   ctt aat gag acc gtt gag gcc gat cca ggg ttc aat ggc ggc gaa tac     691
   Leu Asn Glu Thr Val Glu Ala Asp Pro Gly Phe Asn Gly Gly Glu Tyr
               185                 190                 195
   tcc tcc cat gaa gag gta gct gat gga ctt cgc cgt caa tcg cat ctt     739
   Ser Ser His Glu Glu Val Ala Asp Gly Leu Arg Arg Gln Ser His Leu
           200                 205                 210
   tgg gct gcc atg gga ttt tcc aca gag ttc tgg aag cag gag gca tgg     787
   Trp Ala Ala Met Gly Phe Ser Thr Glu Phe Trp Lys Gln Glu Ala Trp
       215                 220                 225
   cgt cgc ctg gga ctt gaa agt aag gag tca gtg ctc gcg gac ttc ctg     835
   Arg Arg Leu Gly Leu Glu Ser Lys Glu Ser Val Leu Ala Asp Phe Leu
   230                 235                 240                 245
   gat ccg ctg ttc atg tcc atg gat cct aat acc ttg ctc aac aac gct     883
   Asp Pro Leu Phe Met Ser Met Asp Pro Asn Thr Leu Leu Asn Asn Ala
                   250                 255                 260
   tgg aag tgg cag cat ggc gat gtc tct cgc cac acc ggc ggc gac ttg     931
   Trp Lys Trp Gln His Gly Asp Val Ser Arg His Thr Gly Gly Asp Leu
               265                 270                 275
   gca gcg gct ctt ggc cga gtg aag gct aag acc ttc gtt atg ccc atc     979
   Ala Ala Ala Leu Gly Arg Val Lys Ala Lys Thr Phe Val Met Pro Ile
           280                 285                 290
   agc gag gac atg ttc ttt cct gtt cgt gac tgt gcc gca gaa caa gca    1027
   Ser Glu Asp Met Phe Phe Pro Val Arg Asp Cys Ala Ala Glu Gln Ala
       295                 300                 305
   ctc atc cca ggc agc gag ctt cga gtg atc gaa gac atc gcc ggt cac    1075
   Leu Ile Pro Gly Ser Glu Leu Arg Val Ile Glu Asp Ile Ala Gly His
   310                 315                 320                 325
   ctt ggg ctt ttt aac gtc tct gag aat tac atc cca cag atc gac aaa    1123
   Leu Gly Leu Phe Asn Val Ser Glu Asn Tyr Ile Pro Gln Ile Asp Lys
                   330                 335                 340
   aat ctg aaa gag ctg ttc gag agc taaacactga tgtcaaagag cct          1170
   Asn Leu Lys Glu Leu Phe Glu Ser
               345
<210> 70
   <211> 349
   <212> PRT
   <213> Corynebacterium glutamicum
<400> 70
   Met Leu Asp Asn Ser Phe Tyr Thr Ala Glu Val Gln Gly Pro Tyr Glu
   1 5 10 15
   Thr Ala Ser Ile Gly Arg Leu Glu Leu Glu Glu Gly Gly Val Ile Glu
   20 25 30
   Asp Cys Trp Leu Ala Tyr Ala Thr Ala Gly Thr Leu Asn Glu Asp Lys
   35 40 45
   Ser Asn Ala Ile Leu Ile Pro Thr Trp Tyr Ser Gly Thr His Gln Thr
   50 55 60
   Trp Phe Gln Gln Tyr Ile Gly Thr Asp His Ala Leu Asp Pro Ser Lys
   65 70 75 80
   Tyr Phe Ile Ile Ser Ile Asn Gln Ile Gly Asn Gly Leu Ser Val Ser
   85 90 95
   Pro Ala Asn Thr Ala Asp Asp Ser Ile Ser Met Ser Lys Phe Pro Asn
   100 105 110
   Val Arg Ile Gly Asp Asp Val Val Ala Gln Asp Arg Leu Leu Arg Gln
   115 120 125
   Glu Phe Gly Ile Thr Glu Leu Phe Ala Val Val Gly Gly Ser Met Gly
   130 135 140
   Ala Gln Gln Thr Tyr Glu Trp Ile Val Arg Phe Pro Asp Gln Val His
   145 150 155 160
   Arg Ala Ala Pro Ile Ala Gly Thr Ala Lys Asn Thr Pro His Asp Phe
   165 170 175
   Ile Phe Thr Gln Thr Leu Asn Glu Thr Val Glu Ala Asp Pro Gly Phe
   180 185 190
   Asn Gly Gly Glu Tyr Ser Ser His Glu Glu Val Ala Asp Gly Leu Arg
   195 200 205
   Arg Gln Ser His Leu Trp Ala Ala Met Gly Phe Ser Thr Glu Phe Trp
   210 215 220
   Lys Gln Glu Ala Trp Arg Arg Leu Gly Leu Glu Ser Lys Glu Ser Val
   225 230 235 240
   Leu Ala Asp Phe Leu Asp Pro Leu Phe Met Ser Met Asp Pro Asn Thr
   245 250 255
   Leu Leu Asn Asn Ala Trp Lys Trp Gln His Gly Asp Val Ser Arg His
   260 265 270
   Thr Gly Gly Asp Leu Ala Ala Ala Leu Gly Arg Val Lys Ala Lys Thr
   275 280 285
   Phe Val Met Pro Ile Ser Glu Asp Met Phe Phe Pro Val Arg Asp Cys
   290 295 300
   Ala Ala Glu Gln Ala Leu Ile Pro Gly Ser Glu Leu Arg Val Ile Glu
   305 310 315 320
   Asp Ile Ala Gly His Leu Gly Leu Phe Asn Val Ser Glu Asn Tyr Ile
   325 330 335
   Pro Gln Ile Asp Lys Asn Leu Lys Glu Leu Phe Glu Ser
   340 345
<210> 71
   <211> 1254
   <212> DNA
   <213> Corynebacterium glutamicum
<220>
   <221> CDS
   <222> (101)..(1231)
   <223> RXN00403
<400> 71
   tttttcagac tcgtgagaat gcaaactaga ctagacagag ctgtccatat acactggacg 60
   aagttttagt cttgtccacc cagaacaggc ggttattttc atg ccc acc ctc gcg   115
                                               Met Pro Thr Leu Ala
                                               1               5
   cct tca ggt caa ctt gaa atc caa gcg atc ggt gat gtc tcc acc gaa   163
   Pro Ser Gly Gln Leu Glu Ile Gln Ala Ile Gly Asp Val Ser Thr Glu
                   10                  15                  20
   gcc gga gca atc att aca aac gct gaa atc gcc tat cac cgc tgg ggt   211
   Ala Gly Ala Ile Ile Thr Asn Ala Glu Ile Ala Tyr His Arg Trp Gly
               25                  30                  35
   gaa tac cgc gta gat aaa gaa gga cgc agc aat gtc gtt ctc atc gaa   259
   Glu Tyr Arg Val Asp Lys Glu Gly Arg Ser Asn Val Val Leu Ile Glu
           40                  45                  50
   cac gcc ctc act gga gat tcc aac gca gcc gat tgg tgg gct gac ttg   307
   His Ala Leu Thr Gly Asp Ser Asn Ala Ala Asp Trp Trp Ala Asp Leu
       55                  60                  65
   ctc ggt ccc ggc aaa gcc atc aac act gat att tac tgc gtg atc tgt   355
   Leu Gly Pro Gly Lys Ala Ile Asn Thr Asp Ile Tyr Cys Val Ile Cys
   70                  75                  80                  85
   acc aac gtc atc ggt ggt tgc aac ggt tcc acc gga cct ggc tcc atg   403
   Thr Asn Val Ile Gly Gly Cys Asn Gly Ser Thr Gly Pro Gly Ser Met
                   90                  95                  100
   cat cca gat gga aat ttc tgg ggt aat cgc ttc ccc gcc acg tcc att   451
   His Pro Asp Gly Asn Phe Trp Gly Asn Arg Phe Pro Ala Thr Ser Ile
               105                 110                 115
   cgt gat cag gta aac gcc gaa aaa caa ttc ctc gac gca ctc ggc atc   499
   Arg Asp Gln Val Asn Ala Glu Lys Gln Phe Leu Asp Ala Leu Gly Ile
           120                 125                 130
   acc acg gtc gcc gca gta ctt ggt ggt tcc atg ggt ggt gcc cgc acc   547
   Thr Thr Val Ala Ala Val Leu Gly Gly Ser Met Gly Gly Ala Arg Thr
       135                 140                 145
   cta gag tgg gcc gca atg tac cca gaa act gtt ggc gca gct gct gtt   595
   Leu Glu Trp Ala Ala Met Tyr Pro Glu Thr Val Gly Ala Ala Ala Val
   150                 155                 160                 165
   ctt gca gtt tct gca cgc gcc agc gcc tgg caa atc ggc att caa tcc      643
   Leu Ala Val Ser Ala Arg Ala Ser Ala Trp Gln Ile Gly Ile Gln Ser
                   170                 175                 180
   gcc caa att aag gcg att gaa aac gac cac cac tgg cac gaa ggc aac      691
   Ala Gln Ile Lys Ala Ile Glu Asn Asp His His Trp His Glu Gly Asn
               185                 190                 195
   tac tac gaa tcc ggc tgc aac cca gcc acc gga ctc ggc gcc gcc cga      739
   Tyr Tyr Glu Ser Gly Cys Asn Pro Ala Thr Gly Leu Gly Ala Ala Arg
           200                 205                 210
   cgc atc gcc cac ctc acc tac cgt ggc gaa cta gaa atc gac gaa cgc      787
   Arg Ile Ala His Leu Thr Tyr Arg Gly Glu Leu Glu Ile Asp Glu Arg
       215                 220                 225
   ttc ggc acc aaa gcc caa aag aac gaa aac cca ctc ggt ccc tac cgc      835
   Phe Gly Thr Lys Ala Gln Lys Asn Glu Asn Pro Leu Gly Pro Tyr Arg
   230                 235                 240                 245
   aag ccc gac cag cgc ttc gcc gtg gaa tcc tac ttg gac tac caa gca      883
   Lys Pro Asp Gln Arg Phe Ala Val Glu Ser Tyr Leu Asp Tyr Gln Ala
                   250                 255                 260
   gac aag cta gta cag cgt ttc gac gcc ggc tcc tac gtc ttg ctc acc      931
   Asp Lys Leu Val Gln Arg Phe Asp Ala Gly Ser Tyr Val Leu Leu Thr
               265                 270                 275
   gac gcc ctc aac cgc cac gac att ggt cgc gac cgc gga ggc ctc aac      979
   Asp Ala Leu Asn Arg His Asp Ile Gly Arg Asp Arg Gly Gly Leu Asn
           280                 285                 290
   aag gca ctc gaa tcc atc aaa gtt cca gtc ctt gtc gca ggc gta gat     1027
   Lys Ala Leu Glu Ser Ile Lys Val Pro Val Leu Val Ala Gly Val Asp
       295                 300                 305
   acc gat att ttg tac ccc tac cac cag caa gaa cac ctc tcc aga aac     1075
   Thr Asp Ile Leu Tyr Pro Tyr His Gln Gln Glu His Leu Ser Arg Asn
   310                 315                 320                 325
   ctg gga aat cta ctg gca atg gca aaa atc gta tcc cct gtc ggc cac     1123
   Leu Gly Asn Leu Leu Ala Met Ala Lys Ile Val Ser Pro Val Gly His
                   330                 335                 340
   gat gct ttc ctc acc gaa agc cgc caa atg gat cgc atc gtg agg aac     1171
   Asp Ala Phe Leu Thr Glu Ser Arg Gln Met Asp Arg Ile Val Arg Asn
               345                 350                 355
   ttc ttc agc ctc atc tcc cca gac gaa gac aac cct tcg acc tac atc     1219
   Phe Phe Ser Leu Ile Ser Pro Asp Glu Asp Asn Pro Ser Thr Tyr Ile
           360                 365                 370
   gag ttc tac atc taataggtat ttacgacaaa tag                           1254
   Glu Phe Tyr Ile
       375
<210> 72
   <211> 377
   <212> PRT
   <213> Corynebacterium glutamicum
<400> 72
   Met Pro Thr Leu Ala Pro Ser Gly Gln Leu Glu Ile Gln Ala Ile Gly
   1               5                   10                  15
   Asp Val Ser Thr Glu Ala Gly Ala Ile Ile Thr Asn Ala Glu Ile Ala
               20                  25                  30
   Tyr His Arg Trp Gly Glu Tyr Arg Val Asp Lys Glu Gly Arg Ser Asn
           35                  40                  45
   Val Val Leu Ile Glu His Ala Leu Thr Gly Asp Ser Asn Ala Ala Asp
       50                  55                  60
   Trp Trp Ala Asp Leu Leu Gly Pro Gly Lys Ala Ile Asn Thr Asp Ile
   65                  70                  75                  80
   Tyr Cys Val Ile Cys Thr Asn Val Ile Gly Gly Cys Asn Gly Ser Thr
                   85                  90                  95
   Gly Pro Gly Ser Met His Pro Asp Gly Asn Phe Trp Gly Asn Arg Phe
               100                 105                 110
   Pro Ala Thr Ser Ile Arg Asp Gln Val Asn Ala Glu Lys Gln Phe Leu
           115                 120                 125
   Asp Ala Leu Gly Ile Thr Thr Val Ala Ala Val Leu Gly Gly Ser Met
       130                 135                 140
   Gly Gly Ala Arg Thr Leu Glu Trp Ala Ala Met Tyr Pro Glu Thr Val
   145                 150                 155                 160
   Gly Ala Ala Ala Val Leu Ala Val Ser Ala Arg Ala Ser Ala Trp Gln
                   165                 170                 175
   Ile Gly Ile Gln Ser Ala Gln Ile Lys Ala Ile Glu Asn Asp His His
               180                 185                 190
   Trp His Glu Gly Asn Tyr Tyr Glu Ser Gly Cys Asn Pro Ala Thr Gly
           195                 200                 205
   Leu Gly Ala Ala Arg Arg Ile Ala His Leu Thr Tyr Arg Gly Glu Leu
       210                 215                 220
   Glu Ile Asp Glu Arg Phe Gly Thr Lys Ala Gln Lys Asn Glu Asn Pro
   225                 230                 235                 240
   Leu Gly Pro Tyr Arg Lys Pro Asp Gln Arg Phe Ala Val Glu Ser Tyr
                   245                 250                 255
   Leu Asp Tyr Gln Ala Asp Lys Leu Val Gln Arg Phe Asp Ala Gly Ser
               260                 265                 270
   Tyr Val Leu Leu Thr Asp Ala Leu Asn Arg His Asp Ile Gly Arg Asp
           275                 280                 285
   Arg Gly Gly Leu Asn Lys Ala Leu Glu Ser Ile Lys Val Pro Val Leu
       290                 295                 300
   Val Ala Gly Val Asp Thr Asp Ile Leu Tyr Pro Tyr His Gln Gln Glu
   305                 310                 315                 320
   His Leu Ser Arg Asn Leu Gly Asn Leu Leu Ala Met Ala Lys Ile Val
                   325                 330                 335
   Ser Pro Val Gly His Asp Ala Phe Leu Thr Glu Ser Arg Gln Met Asp
               340                 345                 350
   Arg Ile Val Arg Asn Phe Phe Ser Leu Ile Ser Pro Asp Glu Asp Asn
           355                 360                 365
   Pro Ser Thr Tyr Ile Glu Phe Tyr Ile
       370                 375
<210> 73
   <211> 1210
   <212> DNA
   <213> Corynebacterium glutamicum
<220>
   <221> CDS
   <222> (101)..(1210)
   <223> FRXA00403
<400> 73
   tttttcagac tcgtgagaat gcaaactaga ctagacagag ctgtccatat acactggacg 60
   aagttttagt cttgtccacc cagaacaggc ggttattttc atg ccc acc ctc gcg   115
                                               Met Pro Thr Leu Ala
                                               1               5
   cct tca ggt caa ctt gaa atc caa gcg atc ggt gat gtc tcc acc gaa   163
   Pro Ser Gly Gln Leu Glu Ile Gln Ala Ile Gly Asp Val Ser Thr Glu
                   10                  15                  20
   gcc gga gca atc att aca aac gct gaa atc gcc tat cac cgc tgg ggt   211
   Ala Gly Ala Ile Ile Thr Asn Ala Glu Ile Ala Tyr His Arg Trp Gly
               25                  30                  35
   gaa tac cgc gta gat aaa gaa gga cgc agc aat gtc gtt ctc atc gaa   259
   Glu Tyr Arg Val Asp Lys Glu Gly Arg Ser Asn Val Val Leu Ile Glu
           40                  45                  50
   cac gcc ctc act gga gat tcc aac gca gcc gat tgg tgg gct gac ttg   307
   His Ala Leu Thr Gly Asp Ser Asn Ala Ala Asp Trp Trp Ala Asp Leu
       55                  60                  65
   ctc ggt ccc ggc aaa gcc atc aac act gat att tac tgc gtg atc tgt   355
   Leu Gly Pro Gly Lys Ala Ile Asn Thr Asp Ile Tyr Cys Val Ile Cys
   70                  75                  80                  85
   acc aac gtc atc ggt ggt tgc aac ggt tcc acc gga cct ggc tcc atg   403
   Thr Asn Val Ile Gly Gly Cys Asn Gly Ser Thr Gly Pro Gly Ser Met
                   90                  95                  100
   cat cca gat gga aat ttc tgg ggt aat cgc ttc ccc gcc acg tcc att   451
   His Pro Asp Gly Asn Phe Trp Gly Asn Arg Phe Pro Ala Thr Ser Ile
               105                 110                 115
   cgt gat cag gta aac gcc gaa aaa caa ttc ctc gac gca ctc ggc atc   499
   Arg Asp Gln Val Asn Ala Glu Lys Gln Phe Leu Asp Ala Leu Gly Ile
           120                 125                 130
   acc acg gtc gcc gca gta ctt ggt ggt tcc atg ggt ggt gcc cgc acc   547
   Thr Thr Val Ala Ala Val Leu Gly Gly Ser Met Gly Gly Ala Arg Thr
       135                 140                 145
   cta gag tgg gcc gca atg tac cca gaa act gtt ggc gca gct gct gtt   595
   Leu Glu Trp Ala Ala Met Tyr Pro Glu Thr Val Gly Ala Ala Ala Val
   150                 155                 160                 165
   ctt gca gtt tct gca cgc gcc agc gcc tgg caa atc ggc att caa tcc   643
   Leu Ala Val Ser Ala Arg Ala Ser Ala Trp Gln Ile Gly Ile Gln Ser
                   170                 175                 180
   gcc caa att aag gcg att gaa aac gac cac cac tgg cac gaa ggc aac   691
   Ala Gln Ile Lys Ala Ile Glu Asn Asp His His Trp His Glu Gly Asn
               185                 190                 195
   tac tac gaa tcc ggc tgc aac cca gcc acc gga ctc ggc gcc gcc cga   739
   Tyr Tyr Glu Ser Gly Cys Asn Pro Ala Thr Gly Leu Gly Ala Ala Arg
           200                 205                 210
   cgc atc gcc cac ctc acc tac cgt ggc gaa cta gaa atc gac gaa cgc   787
   Arg Ile Ala His Leu Thr Tyr Arg Gly Glu Leu Glu Ile Asp Glu Arg
       215                 220                 225
   ttc ggc acc aaa gcc caa aag aac gaa aac cca ctc ggt ccc tac cgc   835
   Phe Gly Thr Lys Ala Gln Lys Asn Glu Asn Pro Leu Gly Pro Tyr Arg
   230                 235                 240                 245
   aag ccc gac cag cgc ttc gcc gtg gaa tcc tac ttg gac tac caa gca   883
   Lys Pro Asp Gln Arg Phe Ala Val Glu Ser Tyr Leu Asp Tyr Gln Ala
                   250                 255                 260
   gac aag cta gta cag cgt ttc gac gcc ggc tcc tac gtc ttg ctc acc   931
   Asp Lys Leu Val Gln Arg Phe Asp Ala Gly Ser Tyr Val Leu Leu Thr
               265                 270                 275
   gac gcc ctc aac cgc cac gac att ggt cgc gac cgc gga ggc ctc aac   979
   Asp Ala Leu Asn Arg His Asp Ile Gly Arg Asp Arg Gly Gly Leu Asn
           280                 285                 290
   aag gca ctc gaa tcc atc aaa gtt cca gtc ctt gtc gca ggc gta gat   1027
   Lys Ala Leu Glu Ser Ile Lys Val Pro Val Leu Val Ala Gly Val Asp
       295                 300                 305
   acc gat att ttg tac ccc tac cac cag caa gaa cac ctc tcc aga aac   1075
   Thr Asp Ile Leu Tyr Pro Tyr His Gln Gln Glu His Leu Ser Arg Asn
   310                 315                 320                 325
   ctg gga aat cta ctg gca atg gca aaa atc gta tcc cct gtc ggc cac   1123
   Leu Gly Asn Leu Leu Ala Met Ala Lys Ile Val Ser Pro Val Gly His
                   330                 335                 340
   gat gct ttc ctc acc gaa agc cgc caa atg gat cgc atc gtg agg aac   1171
   Asp Ala Phe Leu Thr Glu Ser Arg Gln Met Asp Arg Ile Val Arg Asn
               345                 350                 355
   ttc ttc agc ctc atc tcc cca gac gaa gac aac cct tcg                1210
   Phe Phe Ser Leu Ile Ser Pro Asp Glu Asp Asn Pro Ser
           360                 365                 370
<210> 74
   <211> 370
   <212> PRT
   <213> Corynebacterium glutamicum
<400> 74
   Met Pro Thr Leu Ala Pro Ser Gly Gln Leu Glu Ile Gln Ala Ile Gly
   1               5                   10                  15
   Asp Val Ser Thr Glu Ala Gly Ala Ile Ile Thr Asn Ala Glu Ile Ala
               20                  25                  30
   Tyr His Arg Trp Gly Glu Tyr Arg Val Asp Lys Glu Gly Arg Ser Asn
           35                  40                  45
   Val Val Leu Ile Glu His Ala Leu Thr Gly Asp Ser Asn Ala Ala Asp
       50                  55                  60
   Trp Trp Ala Asp Leu Leu Gly Pro Gly Lys Ala Ile Asn Thr Asp Ile
   65                  70                  75                  80
   Tyr Cys Val Ile Cys Thr Asn Val Ile Gly Gly Cys Asn Gly Ser Thr
                   85                  90                  95
   Gly Pro Gly Ser Met His Pro Asp Gly Asn Phe Trp Gly Asn Arg Phe
               100                 105                 110
   Pro Ala Thr Ser Ile Arg Asp Gln Val Asn Ala Glu Lys Gln Phe Leu
           115                 120                 125
   Asp Ala Leu Gly Ile Thr Thr Val Ala Ala Val Leu Gly Gly Ser Met
       130                 135                 140
   Gly Gly Ala Arg Thr Leu Glu Trp Ala Ala Met Tyr Pro Glu Thr Val
   145                 150                 155                 160
   Gly Ala Ala Ala Val Leu Ala Val Ser Ala Arg Ala Ser Ala Trp Gln
                   165                 170                 175
   Ile Gly Ile Gln Ser Ala Gln Ile Lys Ala Ile Glu Asn Asp His His
               180                 185                 190
   Trp His Glu Gly Asn Tyr Tyr Glu Ser Gly Cys Asn Pro Ala Thr Gly
           195                 200                 205
   Leu Gly Ala Ala Arg Arg Ile Ala His Leu Thr Tyr Arg Gly Glu Leu
       210                 215                 220
   Glu Ile Asp Glu Arg Phe Gly Thr Lys Ala Gln Lys Asn Glu Asn Pro
   225                 230                 235                 240
   Leu Gly Pro Tyr Arg Lys Pro Asp Gln Arg Phe Ala Val Glu Ser Tyr
                   245                 250                 255
   Leu Asp Tyr Gln Ala Asp Lys Leu Val Gln Arg Phe Asp Ala Gly Ser
               260                 265                 270
   Tyr Val Leu Leu Thr Asp Ala Leu Asn Arg His Asp Ile Gly Arg Asp
           275                 280                 285
   Arg Gly Gly Leu Asn Lys Ala Leu Glu Ser Ile Lys Val Pro Val Leu
       290                 295                 300
   Val Ala Gly Val Asp Thr Asp Ile Leu Tyr Pro Tyr His Gln Gln Glu
   305                 310                 315                 320
   His Leu Ser Arg Asn Leu Gly Asn Leu Leu Ala Met Ala Lys Ile Val
                   325                 330                 335
   Ser Pro Val Gly His Asp Ala Phe Leu Thr Glu Ser Arg Gln Met Asp
               340                 345                 350
   Arg Ile Val Arg Asn Phe Phe Ser Leu Ile Ser Pro Asp Glu Asp Asn
           355                 360                 365
   Pro Ser
       370
<210> 75
   <211> 687
   <212> DNA
   <213> Corynebacterium glutamicum
<220>
   <221> CDS
   <222> (101)..(664)
   <223> RXS03158
<400> 75
   caaagctcac cgaaggcacc aacgccaagt tggttgttga caacaccttg gcatccccat 60
   acctgcagca gccactaaaa ctcggcgcac acgcaagtcc ttg cac tcc acc acc   115
                                               Leu His Ser Thr Thr
                                                1               5
   aag tac atc gaa gga cac tcc gac gtt gtt ggc ggc ctt gtg ggt acc   163
   Lys Tyr Ile Glu Gly His Ser Asp Val Val Gly Gly Leu Val Gly Thr
                   10                  15                  20
   aac gac cag gaa atg gac gaa gaa ctg ctg ttc atg cag ggc ggc atc   211
   Asn Asp Gln Glu Met Asp Glu Glu Leu Leu Phe Met Gln Gly Gly Ile
               25                  30                  35
   gga ccg atc cca tca gtt ttc gat gca tac ctg acc gcc cgt ggc ctc   259
   Gly Pro Ile Pro Ser Val Phe Asp Ala Tyr Leu Thr Ala Arg Gly Leu
           40                  45                  50
   aag acc ctt gca gtg cgc atg gat cgc cac tgc gac aac gca gaa aag   307
   Lys Thr Leu Ala Val Arg Met Asp Arg His Cys Asp Asn Ala Glu Lys
       55                  60                  65
   atc gcg gaa ttc ctg gac tcc cgc cca gag gtc tcc acc gtg ctc tac   355
   Ile Ala Glu Phe Leu Asp Ser Arg Pro Glu Val Ser Thr Val Leu Tyr
   70                  75                  80                  85
   cca ggt ctg aag aac cac cca ggc cac gaa gtc gca gcg aag cag atg   403
   Pro Gly Leu Lys Asn His Pro Gly His Glu Val Ala Ala Lys Gln Met
                   90                  95                  100
   aag cgc ttc ggc ggc atg atc tcc gtc cgt ttc gca ggc ggc gaa gaa   451
   Lys Arg Phe Gly Gly Met Ile Ser Val Arg Phe Ala Gly Gly Glu Glu
               105                 110                 115
   gca gct aag aag ttc tgt acc tcc acc aaa ctg atc tgt ctg gcc gag   499
   Ala Ala Lys Lys Phe Cys Thr Ser Thr Lys Leu Ile Cys Leu Ala Glu
           120                 125                 130
   tcc ctc ggt ggc gtg gaa tcc ctc ctg gag cac cca gca acc atg acc   547
   Ser Leu Gly Gly Val Glu Ser Leu Leu Glu His Pro Ala Thr Met Thr
       135                 140                 145
   cac cag tca gct gcc ggc tct cag ctc gag gtt ccc cgc gac ctc gtg   595
   His Gln Ser Ala Ala Gly Ser Gln Leu Glu Val Pro Arg Asp Leu Val
   150                 155                 160                 165
   cgc atc tcc att ggt att gaa gac att gaa gac ctg ctc gca gat gtc   643
   Arg Ile Ser Ile Gly Ile Glu Asp Ile Glu Asp Leu Leu Ala Asp Val
                   170                 175                 180
   gag cag gcc ctc aat aac ctt tagaaactat ttggcggcaa gca             687
   Glu Gln Ala Leu Asn Asn Leu
               185
<210> 76
   <211> 188
   <212> PRT
   <213> Corynebacterium glutamicum
<400> 76
   Leu His Ser Thr Thr Lys Tyr Ile Glu Gly His Ser Asp Val Val Gly
   1               5                   10                  15
   Gly Leu Val Gly Thr Asn Asp Gln Glu Met Asp Glu Glu Leu Leu Phe
               20                  25                  30
   Met Gln Gly Gly Ile Gly Pro Ile Pro Ser Val Phe Asp Ala Tyr Leu
           35                  40                  45
   Thr Ala Arg Gly Leu Lys Thr Leu Ala Val Arg Met Asp Arg His Cys
       50                  55                  60
   Asp Asn Ala Glu Lys Ile Ala Glu Phe Leu Asp Ser Arg Pro Glu Val
   65                  70                  75                  80
   Ser Thr Val Leu Tyr Pro Gly Leu Lys Asn His Pro Gly His Glu Val
                   85                  90                  95
   Ala Ala Lys Gln Met Lys Arg Phe Gly Gly Met Ile Ser Val Arg Phe
               100                 105                 110
   Ala Gly Gly Glu Glu Ala Ala Lys Lys Phe Cys Thr Ser Thr Lys Leu
           115                 120                 125
   Ile Cys Leu Ala Glu Ser Leu Gly Gly Val Glu Ser Leu Leu Glu His
       130                 135                 140
   Pro Ala Thr Met Thr His Gln Ser Ala Ala Gly Ser Gln Leu Glu Val
   145                 150                 155                 160
   Pro Arg Asp Leu Val Arg Ile Ser Ile Gly Ile Glu Asp Ile Glu Asp
                   165                 170                 175
   Leu Leu Ala Asp Val Glu Gln Ala Leu Asn Asn Leu
               180                 185
<210> 77
   <211> 617
   <212> DNA
   <213> Corynebacterium glutamicum
<220>
   <221> CDS
   <222> (1)..(594)
   <223> FRXA00254
<400> 77
   cag cca cta aaa ctc ggc gca cac gca gtc ttg cac tcc acc acc aag    48
   Gln Pro Leu Lys Leu Gly Ala His Ala Val Leu His Ser Thr Thr Lys
    1               5                  10                  15
   tac atc gga gga cac tcc gac gtt gtt ggc ggc ctt gtg gtt acc aac    96
   Tyr Ile Gly Gly His Ser Asp Val Val Gly Gly Leu Val Val Thr Asn
               20                  25                  30
   gac cag gaa atg gac gaa gaa ctg ctg ttc atg cag ggc ggc atc gga   144
   Asp Gln Glu Met Asp Glu Glu Leu Leu Phe Met Gln Gly Gly Ile Gly
           35                  40                  45
   ccg atc cca tca gtt ttc gat gca tac ctg acc gcc cgt ggc ctc aag   192
   Pro Ile Pro Ser Val Phe Asp Ala Tyr Leu Thr Ala Arg Gly Leu Lys
       50                  55                  60
   acc ctt gca gtg cgc atg gat cgc cac tgc gac aac gca gaa aag atc   240
   Thr Leu Ala Val Arg Met Asp Arg His Cys Asp Asn Ala Glu Lys Ile
   65                  70                  75                  80
   gcg gaa ttc ctg gac tcc cgc cca gag gtc tcc acc gtg ctc tac cca   288
   Ala Glu Phe Leu Asp Ser Arg Pro Glu Val Ser Thr Val Leu Tyr Pro
                   85                  90                  95
   ggt ctg aag aac cac cca ggc cac gaa gtc gca gcg aag cag atg aag   336
   Gly Leu Lys Asn His Pro Gly His Glu Val Ala Ala Lys Gln Met Lys
               100                 105                 110
   cgc ttc ggc ggc atg atc tcc gtc cgt ttc gca ggc ggc gaa gaa gca   384
   Arg Phe Gly Gly Met Ile Ser Val Arg Phe Ala Gly Gly Glu Glu Ala
           115                 120                 125
   gct aag aag ttc tgt acc tcc acc aaa ctg atc tgt ctg gcc gag tcc   432
   Ala Lys Lys Phe Cys Thr Ser Thr Lys Leu Ile Cys Leu Ala Glu Ser
       130                 135                 140
   ctc ggt ggc gtg gaa tcc ctc ctg gag cac cca gca acc atg acc cac   480
   Leu Gly Gly Val Glu Ser Leu Leu Glu His Pro Ala Thr Met Thr His
   145                 150                 155                 160
   cag tca gct gcc ggc tct cag ctc gag gtt ccc cgc gac ctc gtg cgc   528
   Gln Ser Ala Ala Gly Ser Gln Leu Glu Val Pro Arg Asp Leu Val Arg
                   165                 170                 175
   atc tcc att ggt att gaa gac att gaa gac ctg ctc gca gat gtc gag   576
   Ile Ser Ile Gly Ile Glu Asp Ile Glu Asp Leu Leu Ala Asp Val Glu
               180                 185                 190
   cag gcc ctc aat aac ctt tagaaactat ttggcggcaa gca                617
   Gln Ala Leu Asn Asn Leu
           195
<210> 78
   <211> 198
   <212> PRT
   <213> Corynebacterium glutamicum
<400> 78
   Gln Pro Leu Lys Leu Gly Ala His Ala Val Leu His Ser Thr Thr Lys
    1               5                  10                  15
   Tyr Ile Gly Gly His Ser Asp Val Val Gly Gly Leu Val Val Thr Asn
               20                  25                  30
   Asp Gln Glu Met Asp Glu Glu Leu Leu Phe Met Gln Gly Gly Ile Gly
           35                  40                  45
   Pro Ile Pro Ser Val Phe Asp Ala Tyr Leu Thr Ala Arg Gly Leu Lys
       50                  55                  60
   Thr Leu Ala Val Arg Met Asp Arg His Cys Asp Asn Ala Glu Lys Ile
   65                  70                  75                  80
   Ala Glu Phe Leu Asp Ser Arg Pro Glu Val Ser Thr Val Leu Tyr Pro
                   85                  90                  95
   Gly Leu Lys Asn His Pro Gly His Glu Val Ala Ala Lys Gln Met Lys
               100                 105                 110
   Arg Phe Gly Gly Met Ile Ser Val Arg Phe Ala Gly Gly Glu Glu Ala
           115                 120                 125
   Ala Lys Lys Phe Cys Thr Ser Thr Lys Leu Ile Cys Leu Ala Glu Ser
       130                 135                 140
   Leu Gly Gly Val Glu Ser Leu Leu Glu His Pro Ala Thr Met Thr His
   145                 150                 155                 160
   Gln Ser Ala Ala Gly Ser Gln Leu Glu Val Pro Arg Asp Leu Val Arg
                   165                 170                 175
   Ile Ser Ile Gly Ile Glu Asp Ile Glu Asp Leu Leu Ala Asp Val Glu
               180                 185                 190
   Gln Ala Leu Asn Asn Leu
           195
<210> 79
   <211> 1170
   <212> DNA
   <213> Corynebacterium glutamicum
<220>
   <221> CDS
   <222> (101)..(1147)
   <223> RXA02532
<400> 79
   gatgaatttt tacccaccat ctgtacctat taaccctgcg tggcgtccac ccacagtaac 60
   tgtgcaagcg ggacggccag ccagaactcc tggtgcgccg atg aac cca cct atc   115
                                               Met Asn Pro Pro Ile
                                               1               5
   acg ttg tcc agc act tat gtt cat gat tca gaa aaa gct tat ggg cgc   163
   Thr Leu Ser Ser Thr Tyr Val His Asp Ser Glu Lys Ala Tyr Gly Arg
                   10                  15                  20
   gat ggc aat gat gga tgg ggt gca ttt gag gct gcc atg gga act cta   211
   Asp Gly Asn Asp Gly Trp Gly Ala Phe Glu Ala Ala Met Gly Thr Leu
               25                  30                  35
   gat ggt ggg ttc gcg gta tct tat tct tca ggt ttg gca gcg gca acg   259
   Asp Gly Gly Phe Ala Val Ser Tyr Ser Ser Gly Leu Ala Ala Ala Thr
           40                  45                  50
   tcg att gct gat ttg gtt cct act ggt ggc aca gtt gtt tta cct aaa   307
   Ser Ile Ala Asp Leu Val Pro Thr Gly Gly Thr Val Val Leu Pro Lys
       55                  60                  65
   gct gcc tat tat ggc gtg acc aat att ttc gcc agg atg gaa gcc cgc   355
   Ala Ala Tyr Tyr Gly Val Thr Asn Ile Phe Ala Arg Met Glu Ala Arg
   70                  75                  80                  85
   gga agg ctg aag gtt cga act gtt gat gca gac aat acc gaa gaa gtg   403
   Gly Arg Leu Lys Val Arg Thr Val Asp Ala Asp Asn Thr Glu Glu Val
                   90                  95                  100
   att gct gct gct caa ggt gca gat gtg gtg tgg gtg gaa tcg atc gct   451
   Ile Ala Ala Ala Gln Gly Ala Asp Val Val Trp Val Glu Ser Ile Ala
               105                 110                 115
   aat ccg acg atg gtg gta gct gat atc cct gca ata gtc gac ggt gtg   499
   Asn Pro Thr Met Val Val Ala Asp Ile Pro Ala Ile Val Asp Gly Val
           120                 125                 130
   cgt ggg ctt gga gtt ttg act gtc gtt gac gcg act ttc gca acg cca   547
   Arg Gly Leu Gly Val Leu Thr Val Val Asp Ala Thr Phe Ala Thr Pro
       135                 140                 145
   ctt cgt caa cgt cca ttg gaa ctt ggt gct gat att gtg ctt tac tcg   595
   Leu Arg Gln Arg Pro Leu Glu Leu Gly Ala Asp Ile Val Leu Tyr Ser
   150                 155                 160                 165
   gca acc aaa ctt atc ggt gga cac tct gat ctt ctt ctt gga gtc gca   643
   Ala Thr Lys Leu Ile Gly Gly His Ser Asp Leu Leu Leu Gly Val Ala
                   170                 175                 180
   gtg tgc aag tct gag cac cat gcg cag ttt ctt gcc act cac cgt cat   691
   Val Cys Lys Ser Glu His His Ala Gln Phe Leu Ala Thr His Arg His
                   185                 190                 195
   gat cat ggt tca gtg ccg gga ggt ctt gaa gcg ttt ctt gct ctc cgt     739
   Asp His Gly Ser Val Pro Gly Gly Leu Glu Ala Phe Leu Ala Leu Arg
           200                 205                 210
   gga ttg tat tcc ttg gcg gtg cgt ctt gat cga gca gaa tcc aac gca     787
   Gly Leu Tyr Ser Leu Ala Val Arg Leu Asp Arg Ala Glu Ser Asn Ala
       215                 220                 225
   gca gaa ctt tcg cgg cga ctt aac gcg cat cct tcg gtt acc cgc gtc     835
   Ala Glu Leu Ser Arg Arg Leu Asn Ala His Pro Ser Val Thr Arg Val
   230                 235                 240                 245
   aat tat cca gga ctt cct gat gat ccc caa cat gaa aaa gcc gtg cga     883
   Asn Tyr Pro Gly Leu Pro Asp Asp Pro Gln His Glu Lys Ala Val Arg
                   250                 255                 260
   gtc cta ccc tct gga tgt gga aac atg ttg tca ttt gag ctt gat gca     931
   Val Leu Pro Ser Gly Cys Gly Asn Met Leu Ser Phe Glu Leu Asp Ala
               265                 270                 275
   aca cct gaa cga act gat gag att ctc gaa agc ctg tca ctt tta acc     979
   Thr Pro Glu Arg Thr Asp Glu Ile Leu Glu Ser Leu Ser Leu Leu Thr
           280                 285                 290
   cac gcg acc agt tgg gga ggt gtg gaa aca gcc att gaa cgt cgc acc     1027
   His Ala Thr Ser Trp Gly Gly Val Glu Thr Ala Ile Glu Arg Arg Thr
       295                 300                 305
   agg cgg gat gct gaa gtg gtg gca gaa gta ccg atg act ctt tgc cgc     1075
   Arg Arg Asp Ala Glu Val Val Ala Glu Val Pro Met Thr Leu Cys Arg
   310                 315                 320                 325
   gtt tcc gta gga att gaa gac gtt gaa gat cta tgg gaa gac ctc aac     1123
   Val Ser Val Gly Ile Glu Asp Val Glu Asp Leu Trp Glu Asp Leu Asn
                   330                 335                 340
   gcc tca atc gac aaa gtt ctg ggt tagaactcgt agccagtaac cag           1170
   Ala Ser Ile Asp Lys Val Leu Gly
                   345
<210> 80
   <211> 349
   <212> PRT
   <213> Corynebacterium glutamicum
<400> 80
   Met Asn Pro Pro Ile Thr Leu Ser Ser Thr Tyr Val His Asp Ser Glu
   1               5                   10                  15
   Lys Ala Tyr Gly Arg Asp Gly Asn Asp Gly Trp Gly Ala Phe Glu Ala
               20                  25                  30
   Ala Met Gly Thr Leu Asp Gly Gly Phe Ala Val Ser Tyr Ser Ser Gly
           35                  40                  45
   Leu Ala Ala Ala Thr Ser Ile Ala Asp Leu Val Pro Thr Gly Gly Thr
       50                  55                  60
   Val Val Leu Pro Lys Ala Ala Tyr Tyr Gly Val Thr Asn Ile Phe Ala
   65                  70                  75                  80
   Arg Met Glu Ala Arg Gly Arg Leu Lys Val Arg Thr Val Asp Ala Asp
                   85                  90                  95
   Asn Thr Glu Glu Val Ile Ala Ala Ala Gln Gly Ala Asp Val Val Trp
               100                 105                 110
   Val Glu Ser Ile Ala Asn Pro Thr Met Val Val Ala Asp Ile Pro Ala
           115                 120                 125
   Ile Val Asp Gly Val Arg Gly Leu Gly Val Leu Thr Val Val Asp Ala
       130                 135                 140
   Thr Phe Ala Thr Pro Leu Arg Gln Arg Pro Leu Glu Leu Gly Ala Asp
   145                 150                 155                 160
   Ile Val Leu Tyr Ser Ala Thr Lys Leu Ile Gly Gly His Ser Asp Leu
                   165                 170                 175
   Leu Leu Gly Val Ala Val Cys Lys Ser Glu His His Ala Gln Phe Leu
               180                 185                 190
   Ala Thr His Arg His Asp His Gly Ser Val Pro Gly Gly Leu Glu Ala
           195                 200                 205
   Phe Leu Ala Leu Arg Gly Leu Tyr Ser Leu Ala Val Arg Leu Asp Arg
       210                 215                 220
   Ala Glu Ser Asn Ala Ala Glu Leu Ser Arg Arg Leu Asn Ala His Pro
   225                 230                 235                 240
   Ser Val Thr Arg Val Asn Tyr Pro Gly Leu Pro Asp Asp Pro Gln His
                   245                 250                 255
   Glu Lys Ala Val Arg Val Leu Pro Ser Gly Cys Gly Asn Met Leu Ser
               260                 265                 270
   Phe Glu Leu Asp Ala Thr Pro Glu Arg Thr Asp Glu Ile Leu Glu Ser
           275                 280                 285
   Leu Ser Leu Leu Thr His Ala Thr Ser Trp Gly Gly Val Glu Thr Ala
       290                 295                 300
   Ile Glu Arg Arg Thr Arg Arg Asp Ala Glu Val Val Ala Glu Val Pro
   305                 310                 315                 320
   Met Thr Leu Cys Arg Val Ser Val Gly Ile Glu Asp Val Glu Asp Leu
                   325                 330                 335
   Trp Glu Asp Leu Asn Ala Ser Ile Asp Lys Val Leu Gly
               340                 345
<210> 81
   <211> 861
   <212> DNA
   <213> Corynebacterium glutamicum
<220>
   <221> CDS
   <222> (101)..(838)
   <223> RXS03159
<400> 81
   aggggctagt tttacacaaa agtggacagc ttggtctatc attgccagaa gaccggtcct 60
   tttagggcca tagaattctg attacaggag ttgatctacc ttg tct ttt gac cca    115
                                               Leu Ser Phe Asp Pro
                                                1               5
   aac acc cag ggt ttc tcc act gca tcg att cac gct ggg tat gag cca    163
   Asn Thr Gln Gly Phe Ser Thr Ala Ser Ile His Ala Gly Tyr Glu Pro
                   10                  15                  20
   gac gac tac tac ggt tcg att aac acc cca atc tat gcc tcc acc acc    211
   Asp Asp Tyr Tyr Gly Ser Ile Asn Thr Pro Ile Tyr Ala Ser Thr Thr
               25                  30                  35
   ttc gcg cag aac gct cca aac gaa ctg cgc aaa ggc tac gag tac acc    259
   Phe Ala Gln Asn Ala Pro Asn Glu Leu Arg Lys Gly Tyr Glu Tyr Thr
           40                  45                  50
   cgt gtg ggc aac ccc acc atc gtg gca tta gag cag acc gtc gca gca    307
   Arg Val Gly Asn Pro Thr Ile Val Ala Leu Glu Gln Thr Val Ala Ala
       55                  60                  65
   ctc gaa ggc gca aag tat ggc cgc gca ttc tcc tcc ggc atg gct gca    355
   Leu Glu Gly Ala Lys Tyr Gly Arg Ala Phe Ser Ser Gly Met Ala Ala
   70                  75                  80                  85
   acc gac atc ctg ttc cgc atc atc ctc aag ccg ggc gat cac atc gtc    403
   Thr Asp Ile Leu Phe Arg Ile Ile Leu Lys Pro Gly Asp His Ile Val
                   90                  95                  100
   ctc ggc aac gat gct tac ggc gga acc tac cgc ctg atc gac acc gta    451
   Leu Gly Asn Asp Ala Tyr Gly Gly Thr Tyr Arg Leu Ile Asp Thr Val
               105                 110                 115
   ttc acc gca tgg ggc gtc gaa tac acc gtt gtt gat acc tcc gtc gtg    499
   Phe Thr Ala Trp Gly Val Glu Tyr Thr Val Val Asp Thr Ser Val Val
           120                 125                 130
   gaa gag gtc aag gca gcg atc aag gac aac acc aag ctg atc tgg gtg    547
   Glu Glu Val Lys Ala Ala Ile Lys Asp Asn Thr Lys Leu Ile Trp Val
       135                 140                 145
   gaa acc cca acc aac cca gca ctt ggc atc acc gac atc gaa gca gta    595
   Glu Thr Pro Thr Asn Pro Ala Leu Gly Ile Thr Asp Ile Glu Ala Val
   150                 155                 160                 165
   gca aag ctc acc gaa ggc acc aac gcc aag ttg gtt gtt gac aac acc    643
   Ala Lys Leu Thr Glu Gly Thr Asn Ala Lys Leu Val Val Asp Asn Thr
                   170                 175                 180
   ttg gca tcc cca tac ctg cag cag cca cta aaa ctc ggc gca cac gca    691
   Leu Ala Ser Pro Tyr Leu Gln Gln Pro Leu Lys Leu Gly Ala His Ala
               185                 190                 195
   agt cct tgc act cca cca cca agt aca tcg aag gac act ccg acg ttg    739
   Ser Pro Cys Thr Pro Pro Pro Ser Thr Ser Lys Asp Thr Pro Thr Leu
               200                 205                 210
   ttg gcg gcc ttg tgg gta cca acg acc agg aaa tgg acg aag aac tgc    787
   Leu Ala Ala Leu Trp Val Pro Thr Thr Arg Lys Trp Thr Lys Asn Cys
       215                 220                 225
   tgt tca tgc agg gcg gca tcg gac cga tcc cat cag ttt tcg atg cat    835
   Cys Ser Cys Arg Ala Ala Ser Asp Arg Ser His Gln Phe Ser Met His
   230                 235                 240                 245
   acc tgaccgcccg tggcctcaag acc                                      861
   Thr
<210> 82
   <211> 246
   <212> PRT
   <213> Corynebacterium glutamicum
<400> 82
   Leu Ser Phe Asp Pro Asn Thr Gln Gly Phe Ser Thr Ala Ser Ile His
   1               5                   10                  15
   Ala Gly Tyr Glu Pro Asp Asp Tyr Tyr Gly Ser Ile Asn Thr Pro Ile
               20                  25                  30
   Tyr Ala Ser Thr Thr Phe Ala Gln Asn Ala Pro Asn Glu Leu Arg Lys
           35                  40                  45
   Gly Tyr Glu Tyr Thr Arg Val Gly Asn Pro Thr Ile Val Ala Leu Glu
       50                  55                  60
   Gln Thr Val Ala Ala Leu Glu Gly Ala Lys Tyr Gly Arg Ala Phe Ser
   65                  70                  75                  80
   Ser Gly Met Ala Ala Thr Asp Ile Leu Phe Arg Ile Ile Leu Lys Pro
                   85                  90                  95
   Gly Asp His Ile Val Leu Gly Asn Asp Ala Tyr Gly Gly Thr Tyr Arg
               100                 105                 110
   Leu Ile Asp Thr Val Phe Thr Ala Trp Gly Val Glu Tyr Thr Val Val
           115                 120                 125
   Asp Thr Ser Val Val Glu Glu Val Lys Ala Ala Ile Lys Asp Asn Thr
       130                 135                 140
   Lys Leu Ile Trp Val Glu Thr Pro Thr Asn Pro Ala Leu Gly Ile Thr
   145                 150                 155                 160
   Asp Ile Glu Ala Val Ala Lys Leu Thr Glu Gly Thr Asn Ala Lys Leu
                   165                 170                 175
   Val Val Asp Asn Thr Leu Ala Ser Pro Tyr Leu Gln Gln Pro Leu Lys
               180                 185                 190
   Leu Gly Ala His Ala Ser Pro Cys Thr Pro Pro Pro Ser Thr Ser Lys
           195                 200                 205
       Asp Thr Pro Thr Leu Leu Ala Ala Leu Trp Val Pro Thr Thr Arg Lys
   210                 215                 220
   Trp Thr Lys Asn Cys Cys Ser Cys Arg Ala Ala Ser Asp Arg Ser His
   225                 230                 235                 240
   Gln Phe Ser Met His Thr
                   245
<210> 83
   <211> 703
   <212> DNA
   <213> Corynebacterium glutamicum
<220>
   <221> CDS
   <222> (101)..(703)
   <223> FRXA02768
<220>
   <223> All occurrences of n = any nucleotide
<220>
   <223> All occurrences of Xaa = any amino acid
<400> 83
   aggggctagt tttacacaaa agtggacagc ttggtctatc attgccagaa gaccggtcct 60
   tttagggcca tagaattctg attacaggag ttgatctacc ttg tct ttt gac cca   115
                                               Leu Ser Phe Asp Pro
                                                1               5
   aac acc cag ggt ttc tcc act gca tcg att cac gct ggg tat gag cca   163
   Asn Thr Gln Gly Phe Ser Thr Ala Ser Ile His Ala Gly Tyr Glu Pro
                   10                  15                  20
   gac gac tac tac ggt tcg att aac acc cca atc tat gcc tcc acc acc   211
   Asp Asp Tyr Tyr Gly Ser Ile Asn Thr Pro Ile Tyr Ala Ser Thr Thr
               25                  30                  35
   ttc gcg cag aac gct cca aac gaa ctg cgc aaa ggc tac gag tac acc   259
   Phe Ala Gln Asn Ala Pro Asn Glu Leu Arg Lys Gly Tyr Glu Tyr Thr
           40                  45                  50
   cgt gtg ggc aac ccc acc atc gtg gca tta gag cag acc gtc gca gca   307
   Arg Val Gly Asn Pro Thr Ile Val Ala Leu Glu Gln Thr Val Ala Ala
       55                  60                  65
   ctc gaa ggc gca aag tat ggc cgc gca ttc tcc tcc ggc atg gct gca   355
   Leu Glu Gly Ala Lys Tyr Gly Arg Ala Phe Ser Ser Gly Met Ala Ala
   70                  75                  80                  85
   acc gac atc ctg ttc cgc atc atc ctc aag ccg ggc gat cac atc gtc   403
   Thr Asp Ile Leu Phe Arg Ile Ile Leu Lys Pro Gly Asp His Ile Val
                   90                  95                  100
   ctc ggc aac gat gct tac ggc gga acc tac cgc ctg atc gac acc gta   451
   Leu Gly Asn Asp Ala Tyr Gly Gly Thr Tyr Arg Leu Ile Asp Thr Val
               105                 110                 115
   ttc acc gca tgg ggc gtc gaa tac acc gtt gtt gat acc tcc gtc gtg   499
   Phe Thr Ala Trp Gly Val Glu Tyr Thr Val Val Asp Thr Ser Val Val
           120                 125                 130
   gaa gag gtc aag gca gcg atc aag gac aac acc aag gct gat ctt ggt      547
   Glu Glu Val Lys Ala Ala Ile Lys Asp Asn Thr Lys Ala Asp Leu Gly
       135                 140                 145
   gga aac ccc aac caa ccc agc act ttg gca tta ccc gac atc gaa gca      595
   Gly Asn Pro Asn Gln Pro Ser Thr Leu Ala Leu Pro Asp Ile Glu Ala
   150                 155                 160                 165
   gtn tgc aaa act tca ccc gaa agg cac caa ccc caa gct tgt tgt ttg      643
   Val Cys Lys Thr Ser Pro Glu Arg His Gln Pro Gln Ala Cys Cys Leu
                   170                 175                 180
   aca aca cct tcg cat tcc cca tac ctg cag can cca ctt aaa ant tnn      691
   Thr Thr Pro Ser His Ser Pro Tyr Leu Gln Xaa Pro Leu Lys Xaa Xaa
               185                 190                 195
   gng cac acg cag                                                      703
   Xaa His Thr Gln
           200
<210> 84
   <211> 201
   <212> PRT
   <213> Corynebacterium glutamicum
<220>
   <223> All occurrences of Xaa = any amino acid
<400> 84
   Leu Ser Phe Asp Pro Asn Thr Gln Gly Phe Ser Thr Ala Ser Ile His
   1               5                   10                  15
   Ala Gly Tyr Glu Pro Asp Asp Tyr Tyr Gly Ser Ile Asn Thr Pro Ile
               20                  25                  30
   Tyr Ala Ser Thr Thr Phe Ala Gln Asn Ala Pro Asn Glu Leu Arg Lys
           35                  40                  45
   Gly Tyr Glu Tyr Thr Arg Val Gly Asn Pro Thr Ile Val Ala Leu Glu
       50                  55                  60
   Gln Thr Val Ala Ala Leu Glu Gly Ala Lys Tyr Gly Arg Ala Phe Ser
   65                  70                  75                  80
   Ser Gly Met Ala Ala Thr Asp Ile Leu Phe Arg Ile Ile Leu Lys Pro
                   85                  90                  95
   Gly Asp His Ile Val Leu Gly Asn Asp Ala Tyr Gly Gly Thr Tyr Arg
               100                 105                 110
   Leu Ile Asp Thr Val Phe Thr Ala Trp Gly Val Glu Tyr Thr Val Val
           115                 120                 125
   Asp Thr Ser Val Val Glu Glu Val Lys Ala Ala Ile Lys Asp Asn Thr
       130                 135                 140
   Lys Ala Asp Leu Gly Gly Asn Pro Asn Gln Pro Ser Thr Leu Ala Leu
   145                 150                 155                 160
   Pro Asp Ile Glu Ala Val Cys Lys Thr Ser Pro Glu Arg His Gln Pro
                   165                 170                 175
   Gln Ala Cys Cys Leu Thr Thr Pro Ser His Ser Pro Tyr Leu Gln Xaa
               180                 185                 190
   Pro Leu Lys Xaa Xaa Xaa His Thr Gln
           195                 200
<210> 85
   <211> 1113
   <212> DNA
   <213> Corynebacterium glutamicum
<220>
   <221> CDS
   <222> (101)..(1090)
   <223> RXA00216
<400> 85
   gtgttgctcg cggccaggca gcagtgctgt acctgcctga cgcggatggt gacatcgttc 60
   ttggatcagg caccatctgc cacacggagt cttaagaaaa ttg ggc gct tat ggt   115
                                               Leu Gly Ala Tyr Gly
                                                1               5
   tta ggt gag ctt cct gga aaa tcc gcc gcg gaa gcc gcc gac att att   163
   Leu Gly Glu Leu Pro Gly Lys Ser Ala Ala Glu Ala Ala Asp Ile Ile
                   10                  15                  20
   cag ggt gaa acg ggc gat ctt ctc cat att cct cag ctt ccg gcg cga   211
   Gln Gly Glu Thr Gly Asp Leu Leu His Ile Pro Gln Leu Pro Ala Arg
               25                  30                  35
   ggt ttg ggt gct gat ctg atc ggt cga acc gtc ggt ctg ctg gac atg   259
   Gly Leu Gly Ala Asp Leu Ile Gly Arg Thr Val Gly Leu Leu Asp Met
           40                  45                  50
   atc aac gtt gat cgc ggg gcc cga tct tgg gtg atg agc aca cgc ccc   307
   Ile Asn Val Asp Arg Gly Ala Arg Ser Trp Val Met Ser Thr Arg Pro
       55                  60                  65
   agc aga ttg acg cac ctg acc ggc gat ttc ctt gac atg gat ttg gat   355
   Ser Arg Leu Thr His Leu Thr Gly Asp Phe Leu Asp Met Asp Leu Asp
   70                  75                  80                  85
   gcg tgc gag gaa acc tgg gga acg ggc gtc gac aag cta aaa atc caa   403
   Ala Cys Glu Glu Thr Trp Gly Thr Gly Val Asp Lys Leu Lys Ile Gln
                   90                  95                  100
   gtt gct ggt ccc tgg act tta ggt gcg cgc att gag ttg gcc aat ggc   451
   Val Ala Gly Pro Trp Thr Leu Gly Ala Arg Ile Glu Leu Ala Asn Gly
               105                 110                 115
   cat cgc gtt ttg tct gat cgc ggt gcg atg cgt gat ctc acg cag gcg   499
   His Arg Val Leu Ser Asp Arg Gly Ala Met Arg Asp Leu Thr Gln Ala
           120                 125                 130
   ctg atc gcc ggc atc gat gcg cat gca cgc aag gtt gct ggg cga ttt    547
   Leu Ile Ala Gly Ile Asp Ala His Ala Arg Lys Val Ala Gly Arg Phe
       135                 140                 145
   cgc gcc gaa gtg cag gtg caa att gat gag ccg gag ctg aaa tcg ctt    595
   Arg Ala Glu Val Gln Val Gln Ile Asp Glu Pro Glu Leu Lys Ser Leu
   150                 155                 160                 165
   atc gac ggc tcc ctc cct ggc act tcc acc ttt gac att att cct gcg    643
   Ile Asp Gly Ser Leu Pro Gly Thr Ser Thr Phe Asp Ile Ile Pro Ala
                   170                 175                 180
   gtg aat gtc gct gat gcc agt gaa cgt ttg cag cag gtc ttt agc tcg    691
   Val Asn Val Ala Asp Ala Ser Glu Arg Leu Gln Gln Val Phe Ser Ser
               185                 190                 195
   att gag ggg ccg aca tat ctc aac ctc acc ggc cag att cct act tgg    739
   Ile Glu Gly Pro Thr Tyr Leu Asn Leu Thr Gly Gln Ile Pro Thr Trp
           200                 205                 210
   gat gtg gct cgg ggt gcg ggc gcc gat act gtg cag att tcc atg gat    787
   Asp Val Ala Arg Gly Ala Gly Ala Asp Thr Val Gln Ile Ser Met Asp
       215                 220                 225
   caa gtc cgt gga aat gaa cat ttg gat ggt ttt ggt gaa acc atc acc    835
   Gln Val Arg Gly Asn Glu His Leu Asp Gly Phe Gly Glu Thr Ile Thr
   230                 235                 240                 245
   agt gga att cgt ctt ggt ttg ggc att acg aca gga aaa gat gtc gta    883
   Ser Gly Ile Arg Leu Gly Leu Gly Ile Thr Thr Gly Lys Asp Val Val
                   250                 255                 260
   gat gaa ctg ctc gag cga ccg cgg caa aag gcc gtt gag gta gca cgc    931
   Asp Glu Leu Leu Glu Arg Pro Arg Gln Lys Ala Val Glu Val Ala Arg
               265                 270                 275
   ttt ttt gat cgt tta ggt gtg ggc cga aac tat ctc gtg gat gct gtt    979
   Phe Phe Asp Arg Leu Gly Val Gly Arg Asn Tyr Leu Val Asp Ala Val
           280                 285                 290
   gat att cat ccg ggt gag gat ttg gtg cag ggg acc atc acc gag gcc    1027
   Asp Ile His Pro Gly Glu Asp Leu Val Gln Gly Thr Ile Thr Glu Ala
       295                 300                 305
   gcg cag gct tat cgc atg gcc cgg gtg atg tcg gag atg ttg tcg aag    1075
   Ala Gln Ala Tyr Arg Met Ala Arg Val Met Ser Glu Met Leu Ser Lys
   310                 315                 320                 325
   gat tca tgc gac ctt taaggcttta ccggcgctgg gtg                      1113
   Asp Ser Cys Asp Leu
                   330
<210> 86
   <211> 330
   <212> PRT
   <213> Corynebacterium glutamicum
<900> 86
<210> 87
   <211> 551
   <212> DNA
   <213> Corynebacterium glutamicum
<220>
   <221> CDS
   <222> (1)..(528)
   <223> RXA02197
<400> 87
   gcc gaa cgc atg cgc ttt agc ttc cca cgc cag cag cgc ggc agg ttc      48
   Ala Glu Arg Met Arg Phe Ser Phe Pro Arg Gln Gln Arg Gly Arg Phe
     1               5                  10                  15
   ttg tgc atc gcg gat ttc att cgc cca cgc gag caa gct gtc aag gac      96
   Leu Cys Ile Ala Asp Phe Ile Arg Pro Arg Glu Gln Ala Val Lys Asp
                20                  25                  30
   ggc caa gtg gac gtc atg cca ttc cag ctg gtc acc atg ggt aat cct     144
   Gly Gln Val Asp Val Met Pro Phe Gln Leu Val Thr Met Gly Asn Pro
            35                  40                  45
   att gct gat ttc gcc aac gag ttg ttc gca gcc aat gaa tac cgc gag     192
   Ile Ala Asp Phe Ala Asn Glu Leu Phe Ala Ala Asn Glu Tyr Arg Glu
        50                  55                  60
   tac ttg gaa gtt cac ggc atc ggc gtg cag ctc acc gaa gca ttg gcc     240
   Tyr Leu Glu Val His Gly Ile Gly Val Gln Leu Thr Glu Ala Leu Ala
    65                  70                  75                  80
   gag tac tgg cac tcc cga gtg cgc agc gaa ctc aag ctg aac gac ggt     288
   Glu Tyr Trp His Ser Arg Val Arg Ser Glu Leu Lys Leu Asn Asp Gly
                    85                  90                  95
   gga tct gtc gct gat ttt gat cca gaa gac aag acc aag ttc ttc gac     336
   Gly Ser Val Ala Asp Phe Asp Pro Glu Asp Lys Thr Lys Phe Phe Asp
               100                 105                 110
   ctg gat tac cgc ggc gcc cgc ttc tcc ttt ggt tac ggt tct tgc cct     384
   Leu Asp Tyr Arg Gly Ala Arg Phe Ser Phe Gly Tyr Gly Ser Cys Pro
           115                 120                 125
   gat ctg gaa gac cgc gca aag ctg gtg gaa ttg ctc gag cca ggc cgt     432
   Asp Leu Glu Asp Arg Ala Lys Leu Val Glu Leu Leu Glu Pro Gly Arg
       130                 135                 140
   atc ggc gtg gag ttg tcc gag gaa ctc cag ctg cac cca gag cag tcc     480
   Ile Gly Val Glu Leu Ser Glu Glu Leu Gln Leu His Pro Glu Gln Ser
   145                 150                 155                 160
   aca gac gcg ttt gtg ctc tac cac cca gag gca aag tac ttt aac gtc     528
   Thr Asp Ala Phe Val Leu Tyr His Pro Glu Ala Lys Tyr Phe Asn Val
                   165                 170                 175
   taacaccttt gagagggaaa act                                           551
<210> 88
   <211> 176
   <212> PRT
   <213> Corynebacterium glutamicum
<400> 88
   Ala Glu Arg Met Arg Phe Ser Phe Pro Arg Gln Gln Arg Gly Arg Phe
   1               5                   10                  15
   Leu Cys Ile Ala Asp Phe Ile Arg Pro Arg Glu Gln Ala Val Lys Asp
               20                  25                  30
   Gly Gln Val Asp Val Met Pro Phe Gln Leu Val Thr Met Gly Asn Pro
           35                  40                  45
   Ile Ala Asp Phe Ala Asn Glu Leu Phe Ala Ala Asn Glu Tyr Arg Glu
       50                  55                  60
   Tyr Leu Glu Val His Gly Ile Gly Val Gln Leu Thr Glu Ala Leu Ala
   65                  70                  75                  80
   Glu Tyr Trp His Ser Arg Val Arg Ser Glu Leu Lys Leu Asn Asp Gly
                   85                  90                  95
   Gly Ser Val Ala Asp Phe Asp Pro Glu Asp Lys Thr Lys Phe Phe Asp
               100                 105                 110
   Leu Asp Tyr Arg Gly Ala Arg Phe Ser Phe Gly Tyr Gly Ser Cys Pro
           115                 120                 125
   Asp Leu Glu Asp Arg Ala Lys Leu Val Glu Leu Leu Glu Pro Gly Arg
       130                 135                 140
   Ile Gly Val Glu Leu Ser Glu Glu Leu Gln Leu His Pro Glu Gln Ser
   145                 150                 155                 160
   Thr Asp Ala Phe Val Leu Tyr His Pro Glu Ala Lys Tyr Phe Asn Val
                   165                 170                 175
<210> 89
   <211> 2599
   <212> DNA
   <213> Corynebacterium glutamicum
<220>
   <221> CDS
   <222> (101)..(2599)
   <223> RXN02198
<400> 89
   agactagtgg cgctttgcct gtgttgctta ggcggcgttg aaaatgaact acgaatgaaa 60
   agttcgggaa ttgtctaatc cgtactaagc tgtctacaca atg tct act tca gtt   115
                                              Met Ser Thr Ser Val
                                              1               5
   act tca cca gcc cac aac aac gca cat tcc tcc gaa ttt ttg gat gcg   163
   Thr Ser Pro Ala His Asn Asn Ala His Ser Ser Glu Phe Leu Asp Ala
                   10                  15                  20
   ttg gca aac cat gtg ttg atc ggc gac ggc gcc atg ggc acc cag ctc   211
   Leu Ala Asn His Val Leu Ile Gly Asp Gly Ala Met Gly Thr Gln Leu
                   25                  30                  35
   caa ggc ttt gac ctg gac gtg gaa aag gat ttc ctt gat ctg gag ggg      259
   Gln Gly Phe Asp Leu Asp Val Glu Lys Asp Phe Leu Asp Leu Glu Gly
           40                  45                  50
   tgt aat gag att ctc aac gac acc cgc cct gat gtg ttg agg cag att      307
   Cys Asn Glu Ile Leu Asn Asp Thr Arg Pro Asp Val Leu Arg Gln Ile
       55                  60                  65
   cac cgc gcc tac ttt gag gcg gga gct gac ttg gtt gag acc aat act      355
   His Arg Ala Tyr Phe Glu Ala Gly Ala Asp Leu Val Glu Thr Asn Thr
   70                  75                  80                  85
   ttt ggt tgc aac ctg ccg aac ttg gcg gat tat gac atc gct gat cgt      403
   Phe Gly Cys Asn Leu Pro Asn Leu Ala Asp Tyr Asp Ile Ala Asp Arg
                   90                  95                  100
   tgc cgt gag ctt gcc tac aag ggc act gca gtg gct agg gaa gtg gct      451
   Cys Arg Glu Leu Ala Tyr Lys Gly Thr Ala Val Ala Arg Glu Val Ala
               105                 110                 115
   gat gag atg ggg ccg ggc cga aac ggc atg cgg cgt ttc gtg gtt ggt      499
   Asp Glu Met Gly Pro Gly Arg Asn Gly Met Arg Arg Phe Val Val Gly
           120                 125                 130
   tcc ctg gga cct gga acg aag ctt cca tcg ctg ggc cat gca ccg tat      547
   Ser Leu Gly Pro Gly Thr Lys Leu Pro Ser Leu Gly His Ala Pro Tyr
       135                 140                 145
   gca gat ttg cgt ggg cac tac aag gaa gca gcg ctt ggc atc atc gac      595
   Ala Asp Leu Arg Gly His Tyr Lys Glu Ala Ala Leu Gly Ile Ile Asp
   150                 155                 160                 165
   ggt ggt ggc gat gcc ttt ttg att gag act gct cag gac ttg ctt cag      643
   Gly Gly Gly Asp Ala Phe Leu Ile Glu Thr Ala Gln Asp Leu Leu Gln
                   170                 175                 180
   gtc aag gct gcg gtt cac ggc gtt caa gat gcc atg gct gaa ctt gat      691
   Val Lys Ala Ala Val His Gly Val Gln Asp Ala Met Ala Glu Leu Asp
               185                 190                 195
   aca ttc ttg ccc att att tgc cac gtc acc gta gag acc acc ggc acc      739
   Thr Phe Leu Pro Ile Ile Cys His Val Thr Val Glu Thr Thr Gly Thr
           200                 205                 210
   atg ctc atg ggt tct gag atc ggt gcc gcg ttg aca gcg ctg cag cca      787
   Met Leu Met Gly Ser Glu Ile Gly Ala Ala Leu Thr Ala Leu Gln Pro
       215                 220                 225
   ctg ggt atc gac atg att ggt ctg aac tgc gcc acc ggc cca gat gag      835
   Leu Gly Ile Asp Met Ile Gly Leu Asn Cys Ala Thr Gly Pro Asp Glu
   230                 235                 240                 245
   atg agc gag cac ctg cgt tac ctg tcc aag cac gcc gat att cct gtg      883
   Met Ser Glu His Leu Arg Tyr Leu Ser Lys His Ala Asp Ile Pro Val
                   250                 255                 260
   tcg gtg atg cct aac gca ggt ctt cct gtc ctg ggt aaa aac ggt gca      931
   Ser Val Met Pro Asn Ala Gly Leu Pro Val Leu Gly Lys Asn Gly Ala
               265                 270                 275
   gaa tac cca ctt gag gct gag gat ttg gcg cag gcg ctg gct gga ttc     979
   Glu Tyr Pro Leu Glu Ala Glu Asp Leu Ala Gln Ala Leu Ala Gly Phe
           280                 285                 290
   gtc tcc gaa tat ggc ctg tcc atg gtg ggt ggt tgt tgt ggc acc aca    1027
   Val Ser Glu Tyr Gly Leu Ser Met Val Gly Gly Cys Cys Gly Thr Thr
       295                 300                 305
   cct gag cac atc cgt gcg gtc cgc gat gcg gtg gtt ggt gtt cca gag    1075
   Pro Glu His Ile Arg Ala Val Arg Asp Ala Val Val Gly Val Pro Glu
   310                 315                 320                 325
   cag gaa acc tcc aca ctg acc aag atc cct gca ggc cct gtt gag cag    1123
   Gln Glu Thr Ser Thr Leu Thr Lys Ile Pro Ala Gly Pro Val Glu Gln
                   330                 335                 340
   gcc tcc cgc gag gtg gag aaa gag gac tcc gtc gcg tcg ctg tac acc    1171
   Ala Ser Arg Glu Val Glu Lys Glu Asp Ser Val Ala Ser Leu Tyr Thr
               345                 350                 355
   tcg gtg cca ttg tcc cag gaa acc ggc att tcc atg atc ggt gag cgc    1219
   Ser Val Pro Leu Ser Gln Glu Thr Gly Ile Ser Met Ile Gly Glu Arg
           360                 365                 370
   acc aac tcc aac ggt tcc aag gca ttc cgt gag gca atg ctg tct ggc    1267
   Thr Asn Ser Asn Gly Ser Lys Ala Phe Arg Glu Ala Met Leu Ser Gly
       375                 380                 385
   gat tgg gaa aag tgt gtg gat att gcc aag cag caa acc cgc gat ggt    1315
   Asp Trp Glu Lys Cys Val Asp Ile Ala Lys Gln Gln Thr Arg Asp Gly
   390                 395                 400                 405
   gca cac atg ctg gat ctt tgt gtg gat tac gtg gga cga gac ggc acc    1363
   Ala His Met Leu Asp Leu Cys Val Asp Tyr Val Gly Arg Asp Gly Thr
                   410                 415                 420
   gcc gat atg gcg acc ttg gca gca ctt ctt gct acc agc tcc act ttg    1411
   Ala Asp Met Ala Thr Leu Ala Ala Leu Leu Ala Thr Ser Ser Thr Leu
               425                 430                 435
   cca atc atg att gac tcc acc gag cca gag gtt att cgc aca ggc ctt    1459
   Pro Ile Met Ile Asp Ser Thr Glu Pro Glu Val Ile Arg Thr Gly Leu
           440                 445                 450
   gag cac ttg ggt gga cga agc atc gtt aac tcc gtc aac ttt gaa gac    1507
   Glu His Leu Gly Gly Arg Ser Ile Val Asn Ser Val Asn Phe Glu Asp
       455                 460                 465
   ggc gat ggc cct gag tcc cgc tac cag cgc atc atg aaa ctg gta aag    1555
   Gly Asp Gly Pro Glu Ser Arg Tyr Gln Arg Ile Met Lys Leu Val Lys
   470                 475                 480                 485
   cag cac ggt gcg gcc gtg gtt gcg ctg acc att gat gag gaa ggc cag    1603
   Gln His Gly Ala Ala Val Val Ala Leu Thr Ile Asp Glu Glu Gly Gln
                   490                 495                 500
   gca cgt acc gct gag cac aag gtg cgc att gct aaa cga ctg att gac    1651
   Ala Arg Thr Ala Glu His Lys Val Arg Ile Ala Lys Arg Leu Ile Asp
               505                 510                 515
   gat atc acc ggc agc tac ggc ctg gat atc aaa gac atc gtt gtg gac    1699
   Asp Ile Thr Gly Ser Tyr Gly Leu Asp Ile Lys Asp Ile Val Val Asp
           520                 525                 530
   tgc ctg acc ttc ccg atc tct act ggc cag gaa gaa acc agg cga gat    1747
   Cys Leu Thr Phe Pro Ile Ser Thr Gly Gln Glu Glu Thr Arg Arg Asp
       535                 540                 545
   ggc att gaa acc atc gaa gcc atc cgc gag ctg aag aag ctc tac cca    1795
   Gly Ile Glu Thr Ile Glu Ala Ile Arg Glu Leu Lys Lys Leu Tyr Pro
   550                 555                 560                 565
   gaa atc cac acc acc ctg ggt ctg tcc aat att tcc ttc ggc ctg aac    1843
   Glu Ile His Thr Thr Leu Gly Leu Ser Asn Ile Ser Phe Gly Leu Asn
                   570                 575                 580
   cct gct gca cgc cag gtt ctt aac tct gtg ttc ctc aat gag tgc att    1891
   Pro Ala Ala Arg Gln Val Leu Asn Ser Val Phe Leu Asn Glu Cys Ile
               585                 590                 595
   gag gct ggt ctg gac tct gcg att gcg cac agc tcc aag att ttg ccg    1939
   Glu Ala Gly Leu Asp Ser Ala Ile Ala His Ser Ser Lys Ile Leu Pro
           600                 605                 610
   atg aac cgc att gat gat cgc cag cgc gaa gtg gcg ttg gat atg gtc    1987
   Met Asn Arg Ile Asp Asp Arg Gln Arg Glu Val Ala Leu Asp Met Val
       615                 620                 625
   tat gat cgc cgc acc gag gat tac gat ccg ctg cag gaa ttc atg cag    2035
   Tyr Asp Arg Arg Thr Glu Asp Tyr Asp Pro Leu Gln Glu Phe Met Gln
   630                 635                 640                 645
   ctg ttt gag ggc gtt tct gct gcc gat gcc aag gat gct cgc gct gaa    2083
   Leu Phe Glu Gly Val Ser Ala Ala Asp Ala Lys Asp Ala Arg Ala Glu
                   650                 655                 660
   cag ctg gcc gct atg cct ttg ttt gag cgt ttg gca cag cgc atc atc    2131
   Gln Leu Ala Ala Met Pro Leu Phe Glu Arg Leu Ala Gln Arg Ile Ile
               665                 670                 675
   gac ggc gat aag aat ggc ctt gag gat gat ctg gaa gca ggc atg aag    2179
   Asp Gly Asp Lys Asn Gly Leu Glu Asp Asp Leu Glu Ala Gly Met Lys
           680                 685                 690
   gag aag tct cct att gcg atc atc aac gag gac ctt ctc aac ggc atg    2227
   Glu Lys Ser Pro Ile Ala Ile Ile Asn Glu Asp Leu Leu Asn Gly Met
       695                 700                 705
   aag acc gtg ggt gag ctg ttt ggt tcc gga cag atg cag ctg cca ttc    2275
   Lys Thr Val Gly Glu Leu Phe Gly Ser Gly Gln Met Gln Leu Pro Phe
   710                 715                 720                 725
   gtg ctg caa tcg gca gaa acc atg aaa act gcg gtg gcc tat ttg gaa    2323
   Val Leu Gln Ser Ala Glu Thr Met Lys Thr Ala Val Ala Tyr Leu Glu
                   730                 735                 740
   ccg ttc atg gaa gag gaa gca gaa gct acc gga tct gcg cag gca gag    2371
   Pro Phe Met Glu Glu Glu Ala Glu Ala Thr Gly Ser Ala Gln Ala Glu
               745                 750                 755
   ggc aag ggc aaa atc gtc gtg gcc acc gtc aag ggt gac gtg cac gat    2419
   Gly Lys Gly Lys Ile Val Val Ala Thr Val Lys Gly Asp Val His Asp
           760                 765                 770
   atc ggc aag aac ttg gtg gac atc att ttg tcc aac aac ggt tac gac    2467
   Ile Gly Lys Asn Leu Val Asp Ile Ile Leu Ser Asn Asn Gly Tyr Asp
       775                 780                 785
   gtg gtg aac ttg ggc atc aag cag cca ctg tcc gcc atg ttg gaa gca    2515
   Val Val Asn Leu Gly Ile Lys Gln Pro Leu Ser Ala Met Leu Glu Ala
   790                 795                 800                 805
   gcg gaa gaa cac aaa gca gac gtc atc ggc atg tcg gga ctt ctt gtg    2563
   Ala Glu Glu His Lys Ala Asp Val Ile Gly Met Ser Gly Leu Leu Val
                   810                 815                 820
   aag tcc acc gtg gtg atg aag caa acc atc agc gac                    2599
   Lys Ser Thr Val Val Met Lys Gln Thr Ile Ser Asp
               825                 830
<210> 90
   <211> 833
   <212> PRT
   <213> Corynebacterium glutamicum
<400> 90
   Met Ser Thr Ser Val Thr Ser Pro Ala His Asn Asn Ala His Ser Ser
    1               5                  10                  15
   Glu Phe Leu Asp Ala Leu Ala Asn His Val Leu Ile Gly Asp Gly Ala
               20                  25                  30
   Met Gly Thr Gln Leu Gln Gly Phe Asp Leu Asp Val Glu Lys Asp Phe
           35                  40                  45
   Leu Asp Leu Glu Gly Cys Asn Glu Ile Leu Asn Asp Thr Arg Pro Asp
       50                  55                  60
   Val Leu Arg Gln Ile His Arg Ala Tyr Phe Glu Ala Gly Ala Asp Leu
    65                  70                  75                  80
   Val Glu Thr Asn Thr Phe Gly Cys Asn Leu Pro Asn Leu Ala Asp Tyr
                   85                  90                  95
   Asp Ile Ala Asp Arg Cys Arg Glu Leu Ala Tyr Lys Gly Thr Ala Val
               100                 105                 110
   Ala Arg Glu Val Ala Asp Glu Met Gly Pro Gly Arg Asn Gly Met Arg
           115                 120                 125
   Arg Phe Val Val Gly Ser Leu Gly Pro Gly Thr Lys Leu Pro Ser Leu
       130                 135                 140
   Gly His Ala Pro Tyr Ala Asp Leu Arg Gly His Tyr Lys Glu Ala Ala
   145                 150                 155                 160
   Leu Gly Ile Ile Asp Gly Gly Gly Asp Ala Phe Leu Ile Glu Thr Ala
                   165                 170                 175
   Gln Asp Leu Leu Gln Val Lys Ala Ala Val His Gly Val Gln Asp Ala
               180                 185                 190
   Met Ala Glu Leu Asp Thr Phe Leu Pro Ile Ile Cys His Val Thr Val
   195 200 205
   Glu Thr Thr Gly Thr Met Leu Met Gly Ser Glu Ile Gly Ala Ala Leu
   210 215 220
   Thr Ala Leu Gln Pro Leu Gly Ile Asp Met Ile Gly Leu Asn Cys Ala
   225 230 235 240
   Thr Gly Pro Asp Glu Met Ser Glu His Leu Arg Tyr Leu Ser Lys His
   245 250 255
   Ala Asp Ile Pro Val Ser Val Met Pro Asn Ala Gly Leu Pro Val Leu
   260 265 270
   Gly Lys Asn Gly Ala Glu Tyr Pro Leu Glu Ala Glu Asp Leu Ala Gln
   275 280 285
   Ala Leu Ala Gly Phe Val Ser Glu Tyr Gly Leu Ser Met Val Gly Gly
   290 295 300
   Cys Cys Gly Thr Thr Pro Glu His Ile Arg Ala Val Arg Asp Ala Val
   305 310 315 320
   Val Gly Val Pro Glu Gln Glu Thr Ser Thr Leu Thr Lys Ile Pro Ala
   325 330 335
   Gly Pro Val Glu Gln Ala Ser Arg Glu Val Glu Lys Glu Asp Ser Val
   340 345 350
   Ala Ser Leu Tyr Thr Ser Val Pro Leu Ser Gln Glu Thr Gly Ile Ser
   355 360 365
   Met Ile Gly Glu Arg Thr Asn Ser Asn Gly Ser Lys Ala Phe Arg Glu
   370 375 380
   Ala Met Leu Ser Gly Asp Trp Glu Lys Cys Val Asp Ile Ala Lys Gln
   385 390 395 400
   Gln Thr Arg Asp Gly Ala His Met Leu Asp Leu Cys Val Asp Tyr Val
   405 410 415
   Gly Arg Asp Gly Thr Ala Asp Met Ala Thr Leu Ala Ala Leu Leu Ala
   420 425 430
   Thr Ser Ser Thr Leu Pro Ile Met Ile Asp Ser Thr Glu Pro Glu Val
   435 440 445
   Ile Arg Thr Gly Leu Glu His Leu Gly Gly Arg Ser Ile Val Asn Ser
   450 455 460
   Val Asn Phe Glu Asp Gly Asp Gly Pro Glu Ser Arg Tyr Gln Arg Ile
   465 470 475 480
   Met Lys Leu Val Lys Gln His Gly Ala Ala Val Val Ala Leu Thr Ile
   485 490 495
   Asp Glu Glu Gly Gln Ala Arg Thr Ala Glu His Lys Val Arg Ile Ala
   500 505 510
   Lys Arg Leu Ile Asp Asp Ile Thr Gly Ser Tyr Gly Leu Asp Ile Lys
   515 520 525
   Asp Ile Val Val Asp Cys Leu Thr Phe Pro Ile Ser Thr Gly Gln Glu
   530 535 540
   Glu Thr Arg Arg Asp Gly Ile Glu Thr Ile Glu Ala Ile Arg Glu Leu
   545 550 555 560
   Lys Lys Leu Tyr Pro Glu Ile His Thr Thr Leu Gly Leu Ser Asn Ile
   565 570 575
   Ser Phe Gly Leu Asn Pro Ala Ala Arg Gln Val Leu Asn Ser Val Phe
   580 585 590
   Leu Asn Glu Cys Ile Glu Ala Gly Leu Asp Ser Ala Ile Ala His Ser
   595 600 605
   Ser Lys Ile Leu Pro Met Asn Arg Ile Asp Asp Arg Gln Arg Glu Val
   610 615 620
   Ala Leu Asp Met Val Tyr Asp Arg Arg Thr Glu Asp Tyr Asp Pro Leu
   625 630 635 640
   Gln Glu Phe Met Gln Leu Phe Glu Gly Val Ser Ala Ala Asp Ala Lys
   645 650 655
   Asp Ala Arg Ala Glu Gln Leu Ala Ala Met Pro Leu Phe Glu Arg Leu
   660 665 670
   Ala Gln Arg Ile Ile Asp Gly Asp Lys Asn Gly Leu Glu Asp Asp Leu
   675 680 685
   Glu Ala Gly Met Lys Glu Lys Ser Pro Ile Ala Ile Ile Asn Glu Asp
   690 695 700
   Leu Leu Asn Gly Met Lys Thr Val Gly Glu Leu Phe Gly Ser Gly Gln
   705 710 715 720
   Met Gln Leu Pro Phe Val Leu Gln Ser Ala Glu Thr Met Lys Thr Ala
   725 730 735
   Val Ala Tyr Leu Glu Pro Phe Met Glu Glu Glu Ala Glu Ala Thr Gly
   740 745 750
   Ser Ala Gln Ala Glu Gly Lys Gly Lys Ile Val Val Ala Thr Val Lys
   755 760 765
   Gly Asp Val His Asp Ile Gly Lys Asn Leu Val Asp Ile Ile Leu Ser
   770 775 780
   Asn Asn Gly Tyr Asp Val Val Asn Leu Gly Ile Lys Gln Pro Leu Ser
   785 790 795 800
   Ala Met Leu Glu Ala Ala Glu Glu His Lys Ala Asp Val Ile Gly Met
   805 810 815
   Ser Gly Leu Leu Val Lys Ser Thr Val Val Met Lys Gln Thr Ile Ser
   820 825 830
   Asp
<210> 91
   <211> 2578
   <212> DNA
   <213> Corynebacterium glutamicum
<220>
   <221> CDS
   <222> (101)..(2578)
   <223> FRXA02198
<400> 91
   agactagtgg cgctttgcct gtgttgctta ggcggcgttg aaaatgaact acgaatgaaa 60
   agttcgggaa ttgtctaatc cgtactaagc tgtctacaca atg tct act tca gtt   115
                                               Met Ser Thr Ser Val
                                                1               5
   act tca cca gcc cac aac aac gca cat tcc tcc gaa ttt ttg gat gcg   163
   Thr Ser Pro Ala His Asn Asn Ala His Ser Ser Glu Phe Leu Asp Ala
                   10                  15                  20
   ttg gca aac cat gtg ttg atc ggc gac ggc gcc atg ggc acc cag ctc   211
   Leu Ala Asn His Val Leu Ile Gly Asp Gly Ala Met Gly Thr Gln Leu
               25                  30                  35
   caa ggc ttt gac ctg gac gtg gaa aag gat ttc ctt gat ctg gag ggg   259
   Gln Gly Phe Asp Leu Asp Val Glu Lys Asp Phe Leu Asp Leu Glu Gly
           40                  45                  50
   tgt aat gag att ctc aac gac acc cgc cct gat gtg ttg agg cag att   307
   Cys Asn Glu Ile Leu Asn Asp Thr Arg Pro Asp Val Leu Arg Gln Ile
       55                  60                  65
   cac cgc gcc tac ttt gag gcg gga gct gac ttg gtt gag acc aat act   355
   His Arg Ala Tyr Phe Glu Ala Gly Ala Asp Leu Val Glu Thr Asn Thr
   70                  75                  80                  85
   ttt ggt tgc aac ctg ccg aac ttg gcg gat tat gac atc gct gat cgt   403
   Phe Gly Cys Asn Leu Pro Asn Leu Ala Asp Tyr Asp Ile Ala Asp Arg
                   90                  95                  100
   tgc cgt gag ctt gcc tac aag ggc act gca gtg gct agg gaa gtg gct   451
   Cys Arg Glu Leu Ala Tyr Lys Gly Thr Ala Val Ala Arg Glu Val Ala
               105                 110                 115
   gat gag atg ggg ccg ggc cga aac ggc atg cgg cgt ttc gtg gtt ggt   499
   Asp Glu Met Gly Pro Gly Arg Asn Gly Met Arg Arg Phe Val Val Gly
           120                 125                 130
   tcc ctg gga cct gga acg aag ctt cca tcg ctg ggc cat gca ccg tat   547
   Ser Leu Gly Pro Gly Thr Lys Leu Pro Ser Leu Gly His Ala Pro Tyr
       135                 140                 145
   gca gat ttg cgt ggg cac tac aag gaa gca gcg ctt ggc atc atc gac   595
   Ala Asp Leu Arg Gly His Tyr Lys Glu Ala Ala Leu Gly Ile Ile Asp
   150                 155                 160                 165
   ggt ggt ggc gat gcc ttt ttg att gag act gct cag gac ttg ctt cag   643
   Gly Gly Gly Asp Ala Phe Leu Ile Glu Thr Ala Gln Asp Leu Leu Gln
                   170                 175                 180
   gtc aag gct gcg gtt cac ggc gtt caa gat gcc atg gct gaa ctt gat     691
   Val Lys Ala Ala Val His Gly Val Gln Asp Ala Met Ala Glu Leu Asp
               185                 190                 195
   aca ttc ttg ccc att att tgc cac gtc acc gta gag acc acc ggc acc     739
   Thr Phe Leu Pro Ile Ile Cys His Val Thr Val Glu Thr Thr Gly Thr
           200                 205                 210
   atg ctc atg ggt tct gag atc ggt gcc gcg ttg aca gcg ctg cag cca     787
   Met Leu Met Gly Ser Glu Ile Gly Ala Ala Leu Thr Ala Leu Gln Pro
       215                 220                 225
   ctg ggt atc gac atg att ggt ctg aac tgc gcc acc ggc cca gat gag     835
   Leu Gly Ile Asp Met Ile Gly Leu Asn Cys Ala Thr Gly Pro Asp Glu
   230                 235                 240                 245
   atg agc gag cac ctg cgt tac ctg tcc aag cac gcc gat att cct gtg     883
   Met Ser Glu His Leu Arg Tyr Leu Ser Lys His Ala Asp Ile Pro Val
                   250                 255                 260
   tcg gtg atg cct aac gca ggt ctt cct gtc ctg ggt aaa aac ggt gca     931
   Ser Val Met Pro Asn Ala Gly Leu Pro Val Leu Gly Lys Asn Gly Ala
               265                 270                 275
   gaa tac cca ctt gag gct gag gat ttg gcg cag gcg ctg gct gga ttc     979
   Glu Tyr Pro Leu Glu Ala Glu Asp Leu Ala Gln Ala Leu Ala Gly Phe
           280                 285                 290
   gtc tcc gaa tat ggc ctg tcc atg gtg ggt ggt tgt tgt ggc acc aca     1027
   Val Ser Glu Tyr Gly Leu Ser Met Val Gly Gly Cys Cys Gly Thr Thr
       295                 300                 305
   cct gag cac atc cgt gcg gtc cgc gat gcg gtg gtt ggt gtt cca gag     1075
   Pro Glu His Ile Arg Ala Val Arg Asp Ala Val Val Gly Val Pro Glu
   310                 315                 320                 325
   cag gaa acc tcc aca ctg acc aag atc cct gca ggc cct gtt gag cag     1123
   Gln Glu Thr Ser Thr Leu Thr Lys Ile Pro Ala Gly Pro Val Glu Gln
                   330                 335                 340
   gcc tcc cgc gag gtg gag aaa gag gac tcc gtc gcg tcg ctg tac acc     1171
   Ala Ser Arg Glu Val Glu Lys Glu Asp Ser Val Ala Ser Leu Tyr Thr
               345                 350                 355
   tcg gtg cca ttg tcc cag gaa acc ggc att tcc atg atc ggt gag cgc     1219
   Ser Val Pro Leu Ser Gln Glu Thr Gly Ile Ser Met Ile Gly Glu Arg
           360                 365                 370
   acc aac tcc aac ggt tcc aag gca ttc cgt gag gca atg ctg tct ggc     1267
   Thr Asn Ser Asn Gly Ser Lys Ala Phe Arg Glu Ala Met Leu Ser Gly
       375                 380                 385
   gat tgg gaa aag tgt gtg gat att gcc aag cag caa acc cgc gat ggt     1315
   Asp Trp Glu Lys Cys Val Asp Ile Ala Lys Gln Gln Thr Arg Asp Gly
   390                 395                 400                 405
   gca cac atg ctg gat ctt tgt gtg gat tac gtg gga cga gac ggc acc     1363
   Ala His Met Leu Asp Leu Cys Val Asp Tyr Val Gly Arg Asp Gly Thr
                   410                     415                     420
   gcc gat atg gcg acc ttg gca gca ctt ctt gct acc agc tcc act ttg     1411
   Ala Asp Met Ala Thr Leu Ala Ala Leu Leu Ala Thr Ser Ser Thr Leu
               425                     430                     435
   cca atc atg att gac tcc acc gag cca gag gtt att cgc aca ggc ctt     1459
   Pro Ile Met Ile Asp Ser Thr Glu Pro Glu Val Ile Arg Thr Gly Leu
           440                     445                     450
   gag cac ttg ggt gga cga agc atc gtt aac tcc gtc aac ttt gaa gac     1507
   Glu His Leu Gly Gly Arg Ser Ile Val Asn Ser Val Asn Phe Glu Asp
       455                     460                     465
   ggc gat ggc cct gag tcc cgc tac cag cgc atc atg aaa ctg gta aag     1555
   Gly Asp Gly Pro Glu Ser Arg Tyr Gln Arg Ile Met Lys Leu Val Lys
   470                     475                     480                     485
   cag cac ggt gcg gcc gtg gtt gcg ctg acc att gat gag gaa ggc cag     1603
   Gln His Gly Ala Ala Val Val Ala Leu Thr Ile Asp Glu Glu Gly Gln
                   490                     495                     500
   gca cgt acc gct gag cac aag gtg cgc att gct aaa cga ctg att gac     1651
   Ala Arg Thr Ala Glu His Lys Val Arg Ile Ala Lys Arg Leu Ile Asp
               505                     510                     515
   gat atc acc ggc agc tac ggc ctg gat atc aaa gac atc gtt gtg gac     1699
   Asp Ile Thr Gly Ser Tyr Gly Leu Asp Ile Lys Asp Ile Val Val Asp
           520                     525                     530
   tgc ctg acc ttc ccg atc tct act ggc cag gaa gaa acc agg cga gat     1747
   Cys Leu Thr Phe Pro Ile Ser Thr Gly Gln Glu Glu Thr Arg Arg Asp
       535                     540                     545
   ggc att gaa acc atc gaa gcc atc cgc gag ctg aag aag ctc tac cca     1795
   Gly Ile Glu Thr Ile Glu Ala Ile Arg Glu Leu Lys Lys Leu Tyr Pro
   550                     555                     560                     565
   gaa atc cac acc acc ctg ggt ctg tcc aat att tcc ttc ggc ctg aac     1843
   Glu Ile His Thr Thr Leu Gly Leu Ser Asn Ile Ser Phe Gly Leu Asn
                   570                     575                     580
   cct gct gca cgc cag gtt ctt aac tct gtg ttc ctc aat gag tgc att     1891
   Pro Ala Ala Arg Gln Val Leu Asn Ser Val Phe Leu Asn Glu Cys Ile
               585                     590                     595
   gag gct ggt ctg gac tct gcg att gcg cac agc tcc aag att ttg ccg     1939
   Glu Ala Gly Leu Asp Ser Ala Ile Ala His Ser Ser Lys Ile Leu Pro
           600                     605                     610
   atg aac cgc att gat gat cgc cag cgc gaa gtg gcg ttg gat atg gtc     1987
   Met Asn Arg Ile Asp Asp Arg Gln Arg Glu Val Ala Leu Asp Met Val
       615                     620                     625
   tat gat cgc cgc acc gag gat tac gat ccg ctg cag gaa ttc atg cag     2035
   Tyr Asp Arg Arg Thr Glu Asp Tyr Asp Pro Leu Gln Glu Phe Met Gln
   630                     635                     640                     645
   ctg ttt gag ggc gtt tct gct gcc gat gcc aag gat gct cgc gct gaa     2083
   Leu Phe Glu Gly Val Ser Ala Ala Asp Ala Lys Asp Ala Arg Ala Glu
                   650                     655                     660
   cag ctg gcc gct atg cct ttg ttt gag cgt ttg gca cag cgc atc atc     2131
   Gln Leu Ala Ala Met Pro Leu Phe Glu Arg Leu Ala Gln Arg Ile Ile
               665                 670                 675
   gac ggc gat aag aat ggc ctt gag gat gat ctg gaa gca ggc atg aag     2179
   Asp Gly Asp Lys Asn Gly Leu Glu Asp Asp Leu Glu Ala Gly Met Lys
           680                 685                 690
   gag aag tct cct att gcg atc atc aac gag gac ctt ctc aac ggc atg     2227
   Glu Lys Ser Pro Ile Ala Ile Ile Asn Glu Asp Leu Leu Asn Gly Met
       695                 700                 705
   aag acc gtg ggt gag ctg ttt ggt tcc gga cag atg cag ctg cca ttc     2275
   Lys Thr Val Gly Glu Leu Phe Gly Ser Gly Gln Met Gln Leu Pro Phe
   710                 715                 720                 725
   gtg ctg caa tcg gca gaa acc atg aaa act gcg gtg gcc tat ttg gaa     2323
   Val Leu Gln Ser Ala Glu Thr Met Lys Thr Ala Val Ala Tyr Leu Glu
                   730                 735                 740
   ccg ttc atg gaa gag gaa gca gaa gct acc gga tct gcg cag gca gag     2371
   Pro Phe Met Glu Glu Glu Ala Glu Ala Thr Gly Ser Ala Gln Ala Glu
               745                 750                 755
   ggc aag ggc aaa atc gtc gtg gcc acc gtc aag ggt gac gtg cac gat     2419
   Gly Lys Gly Lys Ile Val Val Ala Thr Val Lys Gly Asp Val His Asp
           760                 765                 770
   atc ggc aag aac ttg gtg gac atc att ttg tcc aac aac ggt tac gac     2467
   Ile Gly Lys Asn Leu Val Asp Ile Ile Leu Ser Asn Asn Gly Tyr Asp
       775                 780                 785
   gtg gtg aac ttg ggc atc aag cag cca ctg tcc gcc atg ttg gaa gca     2515
   Val Val Asn Leu Gly Ile Lys Gln Pro Leu Ser Ala Met Leu Glu Ala
   790                 795                 800                 805
   gcg gaa gaa cac aaa gca gac gtc atc ggc atg tcg gga ctt ctt gtg     2563
   Ala Glu Glu His Lys Ala Asp Val Ile Gly Met Ser Gly Leu Leu Val
                   810                 815                 820
   aag tcc acc gtg gtg                                                 2578
   Lys Ser Thr Val Val
               825
<210> 92
   <211> 826
   <212> PRT
   <213> Corynebacterium glutamicum
<400> 92
   Met Ser Thr Ser Val Thr Ser Pro Ala His Asn Asn Ala His Ser Ser
    1               5                  10                  15
   Glu Phe Leu Asp Ala Leu Ala Asn His Val Leu Ile Gly Asp Gly Ala
               20                  25                  30
   Met Gly Thr Gln Leu Gln Gly Phe Asp Leu Asp Val Glu Lys Asp Phe
           35                  40                  45
   Leu Asp Leu Glu Gly Cys Asn Glu Ile Leu Asn Asp Thr Arg Pro Asp
        50                  55                  60
   Val Leu Arg Gln Ile His Arg Ala Tyr Phe Glu Ala Gly Ala Asp Leu
   65                  70                  75                  80
   Val Glu Thr Asn Thr Phe Gly Cys Asn Leu Pro Asn Leu Ala Asp Tyr
                   85                  90                  95
   Asp Ile Ala Asp Arg Cys Arg Glu Leu Ala Tyr Lys Gly Thr Ala Val
               100                 105                 110
   Ala Arg Glu Val Ala Asp Glu Met Gly Pro Gly Arg Asn Gly Met Arg
           115                 120                 125
   Arg Phe Val Val Gly Ser Leu Gly Pro Gly Thr Lys Leu Pro Ser Leu
       130                 135                 140
   Gly His Ala Pro Tyr Ala Asp Leu Arg Gly His Tyr Lys Glu Ala Ala
   145                 150                 155                 160
   Leu Gly Ile Ile Asp Gly Gly Gly Asp Ala Phe Leu Ile Glu Thr Ala
                   165                 170                 175
   Gln Asp Leu Leu Gln Val Lys Ala Ala Val His Gly Val Gln Asp Ala
               180                 185                 190
   Met Ala Glu Leu Asp Thr Phe Leu Pro Ile Ile Cys His Val Thr Val
           195                 200                 205
   Glu Thr Thr Gly Thr Met Leu Met Gly Ser Glu Ile Gly Ala Ala Leu
       210                 215                 220
   Thr Ala Leu Gln Pro Leu Gly Ile Asp Met Ile Gly Leu Asn Cys Ala
   225                 230                 235                 240
   Thr Gly Pro Asp Glu Met Ser Glu His Leu Arg Tyr Leu Ser Lys His
                   245                 250                 255
   Ala Asp Ile Pro Val Ser Val Met Pro Asn Ala Gly Leu Pro Val Leu
               260                 265                 270
   Gly Lys Asn Gly Ala Glu Tyr Pro Leu Glu Ala Glu Asp Leu Ala Gln
           275                 280                 285
   Ala Leu Ala Gly Phe Val Ser Glu Tyr Gly Leu Ser Met Val Gly Gly
       290                 295                 300
   Cys Cys Gly Thr Thr Pro Glu His Ile Arg Ala Val Arg Asp Ala Val
   305                 310                 315                 320
   Val Gly Val Pro Glu Gln Glu Thr Ser Thr Leu Thr Lys Ile Pro Ala
                   325                 330                 335
   Gly Pro Val Glu Gln Ala Ser Arg Glu Val Glu Lys Glu Asp Ser Val
               340                 345                 350
   Ala Ser Leu Tyr Thr Ser Val Pro Leu Ser Gln Glu Thr Gly Ile Ser
           355                 360                 365
   Met Ile Gly Glu Arg Thr Asn Ser Asn Gly Ser Lys Ala Phe Arg Glu
           370                 375                 380
   Ala Met Leu Ser Gly Asp Trp Glu Lys Cys Val Asp Ile Ala Lys Gln
   385                 390                 395                 400
   Gln Thr Arg Asp Gly Ala His Met Leu Asp Leu Cys Val Asp Tyr Val
                   405                 410                 415
   Gly Arg Asp Gly Thr Ala Asp Met Ala Thr Leu Ala Ala Leu Leu Ala
               420                 425                 430
   Thr Ser Ser Thr Leu Pro Ile Met Ile Asp Ser Thr Glu Pro Glu Val
           435                 440                 445
   Ile Arg Thr Gly Leu Glu His Leu Gly Gly Arg Ser Ile Val Asn Ser
       450                 455                 460
   Val Asn Phe Glu Asp Gly Asp Gly Pro Glu Ser Arg Tyr Gln Arg Ile
   465                 470                 475                 480
   Met Lys Leu Val Lys Gln His Gly Ala Ala Val Val Ala Leu Thr Ile
                   485                 490                 495
   Asp Glu Glu Gly Gln Ala Arg Thr Ala Glu His Lys Val Arg Ile Ala
               500                 505                 510
   Lys Arg Leu Ile Asp Asp Ile Thr Gly Ser Tyr Gly Leu Asp Ile Lys
           515                 520                 525
   Asp Ile Val Val Asp Cys Leu Thr Phe Pro Ile Ser Thr Gly Gln Glu
       530                 535                 540
   Glu Thr Arg Arg Asp Gly Ile Glu Thr Ile Glu Ala Ile Arg Glu Leu
   545                 550                 555                 560
   Lys Lys Leu Tyr Pro Glu Ile His Thr Thr Leu Gly Leu Ser Asn Ile
                   565                 570                 575
   Ser Phe Gly Leu Asn Pro Ala Ala Arg Gln Val Leu Asn Ser Val Phe
               580                 585                 590
   Leu Asn Glu Cys Ile Glu Ala Gly Leu Asp Ser Ala Ile Ala His Ser
           595                 600                 605
   Ser Lys Ile Leu Pro Met Asn Arg Ile Asp Asp Arg Gln Arg Glu Val
       610                 615                 620
   Ala Leu Asp Met Val Tyr Asp Arg Arg Thr Glu Asp Tyr Asp Pro Leu
   625                 630                 635                 640
   Gln Glu Phe Met Gln Leu Phe Glu Gly Val Ser Ala Ala Asp Ala Lys
                   645                 650                 655
   Asp Ala Arg Ala Glu Gln Leu Ala Ala Met Pro Leu Phe Glu Arg Leu
               660                 665                 670
   Ala Gln Arg Ile Ile Asp Gly Asp Lys Asn Gly Leu Glu Asp Asp Leu
           675                 680                 685
   Glu Ala Gly Met Lys Glu Lys Ser Pro Ile Ala Ile Ile Asn Glu Asp
       690                 695                 700
   Leu Leu Asn Gly Met Lys Thr Val Gly Glu Leu Phe Gly Ser Gly Gln
   705                 710                 715                 720
   Met Gln Leu Pro Phe Val Leu Gln Ser Ala Glu Thr Met Lys Thr Ala
                   725                 730                 735
   Val Ala Tyr Leu Glu Pro Phe Met Glu Glu Glu Ala Glu Ala Thr Gly
               740                 745                 750
   Ser Ala Gln Ala Glu Gly Lys Gly Lys Ile Val Val Ala Thr Val Lys
           755                 760                 765
   Gly Asp Val His Asp Ile Gly Lys Asn Leu Val Asp Ile Ile Leu Ser
       770                 775                 780
   Asn Asn Gly Tyr Asp Val Val Asn Leu Gly Ile Lys Gln Pro Leu Ser
   785                 790                 795                 800
   Ala Met Leu Glu Ala Ala Glu Glu His Lys Ala Asp Val Ile Gly Met
                   805                 810                 815
   Ser Gly Leu Leu Val Lys Ser Thr Val Val
               820                 825
<210> 93
   <211> 621
   <212> DNA
   <213> Corynebacterium glutamicum
<220>
   <221> CDS
   <222> (101)..(598)
   <223> RXN03074
<400> 93
   tttgtgggca atctggtttt ttcgtaattg tgtgggatga atctcttaaa aattcacatt 60
   tagcaggaca agcatactgt tttagttcta tgctgtgggc atg act caa agt gct   115
                                               Met Thr Gln Ser Ala
                                               1               5
   cca gaa ttc att gcc acc gca gac ctc gta gac atc atc ggc gac aac   163
   Pro Glu Phe Ile Ala Thr Ala Asp Leu Val Asp Ile Ile Gly Asp Asn
                   10                  15                  20
   gcg caa tca tgc gac act cag ttt caa aac ctt gga ggt gcc aca gaa   211
   Ala Gln Ser Cys Asp Thr Gln Phe Gln Asn Leu Gly Gly Ala Thr Glu
               25                  30                  35
   ttc cac gga ata ata acc acc gtg aaa tgc ttc caa gac aac gcc ctc   259
   Phe His Gly Ile Ile Thr Thr Val Lys Cys Phe Gln Asp Asn Ala Leu
           40                  45                  50
   ctg aaa tcc atc ctg agc gag gat aat cct ggg gga gtg ctg gtt atc   307
   Leu Lys Ser Ile Leu Ser Glu Asp Asn Pro Gly Gly Val Leu Val Ile
       55                  60                  65
   gat ggc gac gca tcc gtg cac acc gcg cta gtt ggc gac atc att gca   355
   Asp Gly Asp Ala Ser Val His Thr Ala Leu Val Gly Asp Ile Ile Ala
    70                  75                  80                  85
   gga ctt gga aaa gat cat ggt tgg tcc gga gta att gtc aac gga gca     403
   Gly Leu Gly Lys Asp His Gly Trp Ser Gly Val Ile Val Asn Gly Ala
                    90                  95                  100
   att cga gac tcc gca gtc atc ggc acc atg acc ttt ggt tgt aaa gcc     451
   Ile Arg Asp Ser Ala Val Ile Gly Thr Met Thr Phe Gly Cys Lys Ala
               105                 110                 115
   ctt gga acc aac ccg cgg aaa tcc act aaa act ggt tcc ggc gaa cga     499
   Leu Gly Thr Asn Pro Arg Lys Ser Thr Lys Thr Gly Ser Gly Glu Arg
           120                 125                 130
   gac gta gtg gta tcg att ggt ggc att gac ttc att cct ggt cat tac     547
   Asp Val Val Val Ser Ile Gly Gly Ile Asp Phe Ile Pro Gly His Tyr
       135                 140                 145
   gtc tac gcg gac tct gac gga att atc gtc acc gag gcg cca att aag     595
   Val Tyr Ala Asp Ser Asp Gly Ile Ile Val Thr Glu Ala Pro Ile Lys
   150                 155                 160                 165
   cag taatttgttt tgacgacgca gta                                       621
   Gln
<210> 94
   <211> 166
   <212> PRT
   <213> Corynebacterium glutamicum
<400> 94
   Met Thr Gln Ser Ala Pro Glu Phe Ile Ala Thr Ala Asp Leu Val Asp
   1 5 10 15
   Ile Ile Gly Asp Asn Ala Gln Ser Cys Asp Thr Gln Phe Gln Asn Leu
   20 25 30
   Gly Gly Ala Thr Glu Phe His Gly Ile Ile Thr Thr Val Lys Cys Phe
   35 40 45
   Gln Asp Asn Ala Leu Leu Lys Ser Ile Leu Ser Glu Asp Asn Pro Gly
   50 55 60
   Gly Val Leu Val Ile Asp Gly Asp Ala Ser Val His Thr Ala Leu Val
   65 70 75 80
   Gly Asp Ile Ile Ala Gly Leu Gly Lys Asp His Gly Trp Ser Gly Val
   85 90 95
   Ile Val Asn Gly Ala Ile Arg Asp Ser Ala Val Ile Gly Thr Met Thr
   100 105 110
   Phe Gly Cys Lys Ala Leu Gly Thr Asn Pro Arg Lys Ser Thr Lys Thr
   115 120 125
   Gly Ser Gly Glu Arg Asp Val Val Val Ser Ile Gly Gly Ile Asp Phe
   130 135 140
   Ile Pro Gly His Tyr Val Tyr Ala Asp Ser Asp Gly Ile Ile Val Thr
   145 150 155 160
   Glu Ala Pro Ile Lys Gln
   165
<210> 95
   <211> 621
   <212> DNA
   <213> Corynebacterium glutamicum
<220>
   <221> CDS
   <222> (101)..(598)
   <223> FRXA02906
<400> 95
   tttgtgggca atctggtttt ttcgtaattg tgtgggatga atctcttaaa aattcacatt 60
   tagcaggaca agcatactgt tttagttcta tgctgtgggc atg act caa agt gct   115
                                               Met Thr Gln Ser Ala
                                               1               5
   cca gaa ttc att gcc acc gca gac ctc gta gac atc atc ggc gac aac   163
   Pro Glu Phe Ile Ala Thr Ala Asp Leu Val Asp Ile Ile Gly Asp Asn
                   10                  15                  20
   gcg caa tca tgc gac act cag ttt caa aac ctt gga ggt gcc aca gaa   211
   Ala Gln Ser Cys Asp Thr Gln Phe Gln Asn Leu Gly Gly Ala Thr Glu
               25                  30                  35
   ttc cac gga ata ata acc acc gtg aaa tgc ttc caa gac aac gcc ctc   259
   Phe His Gly Ile Ile Thr Thr Val Lys Cys Phe Gln Asp Asn Ala Leu
           40                  45                  50
   ctg aaa tcc atc ctg agc gag gat aat cct ggg gga gtg ctg gtt atc   307
   Leu Lys Ser Ile Leu Ser Glu Asp Asn Pro Gly Gly Val Leu Val Ile
       55                  60                  65
   gat ggc gac gca tcc gtg cac acc gcg cta gtt ggc gac atc att gca   355
   Asp Gly Asp Ala Ser Val His Thr Ala Leu Val Gly Asp Ile Ile Ala
   70                  75                  80                  85
   gga ctt gga aaa gat cat ggt tgg tcc gga gta att gtc aac gga gca   403
   Gly Leu Gly Lys Asp His Gly Trp Ser Gly Val Ile Val Asn Gly Ala
                   90                  95                  100
   att cga gac tcc gca gtc atc ggc acc atg acc ttt ggt tgt aaa gcc   451
   Ile Arg Asp Ser Ala Val Ile Gly Thr Met Thr Phe Gly Cys Lys Ala
               105                 110                 115
   ctt gga acc aac ccg cgg aaa tcc act aaa act ggt tcc ggc gaa cga   499
   Leu Gly Thr Asn Pro Arg Lys Ser Thr Lys Thr Gly Ser Gly Glu Arg
           120                 125                 130
   gac gta gtg gta tcg att ggt ggc att gac ttc att cct ggt cat tac   547
   Asp Val Val Val Ser Ile Gly Gly Ile Asp Phe Ile Pro Gly His Tyr
       135                 140                 145
   gtc tac gcg gac tct gac gga att atc gtc acc gag gcg cca att aag   595
   Val Tyr Ala Asp Ser Asp Gly Ile Ile Val Thr Glu Ala Pro Ile Lys
   150                 155                 160                 165
   cag taatttgttt tgacgacgca gta                                     621
   Gln
<210> 96
   <211> 166
   <212> PRT
   <213> Corynebacterium glutamicum
<400> 96
   Met Thr Gln Ser Ala Pro Glu Phe Ile Ala Thr Ala Asp Leu Val Asp
    1               5                  10                  15
   Ile Ile Gly Asp Asn Ala Gln Ser Cys Asp Thr Gln Phe Gln Asn Leu
               20                  25                  30
   Gly Gly Ala Thr Glu Phe His Gly Ile Ile Thr Thr Val Lys Cys Phe
           35                  40                  45
   Gln Asp Asn Ala Leu Leu Lys Ser Ile Leu Ser Glu Asp Asn Pro Gly
       50                  55                  60
   Gly Val Leu Val Ile Asp Gly Asp Ala Ser Val His Thr Ala Leu Val
   65                  70                  75                  80
   Gly Asp Ile Ile Ala Gly Leu Gly Lys Asp His Gly Trp Ser Gly Val
                   85                  90                  95
   Ile Val Asn Gly Ala Ile Arg Asp Ser Ala Val Ile Gly Thr Met Thr
               100                 105                 110
   Phe Gly Cys Lys Ala Leu Gly Thr Asn Pro Arg Lys Ser Thr Lys Thr
           115                 120                 125
   Gly Ser Gly Glu Arg Asp Val Val Val Ser Ile Gly Gly Ile Asp Phe
       130                 135                 140
   Ile Pro Gly His Tyr Val Tyr Ala Asp Ser Asp Gly Ile Ile Val Thr
   145                 150                 155                 160
   Glu Ala Pro Ile Lys Gln
                   165
<210> 97
   <211> 1557
   <212> DNA
   <213> Corynebacterium glutamicum
<220>
   <221> CDS
   <222> (101)..(1534)
   <223> RXN00132
<400> 97
   aacagcttca atcaattcgg tgtccactcc aacatgtaga gtggtgcgcg ttaaaaaagt 60
   tttcctaatt ttcattttct taaaaggagc tcgccaggac atg gca cag gtt atg   115
   Met Ala Gln Val Met
   1 5
   gac ttc aag gtt gcc gat ctt tca cta gca gag gca gga cgt cac cag 163
   Asp Phe Lys Val Ala Asp Leu Ser Leu Ala Glu Ala Gly Arg His Gln
   10 15 20
   att cgt ctt gca gag tat gag atg cca ggt ctc atg cag ttg cgc aag 211
   Ile Arg Leu Ala Glu Tyr Glu Met Pro Gly Leu Met Gln Leu Arg Lys
   25 30 35
   gaa ttc gca gac gag cag cct ttg aag ggc gcc cga att gct ggt tct 259
   Glu Phe Ala Asp Glu Gln Pro Leu Lys Gly Ala Arg Ile Ala Gly Ser
   40 45 50
   atc cac atg acg gtc cag acc gcc gtg ctt att gag acc ctc act gct 307
   Ile His Met Thr Val Gln Thr Ala Val Leu Ile Glu Thr Leu Thr Ala
   55 60 65
   ttg ggc gct gag gtt cgt tgg gct tcc tgc aac att ttc tcc acc cag 355
   Leu Gly Ala Glu Val Arg Trp Ala Ser Cys Asn Ile Phe Ser Thr Gln
   70 75 80 85
   gat gag gct gca gcg gct atc gtt gtc ggc tcc ggc acc gtc gaa gag 403
   Asp Glu Ala Ala Ala Ala Ile Val Val Gly Ser Gly Thr Val Glu Glu
   90 95 100
   cca gct ggt gtt cca gta ttc gcg tgg aag ggt gag tca ctg gag gag 451
   Pro Ala Gly Val Pro Val Phe Ala Trp Lys Gly Glu Ser Leu Glu Glu
   105 110 115
   tac tgg tgg tgc atc aac cag atc ttc agc tgg ggc gat gag ctg cca 499
   Tyr Trp Trp Cys Ile Asn Gln Ile Phe Ser Trp Gly Asp Glu Leu Pro
   120 125 130
   aac atg atc ctc gac gac ggc ggt gac gcc acc atg gct gtt att cgc 547
   Asn Met Ile Leu Asp Asp Gly Gly Asp Ala Thr Met Ala Val Ile Arg
   135 140 145
   ggt cgc gaa tac gag cag gct ggt ctg gtt cca cca gca gag gcc aac 595
   Gly Arg Glu Tyr Glu Gln Ala Gly Leu Val Pro Pro Ala Glu Ala Asn
   150 155 160 165
   gat tcc gat gag tac atc gca ttc ttg ggc atg ctg cgt gag gtt ctt 643
   Asp Ser Asp Glu Tyr Ile Ala Phe Leu Gly Met Leu Arg Glu Val Leu
   170 175 180
   gct gca gag cct ggc aag tgg ggc aag atc gct gag gcc gtt aag ggt 691
   Ala Ala Glu Pro Gly Lys Trp Gly Lys Ile Ala Glu Ala Val Lys Gly
   185 190 195
   gtc acc gag gaa acc acc acc ggt gtg cac cgc ctg tac cac ttc gct 739
   Val Thr Glu Glu Thr Thr Thr Gly Val His Arg Leu Tyr His Phe Ala
   200 205 210
   gaa gaa ggc gtg ctg cct ttc cca gcg atg aac gtc aac gac gct gtc 787
   Glu Glu Gly Val Leu Pro Phe Pro Ala Met Asn Val Asn Asp Ala Val
   215 220 225
   acc aag tcc aag ttt gat aac aag tac ggc acc cgc cac tcc ctg atc 835
   Thr Lys Ser Lys Phe Asp Asn Lys Tyr Gly Thr Arg His Ser Leu Ile
   230                 235                 240                 245
   gac ggc atc aac cgc gcc act gac atg ctc atg ggc ggc aag aac gtg     883
   Asp Gly Ile Asn Arg Ala Thr Asp Met Leu Met Gly Gly Lys Asn Val
                   250                 255                 260
   ctt gtc tgc ggt tac ggc gat gtc ggc aag ggc tgc gct gag gct ttc     931
   Leu Val Cys Gly Tyr Gly Asp Val Gly Lys Gly Cys Ala Glu Ala Phe
               265                 270                 275
   gac ggc cag ggc gct cgc gtc aag gtc acc gaa gct gac cca atc aac     979
   Asp Gly Gln Gly Ala Arg Val Lys Val Thr Glu Ala Asp Pro Ile Asn
           280                 285                 290
   gct ctt cag gct ctg atg gat ggc tac tct gtg gtc acc gtt gat gag    1027
   Ala Leu Gln Ala Leu Met Asp Gly Tyr Ser Val Val Thr Val Asp Glu
       295                 300                 305
   gcc atc gag gac gcc gac atc gtg atc acc gcg acc ggc aac aag gac    1075
   Ala Ile Glu Asp Ala Asp Ile Val Ile Thr Ala Thr Gly Asn Lys Asp
   310                 315                 320                 325
   atc att tcc ttc gag cag atg ctc aag atg aag gat cac gct ctg ctg    1123
   Ile Ile Ser Phe Glu Gln Met Leu Lys Met Lys Asp His Ala Leu Leu
                   330                 335                 340
   ggc aac atc ggt cac ttt gat aat gag atc gat atg cat tcc ctg ttg    1171
   Gly Asn Ile Gly His Phe Asp Asn Glu Ile Asp Met His Ser Leu Leu
               345                 350                 355
   cac cgc gac gac gtc acc cgc acc acg atc aag cca cag gtc gac gag    1219
   His Arg Asp Asp Val Thr Arg Thr Thr Ile Lys Pro Gln Val Asp Glu
           360                 365                 370
   ttc acc ttc tcc acc ggt cgc tcc atc atc gtc ctg tcc gaa ggt cgc    1267
   Phe Thr Phe Ser Thr Gly Arg Ser Ile Ile Val Leu Ser Glu Gly Arg
       375                 380                 385
   ctg ttg aac ctt ggc aac gcc acc gga cac cca tca ttt gtc atg tcc    1315
   Leu Leu Asn Leu Gly Asn Ala Thr Gly His Pro Ser Phe Val Met Ser
   390                 395                 400                 405
   aac tct ttc gcc gat cag acc att gcg cag atc gaa ctg ttc caa aac    1363
   Asn Ser Phe Ala Asp Gln Thr Ile Ala Gln Ile Glu Leu Phe Gln Asn
                   410                 415                 420
   gaa gga cag tac gag aac gag gtc tac cgt ctg cct aag gtt ctc gac    1411
   Glu Gly Gln Tyr Glu Asn Glu Val Tyr Arg Leu Pro Lys Val Leu Asp
               425                 430                 435
   gaa aag gtg gca cgc atc cac gtt gag gct ctc ggc ggt cag ctc acc    1459
   Glu Lys Val Ala Arg Ile His Val Glu Ala Leu Gly Gly Gln Leu Thr
           440                 445                 450
   gaa ctg acc aag gag cag gct gag tac atc ggc gtt gac gtt gca ggc    1507
   Glu Leu Thr Lys Glu Gln Ala Glu Tyr Ile Gly Val Asp Val Ala Gly
       455                 460                 465
   cca ttc aag ccg gag cac tac cgc tac taatgattgt cagcattgag gga      1557
   Pro Phe Lys Pro Glu His Tyr Arg Tyr
   470                 475
<210> 98
   <211> 478
   <212> PRT
   <213> Corynebacterium glutamicum
<400> 98
   Met Ala Gln Val Met Asp Phe Lys Val Ala Asp Leu Ser Leu Ala Glu
    1               5                  10                  15
   Ala Gly Arg His Gln Ile Arg Leu Ala Glu Tyr Glu Met Pro Gly Leu
               20                  25                  30
   Met Gln Leu Arg Lys Glu Phe Ala Asp Glu Gln Pro Leu Lys Gly Ala
           35                  40                  45
   Arg Ile Ala Gly Ser Ile His Met Thr Val Gln Thr Ala Val Leu Ile
       50                  55                  60
   Glu Thr Leu Thr Ala Leu Gly Ala Glu Val Arg Trp Ala Ser Cys Asn
   65                  70                  75                  80
   Ile Phe Ser Thr Gln Asp Glu Ala Ala Ala Ala Ile Val Val Gly Ser
                   85                  90                  95
   Gly Thr Val Glu Glu Pro Ala Gly Val Pro Val Phe Ala Trp Lys Gly
               100                 105                 110
   Glu Ser Leu Glu Glu Tyr Trp Trp Cys Ile Asn Gln Ile Phe Ser Trp
           115                 120                 125
   Gly Asp Glu Leu Pro Asn Met Ile Leu Asp Asp Gly Gly Asp Ala Thr
       130                 135                 140
   Met Ala Val Ile Arg Gly Arg Glu Tyr Glu Gln Ala Gly Leu Val Pro
   145                 150                 155                 160
   Pro Ala Glu Ala Asn Asp Ser Asp Glu Tyr Ile Ala Phe Leu Gly Met
                   165                 170                 175
   Leu Arg Glu Val Leu Ala Ala Glu Pro Gly Lys Trp Gly Lys Ile Ala
               180                 185                 190
   Glu Ala Val Lys Gly Val Thr Glu Glu Thr Thr Thr Gly Val His Arg
           195                 200                 205
   Leu Tyr His Phe Ala Glu Glu Gly Val Leu Pro Phe Pro Ala Met Asn
       210                 215                 220
   Val Asn Asp Ala Val Thr Lys Ser Lys Phe Asp Asn Lys Tyr Gly Thr
   225                 230                 235                 240
   Arg His Ser Leu Ile Asp Gly Ile Asn Arg Ala Thr Asp Met Leu Met
                   245                 250                 255
   Gly Gly Lys Asn Val Leu Val Cys Gly Tyr Gly Asp Val Gly Lys Gly
               260                 265                 270
   Cys Ala Glu Ala Phe Asp Gly Gln Gly Ala Arg Val Lys Val Thr Glu
           275                 280                 285
   Ala Asp Pro Ile Asn Ala Leu Gln Ala Leu Met Asp Gly Tyr Ser Val
       290                 295                 300
   Val Thr Val Asp Glu Ala Ile Glu Asp Ala Asp Ile Val Ile Thr Ala
   305                 310                 315                 320
   Thr Gly Asn Lys Asp Ile Ile Ser Phe Glu Gln Met Leu Lys Met Lys
                   325                 330                 335
   Asp His Ala Leu Leu Gly Asn Ile Gly His Phe Asp Asn Glu Ile Asp
               340                 345                 350
   Met His Ser Leu Leu His Arg Asp Asp Val Thr Arg Thr Thr Ile Lys
           355                 360                 365
   Pro Gln Val Asp Glu Phe Thr Phe Ser Thr Gly Arg Ser Ile Ile Val
       370                 375                 380
   Leu Ser Glu Gly Arg Leu Leu Asn Leu Gly Asn Ala Thr Gly His Pro
   385                 390                 395                 400
   Ser Phe Val Met Ser Asn Ser Phe Ala Asp Gln Thr Ile Ala Gln Ile
                   405                 410                 415
   Glu Leu Phe Gln Asn Glu Gly Gln Tyr Glu Asn Glu Val Tyr Arg Leu
               420                 425                 430
   Pro Lys Val Leu Asp Glu Lys Val Ala Arg Ile His Val Glu Ala Leu
           435                 440                 445
   Gly Gly Gln Leu Thr Glu Leu Thr Lys Glu Gln Ala Glu Tyr Ile Gly
       450                 455                 460
   Val Asp Val Ala Gly Pro Phe Lys Pro Glu His Tyr Arg Tyr
   465                 470                 475
<210> 99
   <211> 128
   <212> DNA
   <213> Corynebacterium glutamicum
<220>
   <221> CDS
   <222> (1)..(105)
   <223> FRXA00132
<400> 99
   cac gtt gag gct ctc ggc ggt cag ctc acc gaa ctg acc aag gag cag      48
   His Val Glu Ala Leu Gly Gly Gln Leu Thr Glu Leu Thr Lys Glu Gln
     1               5                  10                  15
   gct gag tac atc ggc gtt gac gtt gca ggc cca ttc aag ccg gag cac      96
   Ala Glu Tyr Ile Gly Val Asp Val Ala Gly Pro Phe Lys Pro Glu His
                20                  25                  30
   tac cgc tac taatgattgt cagcattgag gga                                128
   Tyr Arg Tyr
            35
<210> 101
   <211> 1396
   <212> DNA
   <213> Corynebacterium glutamicum
<220>
   <221> CDS
   <222> (101)..(1396)
   <223> FRXA01371
<400> 101
   aacagcttca atcaattcgg tgtccactcc aacatgtaga gtggtgcgcg ttaaaaaagt 60
   tttcctaatt ttcattttct taaaaggagc tcgccaggac atg gca cag gtt atg   115
                                             Met Ala Gln Val Met
                                              1               5
   gac ttc aag gtt gcc gat ctt tca cta gca gag gca gga cgt cac cag   163
   Asp Phe Lys Val Ala Asp Leu Ser Leu Ala Glu Ala Gly Arg His Gln
                   10                  15                  20
   att cgt ctt gca gag tat gag atg cca ggt ctc atg cag ttg cgc aag   211
   Ile Arg Leu Ala Glu Tyr Glu Met Pro Gly Leu Met Gln Leu Arg Lys
               25                  30                  35
   gaa ttc gca gac gag cag cct ttg aag ggc gcc cga att gct ggt tct   259
   Glu Phe Ala Asp Glu Gln Pro Leu Lys Gly Ala Arg Ile Ala Gly Ser
           40                  45                  50
   atc cac atg acg gtc cag acc gcc gtg ctt att gag acc ctc act gct   307
   Ile His Met Thr Val Gln Thr Ala Val Leu Ile Glu Thr Leu Thr Ala
       55                  60                  65
   ttg ggc gct gag gtt cgt tgg gct tcc tgc aac att ttc tcc acc cag   355
   Leu Gly Ala Glu Val Arg Trp Ala Ser Cys Asn Ile Phe Ser Thr Gln
   70                  75                  80                  85
   gat gag gct gca gcg gct atc gtt gtc ggc tcc ggc acc gtc gaa gag   403
   Asp Glu Ala Ala Ala Ala Ile Val Val Gly Ser Gly Thr Val Glu Glu
                   90                  95                  100
   cca gct ggt gtt cca gta ttc gcg tgg aag ggt gag tca ctg gag gag   451
   Pro Ala Gly Val Pro Val Phe Ala Trp Lys Gly Glu Ser Leu Glu Glu
               105                 110                 115
   tac tgg tgg tgc atc aac cag atc ttc agc tgg ggc gat gag ctg cca      499
   Tyr Trp Trp Cys Ile Asn Gln Ile Phe Ser Trp Gly Asp Glu Leu Pro
           120                 125                 130
   aac atg atc ctc gac gac ggc ggt gac gcc acc atg gct gtt att cgc      547
   Asn Met Ile Leu Asp Asp Gly Gly Asp Ala Thr Met Ala Val Ile Arg
       135                 140                 145
   ggt cgc gaa tac gag cag gct ggt ctg gtt cca cca gca gag gcc aac      595
   Gly Arg Glu Tyr Glu Gln Ala Gly Leu Val Pro Pro Ala Glu Ala Asn
   150                 155                 160                 165
   gat tcc gat gag tac atc gca ttc ttg ggc atg ctg cgt gag gtt ctt      643
   Asp Ser Asp Glu Tyr Ile Ala Phe Leu Gly Met Leu Arg Glu Val Leu
                   170                 175                 180
   gct gca gag cct ggc aag tgg ggc aag atc gct gag gcc gtt aag ggt      691
   Ala Ala Glu Pro Gly Lys Trp Gly Lys Ile Ala Glu Ala Val Lys Gly
               185                 190                 195
   gtc acc gag gaa acc acc acc ggt gtg cac cgc ctg tac cac ttc gct      739
   Val Thr Glu Glu Thr Thr Thr Gly Val His Arg Leu Tyr His Phe Ala
           200                 205                 210
   gaa gaa ggc gtg ctg cct ttc cca gcg atg aac gtc aac gac gct gtc      787
   Glu Glu Gly Val Leu Pro Phe Pro Ala Met Asn Val Asn Asp Ala Val
       215                 220                 225
   acc aag tcc aag ttt gat aac aag tac ggc acc cgc cac tcc ctg atc      835
   Thr Lys Ser Lys Phe Asp Asn Lys Tyr Gly Thr Arg His Ser Leu Ile
   230                 235                 240                 245
   gac ggc atc aac cgc gcc act gac atg ctc atg ggc ggc aag aac gtg      883
   Asp Gly Ile Asn Arg Ala Thr Asp Met Leu Met Gly Gly Lys Asn Val
                   250                 255                 260
   ctt gtc tgc ggt tac ggc gat gtc ggc aag ggc tgc gct gag gct ttc      931
   Leu Val Cys Gly Tyr Gly Asp Val Gly Lys Gly Cys Ala Glu Ala Phe
               265                 270                 275
   gac ggc cag ggc gct cgc gtc aag gtc acc gaa gct gac cca atc aac      979
   Asp Gly Gln Gly Ala Arg Val Lys Val Thr Glu Ala Asp Pro Ile Asn
           280                 285                 290
   gct ctt cag gct ctg atg gat ggc tac tct gtg gtc acc gtt gat gag      1027
   Ala Leu Gln Ala Leu Met Asp Gly Tyr Ser Val Val Thr Val Asp Glu
       295                 300                 305
   gcc atc gag gac gcc gac atc gtg atc acc gcg acc ggc aac aag gac      1075
   Ala Ile Glu Asp Ala Asp Ile Val Ile Thr Ala Thr Gly Asn Lys Asp
   310                 315                 320                 325
   atc att tcc ttc gag cag atg ctc aag atg aag gat cac gct ctg ctg      1123
   Ile Ile Ser Phe Glu Gln Met Leu Lys Met Lys Asp His Ala Leu Leu
                   330                 335                 340
   ggc aac atc ggt cac ttt gat aat gag atc gat atg cat tcc ctg ttg      1171
   Gly Asn Ile Gly His Phe Asp Asn Glu Ile Asp Met His Ser Leu Leu
               345                 350                 355
   cac cgc gac gac gtc acc cgc acc acg atc aag cca cag gtc gac gag    1219
   His Arg Asp Asp Val Thr Arg Thr Thr Ile Lys Pro Gln Val Asp Glu
           360                 365                 370
   ttc acc ttc tcc acc ggt cgc tcc atc atc gtc ctg tcc gaa ggt cgc    1267
   Phe Thr Phe Ser Thr Gly Arg Ser Ile Ile Val Leu Ser Glu Gly Arg
       375                 380                 385
   ctg ttg aac ctt ggc aac gcc acc gga cac cca tca ttt gtc atg tcc    1315
   Leu Leu Asn Leu Gly Asn Ala Thr Gly His Pro Ser Phe Val Met Ser
   390                 395                 400                 405
   aac tct ttc gcc gat cag acc att gcg cag atc gaa ctg ttc caa aac    1363
   Asn Ser Phe Ala Asp Gln Thr Ile Ala Gln Ile Glu Leu Phe Gln Asn
                   410                 415                 420
   gaa gga cag tac gag aac gag gtc tac cgt ctg                        1396
   Glu Gly Gln Tyr Glu Asn Glu Val Tyr Arg Leu
               425                 430
<210> 102
   <211> 432
   <212> PRT
   <213> Corynebacterium glutamicum
<400> 102
   Met Ala Gln Val Met Asp Phe Lys Val Ala Asp Leu Ser Leu Ala Glu
   1               5                   10                  15
   Ala Gly Arg His Gln Ile Arg Leu Ala Glu Tyr Glu Met Pro Gly Leu
               20                  25                  30
   Met Gln Leu Arg Lys Glu Phe Ala Asp Glu Gln Pro Leu Lys Gly Ala
           35                  40                  45
   Arg Ile Ala Gly Ser Ile His Met Thr Val Gln Thr Ala Val Leu Ile
       50                  55                  60
   Glu Thr Leu Thr Ala Leu Gly Ala Glu Val Arg Trp Ala Ser Cys Asn
   65                  70                  75                  80
   Ile Phe Ser Thr Gln Asp Glu Ala Ala Ala Ala Ile Val Val Gly Ser
                   85                  90                  95
   Gly Thr Val Glu Glu Pro Ala Gly Val Pro Val Phe Ala Trp Lys Gly
               100                 105                 110
   Glu Ser Leu Glu Glu Tyr Trp Trp Cys Ile Asn Gln Ile Phe Ser Trp
           115                 120                 125
   Gly Asp Glu Leu Pro Asn Met Ile Leu Asp Asp Gly Gly Asp Ala Thr
       130                 135                 140
   Met Ala Val Ile Arg Gly Arg Glu Tyr Glu Gln Ala Gly Leu Val Pro
   145                 150                 155                 160
   Pro Ala Glu Ala Asn Asp Ser Asp Glu Tyr Ile Ala Phe Leu Gly Met
                   165                 170                 175
   Leu Arg Glu Val Leu Ala Ala Glu Pro Gly Lys Trp Gly Lys Ile Ala
   180 185 190
   Glu Ala Val Lys Gly Val Thr Glu Glu Thr Thr Thr Gly Val His Arg
   195 200 205
   Leu Tyr His Phe Ala Glu Glu Gly Val Leu Pro Phe Pro Ala Met Asn
   210 215 220
   Val Asn Asp Ala Val Thr Lys Ser Lys Phe Asp Asn Lys Tyr Gly Thr
   225 230 235 240
   Arg His Ser Leu Ile Asp Gly Ile Asn Arg Ala Thr Asp Met Leu Met
   245 250 255
   Gly Gly Lys Asn Val Leu Val Cys Gly Tyr Gly Asp Val Gly Lys Gly
   260 265 270
   Cys Ala Glu Ala Phe Asp Gly Gln Gly Ala Arg Val Lys Val Thr Glu
   275 280 285
   Ala Asp Pro Ile Asn Ala Leu Gln Ala Leu Met Asp Gly Tyr Ser Val
   290 295 300
   Val Thr Val Asp Glu Ala Ile Glu Asp Ala Asp Ile Val Ile Thr Ala
   305 310 315 320
   Thr Gly Asn Lys Asp Ile Ile Ser Phe Glu Gln Met Leu Lys Met Lys
   325 330 335
   Asp His Ala Leu Leu Gly Asn Ile Gly His Phe Asp Asn Glu Ile Asp
   340 345 350
   Met His Ser Leu Leu His Arg Asp Asp Val Thr Arg Thr Thr Ile Lys
   355 360 365
   Pro Gln Val Asp Glu Phe Thr Phe Ser Thr Gly Arg Ser Ile Ile Val
   370 375 380
   Leu Ser Glu Gly Arg Leu Leu Asn Leu Gly Asn Ala Thr Gly His Pro
   385 390 395 400
   Ser Phe Val Met Ser Asn Ser Phe Ala Asp Gln Thr Ile Ala Gln Ile
   405 410 415
   Glu Leu Phe Gln Asn Glu Gly Gln Tyr Glu Asn Glu Val Tyr Arg Leu
   420 425 430
<210> 103
   <211> 2358
   <212> DNA
   <213> Corynebacterium glutamicum
<220>
   <221> CDS
   <222> (101)..(2335)
   <223> RXN02085
<400> 103
   cacccggtga tttcgcgaac cttgaaacat cgtcagaaga ttgccgtgcg tcctagccgg 60
   gatccgcacg ttcggctcaa gcagaaagtc tttaactcac atg act tcc aac ttt   115
                                               Met Thr Ser Asn Phe
                                                 1               5
   tct tcc act gtc gct ggt ctt cct cgc atc gga gcg aag cgt gaa ctg   163
   Ser Ser Thr Val Ala Gly Leu Pro Arg Ile Gly Ala Lys Arg Glu Leu
                    10                  15                  20
   aag ttc gcg ctc gaa ggc tac tgg aat gga tca att gaa ggt cgc gaa   211
   Lys Phe Ala Leu Glu Gly Tyr Trp Asn Gly Ser Ile Glu Gly Arg Glu
                25                  30                  35
   ctt gcg cag acc gcc cgc caa ttg gtc aac act gca tcg gat tct ttg   259
   Leu Ala Gln Thr Ala Arg Gln Leu Val Asn Thr Ala Ser Asp Ser Leu
            40                  45                  50
   tct gga ttg gat tcc gtt ccg ttt gca gga cgt tcc tac tac gac gca   307
   Ser Gly Leu Asp Ser Val Pro Phe Ala Gly Arg Ser Tyr Tyr Asp Ala
        55                  60                  65
   atg ctc gat acc gcc gct att ttg ggt gtg ctg ccg gag cgt ttt gat   355
   Met Leu Asp Thr Ala Ala Ile Leu Gly Val Leu Pro Glu Arg Phe Asp
    70                  75                  80                  85
   gac atc gct gat cat gaa aac gat ggt ctc cca ctg tgg att gac cgc   403
   Asp Ile Ala Asp His Glu Asn Asp Gly Leu Pro Leu Trp Ile Asp Arg
                    90                  95                 100
   tac ttt ggc gct gct cgc ggt act gag acc ctg cct gca cag gca atg   451
   Tyr Phe Gly Ala Ala Arg Gly Thr Glu Thr Leu Pro Ala Gln Ala Met
               105                 110                 115
   acc aag tgg ttt gat acc aac tac cac tac ctc gtg ccg gag ttg tct   499
   Thr Lys Trp Phe Asp Thr Asn Tyr His Tyr Leu Val Pro Glu Leu Ser
           120                 125                 130
   gcg gat aca cgt ttc gtt ttg gat gcg tcc gcg ctg att gag gat ctc   547
   Ala Asp Thr Arg Phe Val Leu Asp Ala Ser Ala Leu Ile Glu Asp Leu
       135                 140                 145
   cgt tgc cag cag gtt cgt ggc gtt aat gcc cgc cct gtt ctg gtt ggt   595
   Arg Cys Gln Gln Val Arg Gly Val Asn Ala Arg Pro Val Leu Val Gly
   150                 155                 160                 165
   cca ctg act ttc ctt tcc ctt gct cgc acc act gat ggt tcc aat cct   643
   Pro Leu Thr Phe Leu Ser Leu Ala Arg Thr Thr Asp Gly Ser Asn Pro
                   170                 175                 180
   ttg gat cac ctg cct gca ctg ttt gag gtc tac gag cgc ctc atc aag   691
   Leu Asp His Leu Pro Ala Leu Phe Glu Val Tyr Glu Arg Leu Ile Lys
               185                 190                 195
   tct ttc gat act gag tgg gtt cag atc gat gag cct gcg ttg gtc acc   739
   Ser Phe Asp Thr Glu Trp Val Gln Ile Asp Glu Pro Ala Leu Val Thr
           200                 205                 210
   gat gtt gct cct gag gtt ttg gag cag gtc cgc gct ggt tac acc act   787
   Asp Val Ala Pro Glu Val Leu Glu Gln Val Arg Ala Gly Tyr Thr Thr
       215                 220                 225
   ttg gct aag cgc gat ggc gtg ttt gtc aat act tac ttc ggc tct ggc   835
   Leu Ala Lys Arg Asp Gly Val Phe Val Asn Thr Tyr Phe Gly Ser Gly
   230                 235                 240                 245
   gat cag gcg ctg aac act ctt gcg ggc atc ggc ctt ggc gcg att ggc    883
   Asp Gln Ala Leu Asn Thr Leu Ala Gly Ile Gly Leu Gly Ala Ile Gly
                   250                 255                 260
   gtt gac ttg gtc acc cat ggc gtc act gag ctt gct gcg tgg aag ggt    931
   Val Asp Leu Val Thr His Gly Val Thr Glu Leu Ala Ala Trp Lys Gly
               265                 270                 275
   gag gag ctg ctg gtt gcg ggc atc gtt gat ggt cgt aac att tgg cgc    979
   Glu Glu Leu Leu Val Ala Gly Ile Val Asp Gly Arg Asn Ile Trp Arg
           280                 285                 290
   acc gac ctg tgt gct gct ctt gct tcc ctg aag cgc ctg gca gct cgc   1027
   Thr Asp Leu Cys Ala Ala Leu Ala Ser Leu Lys Arg Leu Ala Ala Arg
       295                 300                 305
   ggc cca atc gca gtg tct acc tct tgt tca ctg ctg cac gtt cct tac   1075
   Gly Pro Ile Ala Val Ser Thr Ser Cys Ser Leu Leu His Val Pro Tyr
   310                 315                 320                 325
   acc ctc gag gct gag aac att gag cct gag gtc cgc gac tgg ctt gcc   1123
   Thr Leu Glu Ala Glu Asn Ile Glu Pro Glu Val Arg Asp Trp Leu Ala
                   330                 335                 340
   ttc ggc tcg gag aag atc acc gag gtc aag ctg ctt gcc gac gcc cta   1171
   Phe Gly Ser Glu Lys Ile Thr Glu Val Lys Leu Leu Ala Asp Ala Leu
               345                 350                 355
   gcc ggc aac atc gac gcg gct gcg ttc gat gcg gcg tcc gca gca att   1219
   Ala Gly Asn Ile Asp Ala Ala Ala Phe Asp Ala Ala Ser Ala Ala Ile
           360                 365                 370
   gct tct cga cgc acc tcc cca cgc acc gca cca atc acg cag gaa ctc   1267
   Ala Ser Arg Arg Thr Ser Pro Arg Thr Ala Pro Ile Thr Gln Glu Leu
       375                 380                 385
   cct ggc cgt agc cgt gga tcc ttc gac act cgt gtt acg ctg cag gag   1315
   Pro Gly Arg Ser Arg Gly Ser Phe Asp Thr Arg Val Thr Leu Gln Glu
   390                 395                 400                 405
   aag tca ctg gag ctt cca gct ctg cca acc acc acc att ggt tct ttc   1363
   Lys Ser Leu Glu Leu Pro Ala Leu Pro Thr Thr Thr Ile Gly Ser Phe
                   410                 415                 420
   cca cag acc cca tcc att cgt tct gct cgc gct cgt ctg cgc aag gaa   1411
   Pro Gln Thr Pro Ser Ile Arg Ser Ala Arg Ala Arg Leu Arg Lys Glu
               425                 430                 435
   tcc atc act ttg gag cag tac gaa gag gca atg cgc gaa gaa atc gat   1459
   Ser Ile Thr Leu Glu Gln Tyr Glu Glu Ala Met Arg Glu Glu Ile Asp
           440                 445                 450
   ctg gtc atc gcc aag cag gaa gaa ctt ggt ctt gat gtg ttg gtt cac   1507
   Leu Val Ile Ala Lys Gln Glu Glu Leu Gly Leu Asp Val Leu Val His
       455                 460                 465
   ggt gag cca gag cgc aac gac atg gtt cag tac ttc tct gaa ctt ctc   1555
   Gly Glu Pro Glu Arg Asn Asp Met Val Gln Tyr Phe Ser Glu Leu Leu
   470                 475                 480                 485
   gac ggt ttc ctc tca acc gcc aac ggc tgg gtc caa agc tac ggc tcc    1603
   Asp Gly Phe Leu Ser Thr Ala Asn Gly Trp Val Gln Ser Tyr Gly Ser
                   490                 495                 500
   cgc tgt gtt cgt cct cca gtg ttg ttc gga aac gtt tcc cgc cca gcg    1651
   Arg Cys Val Arg Pro Pro Val Leu Phe Gly Asn Val Ser Arg Pro Ala
               505                 510                 515
   cca atg act gtc aag tgg ttc cag tac gca cag agc ctg acc cag aag    1699
   Pro Met Thr Val Lys Trp Phe Gln Tyr Ala Gln Ser Leu Thr Gln Lys
           520                 525                 530
   cat gtc aag gga atg ctc acc ggt cca gtc acc atc ctt gca tgg tcc    1747
   His Val Lys Gly Met Leu Thr Gly Pro Val Thr Ile Leu Ala Trp Ser
       535                 540                 545
   ttc gtt cgc gat gat cag ccg ctg gct acc act gct gac cag gtt gca    1795
   Phe Val Arg Asp Asp Gln Pro Leu Ala Thr Thr Ala Asp Gln Val Ala
   550                 555                 560                 565
   ctg gca ctg cgc gat gaa att aac gat ctc atc gag gct ggc gcg aag    1843
   Leu Ala Leu Arg Asp Glu Ile Asn Asp Leu Ile Glu Ala Gly Ala Lys
                   570                 575                 580
   atc atc cag gtg gat gag cct gcg att cgt gaa ctg ttg ccg cta cga    1891
   Ile Ile Gln Val Asp Glu Pro Ala Ile Arg Glu Leu Leu Pro Leu Arg
               585                 590                 595
   gac gtc gat aag cct gcc tac ctg cag tgg tcc gtg gac tcc ttc cgc    1939
   Asp Val Asp Lys Pro Ala Tyr Leu Gln Trp Ser Val Asp Ser Phe Arg
           600                 605                 610
   ctg gcg act gcc ggc gca ccc gac gac gtc caa atc cac acc cac atg    1987
   Leu Ala Thr Ala Gly Ala Pro Asp Asp Val Gln Ile His Thr His Met
       615                 620                 625
   tgc tac tcc gag ttc aac gaa gtg atc tcc tcg gtc atc gcg ttg gat    2035
   Cys Tyr Ser Glu Phe Asn Glu Val Ile Ser Ser Val Ile Ala Leu Asp
   630                 635                 640                 645
   gcc gat gtc acc acc atc gaa gca gca cgt tcc gac atg cag gtc ctc    2083
   Ala Asp Val Thr Thr Ile Glu Ala Ala Arg Ser Asp Met Gln Val Leu
                   650                 655                 660
   gct gct ctg aaa tct tcc ggc ttc gag ctc ggc gtc gga cct ggt gtg    2131
   Ala Ala Leu Lys Ser Ser Gly Phe Glu Leu Gly Val Gly Pro Gly Val
               665                 670                 675
   tgg gat atc cac tcc ccg cgc gtt cct tcc gcg cag aaa gtg gac ggt    2179
   Trp Asp Ile His Ser Pro Arg Val Pro Ser Ala Gln Lys Val Asp Gly
           680                 685                 690
   ctc ctc gag gct gca ctg cag tcc gtg gat cct cgc cag ctg tgg gtc    2227
   Leu Leu Glu Ala Ala Leu Gln Ser Val Asp Pro Arg Gln Leu Trp Val
       695                 700                 705
   aac cca gac tgt ggt ctg aag acc cgt gga tgg cca gaa gtg gaa gct    2275
   Asn Pro Asp Cys Gly Leu Lys Thr Arg Gly Trp Pro Glu Val Glu Ala
   710                 715                 720                 725
   tcc cta aag gtt ctc gtt gag tcc gct aag cag gct cgt gag aaa atc    2323
   Ser Leu Lys Val Leu Val Glu Ser Ala Lys Gln Ala Arg Glu Lys Ile
                   730                 735                 740
   gga gca act atc taaattgggt taccgctagg aac                          2358
   Gly Ala Thr Ile
               745
<210> 104
   <211> 745
   <212> PRT
   <213> Corynebacterium glutamicum
<400> 104
   Met Thr Ser Asn Phe Ser Ser Thr Val Ala Gly Leu Pro Arg Ile Gly
    1               5                  10                  15
   Ala Lys Arg Glu Leu Lys Phe Ala Leu Glu Gly Tyr Trp Asn Gly Ser
               20                  25                  30
   Ile Glu Gly Arg Glu Leu Ala Gln Thr Ala Arg Gln Leu Val Asn Thr
           35                  40                  45
   Ala Ser Asp Ser Leu Ser Gly Leu Asp Ser Val Pro Phe Ala Gly Arg
       50                  55                  60
   Ser Tyr Tyr Asp Ala Met Leu Asp Thr Ala Ala Ile Leu Gly Val Leu
   65                  70                  75                  80
   Pro Glu Arg Phe Asp Asp Ile Ala Asp His Glu Asn Asp Gly Leu Pro
                   85                  90                  95
   Leu Trp Ile Asp Arg Tyr Phe Gly Ala Ala Arg Gly Thr Glu Thr Leu
               100                 105                 110
   Pro Ala Gln Ala Met Thr Lys Trp Phe Asp Thr Asn Tyr His Tyr Leu
           115                 120                 125
   Val Pro Glu Leu Ser Ala Asp Thr Arg Phe Val Leu Asp Ala Ser Ala
       130                 135                 140
   Leu Ile Glu Asp Leu Arg Cys Gln Gln Val Arg Gly Val Asn Ala Arg
   145                 150                 155                 160
   Pro Val Leu Val Gly Pro Leu Thr Phe Leu Ser Leu Ala Arg Thr Thr
                   165                 170                 175
   Asp Gly Ser Asn Pro Leu Asp His Leu Pro Ala Leu Phe Glu Val Tyr
               180                 185                 190
   Glu Arg Leu Ile Lys Ser Phe Asp Thr Glu Trp Val Gln Ile Asp Glu
           195                 200                 205
   Pro Ala Leu Val Thr Asp Val Ala Pro Glu Val Leu Glu Gln Val Arg
       210                 215                 220
   Ala Gly Tyr Thr Thr Leu Ala Lys Arg Asp Gly Val Phe Val Asn Thr
   225                 230                 235                 240
   Tyr Phe Gly Ser Gly Asp Gln Ala Leu Asn Thr Leu Ala Gly Ile Gly
   245 250 255
   Leu Gly Ala Ile Gly Val Asp Leu Val Thr His Gly Val Thr Glu Leu
   260 265 270
   Ala Ala Trp Lys Gly Glu Glu Leu Leu Val Ala Gly Ile Val Asp Gly
   275 280 285
   Arg Asn Ile Trp Arg Thr Asp Leu Cys Ala Ala Leu Ala Ser Leu Lys
   290 295 300
   Arg Leu Ala Ala Arg Gly Pro Ile Ala Val Ser Thr Ser Cys Ser Leu
   305 310 315 320
   Leu His Val Pro Tyr Thr Leu Glu Ala Glu Asn Ile Glu Pro Glu Val
   325 330 335
   Arg Asp Trp Leu Ala Phe Gly Ser Glu Lys Ile Thr Glu Val Lys Leu
   340 345 350
   Leu Ala Asp Ala Leu Ala Gly Asn Ile Asp Ala Ala Ala Phe Asp Ala
   355 360 365
   Ala Ser Ala Ala Ile Ala Ser Arg Arg Thr Ser Pro Arg Thr Ala Pro
   370 375 380
   Ile Thr Gln Glu Leu Pro Gly Arg Ser Arg Gly Ser Phe Asp Thr Arg
   385 390 395 400
   Val Thr Leu Gln Glu Lys Ser Leu Glu Leu Pro Ala Leu Pro Thr Thr
   405 410 415
   Thr Ile Gly Ser Phe Pro Gln Thr Pro Ser Ile Arg Ser Ala Arg Ala
   420 425 430
   Arg Leu Arg Lys Glu Ser Ile Thr Leu Glu Gln Tyr Glu Glu Ala Met
   435 440 445
   Arg Glu Glu Ile Asp Leu Val Ile Ala Lys Gln Glu Glu Leu Gly Leu
   450 455 460
   Asp Val Leu Val His Gly Glu Pro Glu Arg Asn Asp Met Val Gln Tyr
   465 470 475 480
   Phe Ser Glu Leu Leu Asp Gly Phe Leu Ser Thr Ala Asn Gly Trp Val
   485 490 495
   Gln Ser Tyr Gly Ser Arg Cys Val Arg Pro Pro Val Leu Phe Gly Asn
   500 505 510
   Val Ser Arg Pro Ala Pro Met Thr Val Lys Trp Phe Gln Tyr Ala Gln
   515 520 525
   Ser Leu Thr Gln Lys His Val Lys Gly Met Leu Thr Gly Pro Val Thr
   530 535 540
   Ile Leu Ala Trp Ser Phe Val Arg Asp Asp Gln Pro Leu Ala Thr Thr
   545 550 555 560
   Ala Asp Gln Val Ala Leu Ala Leu Arg Asp Glu Ile Asn Asp Leu Ile
                   565                 570                 575
   Glu Ala Gly Ala Lys Ile Ile Gln Val Asp Glu Pro Ala Ile Arg Glu
               580                 585                 590
   Leu Leu Pro Leu Arg Asp Val Asp Lys Pro Ala Tyr Leu Gln Trp Ser
           595                 600                 605
   Val Asp Ser Phe Arg Leu Ala Thr Ala Gly Ala Pro Asp Asp Val Gln
       610                 615                 620
   Ile His Thr His Met Cys Tyr Ser Glu Phe Asn Glu Val Ile Ser Ser
   625                 630                 635                 640
   Val Ile Ala Leu Asp Ala Asp Val Thr Thr Ile Glu Ala Ala Arg Ser
                   645                 650                 655
   Asp Met Gln Val Leu Ala Ala Leu Lys Ser Ser Gly Phe Glu Leu Gly
               660                 665                 670
   Val Gly Pro Gly Val Trp Asp Ile His Ser Pro Arg Val Pro Ser Ala
           675                 680                 685
   Gln Lys Val Asp Gly Leu Leu Glu Ala Ala Leu Gln Ser Val Asp Pro
       690                 695                 700
   Arg Gln Leu Trp Val Asn Pro Asp Cys Gly Leu Lys Thr Arg Gly Trp
   705                 710                 715                 720
   Pro Glu Val Glu Ala Ser Leu Lys Val Leu Val Glu Ser Ala Lys Gln
                   725                 730                 735
   Ala Arg Glu Lys Ile Gly Ala Thr Ile
               740                 745
<210> 105
   <211> 1923
   <212> DNA
   <213> Corynebacterium glutamicum
<220>
   <221> CDS
   <222> (101)..(1900)
   <223> FRXA02085
<400> 105
   cacccggtga tttcgcgaac cttgaaacat cgtcagaaga ttgccgtgcg tcctagccgg 60
   gatccgcacg ttcggctcaa gcagaaagtc tttaactcac atg act tcc aac ttt   115
                                               Met Thr Ser Asn Phe
                                               1               5
   tct tcc act gtc gct ggt ctt cct cgc atc gga gcg aag cgt gaa ctg   163
   Ser Ser Thr Val Ala Gly Leu Pro Arg Ile Gly Ala Lys Arg Glu Leu
                   10                  15                  20
   aag ttc gcg ctc gaa ggc tac tgg aat gga tca att gaa ggt cgc gaa   211
   Lys Phe Ala Leu Glu Gly Tyr Trp Asn Gly Ser Ile Glu Gly Arg Glu
               25                  30                  35
   ctt gcg cag acc gcc cgc caa ttg gtc aac act gca tcg gat tct ttg    259
   Leu Ala Gln Thr Ala Arg Gln Leu Val Asn Thr Ala Ser Asp Ser Leu
           40                  45                  50
   tct gga ttg gat tcc gtt ccg ttt gca gga cgt tcc tac tac gac gca    307
   Ser Gly Leu Asp Ser Val Pro Phe Ala Gly Arg Ser Tyr Tyr Asp Ala
       55                  60                  65
   atg ctc gat acc gcc gct att ttg ggt gtg ctg ccg gag cgt ttt gat    355
   Met Leu Asp Thr Ala Ala Ile Leu Gly Val Leu Pro Glu Arg Phe Asp
   70                  75                  80                  85
   gac atc gct gat cat gaa aac gat ggt ctc cca ctg tgg att gac cgc    403
   Asp Ile Ala Asp His Glu Asn Asp Gly Leu Pro Leu Trp Ile Asp Arg
                   90                  95                  100
   tac ttt ggc gct gct cgc ggt act gag acc ctg cct gca cag gca atg    451
   Tyr Phe Gly Ala Ala Arg Gly Thr Glu Thr Leu Pro Ala Gln Ala Met
               105                 110                 115
   acc aag tgg ttt gat acc aac tac cac tac ctc gtg ccg gag ttg tct    499
   Thr Lys Trp Phe Asp Thr Asn Tyr His Tyr Leu Val Pro Glu Leu Ser
           120                 125                 130
   gcg gat aca cgt ttc gtt ttg gat gcg tcc gcg ctg att gag gat ctc    547
   Ala Asp Thr Arg Phe Val Leu Asp Ala Ser Ala Leu Ile Glu Asp Leu
       135                 140                 145
   cgt tgc cag cag gtt cgt ggc gtt aat gcc cgc cct gtt ctg gtt ggt    595
   Arg Cys Gln Gln Val Arg Gly Val Asn Ala Arg Pro Val Leu Val Gly
   150                 155                 160                 165
   cca ctg act ttc ctt tcc ctt gct cgc acc act gat ggt tcc aat cct    643
   Pro Leu Thr Phe Leu Ser Leu Ala Arg Thr Thr Asp Gly Ser Asn Pro
                   170                 175                 180
   ttg gat cac ctg cct gca ctg ttt gag gtc tac gag cgc ctc atc aag    691
   Leu Asp His Leu Pro Ala Leu Phe Glu Val Tyr Glu Arg Leu Ile Lys
               185                 190                 195
   tct ttc gat act gag tgg gtt cag atc gat gag cct gcg ttg gtc acc    739
   Ser Phe Asp Thr Glu Trp Val Gln Ile Asp Glu Pro Ala Leu Val Thr
           200                 205                 210
   gat gtt gct cct gag gtt ttg gag cag gtc cgc gct ggt tac acc act    787
   Asp Val Ala Pro Glu Val Leu Glu Gln Val Arg Ala Gly Tyr Thr Thr
       215                 220                 225
   ttg gct aag cgc gat ggc gtg ttt gtc aat act tac ttc ggc tct ggc    835
   Leu Ala Lys Arg Asp Gly Val Phe Val Asn Thr Tyr Phe Gly Ser Gly
   230                 235                 240                 245
   gat cag gcg ctg aac act ctt gcg ggc atc ggc ctt ggc gcg att ggc    883
   Asp Gln Ala Leu Asn Thr Leu Ala Gly Ile Gly Leu Gly Ala Ile Gly
                   250                 255                 260
   gtt gac ttg gtc acc cat ggc gtc act gag ctt gct gcg tgg aag ggt    931
   Val Asp Leu Val Thr His Gly Val Thr Glu Leu Ala Ala Trp Lys Gly
               265                 270                 275
   gag gag ctg ctg gtt gcg ggc atc gtt gat ggt cgt aac att tgg cgc    979
   Glu Glu Leu Leu Val Ala Gly Ile Val Asp Gly Arg Asn Ile Trp Arg
           280                 285                 290
   acc gac ctg tgt gct gct ctt gct tcc ctg aag cgc ctg gca gct cgc    1027
   Thr Asp Leu Cys Ala Ala Leu Ala Ser Leu Lys Arg Leu Ala Ala Arg
       295                 300                 305
   ggc cca atc gca gtg tct acc tct tgt tca ctg ctg cac gtt cct tac    1075
   Gly Pro Ile Ala Val Ser Thr Ser Cys Ser Leu Leu His Val Pro Tyr
   310                 315                 320                 325
   acc ctc gag gct gag aac att gag cct gag gtc cgc gac tgg ctt gcc    1123
   Thr Leu Glu Ala Glu Asn Ile Glu Pro Glu Val Arg Asp Trp Leu Ala
                   330                 335                 340
   ttc ggc tcg gag aag atc acc gag gtc aag ctg ctt gcc gac gcc cta    1171
   Phe Gly Ser Glu Lys Ile Thr Glu Val Lys Leu Leu Ala Asp Ala Leu
               345                 350                 355
   gcc ggc aac atc gac gcg gct gcg ttc gat gcg gcg tcc gca gca att    1219
   Ala Gly Asn Ile Asp Ala Ala Ala Phe Asp Ala Ala Ser Ala Ala Ile
           360                 365                 370
   gct tct cga cgc acc tcc cca cgc acc gca cca atc acg cag gaa ctc    1267
   Ala Ser Arg Arg Thr Ser Pro Arg Thr Ala Pro Ile Thr Gln Glu Leu
       375                 380                 385
   cct ggc cgt agc cgt gga tcc ttc gac act cgt gtt acg ctg cag gag    1315
   Pro Gly Arg Ser Arg Gly Ser Phe Asp Thr Arg Val Thr Leu Gln Glu
   390                 395                 400                 405
   aag tca ctg gag ctt cca gct ctg cca acc acc acc att ggt tct ttc    1363
   Lys Ser Leu Glu Leu Pro Ala Leu Pro Thr Thr Thr Ile Gly Ser Phe
                   410                 415                 420
   cca cag acc cca tcc att cgt tct gct cgc gct cgt ctg cgc aag gaa    1411
   Pro Gln Thr Pro Ser Ile Arg Ser Ala Arg Ala Arg Leu Arg Lys Glu
               425                 430                 435
   tcc atc act ttg gag cag tac gaa gag gca atg cgc gaa gaa atc gat    1459
   Ser Ile Thr Leu Glu Gln Tyr Glu Glu Ala Met Arg Glu Glu Ile Asp
           440                 445                 450
   ctg gtc atc gcc aag cag gaa gaa ctt ggt ctt gat gtg ttg gtt cac    1507
   Leu Val Ile Ala Lys Gln Glu Glu Leu Gly Leu Asp Val Leu Val His
       455                 460                 465
   ggt gag cca gag cgc aac gac atg gtt cag tac ttc tct gaa ctt ctc    1555
   Gly Glu Pro Glu Arg Asn Asp Met Val Gln Tyr Phe Ser Glu Leu Leu
   470                 475                 480                 485
   gac ggt ttc ctc tca acc gcc aac ggc tgg gtc caa agc tac ggc tcc    1603
   Asp Gly Phe Leu Ser Thr Ala Asn Gly Trp Val Gln Ser Tyr Gly Ser
                   490                 495                 500
   cgc tgt gtt cgt cct cca gtg ttg ttc gga aac gtt tcc cgc cca gcg    1651
   Arg Cys Val Arg Pro Pro Val Leu Phe Gly Asn Val Ser Arg Pro Ala
               505                 510                 515
   cca atg act gtc aag tgg ttc cag tac gca cag agc ctg acc cag aag    1699
   Pro Met Thr Val Lys Trp Phe Gln Tyr Ala Gln Ser Leu Thr Gln Lys
               520                  525                  530
   cat gtc aag gga atg ctc acc ggt cca gtc acc atc ctt gca tgg tcc      1747
   His Val Lys Gly Met Leu Thr Gly Pro Val Thr Ile Leu Ala Trp Ser
       535                 540                 545
   ttc gtt cgc gat gat cag ccg ctg gct acc act gct gac cag gtt gca      1795
   Phe Val Arg Asp Asp Gln Pro Leu Ala Thr Thr Ala Asp Gln Val Ala
   550                 555                 560                 565
   ctg gca ctg cgc gat gaa att aac gat ctc atc gag gct ggc gcg aag      1843
   Leu Ala Leu Arg Asp Glu Ile Asn Asp Leu Ile Glu Ala Gly Ala Lys
                   570                 575                 580
   atc atc cag gtg gat gag cct gcg att cgt gaa ctg ttg ccc gct acg      1891
   Ile Ile Gln Val Asp Glu Pro Ala Ile Arg Glu Leu Leu Pro Ala Thr
               585                 590                 595
   aga cgt cga taagcctgcc tacctgcagt ggt                                1923
   Arg Arg Arg
           600
<210> 106
   <211> 600
   <212> PRT
   <213> Corynebacterium glutamicum
<400> 106
   Met Thr Ser Asn Phe Ser Ser Thr Val Ala Gly Leu Pro Arg Ile Gly
    1               5                  10                  15
   Ala Lys Arg Glu Leu Lys Phe Ala Leu Glu Gly Tyr Trp Asn Gly Ser
               20                  25                  30
   Ile Glu Gly Arg Glu Leu Ala Gln Thr Ala Arg Gln Leu Val Asn Thr
           35                  40                  45
   Ala Ser Asp Ser Leu Ser Gly Leu Asp Ser Val Pro Phe Ala Gly Arg
       50                  55                  60
   Ser Tyr Tyr Asp Ala Met Leu Asp Thr Ala Ala Ile Leu Gly Val Leu
   65                  70                  75                  80
   Pro Glu Arg Phe Asp Asp Ile Ala Asp His Glu Asn Asp Gly Leu Pro
                   85                  90                  95
   Leu Trp Ile Asp Arg Tyr Phe Gly Ala Ala Arg Gly Thr Glu Thr Leu
               100                 105                 110
   Pro Ala Gln Ala Met Thr Lys Trp Phe Asp Thr Asn Tyr His Tyr Leu
           115                 120                 125
   Val Pro Glu Leu Ser Ala Asp Thr Arg Phe Val Leu Asp Ala Ser Ala
       130                 135                 140
   Leu Ile Glu Asp Leu Arg Cys Gln Gln Val Arg Gly Val Asn Ala Arg
   145                 150                 155                 160
   Pro Val Leu Val Gly Pro Leu Thr Phe Leu Ser Leu Ala Arg Thr Thr
                   165                 170                 175
   Asp Gly Ser Asn Pro Leu Asp His Leu Pro Ala Leu Phe Glu Val Tyr
               180                 185                 190
   Glu Arg Leu Ile Lys Ser Phe Asp Thr Glu Trp Val Gln Ile Asp Glu
           195                 200                 205
   Pro Ala Leu Val Thr Asp Val Ala Pro Glu Val Leu Glu Gln Val Arg
       210                 215                 220
   Ala Gly Tyr Thr Thr Leu Ala Lys Arg Asp Gly Val Phe Val Asn Thr
   225                 230                 235                 240
   Tyr Phe Gly Ser Gly Asp Gln Ala Leu Asn Thr Leu Ala Gly Ile Gly
                   245                 250                 255
   Leu Gly Ala Ile Gly Val Asp Leu Val Thr His Gly Val Thr Glu Leu
               260                 265                 270
   Ala Ala Trp Lys Gly Glu Glu Leu Leu Val Ala Gly Ile Val Asp Gly
           275                 280                 285
   Arg Asn Ile Trp Arg Thr Asp Leu Cys Ala Ala Leu Ala Ser Leu Lys
       290                 295                 300
   Arg Leu Ala Ala Arg Gly Pro Ile Ala Val Ser Thr Ser Cys Ser Leu
   305                 310                 315                 320
   Leu His Val Pro Tyr Thr Leu Glu Ala Glu Asn Ile Glu Pro Glu Val
                   325                 330                 335
   Arg Asp Trp Leu Ala Phe Gly Ser Glu Lys Ile Thr Glu Val Lys Leu
               340                 345                 350
   Leu Ala Asp Ala Leu Ala Gly Asn Ile Asp Ala Ala Ala Phe Asp Ala
           355                 360                 365
   Ala Ser Ala Ala Ile Ala Ser Arg Arg Thr Ser Pro Arg Thr Ala Pro
       370                 375                 380
   Ile Thr Gln Glu Leu Pro Gly Arg Ser Arg Gly Ser Phe Asp Thr Arg
   385                 390                 395                 400
   Val Thr Leu Gln Glu Lys Ser Leu Glu Leu Pro Ala Leu Pro Thr Thr
                   405                 410                 415
   Thr Ile Gly Ser Phe Pro Gln Thr Pro Ser Ile Arg Ser Ala Arg Ala
               420                 425                 430
   Arg Leu Arg Lys Glu Ser Ile Thr Leu Glu Gln Tyr Glu Glu Ala Met
           435                 440                 445
   Arg Glu Glu Ile Asp Leu Val Ile Ala Lys Gln Glu Glu Leu Gly Leu
       450                 455                 460
   Asp Val Leu Val His Gly Glu Pro Glu Arg Asn Asp Met Val Gln Tyr
   465                 470                 475                 480
   Phe Ser Glu Leu Leu Asp Gly Phe Leu Ser Thr Ala Asn Gly Trp Val
                   485                 490                 495
   Gln Ser Tyr Gly Ser Arg Cys Val Arg Pro Pro Val Leu Phe Gly Asn
               500                 505                 510
   Val Ser Arg Pro Ala Pro Met Thr Val Lys Trp Phe Gln Tyr Ala Gln
           515                 520                 525
   Ser Leu Thr Gln Lys His Val Lys Gly Met Leu Thr Gly Pro Val Thr
       530                 535                 540
   Ile Leu Ala Trp Ser Phe Val Arg Asp Asp Gln Pro Leu Ala Thr Thr
   545                 550                 555                 560
   Ala Asp Gln Val Ala Leu Ala Leu Arg Asp Glu Ile Asn Asp Leu Ile
                   565                 570                 575
   Glu Ala Gly Ala Lys Ile Ile Gln Val Asp Glu Pro Ala Ile Arg Glu
               580                 585                 590
   Leu Leu Pro Ala Thr Arg Arg Arg
           595                 600
<210> 107
   <211> 603
   <212> DNA
   <213> Corynebacterium glutamicum
<220>
   <221> CDS
   <222> (101)..(580)
   <223> FRXA02086
<400> 107
   gatgatcagc cgctggctac cactgctgac caggttgcac tggcactgcg cgatgaaatt 60
   aacgatctca tcgaggctgg cgcgaagatc atccaggtgg atg agc ctg cga ttc   115
                                               Met Ser Leu Arg Phe
                                                1               5
   gtg aac tgt tgc ccg cta cga gac gtc gat aag cct gcc tac ctg cag   163
   Val Asn Cys Cys Pro Leu Arg Asp Val Asp Lys Pro Ala Tyr Leu Gln
                   10                  15                  20
   tgg tcc gtg gac tcc ttc cgc ctg gcg act gcc ggc gca ccc gac gac   211
   Trp Ser Val Asp Ser Phe Arg Leu Ala Thr Ala Gly Ala Pro Asp Asp
               25                  30                  35
   gtc caa atc cac acc cac atg tgc tac tcc gag ttc aac gaa gtg atc   259
   Val Gln Ile His Thr His Met Cys Tyr Ser Glu Phe Asn Glu Val Ile
           40                  45                  50
   tcc tcg gtc atc gcg ttg gat gcc gat gtc acc acc atc gaa gca gca   307
   Ser Ser Val Ile Ala Leu Asp Ala Asp Val Thr Thr Ile Glu Ala Ala
       55                  60                  65
   cgt tcc gac atg cag gtc ctc gct gct ctg aaa tct tcc ggc ttc gag   355
   Arg Ser Asp Met Gln Val Leu Ala Ala Leu Lys Ser Ser Gly Phe Glu
   70                  75                  80                  85
   ctc ggc gtc gga cct ggt gtg tgg gat atc cac tcc ccg cgc gtt cct   403
   Leu Gly Val Gly Pro Gly Val Trp Asp Ile His Ser Pro Arg Val Pro
                   90                  95                  100
   tcc gcg cag aaa gtg gac ggt ctc ctc gag gct gca ctg cag tcc gtg   451
   Ser Ala Gln Lys Val Asp Gly Leu Leu Glu Ala Ala Leu Gln Ser Val
               105                 110                 115
   gat cct cgc cag ctg tgg gtc aac cca gac tgt ggt ctg aag acc cgt   499
   Asp Pro Arg Gln Leu Trp Val Asn Pro Asp Cys Gly Leu Lys Thr Arg
           120                 125                 130
   gga tgg cca gaa gtg gaa gct tcc cta aag gtt ctc gtt gag tcc gct   547
   Gly Trp Pro Glu Val Glu Ala Ser Leu Lys Val Leu Val Glu Ser Ala
       135                 140                 145
   aag cag gct cgt gag aaa atc gga gca act atc taaattgggt taccgctagg 600
   Lys Gln Ala Arg Glu Lys Ile Gly Ala Thr Ile
   150                 155                 160
   aac                                                               603
<210> 108
   <211> 160
   <212> PRT
   <213> Corynebacterium glutamicum
<400> 108
   Met Ser Leu Arg Phe Val Asn Cys Cys Pro Leu Arg Asp Val Asp Lys
    1               5                  10                  15
   Pro Ala Tyr Leu Gln Trp Ser Val Asp Ser Phe Arg Leu Ala Thr Ala
               20                  25                  30
   Gly Ala Pro Asp Asp Val Gln Ile His Thr His Met Cys Tyr Ser Glu
           35                  40                  45
   Phe Asn Glu Val Ile Ser Ser Val Ile Ala Leu Asp Ala Asp Val Thr
       50                  55                  60
   Thr Ile Glu Ala Ala Arg Ser Asp Met Gln Val Leu Ala Ala Leu Lys
   65                  70                  75                  80
   Ser Ser Gly Phe Glu Leu Gly Val Gly Pro Gly Val Trp Asp Ile His
                   85                  90                  95
   Ser Pro Arg Val Pro Ser Ala Gln Lys Val Asp Gly Leu Leu Glu Ala
               100                 105                 110
   Ala Leu Gln Ser Val Asp Pro Arg Gln Leu Trp Val Asn Pro Asp Cys
           115                 120                 125
   Gly Leu Lys Thr Arg Gly Trp Pro Glu Val Glu Ala Ser Leu Lys Val
       130                 135                 140
   Leu Val Glu Ser Ala Lys Gln Ala Arg Glu Lys Ile Gly Ala Thr Ile
   145                 150                 155                 160
<210> 109
   <211> 1326
   <212> DNA
   <213> Corynebacterium glutamicum
<220>
   <221> CDS
   <222> (101)..(1303)
   <223> RXN02648
<400> 109
   atgaataaaa ttccgggtgc agtgaccgta ggtgaggtaa acgcggttag agtcgaatga 60
   gagtttgata ctttctttcg acttttagat tggattttca atg agc cag aac cgc   115
                                               Met Ser Gln Asn Arg
                                                 1               5
   atc agg acc act cac gtt ggt tcc ttg ccc cgt acc cca gag cta ctt   163
   Ile Arg Thr Thr His Val Gly Ser Leu Pro Arg Thr Pro Glu Leu Leu
                    10                  15                  20
   gat gca aac atc aag cgt tct aac ggt gag att ggg gag gag gaa ttc   211
   Asp Ala Asn Ile Lys Arg Ser Asn Gly Glu Ile Gly Glu Glu Glu Phe
                25                  30                  35
   ttc cag att ctg cag tct tct gta gat gac gtg atc aag cgc cag gtt   259
   Phe Gln Ile Leu Gln Ser Ser Val Asp Asp Val Ile Lys Arg Gln Val
            40                  45                  50
   gac ctg ggt atc gac atc ctt aac gag ggc gaa tac ggc cac gtc acc   307
   Asp Leu Gly Ile Asp Ile Leu Asn Glu Gly Glu Tyr Gly His Val Thr
        55                  60                  65
   tcc ggt gca gtt gac ttc ggt gca tgg tgg aac tac tcc ttc acc cgc   355
   Ser Gly Ala Val Asp Phe Gly Ala Trp Trp Asn Tyr Ser Phe Thr Arg
    70                  75                  80                  85
   ctg ggc gga ctg acc atg acc gat acc gac cgt tgg gca agc cag gaa   403
   Leu Gly Gly Leu Thr Met Thr Asp Thr Asp Arg Trp Ala Ser Gln Glu
                    90                  95                 100
   gca gtg cgt tcc acc cct ggc aac atc gag ctg acc agc ttc tct gat   451
   Ala Val Arg Ser Thr Pro Gly Asn Ile Glu Leu Thr Ser Phe Ser Asp
               105                 110                 115
   cgt cgc gac cgc gca ttg ttc agc gaa gca tac gag gat cca gta tct   499
   Arg Arg Asp Arg Ala Leu Phe Ser Glu Ala Tyr Glu Asp Pro Val Ser
           120                 125                 130
   ggc atc ttc acc ggt cgc gct tct gtg ggc aac cca gag ttc acc gga   547
   Gly Ile Phe Thr Gly Arg Ala Ser Val Gly Asn Pro Glu Phe Thr Gly
       135                 140                 145
   cct att acc tac att ggc cag gaa gaa act cag acg gat gtt gat ctg   595
   Pro Ile Thr Tyr Ile Gly Gln Glu Glu Thr Gln Thr Asp Val Asp Leu
   150                 155                 160                 165
   ctg aag aag ggc atg aac gca gcg gga gct acc gac ggc ttc gtt gca   643
   Leu Lys Lys Gly Met Asn Ala Ala Gly Ala Thr Asp Gly Phe Val Ala
                   170                 175                 180
   gca cta tcc cca gga tct gca gct cga ttg acc aac aag ttc tac gac   691
   Ala Leu Ser Pro Gly Ser Ala Ala Arg Leu Thr Asn Lys Phe Tyr Asp
               185                 190                 195
   act gat gaa gaa gtc gtc gca gca tgt gct gat gcg ctt tcc cag gaa    739
   Thr Asp Glu Glu Val Val Ala Ala Cys Ala Asp Ala Leu Ser Gln Glu
           200                 205                 210
   tac aag atc atc acc gat gca ggt ctg acc gtt cag ctc gac gca ccg    787
   Tyr Lys Ile Ile Thr Asp Ala Gly Leu Thr Val Gln Leu Asp Ala Pro
       215                 220                 225
   gac ttg gca gaa gca tgg gat cag atc aac cca gag cca agc gtg aag    835
   Asp Leu Ala Glu Ala Trp Asp Gln Ile Asn Pro Glu Pro Ser Val Lys
   230                 235                 240                 245
   gat tac ttg gac tgg atc ggt aca cgc atc gat gcc atc aac agt gca    883
   Asp Tyr Leu Asp Trp Ile Gly Thr Arg Ile Asp Ala Ile Asn Ser Ala
                   250                 255                 260
   gtg aag ggc ctt cca aag gaa cag acc cgc ctg cac atc tgc tgg ggc    931
   Val Lys Gly Leu Pro Lys Glu Gln Thr Arg Leu His Ile Cys Trp Gly
               265                 270                 275
   tct tgg cac gga cca cac gtc act gac atc cca ttc ggt gac atc att    979
   Ser Trp His Gly Pro His Val Thr Asp Ile Pro Phe Gly Asp Ile Ile
           280                 285                 290
   ggt gag atc ctg cgc gca gag gtc ggt ggc ttc tcc ttc gaa ggc gca   1027
   Gly Glu Ile Leu Arg Ala Glu Val Gly Gly Phe Ser Phe Glu Gly Ala
       295                 300                 305
   tct cct cgt cac gca cac gag tgg cgt gta tgg gaa gaa aac aag ctt   1075
   Ser Pro Arg His Ala His Glu Trp Arg Val Trp Glu Glu Asn Lys Leu
   310                 315                 320                 325
   cct gaa ggc tct gtt atc tac cct ggt gtt gtg tct cac tcc atc aac   1123
   Pro Glu Gly Ser Val Ile Tyr Pro Gly Val Val Ser His Ser Ile Asn
                   330                 335                 340
   gct gtg gag cac cca cgc ctg gtt gct gat cgt atc gtt cag ttc gcc   1171
   Ala Val Glu His Pro Arg Leu Val Ala Asp Arg Ile Val Gln Phe Ala
               345                 350                 355
   aag ctt gtt ggc cct gag aac gtc att gcg tcc act gac tgt ggt ctg   1219
   Lys Leu Val Gly Pro Glu Asn Val Ile Ala Ser Thr Asp Cys Gly Leu
           360                 365                 370
   ggc gga cgt ctg cat tcc cag atc gca tgg gca aag ctg gag tcc cta   1267
   Gly Gly Arg Leu His Ser Gln Ile Ala Trp Ala Lys Leu Glu Ser Leu
       375                 380                 385
   gta gag ggc gct cgc att gca tca aag gaa ctg ttc taagctagac        1313
   Val Glu Gly Ala Arg Ile Ala Ser Lys Glu Leu Phe
   390                 395                 400
   aacgagggtt gct                                                    1326
<210> 110
   <211>401
   <212> PRT
   <213> Corynebacterium glutamicum
<400> 110
   Met Ser Gln Asn Arg Ile Arg Thr Thr His Val Gly Ser Leu Pro Arg
   1               5                   10                  15
   Thr Pro Glu Leu Leu Asp Ala Asn Ile Lys Arg Ser Asn Gly Glu Ile
               20                  25                  30
   Gly Glu Glu Glu Phe Phe Gln Ile Leu Gln Ser Ser Val Asp Asp Val
           35                  40                  45
   Ile Lys Arg Gln Val Asp Leu Gly Ile Asp Ile Leu Asn Glu Gly Glu
       50                  55                  60
   Tyr Gly His Val Thr Ser Gly Ala Val Asp Phe Gly Ala Trp Trp Asn
   65                  70                  75                  80
   Tyr Ser Phe Thr Arg Leu Gly Gly Leu Thr Met Thr Asp Thr Asp Arg
                   85                  90                  95
   Trp Ala Ser Gln Glu Ala Val Arg Ser Thr Pro Gly Asn Ile Glu Leu
               100                 105                 110
   Thr Ser Phe Ser Asp Arg Arg Asp Arg Ala Leu Phe Ser Glu Ala Tyr
           115                 120                 125
   Glu Asp Pro Val Ser Gly Ile Phe Thr Gly Arg Ala Ser Val Gly Asn
       130                 135                 140
   Pro Glu Phe Thr Gly Pro Ile Thr Tyr Ile Gly Gln Glu Glu Thr Gln
   145                 150                 155                 160
   Thr Asp Val Asp Leu Leu Lys Lys Gly Met Asn Ala Ala Gly Ala Thr
                   165                 170                 175
   Asp Gly Phe Val Ala Ala Leu Ser Pro Gly Ser Ala Ala Arg Leu Thr
               180                 185                 190
   Asn Lys Phe Tyr Asp Thr Asp Glu Glu Val Val Ala Ala Cys Ala Asp
           195                 200                 205
   Ala Leu Ser Gln Glu Tyr Lys Ile Ile Thr Asp Ala Gly Leu Thr Val
       210                 215                 220
   Gln Leu Asp Ala Pro Asp Leu Ala Glu Ala Trp Asp Gln Ile Asn Pro
   225                 230                 235                 240
   Glu Pro Ser Val Lys Asp Tyr Leu Asp Trp Ile Gly Thr Arg Ile Asp
                   245                 250                 255
   Ala Ile Asn Ser Ala Val Lys Gly Leu Pro Lys Glu Gln Thr Arg Leu
               260                 265                 270
   His Ile Cys Trp Gly Ser Trp His Gly Pro His Val Thr Asp Ile Pro
           275                 280                 285
   Phe Gly Asp Ile Ile Gly Glu Ile Leu Arg Ala Glu Val Gly Gly Phe
       290                 295                 300
   Ser Phe Glu Gly Ala Ser Pro Arg His Ala His Glu Trp Arg Val Trp
   305                 310                 315                 320
   Glu Glu Asn Lys Leu Pro Glu Gly Ser Val Ile Tyr Pro Gly Val Val
                   325                 330                 335
   Ser His Ser Ile Asn Ala Val Glu His Pro Arg Leu Val Ala Asp Arg
               340                 345                 350
   Ile Val Gln Phe Ala Lys Leu Val Gly Pro Glu Asn Val Ile Ala Ser
           355                 360                 365
   Thr Asp Cys Gly Leu Gly Gly Arg Leu His Ser Gln Ile Ala Trp Ala
       370                 375                 380
   Lys Leu Glu Ser Leu Val Glu Gly Ala Arg Ile Ala Ser Lys Glu Leu
   385                 390                 395                 400
   Phe
<210> 111
   <211> 548
   <212> DNA
   <213> Corynebacterium glutamicum
<220>
   <221> CDS
   <222> (1)..(525)
   <223> FRXA02648
<400> 111
   gac gca ccg gac ttg gca gaa gca tgg gat cag atc aac cca gag cca    48
   Asp Ala Pro Asp Leu Ala Glu Ala Trp Asp Gln Ile Asn Pro Glu Pro
    1               5                  10                  15
   agc gtg aag gat tac ttg gac tgg atc ggt aca cgc atc gat gcc atc    96
   Ser Val Lys Asp Tyr Leu Asp Trp Ile Gly Thr Arg Ile Asp Ala Ile
               20                  25                  30
   aac agt gca gtg aag ggc ctt cca aag gaa cag acc cgc ctg cac atc    144
   Asn Ser Ala Val Lys Gly Leu Pro Lys Glu Gln Thr Arg Leu His Ile
           35                  40                  45
   tgc tgg ggc tct tgg cac gga cca cac gtc act gac atc cca ttc ggt    192
   Cys Trp Gly Ser Trp His Gly Pro His Val Thr Asp Ile Pro Phe Gly
       50                  55                  60
   gac atc att ggt gag atc ctg cgc gca gag gtc ggt ggc ttc tcc ttc    240
   Asp Ile Ile Gly Glu Ile Leu Arg Ala Glu Val Gly Gly Phe Ser Phe
   65                  70                  75                  80
   gaa ggc gca tct cct cgt cac gca cac gag tgg cgt gta tgg gaa gaa    288
   Glu Gly Ala Ser Pro Arg His Ala His Glu Trp Arg Val Trp Glu Glu
                   85                  90                  95
   aac aag ctt cct gaa ggc tct gtt atc tac cct ggt gtt gtg tct cac    336
   Asn Lys Leu Pro Glu Gly Ser Val Ile Tyr Pro Gly Val Val Ser His
               100                 105                 110
   tcc atc aac gct gtg gag cac cca cgc ctg gtt gct gat cgt atc gtt    384
   Ser Ile Asn Ala Val Glu His Pro Arg Leu Val Ala Asp Arg Ile Val
           115                 120                 125
   cag ttc gcc aag ctt gtt ggc cct gag aac gtc att gcg tcc act gac    432
   Gln Phe Ala Lys Leu Val Gly Pro Glu Asn Val Ile Ala Ser Thr Asp
       130                 135                 140
   tgt ggt ctg ggc gga cgt ctg cat tcc cag atc gca tgg gca aag ctg    480
   Cys Gly Leu Gly Gly Arg Leu His Ser Gln Ile Ala Trp Ala Lys Leu
   145                 150                 155                 160
   gag tcc cta gta gag ggc gct cgc att gca tca aag gaa ctg ttc        525
   Glu Ser Leu Val Glu Gly Ala Arg Ile Ala Ser Lys Glu Leu Phe
                   165                 170                 175
   taagctagac aacgagggtt gct                                          548
<210> 112
   <211> 175
   <212> PRT
   <213> Corynebacterium glutamicum
<400> 112
   Asp Ala Pro Asp Leu Ala Glu Ala Trp Asp Gln Ile Asn Pro Glu Pro
    1               5                  10                  15
   Ser Val Lys Asp Tyr Leu Asp Trp Ile Gly Thr Arg Ile Asp Ala Ile
               20                  25                  30
   Asn Ser Ala Val Lys Gly Leu Pro Lys Glu Gln Thr Arg Leu His Ile
           35                  40                  45
   Cys Trp Gly Ser Trp His Gly Pro His Val Thr Asp Ile Pro Phe Gly
       50                  55                  60
   Asp Ile Ile Gly Glu Ile Leu Arg Ala Glu Val Gly Gly Phe Ser Phe
   65                  70                  75                  80
   Glu Gly Ala Ser Pro Arg His Ala His Glu Trp Arg Val Trp Glu Glu
                   85                  90                  95
   Asn Lys Leu Pro Glu Gly Ser Val Ile Tyr Pro Gly Val Val Ser His
               100                 105                 110
   Ser Ile Asn Ala Val Glu His Pro Arg Leu Val Ala Asp Arg Ile Val
           115                 120                 125
   Gln Phe Ala Lys Leu Val Gly Pro Glu Asn Val Ile Ala Ser Thr Asp
       130                 135                 140
   Cys Gly Leu Gly Gly Arg Leu His Ser Gln Ile Ala Trp Ala Lys Leu
   145                 150                 155                 160
   Glu Ser Leu Val Glu Gly Ala Arg Ile Ala Ser Lys Glu Leu Phe
                   165                 170                 175
<210> 113
   <211> 784
   <212> DNA
   <213> Corynebacterium glutamicum
   <220>
   <221> CDS
   <222> (101)..(784)
   <223> FRXA02658
<400> 113
   atgaataaaa ttccgggtgc agtgaccgta ggtgaggtaa acgcggttag agtcgaatga 60
   gagtttgata ctttctttcg acttttagat tggattttca atg agc cag aac cgc   115
                                               Met Ser Gln Asn Arg
                                                1               5
   atc agg acc act cac gtt ggt tcc ttg ccc cgt acc cca gag cta ctt   163
   Ile Arg Thr Thr His Val Gly Ser Leu Pro Arg Thr Pro Glu Leu Leu
                   10                  15                  20
   gat gca aac atc aag cgt tct aac ggt gag att ggg gag gag gaa ttc   211
   Asp Ala Asn Ile Lys Arg Ser Asn Gly Glu Ile Gly Glu Glu Glu Phe
               25                  30                  35
   ttc cag att ctg cag tct tct gta gat gac gtg atc aag cgc cag gtt   259
   Phe Gln Ile Leu Gln Ser Ser Val Asp Asp Val Ile Lys Arg Gln Val
           40                  45                  50
   gac ctg ggt atc gac atc ctt aac gag ggc gaa tac ggc cac gtc acc   307
   Asp Leu Gly Ile Asp Ile Leu Asn Glu Gly Glu Tyr Gly His Val Thr
       55                  60                  65
   tcc ggt gca gtt gac ttc ggt gca tgg tgg aac tac tcc ttc acc cgc   355
   Ser Gly Ala Val Asp Phe Gly Ala Trp Trp Asn Tyr Ser Phe Thr Arg
   70                  75                  80                  85
   ctg ggc gga ctg acc atg acc gat acc gac cgt tgg gca agc cag gaa   403
   Leu Gly Gly Leu Thr Met Thr Asp Thr Asp Arg Trp Ala Ser Gln Glu
                   90                  95                  100
   gca gtg cgt tcc acc cct ggc aac atc gag ctg acc agc ttc tct gat   451
   Ala Val Arg Ser Thr Pro Gly Asn Ile Glu Leu Thr Ser Phe Ser Asp
               105                 110                 115
   cgt cgc gac cgc gca ttg ttc agc gaa gca tac gag gat cca gta tct   499
   Arg Arg Asp Arg Ala Leu Phe Ser Glu Ala Tyr Glu Asp Pro Val Ser
           120                 125                 130
   ggc atc ttc acc ggt cgc gct tct gtg ggc aac cca gag ttc acc gga   547
   Gly Ile Phe Thr Gly Arg Ala Ser Val Gly Asn Pro Glu Phe Thr Gly
       135                 140                 145
   cct att acc tac att ggc cag gaa gaa act cag acg gat gtt gat ctg   595
   Pro Ile Thr Tyr Ile Gly Gln Glu Glu Thr Gln Thr Asp Val Asp Leu
   150                 155                 160                 165
   ctg aag aag ggc atg aac gca gcg gga gct acc gac ggc ttc gtt gca   643
   Leu Lys Lys Gly Met Asn Ala Ala Gly Ala Thr Asp Gly Phe Val Ala
                   170                 175                 180
   gca cta tcc cca gga tct gca gct cga ttg acc aac aag ttc tac gac   691
   Ala Leu Ser Pro Gly Ser Ala Ala Arg Leu Thr Asn Lys Phe Tyr Asp
               185                 190                 195
   act gat gaa gaa gtc gtc gca gca tgt gct gat gcg ctt tcc cag gaa   739
   Thr Asp Glu Glu Val Val Ala Ala Cys Ala Asp Ala Leu Ser Gln Glu
           200                 205                 210
   tac aag atc atc acc gat gca ggt ctg acc gtt cag ctc gac gca        784
   Tyr Lys Ile Ile Thr Asp Ala Gly Leu Thr Val Gln Leu Asp Ala
       215                 220                 225
<210> 114
   <211> 228
   <212> PRT
   <213> Corynebacterium glutamicum
<400> 114
   Met Ser Gln Asn Arg Ile Arg Thr Thr His Val Gly Ser Leu Pro Arg
    1               5                  10                  15
   Thr Pro Glu Leu Leu Asp Ala Asn Ile Lys Arg Ser Asn Gly Glu Ile
               20                  25                  30
   Gly Glu Glu Glu Phe Phe Gln Ile Leu Gln Ser Ser Val Asp Asp Val
           35                  40                  45
   Ile Lys Arg Gln Val Asp Leu Gly Ile Asp Ile Leu Asn Glu Gly Glu
       50                  55                  60
   Tyr Gly His Val Thr Ser Gly Ala Val Asp Phe Gly Ala Trp Trp Asn
   65                  70                  75                  80
   Tyr Ser Phe Thr Arg Leu Gly Gly Leu Thr Met Thr Asp Thr Asp Arg
                   85                  90                  95
   Trp Ala Ser Gln Glu Ala Val Arg Ser Thr Pro Gly Asn Ile Glu Leu
               100                 105                 110
   Thr Ser Phe Ser Asp Arg Arg Asp Arg Ala Leu Phe Ser Glu Ala Tyr
           115                 120                 125
   Glu Asp Pro Val Ser Gly Ile Phe Thr Gly Arg Ala Ser Val Gly Asn
       130                 135                 140
   Pro Glu Phe Thr Gly Pro Ile Thr Tyr Ile Gly Gln Glu Glu Thr Gln
   145                 150                 155                 160
   Thr Asp Val Asp Leu Leu Lys Lys Gly Met Asn Ala Ala Gly Ala Thr
                   165                 170                 175
   Asp Gly Phe Val Ala Ala Leu Ser Pro Gly Ser Ala Ala Arg Leu Thr
               180                 185                 190
   Asn Lys Phe Tyr Asp Thr Asp Glu Glu Val Val Ala Ala Cys Ala Asp
           195                 200                 205
   Ala Leu Ser Gln Glu Tyr Lys Ile Ile Thr Asp Ala Gly Leu Thr Val
       210                 215                 220
   Gln Leu Asp Ala
   225
<210> 115
   <211> 408
   <212> DNA
   <213> Corynebacterium glutamicum
<220>
   <221> CDS
   <222> (101)..(385)
   <223> RXC02238
<400> 115
   ggcgcttagc caaaacatag agcggtaggg tatgcttatc cgattgagca acctttcccg 60
   ctcttaacac tactgtccat atacttttga aaaggtgtca gtg acc aac gtg agc   115
                                              Val Thr Asn Val Ser
                                              1               5
   aac gag acc aac gcc acc aag gcc gtc ttc gat ccg cca gtg ggc att   163
   Asn Glu Thr Asn Ala Thr Lys Ala Val Phe Asp Pro Pro Val Gly Ile
                   10                  15                  20
   acc gct cct ccg atc gat gaa ctg ctg gat aag gtc act tcc aag tac   211
   Thr Ala Pro Pro Ile Asp Glu Leu Leu Asp Lys Val Thr Ser Lys Tyr
               25                  30                  35
   gcc ctc gtg atc ttc gca gcc aag cgt gcg cgc cag atc aac agc ttc   259
   Ala Leu Val Ile Phe Ala Ala Lys Arg Ala Arg Gln Ile Asn Ser Phe
           40                  45                  50
   tac cat cag gca gat gag gga gta ttc gag ttc atc gga cca ttg gtt   307
   Tyr His Gln Ala Asp Glu Gly Val Phe Glu Phe Ile Gly Pro Leu Val
       55                  60                  65
   act ccg cag cca ggc gaa aag cca ctt tct att gct ctg cgt gag atc   355
   Thr Pro Gln Pro Gly Glu Lys Pro Leu Ser Ile Ala Leu Arg Glu Ile
   70                  75                  80                  85
   aat gca ggt ctg ttg gac cac gag gaa ggt taaaagacct tataacttca     405
   Asn Ala Gly Leu Leu Asp His Glu Glu Gly
                   90                  95
   cac                                                               408
<210> 116
   <211> 95
   <212> PRT
   <213> Corynebacterium glutamicum
<400> 116
   Val Thr Asn Val Ser Asn Glu Thr Asn Ala Thr Lys Ala Val Phe Asp
   1               5                   10                  15
   Pro Pro Val Gly Ile Thr Ala Pro Pro Ile Asp Glu Leu Leu Asp Lys
               20                  25                  30
   Val Thr Ser Lys Tyr Ala Leu Val Ile Phe Ala Ala Lys Arg Ala Arg
           35                  40                  45
   Gln Ile Asn Ser Phe Tyr His Gln Ala Asp Glu Gly Val Phe Glu Phe
       50                  55                  60
   Ile Gly Pro Leu Val Thr Pro Gln Pro Gly Glu Lys Pro Leu Ser Ile
   65 70 75 80
   Ala Leu Arg Glu Ile Asn Ala Gly Leu Leu Asp His Glu Glu Gly
   85 90 95
<210> 117
   <211> 1827
   <212> DNA
   <213> Corynebacterium glutamicum
<220>
   <221> CDS
   <222> (101)..(1804)
   <223> RXC00128
<400> 117
   ccattttccg tttggtcttg cctaaagaac cgcatggaaa ttatcgtgaa gcaccgatcc 60
   cgttgatcgc tccagagaca ccgtgggaag gggagcagca gtg agt aaa att tcg   115
                                              Val Ser Lys Ile Ser
                                               1               5
   acg aaa ctg aag gcc ctc acc gcg gtg ctg tct gtg acc act ctg gtg   163
   Thr Lys Leu Lys Ala Leu Thr Ala Val Leu Ser Val Thr Thr Leu Val
                   10                  15                  20
   gct ggg tgt tcc acg ctt ccg cag aac acg gat ccg caa gtg ctg cgc   211
   Ala Gly Cys Ser Thr Leu Pro Gln Asn Thr Asp Pro Gln Val Leu Arg
               25                  30                  35
   tca ttt tcc ggg tcc caa agc aca caa gag ata gca ggg ccg acc ccg   259
   Ser Phe Ser Gly Ser Gln Ser Thr Gln Glu Ile Ala Gly Pro Thr Pro
           40                  45                  50
   aat caa gat ccg gat ttg ttg atc cgc ggc ttc ttc agc gca ggt gcg   307
   Asn Gln Asp Pro Asp Leu Leu Ile Arg Gly Phe Phe Ser Ala Gly Ala
       55                  60                  65
   tat ccg act cag cag tat gaa gcg gcg aag gcg tat ctg acg gaa ggg   355
   Tyr Pro Thr Gln Gln Tyr Glu Ala Ala Lys Ala Tyr Leu Thr Glu Gly
   70                  75                  80                  85
   acg cgc agc acg tgg aat ccg gct gcg tcg act cgt att ttg gat cgc   403
   Thr Arg Ser Thr Trp Asn Pro Ala Ala Ser Thr Arg Ile Leu Asp Arg
                   90                  95                  100
   att gat ctg aac act ctg cca ggt tcg acg aat gcg gaa cga acg att   451
   Ile Asp Leu Asn Thr Leu Pro Gly Ser Thr Asn Ala Glu Arg Thr Ile
               105                 110                 115
   gcg atc cgt gga acg cag gtc gga acg ttg ctc agc ggt ggc gtg tat   499
   Ala Ile Arg Gly Thr Gln Val Gly Thr Leu Leu Ser Gly Gly Val Tyr
           120                 125                 130
   cag ccg gag aat gcg gag ttt gaa gct gag atc acg atg cgt cgg gaa   547
   Gln Pro Glu Asn Ala Glu Phe Glu Ala Glu Ile Thr Met Arg Arg Glu
       135                 140                 145
   gat ggg gag tgg cgt atc gat gct ttg ccg gac ggg att tta tta gag   595
   Asp Gly Glu Trp Arg Ile Asp Ala Leu Pro Asp Gly Ile Leu Leu Glu
   150                 155                 160                 165
   aga aac gat ctg cgg aac cat tac act ccg cac gat gtg tat ttc ttt     643
   Arg Asn Asp Leu Arg Asn His Tyr Thr Pro His Asp Val Tyr Phe Phe
                   170                 175                 180
   gat cct tct ggc cag gtg ttg gtg ggg gat cgg cgt tgg ttg ttc aat     691
   Asp Pro Ser Gly Gln Val Leu Val Gly Asp Arg Arg Trp Leu Phe Asn
               185                 190                 195
   gag tcg cag tcg atg tcc acg gtg ctg atg gcc ctt ctg gtt aat ggt     739
   Glu Ser Gln Ser Met Ser Thr Val Leu Met Ala Leu Leu Val Asn Gly
           200                 205                 210
   cct tcg ccg gca att tct cct ggt gtg gtc aat cag ctg tcc acg gat     787
   Pro Ser Pro Ala Ile Ser Pro Gly Val Val Asn Gln Leu Ser Thr Asp
       215                 220                 225
   gcg tcg ttc gtg ggg ttc aat gat ggg gag tat cag ttc act ggt ttg     835
   Ala Ser Phe Val Gly Phe Asn Asp Gly Glu Tyr Gln Phe Thr Gly Leu
   230                 235                 240                 245
   gga aat ttg gat gat gat gcg cgt ttg cgt ttc gcc gcc cag gcc gtg     883
   Gly Asn Leu Asp Asp Asp Ala Arg Leu Arg Phe Ala Ala Gln Ala Val
                   250                 255                 260
   tgg acg ttg gcg cat gct gat gtc gca ggc ccc tac act ttg gtc gct     931
   Trp Thr Leu Ala His Ala Asp Val Ala Gly Pro Tyr Thr Leu Val Ala
               265                 270                 275
   gac ggc gcg ccg ttg ctg tcg gag ttc cca acg ctc acc acc gat gac     979
   Asp Gly Ala Pro Leu Leu Ser Glu Phe Pro Thr Leu Thr Thr Asp Asp
           280                 285                 290
   ctc gcc gaa tac aac cca gag gct tac acc aac acg gtg tcc acg ttg     1027
   Leu Ala Glu Tyr Asn Pro Glu Ala Tyr Thr Asn Thr Val Ser Thr Leu
       295                 300                 305
   ttt gcg ttg cag gat gga tcg ttg tcg agg gtc agt tcc ggc aat gtg     1075
   Phe Ala Leu Gln Asp Gly Ser Leu Ser Arg Val Ser Ser Gly Asn Val
   310                 315                 320                 325
   agt cca cta cag ggc att tgg agc ggt gga gat atc gat tct gca gcg     1123
   Ser Pro Leu Gln Gly Ile Trp Ser Gly Gly Asp Ile Asp Ser Ala Ala
                   330                 335                 340
   att tcc tcc tcc gcc aat gtg gtg gca gcg gta cgc cac gaa aac aac     1171
   Ile Ser Ser Ser Ala Asn Val Val Ala Ala Val Arg His Glu Asn Asn
               345                 350                 355
   gag gca gtg ctt act gtt ggc tcc atg gaa ggc gtg act tca gat gcg     1219
   Glu Ala Val Leu Thr Val Gly Ser Met Glu Gly Val Thr Ser Asp Ala
           360                 365                 370
   ttg agg agt gaa acg atc act cgt ccc acc ttt gaa tac gcg tcg agt     1267
   Leu Arg Ser Glu Thr Ile Thr Arg Pro Thr Phe Glu Tyr Ala Ser Ser
       375                 380                 385
   ggg ttg tgg gct gtg gtg gat ggg gag acg cct gtc cga gtc gca cga     1315
   Gly Leu Trp Ala Val Val Asp Gly Glu Thr Pro Val Arg Val Ala Arg
   390                 395                 400                 405
   tcg gca aca acc ggt gag ctc gtc cag acg gag gcg gag att gtg ctg    1363
   Ser Ala Thr Thr Gly Glu Leu Val Gln Thr Glu Ala Glu Ile Val Leu
                   410                 415                 420
   cca agg gat gtg acg ggt ccg atc tct gaa ttc caa ctg tca cga act    1411
   Pro Arg Asp Val Thr Gly Pro Ile Ser Glu Phe Gln Leu Ser Arg Thr
               425                 430                 435
   ggg gtc cgg gcc gcc atg atc att gaa ggc aag gtg tac gtg ggc gtc    1459
   Gly Val Arg Ala Ala Met Ile Ile Glu Gly Lys Val Tyr Val Gly Val
           440                 445                 450
   gta acg cgt cct ggt ccg ggc gag cgg cgc gtg aca aat atc acg gag    1507
   Val Thr Arg Pro Gly Pro Gly Glu Arg Arg Val Thr Asn Ile Thr Glu
       455                 460                 465
   gtg gcg ccg agc ttg ggc gag gcg gcg ctg tcg atc aac tgg cgc cca    1555
   Val Ala Pro Ser Leu Gly Glu Ala Ala Leu Ser Ile Asn Trp Arg Pro
   470                 475                 480                 485
   gac ggc att ttg ctt gtg ggc acg tca att cca gag acg ccg ctg tgg    1603
   Asp Gly Ile Leu Leu Val Gly Thr Ser Ile Pro Glu Thr Pro Leu Trp
                   490                 495                 500
   cgc gtc gag cag gac gga tcg gcg att tcg tcg atg ccg agc ggg aat    1651
   Arg Val Glu Gln Asp Gly Ser Ala Ile Ser Ser Met Pro Ser Gly Asn
               505                 510                 515
   ctc agc gcg ccg gtg gtg gcg gtg gca agt tcc gcg acg acg gtc tac    1699
   Leu Ser Ala Pro Val Val Ala Val Ala Ser Ser Ala Thr Thr Val Tyr
           520                 525                 530
   gtc act gat tcg cat gcg atg ctt cag ctg ccg act gcc gat aat gat    1747
   Val Thr Asp Ser His Ala Met Leu Gln Leu Pro Thr Ala Asp Asn Asp
       535                 540                 545
   att tgg cgc gag gtg ccc ggt ttg ctg ggc acg cgt gcg gcg ccg gtg    1795
   Ile Trp Arg Glu Val Pro Gly Leu Leu Gly Thr Arg Ala Ala Pro Val
   550                 555                 560                 565
   gtt gcg tac tgatggagct gttcttcccg cgc                              1827
   Val Ala Tyr
<210> 118
   <211> 568
   <212> PRT
   <213> Corynebacterium glutamicum
<400> 118
   Val Ser Lys Ile Ser Thr Lys Leu Lys Ala Leu Thr Ala Val Leu Ser
    1               5                  10                  15
   Val Thr Thr Leu Val Ala Gly Cys Ser Thr Leu Pro Gln Asn Thr Asp
               20                  25                  30
   Pro Gln Val Leu Arg Ser Phe Ser Gly Ser Gln Ser Thr Gln Glu Ile
           35                  40                  45
   Ala Gly Pro Thr Pro Asn Gln Asp Pro Asp Leu Leu Ile Arg Gly Phe
       50                  55                  60
   Phe Ser Ala Gly Ala Tyr Pro Thr Gln Gln Tyr Glu Ala Ala Lys Ala
   65                  70                  75                  80
   Tyr Leu Thr Glu Gly Thr Arg Ser Thr Trp Asn Pro Ala Ala Ser Thr
                   85                  90                  95
   Arg Ile Leu Asp Arg Ile Asp Leu Asn Thr Leu Pro Gly Ser Thr Asn
               100                 105                 110
   Ala Glu Arg Thr Ile Ala Ile Arg Gly Thr Gln Val Gly Thr Leu Leu
           115                 120                 125
   Ser Gly Gly Val Tyr Gln Pro Glu Asn Ala Glu Phe Glu Ala Glu Ile
       130                 135                 140
   Thr Met Arg Arg Glu Asp Gly Glu Trp Arg Ile Asp Ala Leu Pro Asp
   145                 150                 155                 160
   Gly Ile Leu Leu Glu Arg Asn Asp Leu Arg Asn His Tyr Thr Pro His
                   165                 170                 175
   Asp Val Tyr Phe Phe Asp Pro Ser Gly Gln Val Leu Val Gly Asp Arg
               180                 185                 190
   Arg Trp Leu Phe Asn Glu Ser Gln Ser Met Ser Thr Val Leu Met Ala
           195                 200                 205
   Leu Leu Val Asn Gly Pro Ser Pro Ala Ile Ser Pro Gly Val Val Asn
       210                 215                 220
   Gln Leu Ser Thr Asp Ala Ser Phe Val Gly Phe Asn Asp Gly Glu Tyr
   225                 230                 235                 240
   Gln Phe Thr Gly Leu Gly Asn Leu Asp Asp Asp Ala Arg Leu Arg Phe
                   245                 250                 255
   Ala Ala Gln Ala Val Trp Thr Leu Ala His Ala Asp Val Ala Gly Pro
               260                 265                 270
   Tyr Thr Leu Val Ala Asp Gly Ala Pro Leu Leu Ser Glu Phe Pro Thr
           275                 280                 285
   Leu Thr Thr Asp Asp Leu Ala Glu Tyr Asn Pro Glu Ala Tyr Thr Asn
       290                 295                 300
   Thr Val Ser Thr Leu Phe Ala Leu Gln Asp Gly Ser Leu Ser Arg Val
   305                 310                 315                 320
   Ser Ser Gly Asn Val Ser Pro Leu Gln Gly Ile Trp Ser Gly Gly Asp
                   325                 330                 335
   Ile Asp Ser Ala Ala Ile Ser Ser Ser Ala Asn Val Val Ala Ala Val
               340                 345                 350
   Arg His Glu Asn Asn Glu Ala Val Leu Thr Val Gly Ser Met Glu Gly
           355                 360                 365
   Val Thr Ser Asp Ala Leu Arg Ser Glu Thr Ile Thr Arg Pro Thr Phe
   370 375 380
   Glu Tyr Ala Ser Ser Gly Leu Trp Ala Val Val Asp Gly Glu Thr Pro
   385 390 395 400
   Val Arg Val Ala Arg Ser Ala Thr Thr Gly Glu Leu Val Gln Thr Glu
   405 410 415
   Ala Glu Ile Val Leu Pro Arg Asp Val Thr Gly Pro Ile Ser Glu Phe
   420 425 430
   Gln Leu Ser Arg Thr Gly Val Arg Ala Ala Met Ile Ile Glu Gly Lys
   435 440 445
   Val Tyr Val Gly Val Val Thr Arg Pro Gly Pro Gly Glu Arg Arg Val
   450 455 460
   Thr Asn Ile Thr Glu Val Ala Pro Ser Leu Gly Glu Ala Ala Leu Ser
   465 470 475 480
   Ile Asn Trp Arg Pro Asp Gly Ile Leu Leu Val Gly Thr Ser Ile Pro
   485 490 495
   Glu Thr Pro Leu Trp Arg Val Glu Gln Asp Gly Ser Ala Ile Ser Ser
   500 505 510
   Met Pro Ser Gly Asn Leu Ser Ala Pro Val Val Ala Val Ala Ser Ser
   515 520 525
   Ala Thr Thr Val Tyr Val Thr Asp Ser His Ala Met Leu Gln Leu Pro
   530 535 540
   Thr Ala Asp Asn Asp Ile Trp Arg Glu Val Pro Gly Leu Leu Gly Thr
   545 550 555 560
   Arg Ala Ala Pro Val Val Ala Tyr
   565
<210> 119
   <211> 1344
   <212> DNA
   <213> Corynebacterium glutamicum
<220>
   <221> CDS
   <222> (101)..(1321)
   <223> RNA02240
<400> 119
   cagctagacc actgacattg cagttttaga cagcttggtc tatattggtt ttttgtattt 60
   aagactattt attctcaact tcttcgaaag aagggtattt gtg gct cag cca acc 115
   Val Ala Gln Pro Thr
   1 5
   gcc gtc cgt ttg ttc acc agt gaa tct gta act gag gga cat cca gac 163
   Ala Val Arg Leu Phe Thr Ser Glu Ser Val Thr Glu Gly His Pro Asp
   10 15 20
   aaa ata tgt gat gct att tcc gat acc att ttg gac gcg ctg ctc gaa    211
   Lys Ile Cys Asp Ala Ile Ser Asp Thr Ile Leu Asp Ala Leu Leu Glu
               25                  30                  35
   aaa gat ccg cag tcg cgc gtc gca gtg gaa act gtg gtc acc acc gga    259
   Lys Asp Pro Gln Ser Arg Val Ala Val Glu Thr Val Val Thr Thr Gly
           40                  45                  50
   atc gtc cat gtt gtt ggc gag gtc cgt acc agc gct tac gta gag atc    307
   Ile Val His Val Val Gly Glu Val Arg Thr Ser Ala Tyr Val Glu Ile
       55                  60                  65
   cct caa tta gtc cgc aac aag ctc atc gaa atc gga ttc aac tcc tct    355
   Pro Gln Leu Val Arg Asn Lys Leu Ile Glu Ile Gly Phe Asn Ser Ser
   70                  75                  80                  85
   gag gtt gga ttc gac gga cgc acc tgt ggc gtc tca gta tcc atc ggt    403
   Glu Val Gly Phe Asp Gly Arg Thr Cys Gly Val Ser Val Ser Ile Gly
                   90                  95                  100
   gag cag tcc cag gaa atc gct gac ggc gtg gat aac tcc gac gaa gcc    451
   Glu Gln Ser Gln Glu Ile Ala Asp Gly Val Asp Asn Ser Asp Glu Ala
               105                 110                 115
   cgc acc aac ggc gac gtt gaa gaa gac gac cgc gca ggt gct ggc gac    499
   Arg Thr Asn Gly Asp Val Glu Glu Asp Asp Arg Ala Gly Ala Gly Asp
           120                 125                 130
   cag ggc ctg atg ttc ggc tac gcc acc aac gaa acc gaa gag tac atg    547
   Gln Gly Leu Met Phe Gly Tyr Ala Thr Asn Glu Thr Glu Glu Tyr Met
       135                 140                 145
   cct ctt cct atc gcg ttg gcg cac cga ctg tca cgt cgt ctg acc cag    595
   Pro Leu Pro Ile Ala Leu Ala His Arg Leu Ser Arg Arg Leu Thr Gln
   150                 155                 160                 165
   gtt cgt aaa gag ggc atc gtt cct cac ctg cgt cca gac gga aaa acc    643
   Val Arg Lys Glu Gly Ile Val Pro His Leu Arg Pro Asp Gly Lys Thr
                   170                 175                 180
   cag gtc acc ttc gca tac gat gcg caa gac cgc cct agc cac ctg gat    691
   Gln Val Thr Phe Ala Tyr Asp Ala Gln Asp Arg Pro Ser His Leu Asp
               185                 190                 195
   acc gtt gtc atc tcc acc cag cac gac cca gaa gtt gac cgt gca tgg    739
   Thr Val Val Ile Ser Thr Gln His Asp Pro Glu Val Asp Arg Ala Trp
           200                 205                 210
   ttg gaa acc caa ctg cgc gaa cac gtc att gat tgg gta atc aaa gac    787
   Leu Glu Thr Gln Leu Arg Glu His Val Ile Asp Trp Val Ile Lys Asp
       215                 220                 225
   gca ggc att gag gat ctg gca acc ggt gag atc acc gtg ttg atc aac    835
   Ala Gly Ile Glu Asp Leu Ala Thr Gly Glu Ile Thr Val Leu Ile Asn
   230                 235                 240                 245
   cct tca ggt tcc ttc att ctg ggt ggc ccc atg ggt gat gcg ggt ctg    883
   Pro Ser Gly Ser Phe Ile Leu Gly Gly Pro Met Gly Asp Ala Gly Leu
                   250                 255                 260
   acc ggc cgc aag atc atc gtg gat acc tac ggt ggc atg gct cgc cat    931
   Thr Gly Arg Lys Ile Ile Val Asp Thr Tyr Gly Gly Met Ala Arg His
               265                 270                 275
   ggt ggt gga gca ttc tcc ggt aag gat cca agc aag gtg gac cgc tct      979
   Gly Gly Gly Ala Phe Ser Gly Lys Asp Pro Ser Lys Val Asp Arg Ser
           280                 285                 290
   gct gca tac gcc atg cgt tgg gta gca aag aac atc gtg gca gca ggc     1027
   Ala Ala Tyr Ala Met Arg Trp Val Ala Lys Asn Ile Val Ala Ala Gly
       295                 300                 305
   ctt gct gat cgc gct gaa gtt cag gtt gca tac gcc att gga cgc gca     1075
   Leu Ala Asp Arg Ala Glu Val Gln Val Ala Tyr Ala Ile Gly Arg Ala
   310                 315                 320                 325
   aag cca gtc gga ctt tac gtt gaa acc ttt gac acc aac aag gaa ggc     1123
   Lys Pro Val Gly Leu Tyr Val Glu Thr Phe Asp Thr Asn Lys Glu Gly
                   330                 335                 340
   ctg agc gac gag cag att cag gct gcc gtg ttg gag gtc ttt gac ctg     1171
   Leu Ser Asp Glu Gln Ile Gln Ala Ala Val Leu Glu Val Phe Asp Leu
               345                 350                 355
   cgt cca gca gca att atc cgt gag ctt gat ctg ctt cgt ccg atc tac     1219
   Arg Pro Ala Ala Ile Ile Arg Glu Leu Asp Leu Leu Arg Pro Ile Tyr
           360                 365                 370
   gct gac act gct gcc tac ggc cac ttt ggt cgc act gat ttg gac ctt     1267
   Ala Asp Thr Ala Ala Tyr Gly His Phe Gly Arg Thr Asp Leu Asp Leu
       375                 380                 385
   cct tgg gag gct atc gac cgc gtt gat gaa ctt cgc gca gcc ctc aag     1315
   Pro Trp Glu Ala Ile Asp Arg Val Asp Glu Leu Arg Ala Ala Leu Lys
   390                 395                 400                 405
   ttg gcc taaaaatctg atgtagtatc ttc                                   1344
   Leu Ala
<210> 120
   <211> 407
   <212> PRT
   <213> Corynebacterium glutamicum
<400> 120
   Val Ala Gln Pro Thr Ala Val Arg Leu Phe Thr Ser Glu Ser Val Thr
    1               5                  10                  15
   Glu Gly His Pro Asp Lys Ile Cys Asp Ala Ile Ser Asp Thr Ile Leu
               20                  25                  30
   Asp Ala Leu Leu Glu Lys Asp Pro Gln Ser Arg Val Ala Val Glu Thr
           35                  40                  45
   Val Val Thr Thr Gly Ile Val His Val Val Gly Glu Val Arg Thr Ser
       50                  55                  60
   Ala Tyr Val Glu Ile Pro Gln Leu Val Arg Asn Lys Leu Ile Glu Ile
   65                  70                  75                  80
   Gly Phe Asn Ser Ser Glu Val Gly Phe Asp Gly Arg Thr Cys Gly Val
                   85                  90                  95
   Ser Val Ser Ile Gly Glu Gln Ser Gln Glu Ile Ala Asp Gly Val Asp
               100                 105                 110
   Asn Ser Asp Glu Ala Arg Thr Asn Gly Asp Val Glu Glu Asp Asp Arg
           115                 120                 125
   Ala Gly Ala Gly Asp Gln Gly Leu Met Phe Gly Tyr Ala Thr Asn Glu
       130                 135                 140
   Thr Glu Glu Tyr Met Pro Leu Pro Ile Ala Leu Ala His Arg Leu Ser
   145                 150                 155                 160
   Arg Arg Leu Thr Gln Val Arg Lys Glu Gly Ile Val Pro His Leu Arg
                   165                 170                 175
   Pro Asp Gly Lys Thr Gln Val Thr Phe Ala Tyr Asp Ala Gln Asp Arg
               180                 185                 190
   Pro Ser His Leu Asp Thr Val Val Ile Ser Thr Gln His Asp Pro Glu
           195                 200                 205
   Val Asp Arg Ala Trp Leu Glu Thr Gln Leu Arg Glu His Val Ile Asp
       210                 215                 220
   Trp Val Ile Lys Asp Ala Gly Ile Glu Asp Leu Ala Thr Gly Glu Ile
   225                 230                 235                 240
   Thr Val Leu Ile Asn Pro Ser Gly Ser Phe Ile Leu Gly Gly Pro Met
                   245                 250                 255
   Gly Asp Ala Gly Leu Thr Gly Arg Lys Ile Ile Val Asp Thr Tyr Gly
               260                 265                 270
   Gly Met Ala Arg His Gly Gly Gly Ala Phe Ser Gly Lys Asp Pro Ser
           275                 280                 285
   Lys Val Asp Arg Ser Ala Ala Tyr Ala Met Arg Trp Val Ala Lys Asn
       290                 295                 300
   Ile Val Ala Ala Gly Leu Ala Asp Arg Ala Glu Val Gln Val Ala Tyr
   305                 310                 315                 320
   Ala Ile Gly Arg Ala Lys Pro Val Gly Leu Tyr Val Glu Thr Phe Asp
                   325                 330                 335
   Thr Asn Lys Glu Gly Leu Ser Asp Glu Gln Ile Gln Ala Ala Val Leu
               340                 345                 350
   Glu Val Phe Asp Leu Arg Pro Ala Ala Ile Ile Arg Glu Leu Asp Leu
           355                 360                 365
   Leu Arg Pro Ile Tyr Ala Asp Thr Ala Ala Tyr Gly His Phe Gly Arg
       370                 375                 380
   Thr Asp Leu Asp Leu Pro Trp Glu Ala Ile Asp Arg Val Asp Glu Leu
   385                 390                 395                 400
   Arg Ala Ala Leu Lys Leu Ala
<210> 121
   <211> 23
   <212> DNA
   <213> Artificial Sequence
<220>
   <223> Description of Artificial Sequence: primer
<400> 121
   tcgggtatcc gcgctccactt aga 23
<210> 122
   <211> 23
   <212> DNA
   <213> Artificial Sequence
<220>
   <223> Description of Artificial Sequence: primer
<400> 122
   GGAAACCGGG GCATCGAAAC TTA 23
<210> 123
   <211> 18
   <212> DNA
   <213> Artificial Sequence
<220>
   <223> Description of Artificial Sequence: primer
<400> 123
   ggaaacagta tgaccatg 18
<210> 124
   <211> 17
   <212> DNA
   <213> Artificial Sequence
<220>
   <223> Description of Artificial Sequence: primer
<400> 124
   gtaaaacgac ggccagt 18
<210> 125
   <211> 4334
   <212> DNA
   <213> Corynebacterium glutamicum
<400> 125
   aaatcgcttg accattgcag gttggtttat gactgttgag ggagagactg gctcgtggcc 60
   gacaatcaat gaagctatgt ctgaatttag cgtgtcacgt cagaccgtga atagagcact 120
   taagtctgcg ggcattgaac ttccacgagg acgccgtaaa gcttcccagt aaatgtgcca 180
   tctcgtaggc agaaaacggt tccccccgta ggggtctctc tcttggcctc ctttctaggt 240
   cgggctgatt gctcttgaag ctctctaggg gggctcacac cataggcaga taacggttcc 300
   ccaccggctc acctcgtaag cgcacaagga ctgctcccaa agatcttcaa agccactgcc 360
   gcgactccgc ttcgcgaagc cttgccccgc ggaaatttcc tccaccgagt tcgtgcacac 420
   ccctatgcca agcttctttc accctaaatt cgagagattg gattcttacc gtggaaattc 480
   ttcgcaaaaa tcgtcccctg atcgcccttg cgacgttgct cgcggcggtg ccgctggttg 540
   cgcttggctt gaccgacttg atcagcttgc atgcctgcag gtcgacggat ccccgggtgg 600
   gaaagccacg ttgtgtctca aaatctctga tgttacattg cacaagataa aaatatatca 660
   tcatgaacaa taaaactgtc tgcttacata aacagtaata caaggggtgt tatgagccat 720
   attcaacggg aaacgtcttg ctcgaggccg cgattaaatt ccaacatgga tgctgattta 780
   tatgggtata aatgggctcg cgataatgtc gggcaatcag gtgcgacaat ctatcgattg 840
   tatgggaagc ccgatgcgcc agagttgttt ctgaaacatg gcaaaggtag cgttgccaat 900
   gatgttacag atgagatggt cagactaaac tggctgacgg aatttatgcc tcttccgacc 960
   atcaagcatt ttatccgtac tcctgatgat gcatggttac tcaccactgc gatccccggg 1020
   aaaacagcat tccaggtatt agaagaatat cctgattcag gtgaaaatat tgttgatgcg 1080
   ctggcagtgt tcctgcgccg gttgcattcg attcctgttt gtaattgtcc ttttaacagc 1140
   gatcgcgtat ttcgtctcgc tcaggcgcaa tcacgaatga ataacggttt ggttgatgcg 1200
   agtgattttg atgacgagcg taatggctgg cctgttgaac aagtctggaa agaaatgcat 1260
   aagcttttgc cattctcacc ggattcagtc gtcactcatg gtgatttctc acttgataac 1320
   cttatttttg acgaggggaa attaataggt tgtattgatg ttggacgagt cggaatcgca 1380
   gaccgatacc aggatcttgc catcctatgg aactgcctcg gtgagttttc tccttcatta 1440
   cagaaacggc tttttcaaaa atatggtatt gataatcctg atatgaataa attgcagttt 1500
   catttgatgc tcgatgagtt tttctaatca gaattggtta attggttgta acactggcag 1560
   agcattacgc tgacttgacg ggacggcggc tttgttgaat aaatcgaact tttgctgagt 1620
   tgaaggatca gatcacgcat cttcccgaca acgcagaccg ttccgtggca aagcaaaagt 1680
   tcaaaatcac caactggtcc acctacaaca aagctctcat caaccgtggc tccctcactt 1740
   tctggctgga tgatggggcg attcaggcct ggtatgagtc agcaacacct tcttcacgag 1800
   gcagacctca gcgcccccga attgatcagt actgcggcgt cgctgatcgc cctcgcgacg 1860
   ttgtgcgggt ggcttgtccc tgagggcgct gcgacagata gctaaaaatc tgcgtcagga 1920
   tcgccgtaga gcgcgcgtcg cgtcgattgg aggcttcccc tttggttgac ggtcttcaat 1980
   cgctctacgg cgatcctgac gcttttttgt tgcgtaccgt cgatcgtttt atttctgtcg 2040
   atcccgaaaa agtttttgcc ttttgtaaaa aacttctcgg tcgccccgca aattttcgat 2100
   tccagatttt ttaaaaacca agccagaaat acgacacacc gtttgcagat aatctgtctt 2160
   tcggaaaaat caagtgcgat acaaaatttt tagcacccct gagctgcgca aagtcccgct 2220
   tcgtgaaaat tttcgtgccg cgtgattttc cgccaaaaac tttaacgaac gttcgttata 2280
   atggtgtcat gaccttcacg acgaagtacc aaaattggcc cgaatcatca gctatggatc 2340
   tctctgatgt cgcgctggag tccgacgcgc tcgatgctgc cgtcgattta aaaacggtga 2400
   tcggattttt ccgagctctc gatacgacgg acgcgccagc atcacgagac tgggccagtg 2460
   ccgcgagcga cctagaaact ctcgtggcgg atcttgagga gctggctgac gagctgcgtg 2520
   ctcggcagcg ccaggaggac gcacagtagt ggaggatcga atcagttgcg cctactgcgg 2580
   tggcctgatt cctccccggc ctgacccgcg aggacggcgc gcaaaatatt gctcagatgc 2640
   gtgtcgtgcc gcagccagcc gcgagcgcgc caacaaacgc cacgccgagg agctggaggc 2700
   ggctaggtcg caaatggcgc tggaagtgcg tcccccgagc gaaattttgg ccatggtcgt 2760
   cacagagctg gaagcggcag cgagaattat ccgcgatcgt ggcgcggtgc ccgcaggcat 2820
   gacaaacatc gtaaatgccg cgtttcgtgt ggccgtggcc gcccaggacg tgtcagcgcc 2880
   gccaccacct gcaccgaatc ggcagcagcg tcgcgcgtcg aaaaagcgca caggcggcaa 2940
   gaagcgataa gctgcacgaa tacctgaaaa atgttgaacg ccccgtgagc ggtaactcac 3000
   agggcgtcgg ctaaccccca gtccaaacca gggagaaagc gctcaaaaat gactctagcg 3060
   gattcacgag acattgacac accggcctgg aaattttccg ctgatctgtt cgacacccat 3120
   cccgagctcg cgctgcgatc acgtggctgg acgagcgaag accgccgcga attcctcgct 3180
   cacctgggca gagaaaattt ccagggcagc aagacccgcg acttcgccag cgcttggatc 3240
   aaagacccgg acacgggaga aacacagccg aagttatacc gagttggttc aaaatcgctt 3300
   gcccggtgcc agtatgttgc tctgacgcac gcgcagcacg cagccgtgct tgtcctggac 3360
   attgatgtgc cgagccacca ggccggcggg aaaatcgagc acgtaaaccc cgaggtctac 3420
   gcgattttgg agcgctgggc acgcctggaa aaagcgccag cttggatcgg cgtgaatcca 3480
   ctgagcggga aatgccagct catctggctc attgatccgg tgtatgccgc agcaggcatg 3540
   agcagcccga atatgcgcct gctggctgca acgaccgagg aaatgacccg cgttttcggc 3600
   gctgaccagg ctttttcaca taggctgagc cggtggccac tgcacgtctc cgacgatccc 3660
   accgcgtacc gctggcatgc ccagcacaat cgcgtggatc gcctagctga tcttatggag 3720
   gttgctcgca tgatctcagg cacagaaaaa cctaaaaaac gctatgagca ggagttttct 3780
   agcggacggg cacgtatcga agcggcaaga aaagccactg cggaagcaaa agcacttgcc 3840
   acgcttgaag caagcctgcc gagcgccgct gaagcgtctg gagagctgat cgacggcgtc 3900
   cgtgtcctct ggactgctcc agggcgtgcc gcccgtgatg agacggcttt tcgccacgct 3960
   ttgactgtgg gataccagtt aaaagcggct ggtgagcgcc taaaagacac caagatcatc 4020
   gacgcctacg agcgtgccta caccgtcgct caggcggtcg gagcagacgg ccgtgagcct 4080
   gatctgccgc cgatgcgtga ccgccagacg atggcgcgac gtgtgcgcgg ctacgtcgct 4140
   aaaggccagc cagtcgtccc tgctcgtcag acagagacgc agagcagccg agggcgaaaa 4200
   gctctggcca ctatgggaag acgtggcggt aaaaaggccg cagaacgctg gaaagaccca 4260
   aacagtgagt acgcccgagc acagcgagaa aaactagcta agtccagtca acgacaagct 4320
   aggaaagcta aagg                                                   4334

## Claims

1. An isolated polypeptide from Corynebacterium glutamicum, said protein being involved in the metabolism of Methionine and Lysine and comprising the amino acid sequence set forth in SEQ ID NO: 2.

2. An isolated polypeptide according to claim 1, further comprising heterologous amino acid sequences.

3. An isolated nucleic acid molecule from Corynebacterium glutamicum encoding the protein of claim 1.

4. The isolated nucleic acid of a claim 3, wherein said nucleic acid comprises the nucleotide sequence set forth in SEQ ID NO: 1 or a complement thereof.

5. A vector comprising the nucleic acid of claim 4.

6. The vector of claim 5, further comprising one or more nucleic acids encoding proteins involved in metabolic pathways.

7. The vector of claim 6, wherein the second and further nucleic acids are selected from the odd-numbered sequences listed in Table 1, excluding any F-designated nucleic acid molecule.

8. The vector of any one of claims 5 or 6, which is an expression vector.

9. A host cell transfected with the expression vector of claim 8, wherein said cell is a microorganism.

10. The host cell of claim 9, wherein the cell belongs to the genus Corynebacterium or Brevibacterium.

11. The cell of claim 10, selected from the group consisting of
*Corynebacterium glutamicum, Corynebacterium herculis, Corynebacterium lilium, Corynebacterium acetoacidophilum, Corynebacterium acetoglutamicum, Corynebacterium acetophilum, Corynebacterium ammoniagenes, Corynebacterium fujiokense, Corynebacterium nitrilophilus, Brevibacterium ammoniagenes, Brevibacterium butanicum, Brevibacterium divaricatum, Breibacterium flavum, Brevibacterium heali, Brevibacterium ketoglutamicum, Brevibacterium ketosoreductum, Brevibacterium lactogermentum, Brevibacterium linens, Brevibacterium paraffinolyticum, and those strains set forth in Table 3.*

12. The host cell of any one of claims 9, 10 or 11, wherein the expression of said nucleic acid molecule results in the modulation of the production of methionine and/or lysine.

13. A method of producing a polypeptide of claim 1 comprising culturing the host cell of claim 9 in an appropriate culture medium.

14. A method of producing a fine chemical, comprising culturing a cell of claim 12.

15. A method of claim 14, wherein said cell is cultured in the presence of a sulphur source.

16. The method of claim 14, wherein said method further comprises the step of recovering the fine chemical from said culture.

17. The method of any one of claims 14, 15 or 16, wherein the fine chemical is an amino acid.

18. The method of claim 17, wherein the amino acid is methionine or lysine.

19. A method for producing a fine chemical comprising culturing a cell whose genomic DNA has been altered by the inclusion of a nucleic acid molecule of claim 4.

20. The method of claim 19, wherein the cell's genomic DNA has been further altered by the inclusion of a nucleic acid encoding a protein involved in metabolic pathways.

21. The method of claim 20, wherein said further nucleic acid is selected from the odd-numbered sequences listed in Table 1, excluding any F-designated nucleic acid molecule.

22. The method of claims 19 or 20, wherein the nucleic acid molecule is selected from the group consisting of metC, metB, metA, metE, metH, hom, asd, lysC, lysC/ask, rxa00657, dapA, dapB, dapC, dapD/argD, dapE, dapF, LysA, ddh, lysE, lysG, lysR, hsk, ppc, pycA, accD, accA, accB, addD, gpdh genes or any combination of the above-mentioned genes.

## Patentansprüche

1. Isoliertes Polypeptid von Corynebacterium glutamicum, wobei das Protein an dem Metabolismus von Methionin und Lysin beteiligt ist, und umfassend die Aminosäuresequenz, wie in der SEQ ID NR.: 2 aufgeführt.

2. Isoliertes Polypeptid gemäß Anspruch 1, weiterhin umfassend heterologe Aminosäuresequenzen.

3. Isoliertes Nukleinsäuremolekül von Corynebacterium glutamicum, codierend das Protein von Anspruch 1.

4. Isolierte Nukleinsäure gemäß Anspruch 3, wobei die Nukleinsäure die Nukleodtidsequenz, wie in der SEQ ID NR.: 1 aufgeführt, oder ein Komplement davon umfasst.

5. Vektor, umfassend die Nukleinsäure von Anspruch 4.

6. Vektor gemäß Anspruch 5, weiterhin umfassend eine oder mehrere Nukleinsäuren, codierend Proteine, die an metabolischen Stoffwechselwegen beteiligt sind.

7. Vektor gemäß Anspruch 6, wobei die zweite und weitere Nukleinsäuren aus den ungeradzahligen Sequenzen, die in Tabelle 1 aufgelistet sind, gewählt sind, davon ausgenommen jegliches mit F bezeichnete Nukleinsäuremolekül.

8. Vektor gemäß einem beliebigen der Ansprüche 5 oder 6, bei dem es sich um einen Expressionsvektor handelt.

9. Wirtszelle, die mit dem Expressionsvektor von Anspruch 8 transfiziert ist, wobei die Zelle ein Mikroorganismus ist.

10. Wirtszelle gemäß Anspruch 9, wobei die Zelle zu der Gattung Corynebacterium oder Brevibacterium gehört.

11. Zelle gemäß Anspruch 10, gewählt aus der Gruppe, die aus Folgendem besteht:
*Corynebacterium glutamicum, Corynebacterium herculis, Corynebacterium lilium*, *Corynebacterium acetoacidophilum, Corynebacterium acetoglutamicum, Corynebacterium acetophilum, Corynebacterium ammoniagenes, Corynebacterium fujiokense, Corynebacterium nitrilophilus, Brevibacterium ammoniagenes, Brevibacterium butanicum, Brevibacterium divaricatum, Brevibacterium flavum, Brevibacterium heali, Brevibacterium ketoglutamicum, Brevibacterium ketosoreductum, Brevibacterium lactoermentum, Brevibacterium linens, Brevibacterium paraffinolyticum,* und jenen Stämmen, wie in der Tabelle 3 aufgeführt.

12. Wirtszelle gemäß einem beliebigen der Ansprüche 9, 10 oder 11, wobei die Expression des Nukleinsäuremoleküls zu der Modulation der Produktion von Methionin und/oder Lysin führt.

13. Verfahren zur Herstellung eines Polypeptids gemäß Anspruch 1, umfassend das Kultivieren der Wirtszelle von Anspruch 9 in einem geeigneten Kulturmedium.

14. Verfahren zur Herstellung einer Feinchemikalie, umfassend das Kultivieren einer Zelle gemäß Anspruch 12.

15. Verfahren gemäß Anspruch 14, wobei die Zelle in Gegenwart einer Schwefelquelle kultiviert wird.

16. Verfahren gemäß Anspruch 14, wobei das Verfahren weiter den Schritt des Rückgewinnens der Feinchemikalie aus der Kultur umfasst.

17. Verfahren gemäß einem beliebigen der Ansprüche 14, 15 oder 16, wobei die Feinchemikalie eine Aminosäure ist.

18. Verfahren gemäß Anspruch 17, wobei die Aminosäure Methionin oder Lysin ist.

19. Verfahren für die Herstellung einer Feinchemikalie, umfassend das Kultivieren einer Zelle, deren genomische DNA durch den Einschluss eines Nukleinsäuremoleküls von Anspruch 4 verändert wurde.

20. Verfahren gemäß Anspruch 19, wobei die genomische DNA der Zelle durch den Einschluss einer Nukleinsäure, die ein Protein codiert, das an metabolischen Stoffwechselwegen beteiligt ist, weiter verändert wurde.

21. Verfahren gemäß Anspruch 20, wobei die weitere Nukleinsäure aus den ungeradzahligen Sequenzen, die in der Tabelle 1 aufgelistet sind, gewählt ist, davon ausgenommen jegliches mit F bezeichnete Nukleinsäuremolekül.

22. Verfahren gemäß den Ansprüchen 19 oder 20, wobei das Nukleinsäuremolekül aus der Gruppe gewählt ist, die aus metC-, metB-, metA-, metE-, metH-, hom-, asd-, lysC-, lysC/ask-, rxa00657-, dapA-, dapB-, dapC-, dapD/argD-, dapE-, dapF-, LysA-, ddh-, lysE-, lysG-, lysR-, hsk-, ppc-, pycA-, accD-, accA-, accB-, addD-, gpdh-Genen oder jeder beliebigen Kombination der oben genannten Gene besteht.

## Revendications

1. Polypeptide isolé à partir de Corynebacterium glutamicum, ladite protéine étant impliquée dans le métabolisme de la méthionine et de la lysine et comprenant la séquence d'acides aminés décrite dans SEQ ID NO: 2.

2. Polypeptide isolé selon la revendication 1, comprenant en outre des séquences d'acides aminés hétérologues.

3. Molécule d'acide nucléique isolée à partir de Corynebacterium glutamicum codant pour la protéine de la revendication 1.

4. Acide nucléique isolé de la revendication 3, **caractérisé en ce que** ledit acide nucléique comprend la séquence nucléotidique décrite dans SEQ ID NO: 1 ou un complément de celle-ci.

5. Vecteur comprenant l'acide nucléique de la revendication 4.

6. Vecteur de la revendication 5, comprenant en outre un ou plusieurs acides nucléiques codant pour des protéines impliquées dans des voies métaboliques.

7. Vecteur de la revendication 6, **caractérisé en ce que** le deuxième acide nucléique et les suivants sont choisis parmi les séquences de numéros impairs répertoriées dans le tableau 1, à l'exclusion de toute molécule d'acide nucléique désignée par F.

8. Vecteur de l'une quelconque des revendications 5 ou 6, qui est un vecteur d'expression.

9. Cellule hôte transfectée avec le vecteur d'expression de la revendication 8, **caractérisée en ce que** ladite cellule est un micro-organisme.

10. Cellule hôte de la revendication 9, **caractérisée en ce que** la cellule appartient au genre Corynebacterium ou Brevibacterium.

11. Cellule de la revendication 10, choisie dans le groupe constitué de
*Corynebacterium glutamicum, Corynebacterium herculis, Corynebacterium lilium, Corynebacterium acetoacidophilum, Corynebacterium acetoglutamicum, Corynebacterium acetophilum, Corynebacterium ammoniagenes, Corynebacterium fujiokense, Corynebacterium nitrilophilus, Brevibacterium ammoniagenes, Brevibacterium butanicum, Brevibacterium divaricatum, Brevibacterium flavum, Brevibacterium heali, Brevibacterium ketoglutamicum, Brevibacterium ketosoreductum, Brevibacterium lactogermentum, Brevibacterium linens, Brevibacterium paraffinolyticum,* et les souches décrites dans le tableau 3.

12. Cellule hôte de l'une quelconque des revendications 9, 10 et 11, **caractérisée en ce que** l'expression de ladite molécule d'acide nucléique conduit à la modulation de la production de méthionine et/ou de lysine.

13. Procédé de production d'un polypeptide de la revendication 1 comprenant la culture de la cellule hôte de la revendication 9 dans un milieu de culture approprié.

14. Procédé de production d'une substance chimique finie, comprenant la culture d'une cellule de la revendication 12.

15. Procédé de la revendication 14, **caractérisé en ce que** ladite cellule est cultivée en présence d'une source de soufre.

16. Procédé de la revendication 14, **caractérisé en ce que** ledit procédé comprend en outre l'étape de récupération de la substance chimique fine à partir de ladite culture.

17. Procédé de l'une quelconque des revendications 14, 15 ou 16, **caractérisé en ce que** la substance chimique fine est un acide aminé.

18. Procédé de la revendication 17, **caractérisé en ce que** l'acide aminé est la méthionine ou la lysine.

19. Procédé pour produire une substance chimique fine comprenant la culture d'une cellule dont l'ADN génomique a été modifié par l'inclusion d'une molécule d'acide nucléique de la revendication 4.

20. Procédé de la revendication 19, **caractérisé en ce que** l'ADN génomique de la cellule a été modifié plus avant par l'inclusion d'un acide nucléique codant pour une protéine impliquée dans des voies métaboliques.

21. Procédé de la revendication 20, **caractérisé en ce que** ledit acide nucléique supplémentaire est choisi parmi les séquences de numéros impairs répertoriées dans le tableau 1, en excluant une molécule d'acide nucléique désignée par F quelconque.

22. Procédé des revendications 19 ou 20, **caractérisé en ce que** la molécule d'acide nucléique est choisie dans le groupe constitué des gènes metC, metB, metA, metE, metH, hom, asd, lysC, lysC/ask, rxa00657, dapA, dapB, dapC, dapD/argD, dapE, dapF, LysA, ddh, lysE, lysG, lysR, hsk, ppc, pycA, accD, accA, accB, addD, gpdh ou une combinaison quelconque des gènes mentionnés ci-dessus.
